# EUROPEAN PATENT APPLICATION

(11) **EP 4 317 139 A1**
(43) Date of publication of application: **07.02.2024**
(21) Application number: 22779044.1
(22) Date of filing: 30.03.2022
(51) Int. Cl.: C07D 257/04, C07D 249/02, A61P 31/10, A61K 31/675

(54) **PREPARATION METHOD FOR BICYCLIC COMPOUND AND APPLICATION AS ANTIFUNGAL AGENT**

(30) Priority: 30.03.2021 CN 202110354834; 13.08.2021 CN 202110933030; 09.09.2021 CN 202111057765; 07.12.2021 CN 202111512144; 23.03.2022 CN 202210294092
(71) Applicant: Shanghai Jemincare Pharmaceuticals Co., Ltd., Shanghai 201203 (CN); Jiangxi Jemincare Group Co., Ltd., Nanchang, Jiangxi 330000 (CN)
(72) Inventor: DENG, Gang, Shanghai 201203 (CN); YAO, Yucai, Shanghai 201203 (CN); LIU, Xiaobin, Shanghai 201203 (CN); LI, Zhongyao, Shanghai 201203 (CN); DAI, Ming, Shanghai 201203 (CN); HUAN, Rui, Shanghai 201203 (CN); TANG, Rui, Shanghai 201203 (CN); HUANG, Daochen, Shanghai 201203 (CN); ZHANG, Qiong, Shanghai 201203 (CN); WANG, Yu, Shanghai 201203 (CN); YE, Yan, Shanghai 201203 (CN); PENG, Jianbiao, Shanghai 201203 (CN)
(74) Representative: Henderson, Helen Lee
(86) International application number: PCT/CN2022/084203
(87) International publication number: WO 2022/206862

(57) **Abstract**

A compound represented by formula (I) and a pharmaceutically acceptable salt thereof, as well as an antifungal application of the compound.

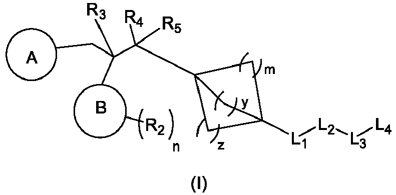

## Description

The present application claims the right of the following priorities:
CN202110354834.2, application date: March 30, 2021;
CN202110933030.8, application date: August 13, 2021;
CN202111057765.5, application date: September 09, 2021;
CN202111512144.1, application date: December 07, 2021;
CN202210294092.3, application date: March 23, 2022.

### TECHNICAL FIELD

The present disclosure relates to a preparation method for bicyclic compounds and an application thereof as an antifungal agent. The present disclosure also relates to a compound of formula (I) and a pharmaceutically acceptable salt thereof, and an antifungal application of the compound.

### BACKGROUND

In recent years, due to the long-term and widespread use of broad-spectrum antibiotics, an increase in radiotherapy and chemotherapy, the popularization of bone marrow and organ transplant surgeries, the rising use of immunosuppressants, and the advent of interventional treatments such as heart valve implants, there has been a significant upward trend in the incidence and mortality rates of clinical invasive fungal infections caused by candida, aspergillus, and cryptococcus neoformans. Annually, tens of millions of individuals worldwide contract fungal infections, with at least 1.5 million succumbing to deep fungal invasions.

Currently, antifungal drugs used in clinical practice include azoles, polyenes, echinocandins, etc. Azole antifungal drugs are the largest category among all antifungal drugs and are the most common antifungal drugs in clinical practice. They offer a broad antibacterial spectrum and less toxicity. Compared with amphotericin B, azoles exhibit better tolerance, making them the most widely used.

Despite their widespread use in clinical practice, these azole antifungal drugs each have their own set of drawbacks and limitations. For instance, ketoconazole has significant toxicity and side effects and is currently primarily used for topical applications. Fluconazole, as a first-line drug for the treatment of both topical and deep fungal infections, has limited activity. Furthermore, serious resistance has emerged due to its prolonged use. Itraconazole has poor water solubility and low bioavailability. The cyclodextrin contained in its oral solution can potentially cause osmotic diarrhea, which is hazardous in patients with renal dysfunction. Posaconazole is a potent CYP3A4 inhibitor. This strong drug-drug interaction (DDI) restricts its clinical application. Additionally, its physicochemical and metabolic properties are not ideal, which greatly reduces the stability of its therapeutic effect. Isavuconazole is a moderate CYP3A4 inhibitor, but issues with drug-drug interactions (DDI) still persist.

Voriconazole is considered the most successful derivative of fluconazole, which exhibits strong activity against deep-seated pathogenic fungi, including fluconazole-resistant strains like candida krusei and candida parapsilosis. Currently, it is the top choice for treating fungal infections, especially invasive fungal diseases caused by Aspergillus. Its drawback lies in that it is primarily metabolized by CYP2C19 *in vivo.* Due to individual variations in CYP2C 19 metabolism, there is a risk of elevated blood drug concentrations, which is especially prone to adverse reactions in Chinese people. Side effects after taking voriconazole, such as abnormal visual response and hepatic dysfunction, have also been reported.

In view of the defects and limitations of the existing conazole antifungal drugs, the development of novel, efficient, broad-spectrum, low-toxicity CYP51 inhibitor antifungal drugs superior to the existing drugs in terms of activity, metabolism, and drug-drug interactions to overcome the limitations and defects of the existing clinical drugs and overcome resistance to fluconazole and the like, is of great clinical value for the treatment and prevention of various fungal infections, especially deep infections caused by Candida and Aspergillus, as well as for the reduction of mortality rates of clinically invasive fungal infections.

### CONTENT OF THE PRESENT INVENTION

In a first aspect of the present disclosure, the present disclosure provides a compound of formula (I), an optical isomer thereof, a tautomer thereof, and a pharmaceutically acceptable salt thereof, 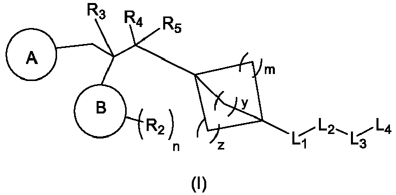 wherein
ring A is selected from 5- to 6-membered heteroaryl;
ring B is selected from phenyl and 5- to 6-membered heteroaryl;
R₃ is selected from OH, NH₂, halogen, C₁₋₆ alkyl, C₁₋₆ heteroalkyl, -OC(=O)C₁₋₆ alkyl, -NHC(=O)C₁₋₆ alkyl, 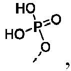 and -OC₁₋₆ alkyl-OP(=O)₂(OH)₂;
R₂, R₄, and R₅ are each independently selected from H, CN, OH, F, Cl, Br, I, C₁₋₆ alkyl, and C₁₋₆ heteroalkyl, and the C₁₋₆ alkyl or C₁₋₆ heteroalkyl is optionally substituted by 1, 2, or 3 CN, OH, F, Cl, Br, I, or C₁₋₆ alkyl groups;
m, y, and z are each independently selected from 1, 2, 3, or 4;
n is selected from 0, 1, 2, or 3;
L₁ is selected from a single bond, -NH-, C₁₋₆ alkyl, C₂₋₆ alkynyl, phenyl, and 5- to 6-membered heteroaryl, and the C₁₋₆ alkyl, C₂₋₆ alkynyl, phenyl, or 5- to 6-membered heteroaryl is optionally substituted by 1, 2, or 3 CN, OH, F, Cl, Br, I, or C₁₋₆ alkyl groups;
L₂ is selected from a single bond, O, S, NH, C₁₋₆ alkyl, C₁₋₆ heteroalkyl, 3- to 6-membered heterocyclyl, C₃₋₆ cycloalkyl, and phenyl-O-C₁₋₆ alkyl-, and the C₁₋₆ alkyl, C₁₋₆ heteroalkyl, 3- to 6-membered heterocyclyl, C₃₋₆ cycloalkyl, or phenyl-O-C₁₋₆ alkyl- is optionally substituted by 1, 2, or 3 CN, OH, F, Cl, Br, I, or C₁₋₆ alkyl groups;
L₃ is selected from a single bond, O, NH, -C(=O)-, -C(=O)NH-, C₁₋₆ alkyl, 3- to 6-membered heterocyclyl, C₃₋₆ cycloalkyl, phenyl, and 5- to 6-membered heteroaryl, and the C₁₋₆ alkyl, 3- to 6-membered heterocyclyl, C₃₋₆ cycloalkyl, phenyl, or 5- to 6-membered heteroaryl is optionally substituted by 1, 2, or 3 CN, CF₃, OH, F, Cl, Br, I, or C₁₋₆ alkyl groups;
L₄ is selected from H, F, Cl, Br, I, OH, CN, NH₂, COOH, 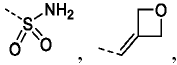 C(=O)C₁₋₆ alkyl, C(=O)NHC₁₋₆ alkyl, C(=O)N(C₁₋₆ alkyl)₂, C₁₋₆ alkyl, C₁₋₆ heteroalkyl, C₃₋₆ cycloalkyl, 4- to 6-membered heterocyclyl, C₁₋₆ alkyl-5- to 6-membered heterocyclyl, 5- to 6-membered heteroaryl, and benzo 4- to 6-membered heterocyclyl, and the C(=O)C₁₋₆ alkyl, C(=O)NHC₁₋₆ alkyl, C(=O)N(C₁₋₆ alkyl)₂, C₁₋₆ alkyl, C₁₋₆ heteroalkyl, C₃₋₆ cycloalkyl, 4- to 6-membered heterocyclyl, C₁₋₆ alkyl-5- to 6-membered heterocyclyl, 5- to 6- membered heteroaryl, or benzo 5- to 6-membered heterocyclyl is optionally substituted by 1, 2, 3, 4, or 5 R_{L};
R_{L} is selected from CN, OH, F, Cl, Br, I, NH₂, C(=O)C₁₋₆ alkyl, C(=O)NHC₁₋₆ alkyl, C(=O)N(C₁₋₆ alkyl)₂, C₁₋₆ alkyl, and C₁₋₆ heteroalkyl, and the C(=O)C₁₋₆ alkyl, C(=O)NHC₁₋₆ alkyl, C(=O)N(C₁₋₆ alkyl)₂, C₁₋₆ alkyl, or C₁₋₆ heteroalkyl is optionally substituted by 1, 2, or 3 CN, OH, NH₂, F, Cl, Br, I, or C₁₋₆ alkyl groups;
the C₁₋₆ heteroalkyl, 3- to 6-membered heterocyclyl, 4- to 6-membered heterocyclyl, 5- to 6-membered heterocyclyl, or 5- to 6-membered heteroaryl comprises 1, 2, 3, or 4 heteroatoms or heteroatom groups independently selected from -O-, -NH-, -N=, -S-, -C(=O)-, -C(=O)O-, -S(=O)-, -S(=O)₂-, and N.

In a second aspect of the present disclosure, the present disclosure also provides a compound of formula (II), an optical isomer thereof, or a tautomer thereof, 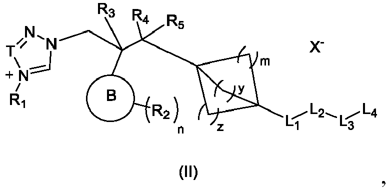
wherein X⁻ is a pharmaceutically acceptable anion;
T is selected from CH or N;
R₁ is selected from 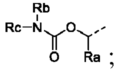
each Ra is independently selected from H and C₁₋₆ alkyl;
each Rb is independently selected from H, C₁₋₆ alkyl, -C(=O)C₁₋₆ alkyl, -OC(=O)C₁₋₆ alkyl, -C(=O)OC₁₋₆ alkyl, and phenyl, and the C₁₋₆ alkyl, -C(=O)C₁₋₆ alkyl, -OC(=O)C₁₋₆ alkyl, -C(=O)OC₁₋₆ alkyl, and phenyl are optionally substituted by 1, 2, or 3 CN, OH, F, Cl, Br, I, or C₁₋₆ alkyl groups;
each Rc is independently selected from H, C₁₋₆ alkyl, phenyl, or 5- to 6-membered heteroaryl, and the phenyl or 5- to 6-membered heteroaryl is optionally substituted by 1, 2, or 3 R;
each R is independently selected from CN, OH, F, Cl, Br, I, C₁₋₆ alkyl, C₁₋₆ heteroalkyl, 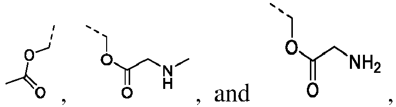 and the C₁₋₆ alkyl or C₁₋₆ heteroalkyl is optionally substituted by 1, 2, or 3 CN, OH, F, Cl, Br, I, or C₁₋₆ alkyl groups;
alternatively, Rb and Rc are linked together to form a 5- to 6-membered heterocyclyl ring, and the 5- to 6-membered heterocyclyl ring is optionally substituted by 1, 2, or 3 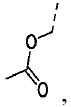 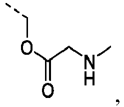 CN, OH, F, Cl, Br, I, or C₁₋₆ alkyl groups;
ring B is selected from phenyl and 5- to 6-membered heteroaryl;
R₃ is selected from OH, NH₂, halogen, C₁₋₆ alkyl, C₁₋₆ heteroalkyl, -OC(=O)C₁₋₆ alkyl, -NHC(=O)C₁₋₆ alkyl, 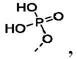 and -OC₁₋₆ alkyl-OP(=O)₂(OH)₂;
R₂, R₄, and R₅ are each independently selected from H, CN, OH, F, Cl, Br, I, C₁₋₆ alkyl, and C₁₋₆ heteroalkyl, and the C₁₋₆ alkyl or C₁₋₆ heteroalkyl is optionally substituted by 1, 2, or 3 CN, OH, F, Cl, Br, I, or C₁₋₆ alkyl groups;
m, y, and z are each independently selected from 1, 2, 3, or 4;
n is selected from 0, 1, 2, or 3;
L₁ is selected from a single bond, -NH-, C₁₋₆ alkyl, C₂₋₆ alkynyl, phenyl, and 5- to 6-membered heteroaryl, and the C₁₋₆ alkyl, C₂₋₆ alkynyl, phenyl, or 5- to 6-membered heteroaryl is optionally substituted by 1, 2, or 3 CN, OH, F, Cl, Br, I, or C₁₋₆ alkyl groups;
L₂ is selected from a single bond, O, S, NH, C₁₋₆ alkyl, C₁₋₆ heteroalkyl, 3- to 6-membered heterocyclyl, C₃₋₆ cycloalkyl, and phenyl-O-C₁₋₆ alkyl-, and the C₁₋₆ alkyl, C₁₋₆ heteroalkyl, 3- to 6-membered heterocyclyl, C₃₋₆ cycloalkyl, or phenyl-O-C₁₋₆ alkyl- is optionally substituted by 1, 2, or 3 CN, OH, F, Cl, Br, I, or C₁₋₆ alkyl groups;
L₃ is selected from a single bond, O, NH, -C(=O)-, -C(=O)NH-, C₁₋₆ alkyl, 3- to 6-membered heterocyclyl, C₃₋₆ cycloalkyl, phenyl, and 5- to 6-membered heteroaryl, and the C₁₋₆ alkyl, 3- to 6-membered heterocyclyl, C₃₋₆ cycloalkyl, phenyl, or 5- to 6-membered heteroaryl is optionally substituted by 1, 2, or 3 CN, CF₃, OH, F, Cl, Br, I, or C₁₋₆ alkyl groups;
L₄ is selected from H, F, Cl, Br, I, OH, CN, NH₂, COOH, 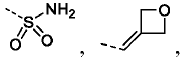 C(=O)C₁₋₆ alkyl, C(=O)NHC₁₋₆ alkyl, C(=O)N(C₁₋₆ alkyl)₂, C₁₋₆ alkyl, C₁₋₆ heteroalkyl, C₃₋₆ cycloalkyl, 4- to 6-membered heterocyclyl, C₁₋₆ alkyl-5- to 6-membered heterocyclyl, 5- to 6- membered heteroaryl, and benzo 5- to 6-membered heterocyclyl, and the C(=O)C₁₋₆ alkyl, C(=O)NHC₁₋₆ alkyl, C(=O)N(C₁₋₆ alkyl)₂, C₁₋₆ alkyl, C₁₋₆ heteroalkyl, C₃₋₆ cycloalkyl, 4- to 6-membered heterocyclyl, C₁₋₆ alkyl-5- to 6-membered heterocyclyl, 5- to 6- membered heteroaryl, or benzo 5- to 6-membered heterocyclyl is optionally substituted by 1, 2, 3, 4, or 5 R_{L};
R_{L} is selected from CN, OH, F, Cl, Br, I, NH₂, 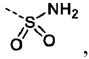 C(=O)C₁₋₆ alkyl, C(=O)NHC₁₋₆ alkyl, C(=O)N(C₁₋₆ alkyl)₂, C₁₋₆ alkyl, and C₁₋₆ heteroalkyl, and the C(=O)C₁₋₆ alkyl, C(=O)NHC₁₋₆ alkyl, C(=O)N(C₁₋₆ alkyl)₂, C₁₋₆ alkyl, or C₁₋₆ heteroalkyl is optionally substituted by 1, 2, or 3 CN, OH, NH₂, F, Cl, Br, I, or C₁₋₆ alkyl groups;
the C₁₋₆ heteroalkyl, 3- to 6-membered heterocyclyl, 4- to 6-membered heterocyclyl, 5- to 6-membered heterocyclyl, or 5- to 6-membered heteroaryl comprises 1, 2, 3, or 4 heteroatoms or heteroatom groups independently selected from -O-, -NH-, -N=, -S-, -C(=O)-, -C(=O)O-, -S(=O)-, -S(=O)₂-, and N.

In yet another aspect of the present disclosure, the present disclosure also provides formula (I-A), an optical isomer thereof, a tautomer thereof, and a pharmaceutically acceptable salt thereof, 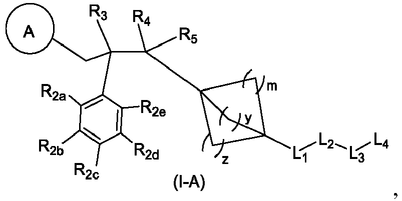 wherein
ring A, R₃, R₄, R₅, L₁, L₂, L₃, L₄, m, y, and z are as defined above, and each R₂ₐ, R_{2b}, R_{2c}, R_{2d}, and R₂ₑ is independently selected from H, CN, OH, F, Cl, Br, I, C₁₋₆ alkyl, and C₁₋₆ heteroalkyl, and the C₁₋₆ alkyl or C₁₋₆ heteroalkyl is optionally substituted by 1, 2, or 3 CN, OH, F, Cl, Br, I, or C₁₋₆ alkyl groups.

In yet another aspect of the present disclosure, the present disclosure also provides a compound of formula (II-A), an optical isomer thereof, or a tautomer thereof, 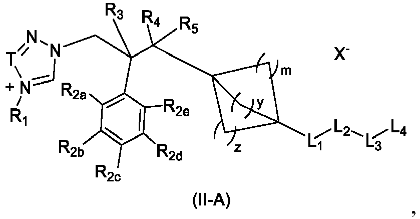 wherein
R₁, R₃, R₄, R₅, L₁, L₂, L₃, L₄, T, m, y, z, and X⁻ are as defined above, and each R₂ₐ, R_{2b}, R_{2c}, R_{2d}, and R₂ₑ is independently selected from H, CN, OH, F, Cl, Br, I, C₁₋₆ alkyl, and C₁₋₆ heteroalkyl, and the C₁₋₆ alkyl or C₁₋₆ heteroalkyl is optionally substituted by 1, 2, or 3 CN, OH, F, Cl, Br, I, or C₁₋₆ alkyl groups.

In some embodiments of the present disclosure, the X⁻ is selected from Cl⁻, I⁻, Br⁻, HSO₄⁻, 1/2 SO₄²⁻, COO⁻, CH₃COO⁻, CF₃COO⁻, and CF₃CH₃COO⁻.

In some embodiments of the present disclosure, the ring A is selected from 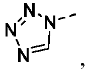 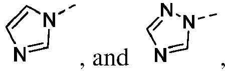 and other variables are as defined in the present disclosure.

In some embodiments of the present disclosure, the R₃ is selected from OH, NH₂, C₁₋₃ alkyl, C₁₋₃ alkoxy, C₁₋₃ alkylamino, C₁₋₃ alkylthio, -OC(=O)C₁₋₃ alkyl, and -NHC(=O)C₁₋₃ alkyl, and other variables are as defined in the present disclosure.

In some embodiments of the present disclosure, the R₃ is selected from OH, F, Cl, Br, I, NH₂, C₁₋₃ alkyl, C₁₋₃ alkoxy, C₁₋₃ alkylamino, C₁₋₃ alkylthio, 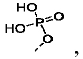 -OC₁₋₃ alkyl-OP(=O)₂(OH)₂, -OC(=O)C₁₋₃ alkyl, and -NHC(=O)C₁₋₃ alkyl, and other variables are as defined in the present disclosure.

In some embodiments of the present disclosure, the R₃ is selected from OH, F, Cl, Br, NH₂, OCH₃, 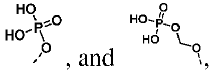 and other variables are as defined in the present disclosure.

In some embodiments of the present disclosure, each Ra is independently selected from H, methyl, ethyl, n-propyl, and isopropyl, and other variables are as defined in the present disclosure.

In some embodiments of the present disclosure, each Rb is independently selected from H, C₁₋₃ alkyl, -C(=O)C₁₋₃ alkyl, -OC(=O)C₁₋₃ alkyl, -C(=O)OC₁₋₃ alkyl, and phenyl, and the C₁₋₃ alkyl, -C(=O)C₁₋₃ alkyl, -OC(=O)C₁₋₃ alkyl, -C(=O)OC₁₋₃ alkyl, and phenyl are optionally substituted by 1, 2, or 3 CN, OH, F, Cl, Br, I, or C₁₋₃ alkyl groups, and other variables are as defined in the present disclosure.

In some embodiments of the present disclosure, the Rb is selected from H, CH₃, 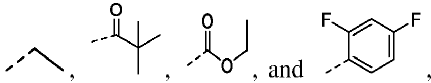 and other variables are as defined in the present disclosure.

In some embodiments of the present disclosure, the Rc is selected from H, -C₁₋₆ alkyl-OC(=O)C₁₋₆ alkyl-NH-C₁₋₆ alkyl, -C₁₋₆ alkyl-NH-C₁₋₆ alkyl, -C₁₋₆ alkyl-OC(=O) -C₁₋₆ alkyl, - phenyl-C₁₋₆ alkyl-OC(=O)-C₁₋₆ alkyl, -phenyl-C₁₋₆ alkyl-OC(=O)-C₁₋₆ alkyl-NH-C₁₋₆ alkyl, -5-to 6-membered heteroaryl-C₁₋₆ alkyl-NH-C₁₋₆ alkyl, -5- to 6-membered heteroaryl-C₁₋₆ alkyl-OC(=O)-C₁₋₆ alkyl, -5- to 6-membered heteroaryl-C₁₋₆ alkyl-OC(=O)-C₁₋₆ alkyl-NH-C₁₋₆ alkyl, and -5- to 6-membered heteroaryl-C₁₋₆ alkyl-NH-C₁₋₆ alkyl, and the -C₁₋₆ alkyl-OC(=O)C₁₋₆ alkyl-NH-C₁₋₆ alkyl, -C₁₋₆ alkyl-NH-C₁₋₆ alkyl, -C₁₋₆ alkyl-OC(=O) -C₁₋₆ alkyl, -phenyl-C₁₋₆ alkyl-OC(=O)-C₁₋₆ alkyl, -phenyl-C₁₋₆ alkyl-OC(=O)-C₁₋₆ alkyl-NH-C₁₋₆ alkyl, -5- to 6-membered heteroaryl-C₁₋₆ alkyl-NH-C₁₋₆ alkyl, -5- to 6-membered heteroaryl-C₁₋₆ alkyl-OC(=O)-C₁₋₆ alkyl, -5- to 6-membered heteroaryl-C₁₋₆ alkyl-OC(=O)-C₁₋₆ alkyl-NH-C₁₋₆ alkyl, or -5- to 6-membered heteroaryl-C₁₋₆ alkyl-NH-C₁₋₆ alkyl is optionally substituted by 1, 2, 3, or 4 F, Cl, Br, I, methoxy groups, or CN, and other variables are as defined in the present disclosure.

In some embodiments of the present disclosure, the Rc is selected from H, -C₁₋₃ alkyl-OC(=O)C₁₋₃ alkyl-NH-C₁₋₃ alkyl, -C₁₋₃ alkyl-NH-C₁₋₃ alkyl, -C₁₋₃ alkyl-OC(=O) -C₁₋₃ alkyl, - phenyl-C₁₋₃ alkyl-OC(=O)-C₁₋₃ alkyl, -phenyl-C₁₋₃ alkyl-OC(=O)-C₁₋₃ alkyl-NH-C₁₋₃ alkyl, - pyridyl-C₁₋₃ alkyl-NH-C₁₋₃ alkyl, -pyridyl-C₁₋₃ alkyl-OC(=O)-C₁₋₃ alkyl, -pyridyl-C₁₋₆ alkyl-OC(=O)-C₁₋₆ alkyl-NH-C₁₋₆ alkyl, and -pyridyl-C₁₋₆ alkyl-NH-C₁₋₆ alkyl, and the -C₁₋₃ alkyl-OC(=O)C₁₋₃ alkyl-NH-C₁₋₃ alkyl, -C₁₋₃ alkyl-NH-C₁₋₃ alkyl, -C₁₋₃ alkyl-OC(=O) -C₁₋₃ alkyl, - phenyl-C₁₋₃ alkyl-OC(=O)-C₁₋₃ alkyl, -phenyl-C₁₋₃ alkyl-OC(=O)-C₁₋₃ alkyl-NH-C₁₋₃ alkyl, - pyridyl-C₁₋₃ alkyl-NH-C₁₋₃ alkyl, -pyridyl-C₁₋₃ alkyl-OC(=O)-C₁₋₃ alkyl, -pyridyl-C₁₋₃ alkyl-OC(=O)-C₁₋₃ alkyl-NH-C₁₋₃ alkyl, or -pyridyl-C₁₋₃ alkyl-NH-C₁₋₃ alkyl is optionally substituted by 1, 2, 3, or 4 F, Cl, Br, I, methoxy groups, or CN, and other variables are as defined in the present disclosure.

In some embodiments of the present disclosure, each Rc is independently selected from H, C₁₋₆ alkyl-O-C(=O)C₁₋₆ alkyl-NH-C₁₋₆ alkyl, C₁₋₆ alkyl-NH-C₁₋₆ alkyl, C₁₋₆ alkyl-C(=O)OC₁₋₆ alkyl, 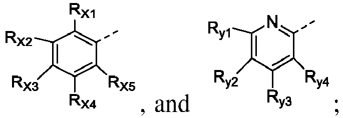 R_{X1}, R_{X2}, R_{X3}, R_{X4}, R_{X5}, R_{y1}, R_{y2}, R_{y3}, and R_{y4} are each independently selected from H, F, Cl, Br, I, CN, C₁₋₆ alkyl, C₁₋₆ alkoxy, C₁₋₆ alkylthio, C₁₋₆ alkylamino, C₁₋₆ alkyl-C(=O)OC₁₋₆ alkyl, C₁₋₆ alkyl-O-C(=O)C₁₋₆ alkyl-NH-C₁₋₆ alkyl, C₁₋₆ alkyl-NH-C₁₋₆ alkyl, and C₁₋₆ alkyl-O-C(=O)C₁₋₆ alkyl-NH₂, and the C₁₋₆ alkyl, C₁₋₆ alkoxy, C₁₋₆ alkylthio, C₁₋₆ alkylamino, C₁₋₆ alkyl-C(=O)OC₁₋₆ alkyl, C₁₋₆ alkyl-O-C(=O)C₁₋₆ alkyl-NH-C₁₋₆ alkyl, C₁₋₆ alkyl-NH-C₁₋₆ alkyl, or C₁₋₆ alkyl-O-C(=O)C₁₋₆ alkyl-NH₂ is optionally substituted by 1, 2, or 3 CN, OH, F, Cl, Br, I, or C₁₋₆ alkyl groups, and other variables are as defined in the present disclosure.

In some embodiments of the present disclosure, each Rc is independently selected from H, C₁₋₃ alkyl-O-C(=O)C₁₋₃ alkyl-NH-C₁₋₃ alkyl, C₁₋₃ alkyl-NH-C₁₋₃ alkyl, C₁₋₃ alkyl-C(=O)OC₁₋₃ alkyl, 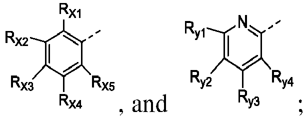 R_{X1}, R_{X2}, R_{X3}, R_{X4}, R_{X5}, R_{y1}, R_{y2}, R_{y3}, and R_{y4} are each independently selected from H, F, Cl, Br, I, CN, C₁₋₃ alkyl, C₁₋₃ alkoxy, C₁₋₃ alkylthio, C₁₋₃ alkylamino, C₁₋₃ alkyl-C(=O)OC₁₋₃ alkyl, C₁₋₃ alkyl-O-C(=O)C₁₋₃ alkyl-NH-C₁₋₃ alkyl, C₁₋₃ alkyl-NH-C₁₋₃ alkyl, and C₁₋₃ alkyl-O-C(=O)C₁₋₃ alkyl-NH₂, and the C₁₋₃ alkyl, C₁₋₃ alkoxy, C₁₋₃ alkylthio, C₁₋₃ alkylamino, C₁₋₃ alkyl-C(=O)OC₁₋₃ alkyl, C₁₋₃ alkyl-O-C(=O)C₁₋₃ alkyl-NH-C₁₋₃ alkyl, C₁₋₃ alkyl-NH-C₁₋₃ alkyl, or C₁₋₃ alkyl-O-C(=O)C₁₋₃ alkyl-NH₂ is optionally substituted by 1, 2, or 3 CN, OH, F, Cl, Br, I, or C₁₋₃ alkyl groups, and other variables are as defined in the present disclosure.

In some embodiments of the present disclosure, the Rc is selected from H, 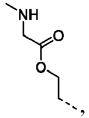 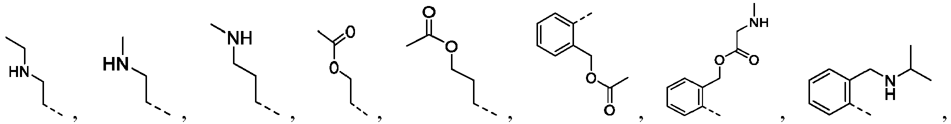 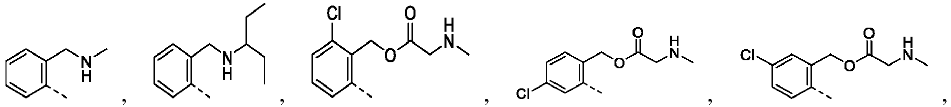 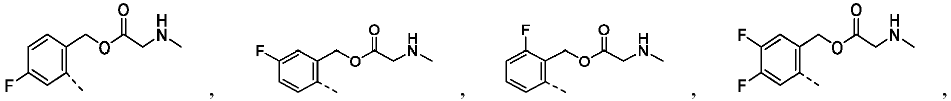 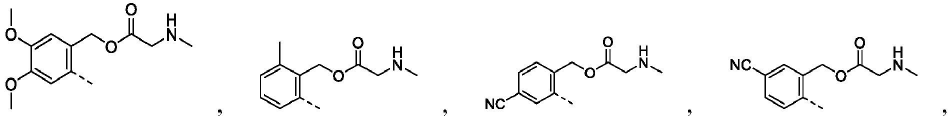 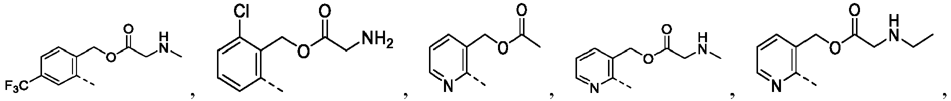 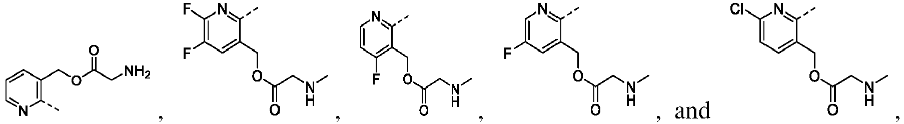 and other variables are as defined in the present disclosure.

In some embodiments of the present disclosure, the R₁ is selected from 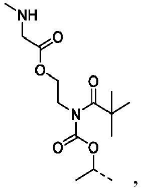 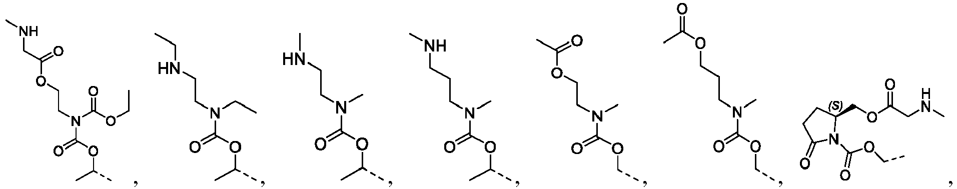 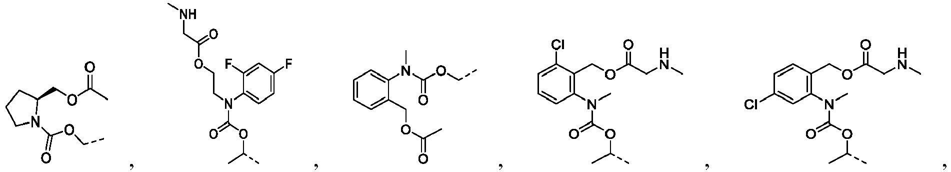 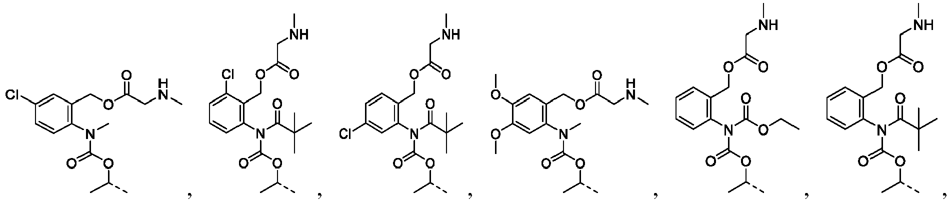 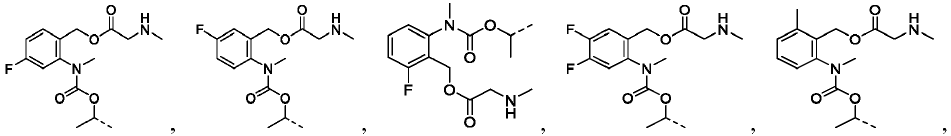 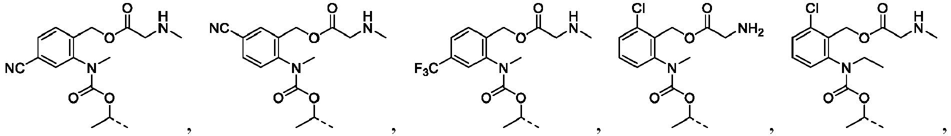 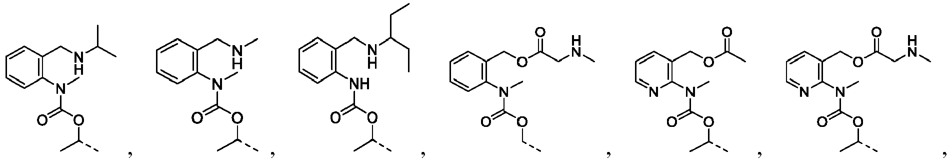 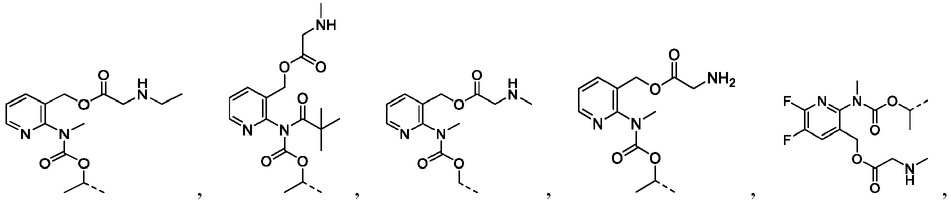 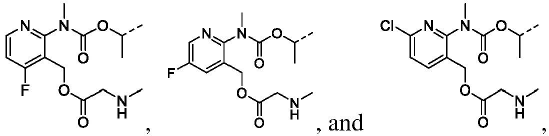 and other variables are as defined in the present disclosure.

In some embodiments of the present disclosure, the structural moiety 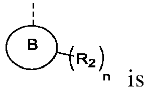 selected from 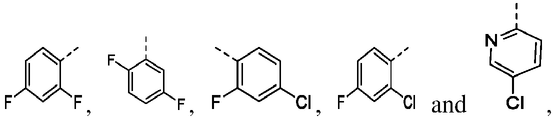 and other variables are as defined in the present disclosure.

In some embodiments of the present disclosure, the L₁ is selected from a single bond, C₁₋₃ alkyl, C₂₋₃ alkynyl, phenyl, pyridyl, thienyl, and oxazolyl, and the C₁₋₃ alkyl, C₂₋₃ alkynyl, phenyl, pyridyl, thienyl, or oxazolyl is optionally substituted by 1, 2, or 3 CN, OH F, Cl, Br, I, or C₁₋₆ alkyl groups, and other variables are as defined in the present disclosure.

In some embodiments of the present disclosure, the L₁ is selected from a single bond, -NH-, CH₂, 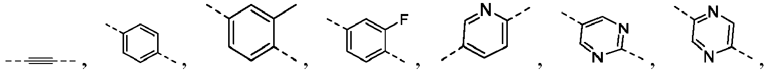 and 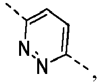 and other variables are as defined in the present disclosure.

In some embodiments of the present disclosure, the L₂ is selected from a single bond, O, S, C₁₋₃ alkyl, 5- to 6-membered heterocyclyl, C₅₋₆ cycloalkyl, and phenyl-O-C₁₋₃ alkyl-, and the C₁₋₃ alkyl, 5- to 6-membered heterocyclyl, C₅₋₆ cycloalkyl, or phenyl-O-C₁₋₃ alkyl- is optionally substituted by 1, 2, or 3 CN, OH, F, Cl, Br, I, or C₁₋₆ alkyl groups, and other variables are as defined in the present disclosure.

In some embodiments of the present disclosure, the L₂ is selected from a single bond, O, S, CH₂, NH, NCH₃, 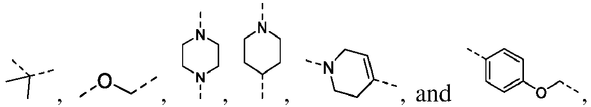 and other variables are as defined in the present disclosure.

In some embodiments of the present disclosure, the L₃ is selected from a single bond, 5- to 6- membered heterocyclyl, C₅₋₆ cycloalkyl, phenyl, and pyridyl, and the 5- to 6-membered heterocyclyl, C₅₋₆ cycloalkyl, phenyl, or pyridyl is optionally substituted by 1, 2, or 3 CN, OH, F, Cl, Br, I, or C₁₋₃ alkyl, and other variables are as defined in the present disclosure.

In some embodiments of the present disclosure, the L₃ is selected from a single bond, O, NH, CH₂, CH₂CH₂, -C(=O)-, -C(=O)NH-, 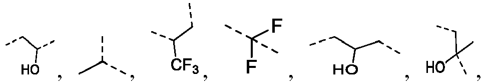 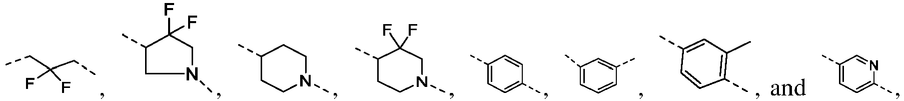 and other variables are as defined in the present disclosure.

In some embodiments of the present disclosure, the L₄ is selected from H, F, Cl, Br, I, OH, CN, NH₂, COOH, 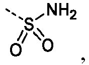 C(=O)C₁₋₃ alkyl, C(=O)NHC₁₋₃ alkyl, C(=O)N(C₁₋₃ alkyl)₂, C₁₋₃ alkyl, C₁₋₃ alkoxy, C₁₋₃ alkylthio, C₁₋₃ alkylamino, cyclopropyl, cyclobutyl, cyclopentyl, cyclohexyl, 2,4-dihydro-3H-1,2,4-triazol-3-one, piperazinyl, benzo-1,3-methylenedioxopentacyclyl, pyridyl, thiazolyl, 1,2,4-oxadiazolyl, pyrrolidinyl, tetrahydrofuranyl, piperidinyl, azetidinyl, morpholinyl, and 1,3-dioxolan-2-one, and the C(=O)C₁₋₃ alkyl, C(=O)NHC₁₋₃ alkyl, C(=O)N(C₁₋₃ alkyl)₂, C₁₋₃ alkyl, C₁₋₃ alkoxy, C₁₋₃ alkylthio, C₁₋₃ alkylamino, cyclopropyl, cyclobutyl, cyclopentyl, cyclohexyl, 2,4-dihydro-3H-1,2,4-triazol-3-one, piperazinyl, benzo-1,3-methylenedioxopentacyclyl, pyridyl, thiazolyl, 1,2,4-oxadiazolyl, pyrrolidinyl, tetrahydrofuranyl, piperidinyl, azetidinyl, morpholinyl, or 1,3-dioxolan-2-one is optionally substituted by 1, 2, 3, 4, or 5 R_{L}, and other variables are as defined in the present disclosure.

In some embodiments of the present disclosure, the R_{L} is selected from CN, OH, F, Cl, Br, I, NH₂, C(=O)C₁₋₃ alkyl, C(=O)NHC₁₋₃ alkyl, C(=O)N(C₁₋₃ alkyl)₂, C₁₋₃ alkyl, C₁₋₃ alkoxy, C₁₋₃ alkylthio, and C₁₋₃ alkylamino, and the C(=O)C₁₋₃ alkyl, C(=O)NHC₁₋₃ alkyl, C(=O)N(C₁₋₃ alkyl)₂, C₁₋₃ alkyl, C₁₋₃ alkoxy, C₁₋₃ alkylthio, or C₁₋₃ alkylamino is optionally substituted by 1, 2, or 3 CN, OH, NH₂, F, Cl, Br, I, or C₁₋₆ alkyl groups, and other variables are as defined in the present disclosure.

In some embodiments of the present disclosure, the L₄ is selected from H, F, Cl, Br, I, OH, CN, CH₃, CH₂CF₃, NH₂, CHF₂, CF₃, OCH₃, OCF₃, OCHF₂, OCH₂CH₃, COOH, CONHMe, CONMe₂, NMe₂, CH₂OH, 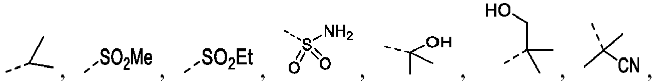 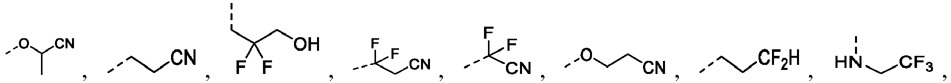 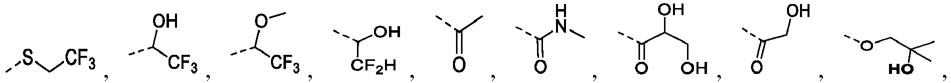 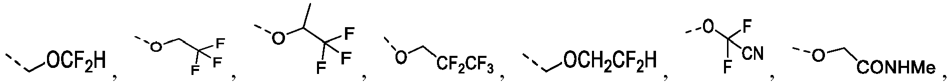 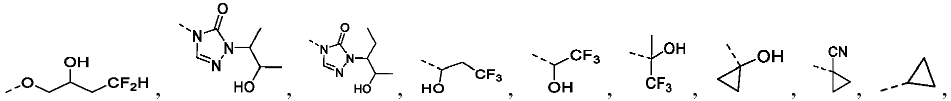 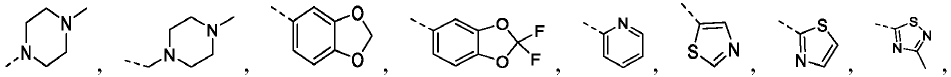 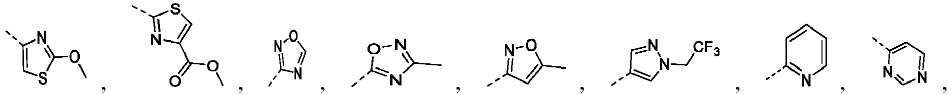 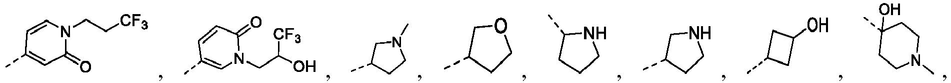 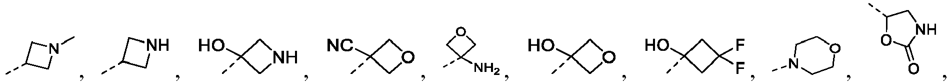 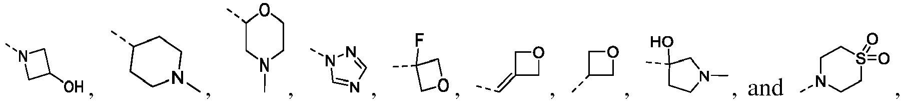 and other variables are as defined in the present disclosure.

In some embodiments of the present disclosure, the structural moiety 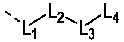 is selected from H, I, CN, OH, COOH, C₁₋₆ alkyl, C₁₋₆ heteroalkyl, -C(=O)-C₁₋₆ alkyl, - C(=O)NHC₁₋₆ alkyl, -C(=O)N(C₁₋₆ alkyl)₂, C₃₋₆ cycloalkyl, 3- to 6-membered heterocyclyl, benzo 3- to 6-membered heterocyclyl, phenyl, 5- to 6-membered heteroaryl, -phenyl-O-C₁₋₆ alkyl, -phenyl-O-C₁₋₆ alkyl- C(=O)NHC₁₋₆ alkyl, -phenyl-S-C₁₋₆ alkyl, -phenyl-NH-C₁₋₆ alkyl, -5- to 6-membered heteroaryl-O-C₁₋₆ alkyl, -5- to 6-membered heteroaryl-S-C₁₋₆ alkyl, -5- to 6-membered heteroaryl-NH-C₁₋₆ alkyl, -phenyl-O-C₁₋₆ alkyl-O-C₁₋₆ alkyl, -phenyl-C(=O)NHC₁₋₆ alkyl, -phenyl-O-C₁₋₆ alkyl-C(=O)OH, -phenyl-O-S(=O)₂NH₂, -C₁₋₆ alkyl-O-phenyl, -C₁₋₆ alkyl-O-phenyl-C₁₋₆ alkyl, -C₁₋₆ alkyl-phenyl, -phenyl-3- to 6- membered heterocyclyl, -phenyl-O-3- to 6- membered heterocyclyl, -phenyl-O-C₃₋₆ cycloalkyl, -phenyl-O- C₁₋₆ alkyl-C₃₋₆ cycloalkyl, -phenyl-O-C₁₋₆ alkyl-3- to 6-membered heterocyclyl, -phenyl-O-C₁₋₆ alkyl-C(=O)-3- to 6-membered heterocyclyl, -phenyl-O-5- to 6-membered heteroaryl, - phenyl-O-C₁₋₆ alkyl-5- to 6-membered heteroaryl, -phenyl-3- to 6-membered heterocyclyl-phenyl-3- to 6-membered heterocyclyl-C₁₋₆ alkyl, -phenyl-3- to 6-membered heterocyclyl- -C₁₋₆ alkyl, -C₂₋₆ alkenyl-phenyl-O- C₁₋₆ alkyl-phenyl, -C₂₋₆ alkenyl-phenyl-C₁₋₆ alkyl, -C₁₋₆ alkyl-3- to 6-membered heterocyclyl-O-phenyl, -phenyl-5- to 6-membered heteroaryl, -5- to 6-membered heteroaryl-3- to 6-membered heterocyclyl, -phenyl-O-3- to 6-membered heterocyclyl-C(=O)-C₁₋₆ alkyl, -phenyl-O-5- to 6-membered heteroaryl-C(=O)O-C₁₋₆ alkyl, and -5- to 6-membered heteroaryl-5- to 6-membered heteroaryl, and the C₁₋₆ alkyl, C₁₋₆ heteroalkyl, -C(=O)-C₁₋₆ alkyl, -C(=O)NHC₁₋₆ alkyl, -C(=O)N(C₁₋₆ alkyl)₂, C₃₋₆ cycloalkyl, 3-to 6- membered heterocyclyl, benzo 3- to 6- membered heterocyclyl, phenyl, 5- to 6-membered heteroaryl, -phenyl-O-C₁₋₆ alkyl, -phenyl-O-C₁₋₆ alkyl- C(=O)NHC₁₋₆ alkyl, -phenyl-S-C₁₋₆ alkyl, -phenyl-NH-C₁₋₆ alkyl, -5- to 6-membered heteroaryl-O-C₁₋₆ alkyl, -5- to 6-membered heteroaryl-S-C₁₋₆ alkyl, -5- to 6-membered heteroaryl-NH-C₁₋₆ alkyl, -phenyl-O-C₁₋₆ alkyl-O-C₁₋₆ alkyl, -phenyl-C(=O)NHC₁₋₆ alkyl, -phenyl-O-C₁₋₆ alkyl-C(=O)OH, -phenyl-O-S(=O)₂NH₂, -C₁₋₆ alkyl-O-phenyl, -C₁₋₆ alkyl-O-phenyl-C₁₋₆ alkyl, -C₁₋₆ alkyl-phenyl, -phenyl-3- to 6- membered heterocyclyl, -phenyl-O-3- to 6- membered heterocyclyl, -phenyl-O-C₃₋₆ cycloalkyl, -phenyl-O- C₁₋₆ alkyl-C₃₋₆ cycloalkyl, -phenyl-O-C₁₋₆ alkyl-3- to 6- membered heterocyclyl, -phenyl-O-C₁₋₆ alkyl-C(=O)-3- to 6- membered heterocyclyl, -phenyl-O-5- to 6-membered heteroaryl, -phenyl-O-C₁₋₆ alkyl-5- to 6-membered heteroaryl, -phenyl-3- to 6-membered heterocyclyl-phenyl-3- to 6- membered heterocyclyl-C₁₋₆ alkyl, -phenyl-3- to 6-membered heterocyclyl- -C₁₋₆ alkyl, -C₂₋₆ alkenyl-phenyl-O- C₁₋₆ alkyl-phenyl, -C₂₋₆ alkenyl-phenyl-C₁₋₆ alkyl, -C₁₋₆ alkyl-3- to 6- membered heterocyclyl-O-phenyl, -phenyl-5- to 6-membered heteroaryl, -5- to 6-membered heteroaryl-3- to 6- membered heterocyclyl, -phenyl-O-3- to 6- membered heterocyclyl-C(=O)-C₁₋₆ alkyl, -phenyl-O-5- to 6-membered heteroaryl-C(=O)O-C₁₋₆ alkyl, or -5- to 6-membered heteroaryl-5- to 6-membered heteroaryl is optionally substituted by 1, 2, 3, 4, 5, or 6 F, Cl, Br, I, CN, CF₃, OH, COOH, C₁₋₆ alkyl, or C₁₋₆ heteroalkyl groups.

In some embodiments of the present disclosure, the structural moiety 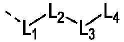 is selected from H, I, CN, OH, CH₃, NHCH₃, CF₃, C(=O)OH, C(=O)NHMe, C(=O)NMe₂, 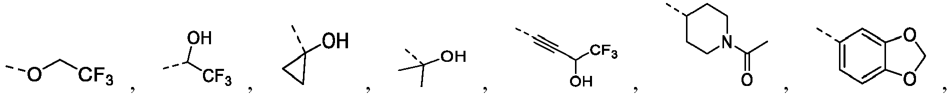 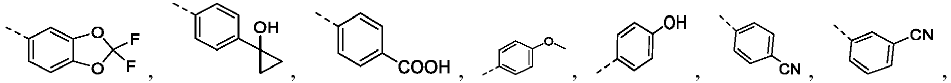 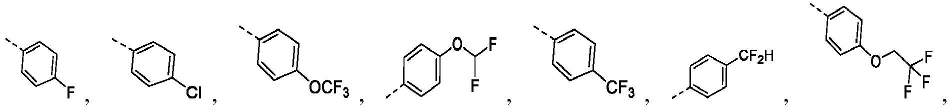 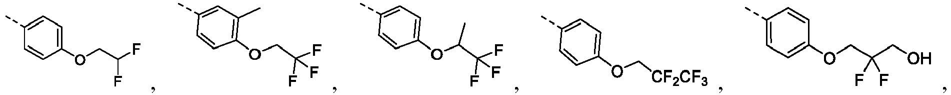 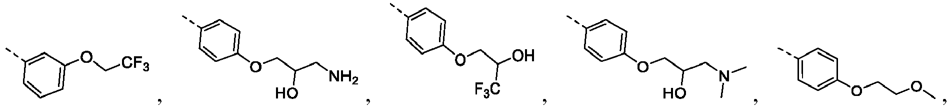 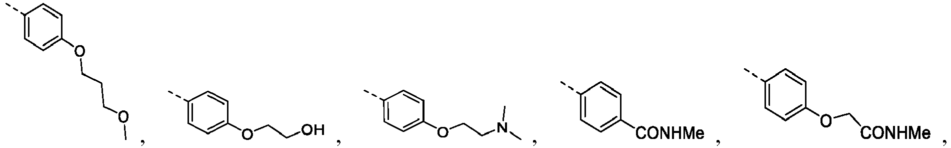 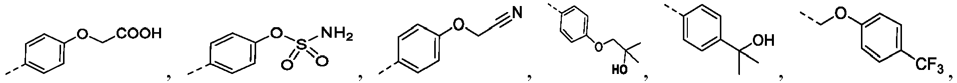 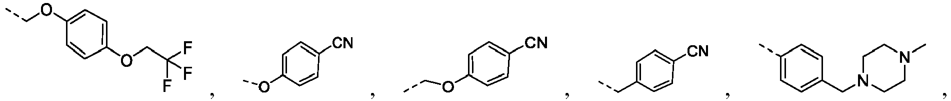 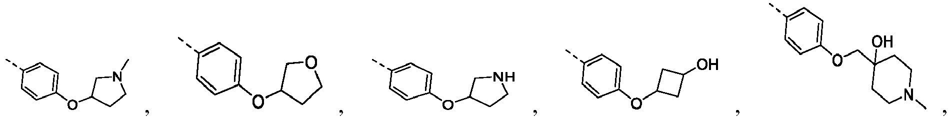 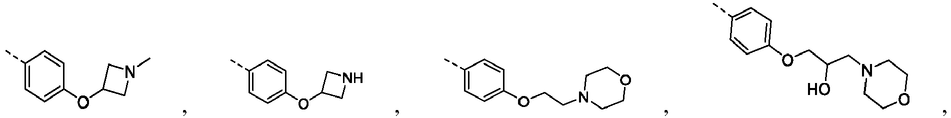 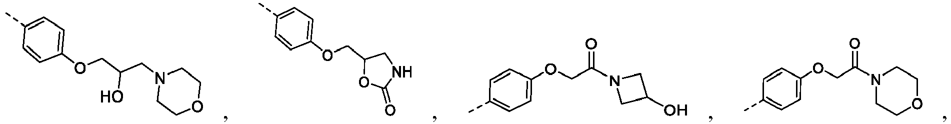 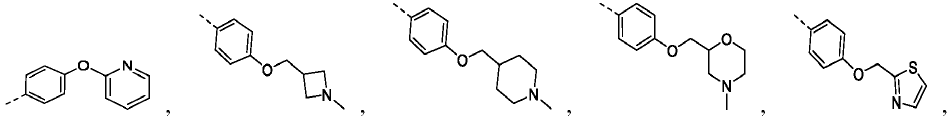 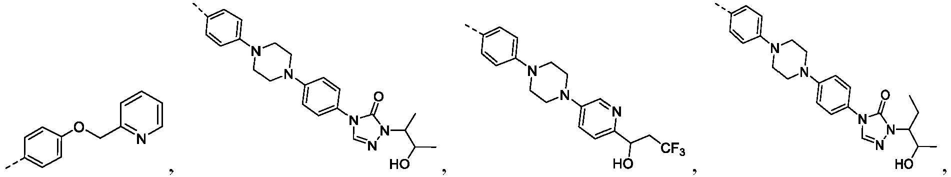 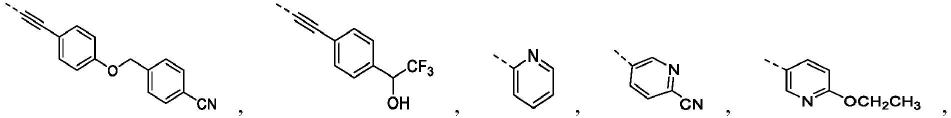 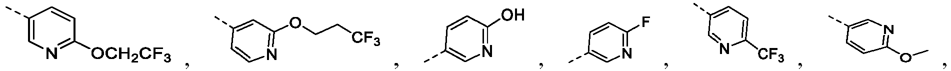 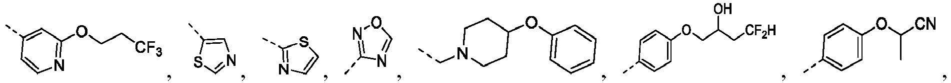 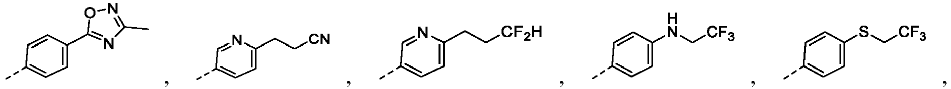 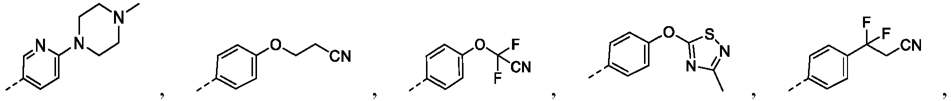 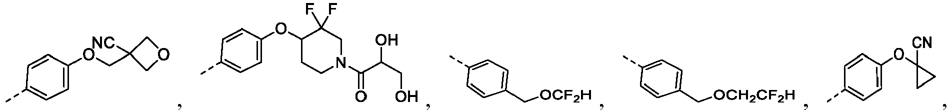 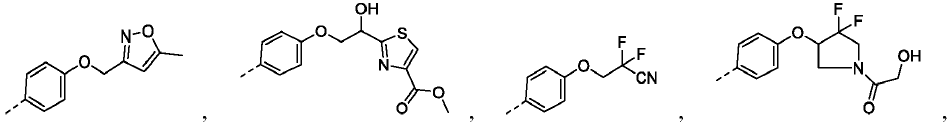 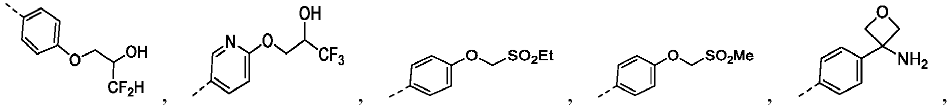 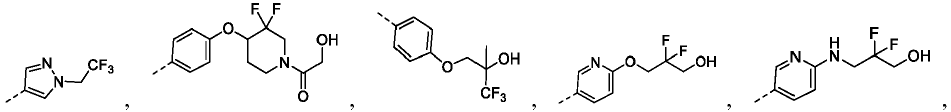 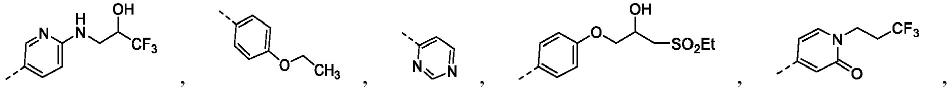 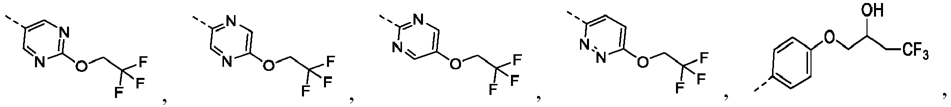 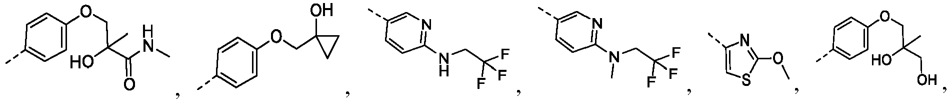 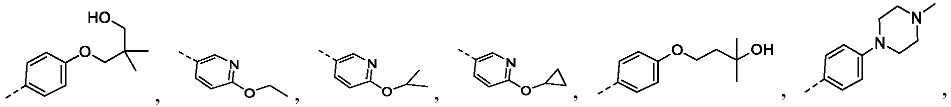 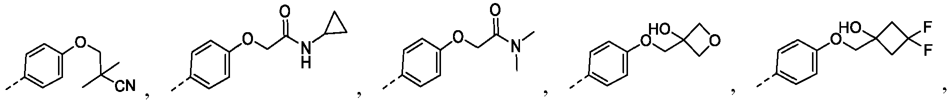 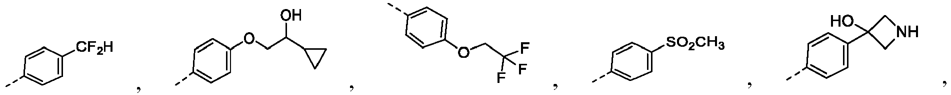 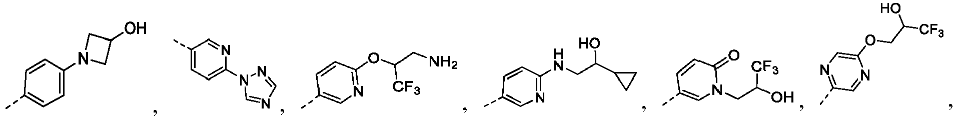 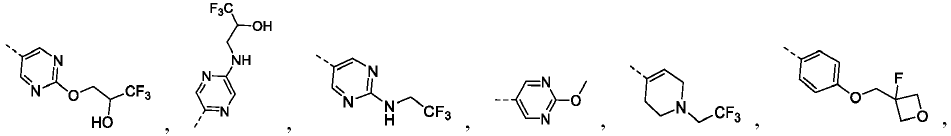 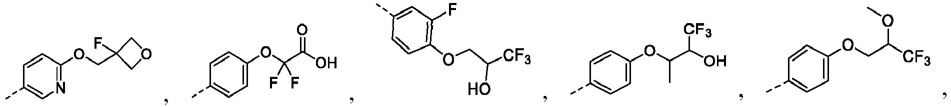 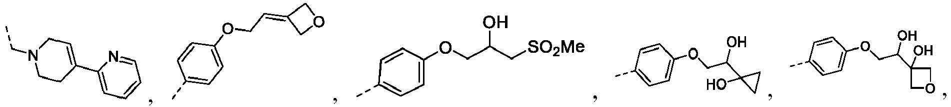 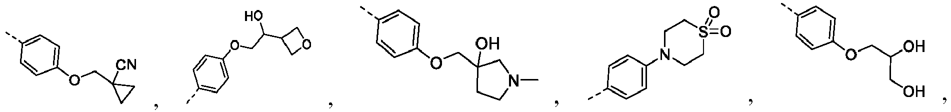 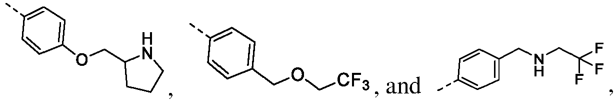 and other variables are as defined in the present disclosure.

In yet another aspect of the present disclosure, the present disclosure also provides a compound of the following formula, an optical isomer thereof, a tautomer thereof, and a pharmaceutically acceptable salt thereof, which is selected from

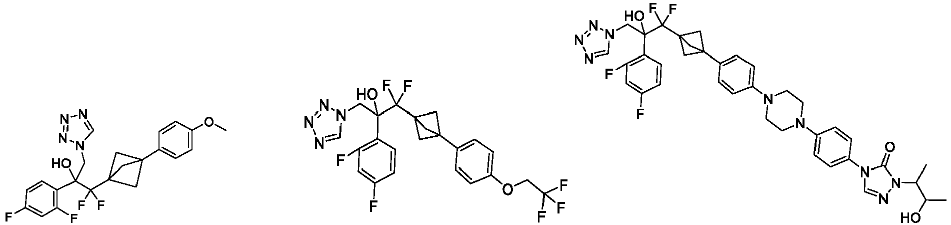

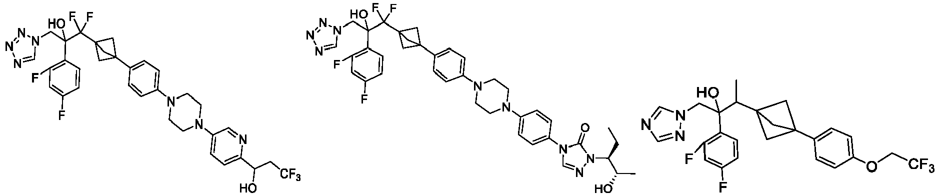

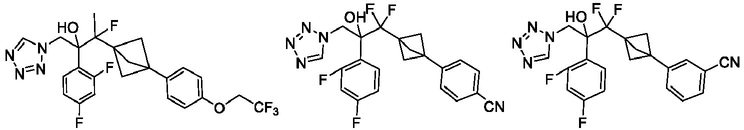

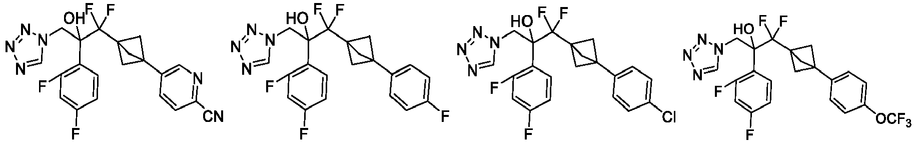

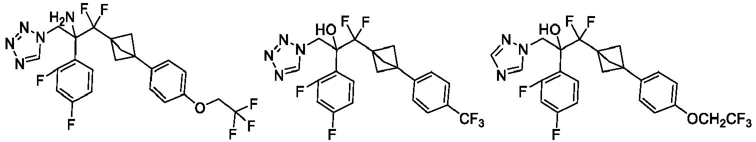

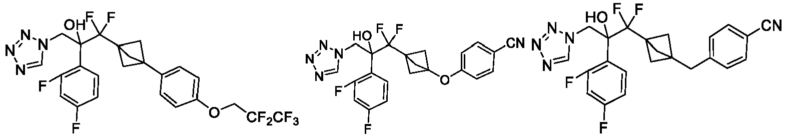

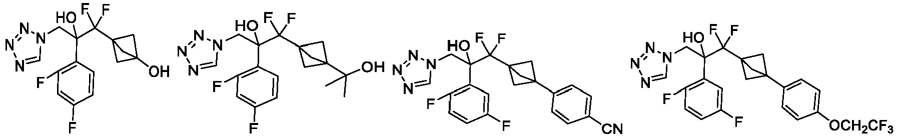

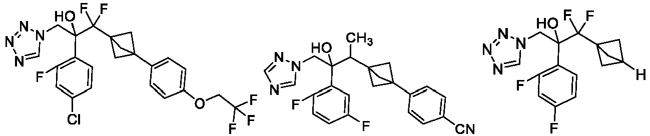

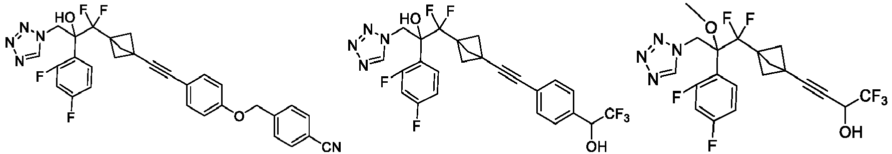

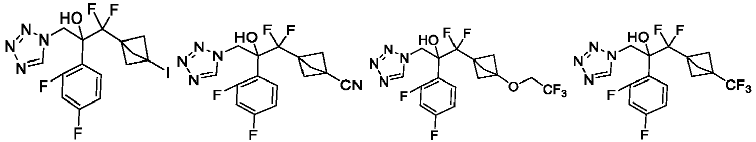

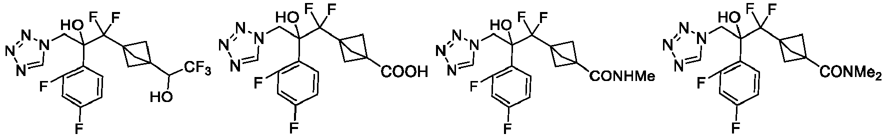

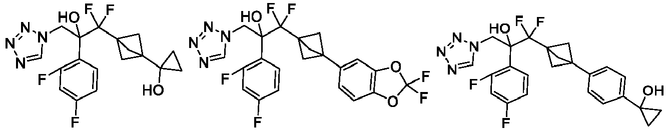

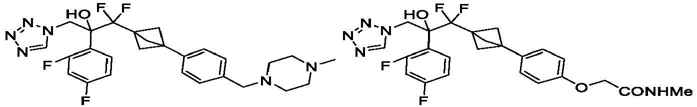

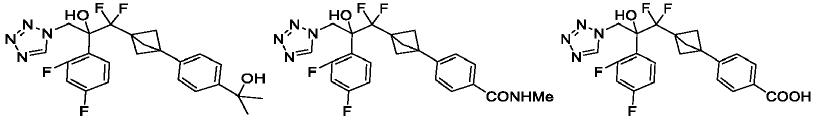

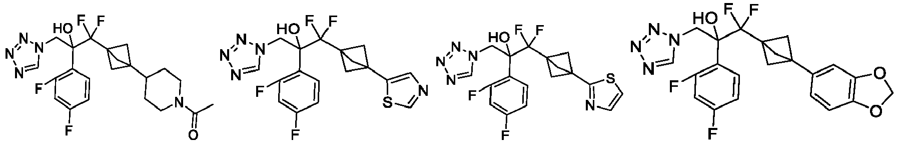

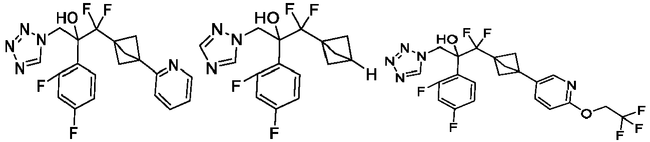

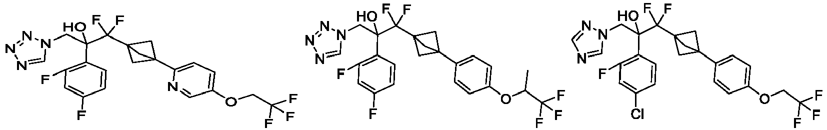

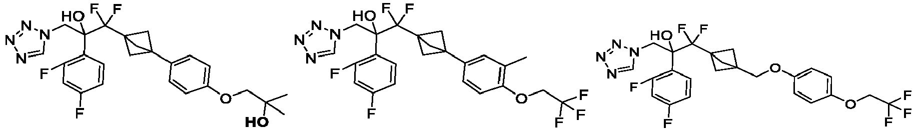

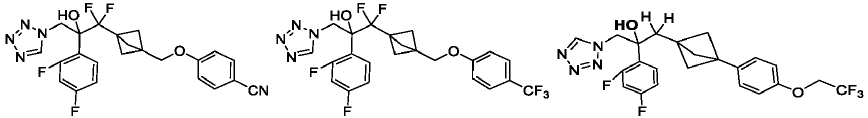

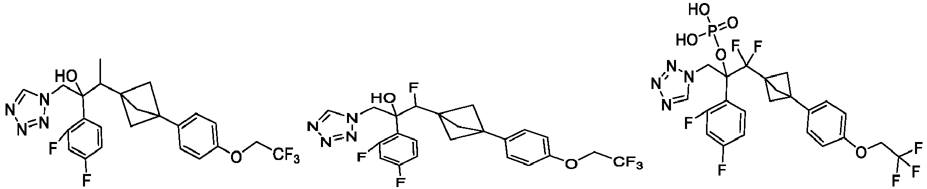

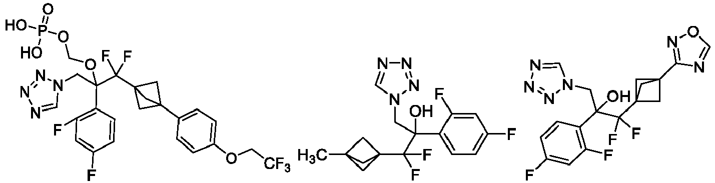

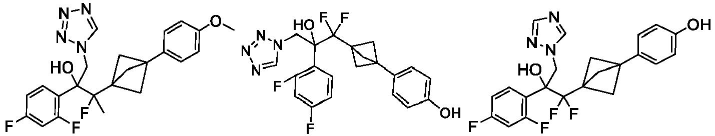

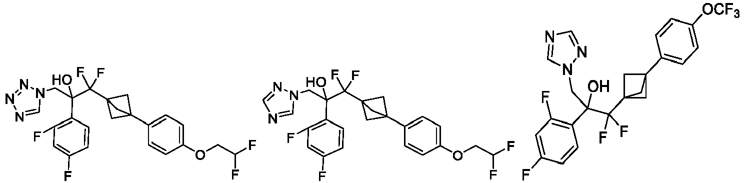

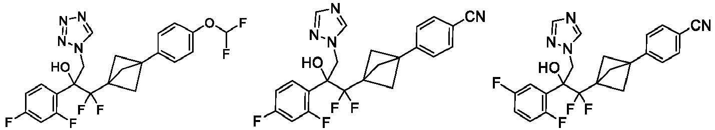

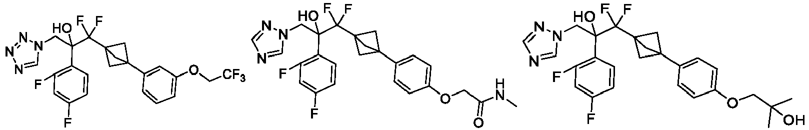

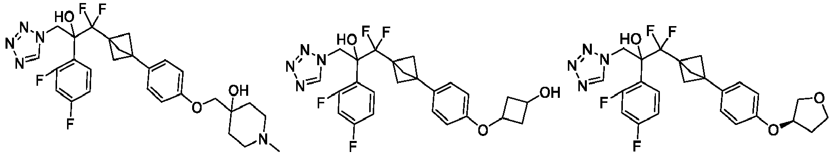

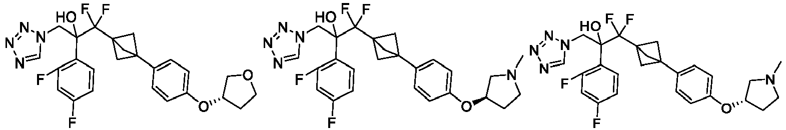

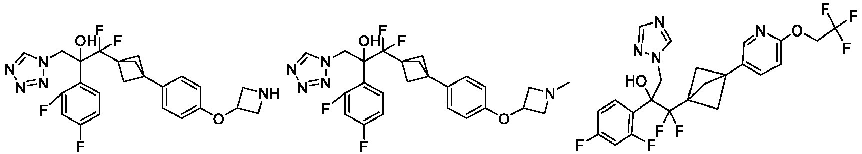

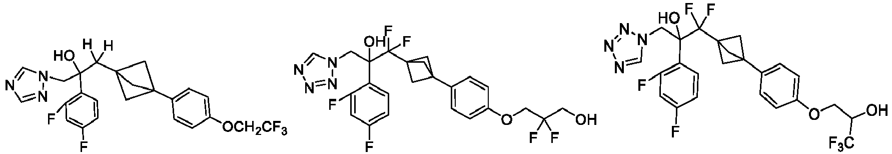

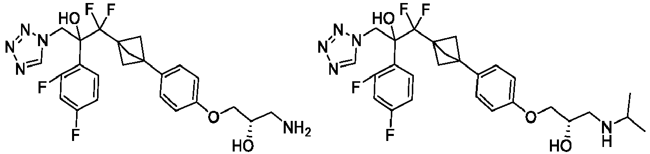

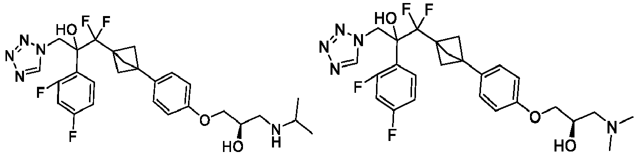

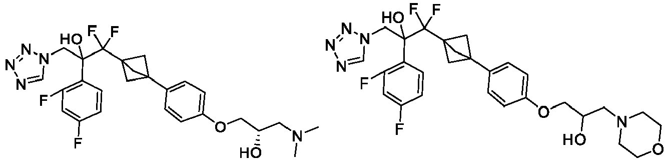

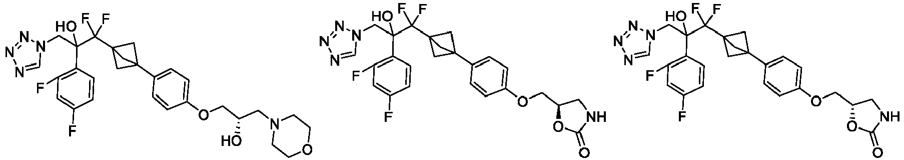

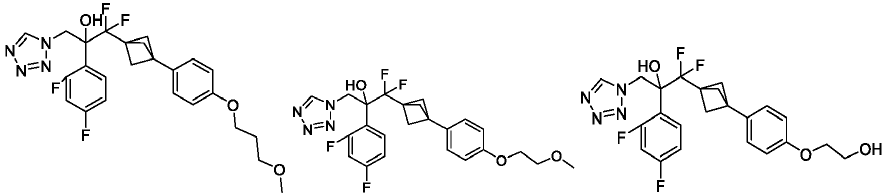

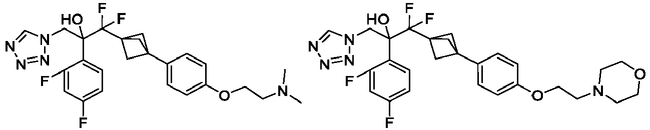

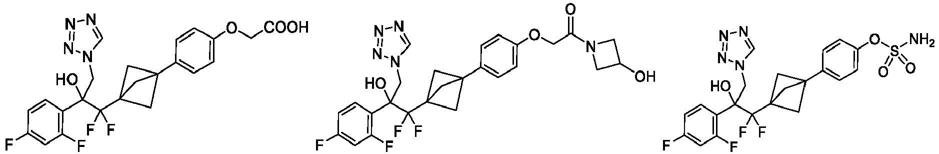

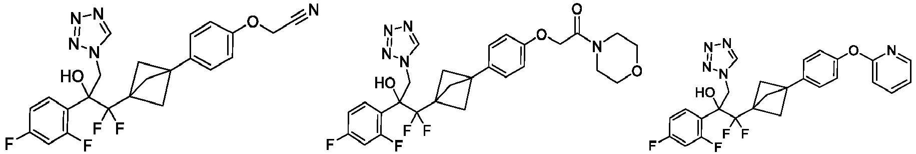

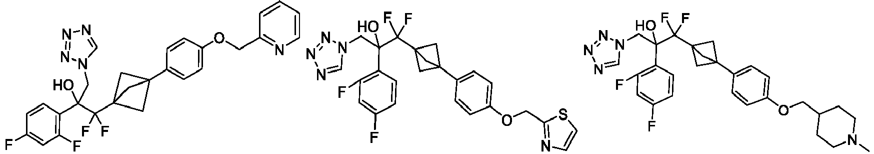

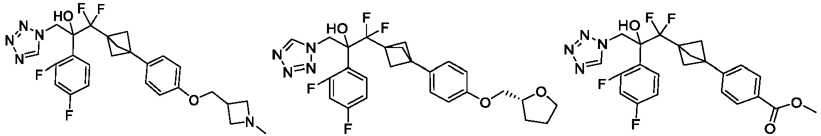

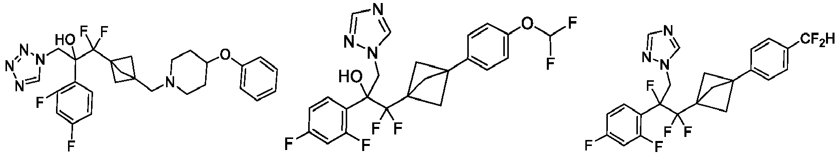

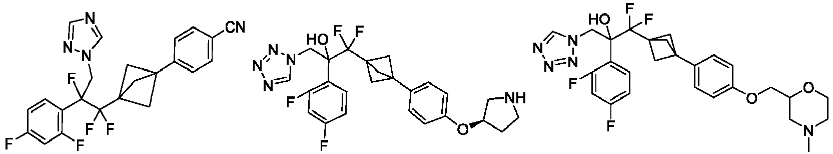

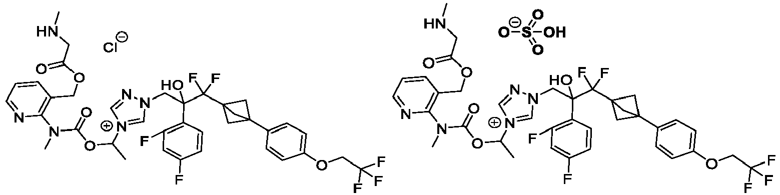

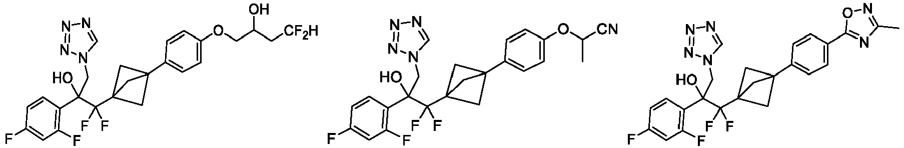

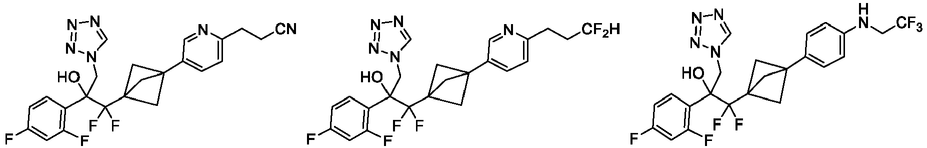

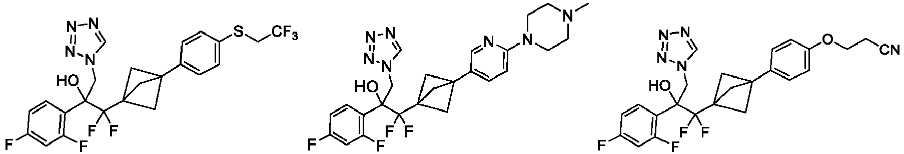

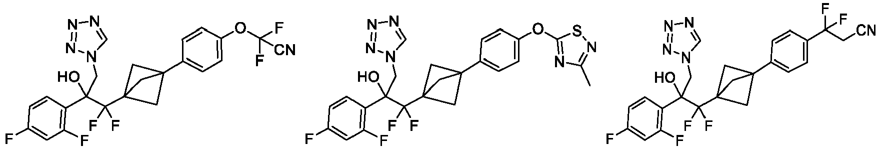

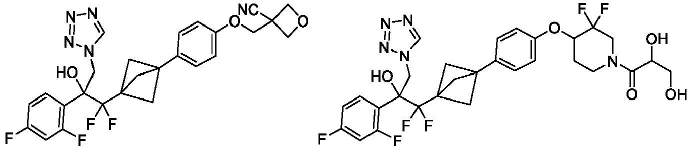

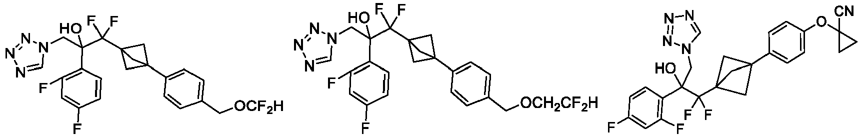

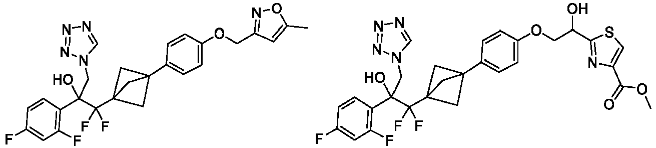

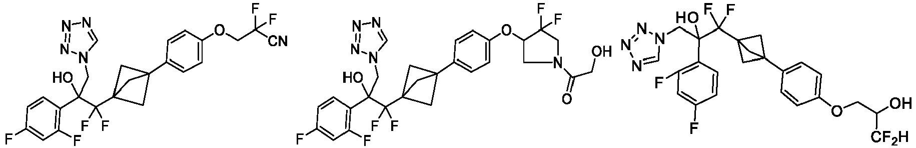

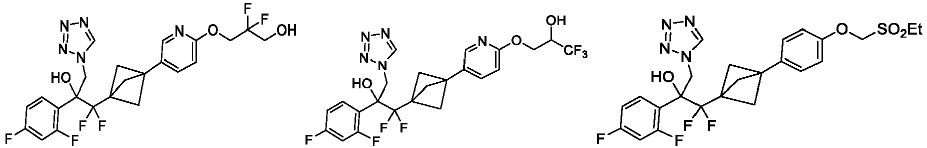

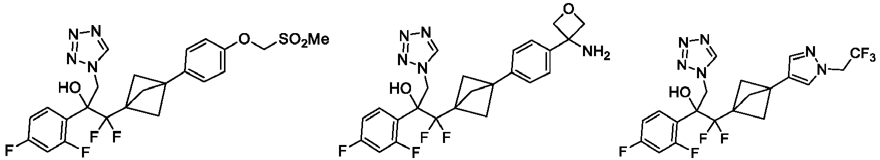

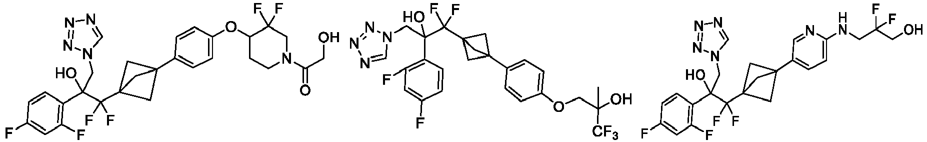

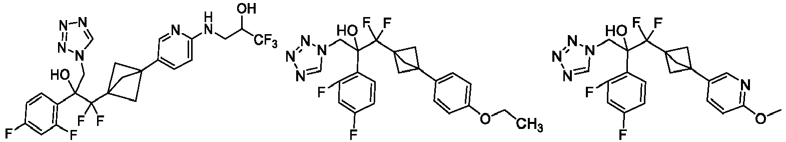

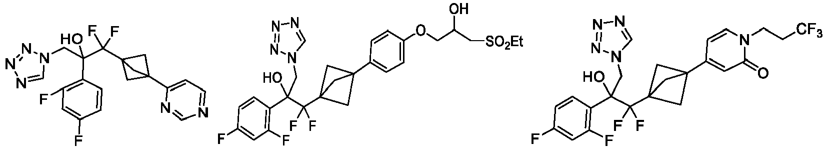

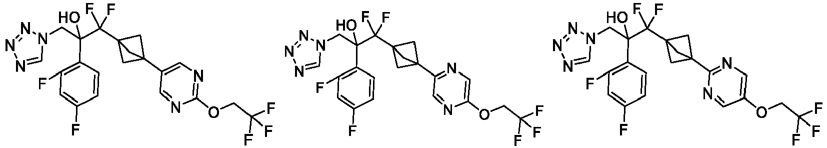

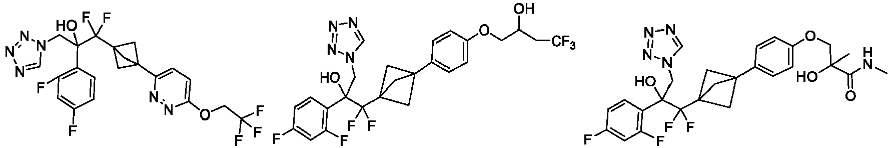

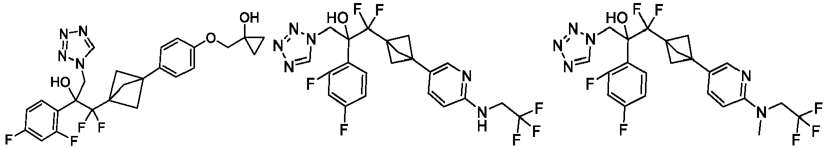

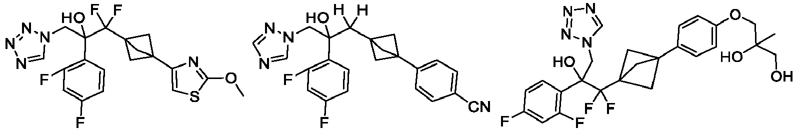

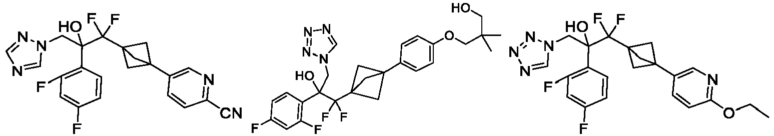

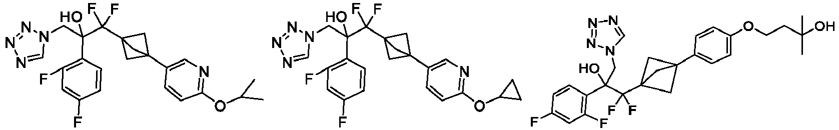

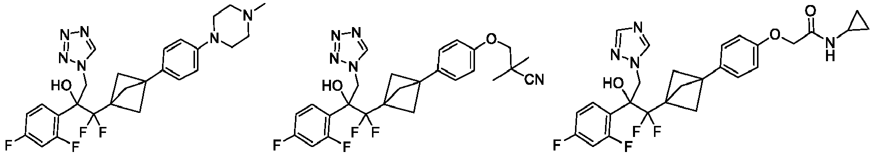

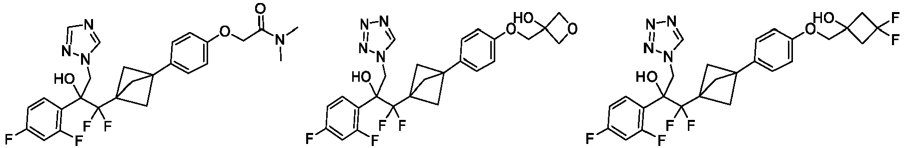

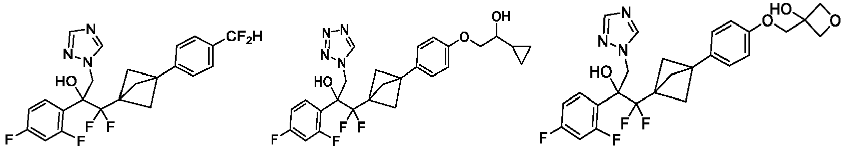

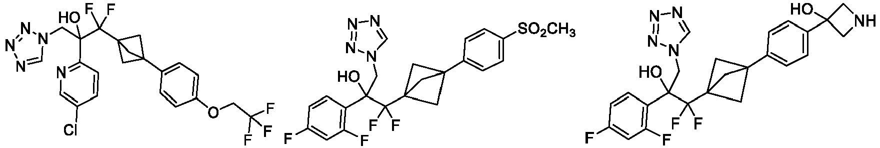

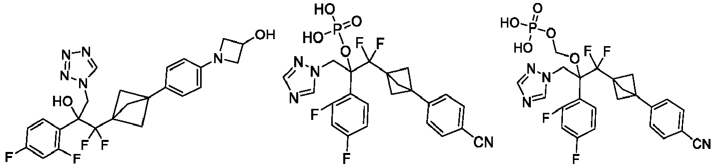

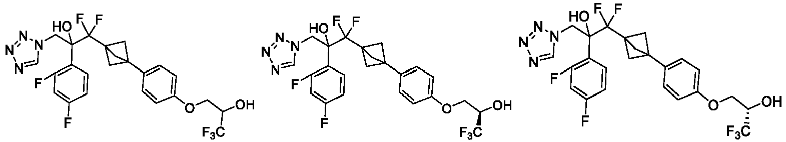

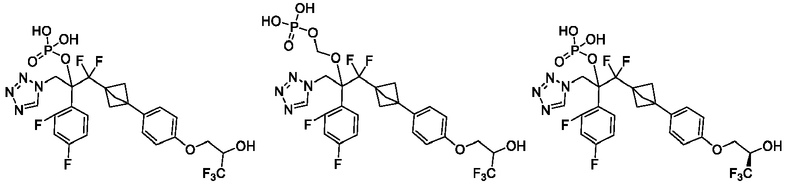

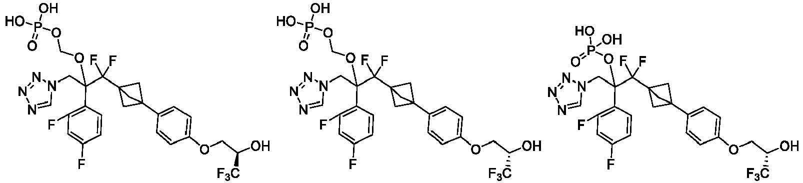

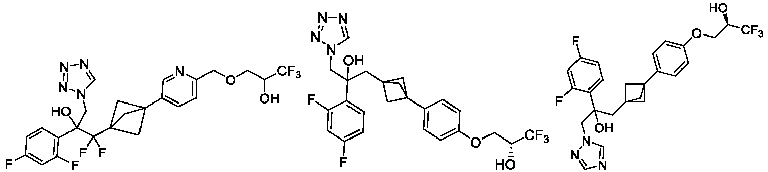

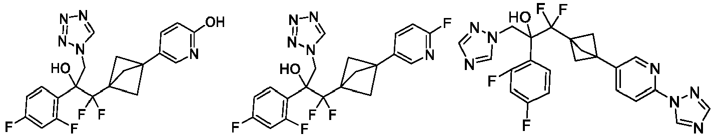

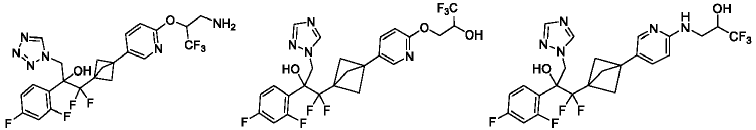

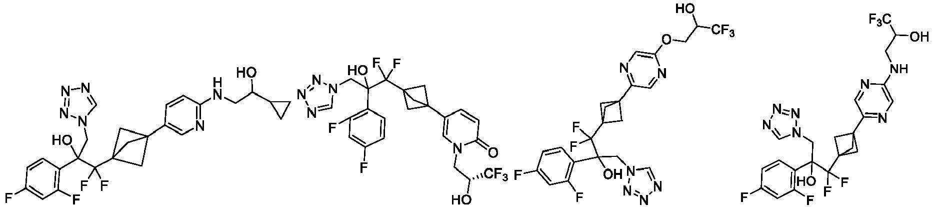

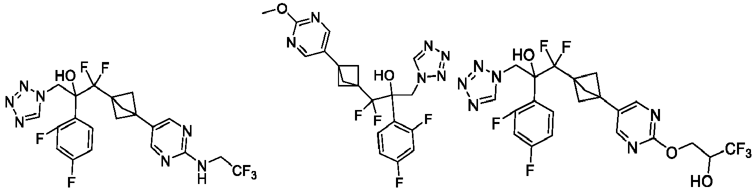

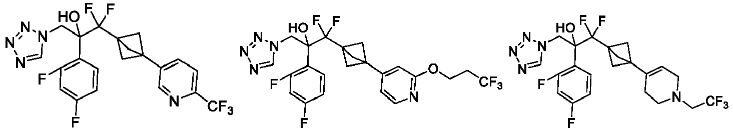

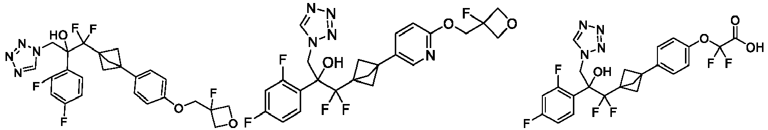

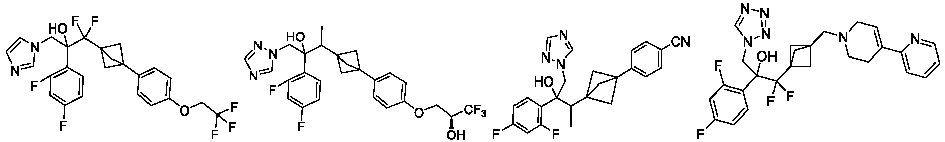

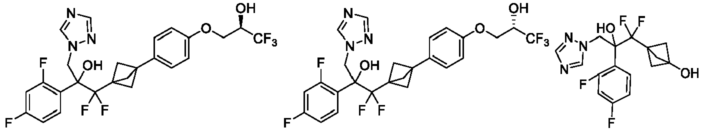

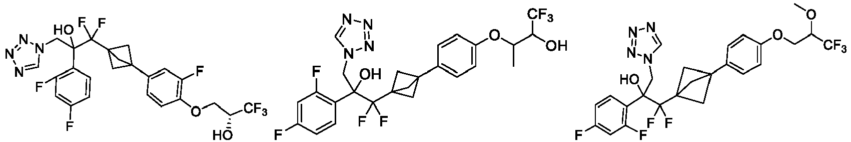

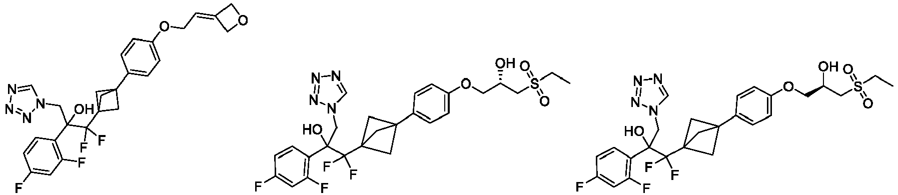

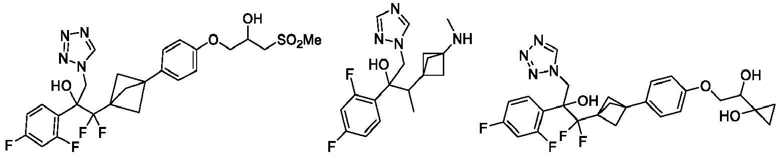

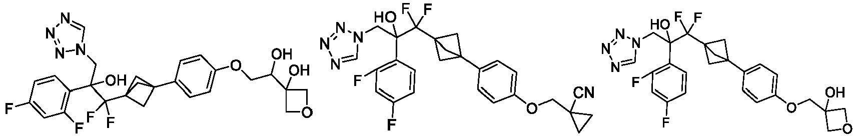

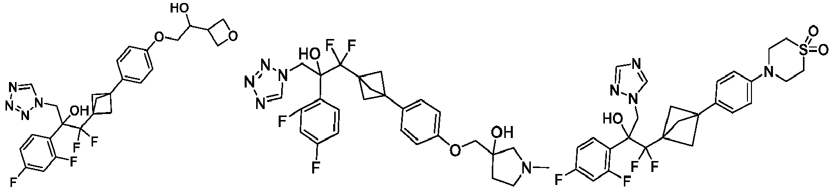

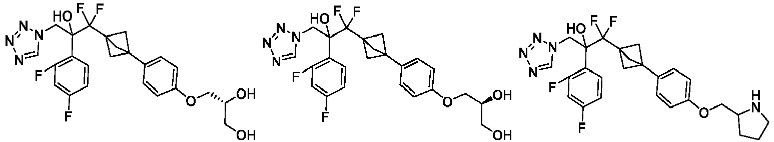

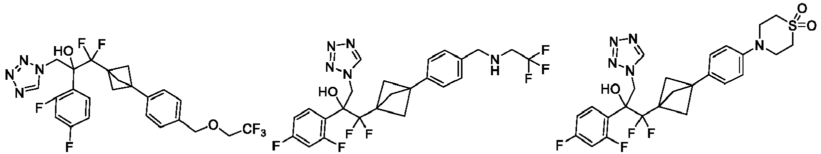

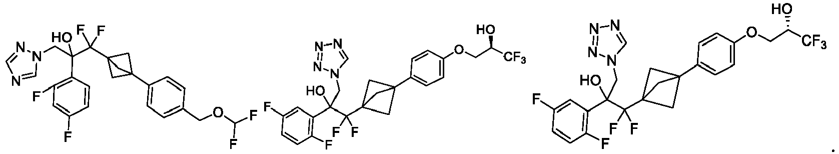

In yet another aspect of the present disclosure, the present disclosure also provides a compound of the following formula, an optical isomer thereof, a tautomer thereof, and a pharmaceutically acceptable salt thereof, which is selected from

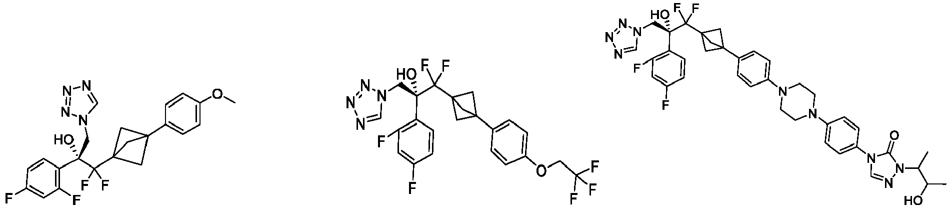

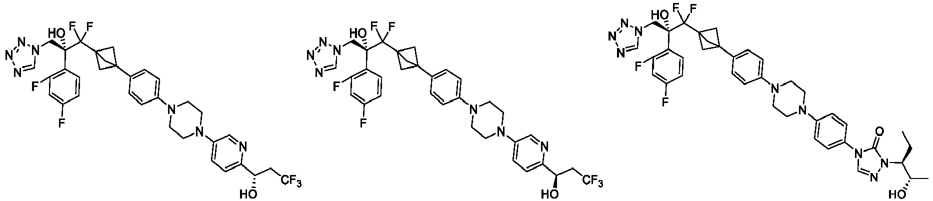

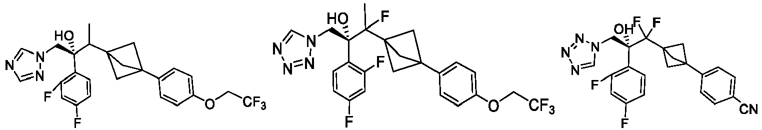

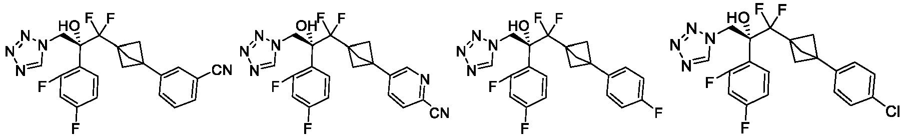

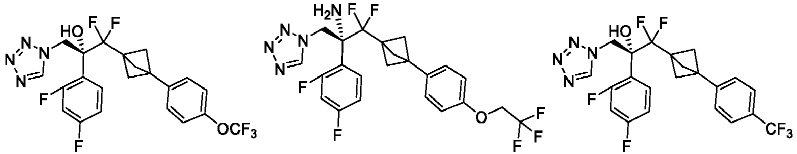

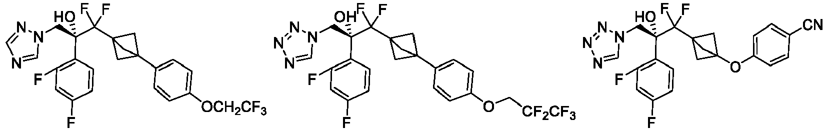

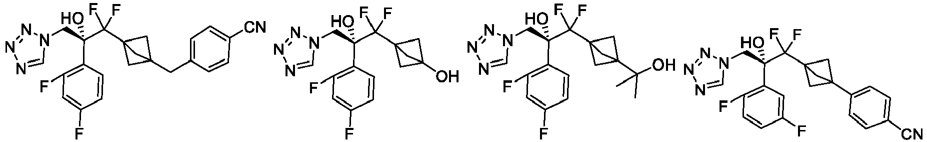

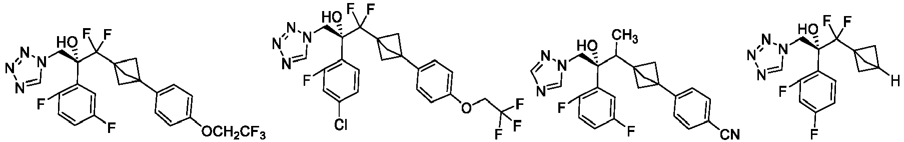

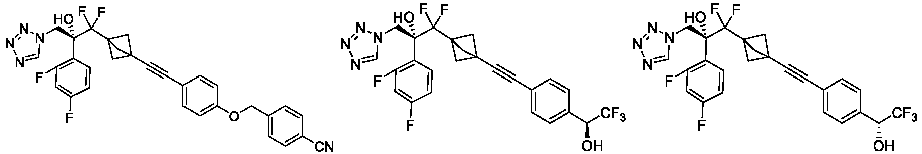

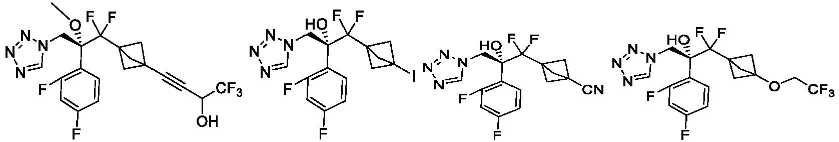

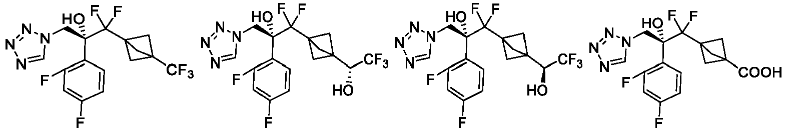

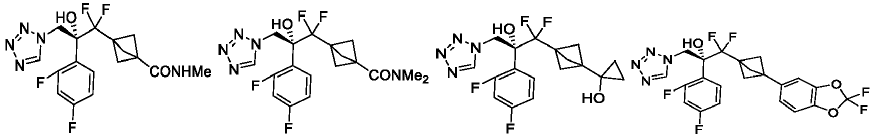

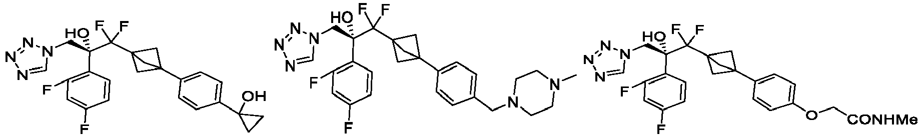

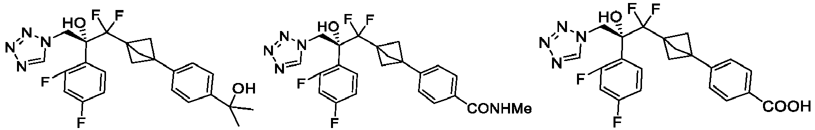

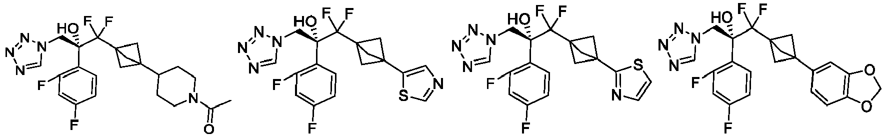

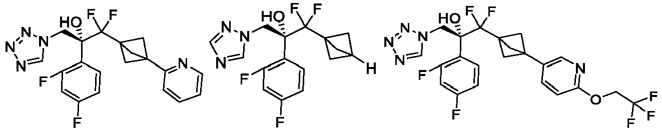

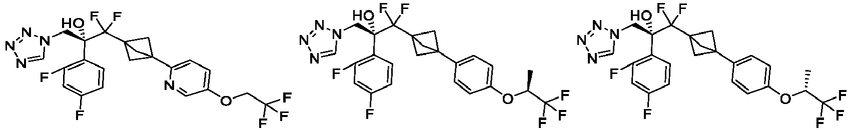

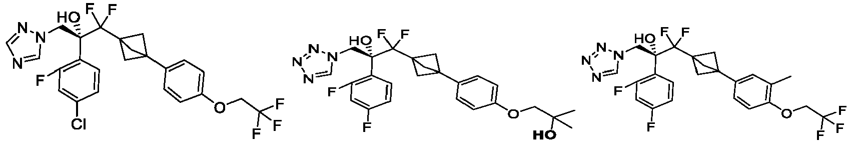

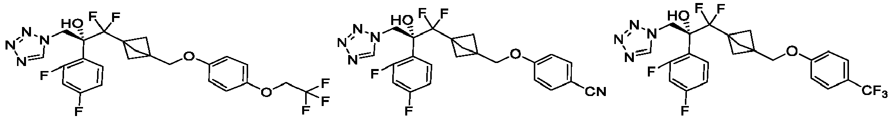

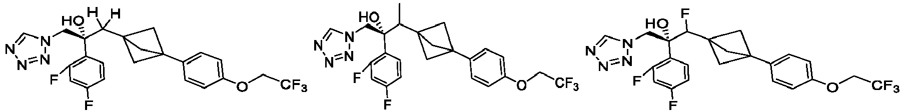

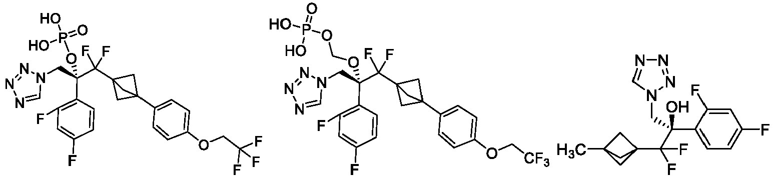

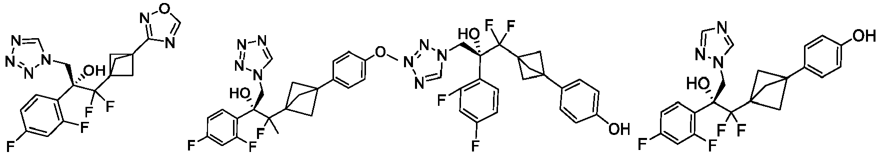

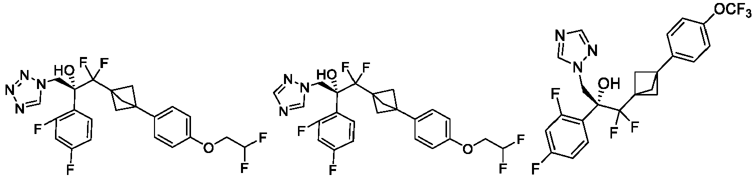

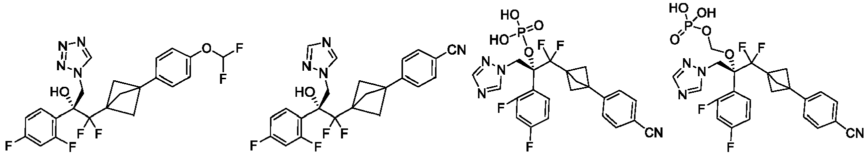

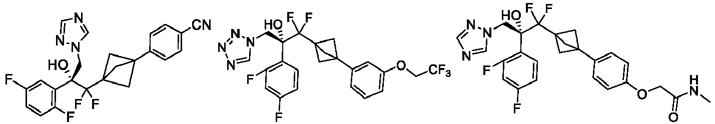

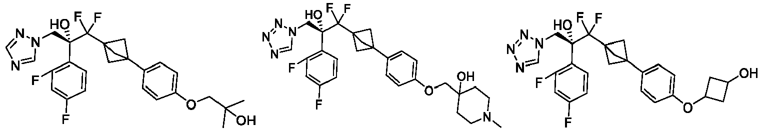

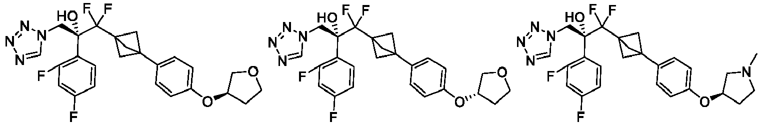

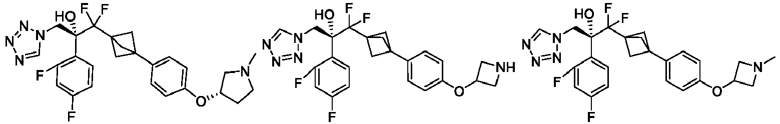

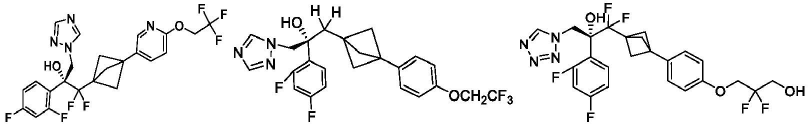

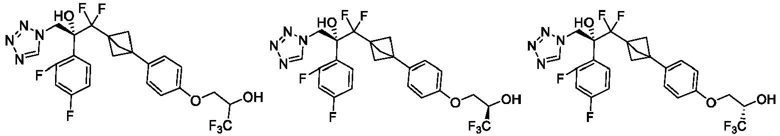

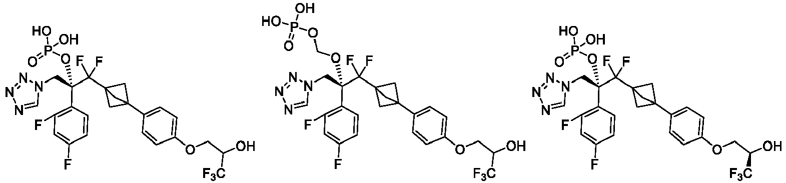

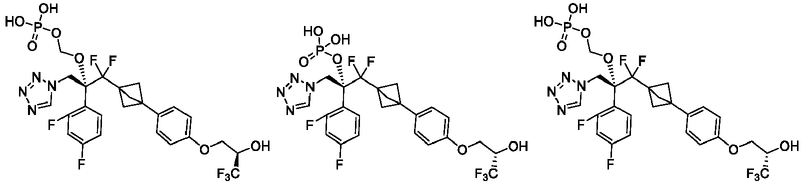

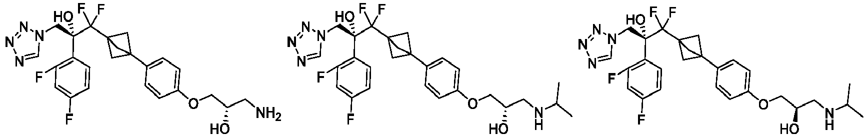

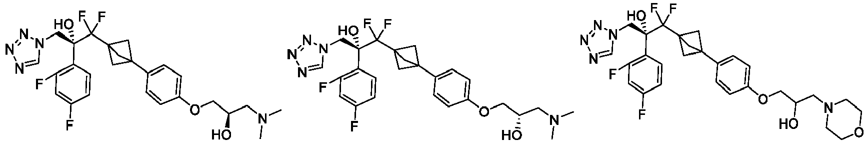

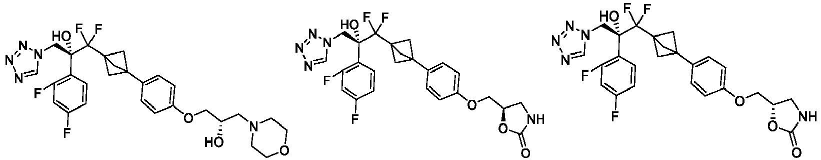

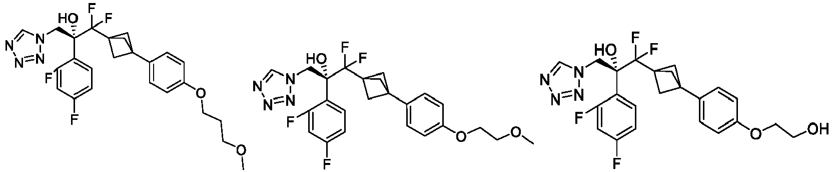

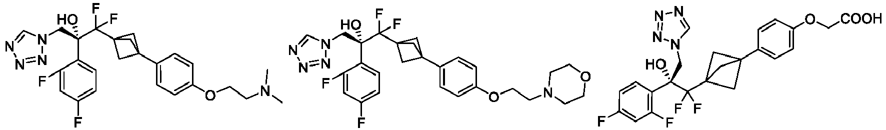

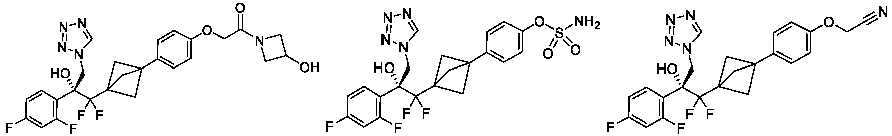

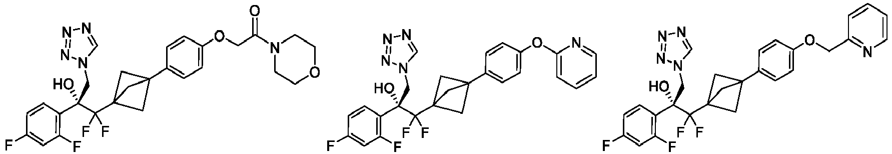

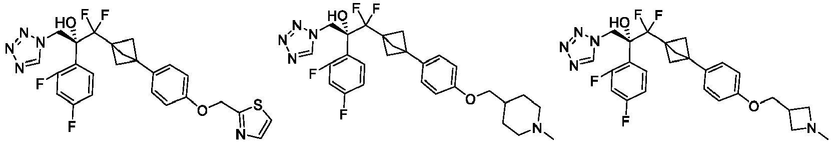

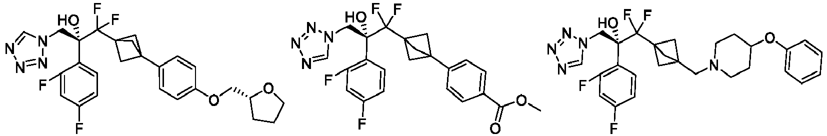

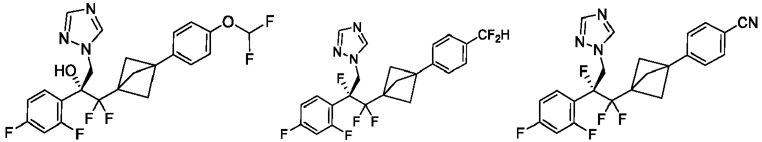

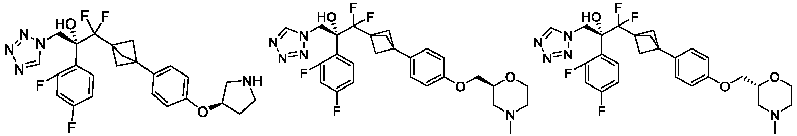

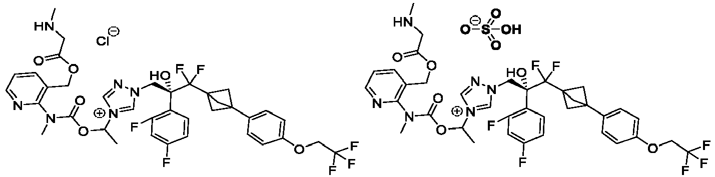

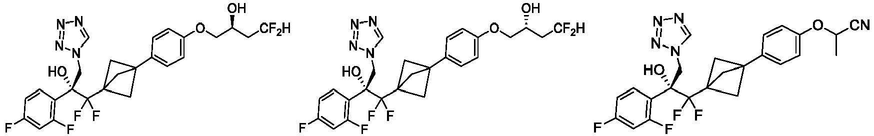

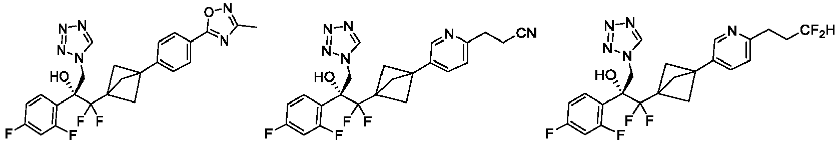

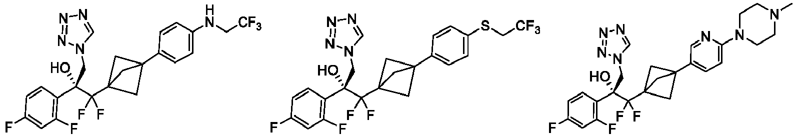

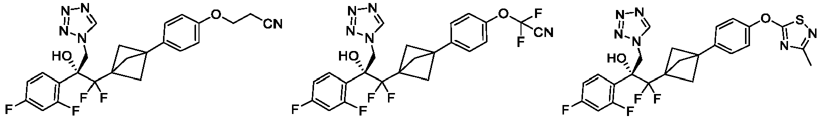

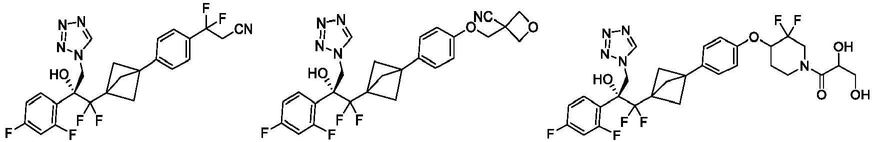

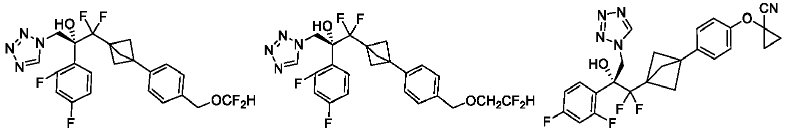

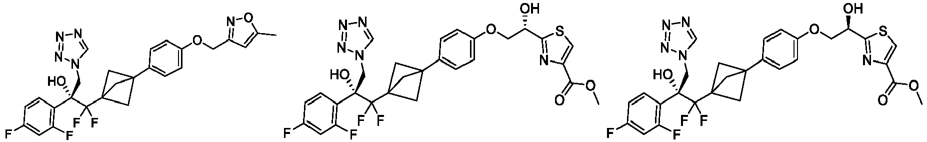

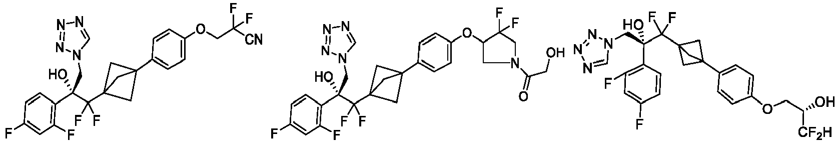

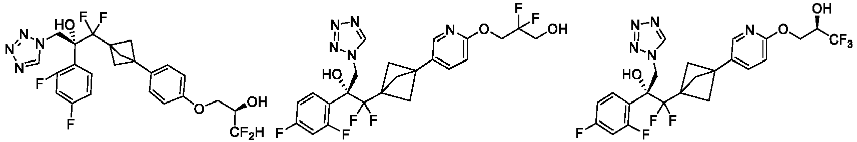

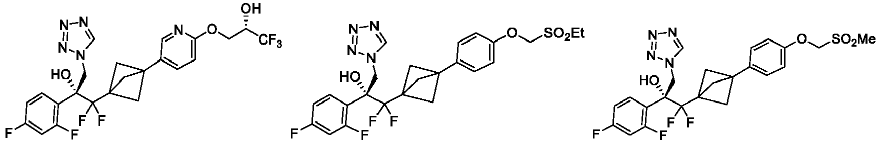

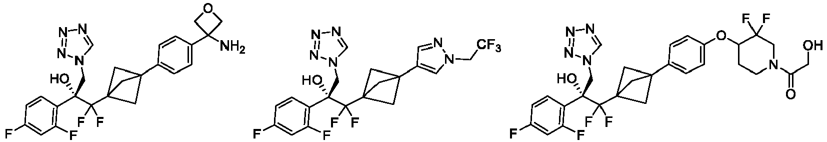

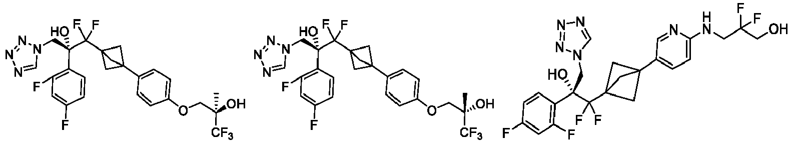

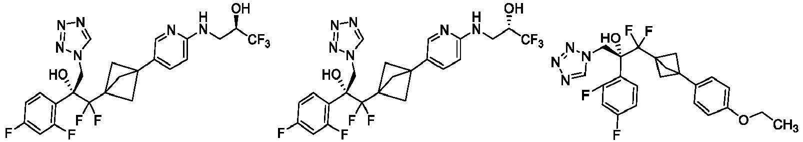

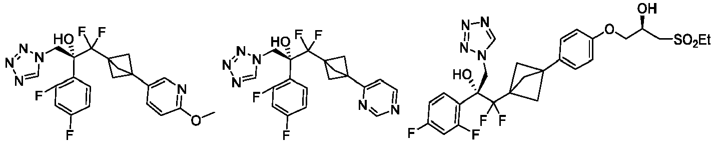

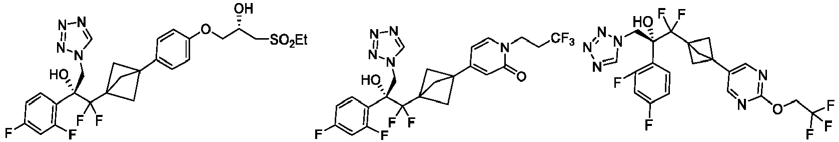

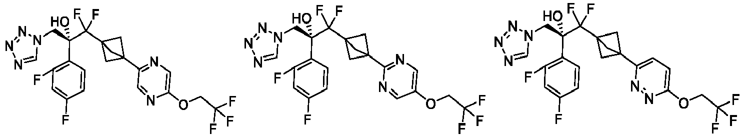

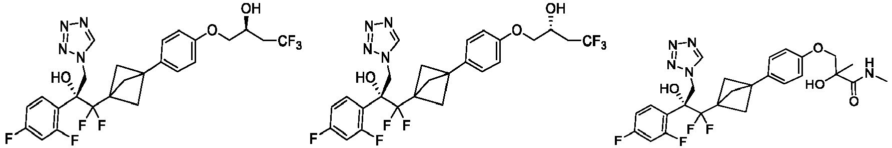

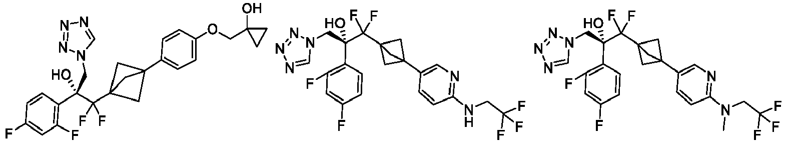

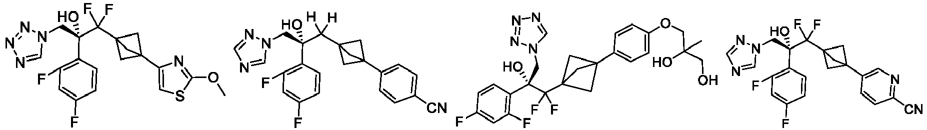

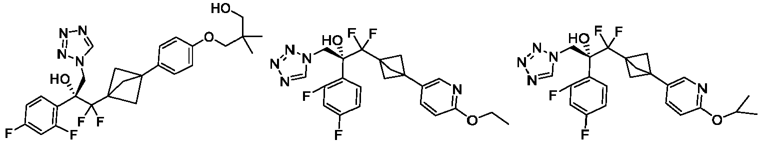

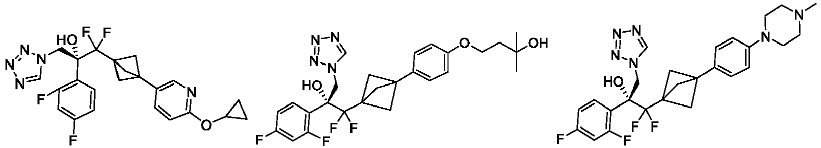

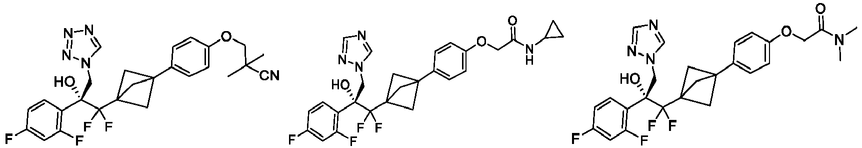

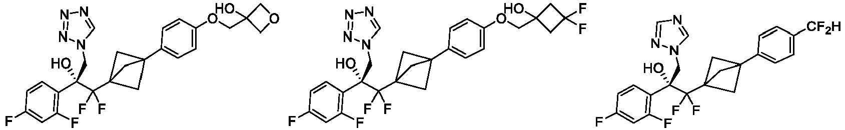

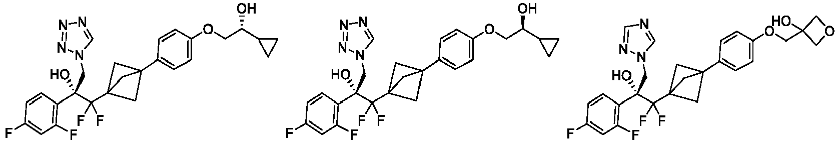

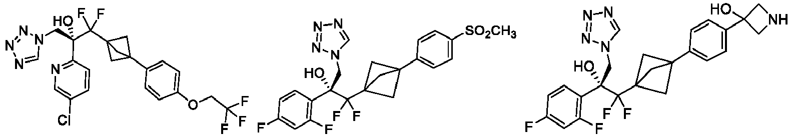

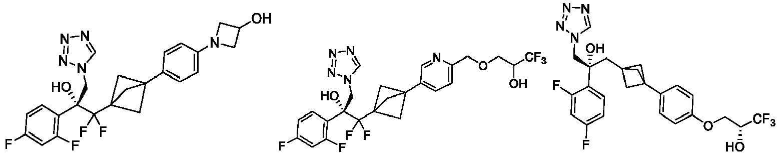

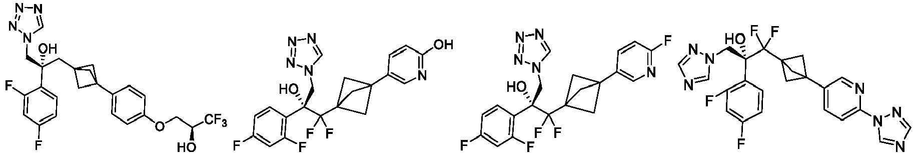

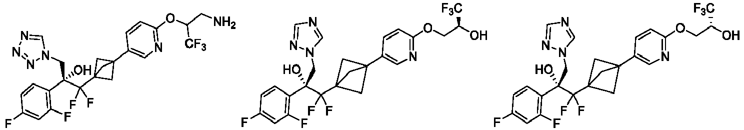

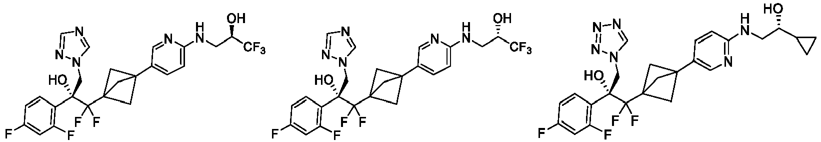

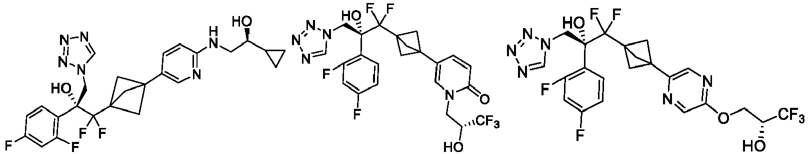

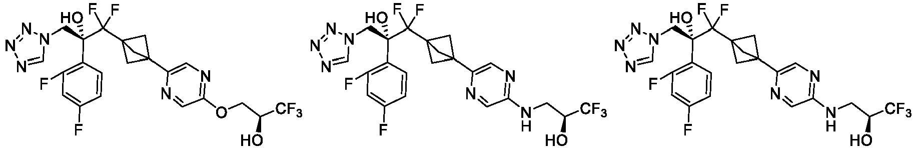

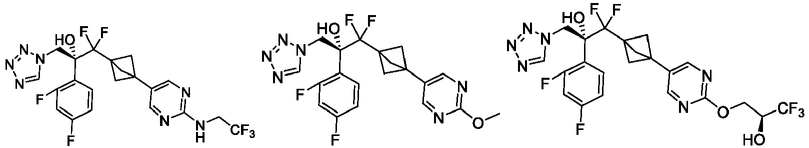

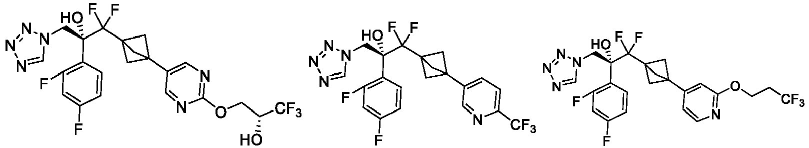

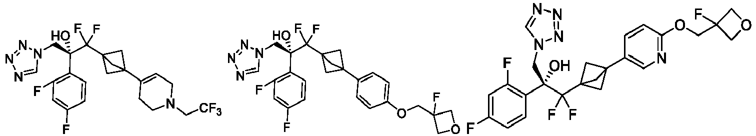

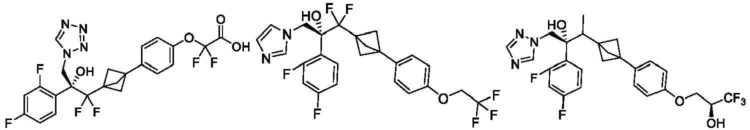

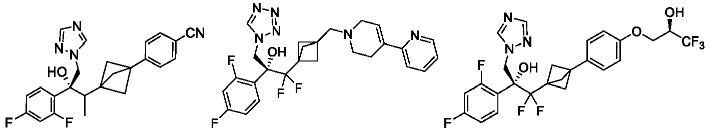

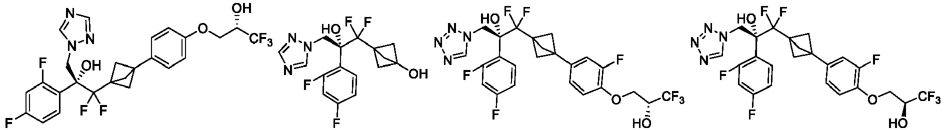

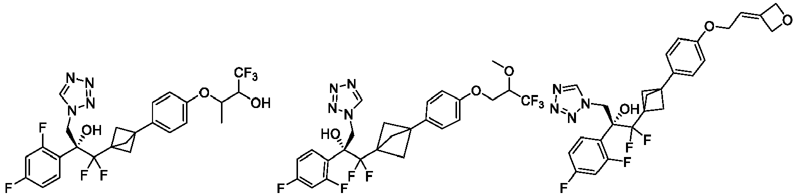

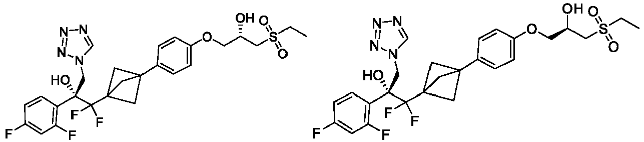

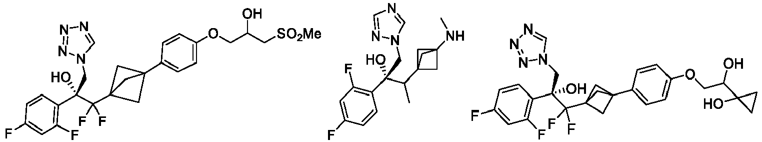

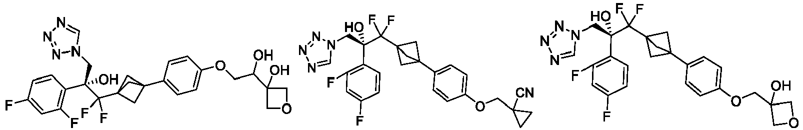

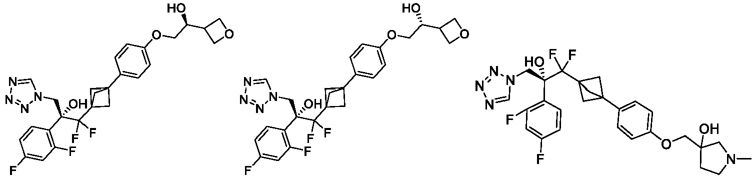

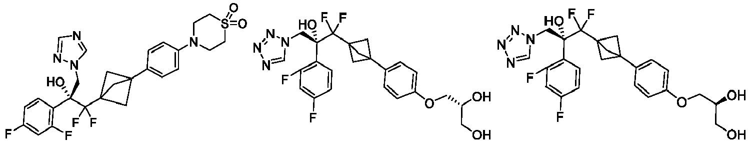

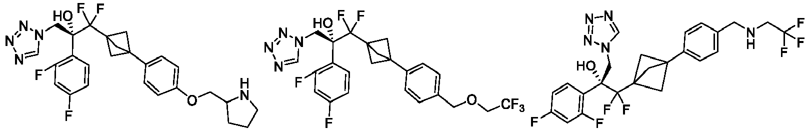

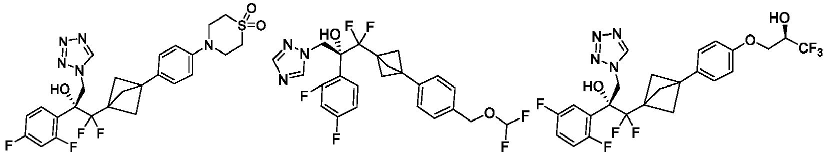

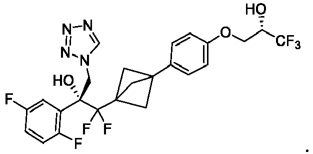

In yet another aspect of the present disclosure, the present disclosure also provides a compound of the following formula, an optical isomer thereof, a tautomer thereof, and a pharmaceutically acceptable salt thereof, which is selected from

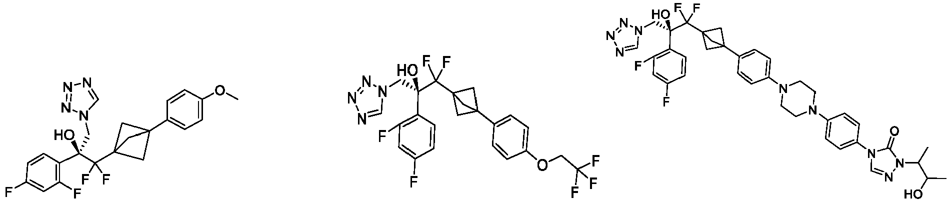

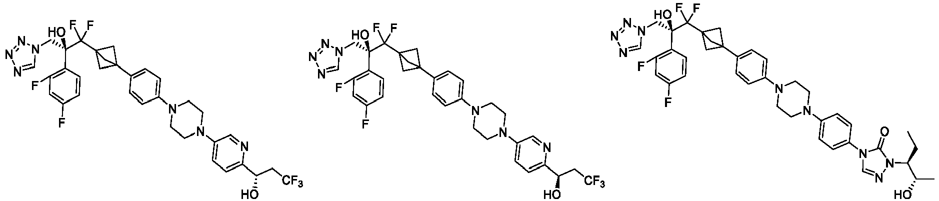

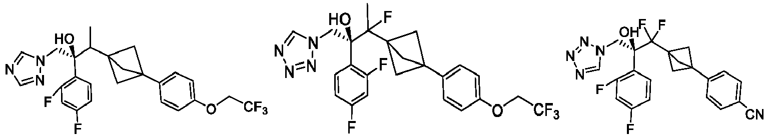

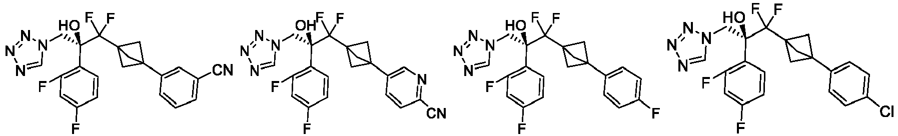

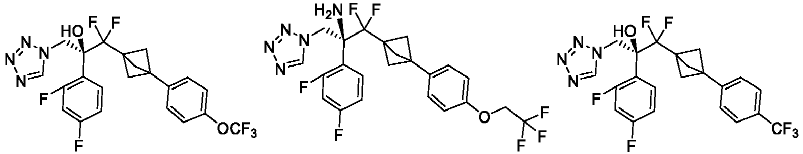

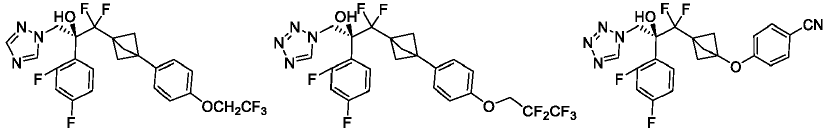

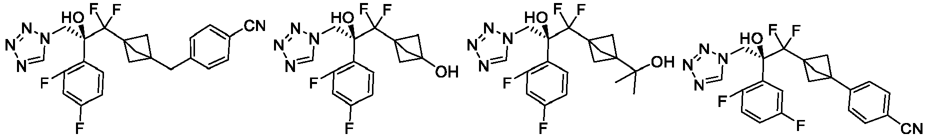

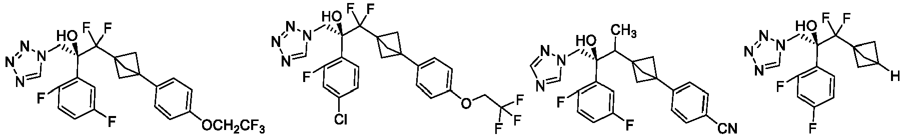

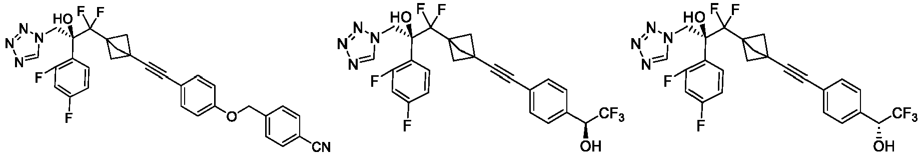

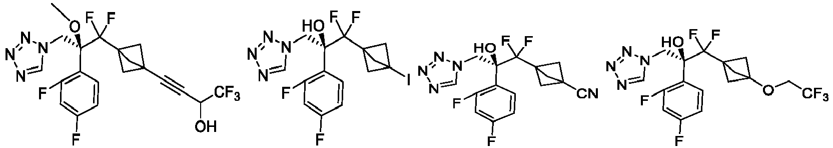

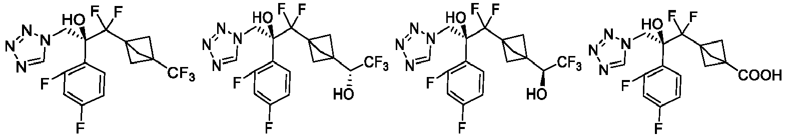

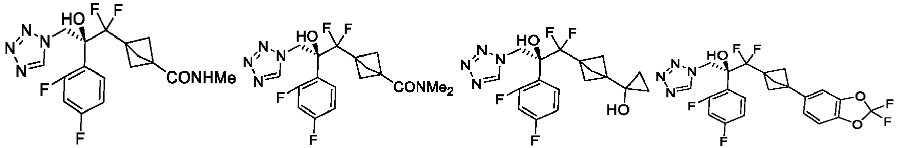

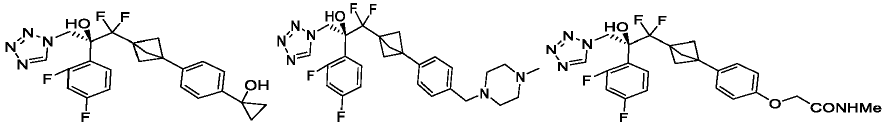

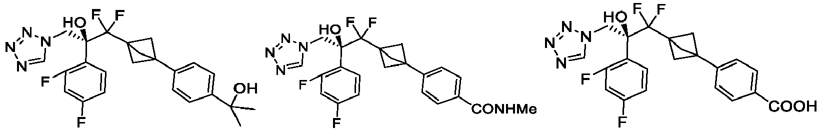

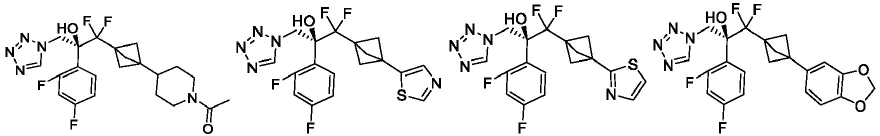

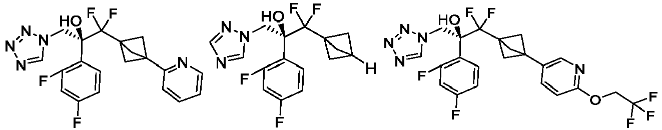

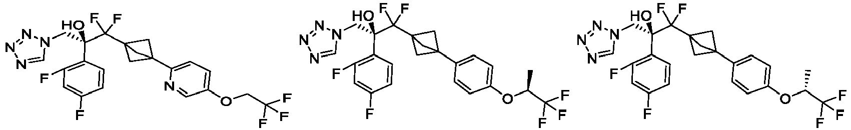

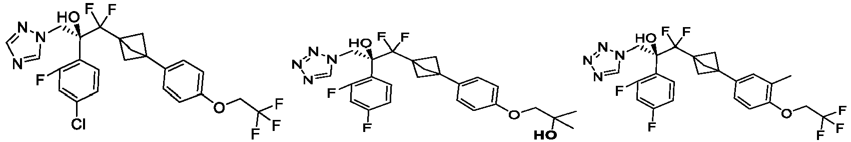

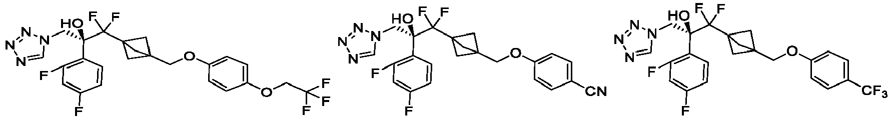

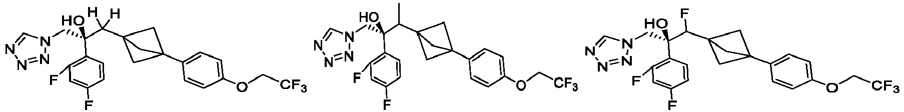

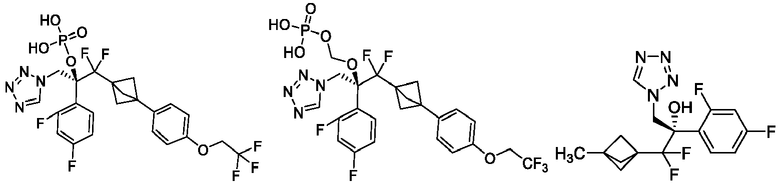

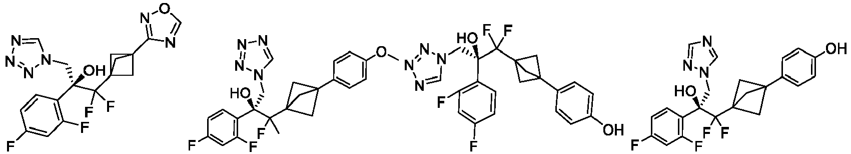

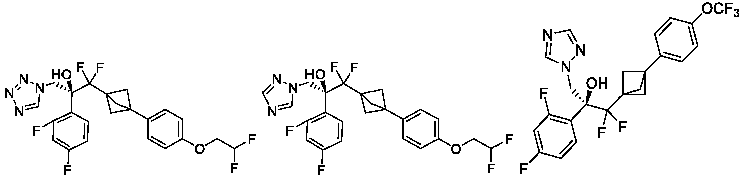

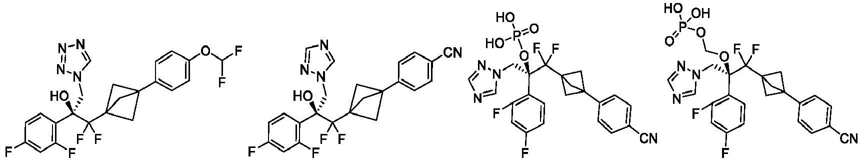

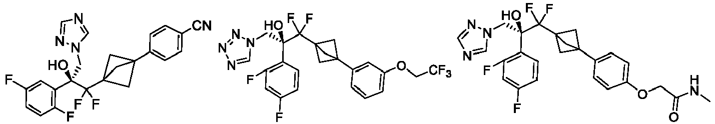

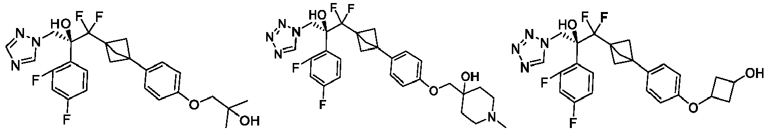

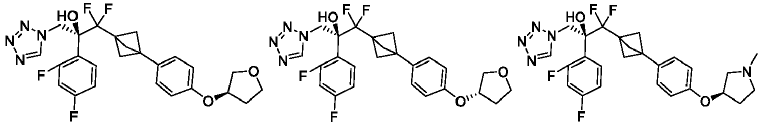

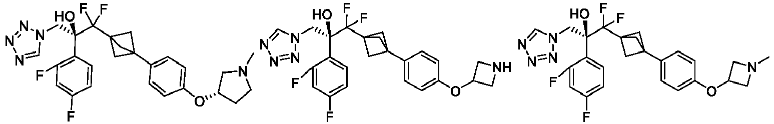

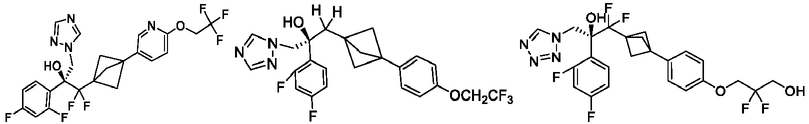

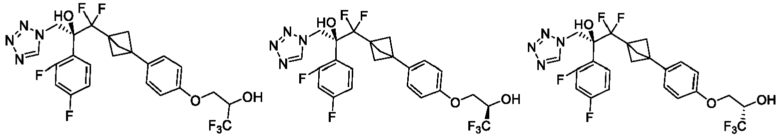

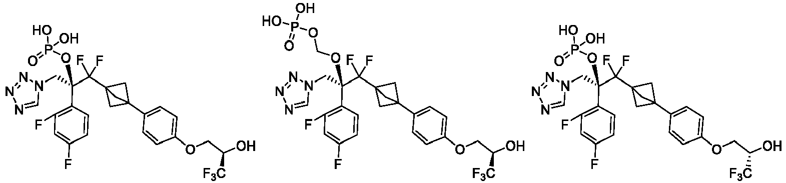

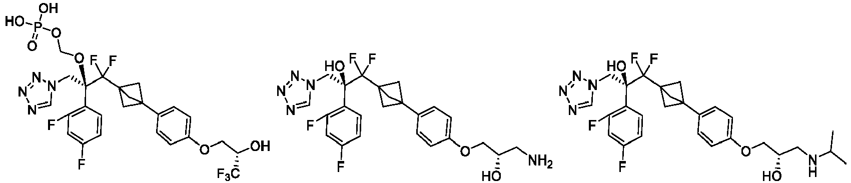

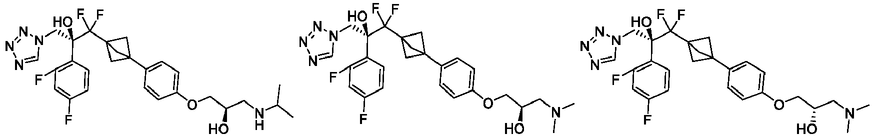

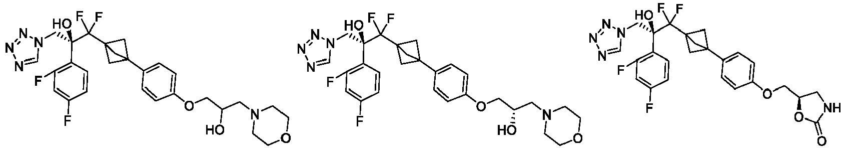

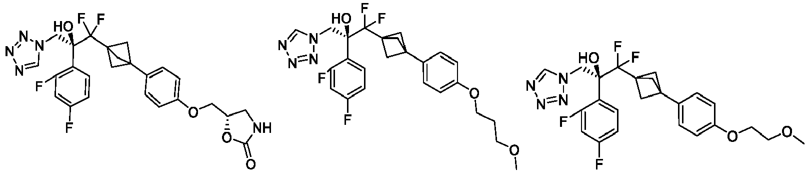

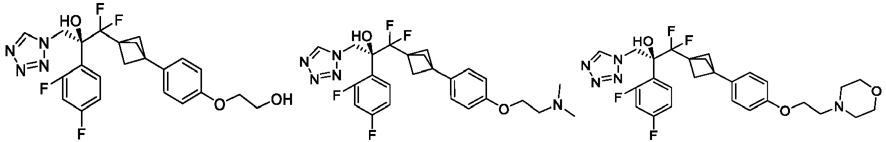

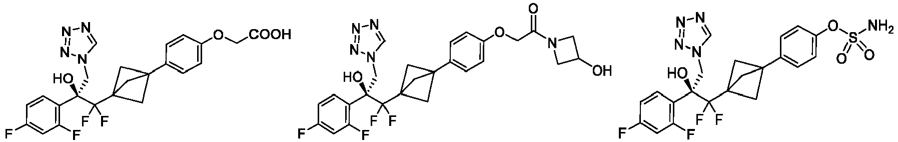

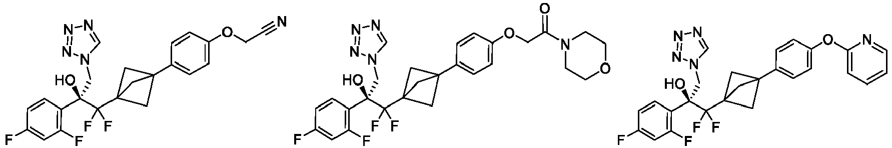

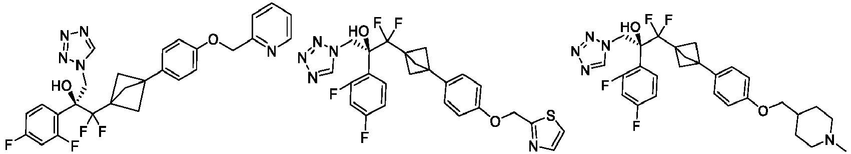

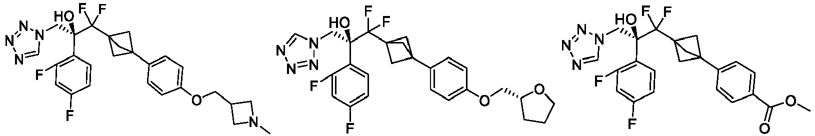

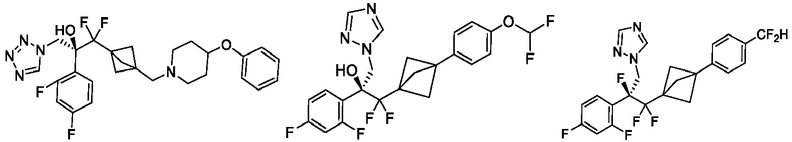

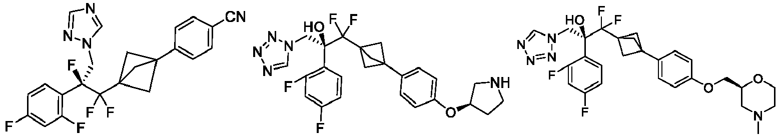

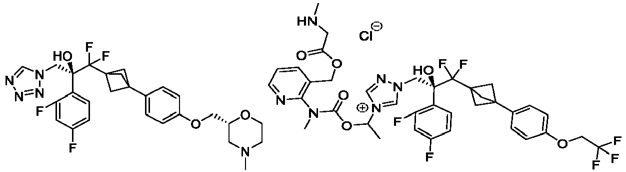

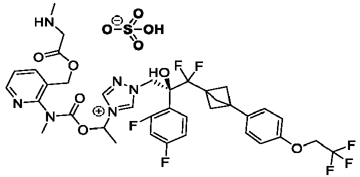

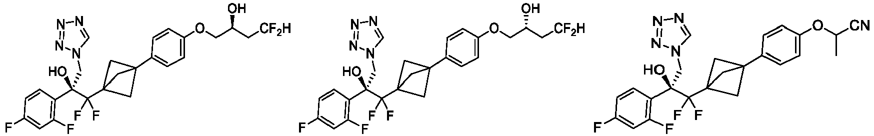

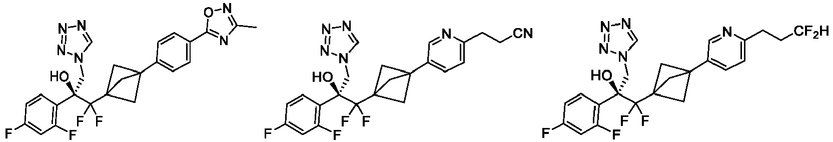

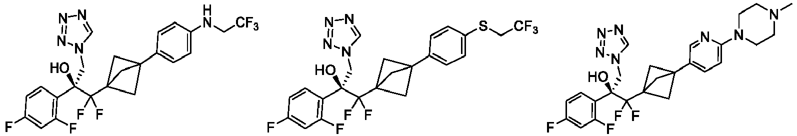

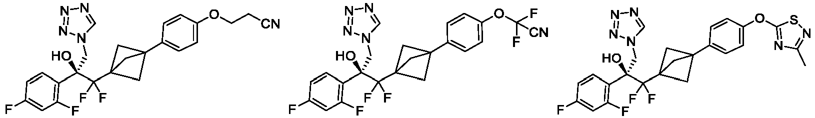

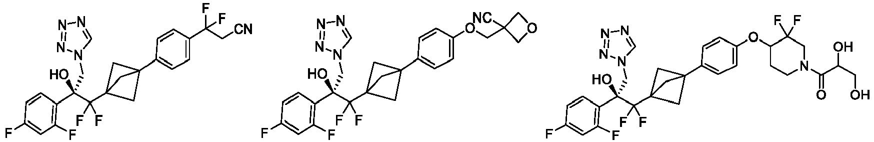

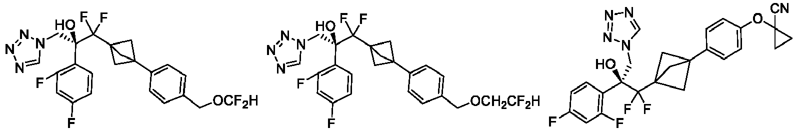

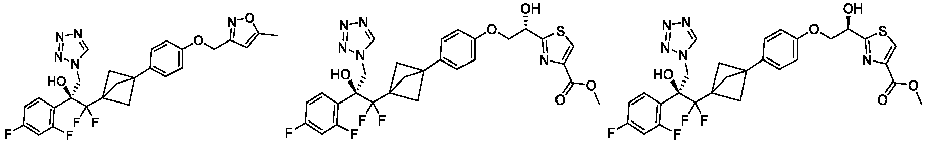

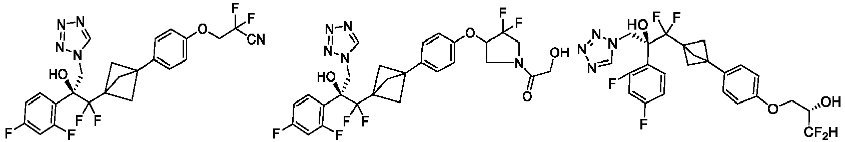

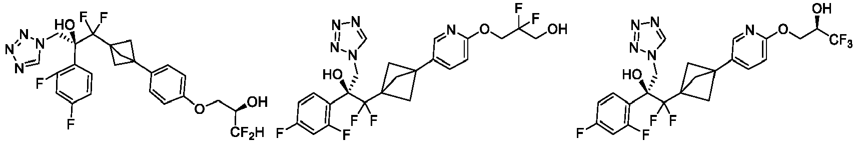

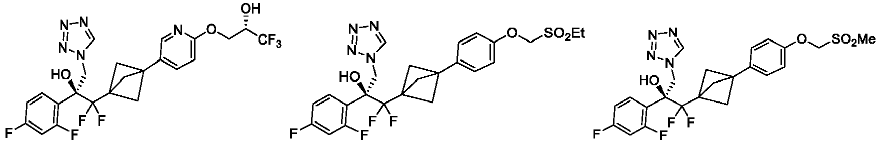

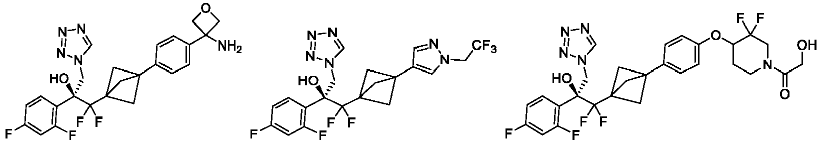

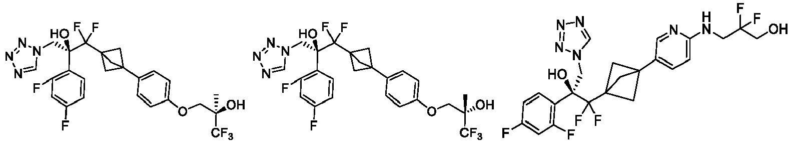

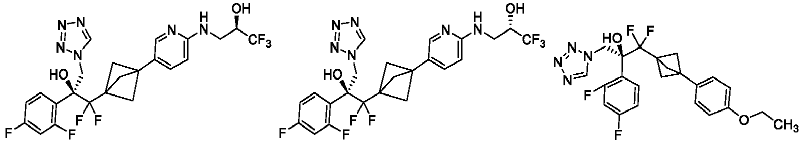

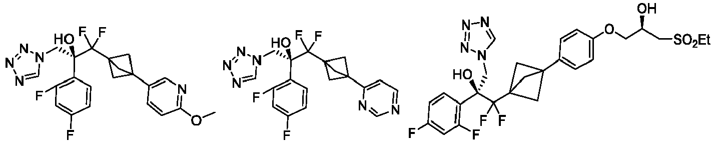

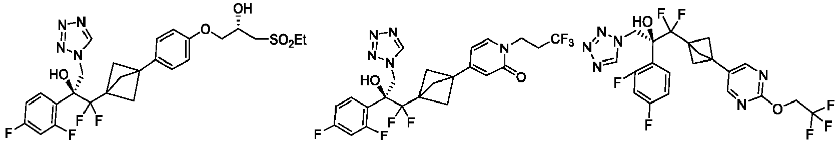

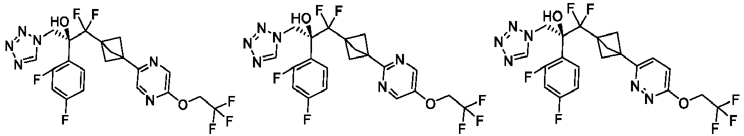

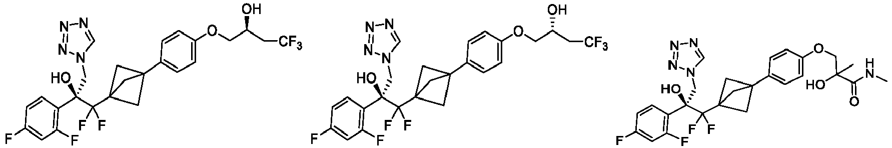

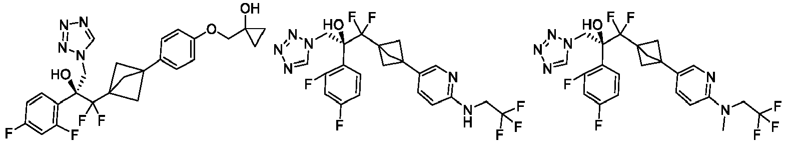

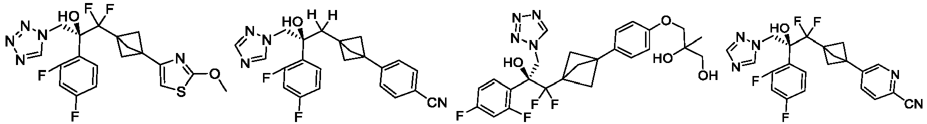

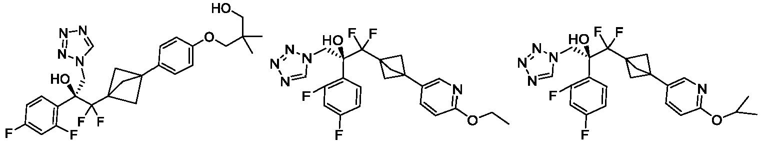

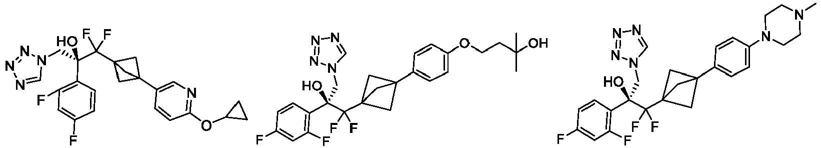

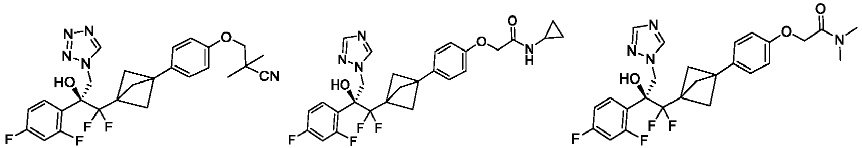

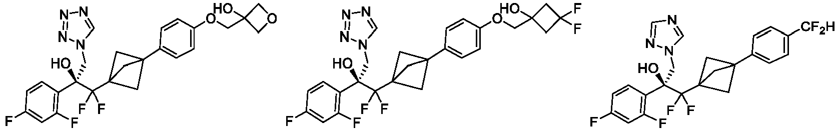

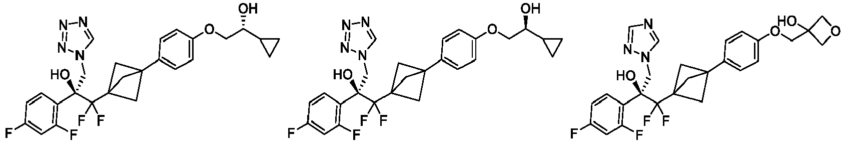

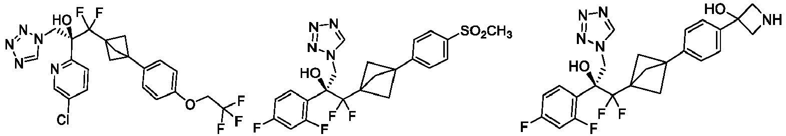

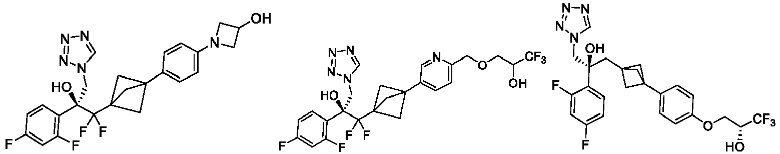

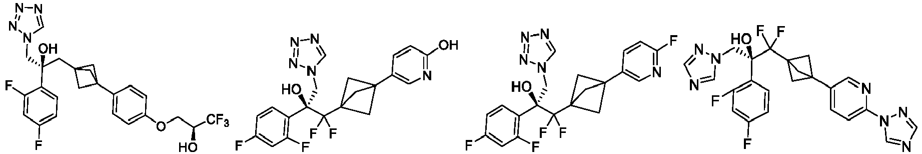

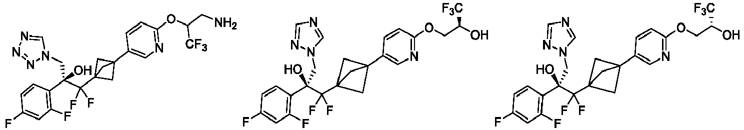

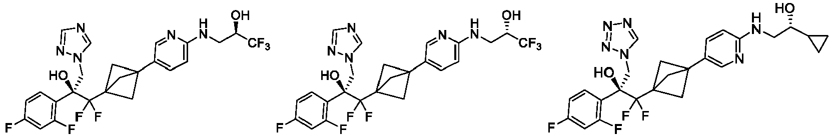

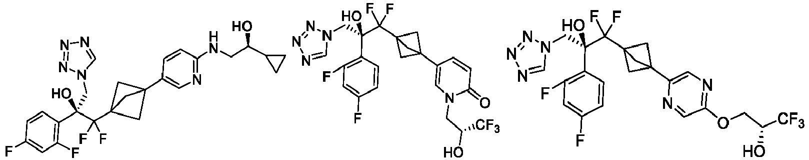

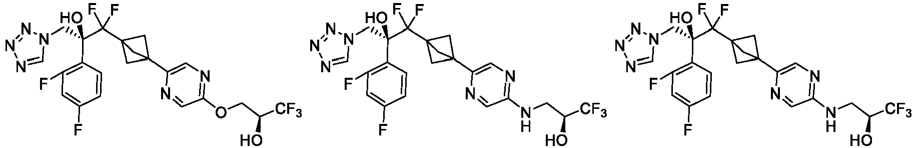

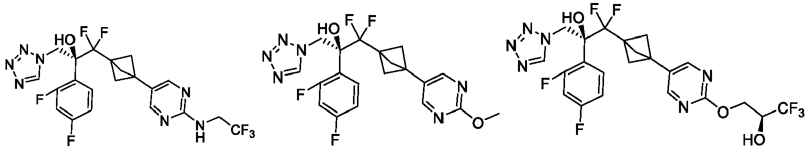

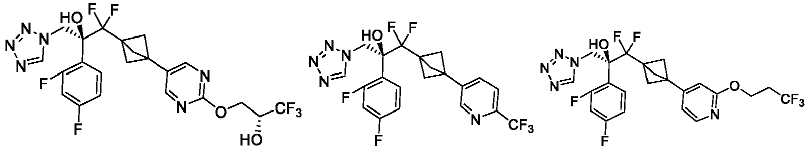

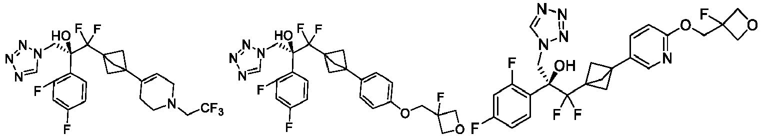

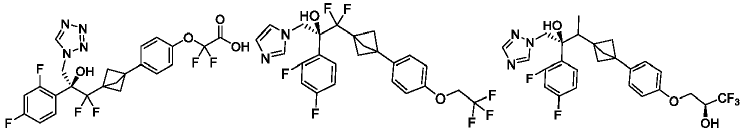

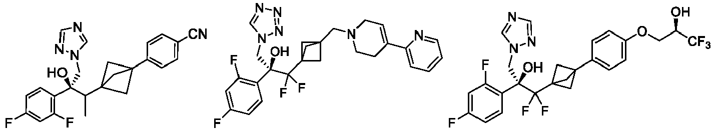

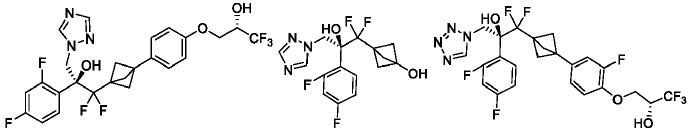

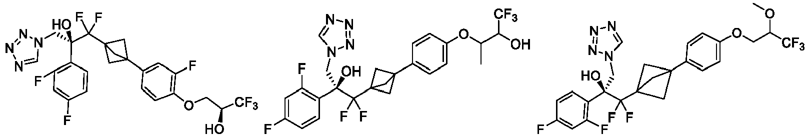

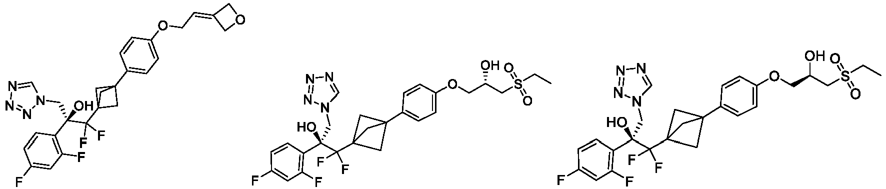

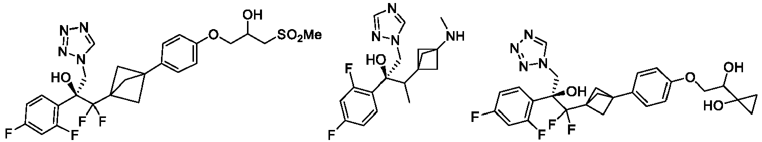

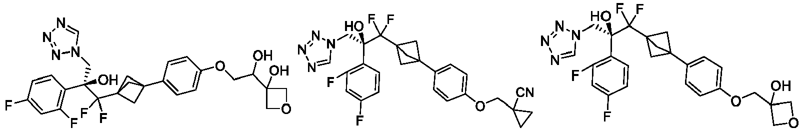

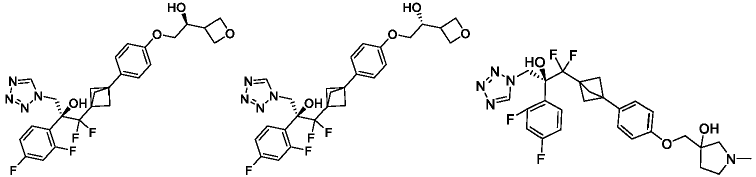

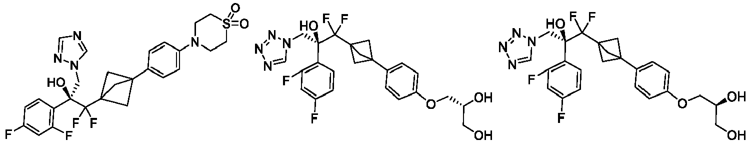

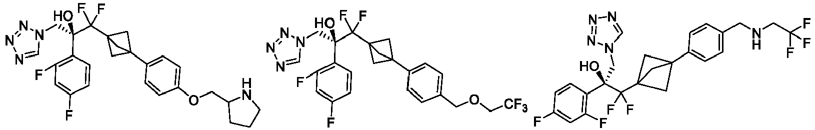

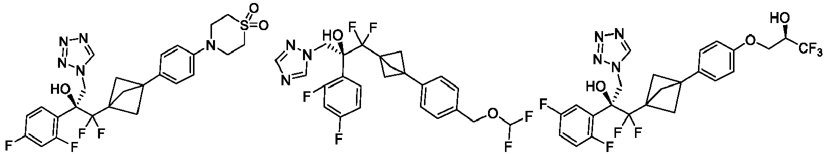

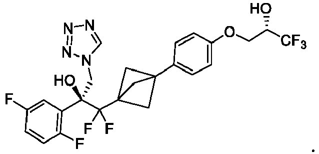

In yet another aspect of the present disclosure, the present disclosure also provides a use of the aforementioned compound, the optical isomer thereof, the tautomer thereof, or the pharmaceutically acceptable salt thereof in the manufacture of a medicament against fungal infections.

### Definition and description

Unless otherwise specified, the following terms and phrases used herein have the following meanings. A specific term or phrase should not be considered indefinite or unclear in the absence of a particular definition, but should be understood in the ordinary sense. When a trade name appears herein, it is intended to refer to its corresponding commodity or active ingredient thereof.

As used herein, the phrase "at least one" when referring to a list of one or more than one element should be understood to mean at least one element selected from any one or more elements in the list of elements, but not necessarily including at least one of each element specified in the list of elements, and not excluding any combination of elements in the list of elements. This definition also allows that elements may optionally be present other than the elements specifically identified within the list of elements to which the phrase "at least one" refers, whether related or unrelated to those elements specifically identified.

The term "pharmaceutically acceptable" is used herein in terms of those compounds, materials, compositions, and/or dosage forms, which are suitable for use in contact with human and animal tissues within the scope of reliable medical judgment, with no excessive toxicity, irritation, an allergic reaction, or other problems or complications, commensurate with a reasonable benefit/risk ratio.

The term "pharmaceutically acceptable anion" refers to an anion of a pharmaceutically acceptable inorganic acids (e.g., a mineral acids) such as a chloride anion, a bromide anion, an iodide anion, a sulfate anion, or a bisulfate anion; or an anion derived from an organic acids (e.g., an aliphatic, aromatic, or arylaliphatic carboxylic, or sulfonic acid) such as an acetoxy anion, a trifluoroacetoxy anion, a methanesulfonyloxy anion.

The term "pharmaceutically acceptable salt" refers to a salt of the compound of the present disclosure that is prepared by reacting the compound having a specific substituent of the present disclosure with a relatively non-toxic acid or base. When the compound of the present disclosure contains a relatively acidic functional group, a base addition salt can be obtained by contacting a neutral form of the compound with a sufficient amount of a base in a pure solution or a suitable inert solvent. The pharmaceutically acceptable base addition salt includes a salt of sodium, potassium, calcium, ammonium, organic amine, magnesium, or similar salts. When the compound of the present disclosure contains a relatively basic functional group, an acid addition salt can be obtained by contacting the neutral form of the compound with a sufficient amount of acid in a solution or a suitable inert solvent. Examples of the pharmaceutically acceptable acid addition salt include an inorganic acid salt, wherein the inorganic acid includes, for example, hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodic acid, phosphorous acid; and an organic acid salt, wherein the organic acid includes, for example, acetic acid, propionic acid, isobutyric acid, trifluoroacetic acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, tartaric acid, and methanesulfonic acid; and salts of amino acid (such as arginine), and a salt of an organic acid such as glucuronic acid. Certain specific compounds of the present disclosure contain both basic and acidic functional groups, thus can be converted to any base or acid addition salt.

The pharmaceutically acceptable salt of the present disclosure can be prepared from the parent compound that contains an acidic or basic moiety by conventional chemical method. Generally, such salt can be prepared by: reacting the free acid or base form of the compound with a stoichiometric amount of an appropriate base or acid in water or an organic solvent or a mixture thereof.

When any variable (such as R) occurs in the constitution or structure of the compound more than once, the definition of the variable at each occurrence is independent. Thus, for example, if a group is substituted by 0 to 2 R, the group can be optionally substituted by up to two R, wherein the definition of R at each occurrence is independent. Moreover, a combination of the substituent and/or the variant thereof is allowed only when the combination results in a stable compound. For example, 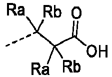 can be selected from 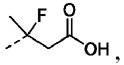 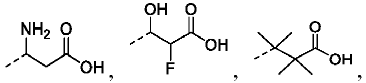 etc.

A hyphen ("-") being not between two letters or symbols indicates the linkage site of a substituent. For example, C₁₋₆ alkylcarbonyl- refers to a C₁₋₆ alkyl linked to the rest of the molecule through a carbonyl group. However, when the linkage site of a substituent is obvious to those skilled in the art, for example, a halogen substituent, "-" can be omitted.

When the valence bond of a group has a dashed line " 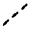 ", such as in 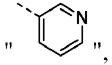 the dashed line indicates the linkage site of the group to the rest of the molecule. When there is " 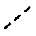 " on a single bond, for example, in " 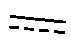 ", the dashed line represents a single bond or is absent, and also means that " 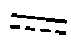 " represents a single bond " 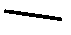 " or a double bond " 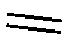 ".

The term "substituted" or "substituted by ..." means one or more than one hydrogen atom(s) on a specific atom are substituted by the substituent including deuterium and hydrogen variables, as long as the valence of the specific atom is normal and the substituted compound is stable. The term "optionally substituted" or "optionally substituted by ..." means an atom can be substituted by a substituent or not, unless otherwise specified, the type and number of the substituent may be arbitrary as long as being chemically achievable.

When any variable (such as R) occurs in the constitution or structure of the compound more than once, the definition of the variable at each occurrence is independent. Thus, for example, if a group is substituted by 1, 2, or 3 R', the group can be optionally substituted by 1, 2, or 3 R', wherein the definition of R' at each occurrence is independent. Moreover, a combination of the substituent and/or the variant thereof is allowed only when the combination results in a stable compound.

When one of the variables is selected from a single bond, it means that the two groups linked by the single bond are connected directly. For example, when L₁ in 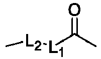 represents a single bond, the structure is actually

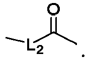

When the enumerative substituent does not indicate by which atom it is linked to the group to be substituted, such substituent can be bonded by any atom thereof. For example, when pyridyl acts as a substituent, it can be linked to the group to be substituted by any carbon atom on the pyridine ring.

When the listed linking group does not indicate the direction for linking, the direction for linking is arbitrary, for example, the linking group L contained in 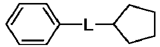 is - CH₂O-, then -CH₂O- can link phenyl and cyclopentyl to form 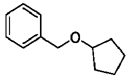 in the direction same as left-to-right reading order, and can link phenyl and cyclopentyl to form 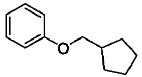 in the direction contrary to a left-to-right reading order. A combination of the linking groups, substituents and/or variables thereof is allowed only when such combination can result in a stable compound.

Unless otherwise specified, the number of atoms in a ring is usually defined as the number of ring members, for example, "3- to 6-membered ring" refers to a "ring" in which 3 to 6 atoms are arranged around.

Unless otherwise specified, the term "C₁₋₆ alkyl" refers to a linear or branched saturated hydrocarbon group consisting of 1 to 6 carbon atoms. The C₁₋₆ alkyl includes C₁₋₅, C₁₋₄, C₁₋₃, C₁₋₂, C₂₋₆, C₂₋₄, C₆, C₅ alkyl, etc.; it can be monovalent (such as CH₃), divalent (such as -CH₂-), or multivalent (such as 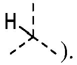 Examples of C₁₋₆ alkyl include, but are not limited to, CH₃, 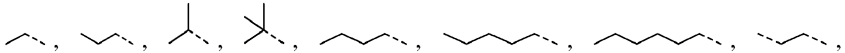 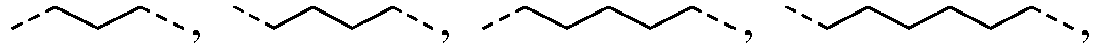 etc.

Unless otherwise specified, the term "C₁₋₄ alkyl" refers to a linear or branched saturated hydrocarbon group consisting of 1 to 4 carbon atoms. The C₁₋₄ alkyl includes C₁₋₂ alkyl, C_{1-3 alkyl}, C₃₋₄ alkyl, C₂₋₃ alkyl, etc.; it can be monovalent (such as CH₃), divalent (such as -CH₂-), or multivalent (such as 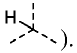 Examples of C₁₋₄ alkyl include, but are not limited to, CH₃, 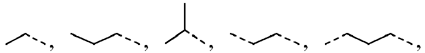 etc.

Unless otherwise specified, "C₂₋₆ alkenyl" refers to a linear or branched hydrocarbon group consisting of 2 to 6 carbon atoms containing at least one carbon-carbon double bond, and the carbon-carbon double bond may be located at any position of the group. The C₂₋₆ alkenyl includes C₂₋₄ alkenyl, C₂₋₃ alkenyl, C₄ alkenyl, C₃ alkenyl, C₂ alkenyl, etc.; it can be monovalent, divalent, or multivalent. Examples of C₂₋₆ alkenyl include, but are not limited to, ethenyl, propenyl, butenyl, pentenyl, hexenyl, butadienyl, pentadienyl, hexadienyl, etc.

Unless otherwise specified, "C₂₋₃ alkenyl" refers to a linear or branched hydrocarbon group consisting of 2 to 3 carbon atoms containing at least one carbon-carbon double bond, and the carbon-carbon double bond may be located at any position of the group. The C₂₋₃ alkenyl includes C₃ and C₂ alkenyl; the C₂₋₃ alkenyl can be monovalent, divalent, or multivalent. Examples of C₂₋₃ alkenyl include, but are not limited to, 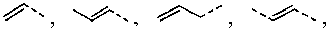 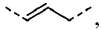 etc.

Unless otherwise specified, "C₂₋₆ alkynyl" refers to a linear or branched hydrocarbon group consisting of 2 to 6 carbon atoms containing at least one carbon-carbon triple bond, and the carbon-carbon triple bond may be located at any position of the group. It can be monovalent, divalent, or multivalent. The C₂₋₆ alkynyl includes C₂₋₃, C₂₋₄, C₂₋₅, C₃₋₄, C₃₋₅, C₃₋₆, C₄₋₅, C₄₋₆, C₅₋₆, C₆, C₅, C₄, C₃, and C₂ alkynyl. Examples of C₂₋₆ alkynyl include, but are not limited to, 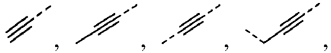 etc.

Unless otherwise specified, "C₂₋₃ alkynyl" refers to a linear or branched hydrocarbon group consisting of 2 to 3 carbon atoms containing at least one carbon-carbon triple bond, and the carbon-carbon triple bond may be located at any position of the group. It can be monovalent, divalent, or multivalent. The C₂₋₃ alkynyl includes C₃ and C₂ alkynyl. Examples of C₂₋₃ alkynyl include, but are not limited to, 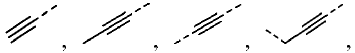 etc.

The term "heteroalkyl" by itself or in combination with another term means a stable linear or branched alkyl group or a combination thereof consisting of a certain number of carbon atoms, and at least one heteroatom or heteroatom group. In some embodiments, the heteroatom is selected from B, O, N, and S, wherein the nitrogen and sulfur atoms are optionally oxidized, and the nitrogen heteroatom is optionally quaternized. In other embodiments, the heteroatom group is selected from -C(=O)O-, -C(=O)-, -C(=S)-, -S(=O), - S(=O)₂-, -C(=O)N(H)-, -N(H)-, -C(=NH)-, -S(=O)₂N(H)-, and -S(=O)N(H)-. In some embodiments, the heteroalkyl is C₁₋₆ heteroalkyl; in other embodiments, the heteroalkyl is C₁₋₃ heteroalkyl. The heteroatom or heteroatom group may be located at any internal position within a heteroalkyl group, including the site where the alkyl group is linked to the rest of the molecule, but the term "alkoxy" is a conventional expression referring to those alkyl groups linked to the rest of the molecule through an oxygen atom. Examples of heteroalkyl include, but are not limited to, -OCH₃, -OCH₂CH₃, -OCH₂CH₂CH₃, -OCH₂(CH₃)₂, -CH₂-CH₂-O-CH₃, -NHCH₃, -N(CH₃)₂, -NHCH₂CH₃, -N(CH₃)(CH₂CH₃), -CH₂-CH₂-NH-CH₃, -CH₂-CH₂-N(CH₃)-CH₃, -SCH₃, -SCH₂CH₃, -SCH₂CH₂CH₃, -SCH₂(CH₃)₂, -CH₂-S-CH₂-CH₃, -CH₂-CH₂, -S(=O)-CH₃, -CH₂-CH₂-S(=O)₂-CH₃, and up to two heteroatoms may be consecutive, e.g., - CH₂-NH-OCH₃.

Unless otherwise specified, the term "C₁₋₆ alkoxy" refers to an alkyl group containing 1 to 6 carbon atoms that are connected to the rest of the molecule through an oxygen atom. The C₁₋₆ alkoxy includes C₁₋₄ alkoxy, C₁₋₃ alkoxy, C₁₋₂ alkoxy, C₂₋₆ alkoxy, C₂₋₄ alkoxy, C₆ alkoxy, C₅ alkoxy, C₄ alkoxy, C₃ alkoxy, etc. Examples of C₁₋₆ alkoxy include, but are not limited to, methoxy, ethoxy, propoxy (including n-propoxy and isopropoxy), butoxy (including n-butoxy, isobutoxy, s-butoxy, and t-butoxy), pentyloxy (including n-pentyloxy, isopentyloxy, and neopentyloxy), hexyloxy, etc.

Unless otherwise specified, the term "C₁₋₃ alkoxy" refers to an alkyl group containing 1 to 3 carbon atoms that are connected to the rest of the molecule through an oxygen atom. The C₁₋₃ alkoxy includes C₁₋₃ alkoxy, C₁₋₂ alkoxy, C₂₋₃ alkoxy, C₁ alkoxy, C₂ alkoxy, C₃ alkoxy, etc. Examples of C₁₋₃ alkoxy include, but are not limited to, methoxy, ethoxy, propoxy (including n-propoxy and isopropoxy), etc.

Unless otherwise specified, the term "C₁₋₆ alkylamino" refers to an alkyl group containing 1 to 6 carbon atoms that are connected to the rest of the molecule through an amino group. The C₁₋₆ alkylamino includes C₁₋₄ alkylamino, C₁₋₃ alkylamino, C₁₋₂ alkylamino, C₂₋₆ alkylamino, C₂₋₄ alkylamino, C₆ alkylamino, C₅ alkylamino, C₄ alkylamino, C₃ alkylamino, C₂ alkylamino, etc. Examples of C₁₋₆ alkylamino include, but are not limited to, -NHCH₃, - N(CH₃)₂, -NHCH₂CH₃, -N(CH₃)CH₂CH₃, -N(CH₂CH₃)(CH₂CH₃), -NHCH₂CH₂CH₃, - NHCH₂(CH₃)₂, -NHCH₂CH₂CH₂CH₃, etc.

Unless otherwise specified, the term "C₁₋₃ alkylamino" refers to an alkyl group containing 1 to 3 carbon atoms that are connected to the rest of the molecule through an amino group. The C₁₋₃ alkylamino includes C₁₋₃ alkylamino, C₁₋₂ alkylamino, C₂₋₃ alkylamino, C₁ alkylamino, C₂ alkylamino, C₃ alkylamino, etc. Examples of C₁₋₃ alkylamino include, but are not limited to, -NHCH₃, -N(CH₃)₂, -NHCH₂CH₃, -N(CH₃)CH₂CH₃, -NHCH₂CH₂CH₃, - NHCH₂(CH₃)₂, etc.

Unless otherwise specified, the term "C₁₋₆ alkylthio" refers to an alkyl group containing 1 to 6 carbon atoms that are connected to the rest of the molecule through a sulfur atom. The C₁₋₆ alkylthio includes C₁₋₄ alkylthio, C₁₋₃ alkylthio, C₁₋₂ alkylthio, C₂₋₆ alkylthio, C₂₋₄ alkylthio, C₆ alkylthio, C₅ alkylthio, C₄ alkylthio, C₃ alkylthio, C₂ alkylthio, etc. Examples of C₁₋₆ alkylthio include, but are not limited to, -SCH₃, -SCH₂CH₃, -SCH₂CH₂CH₃, -SCH₂(CH₃)₂, etc.

Unless otherwise specified, the term "C₁₋₃ alkylthio" refers to an alkyl group containing 1 to 3 carbon atoms that are connected to the rest of the molecule through a sulfur atom. The C₁₋₃ alkylthio includes C₁₋₃ alkylthio, C₁₋₂ alkylthio, C₂₋₃ alkylthio, C₁ alkylthio, C₂ alkylthio, C₃ alkylthio, etc. Examples of C₁₋₃ alkylthio include, but are not limited to, - SCH₃, -SCH₂CH₃, -SCH₂CH₂CH₃, -SCH₂(CH₃)₂, etc.

Unless otherwise specified, "C₃₋₆ cycloalkyl" refers to a saturated cyclic hydrocarbon group consisting of 3 to 6 carbon atoms in monocyclic and bicyclic systems, and the C₃₋₆ cycloalkyl includes C₃₋₅ cycloalkyl, C₄₋₅ cycloalkyl, C₅₋₆ cycloalkyl, etc.; it can be monovalent, divalent, or multivalent. Examples of C₃₋₆ cycloalkyl include, but are not limited to, cyclopropyl, cyclobutyl, cyclopentyl, cyclohexyl, etc.

Unless otherwise specified, the term "3- to 6-membered heterocyclyl" by itself or in combination with other terms refers to a saturated or partly unsaturated cyclic group consisting of 3 to 6 ring atoms, wherein 1, 2, 3, or 4 ring atoms are heteroatoms independently selected from O, S, and N, and the rest are carbon atoms, wherein nitrogen atoms are optionally quaternized, and nitrogen and sulfur heteroatoms can be optionally oxidized (i.e., NO and S(O)ₚ, p is 1 or 2). It includes monocyclic and bicyclic systems, wherein the bicyclic systems include a spiro ring, a fused ring, and a bridged ring. In addition, in the case of the "3- to 6-membered heterocyclyl", a heteroatom may occupy the connection position of the heterocyclyl with the rest of the molecule. The 3- to 6-membered heterocyclyl includes 4- to 6-membered heterocyclyl, 5- to 6-membered heterocyclyl, 4-membered heterocyclyl, 5-membered heterocyclyl, 6-membered heterocyclyl, etc. Examples of 3- to 6-membered heterocyclyl include, but are not limited to, azetidinyl, oxetanyl, thietanyl, 1,3-dioxolane, 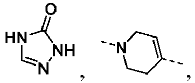 pyrrolidinyl, pyrazolidinyl, imidazolidinyl, tetrahydrothienyl (including tetrahydrothiophen-2-yl, tetrahydrothiophen-3-yl, etc.), tetrahydrofuranyl (including tetrahydrofuran-2-yl, etc.), tetrahydropyranyl, piperidinyl (including 1-piperidinyl, 2-piperidinyl, 3-piperidinyl, etc.), piperazinyl (including 1-piperazinyl, 2-piperazinyl, etc.), morpholinyl (including 3-morpholinyl, 4-morpholinyl, etc.), dioxinyl, dithianyl, isoxazolidinyl, isothiazolidinyl, 1,2-oxazinyl, 1,2-thiazinyl, hexahydropyridazinyl, homopiperazinyl, homopiperidinyl, etc.

Unless otherwise specified, the term "5- to 6-membered heterocyclyl" by itself or in combination with other terms refers to a saturated or partly unsaturated cyclic group consisting of 5 to 6 ring atoms, wherein 1, 2, 3, or 4 ring atoms are heteroatoms independently selected from O, S, and N, and the rest are carbon atoms, wherein nitrogen atoms are optionally quaternized, and nitrogen and sulfur heteroatoms can be optionally oxidized (i.e., NO and S(O)ₚ, p is 1 or 2). It includes monocyclic and bicyclic systems, wherein the bicyclic systems include a spiro ring, a fused ring, and a bridged ring. In addition, in the case of the "5- to 6-membered heterocyclyl", a heteroatom may occupy the connection position of the heterocyclyl with the rest of the molecule. The 5- to 6-membered heterocyclyl includes 5-membered heterocyclyl, 6-membered heterocyclyl, etc. Examples of 5- to 6-membered heterocyclyl include, but are not limited to, 1,3-dioxolane, 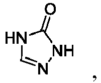 pyrrolidinyl, pyrazolidinyl, imidazolidinyl, tetrahydrothienyl (including tetrahydrothiophen-2-yl, tetrahydrothiophen-3-yl, etc.), tetrahydrofuranyl (including tetrahydrofuran-2-yl, etc.), tetrahydropyranyl, piperidinyl (including 1-piperidinyl, 2-piperidinyl, 3-piperidinyl, etc.), piperazinyl (including 1-piperazinyl, 2-piperazinyl, etc.), morpholinyl (including 3-morpholinyl, 4-morpholinyl, etc.), dioxinyl, dithianyl, isoxazolidinyl, isothiazolidinyl, 1,2-oxazinyl, 1,2-thiazinyl, hexahydropyridazinyl, homopiperazinyl, or homopiperidinyl, etc.

Unless otherwise specified, the terms "5- to 6-membered heteroaryl ring" and "5- to 6-membered heteroaryl" in the present disclosure can be used interchangeably, and the term "5- to 6-membered heteroaryl" means a monocyclic group consisting of 5 to 6 ring atoms with a conjugated π-electron system, wherein 1, 2, 3, or 4 ring atoms are heteroatoms independently selected from O, S, and N, and the rest are carbon atoms, wherein the nitrogen atom is optionally quaternized, and the heteroatoms nitrogen and sulfur can be optionally oxidized (i.e., NO and S(O)ₚ, wherein p is 1 or 2). The 5- to 6-membered heteroaryl can be linked to the rest of the molecule through a heteroatom or a carbon atom. The 5- to 6-membered heteroaryl includes 5- and 6-membered heteroaryl. Examples of the 5- to 6-membered heteroaryl include, but are not limited to, pyrrolyl (including N-pyrrolyl, 2-pyrrolyl, 3-pyrrolyl, etc.), pyrazolyl (including 2-pyrazolyl, 3-pyrrolyl, etc.), imidazolyl (including *N*-imidazolyl, 2-imidazolyl, 4-imidazolyl, 5-imidazolyl, etc.), oxazolyl (including 2-oxazolyl, 4-oxazolyl, 5-oxazolyl, etc.), triazolyl (1*H* -1,2,3-triazolyl, 2*H* -1,2,3-triazolyl, 1*H* -1,2,4-triazolyl, 4*H* -1, 2,4-triazolyl, etc.), tetrazolyl, isoxazolyl (3-isoxazolyl, 4-isoxazolyl, 5-isoxazolyl, etc.), thiazolyl (including 2-thiazolyl, 4-thiazolyl, 5-thiazolyl, etc.), furyl (including 2-furyl, 3-furyl, etc.), thienyl (including 2-thienyl, 3-thienyl, etc.), pyridyl (including 2-pyridyl, 3-pyridyl, 4-pyridyl, etc.), pyrazinyl, or pyrimidyl (including 2-pyrimidyl, 4-pyrimidyl, etc.).

Unless otherwise specified, Cₙ₋ₙ₊ₘ or Cₙ-Cₙ₊ₘ includes any specific case of n to n+m carbons, for example, C₁₋₁₂ includes C₁, C₂, C₃, C₄, C₅, C₆, C₇, C₈, C₉, C₁₀, C₁₁, and C₁₂, and any range from n to n+m is also included, for example C₁₋₁₂ includes C₁₋₃, C₁₋₆, C₁₋₉, C₃₋₆, C₃₋₉, C₃₋₁₂, C₆₋₉, C₆₋₁₂, and C₉₋₁₂; similarly, n-membered to n+m-membered means that the number of atoms on the ring is from n to n+m, for example, 3- to 12-membered ring includes 3-membered ring, 4-membered ring, 5-membered ring, 6-membered ring, 7-membered ring, 8-membered ring, 9-membered ring, 10-membered ring, 11-membered ring, and 12-membered ring, and any range from n to n+m is also included, for example, 3- to 12-membered ring includes 3- to 6-membered ring, 3- to 9-membered ring, 5- to 6-membered ring, 5- to 7-membered ring, 5- to 10-membered ring, 6- to 7-membered ring, 6- to 8-membered ring, 6- to 9- membered ring, and 6- to 10-membered ring.

The term "leaving group" refers to a functional group or atom which can be replaced by another functional group or atom through a substitution reaction (such as nucleophilic substitution reaction). For example, representative leaving groups include triflate; chlorine, bromine, and iodine; sulfonate group, such as mesylate, tosylate, p-bromobenzenesulfonate, p-toluenesulfonate; acyloxy, such as acetoxy, trifluoroacetoxy.

The term "protecting group" includes, but is not limited to, "amino protecting group", "hydroxyl protecting group", or "mercapto protecting group". The term "amino protecting group" refers to a protecting group suitable for preventing the side reactions occurring at the nitrogen of an amino. Representative amino protecting groups include, but are not limited to: formyl; acyl, such as alkanoyl (e.g., acetyl, trichloroacetyl, or trifluoroacetyl); alkoxycarbonyl, such as tert-butoxycarbonyl (Boc); arylmethoxycarbonyl such as benzyloxycarbonyl (Cbz) and 9-fluorenylmethoxycarbonyl (Fmoc); arylmethyl, such as benzyl (Bn), trityl (Tr), 1,1-bis-(4'-methoxyphenyl)methyl; silyl, such as trimethylsilyl (TMS) and tert-butyldimethylsilyl (TBS). The term "hydroxyl protecting group" refers to a protecting group suitable for blocking the side reaction on hydroxyl. Representative hydroxyl protecting groups include, but are not limited to: alkyl, such as methyl, ethyl, and tert-butyl; acyl, such as alkanoyl (e.g., acetyl); arylmethyl, such as benzyl (Bn), p-methoxybenzyl (PMB), 9-fluorenylmethyl (Fm), and diphenylmethyl (benzhydryl, DPM); silyl, such as trimethylsilyl (TMS) and tert-butyl dimethyl silyl (TBS).

It should be understood by those skilled in the art that some compounds of formula (I) may contain one or more than one chiral center, and therefore there exist two or more stereoisomers. Therefore, the compounds of the present disclosure may be present in the form of individual stereoisomer (e.g., enantiomers, diastereomers) and mixtures thereof in arbitrary proportions, such as racemates, and under the proper condition, they may be present in the form of the tautomers and geometric isomers thereof.

The compounds of the present disclosure may exist in specific geometric or stereoisomeric forms. The present disclosure contemplates all such compounds, including cis and trans isomers, (-)- and (+)-enantiomers, (*R*)- and (*S*)-enantiomers, diastereoisomers, (*D*)-isomers, (*L*)-isomers, racemic, and other mixtures thereof, such as enantiomers or diastereomer enriched mixtures, all of which are within the scope of the present disclosure. Additional asymmetric carbon atoms may be present in substituents such as alkyl. All these isomers and their mixtures are encompassed within the scope of the present disclosure.

As used herein, the term "stereoisomer" refers to compounds that have the same chemical constitution, but differ in the arrangement of the atoms or groups in space. Stereoisomer includes enantiomer, diastereomer, conformer, etc.

As used herein, the term "enantiomer" refers to two stereoisomers of a compound that are non-superimposable mirror images of each other.

As used herein, the term "diastereomer" refers to a stereoisomer having two or more chiral centers and whose molecules are not mirror images of each other. Diastereomers have different physical properties such as melting points, boiling points, spectral properties, or biological activities. Mixtures of diastereomers can be separated by high resolution analytical methods (such as electrophoresis) and chromatography (such as HPLC separation).

Stereochemical definitions and conventions can be followed in S. P. Parker ed., McGraw-Hill Dictionary of Chemical Terms (1984) McGraw-Hill Book Company, New York; and Eliel, E. and Wilen, S., "Stereochemistry of Organic Compounds", John Wiley & Sons, Inc., New York, 1994. Many organic compounds exist in optically active forms, i.e., they have the ability to rotate the plane of plane-polarized light. When optically active compounds are described, the prefixes D and L or R and S are used to denote the absolute configuration of the molecule with regard to its chiral centers. The prefixes d and 1 or (+) and (-) are used to denote the symbols of the rotation of planar-polarized light of a compound, wherein (-) or 1 indicates that the compound is levorotatory. Compounds with a prefix of (+) or d are dextrorotatory. For a given chemical structure, these stereoisomers are identical except that they are mirror images of each other. Specific stereoisomers can also be referred to as enantiomers, and mixtures of such isomers are often referred to as enantiomeric mixtures. A mixture of enantiomers in a ratio of 50 to 50 is known as a racemic mixture or racemate, which can be present in a chemical reaction or process without stereoselectivity or stereospecificity. The terms "racemic mixture" and "racemate" refer to an equimolar mixture of two enantiomers that is not optically active.

Racemic mixture can be used in its own form or resolved into individual isomers. Stereochemically pure compounds or a mixture enriched in one or more than one isomer may be obtained by resolution. Methods for separating isomers are well known (see Allinger N. L. and Eliel E. L., "Topics in Stereochemistry", Vol. 6, Wiley Interscience, 1971), including physical methods such as chromatography using chiral adsorbents. Individual isomers in a chiral form can be prepared from chiral precursors. Alternatively, a single isomer can be chemically separated from the mixture by forming a diastereomer salt with a chiral acid (such as a single enantiomer of 10-camphorsulfonic acid, camphoric acid, α-bromocamphoric acid, tartaric acid, diacetyltartaric acid, malic acid, pyrrolidone-5-carboxylic acid). The salt is subjected to fractional crystallization, and then one or two of the split bases are freed. This process can be optionally repeated to obtain one or two isomers that essentially do not contain the other isomer, i.e., the desired stereoisomer with an optical purity of at least 91%, 92%, 93%, 94%, 95%, 96%, 97%, 98%, 99%, or 99.5% by weight. Alternatively, the racemate can be covalently linked to a chiral compound (auxiliary) to obtain diastereomers, as is well known to those skilled in the art.

The compounds of the present disclosure may exist in specific. Unless otherwise specified, the term "tautomer" or "tautomeric form" means that at room temperature, the isomers of different functional groups are in dynamic equilibrium and can be transformed into each other quickly. If tautomers are possible (e.g., in solution), then chemical equilibrium of the tautomers can be achieved. For example, proton tautomer (also called prototropic tautomer) includes interconversion through proton migration, such as keto-enol isomerization and imine-enamine isomerization. Valence tautomer includes some recombination of bonding electrons for mutual transformation. A specific example of keto-enol tautomerization is the tautomerism between two tautomers of pentane-2,4-dione and 4-hydroxypent-3-en-2-one.

The compound of the present disclosure may contain an unnatural proportion of atomic isotope at one or more than one atom that constitute the compound. For example, the compound can be radiolabeled with a radioactive isotope, such as tritium (³H), iodine-125 (¹²⁵I), or C-14 (¹⁴C). For another example, deuterated drugs can be formed by replacing hydrogen with heavy hydrogen, the bond formed by deuterium and carbon is stronger than that of ordinary hydrogen and carbon, compared with non-deuterated drugs, deuterated drugs have the advantages of reduced toxic and side effects, increased drug stability, enhanced efficacy, extended biological half-life of drugs, etc. All isotopic variations of the compound of the present disclosure, whether radioactive or not, are encompassed within the scope of the present disclosure.

"Optional" or "optionally" means that the subsequently described event or circumstance may, but does not necessarily, occur, and the description includes instances where the event or circumstance occurs and instances where it does not.

The compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, preferred embodiments include but are not limited to the examples of the present disclosure.

The solvents used in the present disclosure are commercially available.

The compounds of the present disclosure are named according to the conventional naming principles in the art or by ChemDraw^{®} software, and the commercially available compounds use the supplier catalog names.

The compounds disclosed in the present disclosure may possess one or more than one chiral center, each of which independently exhibits either an R or S configuration. In some compounds disclosed by the present disclosure, chiral centers are labeled as *R, *S, R*, or S*, which indicates that the absolute configuration of the chiral center of the compound has not been identified. However, the compound has been subjected to chiral resolution and the chiral center is of a single configuration. The compound is a single configuration enantiomeric monomer, or a single configuration diastereomeric monomer, or a mixture of diastereomers with a single configuration at that chiral center (for example, where the configurations of other chiral centers have not been resolved). When the absolute configuration (R or S configuration) of the chiral center of the compounds disclosed in the present disclosure has not been identified, such compounds can be identified based on their retention time (R_{T}) under corresponding chromatographic column conditions (e.g., chromatographic column model, chromatographic column packing material, chromatographic column dimensions, mobile phase, etc.).

The present disclosure is further described in detail by the examples below. However, it should be understood that these examples are only used to illustrate the present disclosure and are not intended to limit the scope of the present disclosure. Experimental methods in the following examples in which specific conditions are not indicated are usually in accordance with the conventional conditions for this type of reaction, or in accordance with the conditions recommended by the manufacturer. Unless otherwise specified, percentages and parts are by weight. Unless otherwise specified, ratios of liquids are by volume.

The technical and scientific terms used herein that are not specifically defined have the meanings commonly understood by those skilled in the art to which the present disclosure belongs.

### BRIEF DESCRIPTION OF THE DRAWINGS

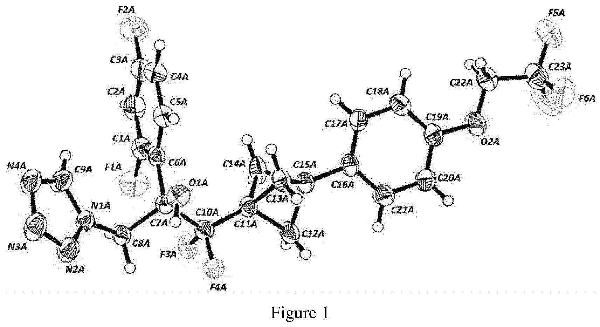

Figure 1 is an X-ray single crystal diffraction pattern of compound 3A.

### DETAILED DESCRIPTION OF THE PREFERRED EMBODIMENT

The present disclosure is described in detail by the examples below, but it does not mean that there are any adverse restrictions on the present disclosure. The present disclosure has been described in detail herein, and its specific embodiments have also been disclosed; for one skilled in the art, it is obvious to make various modifications and improvements to the embodiments of the present disclosure without departing from the spirit and scope of the present disclosure.

The experimental materials and reagents used in the following examples can be obtained from commercially available sources unless otherwise specified.

In all the examples, the ¹H-NMR, ¹³C-NMR, and ¹⁹F-NMR spectra are recorded by using Bruker Ascend 400 mHz nuclear magnetic resonance instrument, and the spectra are processed by using Topspin software, with deuterated solvents as an internal deuterium lock. ¹³C-NMR and ¹⁹F-NMR are decoupled from ¹H. Assignments are made based on explicit chemical shift/coupling patterns, or based on 2D Cosy, HMBC, HSQC, or NOESY experiments. The multiplicity of peaks is defined as: s (singlet), d (doublet), t (triplet), q (quartet), m (multiplet), br (broad peak), br.s (broad singlet); the coupling constant (*J*) is accurate to 0.1 Hz. Mass spectra are recorded by Agilent 1260 (ESI), Shimadzu LC-MS-2020 (ESI), or Agilent 6215 (ESI) mass spectrometer; reversed-phase preparative HPLC separation is carried out by using an Agilent 1290 UV-guided fully automatic purification system (Xtimate ^{®}Prep C18 OBDTM 21.2 * 250 mm 10 µm column), a Gilson GX281 UV-guided fully automatic purification system (xBridge ^{®}Prep C18 OBDTM 19 * 250 mm 10 µm column), or a Waters QDa-guided fully automatic purification system (SunFire ^{®}Prep C18 OBD 29 * 250 mm 10 µm column). Unless otherwise specified, the separation is performed by using a SepaFlash pre-installed normal phase silica gel column (Sinopharm Chemical Reagent Co., Ltd.) or a TLC analysis plate (Yantai Jiangyou Silica Gel Development Co., Ltd., model: HSGF254, specification: 2.5 × 5 cm), and the ratios of the eluent are all volume ratios.

The names of the reagents, represented by chemical formula or alphabetical abbreviations, are as follows:

CD₃OD stands for deuterated methanol; DMSO-*d6* stands for deuterated dimethyl sulfoxide; Chloroform-*d* or CDCl₃ stands for deuterated chloroform; AcOH stands for acetic acid; AlCl₃ stands for aluminum trichloride; Aq stands for aqueous solution; N₂ stands for nitrogen; Ar stands for argon; B₂Pin₂ stands for bis(pinacolato)diboron; BBr₃ stands for boron tribromide; BH₃ stands for borane; (Boc)₂O stands for di-tert-butyl dicarbonate; Et₃SiH stands for triethylsilane; HATU stands for 1-[bis(dimethylamino)methylene]-1H-1,2,3-triazolo[4,5-b]pyridinium 3-oxide hexafluorophosphate; HOBt stands for 1-hydroxybenzotriazole; K₂CO₃ stands for potassium carbonate; KOAc stands for potassium acetate; MeONa stands for sodium methoxide; LDA stands for lithium diisopropylamide; LiHMDS stands for lithium bis(trimethylsilyl)amide; LiOH stands for lithium hydroxide; m-CPBA stands for m-chloroperbenzoic acid; Na₂CO₃ stands for sodium carbonate; NaBH₄ stands for sodium borohydride; NaCl stands for sodium chloride; NaHCOs stands for sodium bicarbonate; NaOH stands for sodium hydroxide; Na₂SO₄ stands for sodium sulfate; NBS stands for N-bromosuccinimide; *n*-BuLi stands for n-butyllithium; NH₄Cl stands for ammonium chloride; NMP stands for N-methyl-2-pyrrolidone; PBr₃ stands for phosphorus tribromide; Pd(dppf)Cl₂ or PdCl₂(dppf) stands for [1,1'-bis(diphenylphosphino)ferrocene]dichloropalladium(II); Pd(OAc)₂ stands for palladium acetate; conc. stands for concentrated; (COCl)₂ stands for oxalyl chloride; Cs₂CO₃ stands for cesium carbonate; CuCl stands for cuprous chloride; CuI stands for cuprous iodide; DCM stands for dichloromethane; Dioxane stands for 1,4-dioxane; MeCN, ACN, or CH₃CN stands for acetonitrile; MeOH stands for methanol; EtOH stands for ethanol; DEA stands for diethylamine; DIPEA or DIEA stands for N,N-diisopropylethylamine; DMAP stands for 4-dimethylaminopyridine; DMF stands for N,N-dimethylformamide; DMSO stands for dimethyl sulfoxide; EA or EtOAc stands for ethyl acetate; PE stands for petroleum ether; THF stands for tetrahydrofuran; tol. stands for toluene; SOCl₂ stands for thionyl chloride; TFA stands for trifluoroacetic acid; FA stands for formic acid; TMSCN stands for trimethylsilyl cyanide; H₂O stands for water; HCl stands for hydrogen chloride gas; HCl aq. stands for hydrochloric acid aqueous solution; °C stands for degrees Celsius; rt or RT stands for room temperature; h stands for hour; min stands for minute; g stands for gram; mg stands for milligram; mL stands for milliliter; mmol stands for millimole; M stands for mole; cm stands for centimeter; mm stands for millimeter; µm stands for micrometre; nm stands for nanometer; mL/min stands for milliliters per minute; Hz stands for hertz; MHz stands for megahertz; bar stands for pressure unit bar; psi stands for pressure unit pound per square inch; N₂ stands for nitrogen; HPLC stands for high performance liquid chromatography; I.D. stands for inner diameter; LCMS or LC-MS stands for liquid chromatography-mass spectrometry; m/z stands for mass-to-charge ratio; ESI stands for electrospray ionization; CO₂ stands for carbon dioxide; TLC stands for thin-layer chromatography; UV stands for ultraviolet; MC stands for methylcellulose; SBECD stands for sodium sulfobutyl ether β-cyclodextrin.

### Example 1: Preparation of compounds 1A and 1B

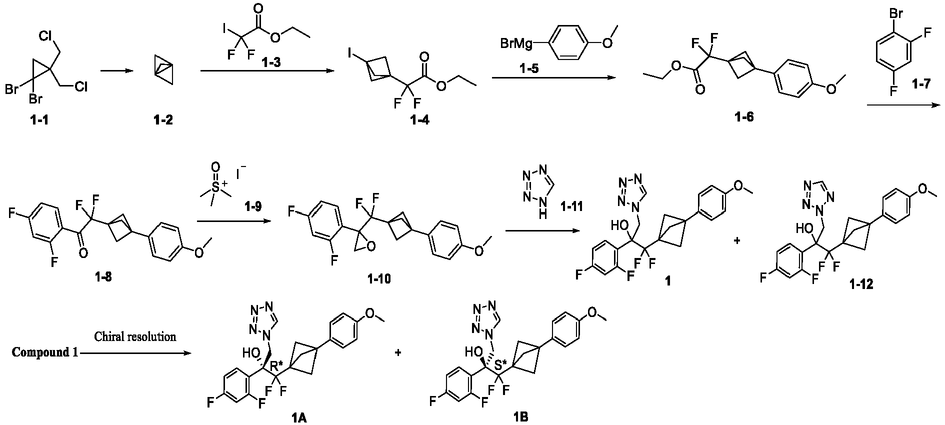

### Preparation of compound 1-4

To a three-necked flask was added compound **1-1** (28.2 g, 96 mmol), and the system was replaced with nitrogen three times. To the three-necked flask was added anhydrous ether (100 mL), and methyllithium (1.6 M, 120 mL, 192 mmol) was added dropwise thereto at a cooling temperature of -78°C. The reaction system was stirred and reacted at -78°C for 5 min, then gradually warmed to 0°C, stirred, and reacted for another 3 hours. Then the reaction system was added with compound **1-3** (15 g, 60 mmol) and stirred for another 16 hours at room temperature. After the reaction was completed, the reaction system was extracted with EtOAc (100 mL × 3), and the organic phases were combined, dried, and concentrated to obtain a crude product. The crude product was purified by normal-phase silica gel column chromatography (EtOAc/PE = 0 to 5%) to obtain the title compound **1-4** (10 g) as a light yellow liquid with a yield of 33%. ¹H NMR (400 MHz, CDCl₃) δ 4.33 (q, *J* = 7.2 Hz, 2H), 2.44 (s, 6H), 1.36 (t, *J* = 7.2 Hz, 3H). ¹⁹F NMR (376 MHz, CDCl₃) δ -109.15 (s, 2F).

### Preparation of compound 1-6

To a three-necked flask was added compound ferric acetylacetonate (2.2 g, 6.3 mmol), and the system was replaced with nitrogen three times. To the three-necked flask were sequentially added anhydrous THF (80 mL), compound **1-4** (10 g, 31.6 mmol), and TMEDA (1.5 g, 12.6 mmol). The reaction system was stirred for 5 min, then a solution **1-5** of Grignard reagent 4-methoxyphenyl magnesium bromide in THF (0.5 M, 102 mL, 50.6 mmol) was slowly added dropwise to the three-necked flask, and the reaction mixture was stirred and reacted at room temperature for another 16 hours. After the reaction was completed, the reaction system was extracted with EtOAc (30 mL × 3), and the organic phases were combined, dried, and concentrated to obtain a crude product. The crude product was purified by normal-phase silica gel column chromatography (EtOAc/PE = 0 to 5%) to obtain the title compound **1-6** (2.1 g) as a light yellow liquid with a yield of 23%. ¹H NMR (400 MHz, CDCl₃) δ 7.19 - 7.04 (m, 2H), 6.92 - 6.77 (m, 2H), 4.36 (q, *J* = 7.1 Hz, 2H), 3.79 (s, 3H), 2.16 (s, 6H), 1.37 (t, *J* = 7.1 Hz, 3H). ¹⁹F NMR (376 MHz, CDCl₃) δ -111.34 (s, 2F).

### Preparation of compound 1-8

To a three-necked flask was added compound **1-7** (1.1 g, 5.6 mmol), and the system was replaced with nitrogen three times. To the three-necked flask was added anhydrous ether (15 mL), and n-butyllithium (1.6 M, 3.5 mL, 5.6 mmol) was added dropwise thereto at a cooling temperature of -78°C. After the reaction system was stirred and reacted at -78°C for 45 min, a solution of compound **1-6** (1.5 g, 5.1 mmol) dissolved in anhydrous ether (10 mL) was added dropwise thereto, and the reaction mixture was stirred and reacted for another 1 hour. After the reaction was completed, the reaction system was quenched with saturated ammonium chloride solution (5 mL), extracted with EtOAc (20 mL × 3), and the organic phases were combined, dried, and concentrated to obtain a crude product. The crude product was purified by normal-phase silica gel column chromatography (EtOAc/PE = 0 to 5%) to obtain the title compound **1-8** (1.6 g) as a yellow solid with a yield of 86%. ¹H NMR (400 MHz, CDCl₃) δ 7.92 - 7.77 (m, 1H), 7.18 - 7.06 (m, 2H), 7.03 - 6.88 (m, 2H), 6.88 - 6.80 (m, 2H), 3.79 (s, 3H), 2.21 (s, 6H). ¹⁹F NMR (376 MHz, CDCl₃) δ -99.65 (d, *J* = 13.4 Hz, 1F), -103.94 (q, *J* = 13.8 Hz, 1F), -107.21 (d, *J* = 14.8 Hz, 2F).

### Preparation of compound 1-10

Compound **1-8** (300 mg, 0.8 mmol) was dissolved in a mixture of dichloromethane (5.0 mL) and water (2.0 mL), then trimethylsulfoxonium iodide **1-9** (272 mg, 1.2 mmol) and solid sodium hydroxide (66 mg, 1.7 mmol) were added thereto, and the reaction system was stirred at 65°C for 48 hours. After the reaction was completed, the reaction system was extracted with dichloromethane (20 mL × 3), and the organic phases were combined, dried, and concentrated to obtain a crude product. The crude product was purified by normal-phase silica gel column chromatography (EtOAc/PE = 0 to 5%) to obtain the title compound **1-10** (275 mg) with a yield of 89%. ¹H NMR (400 MHz, CDCl₃) δ 7.59 (q, *J* = 7.9 Hz, 1H), 7.12 - 7.04 (m, 2H), 6.97 - 6.89 (m, 1H), 6.88 - 6.78 (m, 3H), 3.78 (s, 3H), 3.40 (d, *J* = 5.2 Hz, 1H), 2.95 (qd, *J* = 4.0, 1.4 Hz, 1H), 2.11 - 2.00 (m, 6H).

### Preparation of compound 1

Compound **1-10** (275 mg, 0.73 mmol) was dissolved in DMF solution (1.5 mL), and compound 1H-tetrazole **1-11** (102 mg, 1.5 mmol) and potassium carbonate (151 mg, 1.1 mmol) were added thereto. The reaction system was stirred for 16 hours at 80°C in a sealed tube. After the reaction system was cooled, the reaction mixture was filtered, and the crude filtrate was purified by preparative separation (preparation method: mobile phase: A: 0.1% formic acid aqueous solution; B: acetonitrile; chromatographic column: Agilent 10 Prep-C18 250 × 21.2 mm; column temperature: 25°C; gradient: 55% to 75% acetonitrile in 12 min; flow rate: 30 mL/min) to obtain the title compound **1** (100 mg, yield: 31%) and the corresponding regioisomer compound **1-12** (50 mg, yield: 16%).

Compound **1:** LC-MS (ESI): m/z 447.0 [M-H]⁻. ¹H NMR (400 MHz, CDCl₃) δ 8.52 (s, 1H), 7.71 - 7.58 (m, 1H), 7.01 (d, *J* = 8.7 Hz, 2H), 6.83 (m, 2H), 6.80 (d, *J* = 8.7 Hz, 2H), 5.44 (d, *J* = 14.6 Hz, 1H), 5.04 (d, *J* = 14.6 Hz, 1H), 3.77 (s, 3H), 3.75 (brs, 1H), 2.04 (dd, *J* = 9.4, 1.8 Hz, 3H), 1.85 (dd, *J* = 9.5, 1.9 Hz, 3H).

Compound **1-12:** LC-MS (ESI): m/z 447.0 [M-H]⁻. ¹H NMR (400 MHz, CDCl₃) δ 8.39 (s, 1H), 7.64 (d, *J* = 6.5 Hz, 1H), 7.02 (d, *J* = 8.6 Hz, 2H), 6.87 - 6.76 (m, 4H), 5.74 (d, *J* = 14.3 Hz, 1H), 5.28 (d, *J* = 14.3 Hz, 1H), 4.24 (s, 1H), 3.77 (s, 3H), 2.04 (dd, *J* = 9.5, 1.8 Hz, 3H), 1.85 (dd, *J* = 9.5, 1.8 Hz, 3H).

### Preparation of compounds 1A and 1B

Compound **1** (70 mg) was subjected to SFC chiral preparative resolution (preparative separation method, instrument model: MG II preparative SFC (SFC-14); model of chromatographic column: ChiralCel OX, 250 × 30 mm I.D., 5 µm; mobile phase: A: CO₂ B: ethanol (0.1% NH₃ H₂O); elution gradient: B 30%; flow rate: 60 mL/min; column pressure: 100 bar; column temperature: 38°C; detection wavelength: 220 nm; cycle: about 5 min) to obtain the title compounds **1A** (28 mg) and **1B** (28 mg).

Compound **1A:** LC-MS (ESI): m/z 447.2 [M-H]⁻. Chiral analysis method (model of chromatographic column: Chiralcel OX-3 100 × 4.6 mm I.D., 3 µm; mobile phase: A: CO₂ B: ethanol (0.05% DEA); elution gradient: the mobile phase increased from 5% B to 40% B within 4 min and maintained at 40% B for 2.5 min for elution, then 5% B was equilibrated for 1.5 min; flow rate: 2.8 mL/min; column temperature: 35°C; column pressure: 1500 psi; detection wavelength: 220 nm; R_{T} = 3.413 min). ¹H NMR (400 MHz, CDCl₃) δ 8.55 (s, 1H), 7.64 (td, *J* = 9.1, 6.4 Hz, 1H), 7.08 - 6.91 (m, 2H), 6.91 - 6.69 (m, 4H), 5.45 (d, *J* = 14.5 Hz, 1H), 5.04 (d, *J* = 14.6 Hz, 1H), 4.03 (s, 1H), 3.76 (s, 3H), 2.03 (dd, *J* = 9.5, 1.9 Hz, 3H), 1.84 (dd, *J* = 9.5, 1.9 Hz, 3H).

Compound **1B:** LC-MS (ESI): m/z 447.2 [M-H]⁻. Chiral analysis method (model of chromatographic column: Chiralcel OX-3 100 × 4.6 mm I.D., 3 µm; mobile phase: A: CO₂ B: ethanol (0.05% DEA); elution gradient: the mobile phase increased from 5% B to 40% B within 4 min and maintained at 40% B for 2.5 min for elution, then 5% B was equilibrated for 1.5 min; flow rate: 2.8 mL/min; column temperature: 35°C; column pressure: 1500 psi; detection wavelength: 220 nm; Rt = 2.743 min). ¹H NMR (400 MHz, CDCl₃) δ 8.53 (s, 1H), 7.64 (td, *J* = 9.1, 6.6 Hz, 1H), 7.05 - 6.94 (m, 2H), 6.94 - 6.73 (m, 4H), 5.45 (d, *J* = 14.5 Hz, 1H), 5.04 (d, *J* = 14.6 Hz, 1H), 3.87 (s, 1H), 3.77 (s, 3H), 2.04 (dd, *J* = 9.5, 1.9 Hz, 3H), 1.85 (dd, *J* = 9.4, 1.8 Hz, 3H).

### Example 2: Preparation of compounds 2A and 2B

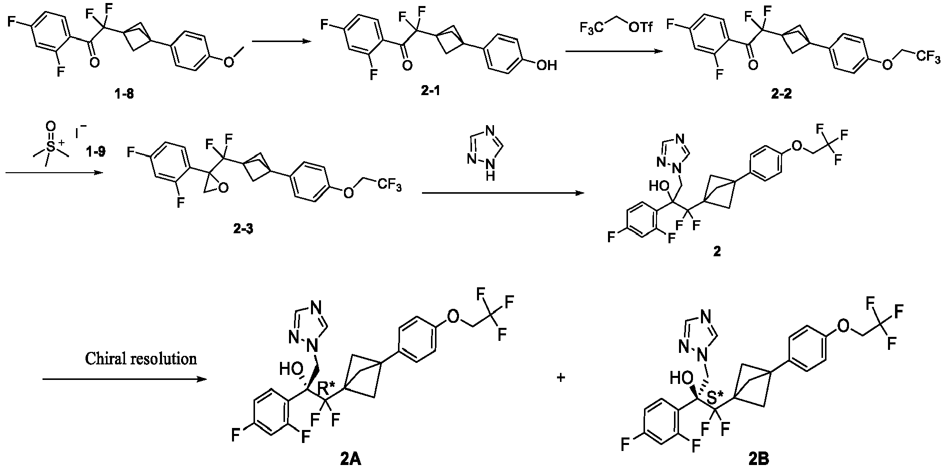

### Preparation of compound 2-1

To a microwave tube was added compound **1-8** (1.8 g, 4.95 mmol), then acetic acid (5 mL) and hydrogen bromide aqueous solution (48 wt. % in H₂O, 5 mL) were added thereto, and the reaction mixture was reacted at 95°C for 16 hours in a sealed tube. The reaction mixture was cooled, then evaporated to dryness by rotary evaporation, extracted with EtOAc (30 mL × 3), and the organic phases were combined, dried, and concentrated to obtain a crude product. The crude product was purified by normal-phase silica gel column chromatography (EtOAc/PE = 0 to 30%) to obtain the title compound **2-1** (1.3 g) as a brownish-yellow liquid with a yield of 72%. LC-MS (ESI): m/z 348.8 [M-H]⁻.

### Preparation of compound 2-2

Compound **2-1** (500 mg, 1.4 mmol) was dissolved in DMF (3 mL) solvent, then potassium carbonate (296 mg, 2.1 mmol) and 2,2,2-trifluoroethyl trifluoromethanesulfonate (500 mg, 2.1 mmol) were added thereto, and the reaction mixture was reacted at 65°C for 16 hours in a sealed tube. The reaction mixture was cooled and then extracted with EtOAc, and the organic phases were combined, dried, and concentrated to obtain a crude product. The crude product was purified by normal-phase silica gel column chromatography (EtOAc/PE = 0 to 5%) to obtain the title compound **2-2** (340 mg) as a light yellow oil with a yield of 55%. ¹H NMR (400 MHz, CDCl₃) δ 7.93 - 7.77 (m, 1H), 7.21 - 7.10 (m, 2H), 7.00 (dd, *J* = 2.3, 1.2 Hz, 1H), 6.93 (ddd, *J* = 9.5, 7.9, 1.6 Hz, 1H), 6.91 - 6.86 (m, 2H), 4.33 (q, *J* = 8.1 Hz, 2H), 2.23 (s, 6H). ¹⁹F NMR (376 MHz, CDCl₃) δ -73.96 (s, 3F), -99.55 (d, *J* = 13.6 Hz, 1F), - 103.71 - -104.08 (m, 1F), -107.08 - -107.36 (d, 2F).

### Preparation of compound 2-3

Compound **2-2** (340 mg, 0.79 mmol) was dissolved in a mixed solvent of dichloromethane (3 mL) and water (3 mL), then trimethylsulfoxonium iodide (433 mg, 1.97 mmol) and sodium hydroxide (79 mg, 1.97 mmol) were added thereto, and the reaction system was refluxed for 16 hours. After cooling to room temperature, the reaction system was extracted with DCM, and the organic phases were combined, dried, and concentrated to obtain a crude product. The crude product was purified by normal-phase silica gel column chromatography (EtOAc/PE = 0 to 5%) to obtain the title compound **2-3** (260 mg) as a light yellow oil with a yield of 73%. ¹H NMR (400 MHz, CDCl₃) δ 7.59 (td, *J* = 8.3, 6.4 Hz, 1H), 7.13 - 7.07 (m, 2H), 6.93 (tdd, *J* = 7.9, 2.6, 1.1 Hz, 1H), 6.89 - 6.80 (m, 3H), 4.32 (q, *J* = 8.1 Hz, 2H), 3.40 (d, *J* = 5.2 Hz, 1H), 2.95 (m, 1H), 2.12 - 2.01 (m, 6H). ¹⁹F NMR (376 MHz, CDCl₃) δ -73.96 (s, 3F), -107.46 (d, *J* = 8.6 Hz, 1F), -108.26 (dt, *J* = 11.9, 8.1 Hz, 1F), -110.91 (dd, *J* = 48.7, 9.6 Hz, 2F).

### Preparation of compound 2

1,2,4-Triazole (171 mg, 2.48 mmol) was dissolved in DMF (3 mL), and NaH (99 mg, 2.48 mmol) was added thereto at 0°C. After the reaction mixture was reacted for 30 min, compound **2-3** (221 mg, 0.49 mmol) was added thereto, and the reaction system was reacted at 70°C for 16 hours in a sealed tube. After the reaction system was cooled, the reaction mixture was filtered, and the crude filtrate was purified by preparative separation (chromatographic column: Agilent 10 Prep-C18 250 × 21.2 mm; column temperature: 25°C; mobile phase: water (0.1% FA)-acetonitrile; proportion of acetonitrile in the mobile phase: increased from 55% to 75% in 12 min; flow rate: 30 mL/min) to obtain the title compound **2** (106 mg, yield: 42%). LC-MS (ESI): m/z 516.2 [M+H]⁺. ¹H NMR (400 MHz, CDCl₃) δ 8.21 (s, 1H), 7.86 (s, 1H), 7.81 - 7.67 (m, 1H), 7.10 - 7.01 (m, 2H), 6.90 - 6.80 (m, 3H), 6.77 (ddd, *J* = 11.4, 8.4, 2.5 Hz, 1H), 5.32 (brs, 1H), 5.22 (d, *J* = 14.1 Hz, 1H), 4.85 (d, *J* = 14.1 Hz, 1H), 4.31 (q, *J* = 8.2 Hz, 2H), 2.07 (dd, *J* = 9.5, 1.9 Hz, 3H), 1.91 (dd, *J* = 9.5, 1.8 Hz, 3H).

### Preparation of compounds 2A and 2B

Compound **2** was subjected to SFC chiral preparative resolution (preparative separation method, instrument model: MG II preparative SFC(SFC-14); model of chromatographic column: ChiralPak AD, 250 × 30 mm I.D., 10 µm; mobile phase: A: CO₂ B: methanol (0.1% NH₃.H₂O); elution gradient: B 10%; flow rate: 60 mL/min; column pressure: 100 bar; column temperature: 38°C; detection wavelength: 220 nm; cycle: about 4.40 min) to obtain the title compounds **2A** (44 mg) and **2B** (42.6 mg).

Compound **2A:** LC-MS (ESI): m/z 516.2 [M+H]⁺. Chiral analysis method (model of chromatographic column: Chiralpak AD-3 150 × 4.6 mm I.D., 3 µm; mobile phase: A: CO₂ B: methanol (0.05% DEA); elution gradient: the mobile phase increased from 5% B to 40% B within 5 min and maintained at 40% B for 2.5 min for elution, then 5% B was equilibrated for 2.5 min; flow rate: 2.5 mL/min; column temperature: 35°C; column pressure: 1500 psi; detection wavelength: 220 nm; R_{T} = 2.557 min). ¹H NMR (400 MHz, CDCl₃) δ 7.91 (s, 1H), 7.59 (s, 1H), 7.54 - 7.41 (m, 1H), 6.89 - 6.74 (m, 2H), 6.62 - 6.55 (m, 3H), 6.50 (ddd, *J* = 11.3, 8.4, 2.5 Hz, 1H), 5.05 (brs, 1H), 4.95 (d, *J* = 14.2 Hz, 1H), 4.58 (d, *J* = 14.1 Hz, 1H), 4.05 (q, *J* = 8.1 Hz, 2H), 1.81 (dd, *J* = 9.5, 1.9 Hz, 3H), 1.65 (dd, *J* = 9.5, 1.9 Hz, 3H).

Compound **2B:** LC-MS (ESI): m/z 516.2 [M+H]⁺. Chiral analysis method (model of chromatographic column: Chiralpak AD-3 150 × 4.6 mm I.D., 3 µm; mobile phase: A: CO₂ B: methanol (0.05% DEA); elution gradient: the mobile phase increased from 5% B to 40% B within 5 min and maintained at 40% B for 2.5 min for elution, then 5% B was equilibrated for 2.5 min; flow rate: 2.5 mL/min; column temperature: 35°C; column pressure: 1500 psi; detection wavelength: 220 nm; R_{T} = 2.298 min). ¹H NMR (400 MHz, CDCl₃) δ 8.09 (s, 1H), 7.83 (s, 1H), 7.74 (td, *J* = 9.0, 6.4 Hz, 1H), 7.13 - 7.02 (m, 2H), 6.90 - 6.80 (m, 3H), 6.76 (ddd, *J* = 12.1, 8.4, 2.6 Hz, 1H), 5.31 (brs, 1H), 5.20 (d, *J* = 14.2 Hz, 1H), 4.83 (d, *J* = 14.2 Hz, 1H), 4.31 (q, *J* = 8.2 Hz, 2H), 2.07 (dd, *J* = 9.5, 1.9 Hz, 3H), 1.91 (dd, *J* = 9.5, 1.9 Hz, 3H).

### Preparation of compound 2-P1

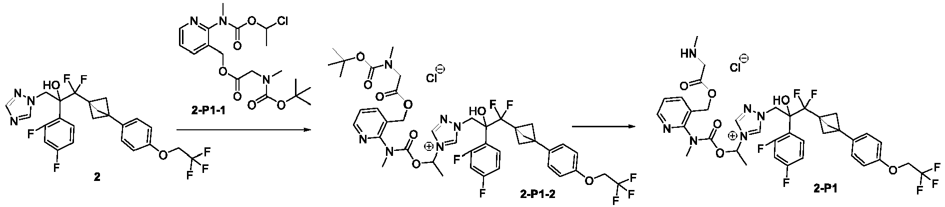

### Preparation of compound 2-P1-2

Compound **2** (100 mg, 0.19 mmol), compound **2-P1-1** (2-(((1-chloroethoxy)carbonyl)(methyl)amino)pyridin-3-yl)methyl N-(tert-butoxycarbonyl)-N-methylglycinate (CAS: 338990-31-1, 103 mg, 0.25 mmol), sodium iodide (38 mg, 0.25 mmol), and EtOAc (1.5 mL) were reacted at 50°C for 16 hours. The reaction mixture was filtered, and the crude filtrate was purified by preparative separation (chromatographic column: Agilent 10 Prep-C18 250 × 21.2 mm; column temperature: 25°C; mobile phase: water (0.1% FA)-acetonitrile; proportion of acetonitrile in the mobile phase: 40% to 60% in 12 min; flow rate: 30 mL/min) to obtain the title compound **2-P1-2** (80 mg, yield: 47%). LC-MS (ESI): m/z 895.50 [M-Cl]⁺.

### Preparation of compound 2-P1

To a reaction flask were added compound **2-P1-2** (80 mg, 0.09 mmol) and EtOAc (1.0 mL), then a solution of hydrochloric acid in 1,4-dioxane (4.0 M, 1 mL) was added dropwise thereto at 0°C, and the reaction mixture was reacted at room temperature for 16 hours. After the reaction was completed, the reaction system was extracted with EtOAc (100 mL × 3), and the aqueous phases were combined and freeze-dried to obtain the title compound **2-P1** (36 mg, yield: 50%) as a light yellow solid. Compound **2-P1:** LC-MS (ESI): m/z 795.40 [M-Cl]⁺.

### Example 3: Preparation of compounds 3A and 3B

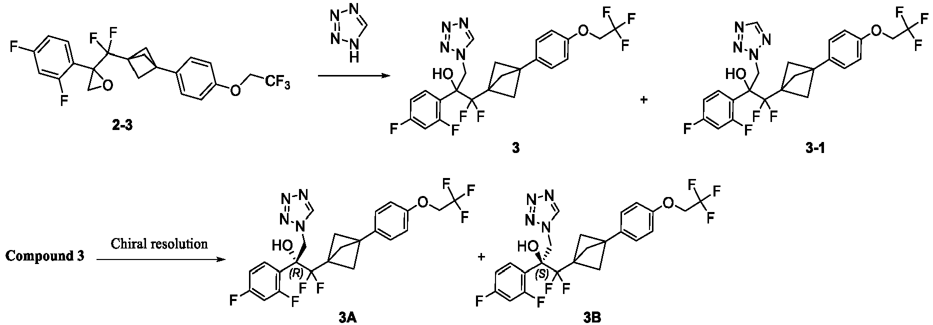

### Preparation of compound 3

Compound **2-3** (260 mg, 0.58 mmol) was dissolved in DMF (1.5 mL), then 1H-tetrazole (82 mg, 1.2 mmol) and potassium carbonate (121 mg, 0.87 mmol) were added thereto, and the reaction mixture was reacted at 80°C for 16 hours in a sealed tube. After the reaction system was cooled, the reaction mixture was filtered, and the crude filtrate was purified by preparative separation (preparation method: chromatographic column: Agilent 10 Prep-C18 250 × 21.2 mm; mobile phase: A: 0.1% formic acid aqueous solution; B: acetonitrile; column temperature: 25°C; gradient: 60% to 80% acetonitrile in 12 min; flow rate: 30 mL/min) to obtain the title compound **3** (120 mg, yield: 40%) and the corresponding regioisomer compound **3-1** (61 mg, yield: 20%).

Compound **3:** LC-MS (ESI): m/z 514.8 [M-H]⁻. ¹H NMR (400 MHz, CDCl₃) δ 8.52 (s, 1H), 7.65 (td, *J* = 8.9, 6.3 Hz, 1H), 7.11 - 6.98 (m, 2H), 6.93 - 6.76 (m, 4H), 5.44 (d, *J* = 14.6 Hz, 1H), 5.05 (d, *J* = 14.6 Hz, 1H), 4.30 (q, *J* = 8.1 Hz, 2H), 3.77 (brs, 1H), 2.05 (dd, *J* = 9.5, 1.9 Hz, 3H), 1.86 (dd, *J* = 9.5, 1.9 Hz, 3H).

Compound **3-1:** LC-MS (ESI): m/z 515.0 [M-H]⁻. ¹H NMR (400 MHz, CDCl₃) δ 8.40 (s, 1H), 7.64 (td, *J* = 9.0, 6.5 Hz, 1H), 7.09 - 7.00 (m, 2H), 6.88 - 6.76 (m, 4H), 5.74 (d, *J* = 14.2 Hz, 1H), 5.28 (d, *J* = 14.2 Hz, 1H), 4.30 (q, *J* = 8.1 Hz, 2H), 4.23 (brs, 1H), 2.05 (dd, *J =* 9.5, 1.9 Hz, 3H), 1.86 (dd, *J* = 9.5, 1.9 Hz, 3H).

### Preparation of compounds 3A and 3B

Compound **3** (55 mg) was subjected to SFC chiral preparative resolution (preparative separation method, instrument model: MG II preparative SFC(SFC-14); model of chromatographic column: ChiralPak IC, 250 × 30 mm I.D., 10 µm; mobile phase: A was CO₂, B was ethanol (containing 0.1% ammonia water); elution gradient: B 15%; flow rate: 60 mL/min; column pressure: 100 bar; column temperature: 38°C; detection wavelength: 220 nm; cycle: about 4 min) to obtain the title compounds **3A** (22 mg) and **3B** (27 mg).

Compound **3A:** LC-MS (ESI): m/z 517.2 [M+H]⁺. Chiral analysis method (model of chromatographic column: Chiralpak IC-3 150 × 4.6 mm I.D., 3 µm; mobile phase: A: CO₂ B: ethanol (0.05% DEA); elution gradient: the mobile phase increased from 5% B to 40% B within 5 min and maintained at 40% B for 2.5 min for elution, then 5% B was equilibrated for 2.5 min; flow rate: 2.5 mL/min; column temperature: 35°C; column pressure: 1500 psi; detection wavelength: 220 nm; R_{T} = 2.547 min). ¹H NMR (400 MHz, CDCl₃) δ 8.52 (s, 1H), 7.65 (td, *J* = 8.9, 6.3 Hz, 1H), 7.09 - 7.01 (m, 2H), 6.93 - 6.77 (m, 4H), 5.44 (d, *J* = 14.5 Hz, 1H), 5.05 (d, *J* = 14.6 Hz, 1H), 4.30 (q, *J* = 8.1 Hz, 2H), 3.83 (s, 1H), 2.05 (dd, *J* = 9.4, 1.9 Hz, 3H), 1.86 (dd, *J* = 9.4, 1.9 Hz, 3H).

Compound **3B:** LC-MS (ESI): m/z 515.0 [M-H]⁻. Chiral analysis method (model of chromatographic column: Chiralpak IC-3 150 × 4.6 mm I.D., 3 µm; mobile phase: A: CO₂ B: ethanol (0.05% DEA); elution gradient: the mobile phase increased from 5% B to 40% B within 5 min and maintained at 40% B for 2.5 min for elution, then 5% B was equilibrated for 2.5 min; flow rate: 2.5 mL/min; column temperature: 35°C; column pressure: 1500 psi; detection wavelength: 220 nm; R_{T} = 2.166 min). ¹H NMR (400 MHz, CDCl₃) δ 8.52 (s, 1H), 7.65 (td, *J* = 8.9, 6.3 Hz, 1H), 7.08 - 7.00 (m, 2H), 6.92 - 6.77 (m, 4H), 5.44 (d, *J* = 14.6 Hz, 1H), 5.05 (d, *J* = 14.6 Hz, 1H), 4.30 (q, *J* = 8.1 Hz, 2H), 3.83 (s, 1H), 2.05 (dd, *J* = 9.5, 1.9 Hz, 3H), 1.86 (dd, *J* = 9.5, 1.9 Hz, 3H).

Compound **3A** was cultured as a single crystal, and the absolute configuration of the chiral center was determined to be R-configuration by X-ray single crystal diffraction (Figure 1).

### Preparation of compound 3A-P1

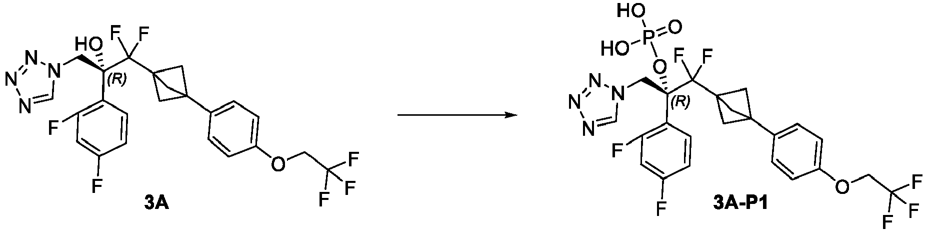

### Preparation of compound 3A-P1

To a sealed reaction tube was added compound **3A** (100 mg, 0.19 mmol), and then NaH (38 mg, 0.95 mmol) and THF (1 mL) were added thereto. The reaction mixture was reacted at 0°C for 30 min, added with phosphorus oxychloride (0.5 mL), and reacted at room temperature for 16 hours. The reaction mixture was added with saturated sodium bicarbonate aqueous solution, and reacted at 50°C for 16 hours. After the reaction system was cooled, the reaction mixture was filtered, and the crude filtrate was purified by preparative separation (chromatographic column: Welch Xtimate@ C18 21.2 × 250 mm; column temperature: 25°C; mobile phase: water (10 mM/L NH₄HCO₃)-acetonitrile; proportion of acetonitrile in the mobile phase: 35% to 75% in 12 min; flow rate: 30 mL/min) to obtain the title compound **3A-P1** (40 mg, yield: 35%).

Compound **3A-P1**: LC-MS (ESI): m/z 597.00 [M+H]⁺. ¹H NMR (400 MHz, DMSO-*d6*) δ 10.15 (s, 1H), 8.06 (td, *J* = 9.0, 6.7 Hz, 1H), 7.14 (ddd, *J* = 12.1, 9.1, 2.7 Hz, 1H), 7.07 - 6.98 (m, 3H), 6.95 - 6.90 (m, 2H), 6.17 (d, *J* = 14.4 Hz, 1H), 5.53 (d, *J* = 14.4 Hz, 1H), 4.68 (q, *J* = 8.9 Hz, 2H), 3.65 (t, *J* = 6.6 Hz, 2H), 1.93 (dd, *J* = 9.5, 1.7 Hz, 3H), 1.81 - 1.75 (m, 3H). ¹⁹F NMR (376 MHz, DMSO-*d6*) δ -72.59 (m, 3F), -101.67 (m, 1F), -108.07 (m, 2F), -110.19 (m, 1F).

### Preparation of compound 3A-P2

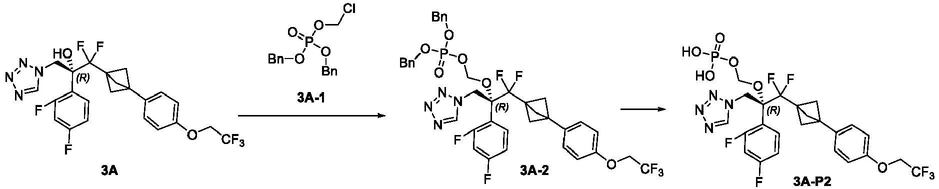

### Preparation of compound 3A-2

Compound **3A** (100 mg, 0.19 mmol) was dissolved in DMF (5.0 mL), then cesium carbonate (190 mg, 0.58 mmol) was added thereto with stirring. After the reaction was carried out for 15 min, the reaction mixture was then added with compound **3A-1** (127 mg, 0.39 mmol), stirred and reacted at 50°C for 12 hours. The reaction mixture was filtered, and the crude filtrate was purified by preparative separation (preparation method: chromatographic column: Welch Xtimate@ C18 21.2 × 250 mm; column temperature: 25°C; mobile phase: water (10 mM/L NH₄HCO₃)-acetonitrile; proportion of acetonitrile in the mobile phase: 25% to 45% in 12 min; flow rate: 30 mL/min) to obtain the title compound **3A-2** (80 mg, yield: 51%). LC-MS (ESI): m/z 807.3 [M+H]⁺.

### Preparation of compound 3A-P2

Compound **3A-2** (70 mg, 0.09 mmol) was dissolved in methanol (5.0 mL), then wet palladium on carbon (10%, 10 mg) was added thereto. The system was replaced with nitrogen three times, and then replaced with hydrogen two times, and hydrogenation was carried out at room temperature for 3 hours. The reaction mixture was filtered, and the crude filtrate was purified by preparative separation (preparation method: chromatographic column: Welch Xtimate@ C18 21.2 × 250 mm; column temperature: 25°C; mobile phase: water (10 mM/L NH₄HCO₃)-acetonitrile; proportion of acetonitrile in the mobile phase: 25% to 45% in 12 min; flow rate: 30 mL/min) to obtain the title compound **3A-P2** (50 mg, yield: 92%).

Compound **3A-P2:** LC-MS (ESI): m/z 625.0 [M-H]⁻. ¹H NMR (400 MHz, DMSO-d6) δ 9.80 (s, 1H), 7.80 (td, *J* = 9.0, 6.6 Hz, 1H), 7.18 (ddd, *J* = 12.1, 9.0, 2.7 Hz, 1H), 7.12 - 7.05 (m, 2H), 7.00 (td, *J* = 8.5, 8.0, 2.7 Hz, 1H), 6.96 - 6.89 (m, 2H), 5.65 - 5.47 (m, 3H), 5.37 - 5.27 (m, 1H), 4.67 (q, *J* = 8.9 Hz, 2H), 1.97 (dd, *J* = 9.4, 1.6 Hz, 3H), 1.84 (dd, *J =* 9.5, 1.6 Hz, 3H).

### Example 4: Preparation of compounds 4A and 4B

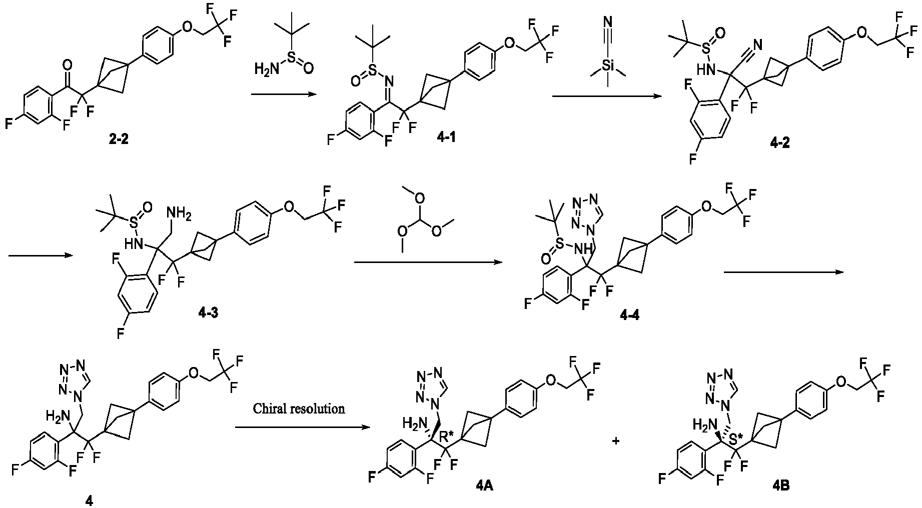

### Preparation of compound 4-1

Compound **2-2** (362 mg, 0.84 mmol) was dissolved in THF (2 mL), then 2-methylpropane-2-sulfinamide (304 mg, 2.5 mmol) and tetraethyl titanate (573 mg, 2.5 mmol) were added thereto, and the reaction mixture was reacted at 80°C for 16 hours in a sealed tube. The reaction was cooled to room temperature and then quenched by adding distilled water (0.2 mL), and the solvent was evaporated to dryness by rotary evaporation. The residue was dissolved by adding DCM (10 mL), filtered, and the filtrate was concentrated to obtain a crude product. The crude product was purified by normal-phase silica gel column chromatography (EtOAc/PE = 0 to 15%) to obtain the title compound **4-1** (336 mg) as a light yellow oil with a yield of 75%. LC-MS (ESI): m/z 536.2 [M+H]⁺.

### Preparation of compound 4-2

Compound **4-1** (316 mg, 0.59 mmol) was dissolved in THF (2 mL), then cesium fluoride (134 mg, 0.88 mmol) and TMSCN (88 mg, 0.88 mmol) were added thereto, and the reaction mixture was reacted at room temperature for 16 hours. The reaction mixture was extracted with EtOAc, and the organic phases were combined, dried, and concentrated to obtain a crude product. The crude product was purified by normal-phase silica gel column chromatography (EtOAc/PE = 0 to 100%) to obtain the title compound **4-2** (330 mg) with a yield of 99%. LC-MS (ESI): m/z 563.0 [M+H]⁺.

### Preparation of compound 4-3

Compound **4-2** (310 mg, 0.55 mmol) was dissolved in MeOH (5 mL). The reaction system was cooled to 0°C and the reaction mixture was added with NiCl₂ (141 mg, 1.1 mmol), and then added with sodium borohydride (314 mg, 8.3 mmol) in batches, and the reaction mixture was stirred and reacted at 0°C for 3 hours. The reaction mixture was added with distilled water (100 mL), extracted with EtOAc (100 mL × 3), and the organic phase was filtered through diatomite, and concentrated to obtain a crude product. The crude product was purified by normal-phase silica gel column chromatography (MeOH/DCM = 0 to 5%) to obtain the title compound **4-3** (159 mg) as a solid with a yield of 51%. LC-MS (ESI): m/z 567.0 [M+H]⁺.

### Preparation of compound 4-4

Compound **4-3** (159 mg, 0.28 mmol) was dissolved in AcOH (5 mL) solution in a microwave tube, then trimethyl orthoformate (149 mg, 1.4 mmol), sodium acetate (23 mg, 0.28 mmol), and TMSN₃ (323 mg, 2.81 mmol) were added thereto. The microwave tube was sealed, and the reaction mixture was reacted at 75°C for 16 hours. The reaction mixture was cooled and then extracted with EtOAc (100 mL × 3), and the organic phases were combined, dried, and concentrated to obtain a crude product. The crude product was purified by normal-phase silica gel column chromatography (EtOAc/PE = 0 to 100%) to obtain the title compound **4-4** (180 mg) with a yield of 99%. LC-MS (ESI): m/z 618.2 [M-H]⁻.

### Preparation of compound 4

Compound **4-4** (180 mg, 0.29 mmol) was dissolved in methanol (3 mL), stirred at 0°C, then a solution of hydrochloric acid in dioxane (4 M, 2 mL) was added dropwise thereto, and the reaction mixture was stirred and reacted at 0°C for 4 hours. The reaction system was added dropwise with saturated NaHCO₃ aqueous solution (5 mL) to quench the reaction, extracted with EtOAc, and the organic phase solvent was concentrated. The residue was filtered by adding methanol, and the crude filtrate was purified by preparative separation (preparation method: chromatographic column: Agilent 10 Prep-C18 250 × 21.2 mm; column temperature: 25°C; mobile phase: water (0.1% FA)-acetonitrile; proportion of acetonitrile in the mobile phase: 55% to 75% in 12 min; flow rate: 30 mL/min) to obtain the title product **4** (50 mg, yield: 33%). LC-MS (ESI): m/z 516.2 [M+H]⁺.

### Preparation of compounds 4A and 4B

Compound **4** (75 mg) was subjected to SFC chiral preparative resolution (preparative separation method, instrument model: MG II preparative SFC (SFC-1); model of chromatographic column: Cellulose-2, 250 × 30 mm I.D., 10 µm; mobile phase: A: CO₂ B: ethanol; elution gradient: B 40%; flow rate: 70 mL/min; column pressure: 100 bar; column temperature: 38°C; detection wavelength: 220 nm; cycle: about 8 min) to obtain the title compounds **4A** (23.3 mg) and **4B** (17.2 mg).

Compound **4A:** LC-MS (ESI): m/z 516.2 [M+H]⁺. Chiral analysis method (model of chromatographic column: Cellulose-2 150 × 4.6 mm I.D., 3 µm; mobile phase: A: CO₂ B: ethanol (0.05% DEA); elution gradient: the mobile phase increased from 5% B to 40% B within 5 min and maintained at 40% B for 2.5 min for elution, then 5% B was equilibrated for 2.5 min; flow rate: 2.5 mL/min; column temperature: 35°C; column pressure: 1500 psi; detection wavelength: 220 nm; R_{T} = 6.026 min). ¹H NMR (400 MHz, CDCl₃) δ 8.51 (s, 1H), 7.69 (td, *J* = 9.3, 6.3 Hz, 1H), 7.14 - 6.96 (m, 2H), 6.96 - 6.76 (m, 4H), 5.47 (d, *J* = 14.4 Hz, 1H), 4.93 (d, *J* = 14.4 Hz, 1H), 4.30 (q, *J* = 8.1 Hz, 2H), 2.03 (dd, *J* = 9.4, 1.8 Hz, 3H), 1.83 (dd, *J* = 9.5, 1.8 Hz, 3H).

Compound **4B:** LC-MS (ESI): m/z 516.2 [M+H]⁺. Chiral analysis method (model of chromatographic column: Cellulose-2 150 × 4.6 mm I.D., 3 µm; mobile phase: A: CO₂ B: ethanol (0.05% DEA); elution gradient: the mobile phase increased from 5% B to 40% B within 5 min and maintained at 40% B for 2.5 min for elution, then 5% B was equilibrated for 2.5 min; flow rate: 2.5 mL/min; column temperature: 35°C; column pressure: 1500 psi; detection wavelength: 220 nm; R_{T} = 4.285 min). ¹H NMR (400 MHz, CDCl₃) δ 8.50 (s, 1H), 7.69 (td, *J* = 9.3, 8.9, 6.3 Hz, 1H), 7.09 - 6.97 (m, 2H), 6.93 - 6.78 (m, 4H), 5.47 (d, *J* = 14.4 Hz, 1H), 4.92 (d, *J* = 14.4 Hz, 1H), 4.30 (q, *J* = 8.1 Hz, 2H), 2.03 (dd, *J* = 9.5, 1.8 Hz, 3H), 1.82 (dd, *J* = 9.5, 1.8 Hz, 3H).

### Example 5: Preparation of compound 5

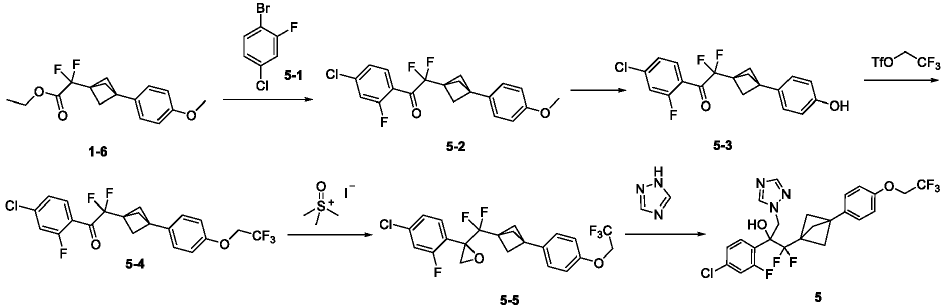

### Preparation of compound 5-2

To a three-necked flask was added compound **5-1** (3.1 g, 14.8 mmol), and the system was replaced with nitrogen three times. To the three-necked flask was added anhydrous ether (20 mL), and n-butyllithium (1.6 M, 9.3 mL, 14.8 mmol) was added dropwise thereto at a cooling temperature of -78°C. After the reaction system was stirred and reacted at -78°C for 45 min, a solution of compound **1-6** (4.0 g, 13.5 mmol) dissolved in anhydrous ether (20 mL) was added dropwise thereto, and the reaction mixture was stirred and reacted for another 1 hour. After the reaction was completed, the reaction system was quenched with saturated ammonium chloride solution (5 mL), extracted with EtOAc (20 mL × 3), and the organic phases were combined, dried, and concentrated to obtain a crude product. The crude product was purified by normal-phase silica gel column chromatography (EtOAc/PE = 0 to 5%) to obtain the title compound **5-2** (2.9 g) as a yellow solid with a yield of 56%. ¹H NMR (400 MHz, CDCl₃) δ 7.77 - 7.73 (m, 1H), 7.28 - 7.25 (m, 1H), 7.24 - 7.21 (m, 1H), 7.14 - 7.11 (m, 2H), 6.86 - 6.83 (m, 2H), 3.79 (s, 3H), 2.21 (s, 6H).

### Preparation of compound 5-3

To a sealed tube was added compound **5-2** (2.9 g, 7.62 mmol), then acetic acid (15 mL) and hydrogen bromide aqueous solution (48 wt. % in H₂O, 15 mL) were added thereto, and the reaction mixture was reacted at 95°C for 16 hours in the sealed tube. The reaction mixture was cooled, then evaporated to dryness by rotary evaporation, extracted with EtOAc (30 mL × 3), and the organic phases were combined, dried, and concentrated to obtain a crude product. The crude product was purified by normal-phase silica gel column chromatography (EtOAc/PE = 0 to 30%) to obtain the title compound **5-3** (1.4 g) as a brownish-yellow solid with a yield of 50%. ¹H NMR (400 MHz, DMSO-*d6*) δ 9.36 (s, 1H), 7.82 (t, *J* = 8.1 Hz, 1H), 7.75 (dd, *J* = 10.8, 2.0 Hz, 1H), 7.57 - 7.50 (m, 1H), 7.09 - 6.99 (m, 2H), 6.74 - 6.62 (m, 2H), 2.11 (s, 6H).

### Preparation of compound 5-4

Compound **5-3** (1.0 g, 2.7 mmol) was dissolved in DMF (5 mL) solvent, then potassium carbonate (376 mg, 4.1 mmol) and 2,2,2-trifluoroethyl trifluoromethanesulfonate (949 mg, 4.1 mmol) were added thereto, and the reaction mixture was reacted at 65°C for 16 hours in a sealed tube. The reaction mixture was cooled and then extracted with EtOAc, and the organic phases were combined, dried, and concentrated to obtain a crude product. The crude product was purified by normal-phase silica gel column chromatography (EtOAc/PE = 0 to 5%) to obtain the title compound **5-4** (350 mg) as a light yellow oil with a yield of 28%. ¹H NMR (400 MHz, CDCl₃) δ 7.78 (t, *J* = 8.0 Hz, 1H), 7.30 - 7.28 (m, 1H), 7.27 - 7.24 (m, 1H), 7.19 - 7.13 (m, 2H), 6.94 - 6.88 (m, 2H), 4.36 (q, *J* = 8.0 Hz, 2H), 2.25 (s, 6H).

### Preparation of compound 5-5

Compound **5-4** (450 mg, 1.0 mmol) was dissolved in a mixed solvent of dichloromethane (3 mL) and water (3 mL), then trimethylsulfoxonium iodide (441 mg, 2.0 mmol) and sodium hydroxide (80 mg, 2.0 mmol) were added thereto, and the reaction system was refluxed for 16 hours. After cooling to room temperature, the reaction system was extracted with DCM, and the organic phases were combined, dried, and concentrated to obtain a crude product. The crude product was purified by normal-phase silica gel column chromatography (EtOAc/PE = 0 to 5%) to obtain the title compound **5-5** (450 mg) as a light yellow oil with a yield of 97%. TLC analysis (EtOAc: PE = 1:20) : Rf =0.2.

### Preparation of compound 5

1,2,4-Triazole (149 mg, 2.1 mmol) was dissolved in DMF (2 mL), and NaH (86 mg, 60%, 2.1 mmol) was added thereto at 0°C. After the reaction mixture was reacted for 30 min, compound **5-5** (200 mg, 0.43 mmol) was added thereto, and the reaction system was reacted at 80°C for 16 hours in a sealed tube. After the reaction system was cooled, the reaction mixture was filtered, and the crude filtrate was purified by preparative separation (chromatographic column: Agilent 10 Prep-C18 250 × 21.2 mm; column temperature: 25°C; mobile phase: water (0.1% FA)-acetonitrile; proportion of acetonitrile in the mobile phase: 50% to 70% in 12 min; flow rate: 30 mL/min) to obtain the title compound **5** (130 mg, yield: 56%). LC-MS (ESI): m/z 532.2 [M+H]⁺. ¹H NMR (400 MHz, DMSO-*d6*) δ 8.37 (s, 1H), 7.70 (s, 1H), 7.54 (t, *J* = 8.8 Hz, 1H), 7.40 (dd, *J* = 11.6, 2.0 Hz, 1H), 7.19 (dd, *J* = 8.8, 2.4 Hz, 1H), 7.12 - 7.05 (m, 2H), 6.95 (m, 3H), 5.14 (d, *J* = 14.4 Hz, 1H), 4.87 - 4.47 (m, 3H), 1.98 (m, 3H), 1.77 (m, 3H). ¹⁹F NMR (376 MHz, DMSO-d6) δ -72.56 (t, 3F), -104.23 (d, 1F), -109.32 (dd, 2F).

### Example 6: Preparation of compounds 6A and 6B

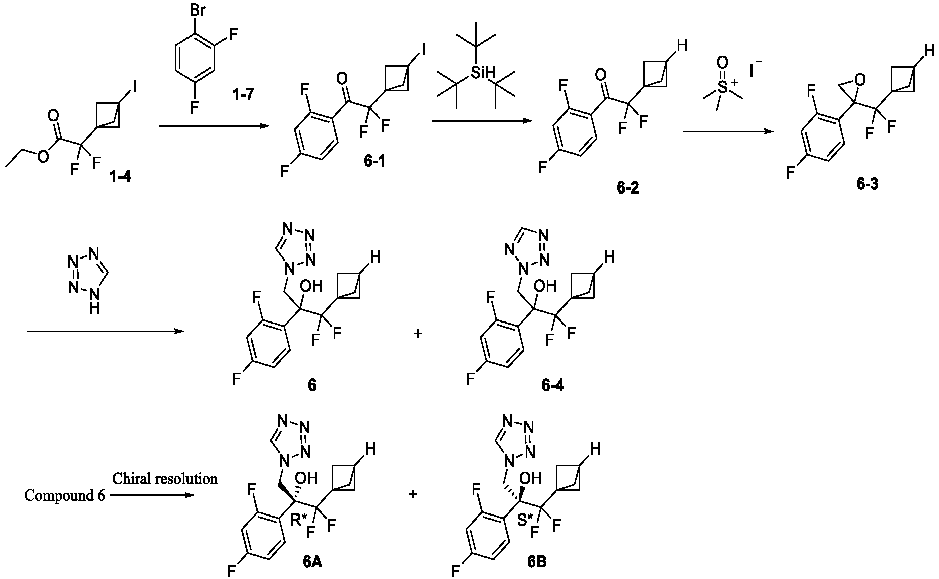

### Preparation of compound 6-1

To a three-necked flask was added compound **1-7** (0.732 g, 3.8 mmol), and the system was replaced with nitrogen three times. To the three-necked flask was added anhydrous ether (10 mL), and n-butyllithium (2.5 M, 1.52 mL, 3.8 mmol) was added dropwise thereto at a cooling temperature of -78°C. After the reaction system was stirred and reacted at -78°C for 45 min, a solution of compound **1-4** (1.0 g, 3.16 mmol) dissolved in anhydrous ether (10 mL) was added dropwise thereto, and the reaction mixture was stirred and reacted for another 1 hour. After the reaction was completed, the reaction system was quenched with saturated ammonium chloride solution (5 mL), extracted with EtOAc (20 mL × 3), and the organic phases were combined, dried, and concentrated to obtain a crude product. The crude product was purified by normal-phase silica gel column chromatography (EtOAc/PE = 0 to 5%) to obtain the title compound **6-1** (0.8 g) as a colorless oil with a yield of 66%. ¹H NMR (400 MHz, CDCl₃) δ 7.83 - 7.79 (m, 1H), 7.02 - 6.97 (m, 1H), 6.95 - 6.90 (m, 1H), 2.51 (s, 6H).

### Preparation of compound 6-2

To a three-necked flask was added compound **6-1** (0.8 g, 2.08 mmol), and the system was replaced with nitrogen three times. To the three-necked flask was added anhydrous methanol (10 mL), and tri-tert-butylsilane (0.543 g, 2.71 mmol) was added dropwise thereto at a cooling temperature of 25°C. After the reaction system was stirred and reacted at 25°C for 45 min and the end of the reaction was detected by LC-MS, the reaction system was added with 2 g of silica gel, concentrated, and purified by normal-phase silica gel column chromatography (EtOAc/PE = 0 to 5%) to obtain the title compound **6-2** (0.4 g) as a colorless oil with a yield of 74%. ¹H NMR (400 MHz, CDCl₃) δ 7.84 - 7.80 (m, 1H), 7.00 - 6.97 (m, 1H), 6.94 - 6.89 (m, 1H), 2.57 (s, 1H), 2.00 (s, 6H).

### Preparation of compound 6-3

Compound **6-2** (400 mg, 1.55 mmol) was dissolved in a mixture of dichloromethane (5.0 mL) and water (2.0 mL), then trimethylsulfoxonium iodide (1.36 g, 6.20 mmol) and solid sodium hydroxide (372 mg, 9.29 mmol) were added thereto, and the reaction system was stirred at 65°C for 48 hours. After the reaction was completed, the reaction system was extracted with dichloromethane (20 mL × 3), and the organic phases were combined, dried, and concentrated to obtain a crude product. The crude product was purified by normal-phase silica gel column chromatography (EtOAc/PE = 0 to 5%) to obtain the title compound **6-3** (400 mg) as a colorless oil with a yield of 95%. ¹H NMR (400 MHz, CDCl₃) δ 7.56 - 7.52 (m, 1H), 6.93 - 6.90 (m, 1H), 6.85 - 6.79 (m, 1H), 3.35 (d, *J* = 5.6 Hz, 1H), 2.92 - 2.90 (m, 1H), 2.45 (s, 1H), 1.88 - 1.82 (m, 6H).

### Preparation of compound 6

Compound **6-3** (400 mg, 1.47 mmol) was dissolved in DMF solution (1.5 mL), and compound 1H-tetrazole (514 mg, 7.35 mmol) and potassium carbonate (1.02 g, 7.35 mmol) were added thereto. The reaction system was stirred for 2 hours at 80°C in a sealed tube. After the reaction system was cooled, the reaction mixture was filtered, and the crude filtrate was purified by preparative separation (preparation method: mobile phase: A: 0.1% formic acid aqueous solution; B: acetonitrile; chromatographic column: Agilent 10 Prep-C18 250 × 21.2 mm; column temperature: 25°C; gradient: 50% to 70% acetonitrile in 12 min; flow rate: 30 mL/min) to obtain the title compound **6** (170 mg, yield: 34%) and the corresponding regioisomer compound **6-4** (75 mg).

Compound **6:** LC-MS (ESI): m/z 343.2 [M+H]⁺. ¹H NMR (400 MHz, DMSO-d6) δ 9.11 (s, 1H), 7.53 - 7.46 (m, 1H), 7.30 - 7.24 (m, 1H), 7.07 (s, 1H) 7.02 - 7.00 (m, 1H), 5.38 (d, *J* = 14.4 Hz, 1H), 4.96 (d, *J* = 14.4 Hz, 1H), 2.34 (s, 1H), 1.78 - 1.75 (m, 3H), 1.57 - 1.54 (m, 3H). ¹⁹F NMR (376 MHz, DMSO-d6) δ -103.14 (m, 1F), -109.63 (d, 1F), -109.72 (m, 2F).

Regioisomer compound **6-4:** LC-MS (ESI): m/z 343.2 [M+H]⁺. ¹H NMR (400 MHz, DMSO-*d6*) δ 8.75 (s, 1H), 7.55 - 7.49 (m, 1H), 7.28 - 7.22 (m, 1H), 7.11 (s, 1H), 7.02 - 6.97 (m, 1H), 5.56 (d, *J* = 14.0 Hz, 1H), 5.19 (d, *J* = 14.0 Hz, 1H), 2.33 (s, 1H), 1.77 - 1.74 (m, 3H), 1.56 - 1.53 (m, 3H). ¹⁹F NMR (376 MHz, DMSO-d6) δ -102.72 (m, 1F), -109.43 (m, 2F), - 110.09 (d, 1F).

### Preparation of compounds 6A and 6B

Compound **6** (170 mg) was subjected to SFC chiral preparative resolution (preparative separation method, instrument model: MG II preparative SFC (SFC-1); model of chromatographic column: Cellulose-2, 250 × 30 mm I.D., 10 µm; mobile phase: A: CO₂ B: ethanol; elution gradient: B 40%; flow rate: 70 mL/min; column pressure: 100 bar; column temperature: 38°C; detection wavelength: 220 nm; cycle: about 8 min) to obtain the title compounds **6A** (69.6 mg, single enantiomer) and **6B** (76 mg, single enantiomer).

Compound **6A:** LC-MS (ESI): m/z 343.2 [M+H]⁺. Chiral analysis method (model of chromatographic column: Cellulose-2 150 × 4.6 mm I.D., 3 µm; mobile phase: A: CO₂ B: ethanol (0.05% DEA); elution gradient: the mobile phase increased from 5% B to 40% B within 5 min and maintained at 40% B for 2.5 min for elution, then 5% B was equilibrated for 2.5 min; flow rate: 2.5 mL/min; column temperature: 35°C; column pressure: 1500 psi; detection wavelength: 220 nm; R_{T} = 6.026 min). ¹H NMR (400 MHz, DMSO-*d6*) δ 9.12 (s, 1H), 7.53 - 7.47 (m, 1H), 7.30 - 7.24 (m, 1H), 7.08 (s, 1H), 7.03 - 6.98 (m, 1H), 5.38 (d, *J* = 14.5 Hz, 1H), 4.96 (d, *J* = 14.6 Hz, 1H), 2.35 (s, 1H), 1.79 - 1.76 (m, 3H), 1.57 - 1.55 (m, 3H). ¹⁹F NMR (376 MHz, Chloroform-*d*) δ -103.03 - -103.25 (m, 1F), -109.61 - -109.63 (m, 1F), -109.72 - - 109.75 (m, 2F).

Compound **6B:** LC-MS (ESI): m/z 343.2 [M+H]⁺. Chiral analysis method (model of chromatographic column: Cellulose-2 150 × 4.6 mm I.D., 3 µm; mobile phase: A: CO₂ B: ethanol (0.05% DEA); elution gradient: the mobile phase increased from 5% B to 40% B within 5 min and maintained at 40% B for 2.5 min for elution, then 5% B was equilibrated for 2.5 min; flow rate: 2.5 mL/min; column temperature: 35°C; column pressure: 1500 psi; detection wavelength: 220 nm; R_{T} = 6.026 min). ¹H NMR (400 MHz, DMSO-*d6*) δ 9.12 (s, 1H), 7.53 - 7.47 (m, 1H), 7.31 - 7.24 (m, 1H), 7.08 (s, 1H), 7.03 - 6.98 (m, 1H), 5.38 (d, *J* = 14.5 Hz, 1H), 4.96 (d, *J* = 14.6 Hz, 1H), 2.35 (s, 1H), 1.79 - 1.76 (m, 3H), 1.57 - 1.55 (m, 3H). ¹⁹F NMR (376 MHz, Chloroform-*d*) δ -103.04 - -103.25 (m, 1F), -109.61 - -109.63 (m, 1F), -109.72 - - 109.75 (m, 2F).

### Example 7: Preparation of compounds 7A and 7B

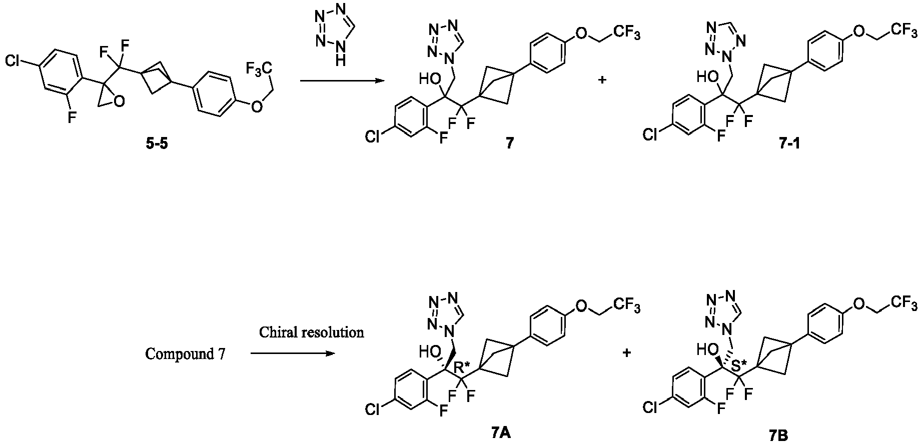

### Preparation of compound 7

Compound **5-5** (250 mg, 0.54 mmol) was dissolved in DMF (2.5 mL), then 1H-tetrazole (189 mg, 2.7 mmol) and potassium carbonate (373 mg, 2.7 mmol) were added thereto, and the reaction mixture was reacted at 80°C for 16 hours in a sealed tube. After the reaction system was cooled, the reaction mixture was filtered, and the crude filtrate was purified by preparative separation (preparation method: chromatographic column: Agilent 10 Prep-C18 250 × 21.2 mm; mobile phase: A: 0.1% formic acid aqueous solution; B: acetonitrile; column temperature: 25°C; gradient: 60% to 80% acetonitrile in 12 min; flow rate: 30 mL/min) to obtain the title compound **7** (120 mg, yield: 34%) and the corresponding regioisomer compound **7-1** (56 mg, yield: 20%).

Compound **7:** LC-MS (ESI): m/z 532.8 [M+H]⁺. ¹H NMR (400 MHz, DMSO-*d6*) δ 9.15 (s, 1H), 7.75 - 7.33 (m, 2H), 7.30 - 7.18 (m, 2H), 7.16 - 7.01 (m, 2H), 7.01 - 6.81 (m, 2H), 5.44 (d, *J* = 14.6 Hz, 1H), 5.02 (d, *J* = 14.6 Hz, 1H), 4.70 (q, *J* = 8.9 Hz, 2H), 2.00 (dd, *J* = 9.4, 1.7 Hz, 3H), 1.79 (dd, *J* = 9.4, 1.7 Hz, 3H). ¹⁹F NMR (376 MHz, DMSO-*d6*) δ -72.56 (m, 3F), -104.65 (m, 1F), -109.14 (d, 2F).

Regioisomer compound **7-1:** LC-MS (ESI): m/z 533.20 [M+H]⁺. ¹H NMR (400 MHz, DMSO-*d6*) δ 8.71 (s, 1H), 7.48 (t, *J* = 8.6 Hz, 1H), 7.40 (dd, *J* = 11.9, 2.2 Hz, 1H), 7.20 (s, 1H), 7.15 (dd, *J* = 8.6, 2.2 Hz, 1H), 7.08 - 6.96 (m, 2H), 6.94 - 6.78 (m, 2H), 5.54 (d, *J =* 14.2 Hz, 1H), 5.18 (d, *J* = 14.2 Hz, 1H), 4.63 (q, *J* = 8.8 Hz, 2H), 1.92 (dd, *J* = 9.4, 1.7 Hz, 3H), 1.71 (dd, *J* = 9.4, 1.7 Hz, 3H). ¹⁹F NMR (376 MHz, DMSO-*d6*) δ -72.57 (s, 3F), -104.28 (dd, 1F), -108.88 (m, 2F).

### Preparation of compounds 7A and 7B

Compound **7** (110 mg) was subjected to SFC chiral preparative resolution (preparative separation method, instrument model: MG II preparative SFC(SFC-14); model of chromatographic column: ChiralCel OX, 250 × 30 mm I.D., 5 µm; mobile phase: A: CO₂ B: ethanol; elution gradient: B 15%; flow rate: 60 mL/min; column pressure: 100 bar; column temperature: 38°C; detection wavelength: 220 nm; cycle: about 16 min) to obtain the title compounds **7A** (54 mg, single enantiomer) and **7B** (51 mg, single enantiomer).

Compound **7A:** LC-MS (ESI): m/z 533.0 [M+H]⁺. Chiral analysis method (model of chromatographic column: Chiralcel OX-3 100 × 4.6 mm I.D., 3 µm; mobile phase: A: CO₂ B: ethanol (0.05% DEA); elution gradient: the mobile phase increased from 5% B to 40% B within 4 min and maintained at 40% B for 2.5 min for elution, then 5% B was equilibrated for 1.5 min; flow rate: 2.8 mL/min; column temperature: 35°C; column pressure: 1500 psi; detection wavelength: 220 nm; Rt = 2.072 min). ¹H NMR (400 MHz, DMSO-*d6*) δ 9.15 (s, 1H), 7.58 - 7.39 (m, 2H), 7.27 - 7.18 (m, 2H), 7.14 - 7.07 (m, 2H), 7.02 - 6.89 (m, 2H), 5.43 (d, *J* = 14.4 Hz, 1H), 5.02 (d, *J* = 14.4 Hz, 1H), 4.70 (q, *J* = 8.9 Hz, 2H), 2.06 - 1.90 (m, 3H), 1.84 -1.70 (m, 3H). ¹⁹F NMR (376 MHz, DMSO-*d6*) δ -72.57 (s, 3F), -103.06 - -105.12 (m, 1F), -107.63 - -112.35 (m, 2F).

Compound **7B:** LC-MS (ESI): m/z 533.0 [M+H]⁺. Chiral analysis method (model of chromatographic column: Chiralcel OX-3 100 × 4.6 mm I.D., 3 µm; mobile phase: A: CO₂ B: ethanol (0.05% DEA); elution gradient: the mobile phase increased from 5% B to 40% B within 4 min and maintained at 40% B for 2.5 min for elution, then 5% B was equilibrated for 1.5 min; flow rate: 2.8 mL/min; column temperature: 35°C; column pressure: 1500 psi; detection wavelength: 220 nm; Rt = 2.445 min). ¹H NMR (400 MHz, DMSO-d6) δ 9.15 (s, 1H), 7.58 - 7.39 (m, 2H), 7.27 - 7.18 (m, 2H), 7.14 - 7.07 (m, 2H), 7.02 - 6.89 (m, 2H), 5.43 (d, *J* = 14.4 Hz, 1H), 5.02 (d, *J =* 14.4 Hz, 1H), 4.70 (q, *J* = 8.9 Hz, 2H), 2.06 - 1.90 (m, 3H), 1.84 - 1.70 (m, 3H). ¹⁹F NMR (376 MHz, DMSO-*d6*) δ -72.57 (s, 3F), -103.06 - -105.12 (m, 1F), -107.63 - -112.35 (m, 2F).

### Example 8: Preparation of compounds 8A and 8B

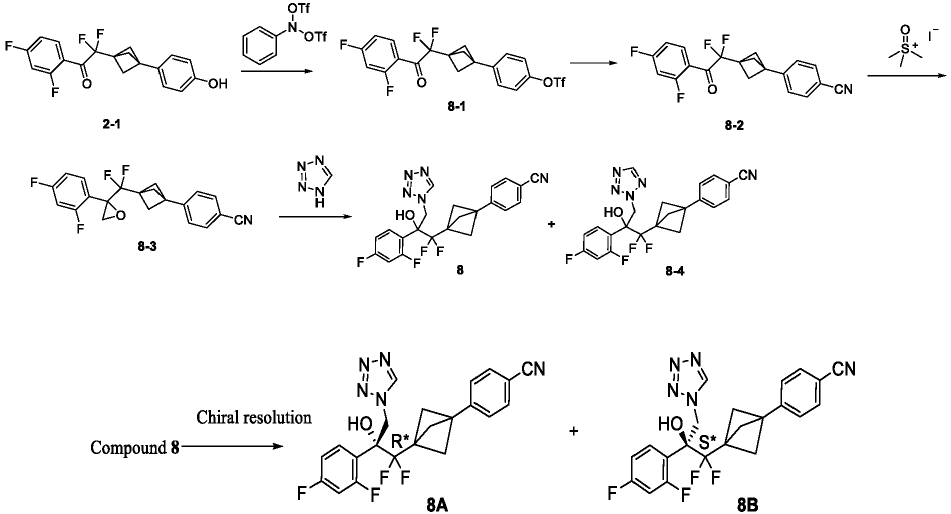

### Preparation of compound 8-1

To a 100 mL three-necked flask was added compound **2-1** (1.0 g, 2.85 mmol), then DMF (10 mL), N-phenyl-bis(trifluoromethanesulfonimide) (1.33 g, 3.43 mmol), and TEA (866 mg, 8.56 mmol) were added thereto respectively, and the reaction system was stirred and reacted at room temperature for 4 hours under nitrogen atmosphere. The reaction mixture was added with water (20 mL) and extracted with EtOAc (10 mL × 3), and the organic phases were combined, dried, and concentrated to obtain a crude product. The crude product was purified by normal-phase silica gel column chromatography (EtOAc/PE = 0 to 5%) to obtain the title compound **8-1** (900 mg) as a brown solid with a yield of 65%. TLC analysis (EtOAc: PE = 1:20) : Rf =0.2.

### Preparation of compound 8-2

To a microwave tube was added compound **8-1** (300 mg, 0.62 mmol), then DMF (3 mL), zinc cyanide (87 mg, 0.75 mmol), and tetrakis(triphenylphosphine)palladium (36 mg, 0.03 mmol) were added thereto, and the reaction system was reacted at 80°C under microwave irradiation for 40 hours under nitrogen atmosphere. The reaction mixture was cooled, then added with water (10 mL), and extracted with EtOAc (10 mL × 3). The organic phases were combined, dried, and concentrated to obtain a crude product. The crude product was purified by normal-phase silica gel column chromatography (EtOAc/PE = 0 to 5%) to obtain the title compound **8-2** (110 mg) as a yellow solid with a yield of 49%. ¹H NMR (400 MHz, Chloroform-*d*) δ 7.92 - 7.82 (m, 1H), 7.68 - 7.54 (m, 2H), 7.35 - 7.27 (m, 2H), 7.05 - 6.96 (m, 1H), 6.99 - 6.88 (m, 1H), 2.30 (s, 6H).

### Preparation of compound 8-3

Compound **8-2** (110 mg, 0.30 mmol) was dissolved in a mixed solvent of dichloromethane (3 mL) and water (3 mL), then trimethylsulfoxonium iodide (202 mg, 0.91 mmol) and sodium hydroxide (37 mg, 0.91 mmol) were added thereto, and the reaction system was refluxed for 16 hours. After cooling to room temperature, the reaction system was extracted with DCM (10 mL × 3), and the organic phases were combined, dried, and concentrated to obtain a crude product. The crude product was purified by normal-phase silica gel column chromatography (EtOAc/PE = 0 to 5%) to obtain the title compound **8-3** (110 mg, yield: 96%) as a light yellow oil. LC-MS (ESI): m/z 374.2 [M+H]⁺.

### Preparation of compound 8

Compound **8-3** (110 mg, 0.29 mmol) was dissolved in DMF (2.5 mL), then 1H-tetrazole (103 mg, 1.47 mmol) and potassium carbonate (203 mg, 1.47 mmol) were added thereto, and the reaction mixture was reacted at 80°C for 16 hours in a sealed tube. After the reaction system was cooled, the reaction mixture was filtered, and the crude filtrate was purified by preparative separation (preparation method: chromatographic column: Agilent 10 Prep-C18 250 × 21.2 mm; mobile phase: A: 0.1% formic acid aqueous solution; B: acetonitrile; column temperature: 25°C; gradient: 60% to 80% acetonitrile in 12 min; flow rate: 30 mL/min) to obtain the title compound **8** (40 mg, yield: 30%) and the corresponding regioisomer compound **8-4** (10 mg, yield: 7%).

Compound **8:** LC-MS (ESI): m/z 444.2 [M+H]⁺. ¹H NMR (400 MHz, DMSO-d6) δ 9.16 (s, 1H), 7.75 (d, *J* = 7.8 Hz, 2H), 7.43 - 7.14 (m, 6H), 5.45 (d, *J* = 14.4 Hz, 1H), 5.03 (d, *J* = 14.4 Hz, 1H), 2.10 (d, *J* = 9.4 Hz, 3H), 1.90 (d, J = 9.4 Hz, 3H). ¹⁹F NMR (376 MHz, DMSO-*d6*) δ -108.33 - -109.44 (m, 2F), -112.88 - -113.99 (m, 1F), -118.19 (d, 1F).

Compound **8-4:** LC-MS (ESI): m/z 444.2 [M+H]⁺. ¹H NMR (400 MHz, DMSO-^6) δ 8.78 (s, 1H), 7.75 (d, *J* = 7.9 Hz, 2H), 7.45 - 7.18 (m, 6H), 5.63 (d, *J* = 14.2 Hz, 1H), 5.26 (d, *J* = 14.2 Hz, 1H), 2.08 (d, *J* = 9.4 Hz, 3H), 1.88 (d, *J* = 9.4 Hz, 3H). ¹⁹F NMR (376 MHz, DMSO-*d6*) δ -108.78 (m, 2F), -112.97 (m, 1F), -118.53 (d, 1F).

### Preparation of compounds 8A and 8B

Compound 8 (110 mg) was subjected to SFC chiral preparative resolution (preparative separation method, instrument model: MG II preparative SFC(SFC-14); model of chromatographic column: ChiralPak AD, 250 × 30 mm I.D., 10 µm; mobile phase: A: CO₂ B: ethanol(0.1% NH₃H₂O); elution gradient: B 20%; flow rate: 60 mL/min; column pressure: 100 bar; column temperature: 38°C; detection wavelength: 220 nm; cycle: about 4 min) to obtain the title compounds **8A** (48 mg, single enantiomer) and **8B** (48 mg, single enantiomer).

Compound **8A:** LC-MS (ESI): m/z 444.2 [M+H]⁺. Chiral analysis method (model of chromatographic column: AD-3 150 × 4.6 mm I.D., 3 µm; mobile phase: A: CO₂ B: ethanol (0.05% DEA); elution gradient: the mobile phase increased from 5% B to 40% B within 5 min and maintained at 40% B for 2.5 min for elution, then 5% B was equilibrated for 2.5 min; flow rate: 2.5 mL/min; column temperature: 35°C; column pressure: 1500 psi; detection wavelength: 220 nm; Rt = 3.242 min). ¹H NMR (400 MHz, DMSO-*d6*) δ 9.14 (s, 1H), 7.79 - 7.69 (m, 2H), 7.62 - 7.47 (m, 1H), 7.40 - 7.24 (m, 3H), 7.21 (s, 1H), 7.08 - 6.94 (m, 1H), 5.44 (d, *J =* 14.6 Hz, 1H), 5.02 (d, *J* = 14.6 Hz, 1H), 2.19 - 2.02 (m, 3H), 1.92 - 1.76 (m, 3H). ¹⁹F NMR (376 MHz, DMSO-*d6*) δ -102.76 - -103.20 (m, 1F), -109.37 - -109.47 (m, 1F), -109.48 - - 109.61 (m, 2F).

Compound **8B:** LC-MS (ESI): m/z 444.2 [M+H]⁺. Chiral analysis method (model of chromatographic column: AD-3 150 × 4.6 mm I.D., 3 µm; mobile phase: A: CO₂ B: ethanol (0.05% DEA); elution gradient: the mobile phase increased from 5% B to 40% B within 5 min and maintained at 40% B for 2.5 min for elution, then 5% B was equilibrated for 2.5 min; flow rate: 2.5 mL/min; column temperature: 35°C; column pressure: 1500 psi; detection wavelength: 220 nm; Rt = 2.878 min). ¹H NMR (400 MHz, DMSO-d6) δ 9.14 (s, 1H), 7.79 - 7.69 (m, 2H), 7.62 - 7.47 (m, 1H), 7.40 - 7.24 (m, 3H), 7.21 (s, 1H), 7.08 - 6.94 (m, 1H), 5.44 (d, *J =* 14.5 Hz, 1H), 5.02 (d, *J* = 14.6 Hz, 1H), 2.19 - 2.02 (m, 3H), 1.92 - 1.76 (m, 3H). ¹⁹F NMR (376 MHz, DMSO-*d6*) δ -102.76 - -103.20 (m, 1F), -109.37 - -109.47 (m, 1F), -109.48 - - 109.61 (m, 2F).

### Example 9: Preparation of compounds 9A and 9B

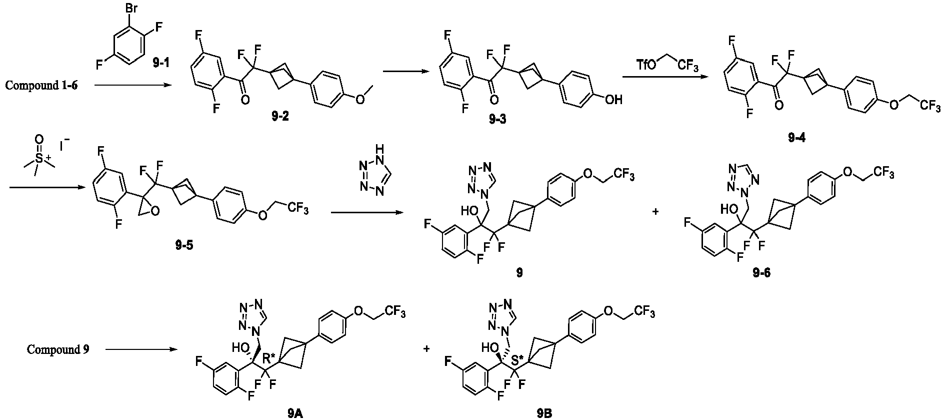

### Preparation of compound 9-2

To a three-necked flask was added compound **9-1** (2.5 g, 13.0 mmol), and the system was replaced with nitrogen three times. To the three-necked flask was added anhydrous ether (20 mL), and n-butyllithium (2.5 M, 5.7 mL, 14.3 mmol) was added dropwise thereto at a cooling temperature of -78°C. After the reaction system was stirred and reacted at -78°C for 45 min, a solution of compound **1-6** (4.2 g, 14.3 mmol) dissolved in anhydrous ether (20 mL) was added dropwise thereto, and the reaction mixture was stirred and reacted for another 1 hour. After the reaction was completed, the reaction system was quenched with saturated ammonium chloride aqueous solution (5 mL), extracted with EtOAc (20 mL × 3), and the organic phases were combined, dried, and concentrated to obtain a crude product. The crude product was purified by normal-phase silica gel column chromatography (EtOAc/PE = 0 to 5%) to obtain the title compound **9-2** (1.9 g) as a yellow solid with a yield of 63%. ¹H NMR (400 MHz, CDCl₃) δ 7.91 - 7.81 (m, 1H), 7.15 - 7.10 (d, *J* = 8.0 Hz, 2H), 7.01 - 6.90 (m, 2H), 6.87 - 6.83 (d, *J* = 8.0 Hz, 2H), 3.79 (s, 3H), 2.21 (s, 6H).

### Preparation of compound 9-3

To a sealed tube was added compound **9-2** (1.9 g, 5.2 mmol), then acetic acid (15 mL) and hydrogen bromide aqueous solution (48 wt. % in H₂O, 15 mL) were added thereto, and the reaction mixture was reacted at 95°C for 16 hours in the sealed tube. The reaction mixture was cooled, then evaporated to dryness by rotary evaporation, extracted with EtOAc (30 mL × 3), and the organic phases were combined, dried, and concentrated to obtain a crude product. The crude product was purified by normal-phase silica gel column chromatography (EtOAc/PE = 0 to 30%) to obtain the title compound **9-3** (1.4 g) as a brownish-yellow solid with a yield of 77%. ¹H NMR (400 MHz, DMSO-*d6*) δ 9.37 (s, 1H), 7.93 - 7.87 (m, 1H), 7.58 - 7.52 (m, 1H), 7.35 - 7.30 (m, 1H), 7.04 (d, *J* = 8.0 Hz, 2H), 7.70 (d, *J* = 8.0 Hz, 2H), 2.11 (s, 6H).

### Preparation of compound 9-4

Compound **9-3** (300 mg, 0.86 mmol) was dissolved in DMF (5 mL) solvent, then potassium carbonate (178 mg, 1.29 mmol) and 2,2,2-trifluoroethyl trifluoromethanesulfonate (299 mg, 1.29 mmol) were added thereto, and the reaction mixture was reacted at 65°C for 16 hours in a sealed tube. The reaction mixture was cooled and then extracted with EtOAc (30 mL × 3), and the organic phases were combined, dried, and concentrated to obtain a crude product. The crude product was purified by normal-phase silica gel column chromatography (EtOAc/PE = 0 to 5%) to obtain the title compound **9-4** (130 mg) as a light yellow oil with a yield of 35%. ¹H NMR (400 MHz, DMSO-*d6*) δ 7.94 - 7.88 (m, 1H), 7.59 - 7.53 (m, 1H), 7.36 - 7.31 (m, 1H), 7.22 (d, *J* = 8.0 Hz, 2H), 7.02 (d, *J* = 8.0 Hz, 2H), 4.77 - 4.70 (q, *J* = 8.0 Hz, 2H), 2.17 (s, 6H).

### Preparation of compound 9-5

Compound **9-4** (130 mg, 0.3 mmol) was dissolved in a mixed solvent of dichloromethane (3 mL) and water (3 mL), then trimethylsulfoxonium iodide (198 mg, 0.9 mmol) and sodium hydroxide (36 mg, 0.9 mmol) were added thereto, and the reaction system was refluxed for 16 hours. After cooling to room temperature, the reaction system was extracted with DCM (30 mL × 3), and the organic phases were combined, dried, and concentrated to obtain a crude product. The crude product was purified by normal-phase silica gel column chromatography (EtOAc/PE = 0 to 5%) to obtain the title compound **9-5** (110 mg) as a light yellow oil with a yield of 82%. TLC analysis (EtOAc: PE = 1:20) : Rf = 0.2. ¹H NMR (400 MHz, DMSO-*d6*) δ 7.70 - 7.65 (m, 1H), 7.38 - 7.33 (m, 1H), 7.19 - 7.17 (m, 1H), 7.17 - 7.14 (d, *J* = 8.0 Hz, 2H), 7.00 (d, *J* = 8.0 Hz, 2H), 4.72 (q, *J* = 8.8 Hz, 2H), 3.39 (d, *J* = 4.0 Hz, 1H), 3.10 (d, *J* = 4.0 Hz, 1H), 2.04 (s, 6H).

### Preparation of compound 9

Compound **9-5** (130 mg, 0.29 mmol) was dissolved in DMF solution (1.5 mL), and compound 1H-tetrazole (102 mg, 1.45 mmol) and potassium carbonate (200 mg, 1.45 mmol) were added thereto. The reaction system was stirred for 2 hours at 80°C in a sealed tube. After the reaction system was cooled, the reaction mixture was filtered, and the crude filtrate was purified by preparative separation (preparation method: mobile phase: A: 0.1% formic acid aqueous solution; B: acetonitrile; chromatographic column: Agilent 10 Prep-C18 250 × 21.2 mm; column temperature: 25°C; gradient: 60% to 80% acetonitrile in 12 min; flow rate: 30 mL/min) to obtain the title compound **9** (36 mg, yield: 24%) and the corresponding regioisomer compound **9-6** (10 mg, yield: 6%).

Compound **9:** LC-MS (ESI): m/z 517.0 [M+H]⁺. ¹H NMR (400 MHz, DMSO-d6) δ 9.13 (s, 1H), 7.57 - 7.51 (m, 1H), 7.32 - 7.26 (m, 1H), 7.16 (s, 1H), 7.09 (d, *J =* 8.0, 2H), 7.03 - 6.98 (m, 1H), 6.96 (d, *J* = 8.0, 2H), 5.45 (d, *J* = 16.0 Hz, 1H), 5.03 (d, *J* = 16.0 Hz, 1H), 4.70 (q, *J* = 8.8 Hz, 2H), 2.00 (d, *J* = 8.0 Hz, 3H), 1.79 (d, *J* = 8.0 Hz, 3H). ¹⁹F NMR (376 MHz, DMSO-d6) δ -72.32 (s, 3F), -103.09 (m, 1F), -109.23 (d, 2F), -109.60 (d, 1F).

Compound **9-6:** LC-MS (ESI): m/z 517.0 [M+H]⁺. ¹H NMR (400 MHz, DMSO-*d6*) δ 8.76 (s, 1H), 7.59 - 7.53 (m, 1H), 7.30 - 7.24 (m, 1H), 7.20 (s, 1H), 7.09 (d, *J* = 8.0, 2H), 7.03 - 6.97 (m, 1H), 6.95 (d, *J* = 8.0, 2H), 5.63 (d, *J* = 16.0 Hz, 1H), 5.26 (d, *J* = 16.0 Hz, 1H), 4.69 (q, *J* = 8.8 Hz, 2H), 1.99 (d, *J* = 8.0 Hz, 3H), 1.77 (d, *J* = 8.0 Hz, 3H). ¹⁹F NMR (376 MHz, DMSO-*d6*) δ -72.32 (s, 3F), -102.56 - -102.78 (m, 1F), -108.90 - -109.11 (m, 2F), - 109.96 (d, 1F).

### Preparation of compounds 9A and 9B

Compound **9** (96 mg) was subjected to SFC chiral preparative resolution (preparative separation method, instrument model: MG II preparative SFC(SFC-14); model of chromatographic column: ChiralPak AD, 250 × 30 mm I.D., 10 µm; mobile phase: A: CO₂ B: ethanol; elution gradient: B 10%; flow rate: 60 mL/min; column pressure: 100 bar; column temperature: 38°C; detection wavelength: 220 nm; cycle: about 5.2 min) to obtain the title compounds **9A** (23 mg, single enantiomer) and **9B** (23 mg, single enantiomer).

Compound **9A:** LC-MS (ESI): m/z 517.0 [M+H]⁺. Chiral analysis method (model of chromatographic column: AD-3 150 × 4.6 mm I.D., 3 µm; mobile phase: A: CO₂ B: ethanol (0.05% DEA); elution gradient: the mobile phase increased from 5% B to 40% B within 5 min and maintained at 40% B for 2.5 min for elution, then 5% B was equilibrated for 2.5 min; flow rate: 2.5 mL/min; column temperature: 35°C; column pressure: 1500 psi; detection wavelength: 220 nm; Rt = 2.838 min). ¹H NMR (400 MHz, DMSO-d6) δ 9.16 (s, 1H), 7.34 - 7.20 (m, 4H), 7.12 - 7.07 (m, 2H), 6.97 - 6.92 (m, 2H), 5.44 (d, *J* = 14.4 Hz, 1H), 5.02 (d, *J* = 14.4 Hz, 1H), 4.78 - 4.61 (q, *J* = 8.8 Hz, 2H), 2.01 (d, *J* = 9.6, 3H), 1.81 (d, *J* = 9.6, 3H). ¹⁹F NMR (376 MHz, DMSO-d6) δ -72.57 (s, 3F), -108.73 - -108.96 (m, 2F), -113.15 - -113.47 (m, 1F), -118.27 - -118.42 (m, 1F).

Compound **9B:** LC-MS (ESI): m/z 517.0 [M+H]⁺. Chiral analysis method (model of chromatographic column: AD-3 150 × 4.6 mm I.D., 3 µm; mobile phase: A: CO₂ B: ethanol (0.05% DEA); elution gradient: the mobile phase increased from 5% B to 40% B within 5 min and maintained at 40% B for 2.5 min for elution, then 5% B was equilibrated for 2.5 min; flow rate: 2.5 mL/min; column temperature: 35°C; column pressure: 1500 psi; detection wavelength: 220 nm; Rt = 2.108 min). ¹H NMR (400 MHz, DMSO-d6) δ 9.15 (s, 1H), 7.35 - 7.20 (m, 4H), 7.12 - 7.07 (m, 2H), 6.98 - 6.93 (m, 2H), 5.44 (d, *J* = 14.4 Hz, 1H), 5.02 (d, *J* = 14.4 Hz, 1H), 4.74 - 4.67 (q, *J* = 8.8 Hz, 2H), 2.01 (d, *J* = 9.2, 3H), 1.81 (d, *J* = 9.6, 3H). ¹⁹F NMR (376 MHz, DMSO-*d6*) δ -72.57 (s, 3F), -108.71 - -108.96 (m, 2F), -113.13 - -113.47 (m, 1F), -118.27 - -118.42 (m, 1F).

### Example 10: Preparation of compounds 10A and 10B

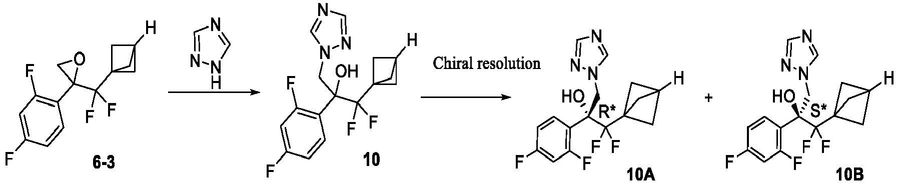

### Preparation of compound 10

Compound 1,2,4-triazole (380 mg, 5.51 mmol) was dissolved in DMF solution (1.5 mL) and cooled to 0°C. Sodium hydride (133 mg, 5.51 mmol) was added thereto and reacted for half an hour. Compound **6-3** (300 mg, 1.1 mmol) was added thereto, and the reaction system was stirred at 80°C for 2 hours. After the reaction system was cooled, the reaction mixture was filtered, and the crude filtrate was purified by preparative separation (preparation method: mobile phase: A: 0.1% formic acid aqueous solution; B: acetonitrile; chromatographic column: Agilent 10 Prep-C18 250 × 21.2 mm; column temperature: 25°C; gradient: 40% to 60% acetonitrile in 12 min; flow rate: 30 mL/min) to obtain the title compound **10** (130 mg, yield: 34%).

Compound **10:** LC-MS (ESI): m/z 342.2 [M+H]⁺. ¹H NMR (400 MHz, DMSO-^6) δ 8.34 (s, 1H), 7.68 (s, 1H), 7.55 - 7.49 (m, 1H), 7.22 - 7.16 (m, 1H), 6.99 - 6.94 (m, 1H), 6.81 (s, 1H), 5.09 (d, *J* = 14.4 Hz, 1H), 4.71 (d, *J* = 14.4 Hz, 1H), 2.33 (s, 1H), 1.76 (dd, *J* = 9.6, 2.0 Hz, 3H), 1.54 (dd, *J* = 9.6, 2.0 Hz, 3H). ¹⁹F NMR (376 MHz, DMSO-*d6*) δ -102.60 - -102.82 (m, 1F), -109.85 (d, 1F), -109.95 (s, 1F), -110.57 (d, 1F).

### Preparation of compounds 10A and 10B

Compound **10** (130 mg) was subjected to SFC chiral preparative resolution (preparative separation method, instrument model: MG II preparative SFC (SFC-1); model of chromatographic column: Cellulose-2, 250 × 30 mm I.D., 10 µm; mobile phase: A: CO₂ B: ethanol; elution gradient: B 40%; flow rate: 70 mL/min; column pressure: 100 bar; column temperature: 38°C; detection wavelength: 220 nm; cycle: about 8 min) to obtain the title compounds **10A** (59 mg, single enantiomer) and **10B** (65 mg, single enantiomer).

Compound **10A:** LC-MS (ESI): m/z 342.2 [M+H]⁺. Chiral analysis method (model of chromatographic column: Cellulose-2 150 × 4.6 mm I.D., 3 µm; mobile phase: A: CO₂ B: ethanol (0.05% DEA); elution gradient: the mobile phase increased from 5% B to 40% B within 5 min and maintained at 40% B for 2.5 min for elution, then 5% B was equilibrated for 2.5 min; flow rate: 2.5 mL/min; column temperature: 35°C; column pressure: 1500 psi; detection wavelength: 220 nm; R_{T} = 6.026 min). ¹H NMR (400 MHz, DMSO-*d6*) δ 8.41 (s, 1H), 7.74 (s, 1H), 7.61 - 7.55 (m, 1H), 7.28 - 7.22 (m, 1H), 7.06 - 7.01 (m, 1H), 6.88 (s, 1H), 5.15 (d, *J* = 14.4 Hz, 1H), 4.77 (d, *J =* 14.4 Hz, 1H), 2.39 (s, 1H), 1.83 - 1.81 (m, 3H), 1.62 - 1.59 (m, 3H). ¹⁹F NMR (376 MHz, Chloroform-*d*) δ -102.60- -102.81 (m, 1F), -109.84 - -109.95 (m, 2F), -110.56 - -110.58 (m, 1F).

Compound **10B:** LC-MS (ESI): m/z 342.2 [M+H]⁺. Chiral analysis method (model of chromatographic column: Cellulose-2 150 × 4.6 mm I.D., 3 µm; mobile phase: A: CO₂ B: ethanol (0.05% DEA); elution gradient: the mobile phase increased from 5% B to 40% B within 5 min and maintained at 40% B for 2.5 min for elution, then 5% B was equilibrated for 2.5 min; flow rate: 2.5 mL/min; column temperature: 35°C; column pressure: 1500 psi; detection wavelength: 220 nm; R_{T} = 6.026 min). ¹H NMR (400 MHz, DMSO-d6) δ 8.41 (s, 1H), 7.74 (s, 1H), 7.61 - 7.55 (m, 1H), 7.28 - 7.22 (m, 1H), 7.06 - 7.01 (m, 1H), 6.88 (s, 1H), 5.15 (d, *J* = 14.4 Hz, 1H), 4.77 (d, *J =* 14.4 Hz, 1H), 2.39 (s, 1H), 1.83 - 1.81 (m, 3H), 1.62 - 1.59 (m, 3H). ¹⁹F NMR (376 MHz, Chloroform-*d*) δ -102.60- -102.82 (m, 1F), -109.84 - -109.95 (m, 2F), -110.56 - -110.58 (m, 1F).

### Example 11: Preparation of compound 11

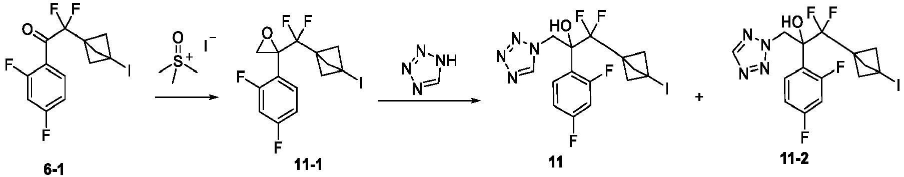

### Preparation of compound 11-1

Compound trimethylsulfoxonium iodide (790 mg, 3.59 mmol) was dissolved in a mixed solvent of THF (10 mL) and DMSO (6 mL), then potassium tert-butoxide (402.8 mg, 3.59 mmol) was added thtereto, and the mixture was stirred at room temperature for 1 hour, then cooled to 0°C, added with compound **6-1** (1.25 g, 3.26 mmol), and stirred for 2 hours. The reaction was quenched by the adding hydrochloric acid aqueous solution (1 M, 1 mL). The reaction system was extracted with EtOAc (30 mL × 3), and the organic phases were combined, dried, and concentrated to obtain a crude product. The crude product was subjected to normal-phase silica gel column chromatography (PE/EtOAc = 0 to 100%) to obtain a pale yellow liquid (480 mg, yield: 35%). ¹H NMR (400 MHz, CDCl₃) δ 7.54 - 7.48 (m, 1H), 6.95 - 6.82 (m, 2H), 3.34 (d, *J* = 8 Hz, 1H), 2.90 - 2.89 (m, 1H), 2.36 - 2.31 (m, 6H).

### Preparation of compound 11

Compound **11-1** (230 mg, 0.58 mmol) was dissolved in DMF solution (3.0 mL), and compound 1H-tetrazole (48.1 mg, 0.69 mmol) and potassium carbonate (91.8 mg, 0.66 mmol) were added thereto. The reaction system was stirred for 2 hours at 80°C in a sealed tube. After the reaction system was cooled, the reaction mixture was filtered, and the crude filtrate was purified by preparative separation (preparation method: instrument: Gilson-GX-281, flow rate: 25 mL/min, mobile phase: acetonitrile, 0.1% FA aqueous solution, method: 5% to 73% acetonitrile, Agilent Pursuit XRs 10 C18 250 * 21.2 mm column; peak time: 9.0 to 9.6 min) to obtain the title compound **11** (34.7 mg) and the corresponding regioisomer compound **11-2** (17.7 mg).

Compound **11:** LC-MS (ESI): m/z 469.1 [M+H]⁺. ¹H NMR (400 MHz, CD₃OD) δ 8.87 (s, 1H), 7.58 - 7.57 (m, 1H), 7.00 (m, 1H), 6.92 (m, 1H), 5.55 (d, *J* = 13.6 Hz, 1H), 4.97 (d, *J* = 13.6 Hz, 1H) 2.30 (dd, *J* = 9.2, 2 Hz, 3H), 2.09 (dd, *J* = 9.2, 2 Hz, 3H).

Compound **11-2:** LC-MS (ESI): m/z 469.1 [M+H]⁺. ¹H NMR (400 MHz, CD₃OD) δ 8.46 (s, 1H), 7.58 - 7.55 (m, 1H), 7.00 - 6.95 (m, 1H), 6.91 - 6.87 (m, 1H), 5.68 (d, *J* = 13.6 Hz, 1H), 5.27 (d, *J* = 13.6 Hz, 1H) 2.30 (dd, *J* = 9.2, 2 Hz, 3H), 2.09 (dd, *J* = 9.2, 2 Hz, 3H).

### Example 12: Preparation of compound 12

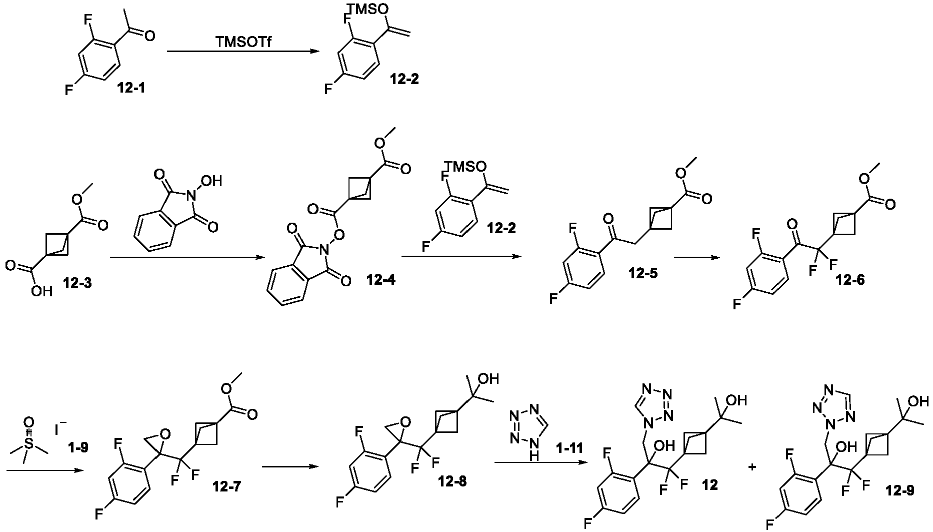

### Preparation of compound 12-2

Compound **12-1** (4 g, 25.62 mmol) was dissolved in dichloromethane solution (50 mL), then the reaction system was cooled to -10°C, added with triethylamine (3.88 g, 38.39 mmol), and stirred for 0.5 hours. Then TMSOTf (6.38 g, 30.71 mmol) was slowly added dropwise to the reaction system. The reaction system was stirred and reacted at -10°C for 2 hours. The reaction mixture was added with water (50 mL), and the dichloromethane phase was separated, dried over anhydrous sodium sulfate, added with petroleum ether (100 mL), and filtered through silica gel to obtain the title compound **12-2** (4.4 g, yield: 75%). ¹H NMR (400 MHz, Chloroform-*d*) δ 7.57 - 7.51 (m, 1H), 6.88 - 6.77 (m, 2H), 4.96 (t, *J* = 1.2 Hz, 1H), 4.66 (t, *J =* 1.6 Hz, 1H), 0.25 (s, 9H).

### Preparation of compound 12-4

Compound **12-3** (4 g, 23.51 mmol) was dissolved in dichloromethane solution (50 mL) and cooled to 0°C. DMAP (0.574 g, 4.7 mmol) and N-hydroxyphthalimide (4.22 g, 25.9 mmol) were added thereto, and the reaction mixture was stirred and reacted for 0.5 hours. EDCI (6.78 g, 35.3 mmol) was added thereto. The reaction system was stirred at 0°C for 2 hours. The reaction mixture was added with water (50 mL) to quench the reaction, and the organic phase of dichloromethane was separated, dried, and filtered through silica gel to obtain the title compound **12-4** (6 g, yield: 81%). ¹H NMR (400 MHz, Chloroform-*d*) δ 7.90 (dd, *J* = 5.6, 2.8 Hz, 2H), 7.80 (dd, *J* = 5.6, 2.8 Hz, 2H), 3.73 (s, 3H), 2.56 (s, 6H).

### Preparation of compound 12-5

Compound **12-4** (3.3 g, 10.5 mmol), compound **12-2** (4.78 g, 20.9 mmol), and tris(2-phenylpyridine)iridium(III) (CAS No.: 94928-86-6, 69 mg, 0.1 mmol) were dissolved in DMF solution (50 mL), and argon was introduced for 3 min to replace the air. The reaction mixture was reacted for 24 hours under the irradiation of 12 w blue light (LED fixed track lamp, Zhoneshan Huifan Lighting Co., Ltd., product model G1006-12W). The reaction system was concentrated to obtain a crude product, and the crude product was purified by normal-phase silica gel column chromatography (EtOAc/PE = 0 to 5%) to obtain the title compound **12-5** (900 mg, yield: 30%) as a colorless liquid. ¹H NMR (400 MHz, Chloroform-*d*) δ 7.93 - 7.86 (m, 1H), 6.98 - 6.93 (m, 1H), 6.88 - 6.82 (m, 1H), 3.64 (s, 3H), 3.15 (d, *J =* 2.8 Hz, 2H), 2.06 (s, 6H).

### Preparation of compound 12-6

To a three-necked flask were added compound **12-5** (0.55 g, 2.0 mmol), N-fluorobenzenesulfonimide (NFSI, 2.5 g, 7.9 mmol), and 1,3-dimethyl-2-imidazolinone (1.0 g, 8.7 mmol), and the system was replaced with nitrogen three times. To the three-necked flask was added anhydrous THF (15 mL), and LiHMDS (1.3 M, 7.5 mL, 9.9 mmol) was added dropwise thereto at a cooling temperature of -78°C. After the reaction system was stirred and reacted at -78°C for 10 min, 25% sodium thiosulfate aqueous solution (20 mL) was added dropwise to the reaction system to quench the reaction. The reaction system was stirred for 0.5 hours and then gradually warmed to room temperature. The reaction mixture was extracted with EtOAc (20 mL × 3), and the organic phases were combined, dried, and concentrated to obtain a crude product. The crude product was purified by normal-phase silica gel column chromatography (EtOAc/PE = 0 to 5%) to obtain the title compound **12-6** (300 mg, yield: 48%) as a yellow oil. ¹H NMR (400 MHz, Chloroform-*d*) δ 7.86 - 7.80 (m, 1H), 7.02 - 6.97 (m, 1H), 6.95 - 6.90 (m, 1H), 3.70 (s, 3H), 2.27 (s, 6H). ¹⁹F NMR (376 MHz, Chloroform-*d*) δ -99.15 (s, 1F), -103.57 - -103.80 (m, 1F), -107.43 (s, 1F), -107.48 (s, 1F).

### Preparation of compound 12-7

Compound **12-6** (300 mg, 0.95 mmol) was dissolved in a mixed solvent of dichloromethane (3 mL) and water (3 mL), then trimethylsulfoxonium iodide (835 mg, 3.8 mmol) and sodium hydroxide (227 mg, 5.7 mmol) were added thereto, and the reaction system was refluxed for 16 hours. After cooling to room temperature, the reaction system was extracted with DCM (20 mL × 3,) and the organic phases were combined, dried, and concentrated to obtain a crude product. The crude product was purified by normal-phase silica gel column chromatography (EtOAc/PE = 0 to 5%) to obtain the title compound **12-7** (313 mg, crude product, yield: 100%, containing a pair of enantiomers) as a light yellow oil. ¹H NMR (400 MHz, Chloroform-*d*) δ 7.63 - 7.48 (m, 1H), 6.95 - 6.89 (m, 1H), 6.87 - 6.81 (m, 1H), 3.67 (s, 3H), 3.36 (d, *J =* 5.2 Hz, 1H), 2.94 - 2.89 (m, 1H), 2.15 - 2.06 (m, 6H).

### Preparation of compound 12-8

To a three-necked flask was added compound **12-7** (300 mg, 0.9 mmol), and the system was replaced with nitrogen three times. To the three-necked flask was added anhydrous THF (20 mL), and methylmagnesium bromide (3.0 M, 1.2 mL, 3.6 mmol) was added dropwise thereto at a cooling temperature of -78°C. After the reaction system was stirred and reacted at -78°C for 45 min, LC-MS detected that the reaction was completed. The reaction system was added with saturated ammonium chloride solution (5 mL) to quench the reaction, extracted with EtOAc (20 mL × 3), and the organic phases were combined, dried, and concentrated to obtain a crude product. The crude product was purified by normal-phase silica gel column chromatography (EtOAc/PE = 0 to 5%) to obtain the title compound **12-8** (300 mg, crude product, yield: 100%) as a yellow solid. ¹H NMR (400 MHz, Chloroform-*d*) δ 7.58 - 7.52 (m, 1H), 6.93 - 6.88 (m, 1H), 6.85 - 6.80 (m, 1H), 3.35 (d, *J* = 5.2 Hz, 1H), 2.97 - 2.88 (m, 1H), 1.74 - 1.68 (m, 6H), 1.13 (s, 6H).

### Preparation of compound 12

Compound **12-8** (300 mg, 0.9 mmol) was dissolved in DMF solution (1.5 mL), and compound 1H-tetrazole **1-11** (191 mg, 2.72 mmol) and potassium carbonate (376 mg, 2.7 mmol) were added thereto. The reaction system was stirred for 16 hours at 80°C in a sealed tube. After the reaction system was cooled, the reaction mixture was filtered, and the crude filtrate was purified by preparative separation (preparation method: mobile phase: A: 0.1% formic acid aqueous solution; B: acetonitrile; chromatographic column: Agilent 10 Prep-C18 250 × 21.2 mm; column temperature: 25°C; gradient: 35% to 55% acetonitrile in 12 min; flow rate: 30 mL/min) to obtain the title compound **12** (60 mg, yield: 16%, containing a pair of enantiomers) and the corresponding regioisomer compound **12-9** (12 mg, yield: 4%, containing a pair of enantiomers).

Compound **12:** LC-MS (ESI): m/z 401.0 [M+H]⁺. ¹H NMR (400 MHz, DMSO-d6) δ 9.12 (s, 1H), 7.51 - 7.45 (m, 1H), 7.31 - 7.25 (m, 1H), 7.06 (s, 1H), 7.03 - 6.98 (m, 1H), 5.39 (d, *J* = 14.4Hz, 1H), 4.97 (d, *J* = 14.4 Hz, 1H), 4.12 (s, 1H), 1.56 (dd, *J* = 9.6, 1.6 Hz, 3H), 1.36 (dd, *J* = 9.6, 1.6 Hz, 3H), 0.92 (d, *J* = 2.4 Hz, 6H). ¹⁹F NMR (376 MHz, DMSO-d6) δ -102.90 - -103.14 (m, 1F), -103.18 (d, *J* = 9.6 Hz, 1F), -109.26 (d, *J* = 11.3 Hz, 1F), -109.80 (d, *J* = 9.5 H, 1F).

Compound **12-9:** LC-MS (ESI): m/z 401.0 [M+H]⁺. ¹H NMR (400 MHz, DMSO-*d6)* δ 8.76 (s, 1H), 7.54 - 7.48 (m, 1H), 7.28 - 7.22 (m, 1H), 7.10 (s, 1H), 7.03 - 6.98 (m, 1H), 5.57 (d, *J* = 14.4 Hz, 1H), 5.20 (d, *J* = 14.4 Hz, 1H), 4.11 (s, 1H), 1.55 (dd, *J* = 9.4, 1.6 Hz, 3H), 1.35 (dd, *J* = 9.6, 1.6 Hz, 3H), 0.91 (d, *J* = 2.4 Hz, 6H). ¹⁹F NMR (376 MHz, DMSO-d6) δ -102.58 - -102.21 (m, 1F), -102.76 (dd, *J*= 21.9, 9.4 Hz, 1F), -109.00 - -109.20 (m, 1F), -110.16 (d, *J* = 9.5 Hz, 1F).

### Example 13: Preparation of compound 13

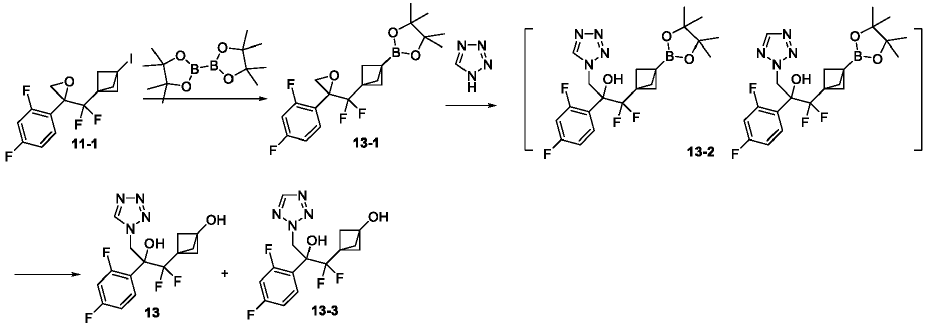

### Preparation of compound 13-1

To a three-necked flask were added compound **11-1** (3 g, 7.5 mmol) and bis(pinacolato)diboron (4.21 g, 22.6 mmol), and the system was replaced with nitrogen three times. To the three-necked flask was added anhydrous ether (150 mL), and tert-butyllithium (1.3 M, 17.4 mL, 22.6 mmol) was added dropwise thereto at a cooling temperature of -78°C. The reaction system was stirred and reacted at -78°C for 120 min. The reaction system was quenched with saturated ammonium chloride solution (50 mL), extracted with EtOAc (20 mL × 3), and the organic phases were combined, dried, and concentrated to obtain a crude product. The crude product was purified by normal-phase silica gel column chromatography (EtOAc/PE = 0 to 5%) to obtain the title compound (1.2 g, yield: 40%) as a colorless oil. ¹H NMR (400 MHz, Chloroform-*d*) δ 7.59 - 7.52 (m, 1H), 6.92 - 6.87 (m, 1H), 6.84 - 6.79 (m, 1H), 3.34 (d, *J* = 5.2 Hz, 1H), 2.92 - 2.89 (m, 1H), 1.96 - 1.87 (m, 6H), 1.22 (s, 12H).

### Preparation of compound 13-2

Compound **13-1** (500 mg, 1.26 mmol) was dissolved in DMF solution (1.5 mL), and compound 1H-tetrazole **1-11** (439 mg, 6.3 mmol) and potassium carbonate (0.867 g, 6.3 mmol) were added thereto. The reaction system was stirred for 2 hours at 80°C in a sealed tube. After the reaction system was cooled, the reaction mixture was filtered, and the filtrate was evaporated to dryness by rotary evaporation to obtain the crude mixed compound **13-2,** which was directly used in the next reaction step. LC-MS (ESI): m/z 469.2 [M+H]⁺.

### Preparation of compound 13

Mixed compound **13-2** (580 mg, 1.47 mmol, crude product) was dissolved in a mixed solvent of tetrahydrofuran (15 mL) and water (5 mL). The reaction system was cooled to - 5°C. Sodium perborate trihydrate (441 mg, 4.4 mmol) was added thereto in batches. The reaction mixture was stirred for 5 min and LC-MS detected that the reaction was completed. The reaction mixture was extracted with ethyl acetate (30 mL × 3), washed with water, dried, and the organic phase was concentrated to obtain a crude product. The crude product was purified by preparative separation (preparation method: mobile phase: A: 0.1% formic acid aqueous solution; B: acetonitrile; chromatographic column: Agilent 10 Prep-C18 250 × 21.2 mm; column temperature: 25°C; gradient: 30% to 50% acetonitrile in 12 min; flow rate: 30 mL/min) to obtain the title compound **13** (200 mg, yield: 47%, containing a pair of enantiomers) and the corresponding regioisomer compound **13-3** (100 mg, yield: 23%, containing a pair of enantiomers).

Compound **13:** LC-MS (ESI): m/z 359.0 [M+H]⁺. ¹H NMR (400 MHz, DMSO-d6) δ 9.11 (s, 1H), 7.51 - 7.45 (m, 1H), 7.29 - 7.23 (m, 1H), 7.07 (br.s, 1H), 7.03 - 6.98 (m, 1H), 6.34 (br.s, 1H), 5.39 (d, *J =* 14.5 Hz, 1H), 4.96 (d, *J* = 14.6 Hz, 1H), 1.78 - 1.76 (m, 3H), 1.55 - 1.53 (m, 3H). ¹⁹F NMR (376 MHz, DMSO-*d6*) δ -103.07 - -103.29 (m, 1F), -106.29 (s, 1F), -106.39 (s, 1F), -109.73- -109.75 (d, *J* = 9.6 Hz, 1F).

Compound **13-3:** LC-MS (ESI): m/z 359.0 [M+H]⁺. ¹H NMR (400 MHz, DMSO-*d6*) δ 8.75 (s, 1H), 7.54 - 7.47 (m, 1H), 7.28 - 7.21 (m, 1H), 7.11 (s, 1H), 7.02 - 6.97 (m, 1H), 6.31 (s, 1H), 5.57 (d, *J* = 14.2 Hz, 1H), 5.19 (d, *J =* 14.2 Hz, 1H), 1.77 - 1.74 (m, 3H), 1.54 - 1.52 (m, 3H). ¹⁹F NMR (376 MHz, DMSO-*d6*) δ -102.69 - -102.91 (m, 1F), -105.98 - - 106.15 (m, 2F), -109.11 (d, *J* = 9.6 Hz, 1F).

### Example 14: Preparation of compounds 14A and 14B

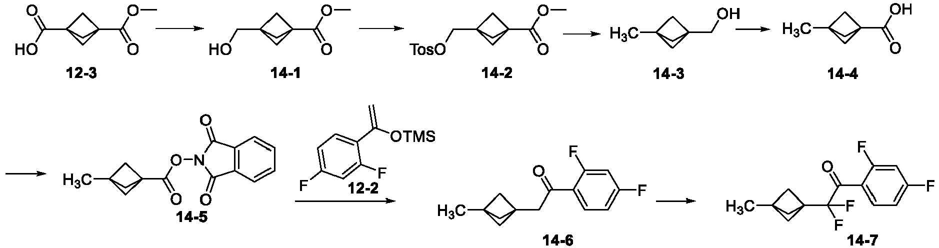

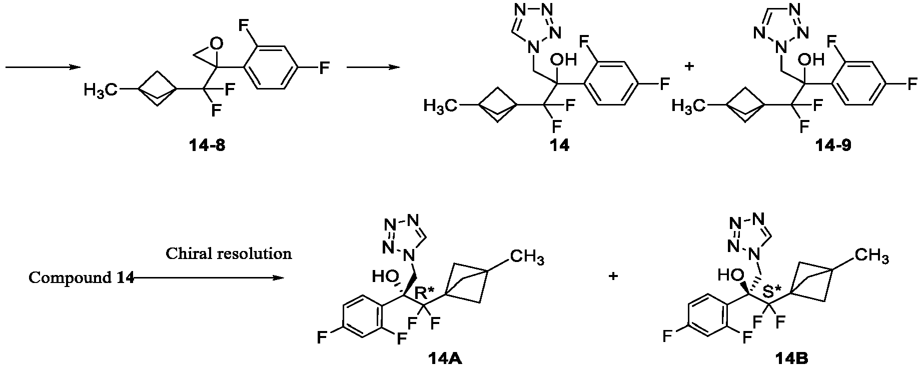

### Preparation of compound 14-1

To a three-necked flask was added compound **12-3** (4 g, 23.5 mmol), and the system was replaced with argon three times. Then tetrahydrofuran (40 mL) was added thereto, and the reaction mixture was cooled to 0°C. Borane dimethyl sulfide (2 M in THF, 13 mL, 26 mmol) was slowly added dropwise thereto. After the reaction was completed, the reaction system was stirred at room temperature for 16 hours. The reaction mixture was added with 10 mL of methanol to quench the reaction, and evaporated to dryness by rotary evaporation to obtain the title compound **14-1** (3.6 g, yield: 98.0%). ¹H NMR (400 MHz, Chloroform-*d*) δ 3.67 (s, 3H), 3.62 (s, 2H), 1.98 (s, 6H).

### Preparation of compound 14-2

To a three-necked flask were added compound **14-1** (3.3 g, 21.1 mmol) and p-toluenesulfonyl chloride (4.83 g, 25.4 mmol), and the system was replaced with argon three times. Then dichloromethane (40 mL) was added thereto, and the reaction mixture was cooled to 0°C. Triethylamine (2.57 g, 25.4 mmol) was slowly added dropwise thereto. The reaction system was stirred at room temperature for 16 hours, then the organic phase was evaporated to dryness by rotary evaporation, and purified by normal-phase silica gel column chromatography (EtOAc/PE = 0 to 50%) to obtain the title compound **14-2** (5.5 g, yield: 84%) as a white solid. ¹H NMR (400 MHz, DMSO-*d6*) δ 7.85 - 7.72 (dd, *J* = 8.0, 4.0 Hz, 2H), 7.56 - 7.41 (dd, *J* = 8.0, 4.0 Hz, 2H), 4.07 (s, 2H), 3.58 (s, 3H), 2.43 (s, 3H), 1.86 (s, 6H).

### Preparation of compound 14-3

To a three-necked flask was added lithium aluminum hydride (0.7 g, 19.33 mmol), and the system was replaced with argon three times. Then anhydrous tetrahydrofuran (40 mL) was added thereto, and the reaction mixture was cooled to 0°C. Compound **14-2** (3 g, 9.67 mmol) was dissolved in anhydrous tetrahydrofuran (15 mL) solution and slowly added dropwise to the reaction system. After the reaction was completed, the reaction system was stirred at room temperature for 16 hours. Water (0.7 mL), sodium hydroxide aqueous solution (15%, 0.7 mL), and water (2.1 mL) were slowly added dropwise to the system to quench the reaction at 0°C, then anhydrous magnesium sulfate was added thereto. The resulting mixture was stirred vigorously, filtered, and the filtrate was evaporated to dryness by rotary evaporation to obtain the title compound **14-3** (0.950 g, yield: 87%) as a colorless oil. ¹H NMR (400 MHz, Chloroform-*d*) δ 3.54 (s, 2H), 1.55 (s, 6H), 1.16 (s, 3H).

### Preparation of compound 14-4

To a three-necked flask was added compound **14-3** (0.950 g, 8.47 mmol). Then carbon tetrachloride (15 mL), hexanenitrile (15 mL), and water (15 mL) were added thereto, and the reaction mixture was cooled to 0°C. Sodium periodate (7.25 g, 33.88 mmol) and RuCl₃ trihydrate (0.332 g, 1.3 mmol) were added thereto, and the reaction system was stirred at room temperature for 16 hours. The organic phase was evaporated to dryness by rotary evaporation, and the residue was extracted with dichloromethane. The organic phase was dried, filtered, and evaporated to dryness by rotary evaporation, and the residue was purified by normal-phase silica gel column chromatography (EtOAc/PE = 0 to 50%) to obtain the title compound **14-5** (0.750 g, yield: 70%) as a white solid. ¹H NMR (400 MHz, Chloroform-*d*) δ 8.97 (br.s, 1H), 1.95 (s, 6H), 1.19 (s, 3H).

### Preparation of compound 14-5

Compound **14-4** (0.750 g, 5.95 mmol) was dissolved in dichloromethane solution (15 mL). The reaction system was cooled to 0°C, then DMAP (0.145 g, 1.19 mmol) and N-hydroxyphthalimide (1.16 g, 7.13 mmol) were added thereto, and the reaction system was stirred for half an hour. EDCI (1.71 g, 8.92 mmol) was further added thereto. The reaction system was stirred at 0°C for 2 hours. The reaction mixture was added with water (50 mL) to quench the reaction, and the dichloromethane phase was separated, dried, and filtered through silica gel to obtain the title compound **14-5** (1.3 g, yield: 80%). ¹H NMR (400 MHz, Chloroform-*d*) δ 7.89 - 7.77 (m, 2H), 7.80 - 7.77 (m, 2H), 2.17 (s, 6H), 1.25 (s, 3H).

### Preparation of compound 14-6

Compound **14-5** (2.9 g, 10.69 mmol), compound **12-2** (4.88 g, 21.38 mmol), and tris(2-phenylpyridine)iridium(III) (703 mg, 1.07 mmol) were dissolved in DMF solution (25 mL), and the system was replaced with argon for 3 min. The reaction mixture was reacted for 24 hours under the irradiation of 12 w blue light (LED fixed track lamp, Zhoneshan Huifan Lighting Co., Ltd., product model G1006-12W). The reaction mixture was concentrated to obtain a crude product, and the crude product was purified by normal-phase silica gel column chromatography (EtOAc/PE = 0 to 5%) to obtain the title compound **14-6** (230 mg, yield: 9%) as a colorless liquid. ¹H NMR (400 MHz, Chloroform-*d*) δ 7.90 - 7.84 (m, 1H), 6.97 - 6.92 (m, 1H), 6.88 - 6.82 (m, 1H), 3.10 (d, *J* = 2.8 Hz, 2H), 1.60 (s, 6H), 1.11 (s, 3H).

### Preparation of compound 14-7

To a three-necked flask were added compound **14-6** (0.230 g, 0.973 mmol), N-fluorobenzenesulfonimide (NFSI, 1.23 g, 3.89 mmol), and 1,3-dimethyl-2-imidazolinone (0.333 g, 2.92 mmol), and the system was replaced with nitrogen three times. To the three-necked flask was added anhydrous THF (15 mL), and LiHMDS (1.3 M, 3.89 mL, 5.1 mmol) was added dropwise thereto at a cooling temperature of -78°C. After the reaction system was stirred and reacted at -78°C for 10 min, then 25% sodium thiosulfate aqueous solution (20 mL) was added dropwise to the reaction system to quench the reaction, and the temperature was gradually warmed to room temperature with stirring. The reaction mixture was extracted with EtOAc (20 mL × 3), and the organic phases were combined, dried, and concentrated to obtain a crude product. The crude product was purified by normal-phase silica gel column chromatography (EtOAc/PE = 0 to 5%) to obtain the title compound **14-6** (150 mg, yield: 57%) as a yellow oil. ¹H NMR (400 MHz, Chloroform-*d*) δ 7.85 - 7.77 (m, 1H), 7.00 - 6.95 (m, 1H), 6.93 - 6.88 (m, 1H), 1.83 (s, 6H), 1.20 (s, 3H). ¹⁹F NMR (376 MHz, Chloroform-*d*) δ - 100.02 (m, 1F), -104.21 (m, 1F), -107.36 (d, *J =* 13.4 Hz, 2F).

### Preparation of compound 14-8

Compound **14-7** (150 mg, 0.555 mmol) was dissolved in a mixed solvent of dichloromethane (3 mL) and water (3 mL), then trimethylsulfoxonium iodide (363 mg, 1.65 mmol) and sodium hydroxide (99 mg, 2.48 mmol) were added thereto, and the reaction system was refluxed for 16 hours. After cooling to room temperature, the reaction system was extracted with DCM (30 mL × 3,) and the organic phases were combined, dried, and concentrated to obtain a crude product. The crude product was purified by normal-phase silica gel column chromatography (EtOAc/PE = 0 to 5%) to obtain the title compound **14-8** (150 mg, yield: 100%, crude product, containing a pair of enantiomers) as a light yellow oil. ¹H NMR (400 MHz, Chloroform-*d*) δ 7.56 - 7.43 (m, 1H), 6.86 - 6.81 (m, 1H), 6.78 - 6.72 (m, 1H), 3.27 (dd, *J* = 5.6, 0.8 Hz, 1H), 2.86 - 2.83 (m, 1H), 1.66 - 1.56 (m, 6H), 1.07 (s, 3H).

### Preparation of compound 14

Compound **14-8** (150 mg, 0.523 mmol) was dissolved in DMF solution (1.5 mL), and compound 1H-tetrazole **1-11** (183 mg, 2.72 mmol) and potassium carbonate (362 mg, 2.62 mmol) were added thereto. The reaction system was stirred for 16 hours at 80°C in a sealed tube. After the reaction system was cooled, the reaction mixture was filtered, and the crude filtrate was purified by preparative separation (preparation method: mobile phase: A: 0.1% formic acid aqueous solution; B: acetonitrile; chromatographic column: Agilent 10 Prep-C18 250 × 21.2 mm; column temperature: 25°C; gradient: 55% to 75% acetonitrile in 12 min; flow rate: 30 mL/min) to obtain the title compound **14** (90 mg, yield: 48.0%, containing a pair of enantiomers) and its regioisomer compound **14-9** (45 mg, yield: 24.0%, containing a pair of enantiomers).

Compound **14:** LC-MS (ESI): m/z 357.2 [M+H]⁺. ¹H NMR (400 MHz, DMSO-*d6*) δ 9.10 (s, 1H), 7.51-7.45 (m, 1H), 7.29 - 7.22 (m, 1H), 7.03 (s, 1H), 7.02 - 6.97 (m, 1H), 5.38 (d, *J* = 14.6 Hz, 1H), 4.95 (d, *J* = 14.6 Hz, 1H), 1.61 (dd, *J* = 9.6, 1.8 Hz, 3H), 1.40 (dd, *J =* 9.6, 1.8 Hz, 3H), 1.03 (s, 3H). ¹⁹F NMR (376 MHz, DMSO-d6) δ -103.04 - -103.26 (m, 1F), -109.17 - -109.28 (m, 2F), -109.82 (d, *J* = 9.6 Hz, 1F).

Compound **14-9:** LC-MS (ESI): m/z 357.2 [M+H]⁺. ¹H NMR (400 MHz, DMSO-*d6)* δ 8.74 (s, 1H), 7.54 - 7.48 (m, 1H), 7.27 - 7.21 (m, 1H), 7.07 (s, 1H), 7.01 - 6.96 (m, 1H), 5.56 (d, *J* = 14.2 Hz, 1H), 5.18 (d, *J* = 14.2 Hz, 1H), 1.61 - 1.58 (m, 3H), 1.41 - 1.38 (m, 3H), 1.03 (s, 3H). ¹⁹F NMR (376 MHz, DMSO-d6) δ -102.64-102.86 (m, 1F), -108.82 - -109.04 (m, 2F), -110.19 (d, *J* = 9.6 Hz, 1F).

### Preparation of compounds 14A and 14B

Compound **14** (90 mg) was subjected to SFC chiral preparative resolution (preparative separation method, instrument model: MG II preparative SFC (SFC-1); model of chromatographic column: Cellulose-2, 250 × 30 mm I.D., 10 µm; mobile phase: A: CO₂ B:ethanol; elution gradient: B 40%; flow rate: 70 mL/min; column pressure: 100 bar; column temperature: 38°C; detection wavelength: 220 nm; cycle: about 8 min) to obtain the title compounds **14A** (43 mg, single enantiomer) and **14B** (41 mg, single enantiomer).

CCompound **14A:** LC-MS (ESI): m/z 357.0 [M+H]⁺. Chiral analysis method (model of chromatographic column: Cellulose-2 150 × 4.6 mm I.D., 3 µm; mobile phase: A: CO₂ B: ethanol (0.05% DEA); elution gradient: the mobile phase increased from 5% B to 40% B within 5 min and maintained at 40% B for 2.5 min for elution, then 5% B was equilibrated for 2.5 min; flow rate: 2.5 mL/min; column temperature: 35°C; column pressure: 1500 psi; detection wavelength: 220 nm; R_{T} = 6.026 min). ¹H NMR (400 MHz, DMSO-d6) δ 9.10 (s, 1H), 7.51 - 7.45 (m, 1H), 7.28 - 7.22 (m, 1H), 7.03 (s, 1H), 7.02 - 6.97 (m, 1H), 5.38 (d, *J =* 14.5 Hz, 1H), 4.95 (d, *J* = 14.6 Hz, 1H), 1.63 - 1.60 (m, 3H), 1.41 - 1.39 (m, 3H), 1.03 (s, 3H). ¹⁹F NMR (376 MHz, Chloroform-J) δ -103.04- -103.26 (m, 1F), -109.18 - -109.28 (m, 2F), - 109.82 (d, *J* = 9.6 Hz, 1F).

Compound **14B:** LC-MS (ESI): m/z 357.0 [M+H]⁺. Chiral analysis method (model of chromatographic column: Cellulose-2 150 × 4.6 mm I.D., 3 µm; mobile phase: A: CO₂ B: ethanol (0.05% DEA); elution gradient: the mobile phase increased from 5% B to 40% B within 5 min and maintained at 40% B for 2.5 min for elution, then 5% B was equilibrated for 2.5 min; flow rate: 2.5 mL/min; column temperature: 35°C; column pressure: 1500 psi; detection wavelength: 220 nm; R_{T} = 6.026 min). ¹H NMR (400 MHz, DMSO-*d6*) δ 9.10 (s, 1H), 7.51 - 7.45 (m, 1H), 7.28 - 7.22 (m, 1H), 7.03 (s, 1H), 7.02 - 6.96 (m, 1H), 5.38 (d, *J =* 14.5 Hz, 1H), 4.95 (d, *J* = 14.6 Hz, 1H), 1.63 - 1.60 (m, 3H), 1.41 - 1.39 (m, 3H), 1.03 (s, 3H). ¹⁹F NMR (376 MHz, Chloroform-J) δ -103.04- -103.26 (m, 1F), -109.18 - -109.28 (m, 2F), - 109.84 (d, *J* = 9.6 Hz, 1F).

### Example 15: Preparation of compound 15

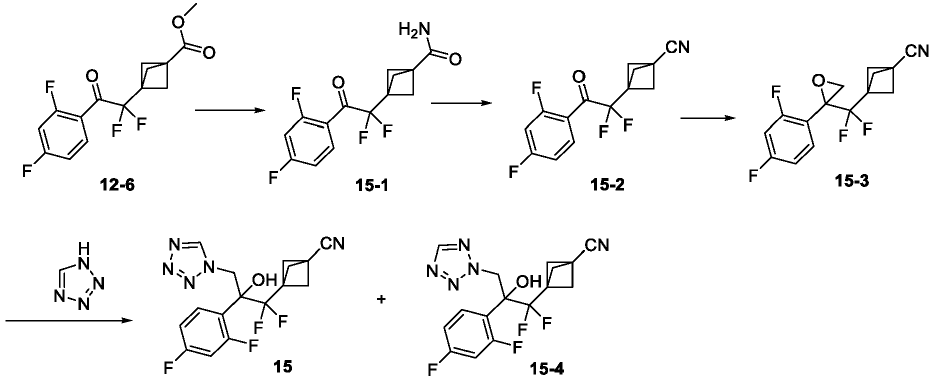

### Preparation of compound 15-1

Compound **12-6** (600 mg, 1.9 mmol) was dissolved in a methanol solution of ammonia (7.0 M, 6 mL). The reaction mixture was stirred at 80°C for 16 hours in a sealed tube. After the reaction was completed, the reaction mixture was dried and concentrated to obtain a crude product, and the crude product was purified by normal-phase silica gel column chromatography (EtOAc/PE = 0 to 30%) to obtain the title compound **15-1** (420 mg, yield: 73%). LC-MS (ESI): m/z 302.2 [M+H]⁺.

### Preparation of compound 15-2

Compound **15-1** (420 mg, 1.39 mmol) was dissolved in DMF (4 mL), and the system was replaced with nitrogen three times. Cyanuric chloride (386 mg, 2.09 mmol) was added thereto at 0°C. The reaction system was slowly warmed to room temperature and stirred for 2 hours. After the reaction was completed, the reaction system was extracted with EtOAc (30 mL × 3), and the organic phases were combined, dried, and concentrated to obtain a crude product. The crude product was purified by normal-phase silica gel column chromatography (EtOAc/PE = 0 to 5%) to obtain the title compound **15-2** (180 mg, yield: 45%). LC-MS (ESI): m/z 284.1 [M+H]⁺.

### Preparation of compound 15-3

Compound **15-2** (180 mg, 0.63 mmol) was dissolved in a mixture of dichloromethane (5.0 mL) and water (2.0 mL), then trimethylsulfoxonium iodide **1-9** (209 mg, 0.95 mmol) and solid sodium hydroxide (38 mg, 0.95 mmol) were added thereto, and the reaction system was stirred at 65°C for 16 hours. After the reaction was completed, the reaction system was extracted with dichloromethane (20 mL × 3), and the organic phases were combined, dried, and concentrated to obtain a crude product. The crude product was purified by normal-phase silica gel column chromatography (EtOAc/PE = 0 to 5%) to obtain the title compound **15-3** (180 mg, yield: 95%). LC-MS (ESI): m/z 298.1 [M+H]⁺.

### Preparation of compound 15

Compound **15-3** (180 mg, 0.73 mmol) was dissolved in DMF solution (1.5 mL), and compound 1H-tetrazole **1-11** (222 mg, 3.18 mmol) and potassium carbonate (439 mg, 3.18 mmol) were added thereto. The reaction system was stirred for 16 hours at 80°C in a sealed tube. After the reaction system was cooled, the reaction mixture was filtered, and the crude filtrate was purified by preparative separation (preparation method: mobile phase: A: 0.1% formic acid aqueous solution; B: acetonitrile; chromatographic column: Agilent 10 Prep-C18 250 × 21.2 mm; column temperature: 25°C; gradient: 55% to 85% acetonitrile in 12 min; flow rate: 30 mL/min) to obtain the title compound **15** (75 mg, yield: 33%, containing a pair of enantiomers) and the corresponding regioisomer compound **15-4** (35 mg, yield: 16%, containing a pair of enantiomers).

Compound **15:** LC-MS (ESI): m/z 368.2 [M+H]⁺. ¹H NMR (400 MHz, DMSO-d6) δ 9.05 (s, 1H), 7.55 - 7.34 (m, 1H), 7.34 - 7.15 (m, 2H), 7.09 - 6.84 (m, 1H), 5.30 (d, *J* = 14.6 Hz, 1H), 4.90 (d, *J* = 14.6 Hz, 1H), 2.33 - 2.11 (m, 3H), 2.12 - 1.78 (m, 3H). ¹⁹F NMR (376 MHz, DMSO-d6) δ -102.74 - -103.17 (m, 1F), -109.14 (m, 1F), -109.95 (m, 1F), -110.05 (s, 1F).

Compound **15-4:** LC-MS (ESI): m/z 368.2 [M+H]⁺. ¹H NMR (400 MHz, DMSO-*d6)* δ 8.76 (s, 1H), 7.64 - 7.47 (m, 1H), 7.35 (s, 1H), 7.33 - 7.22 (m, 1H), 7.05 - 6.93 (m, 1H), 5.56 (d, *J* = 14.3 Hz, 1H), 5.19 (d, J = 14.3 Hz, 1H), 2.31 - 2.16 (m, 3H), 2.11 - 1.97 (m, 3H). ¹⁹F NMR (376 MHz, DMSO-d6) δ -102.29 - -102.88 (m, 1F), -109.34 - -109.55 (m, 1F), - 109.60 - -109.97 (m, 2F).

### Example 16: Preparation of compound 16

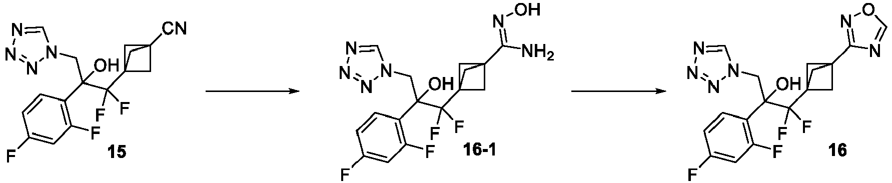

### Preparation of compound 16-1

Compound **15** (50 mg, 0.14 mmol) was dissolved in methanol (2.0 mL), and DIPEA (26 mg, 0.2 mmol) and hydroxylamine hydrochloride (14 mg, 0.2 mmol) were added thereto respectively. The reaction mixture was stirred at 80°C for 16 hours in a sealed tube. After the reaction was completed, the reaction mixture was dried and concentrated to obtain the title compound **16-1** (54 mg, crude product). LC-MS (ESI): m/z 401.2 [M+H]⁺.

### Preparation of compound 16

Compound **16-1** (54 mg, 0.14 mmol) was dissolved in trimethyl orthoformate (1.0 mL), and one drop of TFA was added thereto. The reaction system was stirred for 2 hours at 60°C in a sealed tube. After the reaction system was cooled, the reaction mixture was evaporated to dryness by rotary evaporation to obtain a crude product, and the crude product was purified by preparative separation (preparation method: mobile phase: A: 0.1% formic acid aqueous solution; B: acetonitrile; chromatographic column: Agilent 10 Prep-C18 250 × 21.2 mm; column temperature: 25°C; gradient: 55% to 75% acetonitrile in 12 min; flow rate: 30 mL/min) to obtain the title compound **16** (20 mg, yield: 36%, containing a pair of enantiomers).

Compound **16:** LC-MS (ESI): m/z 411.0 [M+H]⁺. ¹H NMR (400 MHz, DMSO-d6) δ 9.53 (s, 1H), 9.14 (s, 1H), 7.61 - 7.49 (m, 1H), 7.35 - 7.25 (m, 1H), 7.28 (s, 1H), 7.09 - 6.98 (m, 1H), 5.42 (d, *J* = 14.6 Hz, 1H), 5.02 (d, *J* = 14.6 Hz, 1H), 2.17 (dd, *J* = 9.4, 1.9 Hz, 3H), 1.96 (dd, *J* = 9.4, 1.9 Hz, 3H). ¹⁹F NMR (376 MHz, DMSO-*d6*) δ -102.94 (m, 1F), -109.36 (m, 1F), -109.73 (m, 2F).

### Example 17: Preparation of compound 17

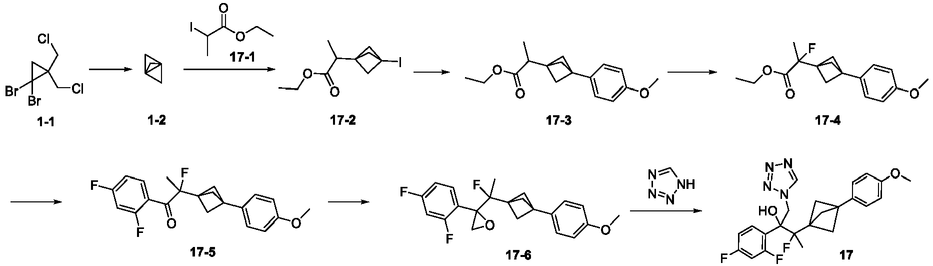

### Preparation of compound 17-2

To a three-necked flask was added compound **1-1** (100 g, 336.9 mmol), and the system was replaced with nitrogen three times. To the three-necked flask was added anhydrous ether (500 mL), and methyllithium (1.6 M, 421 mL, 673.8 mmol) was added dropwise thereto at a cooling temperature of -78°C. The reaction system was stirred and reacted at -78°C for 5 min, then gradually warmed to 0 °C, stirred and reacted for another 3 hours. Then the reaction system was added with compound triethylboron (1.0 M, 16.8 mmol, 16.8 mL) and compound **17-1** (38.4 g, 168 mmol), and stirred for another 16 hours at room temperature. After the reaction was completed, the reaction system was extracted with EtOAc (500 mL × 3), and the organic phases were combined, dried, and concentrated to obtain a crude product. The crude product was purified by normal-phase silica gel column chromatography (EtOAc/PE = 0 to 5%) to obtain the title compound **17-2** (32 g, yield: 64%) as a light yellow liquid. ¹H NMR (400 MHz, Chloroform-*d*) δ 4.14 (q, *J* = 8.0 Hz, 2H), 2.64 (q, *J* = 8.0 Hz, 1H), 2.24 (s, 6H), 1.26 (t, *J* = 8.0 Hz, 3H), 1.09 (d, *J* = 8.0 Hz, 3H).

### Preparation of compound 17-3

To a three-necked flask was added ferric acetylacetonate (7.9 g, 21.7 mmol), and the system was replaced with nitrogen three times. To the three-necked flask were sequentially added anhydrous THF (80 mL), compound **17-2** (32 g, 108.8 mmol), and TMEDA (5.0 g, 43.52 mmol). The reaction system was stirred for 5 min, then a solution of Grignard reagent compound 4-methoxyphenyl magnesium bromide in THF (0.5 M, 435 mL, 217.6 mmol) was slowly added dropwise to the three-necked flask, and the reaction mixture was stirred and reacted at room temperature for another 16 hours. After the reaction was completed, the reaction system was extracted with EtOAc (300 mL × 3), and the organic phases were combined, dried, and concentrated to obtain a crude product. The crude product was purified by normal-phase silica gel column chromatography (EtOAc/PE = 0 to 5%) to obtain the title compound **17-3** (8.0 g, yield: 26.8%) as a light yellow liquid. ¹H NMR (400 MHz, Chloroform-*d*) δ 7.14 - 7.12 (m, 2H), 6.84 - 6.82 (m, 2H), 4.16 (q, *J* = 8.0 Hz, 2H), 3.79 (s, 3H), 2.65 (q, *J* = 8.0 Hz, 1H), 1.92 (s, 6H), 1.28 (t, *J* = 8.0 Hz, 3H), 1.14 (d, *J* = 8.0 Hz, 3H).

### Preparation of compound 17-4

To a three-necked flask was added compound **17-3** (5.5 g, 20 mmol), and the system was replaced with nitrogen three times. To the three-necked flask was added anhydrous THF (50 mL), and LDA (2.0 M, 20 mL, 40 mmol) was added dropwise thereto at a cooling temperature of -78°C. After the reaction system was stirred and reacted at -78°C for 45 min, a solution of compound N-fluorobenzenesulfonimide (NFSI, 10.3 g, 40 mmol) dissolved in anhydrous THF (50 mL) was added dropwise thereto, and the reaction mixture was stirred and reacted for another 12 hours. After the reaction was completed, the reaction system was quenched with saturated ammonium chloride solution (50 mL), extracted with EtOAc (200 mL × 3), and the organic phases were combined, dried, and concentrated to obtain a crude product. The crude product was purified by normal-phase silica gel column chromatography (EtOAc/PE = 0 to 20%) to obtain the title compound **17-4** (3.2 g, yield: 54.7%) as a light yellow liquid. ¹H NMR (400 MHz, Chloroform-*d*) δ 7.14 - 7.12 (m, 2H), 6.86 - 6.83 (m, 2H), 4.27 (q, *J* = 8.0 Hz, 2H), 3.79 (s, 3H), 2.08 - 2.00 (m, 6H), 1.58 - 1.52 (m, 3H), 1.30 (t, *J* = 8.0 Hz, 3H).

### Preparation of compound 17-5

To a three-necked flask was added compound 2,4-difluorobromobenzene (2.5 g, 13.1 mmol), and the system was replaced with nitrogen three times. To the three-necked flask was added anhydrous ether (15 mL), and n-butyllithium (1.6 M, 8.2 mL, 13.1 mmol) was added dropwise thereto at a cooling temperature of -78°C. After the reaction system was stirred and reacted at -78°C for 45 min, a solution of compound **17-4** (3.2 g, 10.9 mmol) dissolved in anhydrous ether (10 mL) was added dropwise thereto, and the reaction mixture was stirred and reacted for another 1 hour. After the reaction was completed, the reaction system was quenched with saturated ammonium chloride solution (5 mL), extracted with EtOAc (20 mL × 3), and the organic phases were combined, dried, and concentrated to obtain a crude product. The crude product was purified by normal-phase silica gel column chromatography (EtOAc/PE = 0 to 5%) to obtain the title compound **17-5** (2.8 g, yield: 71%) as a yellow solid. ¹H NMR (400 MHz, Chloroform-*d*) δ 7.60 - 7.54 (m, 1H), 7.13 - 7.11 (m, 2H), 7.94 - 6.91 (m, 2H), 6.88 - 6.83 (m, 2H), 3.79 (s, 3H), 2.11 - 2.01 (s, 6H), 1.67 - 1.61 (m, 3H)

### Preparation of compound 17-6

Compound trimethylsulfonium iodide (1.8 g, 8.3 mmol) was dissolved in DMSO (15.0 mL) and THF (5.0 mL), then NaH (purity: 60%, 319 mg, 8.3 mmol) was added thereto, and the mixture was stirred at room temperature for 1 hour. Then a solution of compound **17-5** (1.0 g, 2.77 mmol) dissolved in DMSO (5 mL) was added thereto, and the reaction system was stirred at room temperature for 16 hours. After the reaction was completed, the reaction system was quenched with saturated ammonium chloride solution (50 mL), extracted with EtOAc (20 mL × 3), and the organic phases were combined, dried, and concentrated to obtain a crude product. The crude product was purified by normal-phase silica gel column chromatography (EtOAc/PE = 0 to 30%) to obtain the title compound **17-6** (580 mg, yield: 55%, containing two pairs of enantiomers) as a yellow oil. ¹H NMR (400 MHz, Chloroform-*d*) δ 7.62 - 7.53 (m, 1H), 7.26 - 7.10 (m, 2H), 6.90 - 6.89 (m, 2H), 6.83 - 6.77 (m, 2H), 3.78 (s, 3H), 3.32 - 3.32 (m, 1H), 2.91 - 2.89 (m, 1H), 2.05 - 1.96 (m, 6H), 1.50 - 1.44 (m, 3H).

### Preparation of compound 17

Compound **17-6** (240 mg, 0.64 mmol) was dissolved in DMF solution (1.5 mL), and compound 1H-tetrazole (90 mg, 1.28 mmol) and potassium carbonate (132 mg, 0.96 mmol) were added thereto. The reaction system was stirred for 16 hours at 80°C in a sealed tube. After the reaction system was cooled, the reaction mixture was filtered, and the crude filtrate was purified by preparative separation (preparation method: mobile phase: A: 0.1% formic acid aqueous solution; B: acetonitrile; chromatographic column: Agilent 10 Prep-C18 250 × 21.2 mm; column temperature: 25°C; gradient: 50% to 70% acetonitrile in 12 min; flow rate: 30 mL/min) to obtain the title compound **17** (56.5 mg, yield: 19.8%, containing two pairs of enantiomers).

Compound **17:** LC-MS (ESI): 445.2 [M+H]⁺. ¹H NMR (400 MHz, Chloroform-*d*) δ 9.11 (s, 1H), 8. 11 (br s, 1H), 7.85 (s, 1H), 7.84 - 7.81 (m, 1H), 7.55 - 7.49 (m, 1H), 7.18 - 7.13 (m, 3H), 7.12 - 7.09 (m, 4H), 6.97 - 6.93 (m, 1H), 6.82 - 6.80 (m, 4H), 6.40 (s, 1H), 6.23 (s, 1H), 5.44 (d, *J* = 16 Hz, 1H), 4.95 (d, *J* = 16 Hz, 1H), 4.05 - 3.99 (m, 1H), 3.72 (s, 3H), 3.69 (s, 3H), 1.87 - 1.83 (m, 6H), 1.68 - 1.65 (m, 6H), 1.43 (d, *J =* 12 Hz, 3H), 1.37 (d, *J* = 8 Hz, 3H).

### Example 18: Preparation of compounds 18A and 18B

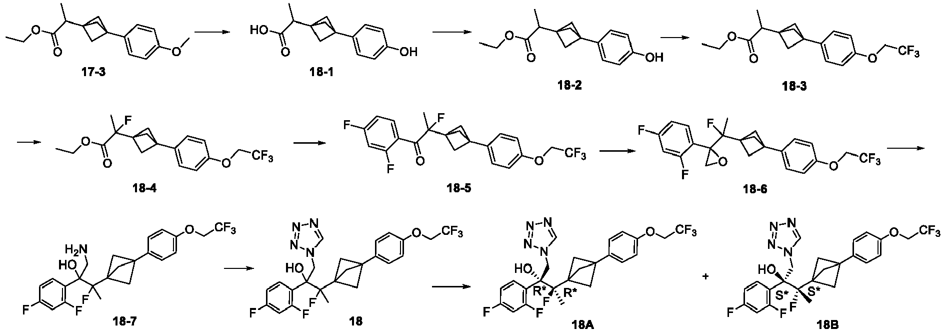

### Preparation of compound 18-1

To a three-necked flask was added compound **17-3** (29 g, 105.8 mmol), and the system was replaced with nitrogen three times. To the three-necked flask were respectively added acetic acid (300 mL) and hydrobromic acid aqueous solution (300 mL). After the reaction system was stirred and reacted at 110°C for 16 hours, the reaction mixture was concentrated to obtain a crude product, which was slurried by adding dichloromethane (100 mL), and filtered to obtain the title compound **18-1** (22 g, yield: 89%) as a brown solid. LC-MS (ESI): 233.2 [M+H]⁺.

### Preparation of compound 18-2

To a three-necked flask was added compound **18-1** (22 g, 94.8 mmol), and the system was replaced with nitrogen three times. To the three-necked flask were sequentially added anhydrous DCM (200 mL) and DMF (2 mL). The reaction system was stirred for 5 min, then oxalyl chloride (12.1 mL, 142.2 mmol) was slowly added dropwise to the three-necked flask, and the reaction mixture was stirred and reacted at room temperature for another 2 hours. The reaction mixture was evaporated to dryness by rotary evaporation, dissolved in DCM (80 mL), then added dropwise to a three-necked flask filled with ethanol (150 mL), and stirred for 2 hours. After the reaction was completed, the reaction system was poured into water (100 mL), extracted with EtOAc (200 mL × 3), and the organic phases were combined, dried, and concentrated to obtain a crude product. The crude product was purified by normal-phase silica gel column chromatography (EtOAc/PE = 0 to 20%) to obtain the title compound **18-2** (18 g, yield: 73%) as a light yellow liquid. LC-MS (ESI): 261.2 [M+H]⁺.

### Preparation of compound 18-3

To a three-necked flask was added compound **18-2** (18 g, 69.2 mmol), then trifluoroethyl trifluoromethanesulfonate (CAS: 6226-25-1, 24.08 g, 103.8 mmol) and potassium carbonate (19.1 g, 138.4 mmol) were added thereto. The system was replaced with nitrogen three times, and then DMF (120 mL) was added thereto. After the reaction system was stirred and reacted at 70°C for 16 hours, the reaction system was added with water (500 mL) and extracted with EtOAc (200 mL × 3), and the organic phases were combined, dried, and concentrated to obtain a crude product. The crude product was purified by normal-phase silica gel column chromatography (EtOAc/PE = 0 to 20%) to obtain the title compound **18-3** (15 g, yield: 63.3%) as a light yellow liquid. LC-MS (ESI): 343.0 [M+H]⁺.

### Preparation of compound 18-4

To a three-necked flask was added compound **18-3** (15 g, 43.8 mmol), and the system was replaced with nitrogen three times. To the three-necked flask was added anhydrous THF (150 mL), and LDA (2.0 M, 44 mL, 88 mmol) was added dropwise thereto at a cooling temperature of -78°C. After the reaction system was stirred and reacted at -78°C for 45 min, a solution of compound N-fluorobenzenesulfonimide (NFSI, 22.6 g, 87.6 mmol) dissolved in anhydrous THF (100 mL) was added dropwise thereto, and the reaction mixture was slowly warmed to room temperature and reacted for 16 hours. After the reaction was completed, the reaction system was quenched with saturated ammonium chloride solution (300 mL), extracted with EtOAc (100 mL × 3), and the organic phases were combined, dried, and concentrated to obtain a crude product. The crude product was purified by normal-phase silica gel column chromatography (EtOAc/PE = 0 to 20%) to obtain the title compound **18-4** (5 g, yield: 31%) as a light yellow liquid. ¹H NMR (400 MHz, Chloroform-*d*) δ 7.18 - 7.15 (m, 2H), 6.96 - 6.86 (m, 2H), 4.36 - 4.27 (m, 2H), 4.30 - 4.24 (m, 2H), 2.16 - 1.93 (m, 6H), 1.57 (d, *J* = 8.0 Hz, 3H), 1.26 (t, *J* = 8.0 Hz, 3H).

### Preparation of compound 18-5

To a three-necked flask was added compound 2,4-difluorobromobenzene (3.0 g, 15.9 mmol), and the system was replaced with nitrogen three times. To the three-necked flask was added anhydrous ether (30 mL), and n-butyllithium (1.6 M, 10 mL, 16.0 mmol) was added dropwise thereto at a cooling temperature of -78°C. After the reaction system was stirred and reacted at -78°C for 45 min, a solution of compound **18-4** (4.8 g, 13.3 mmol) dissolved in anhydrous ether (20 mL) was added dropwise thereto, and the reaction mixture was stirred and reacted for another 1 hour. After the reaction was completed, the reaction system was quenched with saturated ammonium chloride aqueous solution (100 mL), extracted with EtOAc (20 mL × 3), and the organic phases were combined, dried, and concentrated to obtain a crude product. The crude product was purified by normal-phase silica gel column chromatography (EtOAc/PE = 0 to 5%) to obtain the title compound **18-5** (4.6 g, yield: 80%) as a yellow solid. ¹H NMR (400 MHz, Chloroform-*d*) δ 7.59 - 7.53 (m, 1H), 7.17 - 7.13 (m, 2H), 7.07 - 6.96 (m, 1H), 6.95 - 6.91 (m, 3H), 4.32 (q, *J* = 8.0 Hz, 2H), 2.13 - 2.10 (m, 6H), 1.64 (d, *J* = 30 Hz, 3H)

### Preparation of compound 18-6

Compound trimethylsulfonium iodide (8.1 g, 37.3 mmol) was dissolved in a mixed solvent of DMSO (15.0 mL) and THF (10.0 mL), then NaH (purity: 60%, 1.4 g, 37.3 mmol) was added thereto, and the mixture was stirred at room temperature for 1 hour. Then a solution of compound **18-5** (4.5 g, 10.5 mmol) dissolved in DMSO (20.0 mL) was added thereto, and the reaction system was stirred at room temperature for 16 hours. After the reaction was completed, the reaction system was quenched with saturated ammonium chloride aqueous solution (100 mL), extracted with EtOAc (50 mL × 3), and the organic phases were combined, dried, and concentrated to obtain a crude product. The crude product was purified by normal-phase silica gel column chromatography (EtOAc/PE = 0 to 30%) to obtain the title compound **18-6** (1.5 g, yield: 32%, containing a pair of enantiomers) as a yellow oil. ¹H NMR (400 MHz, Chloroform-*d*) δ 7.62 - 7.53 (m, 1H), 7.26 - 7.10 (m, 2H), 6.90 - 6.89 (m, 2H), 6.83 - 6.77 (m, 2H), 4.35 - 4.29 (q, *J* = 8 Hz, 2H), 3.32 (m, 1H), 2.91 - 2.89 (m, 1H), 2.05 - 1.96 (m, 6H), 1.47 (d, *J* = 30 Hz, 3H).

### Preparation of compound 18-7

Compound **18-6** (1.5 g, 3.39 mmol) was dissolved in NH₃/MeOH (7.0 M, 70 mL, 490 mmol), then the reaction mixture was reacted at 80°C for 16 hours in a sealed tube. After the reaction was completed, the reaction mixture was concentrated to obtain a crude product, and the crude product was subjected to a reversed-phase C18 column (ACN/H₂O = 0 to 50%) to obtain the title compound **18-7** (450 mg, yield: 32%, containing a pair of enantiomers) as a yellow oil. LC-MS (ESI): 460.2 [M+H]⁺.

### Preparation of compound 18

Compound **18-7** (450 mg, 0.98 mmol) was dissolved in HOAc solution (3 mL), then sodium acetate (80 mg, 0.98 mmol), trimethyl orthoformate (311 mg, 2.94 mmol), and TMSN₃ (87.4 mg, 4.9 mmol) were added thereto respectively. The reaction system was stirred for 16 hours at 70°C in a sealed tube. After the reaction system was cooled, the reaction mixture was purified by preparative separation (preparation method: mobile phase: A: 0.1% formic acid aqueous solution; B: acetonitrile; chromatographic column: Agilent 10 Prep-C18 250 × 21.2 mm; column temperature: 25°C; gradient: 55% to 75% acetonitrile in 12 min; flow rate: 30 mL/min) to obtain the title compound **18** (380 mg, yield: 75%, containing a pair of enantiomers).

Compound **18:** LC-MS (ESI): 513.2 [M+H]⁺. ¹H NMR (400 MHz, Chloroform-*d*) δ 9.11 (s, 1H), 7.53 - 7.49 (m, 1H), 7.18 - 7.14 (m, 1H), 7.30 - 7.08 (m, 2H), 7.07 - 6.92 (m, 3H), 6.24 (s, 1H), 5.45 (d, *J* = 12 Hz, 1H), 4.95 (d, *J* = 20 Hz, 1H), 4.70 (q, *J* = 8.0 Hz, 2H), 1.92 - 1.89 (m, 3H), 1.87 - 1.85 (m, 3H), 1.66 (d, *J* = 8.0 Hz, 3H). ¹⁹F NMR (376 MHz, DMSO-d6) δ -100.27 (m, 1F), -111.20 (m, 2F), -112.38 (m, 1F), -154.99 (m, 3F).

### Preparation of compounds 18A and 18B

Compound **18** (360 mg, containing a pair of enantiomers) was subjected to SFC chiral preparative resolution (preparative separation method, instrument model: MG II preparative SFC(SFC-14); chromatographic column: ChiralCel OD, 250 × 30 mm I.D., 10 µm; mobile phase: A: CO₂ B: isopropanol (0.1% NH₃H₂O); elution gradient: B 40%; flow rate: 100 mL/min; column pressure: 100 bar; chromatographic column temperature: 35°C; detection wavelength: 220 nM; cycle: about 9.5 min) to obtain the title compound **18A** (164 mg, single enantiomer) and the title compound **18B** (167 mg, single enantiomer).

Compound **18A:** LC-MS (ESI): 513.2 [M+H]⁺. Chiral resolution (chromatographic column: Chiralpak OD-3 150 × 4.6 mm I.D., 3 µm; mobile phase: A: CO₂ B: isopropanol (0.05% DEA); elution gradient: the mobile phase increased from 5% B to 40% B within 5 min and maintained at 40% B for 2.5 min for elution, then 5% B was equilibrated for 2.5 min flow rate; chromatographic column temperature: 35°C; column pressure: 1500 psi; detection wavelength: 220 nM; R_{T} = 2.464 min). ¹H NMR (400 MHz, Chloroform-*d*) δ 9.10 (s, 1H), 7.55 - 7.49 (m, 1H), 7.18 - 7.12 (m, 1H), 7.06 - 7.04 (m, 2H), 6.97 - 6.92 (m, 3H), 6.38 (s, 1H), 5.45 (d, *J =* 16 Hz, 1H), 4.95 (q, *J* = 16 Hz, 1H), 4.70 (q, *J* = 8 Hz, 2H), 1.92 - 1.89 (m, 3H), 1.70 - 1.68 (m, 3H), 1.54 (d, *J* = 20 Hz, 3H). ¹⁹F NMR (376 MHz, DMSO-d6) δ -100.45 (m, 1F), -111.24 (m, 3F), -155.15 (m, 3F).

Compound **18B:** LC-MS (ESI): 513.2 [M+H]⁺. Chiral resolution (chromatographic column: Chiralpak OD-3 150 × 4.6 mm I.D., 3 µm; mobile phase: A: CO₂ B: isopropanol (0.05% DEA); elution gradient: the mobile phase increased from 5% B to 40% B within 5 min and maintained at 40% B for 2.5 min for elution, then 5% B was equilibrated for 2.5 min; flow rate: 2.5 mL/min; chromatographic column temperature: 35°C; column pressure: 1500 psi; detection wavelength: 220 nM; R_{T} = 3.509 min).¹H NMR (400 MHz, Chloroform-*d*) δ 9.11 (s, 1H), 7.55 - 7.49 (m, 1H), 7.18 - 7.16 (m, 1H), 7.15 - 7.07 (m, 2H), 6.97 - 6.92 (m, 3H), 6.38 (s, 1H), 5.45 (d, *J* = 16 Hz, 1H), 4.95 (d, *J* = 16 Hz, 1H), 4.69 (q, *J* = 8 Hz, 2H), 1.92 - 1.89 (m, 3H), 1.70 - 1.68 (m, 3H), 1.54 (d, *J* = 20 Hz, 3H). ¹⁹F NMR (376 MHz, DMSO-d6) δ -100.43 (m, 1F), -111.25 (m, 3F), -155.05 (m, 3F).

### Example 19: Preparation of compound 19

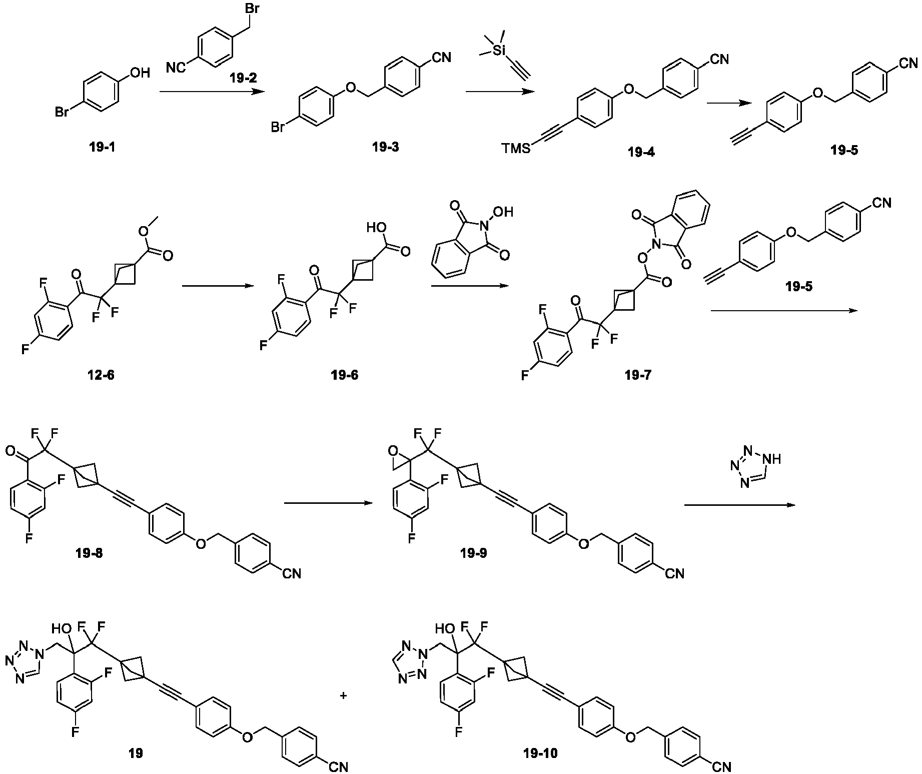

### Preparation of compound 19-3

To a reaction flask were sequentially added compound **19-1** (2.9 g, 14.45 mmol), solvent DMF (20 mL), K₂CO₃ (4.0 g, 28.9 mmol), and compound **19-2** (3.4 g, 17.34 mmol) at room temperature, and the reaction system was reacted at 80°C for 3 hours. After the reaction was completed, the reaction mixture was cooled to room temperature, added with ice water (5 mL), extracted with EtOAc (20 mL × 3), and the organic phase was dried, filtered, concentrated, and then purified by normal-phase column chromatography (petroleum ether/ethyl acetate = 0 to 100%) to obtain the title compound **19-3** (2.9 g, yield: 71%) as a colorless liquid. ¹H NMR (400 MHz, CDCl₃): δ 7.69 (d, *J* = 8.4 Hz, 2H), 7.54 (d, *J* = 8.4 Hz, 2H), 7.42 (d, *J* = 9.2 Hz, 2H), 6.88 (d, *J* = 9.2 Hz, 2H), 5.12 (s, 2H).

### Preparation of compound 19-4

To a reaction flask were sequentially added compound **19-3** (2.9 g, 10.1 mmol), trimethylsilylacetylene (3.95 g, 40.3 mmol), TEA (5 mL), DMF (15 mL), Pd(PPh₃)₂Cl₂ (0.7 g, 1.0 mmol), and CuI (95.3 mg, 0.5 mmol), and the system was replaced with nitrogen three times. The reaction mixture was reacted at 80°C for 15 hours, cooled to room temperature, added with ice water (5 mL), and extracted with EtOAc (20 mL × 3). The organic phase was dried, filtered, concentrated, and then purified by normal-phase column chromatography (petroleum ether/ethyl acetate = 0% to 100%) obtain the title compound **19-4** (1.66 g, yield: 54%) as a colorless liquid. ¹H NMR (400 MHz, CDCl₃): δ 7.68 (d, *J* = 8.4 Hz, 2H), 7.52 (d, *J* = 8.4 Hz, 2H), 7.39 (d, *J* = 9.2 Hz, 2H), 6.83 (d, *J* = 9.2 Hz, 2H), 5.09 (s, 2H), 0.24 (s, 9H).

### Preparation of compound 19-5

Compound **19-4** (4.0 g, 13.1 mmol) was dissolved in a mixture of THF (40 mL) and MeOH (40 mL) at room temperature, and K₂CO₃ (18.1 g, 131 mmol) was added thereto. The reaction system was stirred at room temperature for 6 hours, added with ice water (5 mL), extracted with EtOAc (20 mL × 3), dried and concentrated, and then purified by normal-phase column chromatography (petroleum ether/ethyl acetate = 0 to 100%) to obtain the title compound **19-5** (1.8 g, yield: 59%) as a colorless solid. ¹H NMR (400 MHz, CDCl₃): δ 7.69 (d, *J* = 8.4 Hz, 2H), 7.54 (d, *J* = 8.4 Hz, 2H), 7.45 (d, *J* = 9.2 Hz, 2H), 6.90 (d, *J* = 9.2 Hz, 2H), 5.13 (s, 2H), 3.00 (s, 1H).

### Preparation of compound 19-6

Compound **12-6** (316 mg, 1.0 mmol) was dissolved in a mixed solvent of THF (6 mL) and H₂O (2 mL) at room temperature, and LiOH (48 mg, 2 mmol) was added thereto. The reaction system was reacted at room temperature for 16 hours. After the reaction was completed, the pH of the reaction system was adjusted to about 3 with hydrochloric acid aqueous solution (1.0 M), and the mixture was extracted with DCM (20 mL × 3). The organic phases were combined, dried, concentrated, and then purified by normal-phase column chromatography (petroleum ether/ethyl acetate = 0 to 100%) to obtain the title compound **19-6** (0.23 g, yield: 76%). LC-MS (ESI): 303.0 [M+H]⁺.

### Preparation of compound 19-7

Compound **19-6** (0.23 g, 0.76 mmol) was dissolved in DCM (20 mL) at room temperature, and compounds N-hydroxyphthalimide (124 mg, 0.76 mmol), DMAP (9.3 mg, 0.076 mmol), and N,N'-diisopropylcarbodiimide (106 mg, 0.84 mmol) were added thereto respectively. The reaction system was reacted under nitrogen atmosphere for 16 hours. After the reaction was completed, the reaction mixture was added with ice water (5 mL) to quench the reaction, extracted with EtOAc (20 mL × 3), dried, concentrated, and then purified by normal-phase column chromatography (petroleum ether/ethyl acetate = 0 to 100%) to obtain the title compound **19-7** (0.17 g, yield: 50%) as a colorless solid. ¹H NMR (400 MHz, CDCl₃): δ 7.91 - 7.79 (m, 5H), 7.04 - 6.91 (m, 2H), 2.53 (s, 6H).

### Preparation of compound 19-8

To a reaction flask were respectively added compound CuCl (0.85 mg, 0.0086 mmol), Cu(acac)₂ (2.25 mg, 0.0086 mmol), and compound **19-7** (50 mg, 0.11 mmol) at room temperature, then THF (5 mL) solvent was added thereto, and the system was replaced with nitrogen three times. Then compound **19-5** (20 mg, 0.086 mmol) and TEA (21.8 mg, 0.215 mmol) were sequentially added thereto, and the system was replaced with nitrogen three times again, and the reaction mixture was reacted for 20 hours under the irradiation of Blue LED lamp. After the reaction was completed, the reaction system was mixed with silica gel, and purified by normal-phase column chromatography (petroleum ether/ethyl acetate = 0 to 100%) to obtain the title compound **19-8** (5 mg, yield: 11.9 %). ¹H NMR (400 MHz, CDCl₃): δ 7.85 - 7.82 (m, 1H), 7.59 - 7.57 (m, 2H), 7.54 - 7.52 (m, 2H), 7.36 - 7.34 (m, 2H), 7.01 - 6.85 (m, 4H), 5.12 (s, 2H), 2.31 (s, 6H).

### Preparation of compound 19-9

To a mixed solvent of THF (5 mL) and DMSO (3 mL) were respectively added compound trimethylsulfoxonium iodide (24.2 mg, 0.11 mmol) and reagent potassium tert-butoxide (12.1 mg, 0.11 mmol) at room temperature. After stirring at room temperature for 1 hour, the reaction system was cooled to 0°C. Compound **19-8** (50 mg, 0.10 mmol) was added thereto, and the reaction mixture was reacted and stirred at 60°C for 12 hours. The reaction mixture was added with ice water (5 mL) to quench the reaction, extracted with EtOAc (20 mL × 3), washed with water, washed with saturated brine, dried, filtered, concentrated, and then purified by normal-phase column chromatography (petroleum ether/ethyl acetate = 0 to 100%) to obtain the title compound **19-9** (45 mg, yield: 87.1%) as a pale yellow liquid. ¹H NMR (400 MHz, CDCl₃): δ 7.68 - 7.36 (m, 2H), 7.55 - 7.51 (m, 3H), 7.34 - 7.32 (m, 2H), 6.92 - 6.84 (m, 4H), 5.10 (s, 2H), 3.36 (d, *J =* 4 Hz, 1H), 2.91 (d, *J =* 4 Hz, 1H), 2.18 - 2.12 (m, 6H).

### Preparation of compound 19

To DMF (3 mL) solvent was added compound **19-9** (40 mg, 0.08 mmol) at room temperature, and 1H-tetrazole (7.23 mg, 0.10 mmol) and potassium carbonate (13.1 mg, 0.095 mmol) were added thereto, respectively. The reaction mixture was reacted and stirred at 80°C for 16 hours. The reaction mixture was added with ice water (5 mL) to quench the reaction, extracted with EtOAc (20 mL × 3), dried, concentrated, and prepared by reversed-phase column chromatography to obtain the title compound **19** (3.25 mg) and its regioisomer compound **19-10** (1.98 mg).

**Compound 19:** LC-MS: m/z 574.3 [M+H]⁺. ¹H NMR (400 MHz, CD₃OD) δ 8.89 (s, 1H), 7.74 - 7.72 (m, 2H), 7.61 - 7.59 (m, 3H), 7.26 - 7.24 (m, 2H), 6.93 - 6.90 (m, 4H), 5.55 (d, *J* = 12 Hz, 1H), 5.17 (s, 2H), 4.95 (d, *J* = 12 Hz, 1H), 2.10 (dd, *J* = 9.2, 1.6 Hz, 3H), 1.89 (dd, *J* = 9.2, 1.6 Hz, 3H).

**Compound 19-10:** LC-MS: m/z 574.3 [M+H]⁺. ¹H NMR (400 MHz, CD₃OD) δ 8.46 (s, 1H), 7.14 - 7.12 (m, 2H), 7.63 - 7.58 (m, 3H), 7.26 - 7.24 (m, 2H), 6.97 - 6.89 (m, 4H), 5.70 (d, *J* = 14.4 Hz, 1H), 5.28 (d, *J* = 14.4 Hz, 1H), 5.18 (s, 2H), 2.08 (dd, *J* = 9.2, 1.6 Hz, 3H), 1.87 (dd, *J* = 9.2, 1.6 Hz, 3H).

### Example 20: Preparation of compounds 20A and 20B

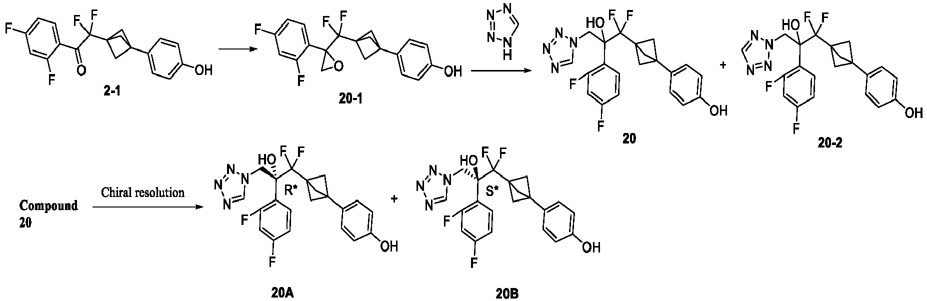

### Preparation of compound 20-1

Compound **2-1** (1.2 g, 3.43 mmol) was dissolved in a mixed solvent of dichloromethane (15 mL) and water (15 mL), then trimethylsulfoxonium iodide (3.02 g, 13.7 mmol) and sodium hydroxide (549 mg, 13.7 mmol) were added thereto, and the reaction system was refluxed for 16 hours. After cooling to room temperature, the pH of the reaction system was adjusted to between 6 and 7 with dilute hydrochloric acid, and the mixture was extracted with DCM (20 mL × 3). The organic phases were combined, dried, and concentrated to obtain a crude product. The crude product was purified by normal-phase silica gel column chromatography (EtOAc/PE = 0 to 10%) to obtain the title compound **20-1** (1.17 g, yield: 94%) as a light yellow oil. LC-MS (ESI): m/z 363.0 [M-H]⁻. ¹H NMR (400 MHz, DMSO-*d6*) δ 9.34 (s, 1H), 7.67 (td, *J* = 8.5, 6.5 Hz, 1H), 7.35 (ddd, *J* = 10.5, 9.3, 2.6 Hz, 1H), 7.16 (tdd, *J* = 8.5, 2.6, 0.9 Hz, 1H), 7.02 - 6.93 (m, 2H), 6.74 - 6.63 (m, 2H), 3.40 - 3.36 (m, 1H), 3.11 - 3.05 (m, 1H), 2.03 - 1.94 (m, 6H). ¹⁹F NMR (376 MHz, DMSO-*d6*) δ -107.77 - -107.88 (m, 1F), -108.07 - -108.22 (m, 1F), -108.71 - -108.81 (m, 1F), -108.93 - -109.06 (m, 1F).

### Preparation of compound 20

Compound **20-1** (1.17 g, 3.21 mmol) was dissolved in DMF solution (10 mL), and compound 1H-tetrazole (225 mg, 12.9 mmol) and potassium carbonate (444 mg, 12.9 mmol) were added thereto respectively. The reaction system was stirred for 16 hours at 80°C in a sealed tube. After the reaction system was cooled, the reaction mixture was filtered, and the crude filtrate was purified by preparative separation (preparation method: mobile phase: A: 0.1% formic acid aqueous solution; B: acetonitrile; chromatographic column: Agilent 10 Prep-C18 250 × 21.2 mm; column temperature: 25°C; gradient: 40% to 60% acetonitrile in 12 min; flow rate: 30 mL/min) to obtain the title compound **20** (500 mg, yield: 36%, containing a pair of enantiomers) and its corresponding regioisomer compound **20-2** (200 mg, yield: 14%, containing a pair of enantiomers).

Compound **20:** LC-MS (ESI): m/z 435.2 [M+H]⁺. ¹H NMR (400 MHz, DMSO-d6) δ 9.31 (br.s, 1H), 9.14 (s, 1H), 7.53 (m, 1H), 7.28 (m, 1H), 7.16 (s, 1H), 7.00 (m, 1H), 6.96 - 6.84 (m, 2H), 6.67 - 6.58 (m, 2H), 5.42 (d, *J* = 12 Hz, 1H), 5.01 (d, *J* = 12 Hz, 1H), 1.94 (dd, *J* = 9.5, 1.7 Hz, 3H), 1.72 (dd, *J* = 9.5, 1.7 Hz, 3H). ¹⁹F NMR (376 MHz, DMSO-*d*6) δ - 102.85 - -103.32 (m, 1F), -109.01 - -109.42 (m, 2F), -109.67 (d, *J* = 9.2 Hz, 1F).

Compound **20-2:** LC-MS (ESI): m/z 435.2 [M+H]⁺. ¹H NMR (400 MHz, DMSO-*d6)* δ 9.30 (s, 1H), 8.76 (s, 1H), 7.56 (m, 1H), 7.26 (m, 1H), 7.18 (s, 1H), 7.00 (m, 1H), 6.95 - 6.85 (m, 2H), 6.69 - 6.55 (m, 2H), 5.42 (d, *J* = 14.2 Hz, 1H), 5.23 (d, *J* = 14.2 Hz, 1H), 1.93 (dd, *J* = 9.5, 1.7 Hz, 3H), 1.71 (dd, *J* = 9.5, 1.7 Hz, 3H). ¹⁹F NMR (376 MHz, DMSO-d6) δ -102.38 - -102.95 (m, 1F), -108.69 - -109.22 (m, 2F), -110.02 (d, *J* = 9.6 Hz, 1F).

### Preparation of compounds 20A and 20B

Compound **20** (100 mg) was subjected to SFC chiral preparative resolution (preparative separation method, instrument model: MG II preparative SFC(SFC-14); model of chromatographic column: ChiralPak AD, 250 × 30 mm I.D., 10 µm; mobile phase: A: CO₂ B: ethanol; elution gradient: B 20%; flow rate: 60 mL/min; column pressure: 100 bar; column temperature: 38°C; detection wavelength: 220 nm; cycle: about 5 min) to obtain the title compounds **20A** (48 mg, single enantiomer) and **20B** (46 mg, single enantiomer).

Compound **20A:** LC-MS (ESI): 435.0 [M+H]⁺. ¹H NMR (400 MHz, DMSO-d6) δ 9.30 (br.s, 1H), 9.13 (s, 1H), 7.53 (m, 1H), 7.28 (m, 1H), 7.25 - 7.15 (br.s, 1H), 7.00 (m, 1H), 6.96 - 6.84 (m, 2H), 6.71 - 6.55 (m, 2H), 5.42 (d, *J* = 12 Hz, 1H), 5.01 (d, *J* = 12 Hz, 1H), 1.94 (m, 3H), 1.72 (m, 3H). ¹⁹F NMR (376 MHz, DMSO-*d6*) δ -102.97 - -103.19 (m, 1F), -109.17 - -109.27 (m, 2F), -109.67 (d, *J* = 8.9 Hz, 1F).

Compound **20B:** LC-MS (ESI): 435.0 [M+H]⁺. ¹H NMR (400 MHz, DMSO-d6) δ 9.30 (br.s, 1H), 9.14 (s, 1H), 7.53 (m, 1H), 7.28 (m, 1H), 7.25 - 7.14 (br.s, 1H), 7.00 (m, 1H), 6.95 - 6.84 (m, 2H), 6.70 - 6.57 (m, 2H), 5.41 (d, *J* = 14.6 Hz, 1H), 5.01 (d, *J* = 14.6 Hz, 1H), 1.94 (dd, *J* = 9.5, 1.7 Hz, 3H), 1.72 (dd, *J* = 9.5, 1.7 Hz, 3H). ¹⁹F NMR (376 MHz, DMSO-d6) δ -102.97 - -103.19 (m, 1F), -109.18 - -109.28 (m, 2F), -109.68 (d, *J* = 9.5 Hz, 1F).

### Example 21: Preparation of compounds 21A and 21B

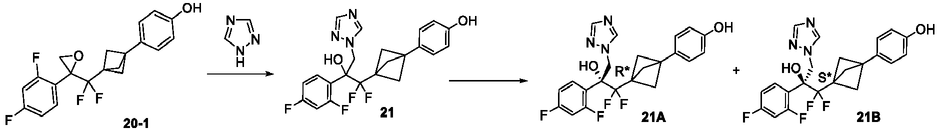

### Preparation of compound 21

1,2,4-Triazole (415 mg, 6 mmol) was dissolved in DMF (6 mL), and NaH (240 mg, 6 mmol) was added thereto at 0°C. After the reaction mixture was reacted for 10 min, **20-1** (364 mg, 1 mmol) was added thereto, and the reaction system was reacted at 70°C for 16 hours in a sealed tube. After the reaction system was cooled, the reaction mixture was filtered, and the crude filtrate was purified by preparative separation (chromatographic column: Welch Xtimate@ C18 21.2 × 250 mm; column temperature: 25°C; mobile phase: water (10 mM/L NH₄HCO₃)-acetonitrile; proportion of acetonitrile in the mobile phase: 55% to 75% in 12 min; flow rate: 30 mL/min) to obtain the title compound **21** (194 mg, yield: 45%, containing a pair of enantiomers). LC-MS (ESI): 434.0 [M+H]+. ¹H NMR (400 MHz, Chloroform-J) δ 8.14 (br.s, 1H), 7.84 (s, 1H), 7.74 (td, *J* = 9.0, 6.5 Hz, 1H), 7.04 - 6.93 (m, 2H), 6.88 - 6.62 (m, 4H), 5.28 (br.s, 1H), 5.21 (d, *J* = 14.2 Hz, 1H), 4.84 (d, *J* = 14.2 Hz, 1H), 2.05 (dd, *J* = 9.5, 1.8 Hz, 3H), 1.89 (dd, *J* = 9.5, 1.8 Hz, 3H).

### Preparation of compounds 21A and 21B

Compound **21** (115 mg) was subjected to SFC chiral preparative resolution (preparative separation method, instrument model: MG II preparative SFC (SFC-14); chromatographic column: ChiralPak AD, 250 × 30 mm I.D., 10 µm; mobile phase: A: CO₂ B: methanol; elution gradient: B 35%; flow rate: 80 mL/min; column pressure: 100 bar; chromatographic column temperature: 38°C; detection wavelength: 220 nm; cycle: about 3 min) to obtain the title compounds **21A** (36 mg, single enantiomer) and **21B** (40 mg, single enantiomer).

Compound **21A:** LC-MS (ESI): 434.2 [M+H]⁺. Chiral analysis method (chromatographic column: Chiralpak AD-3 150 × 4.6 mm I.D., 3 µm; mobile phase: A: CO₂ B: methanol (0.05% DEA); elution gradient: B 40%; flow rate: 2.5 mL/min; chromatographic column temperature: 35°C; column pressure: 1500 psi; detection wavelength: 220 nM; RT = 1.262 min). ¹H NMR (400 MHz, Chloroform-*d*) δ 8.05 (s, 1H), 7.83 (s, 1H), 7.74 (td, *J* = 9.0, 6.5 Hz, 1H), 7.06 - 6.91 (m, 2H), 6.91 - 6.80 (m, 1H), 6.80 - 6.68 (m, 2H), 5.29 - 5.15 (m, 2H), 4.82 (d, *J* = 14.2 Hz, 1H), 2.05 (dd, *J* = 9.4, 1.7 Hz, 3H), 1.97 - 1.83 (m, 3H). ¹⁹F NMR (376 MHz, Chloroform-*d*) δ -105.26 (d, *J* = 42.5 Hz, 1F), -108.38 (d, *J* = 13.8 Hz, 1F), -110.73 (d, *J* = 45.7 Hz, 1F), -111.11 (d, *J* = 13.4 Hz, 1F).

Compound **21B:** LC-MS (ESI): 434.2 [M+H]⁺. Chiral analysis method (chromatographic column: Chiralpak AD-3 150 × 4.6 mm I.D., 3 µm; mobile phase: A: CO₂ B: methanol (0.05% DEA); elution gradient: B 40%; flow rate: 2.5 mL/min; chromatographic column temperature: 35°C; column pressure: 1500 psi; detection wavelength: 220 nM; RT = 0.969 min). ¹H NMR (400 MHz, Chloroform-d) δ 8.03 (s, 1H), 7.77 (s, 1H), 7.67 (td, *J* = 9.0, 6.5 Hz, 1H), 6.96 - 6.86 (m, 2H), 6.82 - 6.69 (m, 1H), 6.69 - 6.62 (m, 2H), 5.13 (d, *J* = 14.4 Hz, 2H), 4.76 (d, *J* = 14.2 Hz, 1H), 1.98 (dd, *J* = 9.4, 1.8 Hz, 3H), 1.83 (dd, *J* = 9.5, 1.8 Hz, 3H). ¹⁹F NMR (376 MHz, Chloroform-*d*) δ -105.20 (d, *J* = 49.2 Hz, 1F), -108.36 (d, *J* = 13.8 Hz, 1F), -110.73 (d, *J* = 46.3 Hz, 1F), -111.08 (d, *J* = 12.9 Hz, 1F).

### Example 22: Preparation of compounds 22A and 22B

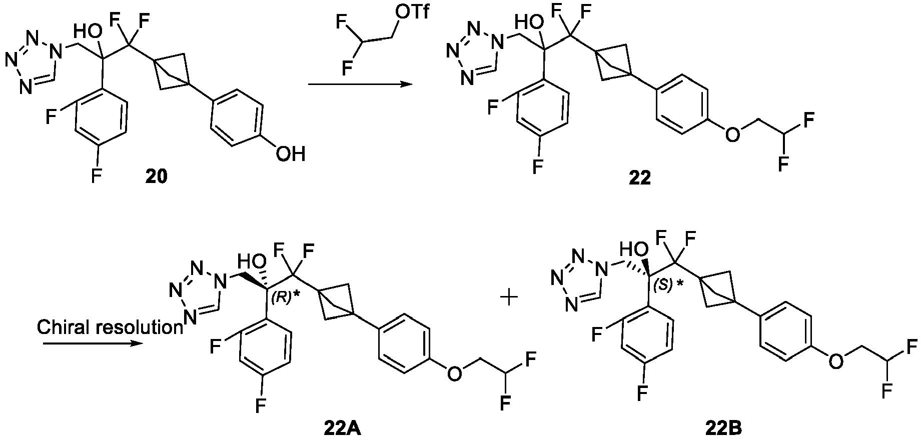

### Preparation of compound 22

Compound **20** (100 mg, 0.23 mmol) was dissolved in DMF solution (1.5 mL), and compound 2,2-difluoroethyl trifluoromethanesulfonate (247 mg, 1.15 mmol) and potassium carbonate (159 mg, 1.15 mmol) were added thereto. The reaction system was stirred for 16 hours at 80°C in a sealed tube. After the reaction system was cooled, the reaction mixture was filtered, and the crude filtrate was purified by preparative separation (chromatographic column: Welch Xtimate@ C18 21.2 × 250 mm; column temperature: 25°C; mobile phase: water (10 mM/L NH₄HCO₃)-acetonitrile; proportion of acetonitrile in the mobile phase: 50% to 70% in 12 min; flow rate: 30 mL/min) to obtain the title compound **22** (35 mg, yield: 30%, containing a pair of enantiomers).

Compound **22:** LC-MS (ESI): m/z 499.2 [M+H]⁺. ¹H NMR (400 MHz, DMSO-d6) δ 9.14 (s, 1H), 7.69 - 7.47 (m, 1H), 7.36 - 7.24 (m, 1H), 7.16 (s, 1H), 7.10 - 6.97 (m, 3H), 6.97 - 6.84 (m, 2H), 6.35 (tt, *J* = 56, 4 Hz, 1H), 5.43 (d, *J* = 14.5 Hz, 1H), 5.01 (d, *J* = 14.6 Hz, 1H), 4.25 (dt, *J* = 16, 4 Hz, 2H), 2.00 - 1.96 (m, 3H), 1.83 - 1.70 (m, 3H). ¹⁹F NMR (376 MHz, DMSO-*d6*) δ -102.51 - -103.44 (m, 1F), -108.76 - -109.48 (m, 1F), -109.38 - -110.30 (m, 2F), -125.74 (s, 2F).

### Preparation of compounds 22A and 22B

Compound **22** (100 mg) was subjected to SFC chiral preparative resolution (preparative separation method, instrument model: MG II preparative SFC (SFC-1); model of chromatographic column: Cellulose-2, 250 × 30 mm I.D., 5 µm; mobile phase: A: CO₂ B: ethanol; elution gradient: B 30%; flow rate: 70 mL/min; column pressure: 100 bar; column temperature: 38°C; detection wavelength: 220 nm; cycle: about 4.33 min) to obtain the title compounds **22A** (45 mg) and **22B** (43 mg).

Compound **22A:** Chiral analysis method (model of chromatographic column: Cellulose-2, 150 × 4.6 mm I.D., 3 µm; mobile phase: A: CO₂ B: ethanol (0.05% DEA); elution gradient: B 40%; flow rate: 2.5 mL/min; column temperature: 35°C; column pressure: 100 bar; detection wavelength: 220 nm; Rt = 1.465 min). LC-MS (ESI): m/z 499.2 [M+H]⁺. ¹H NMR (400 MHz, DMSO-*d6*) δ 9.14 (s, 1H), 7.69 - 7.47 (m, 1H), 7.36 - 7.24 (m, 1H), 7.16 (s, 1H), 7.10 - 6.97 (m, 3H), 6.97 - 6.84 (m, 2H), 6.54 - 6.12 (m, 1H), 5.43 (d, *J =* 14.5 Hz, 1H), 5.01 (d, J = 14.6 Hz, 1H), 4.38 - 4.13 (m, 2H), 2.00 - 1.96 (m, 3H), 1.83 - 1.70 (m, 3H). ¹⁹F NMR (376 MHz, DMSO-*d6*) δ -102.51 - -103.44 (m, 1F), -109.19 - -109.64 (m, 3F), -125.74 (s, 2F).

Compound **22B:** Chiral analysis method (model of chromatographic column: Cellulose-2, 150 × 4.6 mm I.D., 3 µm; mobile phase: A: CO₂ B: ethanol (0.05% DEA); elution gradient: B 40%; flow rate: 2.5 mL/min; column temperature: 35°C; column pressure: 100 bar; detection wavelength: 220 nm; Rt = 1.134 min). LC-MS (ESI): m/z 499.2 [M+H]⁺. ¹H NMR (400 MHz, DMSO-d6) δ 9.14 (s, 1H), 7.69 - 7.47 (m, 1H), 7.36 - 7.24 (m, 1H), 7.16 (s, 1H), 7.10 - 6.97 (m, 3H), 6.97 - 6.84 (m, 2H), 6.54 - 6.12 (m, 1H), 5.43 (d, *J* = 14.5 Hz, 1H), 5.01 (d, J = 14.6 Hz, 1H), 4.38 - 4.13 (m, 2H), 2.00 - 1.96 (m, 3H), 1.83 - 1.70 (m, 3H). ¹⁹F NMR (376 MHz, DMSO-d6) δ -102.95 - -103.16 (m, 1F), -109.20 - -109.64 (m, 3F), -125.73 (s, 2F).

### Example 23: Preparation of compound 23

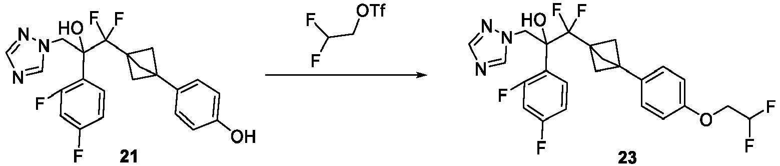

### Preparation of compound 23

Compound **21** (100 mg, 0.23 mmol) was dissolved in DMF solution (1.5 mL), and compound 2,2-difluoroethyl trifluoromethanesulfonate (247 mg, 1.15 mmol) and potassium carbonate (159 mg, 1.15 mmol) were added thereto. The reaction system was stirred for 16 hours at 80°C in a sealed tube. After the reaction system was cooled, the reaction mixture was filtered, and the crude filtrate was purified by preparative separation (preparation method: mobile phase: A: 0.1% formic acid aqueous solution; B: acetonitrile; chromatographic column: Agilent 10 Prep-C18 250 × 21.2 mm; column temperature: 25°C; gradient: 50% to 70% acetonitrile in 12 min; flow rate: 30 mL/min) to obtain the title compound **23** (20 mg, yield: 17%, containing a pair of enantiomers).

Compound **23:** LC-MS (ESI): m/z 498.2 [M+H]⁺. ¹H NMR (400 MHz, DMSO-d6) δ 8.36 (s, 1H), 7.69 (s, 1H), 7.60 - 7.48 (m, 1H), 7.24 - 7.14 (m, 1H), 7.11 - 7.01 (m, 2H), 7.00 - 6.95 (m, 1H), 6.90 (d, *J* = 8.2 Hz, 3H), 6.35 (tt, *J* = 56, 4 Hz, 1H), 5.14 (d, *J =* 14.4 Hz, 1H), 4.76 (d, *J* = 14.4 Hz, 1H), 4.24 (dt, *J* = 16, 4 Hz, 2H), 2.00 - 1.93 (m, 3H), 1.78 - 1.72 (m, 3H). ¹⁹F NMR (376 MHz, DMSO-d6) δ -102.34 - -102.94 (m, 1F), -109.33 - -109.70 (m, 2F), - 110.34 - -110.60 (m, 1F), -125.73 (s, 2F).

### Example 24: Preparation of compound 24

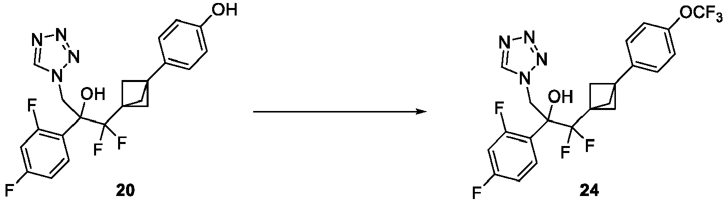

### Preparation of compound 24

To a three-necked flask were respectively added compound **20** (60 mg, 0.14 mmol), silver trifluoromethanesulfonate (177 mg, 0.69 mmol), selective fluorine reagent (SelectFluor, 98 mg, 0.276 mmol), N-fluorobenzenesulfonimide (NFSI, 87 mg, 0.28 mmol), and cesium fluoride (126 mg, 0.83 mmol), and the system was replaced with argon three times. Then trifluorotoluene (5 mL), toluene (2.5 mL), 2-fluoropyridine (67 mg, 0.69 mmol), and (trifluoromethyl)trimethylsilane (98 mg, 0.69 mmol) were added thereto. The reaction system was stirred at room temperature for 16 hours. The reaction mixture was filtered, and the crude filtrate was purified by preparative separation (preparation method: mobile phase: A: 0.1% formic acid aqueous solution; B: acetonitrile; chromatographic column: Agilent 10 Prep-C18 250 × 21.2 mm; column temperature: 25°C; gradient: 55% to 75% acetonitrile in 12 min; flow rate: 30 mL/min) to obtain the title compound **24** (10 mg, yield: 16.0%, containing a pair of enantiomers).

Compound **24:** LC-MS (ESI): m/z 503.0 [M+H]⁺. ¹H NMR (400 MHz, DMSO-d6) δ 9.16 (s, 1H), 7.60 - 7.53 (m, 1H), 7.34 - 7.30 (m, 1H), 7.29 - 7.27 (m, 4H), 7.20 (s, 1H), 7.05 - 7.00 (m, 1H), 5.45 (d, *J* = 14.6 Hz, 1H), 5.04 (d, *J* = 14.6 Hz, 1H), 2.06 (dd, *J* = 9.6, 1.8 Hz, 3H), 1.85 (dd, *J* = 9.6, 1.8 Hz, 3H).

### Example 25: Preparation of compound 25

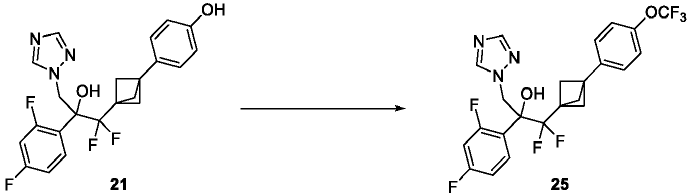

### Preparation of compound 25

To a three-necked flask were respectively added compound **21** (50 mg, 0.137 mmol), silver trifluoromethanesulfonate (70 mg, 0.274 mmol), selective fluorine reagent (SelectFluor, 121 mg, 0.343 mmol), N-fluorobenzenesulfonimide (NFSI, 108 mg, 0.343 mmol), and cesium fluoride (104 mg, 0.686 mmol), and the system was replaced with argon three times. Then trifluorotoluene (5 mL), toluene (2.5 mL), 2-fluoropyridine (66 mg, 0.686 mmol), and (trifluoromethyl)trimethylsilane (97 mg, 0.686 mmol) were added thereto. The reaction system was stirred at room temperature for 16 hours. The reaction mixture was filtered, and the crude filtrate was purified by preparative separation (chromatographic column: Welch Xtimate@ C18 21.2 × 250 mm; column temperature: 25°C; mobile phase: water (10 mM/L NH₄HCO₃)-acetonitrile; proportion of acetonitrile in the mobile phase: 55% to 75% in 12 min; flow rate: 30 mL/min) to obtain the title compound **25** (3.5 mg, containing a pair of enantiomers).

Compound **25:** ¹H NMR (400 MHz, DMSO-d6) δ 8.37 (s, 1H), 7.69 (s, 1H), 7.60 - 7.53 (m, 1H), 7.27 - 7.17 (m, 5H), 7.00 - 6.97 (m, 1H), 6.92 (s, 1H), 5.14 (d, *J* = 14.4 Hz, 1H), 4.77 (d, *J* = 14.5 Hz, 1H), 2.04 - 2.01 (m, 3H), 1.82 - 1.80 (m, 3H). ¹⁹F NMR (376 MHz, DMSO-d6) δ -56.80 (s, 3F), -102.51 - -102.62 (m, 1F), -109.55--109.66 (m, 2F), -110.40 (d, *J* = 9.6 Hz, 1F).

### Example 26: Preparation of compound 26

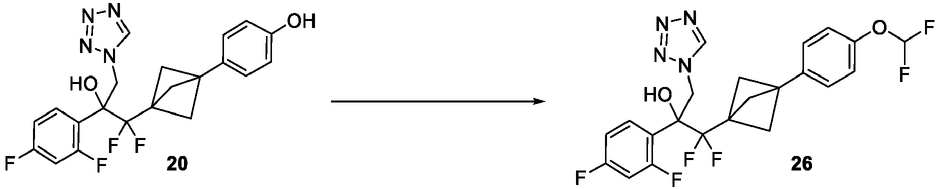

### Preparation of compound 26

Compound **20** (100 mg, 0.23 mmol) was dissolved in THF (3.0 mL), and NaH (19 mg, 0.46 mmol) was added thereto at 0°C. After 5 min, ethyl 2-bromo-2,2-difluoroacetate (140 mg, 0.69 mmol) was added thereto, and the reaction mixture was reacted at room temperature for 16 hours in a sealed tube. The reaction mixture was filtered, and the crude filtrate was purified by preparative separation (preparation method: chromatographic column: Welch Xtimate@ C18 21.2 × 250 mm; column temperature: 25°C; mobile phase: water (10 mM/L NH₄HCO₃)-acetonitrile; proportion of acetonitrile in the mobile phase: 45% to 75% in 12 min; flow rate: 30 mL/min) to obtain the title compound **26** (4 mg, yield: 3.6%).

Compound **26:** LC-MS: 485.2 [M+H]⁺. ¹H NMR (400 MHz, Chloroform-*d*) δ 8.53 (s, 1H), 7.65 (td, *J* = 8.9, 6.3 Hz, 1H), 7.11 - 7.05 (m, 2H), 7.02 (d, *J* = 8.6 Hz, 2H), 6.94 - 6.76 (m, 2H), 6.45 (t, *J* = 73.9 Hz, 1H), 5.44 (d, *J* = 14.6 Hz, 1H), 5.05 (d, *J* = 14.6 Hz, 1H), 3.96 (s, 1H), 2.06 (dd, *J* = 9.4, 1.9 Hz, 3H), 1.87 (dd, *J* = 9.4, 1.9 Hz, 3H). ¹⁹F NMR (376 MHz, Chloroform-*d*) δ -80.80 (s, 2F), -106.83 (d, *J* = 9.5 Hz, 1F), -110.19 - -110.31 (m, 2F), - 110.44 - -110.48 (m, 1F).

### Example 27: Preparation of compounds 27, 27A, 27B, 27A-P1, and 27A-P2

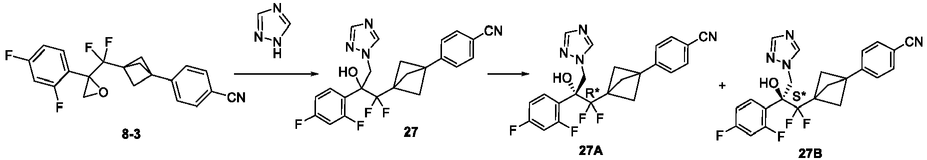

### Preparation of compound 27

Compound 1,2,4-triazole (101 mg, 1.47 mmol) was dissolved in DMF solution (2.0 mL) and cooled to 0°C. Sodium hydride (60%, 59 mg, 1.47 mmol) was added thereto and reacted for half an hour. Compound **8-3** (110 mg, 0.29 mmol) was added thereto. The reaction system was stirred at 80°C for 2 hours. After the reaction system was cooled, the reaction mixture was filtered, and the crude filtrate was purified by preparative separation (preparation method: mobile phase: A: 0.1% formic acid aqueous solution; B: acetonitrile; chromatographic column: Agilent 10 Prep-C18 250 × 21.2 mm; column temperature: 25°C; gradient: 70% to 90% acetonitrile in 12 min; flow rate: 30 mL/min) to obtain the title compound **27** (77 mg, yield: 59%, containing a pair of enantiomers).

Compound **27:** LC-MS (ESI): m/z 443.2 [M+H]⁺. ¹H NMR (400 MHz, DMSO-d6) δ 8.43 (s, 1H), 7.87 - 7.77 (m, 2H), 7.75 (s, 1H), 7.68 - 7.55 (m, 1H), 7.47 - 7.34 (m, 2H), 7.31 - 7.20 (m, 1H), 7.11 - 6.94 (m, 2H), 5.20 (d, *J* = 14.4 Hz, 1H), 4.83 (d, *J* = 14.4 Hz, 1H), 2.23 - 2.04 (m, 3H), 2.04 - 1.79 (m, 3H). ¹⁹F NMR (376 MHz, DMSO-d6) δ -102.19 - -102.99 (m, 1F), -109.38 - -109.93 (m, 2F), -110.23 - -110.53 (m, 1F).

### Preparation of compounds 27A and 27B

Compound **27** (70 mg) was subjected to SFC chiral preparative resolution (preparative separation method, instrument model: MG II preparative SFC(SFC-14); model of chromatographic column: ChiralPak AD, 250 × 30 mm I.D., 10 µm; mobile phase: A: CO₂ B: ethanol (0.1% NH₃H₂O); elution gradient: B 20%; flow rate: 60 mL/min; column pressure: 100 bar; column temperature: 38°C; detection wavelength: 220 nm; cycle: about 2.8 min) to obtain the title compounds **27A** (30 mg, single enantiomer) and **27B** (35 mg, single enantiomer).

Compound **27A:** LC-MS (ESI): m/z 443.2 [M+H]⁺. Chiral analysis method (model of chromatographic column: AD-3 150 × 4.6 mm I.D., 3 µm; mobile phase: A: CO₂ B: ethanol (0.05% DEA); elution gradient: the mobile phase increased from 5% B to 40% B within 5 min and maintained at 40% B for 2.5 min for elution, then 5% B was equilibrated for 2.5 min; flow rate: 2.5 mL/min; column temperature: 35°C; column pressure: 1500 psi; detection wavelength: 220 nm; Rt = 3.226 min). ¹H NMR (400 MHz, DMSO-*d6*) δ 8.43 (s, 1H), 7.87 - 7.77 (m, 2H), 7.75 (s, 1H), 7.68 - 7.55 (m, 1H), 7.47 - 7.34 (m, 2H), 7.31 - 7.20 (m, 1H), 7.11 - 6.94 (m, 2H), 5.20 (d, *J* = 14.4 Hz, 1H), 4.83 (d, *J* = 14.4 Hz, 1H), 2.23 - 2.04 (m, 3H), 2.04 - 1.79 (m, 3H). ¹⁹F NMR (376 MHz, DMSO-d6) δ -102.19 - -102.99 (m, 1F), -109.38 - -109.93 (m, 2F), -110.23 - -110.53 (m, 1F).

Compound **27B:** LC-MS (ESI): m/z 443.2 [M+H]⁺. Chiral analysis method (model of chromatographic column: AD-3 150 × 4.6 mm I.D., 3 µm; mobile phase: A: CO₂ B: ethanol (0.05% DEA); elution gradient: the mobile phase increased from 5% B to 40% B within 5 min and maintained at 40% B for 2.5 min for elution, then 5% B was equilibrated for 2.5 min; flow rate: 2.5 mL/min; column temperature: 35°C; column pressure: 1500 psi; detection wavelength: 220 nm; Rt = 3.000 min). ¹H NMR (400 MHz, DMSO-*d6*) δ 8.43 (s, 1H), 7.87 - 7.77 (m, 2H), 7.75 (s, 1H), 7.68 - 7.55 (m, 1H), 7.47 - 7.34 (m, 2H), 7.31 - 7.20 (m, 1H), 7.11 - 6.94 (m, 2H), 5.20 (d, *J* = 14.4 Hz, 1H), 4.83 (d, *J* = 14.4 Hz, 1H), 2.23 - 2.04 (m, 3H), 2.04 - 1.79 (m, 3H). ¹⁹F NMR (376 MHz, DMSO-*d6*) δ -102.19 - -102.99 (m, 1F), -109.38 - -109.93 (m, 2F), -110.23 - -110.53 (m, 1F).

### Preparation of compound 27A-P1

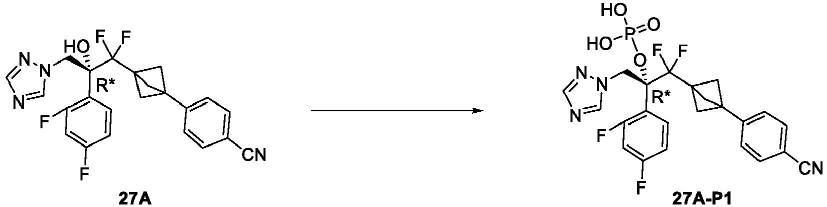

Compound **27A** (100 mg, 0.23 mmol) was dissolved in THF (1 mL), then NaH (45 mg, 1.13 mmol) was added thereto at 0 °C, and the reaction system reacted for another 30 min, added dropwise with phosphorus oxychloride (0.5 mL), and transferred to room temperature to react for 16 hours. The reaction mixture was added with saturated sodium bicarbonate solution, and reacted at 50°C for 16 hours. After the reaction system was cooled, the reaction mixture was filtered, and the crude filtrate was purified by preparative separation (chromatographic column: Welch Xtimate@ C18 21.2 × 250 mm; column temperature: 25°C; mobile phase: water (10 mM/L NH₄HCO₃)-acetonitrile; proportion of acetonitrile in the mobile phase: 20% to 40% in 12 min; flow rate: 30 mL/min) to obtain the title compound **27A-P1** (40 mg, yield: 35%). LC-MS (ESI): m/z 523.0 [M+H]⁺. ¹H NMR (400 MHz, Methanol-d4) δ 8.87 (s, 1H), 7.98 (q, *J* = 8.4 Hz, 1H), 7.72 (s, 1H), 7.65 - 7.57 (m, 2H), 7.31 - 7.23 (m, 2H), 6.99 - 6.81 (m, 2H), 6.08 (d, *J* = 15.0 Hz, 1H), 5.36 (d, *J* = 15.0 Hz, 1H), 2.11 (dd, *J* = 9.4, 1.9 Hz, 3H), 1.93 (dd, *J* = 9.5, 1.8 Hz, 3H). ¹⁹F NMR (376 MHz, Methanol-d4) δ -101.27 - - 101.95 (m, 2F), -108.52 - -108.97 (m, 1F), -111.67 (d, 1F).

### Preparation of compound 27A-P2

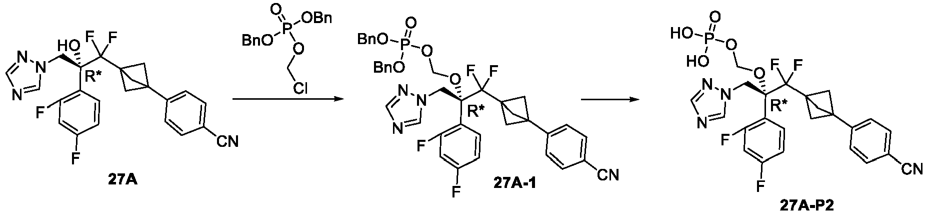

### Preparation of compound 27A-1

Compound **27A** (200 mg, 0.45 mmol) was dissolved in DMF (2 mL), then dibenzyl chloromethyl phosphate (294 mg, 0.9 mmol) and Cs₂CO₃ (440 mg, 1.35 mmol) were added thereto, and the reaction system was reacted at 80°C for 16 hours. After the reaction system was cooled, the reaction mixture was extracted and evaporated to dryness by rotary evaporation to obtain the title compound **27A-1**, which was directly used in the next reaction step.

### Preparation of compound 27A-P2

Compound **27A-1** (200 mg, 0.45 mmol) was dissolved in MeOH (2 mL), then palladium on carbon (20 mg) was added thereto, and the reaction system was replaced with hydrogen three times and reacted at room temperature for 16 hours. The reaction mixture was filtered, and the crude filtrate was purified by preparative separation (chromatographic column: Welch Xtimate@ C18 21.2 × 250 mm; column temperature: 25°C; mobile phase: water (10 mM/L NH₄HCO₃)-acetonitrile; proportion of acetonitrile in the mobile phase: 15% to 35% in 12 min; flow rate: 30 mL/min) to obtain the title compound **27A-P2:** LC-MS (ESI): m/z 553.2 [M+H]⁺. ¹H NMR (400 MHz, DMSO-d6) δ 8.70 (s, 1H), 7.88 - 7.77 (m, 1H), 7.75 - 7.67 (m, 3H), 7.33 (d, *J* = 8.0 Hz, 2H), 7.25 (m, 2H), 7.18 -7.10 (m, 1H), 7.01 - 6.94 (m, 1H), 5.59 (dd, *J* = 10.6, 5.3 Hz, 1H), 5.38 (t, *J* = 7.2 Hz, 1H), 5.31 (d, *J* = 15.3 Hz, 1H), 5.16 (d, *J* = 15.3 Hz, 1H), 2.11 - 2.03 (m, 3H), 1.97 - 1.87 (m, 3H).

### Example 28: Preparation of compounds 28A and 28B

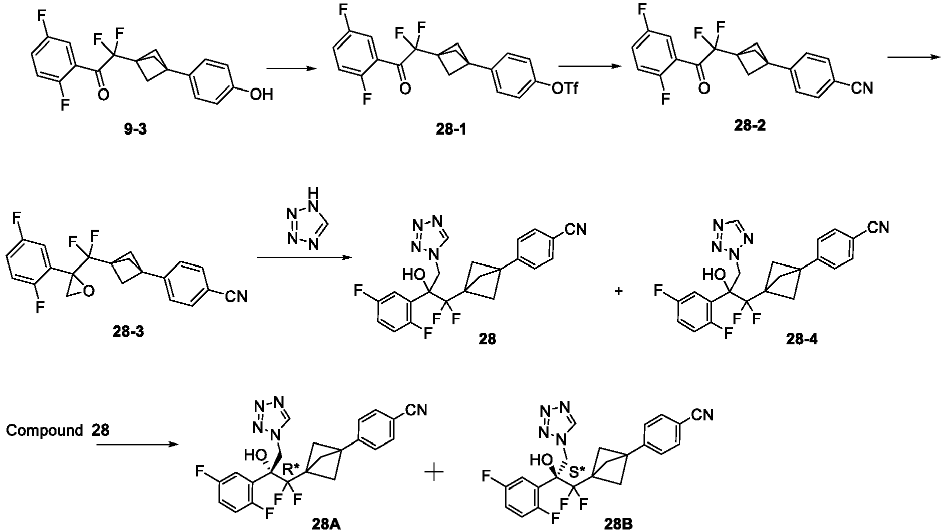

### Preparation of compound 28-1

To a three-necked flask were added compound **9-3** (1.21 g, 3.4 mmol), triethylamine (1.04 g, 10.4 mmol), and DMF (10 mL), and the system was replaced with nitrogen three times. After the reaction system was stirred and reacted at 0°C for 5 min, N-phenyl-bis(trifluoromethanesulfonimide) (CAS: 37595-74-7, 1.6 g, 4.5 mmol) was slowly added thereto. The reaction system was stirred and reacted at room temperature for 1 hour. After the reaction was completed, the reaction system was added with saturated sodium chloride solution (20 mL), extracted with EtOAc (20 mL × 3), and the organic phases were combined, dried, and concentrated to obtain a crude product. The crude product was purified by normal-phase silica gel column chromatography (EtOAc/PE = 0 to 5%) to obtain the title compound **28-1** (1.2 g, yield: 72%) as a yellow oil. ¹H NMR (400 MHz, DMSO-*d6*) δ 7.96 - 7.85 (m, 1H), 7.61 - 7.53 (m, 1H), 7.49 - 7.41 (m, 4H), 7.37 - 7.30 (m, 1H), 2.24 (s, 6H).

### Preparation of compound 28-2

To a microwave tube was added compound **28-1** (1.46 g, 3.02 mmol), then DMF (5 mL), zinc cyanide (0.43 g, 3.63 mmol), and tetrakis(triphenylphosphine)palladium (0.17 g, 0.15 mmol) were added thereto, and the reaction mixture was reacted at 80°C under microwave irradiation for 40 hours under nitrogen atmosphere. The reaction mixture was cooled, then added with water (10 mL), and extracted with EtOAc (10 mL × 3), and the organic phases were combined, dried, and concentrated to obtain a crude product. The crude product was purified by normal-phase silica gel column chromatography (EtOAc/PE = 0 to 5%) to obtain the title compound **28-2** (0.66 g, yield: 61%) as a yellow solid. ¹H NMR (400 MHz, Chloroform-*d*) δ 7.90 - 7.84 (m, 1H), 7.62 (d, *J* = 8.0 Hz, 2H), 7.32 (d, *J* = 8.0 Hz, 2H), 7.04 - 6.91 (m, 2H), 2.30 (s, 6H).

### Preparation of compound 28-3

Compound **28-2** (0.66 g, 1.84 mmol) was dissolved in a mixed solvent of dichloromethane (3 mL) and water (3 mL), then trimethylsulfoxonium iodide (1.21 g, 5.52 mmol) and sodium hydroxide (0.22 g, 5.52 mmol) were added thereto, and the reaction system was refluxed for 16 hours. After cooling to room temperature, the reaction system was extracted with DCM, and the organic phases were combined, dried, and concentrated to obtain a crude product. The crude product was purified by normal-phase silica gel column chromatography (EtOAc/PE = 0 to 5%) to obtain the title compound **28-3** (560 mg, yield: 82%, containing a pair of enantiomers) as a light yellow oil. ¹H NMR (400 MHz, DMSO-*d6*) δ 7.80 (d, *J* = 8.0 Hz, 2H), 7.49 - 7.33 (m, 5H), 3.42 (d, *J* = 4.0 Hz, 1H), 3.20 - 3.07 (m, 1H), 2.15 (s, 6H).

### Preparation of compound 28

Compound **28-3** (110 mg, 0.29 mmol) was dissolved in DMF (2.5 mL), then 1H-tetrazole (103 mg, 1.47 mmol) and potassium carbonate (203 mg, 1.47 mmol) were added thereto, and the reaction mixture was reacted at 80°C for 16 hours in a sealed tube. After the reaction system was cooled, the reaction mixture was filtered, and the crude filtrate was purified by preparative separation (preparation method: chromatographic column: Agilent 10 Prep-C18 250 × 21.2 mm; mobile phase: A: 0.1% formic acid aqueous solution; B: acetonitrile; column temperature: 25°C; gradient: 60% to 80% acetonitrile in 12 min; flow rate: 30 mL/min) to obtain the title compound **28** (30 mg, yield: 22%, containing a pair of enantiomers) and the corresponding regioisomer compound **28-4** (10 mg, yield: 7%, containing a pair of enantiomers).

Compound **28:** LC-MS (ESI): m/z 444.0 [M+H]⁺. ¹H NMR (400 MHz, DMSO-d6) δ 9.16 (s, 1H), 7.75 (d, *J* = 8.4 Hz, 2H), 7.43 - 7.17 (m, 6H), 5.45 (d, *J* = 14.4 Hz, 1H), 5.03 (d, *J* = 14.4 Hz, 1H), 2.10 (d, *J* = 9.2 Hz, 3H), 1.89 (d, *J* = 9.2 Hz, 3H). ¹⁹F NMR (376 MHz, DMSO-*d6*) δ -108.80 - -109.14 (m, 2F), -113.09 - -113.50 (m, 1F), -118.23 (d, 1F).

Compound **28-4:** LC-MS (ESI): m/z 444.0 [M+H]⁺. ¹H NMR (400 MHz, DMSO-*d6)* δ 8.78 (s, 1H), 7.75 (d, *J* = 8.4 Hz, 2H), 7.38 - 7.31 (m, 3H), 7.31 - 7.22 (m, 3H), 5.63 (d, *J* = 14.4 Hz, 1H), 5.26 (d, *J* = 14.4 Hz, 1H), 2.08 (d, *J* = 9.2 Hz, 3H), 1.88 (d, *J* = 9.2 Hz, 3H). ¹⁹F NMR (376 MHz, DMSO-d6) δ -108.31 - -109.09 (m, 2F), -112.79 - -113.19 (m, 1F), - 118.54 (d, 1F).

### Preparation of compounds 28A and 28B

Compound **28** (147 mg) was subjected to SFC chiral preparative resolution (preparative separation method, instrument model: MG II preparative SFC(SFC-14); model of chromatographic column: ChiralPak AD, 250 × 30 mm I.D., 10 µm; mobile phase: A: CO₂ B: ethanol; elution gradient: B 25%; flow rate: 70 mL/min; column pressure: 100 bar; column temperature: 38°C; detection wavelength: 220 nm; cycle: about 3 min) to obtain the title compounds **28A** (78 mg, single enantiomer) and **28B** (76 mg, single enantiomer).

Compound **28A:** LC-MS (ESI): m/z 444.0 [M+H]⁺. Chiral analysis method (model of chromatographic column: AD-3 150 × 4.6 mm I.D., 3 µm; mobile phase: A: CO₂ B: ethanol (0.05% DEA); elution gradient: the mobile phase increased from 5% B to 40% B within 5 min and maintained at 40% B for 2.5 min for elution, then 5% B was equilibrated for 2.5 min; flow rate: 2.5 mL/min; column temperature: 35°C; column pressure: 1500 psi; detection wavelength: 220 nm; Rt = 1.464 min). ¹H NMR (400 MHz, DMSO-*d6*) ¹H NMR (400 MHz, DMSO-*d6*) δ 9.15 (s, 1H), 7.78 - 7.71 (m, 2H), 7.38 - 7.33 (m, 2H), 7.33 - 7.21 (m, 4H), 5.45 (d, *J* = 14.4 Hz, 1H), 5.03 (d, *J* = 14.4 Hz, 1H), 2.13 - 2.07 (m, 3H), 1.93 - 1.86 (m, 3H). ¹⁹F NMR (376 MHz, DMSO-*d6*) δ -108.94 - -109.04 (m, 2F), -113.06 - -113.59 (m, 1F), -118.21 (d, 1F).

Compound **28B:** LC-MS (ESI): m/z 444.0 [M+H]⁺. Chiral analysis method (model of chromatographic column: AD-3 150 × 4.6 mm I.D., 3 µm; mobile phase: A: CO₂ B: ethanol (0.05% DEA); elution gradient: the mobile phase increased from 5% B to 40% B within 5 min and maintained at 40% B for 2.5 min for elution, then 5% B was equilibrated for 2.5 min; flow rate: 2.5 mL/min; column temperature: 35°C; column pressure: 1500 psi; detection wavelength: 220 nm; Rt = 1.025 min). ¹H NMR (400 MHz, DMSO-*d6*) δ 9.15 (s, 1H), 7.78 - 7.71 (m, 2H), 7.38 - 7.33 (m, 2H), 7.33 - 7.21 (m, 4H), 5.45 (d, *J* = 14.4 Hz, 1H), 5.03 (d, *J* = 14.4 Hz, 1H), 2.13 - 2.07 (m, 3H), 1.93 - 1.86 (m, 3H). ¹⁹F NMR (376 MHz, DMSO-d6) δ -108.94 - -109.04 (m, 2F), -113.06 - -113.59 (m, 1F), -118.21 (m, 1F).

### Example 29: Preparation of compounds 29A and 29B

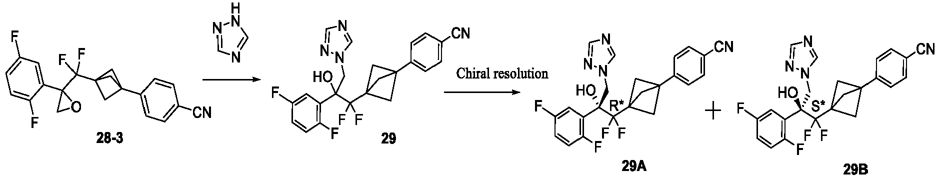

### Preparation of compound 29

Compound **28-3** (110 mg, 0.29 mmol) was dissolved in DMF (2.5 mL), then 1,2,4-triazole (103 mg, 1.47 mmol) and potassium carbonate (203 mg, 1.47 mmol) were added thereto, and the reaction mixture was reacted at 80°C for 16 hours in a sealed tube. After the reaction system was cooled, the reaction mixture was filtered, and the crude filtrate was purified by preparative separation (preparation method: chromatographic column: Agilent 10 Prep-C18 250 × 21.2 mm; mobile phase: A: 0.1% formic acid aqueous solution; B: acetonitrile; column temperature: 25°C; gradient: 60% to 80% acetonitrile in 12 min; flow rate: 30 mL/min) to obtain the title compound **29** (50 mg, yield: 36%, containing a pair of enantiomers).

Compound **29:** LC-MS (ESI): m/z 443.0 [M+H]⁺. ¹H NMR (400 MHz, DMSO-d6) δ 8.31 (s, 1H), 7.68 (d, *J* = 8.4 Hz, 2H), 7.63 (s, 1H), 7.32 - 7.25 (m, 2H), 7.24 - 7.09 (m, 3H), 6.97 (s, 1H), 5.09 (d, *J* = 14.4 Hz, 1H), 4.71 (d, *J* = 14.4 Hz, 1H), 2.01 (d, *J* = 9.2 Hz, 3H), 1.80 (d, *J* = 9.2 Hz, 3H). ¹⁹F NMR (376 MHz, DMSO-*d6*) δ -108.99 - -109.54 (m, 2F), -112.51 - -113.38 (m, 1F), -118.79 (d, 1F).

### Preparation of compounds 29A and 29B

Compound **29** (45 mg) was subjected to SFC chiral preparative resolution (preparative separation method, instrument model: MG II preparative SFC(SFC-14); model of chromatographic column: ChiralPak AD, 250 × 30 mm I.D., 10 µm; mobile phase: A: CO₂ B: ethanol (0.1% NH₃H₂O); elution gradient: B 20%; flow rate: 60 mL/min; column pressure: 100 bar; column temperature: 38°C; detection wavelength: 220 nm; cycle: about 4 min) to obtain the title compounds **29A** (12 mg, single enantiomer) and **29B** (18 mg, single enantiomer).

Compound **29A:** LC-MS (ESI): m/z 443.0 [M+H]⁺. Chiral analysis method (model of chromatographic column: AD-3 150 × 4.6 mm I.D., 3 µm; mobile phase: A: CO₂ B: ethanol (0.05% DEA); elution gradient: the mobile phase increased from 5% B to 40% B within 5 min and maintained at 40% B for 2.5 min for elution, then 5% B was equilibrated for 2.5 min; flow rate: 2.5 mL/min; column temperature: 35°C; column pressure: 1500 psi; detection wavelength: 220 nm; Rt = 3.135 min). ¹H NMR (400 MHz, DMSO-d6) δ 8.38 (s, 1H), 7.75 (d, *J* = 8.4 Hz, 2H), 7.70 (s, 1H), 7.35 (d, *J* = 8.4 Hz, 2H), 7.29 -7.18 (m, 3H), 7.02 (s, 1H), 5.15 (d, *J =* 14.4 Hz, 1H), 4.78 (d, *J* = 14.4 Hz, 1H), 2.08 (d, *J* = 9.2 Hz, 3H), 1.87 (d, *J* = 9.2 Hz, 3H). ¹⁹F NMR (376 MHz, DMSO-d6) δ -109.18 - -109.28 (m, 2F), -112.68 - -113.21 (m, 1F), -118.79 (d, *J* = 22.6 Hz, 1F).

Compound **29B:** LC-MS (ESI): m/z 443.0 [M+H]⁺. Chiral analysis method (model of chromatographic column: Chiralpak AD-3 150 × 4.6 mm I.D., 3 µm; mobile phase: A: CO₂ B: ethanol (0.05% DEA); elution gradient: the mobile phase increased from 5% B to 40% B within 5 min and maintained at 40% B for 2.5 min for elution, then 5% B was equilibrated for 2.5 min; flow rate: 2.5 mL/min; column temperature: 35°C; column pressure: 1500 psi; detection wavelength: 220 nm; Rt = 2.753 min). ¹H NMR (400 MHz, DMSO-*d6*) δ 8.38 (s, 1H), 7.75 (d, *J* = 8.4 Hz, 2H), 7.70 (s, 1H), 7.35 (d, *J* = 8.4 Hz, 2H), 7.29 - 7.18 (m, 3H), 7.02 (s, 1H), 5.15 (d, *J* = 14.4 Hz, 1H), 4.78 (d, *J* = 14.4 Hz, 1H), 2.08 (d, *J* = 9.6 Hz, 3H), 1.87 (d, *J* = 9.6 Hz, 3H). ¹⁹F NMR (376 MHz, DMSO-d6) δ -109.18 - -109.27 (m, 2F), -112.73 - - 113.17 (m, 1F), -118.79 (d, *J* = 18.8 Hz, 1F).

### Example 30: Preparation of compound 30

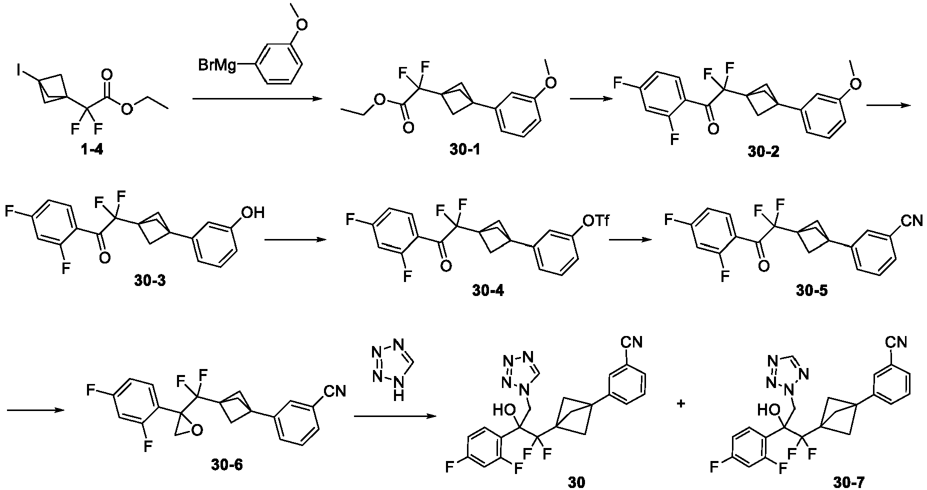

### Preparation of compound 30-1

To a three-necked flask was added ferric acetylacetonate (2.2 g, 6.3 mmol), and the system was replaced with nitrogen three times. To the three-necked flask were sequentially added anhydrous THF (80 mL), compound **1-4** (10 g, 31.6 mmol), and TMEDA (1.5 g, 12.6 mmol). The reaction system was stirred for 5 min, then a solution of Grignard reagent 3-methoxyphenyl magnesium bromide in THF (1.0 M, 51 mL, 51 mmol) was slowly added dropwise to the three-necked flask, and the reaction mixture was stirred and reacted at room temperature for another 16 hours. After the reaction was completed, the reaction system was extracted with EtOAc (30 mL × 3), and the organic phases were combined, dried, and concentrated to obtain a crude product. The crude product was purified by normal-phase silica gel column chromatography (EtOAc/PE = 0 to 5%) to obtain the title compound **30-1** (2.1 g, yield: 23%) as a light yellow liquid. ¹H NMR (400 MHz, DMSO-*d6*) δ 7.28 - 7.20 (m, 1H), 6.87 - 6.77 (m, 3H), 4.35 (q, *J* = 7.1 Hz, 2H), 3.74 (s, 3H), 2.14 (s, 6H), 1.29 (t, *J* = 7.1 Hz, 3H).

### Preparation of compound 30-2

To a three-necked flask was added compound 2,4-difluorobromobenzene (1.1 g, 5.6 mmol), and the system was replaced with nitrogen three times. To the three-necked flask was added anhydrous ether (15 mL), and n-butyllithium (1.6 M, 3.5 mL, 5.6 mmol) was added dropwise thereto at a cooling temperature of -78°C. After the reaction system was stirred and reacted at -78°C for 45 min, a solution of compound **30-1** (1.5 g, 5.1 mmol) dissolved in anhydrous ether (10 mL) was added dropwise thereto, and the reaction mixture was stirred and reacted for another 1 hour. After the reaction was completed, the reaction system was quenched with saturated ammonium chloride solution (5 mL), extracted with EtOAc (20 mL × 3), and the organic phases were combined, dried, and concentrated to obtain a crude product. The crude product was purified by normal-phase silica gel column chromatography (EtOAc/PE = 0 to 5%) to obtain the title compound **30-2** (1.6 g, yield: 86%) as a yellow solid. ¹H NMR (400 MHz, Chloroform-*d*) δ 7.92 - 7.82 (m, 1H), 7.25 - 7.20 (m, 1H), 7.05 - 6.97 (m, 1H), 6.96 - 6.89 (m, 1H), 6.83 - 6.77 (m, 2H), 6.75 - 6.71 (m, 1H), 3.80 (s, 3H), 2.24 (s, 6H).

### Preparation of compound 30-3

To a microwave tube was added compound **30-2** (1.8 g, 4.95 mmol), then acetic acid (5 mL) and hydrogen bromide aqueous solution (48 wt% aqueous solution, 5 mL) were added thereto, and the reaction mixture was reacted at 95°C for 16 hours in a sealed tube. The reaction mixture was cooled, then evaporated to dryness by rotary evaporation, extracted with EtOAc (30 mL × 3), and the organic phases were combined, dried, and concentrated to obtain a crude product. The crude product was purified by normal-phase silica gel column chromatography (EtOAc/PE = 0 to 30%) to obtain the title compound **30-3** (1.3 g, yield: 72%) as a brownish-yellow liquid. LC-MS (ESI): m/z 349.0 [M-H]⁻.

### Preparation of compound 30-4

To a 100 mL three-necked flask was added compound **30-3** (1.0 g, 2.85 mmol), then DMF (10 mL), N-phenyl-bis(trifluoromethanesulfonimide) (1.33 g, 3.43 mmol), and TEA (866 mg, 8.56 mmol) were added thereto respectively, and the reaction system was stirred and reacted at room temperature for 4 hours under nitrogen atmosphere. The reaction mixture was added with water (20 mL) to quench the reaction and extracted with EtOAc (10 mL × 3), and the organic phases were combined, dried, and concentrated to obtain a crude product. The crude product was purified by normal-phase silica gel column chromatography (EtOAc/PE = 0 to 5%) to obtain the title compound **30-4** (450 mg, yield: 33%) as a brown solid. LC-MS (ESI): m/z 483.0 [M+H]⁺.

### Preparation of compound 30-5

To a microwave tube was added compound **30-4** (300 mg, 0.62 mmol), then DMF (3 mL), zinc cyanide (87 mg, 0.75 mmol), and tetrakis(triphenylphosphine)palladium (36 mg, 0.03 mmol) were added thereto, and the reaction system was reacted at 80°C under microwave irradiation for 40 hours under nitrogen atmosphere. The reaction mixture was cooled, then added with water (10 mL) and extracted with EtOAc (10 mL × 3), and the organic phases were combined, dried, and concentrated to obtain a crude product. The crude product was purified by normal-phase silica gel column chromatography (EtOAc/PE = 0 to 5%) to obtain the title compound **30-5** (110 mg, yield: 49%) as a yellow solid. LC-MS (ESI): m/z 376.0 [M+H₂O-H]⁻.

### Preparation of compound 30-6

Compound **30-5** (110 mg, 0.30 mmol) was dissolved in a mixed solvent of dichloromethane (3 mL) and water (3 mL), then trimethylsulfoxonium iodide (202 mg, 0.91 mmol) and sodium hydroxide (37 mg, 0.91 mmol) were added thereto, and the reaction system was refluxed for 16 hours. After cooling to room temperature, the reaction system was extracted with DCM (10 mL × 3,) and the organic phases were combined, dried, and concentrated to obtain a crude product. The crude product was purified by normal-phase silica gel column chromatography (EtOAc/PE = 0 to 5%) to obtain the title compound **30-6** (110 mg, yield: 96%, containing a pair of enantiomers) as a light yellow oil. LC-MS (ESI): m/z 374.2 [M+H]⁺.

### Preparation of compound 30

Compound **30-6** (300 mg, 0.80 mmol) was dissolved in DMF (3 mL), then 1H-tetrazole (281 mg, 4.0 mmol) and potassium carbonate (555 mg, 4.0 mmol) were added thereto, and the reaction mixture was reacted at 80°C for 16 hours in a sealed tube. After the reaction system was cooled, the reaction mixture was filtered, and the crude filtrate was purified by preparative separation (preparation method: chromatographic column: Agilent 10 Prep-C18 250 × 21.2 mm; mobile phase: A: 0.1% formic acid aqueous solution; B: acetonitrile; column temperature: 25°C; gradient: 60% to 80% acetonitrile in 12 min; flow rate: 30 mL/min) to obtain the title compound **30** (110 mg, yield: 30%, containing a pair of enantiomers) and the corresponding regioisomer compound **30-7** (55 mg, yield: 15%, containing a pair of enantiomers).

Compound **30:** LC-MS (ESI): m/z 444.2 [M+H]⁺. ¹H NMR (400 MHz, DMSO-^6) δ 9.20 (s, 1H), 7.80 - 7.72 (m, 1H), 7.72 - 7.66 (m, 1H), 7.65 - 7.56 (m, 1H), 7.57 - 7.50 (m, 2H), 7.40 - 7.31 (m, 1H), 7.26 (s, 1H), 7.12 - 7.01 (m, 1H), 5.50 (d, *J* = 14.6 Hz, 1H), 5.08 (d, *J* = 14.6 Hz, 1H), 2.21 - 2.02 (m, 3H), 2.02 - 1.67 (m, 3H). ¹⁹F NMR (376 MHz, DMSO-*d6*) δ -102.81 - -103.30 (m, 1F), -109.26 - -109.42 (m, 1F), -109.42 - -109.65 (m, 2F).

Compound **30-7:** LC-MS (ESI): m/z 444.2 [M+H]⁺. ¹H NMR (400 MHz, DMSO-*d6*) δ 8.77 (s, 1H), 7.73 - 7.66 (m, 1H), 7.66 - 7.60 (m, 1H), 7.60 - 7.52 (m, 1H), 7.52 - 7.43 (m, 2H), 7.32 - 7.21 (m, 2H), 7.06 - 6.95 (m, 1H), 5.61 (d, *J* = 14.2 Hz, 1H), 5.25 (d, *J* = 14.2 Hz, 1H), 2.19 - 1.98 (m, 3H), 1.90 - 1.75 (m, 3H). ¹⁹F NMR (376 MHz, DMSO-d6) δ - 102.31 - -102.85 (m, 1F), -108.91 - -109.33 (m, 2F), -109.81 - -110.08 (m, 1F).

### Example 31: Preparation of compound 31

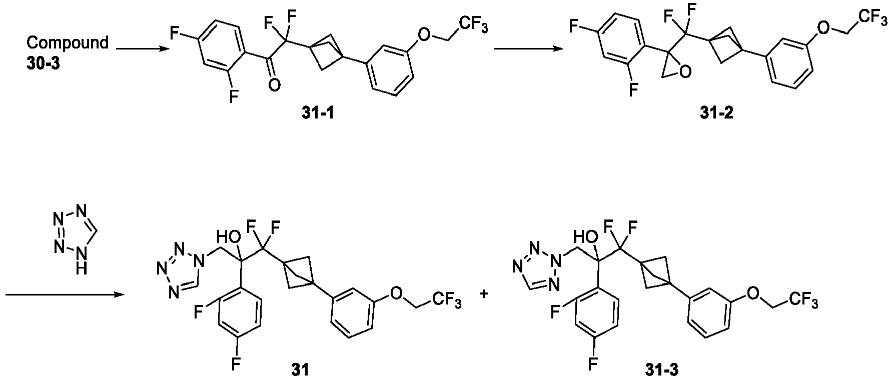

### Preparation of compound 31-1

Compound **30-3** (700 mg, 2 mmol) was dissolved in DMF (8 mL) solvent, then potassium carbonate (552 mg, 4 mmol) and 2,2,2-trifluoroethyl trifluoromethanesulfonate (928 mg, 4 mmol) were added thereto, and the reaction mixture was reacted at 65°C for 16 hours in a sealed tube. After the reaction system was cooled, the reaction mixture was extracted with EtOAc (10 mL × 3), and the organic phases were combined, dried, and concentrated to obtain a crude product. The crude product was purified by normal-phase silica gel column chromatography (EtOAc/PE = 0 to 10%) to obtain the title compound **31-1** (750 mg, yield: 87%) as a colorless oil. LC-MS (ESI): m/z 431.0 [M-H]⁻.

### Preparation of compound 31-2

Compound **31-1** (750 mg, 1.74 mmol) was dissolved in a mixture of dichloromethane (15.0 mL) and water (5.0 mL), then trimethylsulfoxonium iodide (1.53 g, 6.94 mmol) and solid sodium hydroxide (278 mg, 6.94 mmol) were sequentially added thereto, and the reaction system was stirred and reacted at 65°C for 12 hours. After the reaction was completed, the reaction system was extracted with dichloromethane (20 mL × 3), and the organic phases were combined, dried, and concentrated to obtain a crude product. The crude product was purified by normal-phase silica gel column chromatography (EtOAc/PE = 0 to 10%) to obtain the title compound **31-2** (540 mg, yield: 70%). ¹H NMR (400 MHz, DMSO-*d6*) δ 7.68 (td, *J* = 8.4, 6.4 Hz, 1H), 7.35 (ddd, *J* = 10.5, 9.3, 2.6 Hz, 1H), 7.27 (t, *J* = 7.8 Hz, 1H), 7.23 -7.11 (m, 1H), 6.98 - 6.82 (m, 3H), 4.74 (q, *J* = 8.9 Hz, 2H), 3.39 (d, *J* = 4.7 Hz, 1H), 3.09 (dt, *J* = 4.3, 1.8 Hz, 1H), 2.07 (s, 6H).

### Preparation of compound 31

Compound **31-2** (540 mg, 1.2 mmol) was dissolved in DMF solution (10 mL), and compound 1H-tetrazole (339 mg, 4.84 mmol) and potassium carbonate (668 mg, 4.84 mmol) were added thereto. The reaction system was stirred for 12 hours at 80°C in a sealed tube. After the reaction system was cooled, the reaction mixture was filtered, and the crude filtrate was purified by preparative separation (preparation method: mobile phase: A: 0.1% formic acid aqueous solution; B: acetonitrile; chromatographic column: Agilent 10 Prep-C18 250 × 21.2 mm; column temperature: 25°C; gradient: 60% to 80% acetonitrile in 12 min; flow rate: 30 mL/min) to obtain the title compound **31** (310 mg, yield: 50%, containing a pair of enantiomers) and the corresponding regioisomer compound **31-3** (120 mg, yield: 19%, containing a pair of enantiomers).

Compound **31:** LC-MS (ESI): m/z 517.0 [M+H]⁺. ¹H NMR (400 MHz, DMSO-d6) δ 9.14 (s, 1H), 7.54 (td, *J* = 9.0, 6.7 Hz, 1H), 7.36 - 7.15 (m, 3H), 7.01 (td, *J* = 8.4, 2.6 Hz, 1H), 6.95 - 6.85 (m, 1H), 6.86 - 6.74 (m, 2H), 5.43 (d, *J* = 14.6 Hz, 1H), 5.02 (d, *J* = 14.6 Hz, 1H), 4.72 (q, *J* = 8.9 Hz, 2H), 2.01 (dd, *J* = 9.3, 1.7 Hz, 3H), 1.80 (dd, *J* = 9.4, 1.7 Hz, 3H). ¹⁹F NMR (376 MHz, DMSO-d6) δ -72.59 (s, 3F), -102.95 - -103.16 (m, 1F), -109.27 - -109.38 (m, 2F), -109.59 (d, *J* = 9.6 Hz, 1F).

Compound **31-3:** LC-MS (ESI): m/z 517.0 [M+H]⁺. ¹H NMR (400 MHz, DMSO-*d6)* δ 8.77 (s, 1H), 7.57 (td, *J* = 9.0, 6.7 Hz, 1H), 7.34 - 7.15 (m, 3H), 7.00 (td, *J* = 8.5, 2.6 Hz, 1H), 6.89 (ddd, *J* = 8.2, 2.6, 0.9 Hz, 1H), 6.84 - 6.74 (m, 2H), 5.61 (d, *J* = 14.2 Hz, 1H), 5.25 (d, *J* = 14.2 Hz, 1H), 4.72 (q, *J* = 8.9 Hz, 2H), 2.00 (dd, *J* = 9.4, 1.7 Hz, 3H), 1.79 (dd, *J* = 9.5, 1.7 Hz, 3H). ¹⁹F NMR (376 MHz, DMSO-*d6*) δ -72.59 (s, 3F), -102.54 - -102.76 (m, 1F), - 108.93 - -109.13 (m, 2F), -109.94 (d, *J* = 9.6 Hz, 1F).

### Example 32: Preparation of compounds 32A and 32B

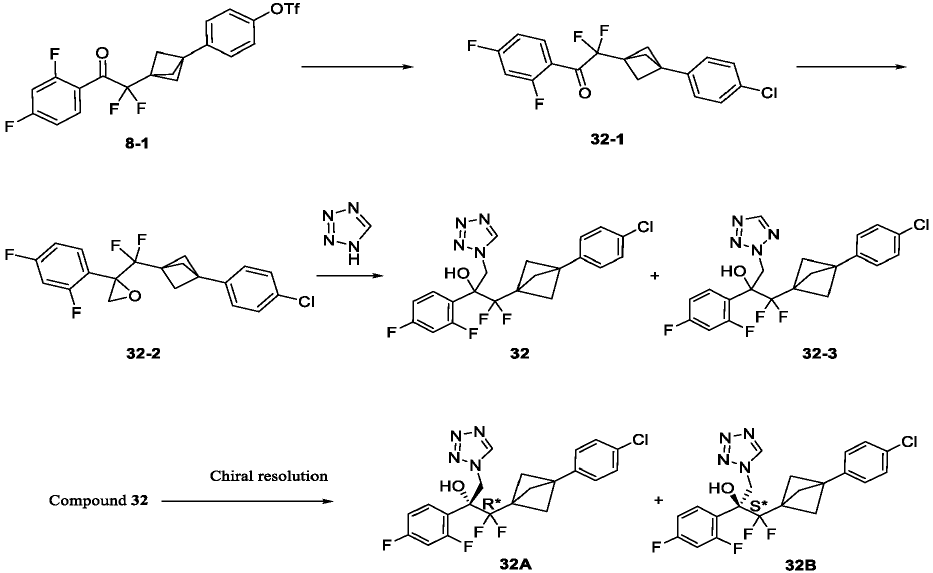

### Preparation of compound 32-1

To a microwave tube were added compound **8-1** (500 mg, 1.04 mmol), tris(dibenzylideneacetone)dipalladium (CAS: 51364-51-3, 95 mg, 0.1 mmol), 2-(di-t-butylphosphino)-3,6-dimethoxy-2'-4'-6'-tri-i-propyl-1,1'-biphenyl (*t*-BuBrettPhos, 101 mg, 0.21 mmol), potassium chloride (156 mg, 2.07 mmol), and potassium fluoride (30 mg, 0.52 mmol), then the system was replaced with nitrogen three times, and 1,4-dioxane (15 mL) was added thereto as a solvent. The reaction system was reacted at 120°C under microwave irradiation for 2 hours. The reaction mixture was cooled, then extracted with EtOAc (20 mL × 3), and the organic phases were combined, dried, and concentrated to obtain a crude product. The crude product was purified by normal-phase silica gel column chromatography (EtOAc/PE = 0 to 10%) to obtain the title compound **32-1** (260 mg, yield: 68%) as a colorless liquid. LC-MS (ESI): m/z 385.0 [M+H₂O-H]⁻. ¹H NMR (400 MHz, DMSO-*d6*) δ 7.98 - 7.86 (m, 1H), 7.62 - 7.51 (m, 1H), 7.44 - 7.21 (m, 5H), 2.20 (s, 6H). ¹⁹F NMR (376 MHz, DMSO-d6) δ - 99.53 - -100.21 (m, 1F), -105.17 - -105.88 (m, 1F), -106.37 - -107.02 (m, 2F).

### Preparation of compound 32-2

Compound **32-1** (250 mg, 0.68 mmol) was dissolved in a mixed solvent of dichloromethane (8 mL) and water (8 mL), then trimethylsulfoxonium iodide (598 mg, 2.72 mmol) and sodium hydroxide (109 mg, 2.72 mmol) were added thereto, and the reaction system was refluxed for 16 hours. After cooling to room temperature, the reaction system was extracted with DCM (20 mL × 3), and the organic phases were combined, dried, and concentrated to obtain a crude product. The crude product was purified by normal-phase silica gel column chromatography (EtOAc/PE = 0 to 10%) to obtain the title compound **32-2** (210 mg, yield: 81%) as a light yellow oil. ¹H NMR (400 MHz, DMSO-*d6*) δ 7.76 - 7.60 (m, 1H), 7.44 - 7.29 (m, 3H), 7.28 - 7.12 (m, 3H), 3.43 - 3.37 (m, 1H), 3.13 - 3.04 (m, 1H), 2.08 (s, 6H). ¹⁹F NMR (376 MHz, DMSO-*d6*) δ -107.77 (d, *J =* 8.8 Hz, 1F), -108.18 (q, *J* = 11.1 Hz, 1F), -108.83 (dd, *J* = 61.0, 12.2 Hz, 2F).

### Preparation of compound 32

Compound **32-2** (200 mg, 0.52 mmol) was dissolved in DMF solution (5 mL), and compound 1H-tetrazole (73 mg, 1.05 mmol) and potassium carbonate (145 mg, 1.05 mmol) were added thereto. The reaction system was stirred for 16 hours at 80°C in a sealed tube. After the reaction system was cooled, the reaction mixture was filtered, and the crude filtrate was purified by preparative separation (preparation method: mobile phase: A: 0.1% formic acid aqueous solution; B: acetonitrile; chromatographic column: Agilent 10 Prep-C18 250 × 21.2 mm; column temperature: 25°C; gradient: 60% to 80% acetonitrile in 12 min; flow rate: 30 mL/min) to obtain the title compound **32** (110 mg, yield: 46%, containing a pair of enantiomers) and the corresponding regioisomer compound **32-3** (47 mg, yield: 20%, containing a pair of enantiomers).

Compound **32:** LC-MS (ESI): m/z 453.2 [M+H]⁺. ¹H NMR (400 MHz, DMSO-*d6*) δ 9.13 (s, 1H), 7.54 (td, *J* = 9.0, 6.7 Hz, 1H), 7.37 - 7.22 (m, 3H), 7.21 - 7.11 (m, 3H), 7.00 (td, *J* = 8.5, 2.7 Hz, 1H), 5.42 (d, *J* = 14.5 Hz, 1H), 5.01 (d, *J* = 14.5 Hz, 1H), 2.02 (dd, *J* = 9.4, 1.7 Hz, 3H), 1.80 (dd, *J* = 9.5, 1.7 Hz, 3H). ¹⁹F NMR (376 MHz, DMSO-d6) δ -102.82 - - 103.26 (m, 1F), -109.26 - -109.50 (m, 2F), -109.58 (d, *J* = 9.5 Hz, 1F).

Compound **32-3:** LC-MS (ESI): m/z 453.2 [M+H]⁺. ¹H NMR (400 MHz, DMSO-*d6)* δ 8.76 (s, 1H), 7.56 (td, *J* = 9.0, 6.7 Hz, 1H), 7.36 - 7.29 (m, 2H), 7.24 (d, *J* = 17.3 Hz, 2H), 7.18 -7.11 (m, 2H), 6.99 (td, *J* = 8.5, 2.6 Hz, 1H), 5.61 (d, *J* = 14.2 Hz, 1H), 5.24 (d, *J* = 14.3 Hz, 1H), 2.00 (dd, *J* = 9.5, 1.8 Hz, 3H), 1.79 (dd, *J* = 9.4, 1.7 Hz, 3H). ¹⁹F NMR (376 MHz, DMSO-*d6*) δ -102.42 - -102.83 (m, 1F), -108.87 - -109.32 (m, 2F), -109.93 (d, *J* = 9.4 Hz, 1F).

### Preparation of compounds 32A and 32B

Compound **32** (90 mg) was subjected to SFC chiral preparative resolution (preparative separation method, instrument model: MG II preparative SFC (SFC-14); model of chromatographic column: ChiralPak AD, 250 × 30 mm I.D., 10 µm; mobile phase: A: CO₂ B: ethanol (0.1% NH₃ H₂O); elution gradient: B 15%; flow rate: 60 mL/min; column pressure: 100 bar; column temperature: 38°C; detection wavelength: 220 nm; cycle: about 3.5 min) to obtain the title compounds **32A** (54 mg, single enantiomer) and **32B** (44 mg, single enantiomer).

Compound **32A:** LC-MS (ESI): m/z 453.2 [M+H]⁺. ¹H NMR (400 MHz, DMSO-*d6*) δ 9.13 (s, 1H), 7.54 (td, *J* = 9.0, 6.7 Hz, 1H), 7.37 - 7.24 (m, 3H), 7.20 - 7.11 (m, 3H), 7.01 (td, *J* = 8.5, 2.6 Hz, 1H), 5.43 (d, *J* = 14.5 Hz, 1H), 5.01 (d, *J* = 14.6 Hz, 1H), 2.02 (dd, *J* = 9.4, 1.7 Hz, 3H), 1.80 (dd, *J* = 9.4, 1.7 Hz, 3H). ¹⁹F NMR (376 MHz, DMSO-d6) δ -102.81 - - 103.31 (m, 1F), -109.22 - -109.51 (m, 2F), -109.57 (d, *J* = 9.6 Hz, 1F).

Compound **32B:** LC-MS (ESI): m/z 453.2 [M+H]⁺. ¹H NMR (400 MHz, DMSO-d6) δ 9.14 (s, 1H), 7.54 (td, *J =* 9.0, 6.7 Hz, 1H), 7.37 - 7.23 (m, 3H), 7.22 - 7.09 (m, 3H), 7.01 (td, *J* = 8.5, 2.7 Hz, 1H), 5.43 (d, *J* = 14.5 Hz, 1H), 5.01 (d, *J* = 14.6 Hz, 1H), 2.02 (dd, *J* = 9.5, 1.7 Hz, 3H), 1.80 (dd, *J* = 9.4, 1.7 Hz, 3H). ¹⁹F NMR (376 MHz, DMSO-*d6*) δ -102.81 - - 103.35 (m, 1F), -109.25 - -109.50 (m, 2F), -109.58 (d, *J* = 9.3 Hz, 1F).

### Example 33: Preparation of compound 33

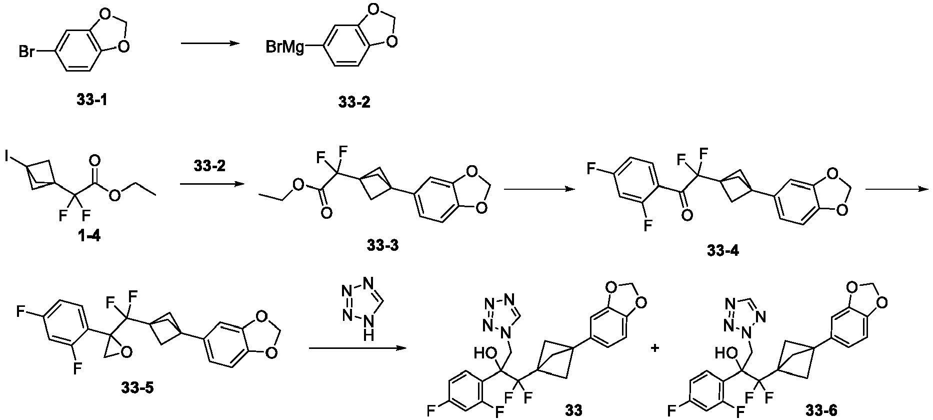

### Preparation of Grignard reagent 33-2

To a three-necked flask were added magnesium chips (2.9 g, 120 mmol) and a piece of elemental iodine, and the system was replaced with nitrogen three times. To the three-necked flask was added anhydrous THF (100 mL), and a solution of compound **33-1** (12.46 g, 62 mmol) dissolved in THF (50 mL) was added dropwise thereto at a temperature of 78°C. The reaction system was stirred and reacted at 78°C for 30 min, and then gradually cooled to room temperature to obtain a solution of the title compound **33-2** in THF (0.62 M, 100 mL) as a light brown liquid.

### Preparation of compound 33-3

To a three-necked flask was added ferric acetylacetonate (871 mg, 2.4 mmol), and the system was replaced with nitrogen three times. To the three-necked flask were sequentially added anhydrous THF (20 mL), compound **1-4** (3.9 g, 12.34 mmol), and TMEDA (572 mg, 4.9 mmol). The reaction system was stirred for 5 min, then the Grignard reagent compound **33-2** prepared as above was slowly added dropwise to the three-necked flask, and the reaction mixture was stirred and reacted at room temperature for another 16 hours. After the reaction was completed, the reaction system was extracted with EtOAc (30 mL × 3), and the organic phases were combined, dried, and concentrated to obtain a crude product. The crude product was purified by normal-phase silica gel column chromatography (EtOAc/PE = 0 to 15%) to obtain the title compound **33-3** (0.65 g, yield: 17%) as a light yellow liquid. ¹H NMR (400 MHz, Chloroform-*d*) δ 6.76 - 6.74 (m, 1H), 6.69 - 6.64 (m, 2H), 5.93 (s, 2H), 4.36 (q, *J* = 8.0 Hz, 2H), 2.14 (s, 6H), 1.37 (t, *J* = 8.0 Hz, 3H).

### Preparation of compound 33-4

To a three-necked flask was added 1-bromo-2,4-difluorobenzene **1-7** (405 mg, 2.1 mmol), and the system was replaced with nitrogen three times. To the three-necked flask was added anhydrous ether (15 mL), and n-butyllithium (1.6 M, 1.3 mL, 2.1 mmol) was added dropwise thereto at a cooling temperature of -78°C. After the reaction system was stirred and reacted at -78°C for 45 min, a solution of compound **33-3** (550 mg, 1.77 mmol) dissolved in anhydrous ether (10 mL) was added dropwise thereto, and the reaction mixture was stirred and reacted for another 1 hour. After the reaction was completed, the reaction system was quenched with saturated ammonium chloride solution (5 mL), extracted with EtOAc (20 mL × 3), and the organic phases were combined, dried, and concentrated to obtain a crude product. The crude product was purified by normal-phase silica gel column chromatography (EtOAc/PE = 0 to 5%) to obtain the title compound **33-4** (400 mg, yield: 59.7%) as a yellow oil. ¹H NMR (400 MHz, Chloroform-*d*) δ 7.86 - 7.85 (m, 1H), 7.00 - 7.92 (m, 2H), 6.90 (s, 1H), 6.76 - 6.64 (m, 2H), 5.94 (s, 2H), 2.14 (s, 6H).

### Preparation of compound 33-5

Compound **33-4** (400 mg, 1.0 mmol) was dissolved in a mixture of dichloromethane (5.0 mL) and water (2.0 mL), then trimethylsulfoxonium iodide **1-9** (544 mg, 2.4 mmol) and solid sodium hydroxide (132 mg, 3.4 mmol) were added thereto, and the reaction system was stirred at 65°C for 48 hours. After the reaction was completed, the reaction system was extracted with dichloromethane (20 mL × 3), and the organic phases were combined, dried, and concentrated to obtain a crude product. The crude product was purified by normal-phase silica gel column chromatography (EtOAc/PE = 0 to 5%) to obtain the title compound **33-5** (360 mg, yield: 91%, containing a pair of enantiomers). ¹H NMR (400 MHz, Chloroform-*d*) δ 7.60 - 7.55 (m, 1H), 6.95 - 6.93 (m, 1H), 6.92 - 6.90 (m, 1H), 6.86 - 6.84 (m, 1H), 6.82 - 6.59 (m, 2H), 5.92 (s, 2H), 3.40 (d, *J* = 4.0 Hz, 1H), 2.96 - 2.94 (m, 1H), 2.07 - 2.00 (m, 6H).

### Preparation of compound 33

Compound **33-5** (360 mg, 0.91 mmol) was dissolved in DMF solution (1.5 mL), and compound 1H-tetrazole **1-11** (102 mg, 1.5 mmol) and potassium carbonate (151 mg, 1.1 mmol) were added thereto. The reaction system was stirred for 16 hours at 80°C in a sealed tube. After the reaction system was cooled, the reaction mixture was filtered, and the crude filtrate was purified by preparative separation (preparation method: mobile phase: A: 0.1% formic acid aqueous solution; B: acetonitrile; chromatographic column: Agilent 10 Prep-C18 250 × 21.2 mm; column temperature: 25°C; gradient: 50% to 70% acetonitrile in 12 min; flow rate: 30 mL/min) to obtain the title compound **33** (106 mg, yield: 25%, containing a pair of enantiomers) and compound **33-6** (28 mg, yield: 6.5%, containing a pair of enantiomers).

Compound **33:** LC-MS (ESI): 463.2 [M+H]⁺. ¹H NMR (400 MHz, DMSO-d6) δ 9.13 (s, 1H), 7.56 - 7.50 (m, 1H), 7.38 - 7.21 (m, 1H), 7.15 - 7.02 (m, 1H), 7.03 - 7.01 (m, 1H), 7.00- 6.98 (m, 1H), 6.80- 6.78 (m, 1H), 6.56- 6.54 (m, 1H), 5.94 (s, 2H), 5.44- 5.41 (d, *J* = 12 Hz, 1H), 5.02- 4.99 (d, *J* = 12 Hz, 1H), 1.98- 1.95 (m, 3H), 1.76- 1.73 (m, 3H).

Compound **33-6:** LC-MS (ESI): 463.2 [M+H]⁺. ¹H NMR (400 MHz, DMSO-*d6*) δ 8.76 (s, 1H), 7.59 - 7.52 (m, 1H), 7.29 - 7.27 (m, 1H) 7.26 - 7.24 (m, 1H) 7.19 - 6.99 (m, 1H) 6.79 - 6.77 (m, 1H), 6.72 (s, 1H), 6.56 - 6.54 (m, 1H), 5.94 (s, 2H), 5.62 - 5.59 (m, 1H), 5.25 - 5.22 (m, 1H), 1.96 - 1.93 (m, 3H), 1.75 - 1.72 (m, 3H).

### Example 34: Preparation of compounds 34A and 34B

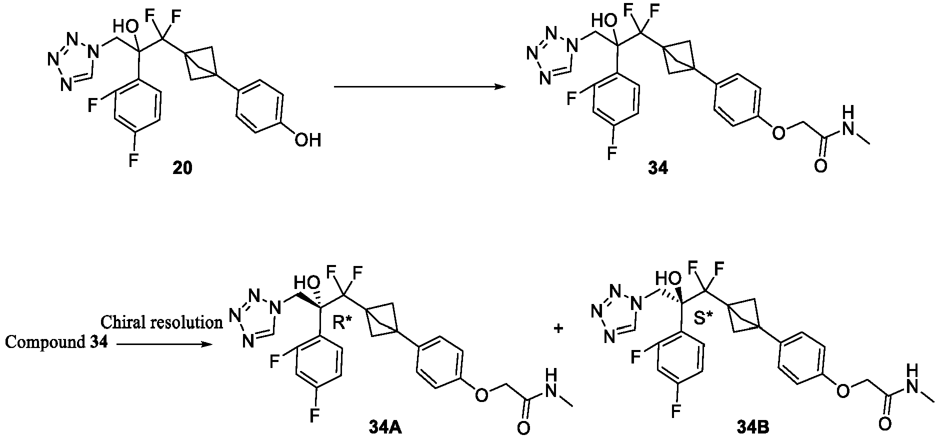

### Preparation of compound 34

Compound **20** (120 mg, 0.28 mmol) was dissolved in acetonitrile (5.0 mL), then potassium carbonate (76 mg, 0.55 mmol) was added thereto with stirring. After the reaction was carried out for 5 min, the reaction mixture was then added with 2-bromo-N-methylacetamide (84 mg, 0.55 mmol), stirred and reacted at 70°C for 3 hours. The reaction mixture was filtered, and the crude filtrate was purified by preparative separation (preparation method: chromatographic column: Agilent 10 Prep-C18 250 × 21.2 mm; column temperature: 25°C; mobile phase: water (0.1% FA)-acetonitrile; proportion of acetonitrile in the mobile phase: 40% to 60% in 12 min; flow rate: 30 mL/min) to obtain the title compound **34** (105 mg, yield: 75%, containing a pair of enantiomers). LC-MS (ESI): m/z 506.2 [M+H]⁺. ¹H NMR (400 MHz, DMSO-*d6*) δ 9.13 (s, 1H), 7.99 (d, *J* = 5.1 Hz, 1H), 7.54 (td, *J* = 9.0, 6.7 Hz, 1H), 7.28 (ddd, *J* = 12.0, 9.1, 2.6 Hz, 1H), 7.15 (s, 1H), 7.09 - 6.96 (m, 3H), 6.89 - 6.79 (m, 2H), 5.42 (d, *J* = 14.5 Hz, 1H), 5.02 (d, *J* = 14.5 Hz, 1H), 4.40 (s, 2H), 2.62 (d, *J* = 4.6 Hz, 3H), 1.98 (dd, *J* = 9.4, 1.7 Hz, 3H), 1.76 (dd, *J* = 9.4, 1.7 Hz, 3H). ¹⁹F NMR (376 MHz, DMSO-*d6*) δ -102.96 - -103.18 (m, 1F), -109.21 - -109.31 (m, 2F), -109.63 (d, *J* = 9.2 Hz, 1F).

### Preparation of compounds 34A and 34B

Compound **34** (100 mg) was subjected to SFC chiral preparative resolution (preparative separation method, instrument model: Thar 80 preparative SFC(SFC-17); model of chromatographic column: ChiralCel OD, 250 × 30 mm I.D., 10 µm; mobile phase: A: CO₂ B: ethanol(0.1% NH₃H₂O); elution gradient: B 40%; flow rate: 80 mL/min; column pressure: 100 bar; column temperature: 38°C; detection wavelength: 220 nm; cycle: about 5.1 min) to obtain the title compounds **34A** (50 mg, single enantiomer) and **34B** (49 mg, single enantiomer).

Compound **34A:** LC-MS (ESI): 506.2 [M+H]⁺. ¹H NMR (400 MHz, DMSO-*d6*) δ 9.13 (s, 1H), 7.98 (d, *J* = 5.4 Hz, 1H), 7.54 (td, *J* = 9.0, 6.7 Hz, 1H), 7.28 (ddd, *J* = 12.0, 9.0, 2.6 Hz, 1H), 7.14 (s, 1H), 7.09 - 6.95 (m, 3H), 6.90 - 6.80 (m, 2H), 5.42 (d, *J* = 14.5 Hz, 1H), 5.02 (d, *J* = 14.5 Hz, 1H), 4.40 (s, 2H), 2.62 (d, *J* = 4.7 Hz, 3H), 1.98 (dd, *J* = 9.5, 1.7 Hz, 3H), 1.76 (dd, *J* = 9.5, 1.7 Hz, 3H). ¹⁹F NMR (376 MHz, DMSO-*d6*) δ -102.96 - -103.17 (m, 1F), -109.19 - -109.30 (m, 2F), -109.63 (d, *J* = 9.2 Hz, 1F).

Compound **34B:** LC-MS (ESI): 506.2 [M+H]⁺. ¹H NMR (400 MHz, DMSO-*d6*) δ 9.13 (s, 1H), 7.98 (d, *J* = 4.4 Hz, 1H), 7.54 (td, *J* = 9.0, 6.7 Hz, 1H), 7.28 (ddd, *J* = 12.0, 9.1, 2.7 Hz, 1H), 7.14 (s, 1H), 7.10 - 6.95 (m, 3H), 6.92 - 6.78 (m, 2H), 5.42 (d, *J* = 14.5 Hz, 1H), 5.02 (d, *J* = 14.5 Hz, 1H), 4.40 (s, 2H), 2.62 (d, *J* = 4.7 Hz, 3H), 1.98 (dd, *J* = 9.4, 1.7 Hz, 3H), 1.76 (dd, *J* = 9.5, 1.7 Hz, 3H). ¹⁹F NMR (376 MHz, DMSO-d6) δ -102.96 - -103.17 (m, 1F), -109.19 - -109.29 (m, 2F), -109.63 (d, *J* = 9.2 Hz, 1F).

### Example 35: Preparation of compounds 35A and 35B

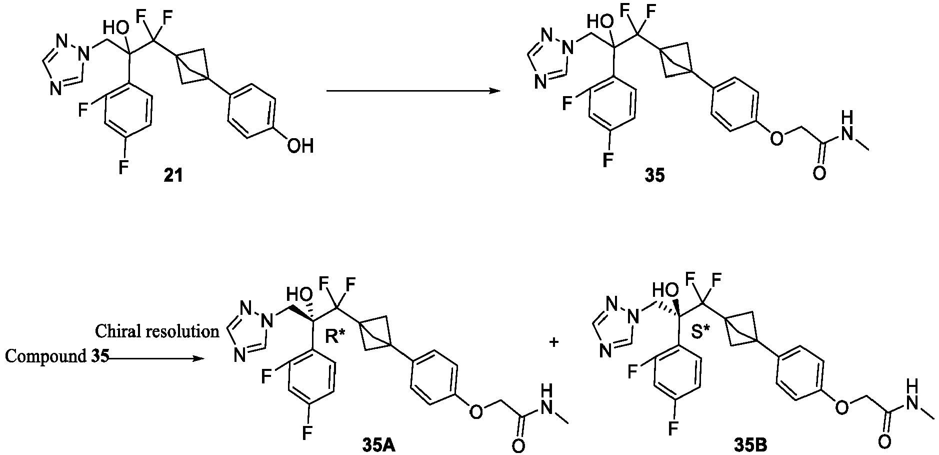

### Preparation of compound 35

Compound **21** (100 mg, 0.23 mmol) was dissolved in acetonitrile (5 mL), then potassium carbonate (32 mg, 0.46 mmol) was added thereto with stirring. After the reaction was carried out for 5 min, the reaction mixture was then added with 2-bromo-N-methylacetamide (35 mg, 0.46 mmol), stirred and reacted at 70°C for 3 hours. The reaction mixture was filtered, and the crude filtrate was purified by preparative separation (preparation method: chromatographic column: Agilent 10 Prep-C18 250 × 21.2 mm; column temperature: 25°C; mobile phase: water (0.1% FA)-acetonitrile; proportion of acetonitrile in the mobile phase: 35% to 55% in 12 min; flow rate: 30 mL/min) to obtain the title compound **35** (90 mg, yield: 77%, containing a pair of enantiomers). LC-MS (ESI): m/z 505.2 [M+H]⁺. ¹H NMR (400 MHz, DMSO-d6) δ 8.36 (s, 1H), 7.99 (d, *J* = 4.9 Hz, 1H), 7.69 (s, 1H), 7.56 (td, *J* = 9.0, 6.8 Hz, 1H), 7.20 (ddd, *J* = 12.0, 9.1, 2.6 Hz, 1H), 7.08 - 7.01 (m, 2H), 6.97 (td, *J* = 8.4, 2.6 Hz, 1H), 6.89 (s, 1H), 6.88 - 6.79 (m, 2H), 5.13 (d, *J* = 14.4 Hz, 1H), 4.76 (d, *J* = 14.4 Hz, 1H), 4.39 (s, 2H), 2.62 (d, *J* = 4.6 Hz, 3H), 1.96 (dd, *J* = 9.5, 1.7 Hz, 3H), 1.74 (dd, *J* = 9.5, 1.7 Hz, 3H). ¹⁹F NMR (376 MHz, DMSO-d6) δ -102.41 - -102.89 (m, 1F), -109.28 - -109.73 (m, 2F), -110.46 (d, *J =* 9.5 Hz, 1F).

### Preparation of compounds 35A and 35B

Compound **35** (87 mg) was subjected to SFC chiral preparative resolution (preparative separation method, instrument model: MG II preparative SFC (SFC-14); model of chromatographic column: ChiralCel OX, 250 × 30 mm I.D., 5 µm; mobile phase: A: CO₂ B: ethanol (0.1% NH₃ H₂O); elution gradient: B 30%; flow rate: 60 mL/min; column pressure: 100 bar; column temperature: 38°C; detection wavelength: 220 nm; cycle: about 11.5 min) to obtain the title compounds **35A** (39 mg, single enantiomer) and **35B** (39 mg, single enantiomer).

Compound **35A:** LC-MS (ESI): m/z 505.0 [M+H]⁺. ¹H NMR (400 MHz, DMSO-*d6)* δ 8.36 (s, 1H), 8.05 - 7.91 (m, 1H), 7.69 (s, 1H), 7.56 (td, *J* = 8.9, 6.7 Hz, 1H), 7.20 (ddd, *J* = 12.0, 9.1, 2.6 Hz, 1H), 7.07 -7.01 (m, 2H), 6.97 (td, *J* = 8.5, 2.7 Hz, 1H), 6.89 (s, 1H), 6.87 - 6.81 (m, 2H), 5.14 (d, *J* = 14.5 Hz, 1H), 4.76 (d, *J* = 14.5 Hz, 1H), 4.40 (s, 2H), 2.62 (d, *J =* 4.7 Hz, 3H), 1.96 (dd, *J* = 9.5, 1.7 Hz, 3H), 1.74 (dd, *J* = 9.5, 1.7 Hz, 3H). ¹⁹F NMR (376 MHz, DMSO-d6) δ -102.37 - -102.91 (m, 1F), -109.30 - -109.68 (m, 2F), -110.46 (d, *J* = 8.5 Hz, 1F).

Compound **35B:** LC-MS (ESI): 505.0 [M+H]⁺. ¹H NMR (400 MHz, DMSO-d6) δ 8.36 (s, 1H), 8.04 - 7.94 (m, 1H), 7.69 (s, 1H), 7.56 (td, *J* = 9.0, 6.8 Hz, 1H), 7.20 (ddd, *J =* 12.0, 9.1, 2.7 Hz, 1H), 7.08 - 7.01 (m, 2H), 6.97 (td, *J* = 8.5, 2.7 Hz, 1H), 6.89 (s, 1H), 6.88 - 6.80 (m, 2H), 5.14 (d, *J* = 14.5 Hz, 1H), 4.76 (d, *J* = 14.4 Hz, 1H), 4.40 (s, 2H), 2.62 (d, *J =* 4.6 Hz, 3H), 1.96 (dd, *J* = 9.4, 1.7 Hz, 3H), 1.74 (dd, *J* = 9.4, 1.7 Hz, 3H). ¹⁹F NMR (376 MHz, DMSO-*d6*) δ -102.41 - -102.88 (m, 1F), -109.27 - -109.75 (m, 2F), -110.46 (d, *J* = 9.1 Hz, 1F).

### Example 36: Preparation of compounds 36A and 36B

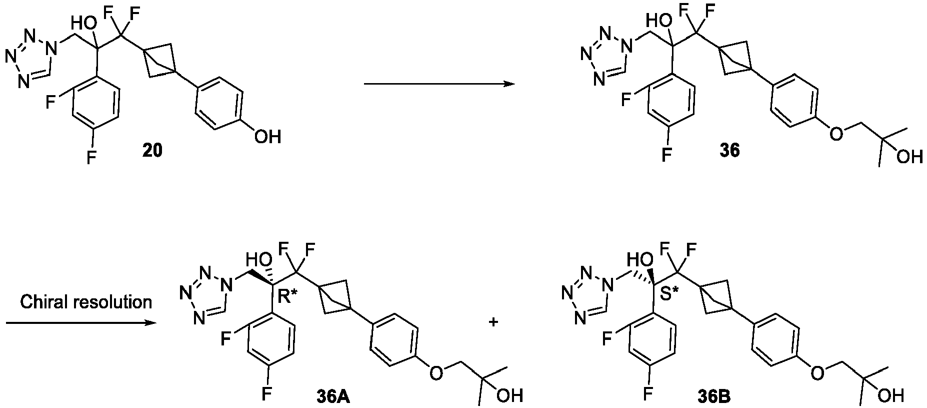

### Preparation of compound 36

To a sealed tube were added compound **20** (100 mg, 0.23 mmol), potassium carbonate (127 mg, 0.92 mmol), isobutylene oxide (CAS: 558-30-5, 132 mg, 1.84 mmol), and acetonitrile (1 mL), and the reaction mixture was reacted at 70°C for 16 hours. After the reaction system was cooled, the reaction mixture was filtered, and the crude filtrate was purified by preparative separation (chromatographic column: Welch Xtimate^{®} C18 21.2 × 250 mm; column temperature: 25°C; mobile phase: water (10 mM/L NH₄HCO₃)-acetonitrile; proportion of acetonitrile in the mobile phase: 45% to 65% in 12 min; flow rate: 30 mL/min) to obtain the title compound **36** (55 mg, yield: 47%).

Compound **36:** LC-MS (ESI): 507.20 [M+H]⁺. ¹H NMR (400 MHz, Chloroform-*d*) δ 8.56 (br.s, 1H), 7.70 - 7.59 (m, 1H), 7.04 - 6.97 (m, 2H), 6.91 - 6.77 (m, 4H), 5.48 (d, *J =* 14.3 Hz, 1H), 5.05 (d, *J* = 14.4 Hz, 1H), 3.74 (s, 2H), 2.04 (dd, *J* = 9.4, 1.8 Hz, 3H), 1.85 (dd, *J* = 9.4, 1.8 Hz, 3H), 1.32 (s, 6H). ¹⁹F NMR (376 MHz, Chloroform-*d*) δ -106.95 (m, 1F), - 110.15 (m, 2F), -110.33 (m, 1F).

### Preparation of compounds 36A and 36B

Compound **36** (150 mg) was subjected to SFC chiral preparative resolution (preparative separation method, instrument model: Waters UPC2 analytical SFC (SFC-H)); model of chromatographic column: ChiralPak AD, 150 × 4.6 mm I.D., 3 µm; mobile phase: A: CO₂ B: methanol (0.05% DEA); elution gradient: B 40%; flow rate: 2.5 mL/min; column pressure: 100 bar; column temperature: 35°C; detection wavelength: 220 nm; cycle: about 2.8 min) to obtain the title compounds 36A (70 mg, single enantiomer) and 36B (67 mg, single enantiomer).

Compound **36A:** LC-MS (ESI): 507.00 [M+H]⁺. Chiral analysis method (model of chromatographic column: ChiralPak AD, 250 × 30 mm I.D., 10 µm; mobile phase: A: CO₂ B: methanol (0.05% DEA); elution gradient: the mobile phase increased from 5% B to 40% B within 5 min and maintained at 40% B for 2.5 min for elution, then 5% B was equilibrated for 2.5 min; flow rate: 70 mL/min; column temperature: 35°C; column pressure: 1500 psi; detection wavelength: 220 nm; Rt = 2.490 min). ¹H NMR (400 MHz, DMSO-*d6*) δ 9.13 (s, 1H), 7.64 - 7.47 (m, 1H), 7.34 - 7.21 (m, 1H), 7.14 (s, 1H), 7.07 - 6.96 (m, 3H), 6.86 - 6.77 (m, 2H), 5.42 (d, *J* = 14.5 Hz, 1H), 5.00 (d, *J* = 14.6 Hz, 1H), 4.59 (s, 1H), 3.63 (s, 2H), 2.06 - 1.89 (m, 3H), 1.85 - 1.68 (m, 3H), 1.16 (s, 6H). ¹⁹F NMR (376 MHz, DMSO-*d6*) δ -102.67 - -103.41 (m, 1F), -108.99 - -109.37 (m, 2F), -109.64 (m, 1F).

Compound **36B:** LC-MS (ESI): 507.00 [M+H]⁺. Chiral analysis method (model of chromatographic column: ChiralPak AD, 250 × 30 mm I.D., 10 µm; mobile phase: A: CO₂ B: methanol (0.05% DEA); elution gradient: the mobile phase increased from 5% B to 40% B within 5 min and maintained at 40% B for 2.5 min for elution, then 5% B was equilibrated for 2.5 min; flow rate: 70 mL/min; column temperature: 35°C; column pressure: 1500 psi; detection wavelength: 220 nm; Rt = 1.989 min). ¹H NMR (400 MHz, DMSO-d6) δ 9.13 (s, 1H), 7.60 - 7.49 (m, 1H), 7.34 - 7.24 (m, 1H), 7.14 (s, 1H), 7.06 - 6.96 (m, 3H), 6.85 - 6.78 (m, 2H), 5.42 (d, *J* = 14.5 Hz, 1H), 5.01 (d, *J* = 14.6 Hz, 1H), 4.59 (s, 1H), 3.64 (s, 2H), 2.02 - 1.93 (m, 3H), 1.81 - 1.69 (m, 3H), 1.16 (s, 6H). ¹⁹F NMR (376 MHz, DMSO-*d6*) δ -102.42 - -103.89 (m, 1F), -109.08 - -109.43 (m, 2F), -109.64 (m, 1F).

### Example 37: Preparation of compounds 37A and 37B

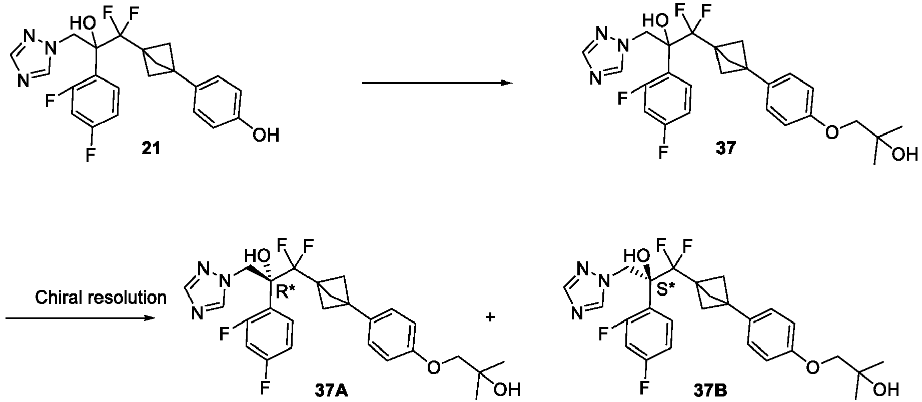

### Preparation of compound 37

To a sealed tube was added compound **21** (100 mg, 0.23 mmol), then potassium carbonate (127 mg, 0.92 mmol), isobutylene oxide (CAS: 558-30-5, 132 mg, 1.84 mmol), DMF (1 mL) were added thereto, and the reaction system was kept at 100°C for 2 days. After the reaction system was cooled, the reaction mixture was filtered, and the crude filtrate was purified by preparative separation (chromatographic column: Welch Xtimate^{®} C18 21.2 × 250 mm; column temperature: 25°C; mobile phase: water (10 mM/L NH₄HCO₃)-acetonitrile; proportion of acetonitrile in the mobile phase: 55% to 75% in 12 min; flow rate: 30 mL/min) to obtain the title compound **37** (61 mg, yield: 53%).

Compound **37:** LC-MS (ESI): m/z 506.2 [M+H]⁺. ¹H NMR (400 MHz, Chloroform-*d*) δ 8.13 (br.s, 1H), 7.84 (s, 1H), 7.79 - 7.67 (m, 1H), 7.07 - 6.98 (m, 2H), 6.88 - 6.70 (m, 4H), 5.34 (br.s, 1H), 5.21 (d, *J* = 14.4 Hz, 1H), 4.84 (d, *J* = 14.4 Hz, 1H), 3.75 (s, 2H), 2.25 (m, 1H), 2.06 (dd, *J* = 9.4, 1.8 Hz, 3H), 1.90 (dd, *J* = 9.4, 1.8 Hz, 3H), 1.32 (s, 6H). ¹⁹F NMR (376 MHz, Chloroform-*d*) δ -104.77 - 105.49 (m, 1F), -108.21 - 108.62 (m, 1F), -110.53 - 111.25 (m, 2F).

### Preparation of compounds 37A and 37B

Compound **37** (50 mg) was subjected to SFC chiral preparative resolution (preparative separation method, instrument model: MG II preparative SFC (SFC-14); model of chromatographic column: ChiralPak IG, 250 × 30 mm I.D., 10 µm; mobile phase: A: CO₂ B: ethanol (0.1% NH₃H₂O); elution gradient: B 30%; flow rate: 70 mL/min; column pressure: 100 bar; column temperature: 38°C; detection wavelength: 220 nm; cycle: about 5 min) to obtain the title compounds **37A** (23 mg, single enantiomer) and **37B** (25 mg, single enantiomer).

Compound **37A:** LC-MS (ESI): m/z 506.00 [M+H]⁺. Chiral analysis method (model of chromatographic column: Waters UPC2 analytical SFC (SFC-H); mobile phase: A: CO₂ B: ethanol (0.05% DEA); elution gradient: B 30%; flow rate: 2.5 mL/min; column temperature: 35°C; column pressure: 100 psi; detection wavelength: 220 nm; Rt = 2.528 min); ¹H NMR (400 MHz, DMSO-*d6*) δ 8.36 (s, 1H), 7.68 (s, 1H), 7.59 - 7.53 (m, 1H), 7.22 - 7.16 (m, 1H), 7.03 - 6.96 (m, 3H), 6.88 (s, 1H), 6.83 - 6.79 (m, 2H), 5.13 (d, *J* = 14.4 Hz, 1H), 4.76 (d, *J* = 14.4 Hz, 1H), 4.59 (s, 1H), 3.63 (s, 2H), 1.96 (dd, *J* = 9.4, 1.8 Hz, 3H), 1.73 (dd, *J =* 9.4, 1.8 Hz, 3H), 1.16 (s, 6H). ¹⁹F NMR (376 MHz, DMSO-d6) δ -102.51 - -102.73 (m, 1F), -109.42 - -109.52 (m, 2F), -110.47 - -110.49 (m, 1F).

Compound **37B:** LC-MS (ESI): m/z 506.20 [M+H]⁺. Chiral analysis method (model of chromatographic column: Waters UPC2 analytical SFC (SFC-H); mobile phase: A: CO₂ B: ethanol (0.05% DEA); elution gradient: B 30%; flow rate: 2.5 mL/min; column temperature: 35°C; column pressure: 100 psi; detection wavelength: 220 nm; Rt = 2.516 min). ¹H NMR (400 MHz, DMSO-*d6*) δ 8.36 (s, 1H), 7.68 (s, 1H), 7.59 - 7.53 (m, 1H), 7.22 - 7.16 (m, 1H), 7.03 - 6.96 (m, 3H), 6.88 (s, 1H), 6.83 - 6.79 (m, 2H), 5.13 (d, *J* = 14.4 Hz, 1H), 4.76 (d, *J =* 14.4 Hz, 1H), 4.59 (s, 1H), 3.63 (s, 2H), 1.96 (dd, *J* = 9.4, 1.8 Hz, 3H), 1.73 (dd, *J =* 9.4, 1.8 Hz, 3H), 1.16 (s, 6H). ¹⁹F NMR (376 MHz, DMSO-d6) δ -102.51 - -102.73 (m, 1F), -109.42 - -109.52 (m, 2F), -110.46 - -110.49 (m, 1F).

### Example 38: Preparation of compound 38

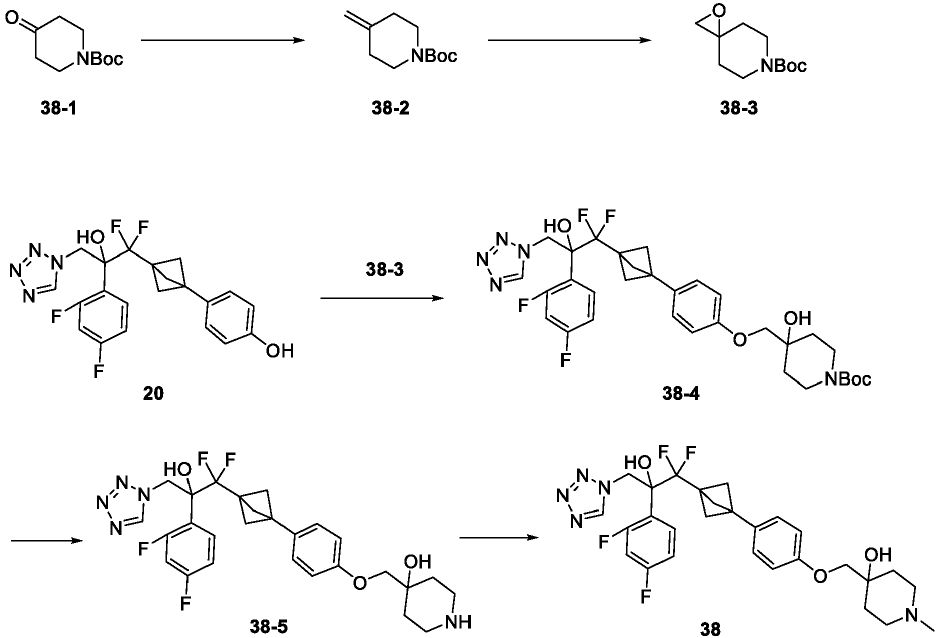

### Preparation of compound 38-2

To a three-necked flask was added compound methyltriphenylphosphonium bromide (CAS: 1779-49-3, 4.86 g, 12 mmol), and the system was replaced with nitrogen three times. To the three-necked flask was added anhydrous THF (50 mL), and n-butyllithium (1.6 M, 7.5 mL, 12 mmol) was added dropwise thereto at a cooling temperature of -78°C. After the reaction system was stirred and reacted at -78°C for 30 min, a solution of compound **38-1** (2 g, 10 mmol) in THF (10 mL) was added thereto, and the reaction mixture was slowly warmed to room temperature and reacted for 16 hours. After the reaction was completed, the reaction mixture was quenched by adding ice water (10 mL), extracted with EtOAc (50 mL × 3), and the organic phases were combined, dried, and concentrated to obtain a crude product. The crude product was purified by normal-phase silica gel column chromatography (EtOAc/PE = 0 to 10%) to obtain the title compound **38-2** (1.2 g, yield: 60%) as a light yellow liquid. ¹H NMR (400 MHz, Chloroform-*d*) δ 4.74 (s, 2H), 3.42 (m, 4H), 2.18 (m, 4H), 1.47 (s, 9H).

### Preparation of compound 38-3

To a three-necked flask was added compound **38-2** (1.2 g, 6.1 mmol), and the system was replaced with nitrogen three times. To the three-necked flask was sequentially added anhydrous DCM (20 mL), cooled to 0°C, and then *m*-CPBA (purity: 85%, 1.84 g, 9.15 mmol) was added thereto. After the reaction system was stirred for 3 hours, the reaction was quenched with saturated sodium sulfite aqueous solution, filtered, and the filtrate was evaporated to dryness by rotary evaporation, then slurried with petroleum ether (20 mL), filtered, and the filtrate was concentrated to obtain the title compound **38-3** (800 mg, yield: 26.8%) as a light yellow liquid. ¹H NMR (400 MHz, Chloroform-*d*) δ 3.74 (m, 2H), 3.46 - 3.40 (m, 2H), 2.70 (s, 2H), 1.84 - 1.77 (m, 2H), 1.58 (s, 9H), 1.50 - 1.42 (m, 2H).

### Preparation of compound 38-4

Compound **38-3** (35 mg, 0.16 mmol) was dissolved in DMF solution (1.5 mL), and compound **20** (50 mg, 0.11 mmol) and cesium carbonate (72 mg, 0.22 mmol) were added thereto. The reaction system was stirred for 16 hours at 60°C in a sealed tube. After the reaction system was cooled, the reaction mixture was poured into water (20 mL), extracted with EtOAc (50 mL × 3), and the organic phases were combined and concentrated to obtain the title compound **38-4** (68 mg, crude product) as a yellow oil. The crude compound was directly used as the raw material for the next reaction step. LC-MS (ESI): m/z 648.3 [M+H]⁺.

### Preparation of compound 38-5

To a three-necked flask was added compound **38-4** (68 mg, crude product), and the system was replaced with nitrogen three times. To the three-necked flask was added a methanol solution of hydrochloric acid (4.0 M, 10 mL), stirred and reacted at room temperature for 3 hours. The reaction mixture was concentrated to obtain the title compound **38-5** (60 mg, crude product) as a yellow oil, and the crude compound was directly used as the raw material for the next reaction step. LC-MS (ESI): m/z 548.2 [M+H]⁺.

### Preparation of compound 38

Compound **38-5** (60 mg, crude product) was dissolved in MeOH (3.0 mL), and formaldehyde aqueous solution (40%, 0.5 mL, 8.3 mmol) was added thereto. The mixture was stirred at room temperature for 1 hour, and then sodium cyanoborohydride (20.7 mg, 0.33 mmol) was added thereto. The reaction system was stirred at room temperature for 1 hour. After the reaction was completed, the reaction mixture was filtered, and the crude filtrate was purified by preparative separation (chromatographic column: Welch Xtimate^{®} C18 21.2 × 250 mm; column temperature: 25°C; mobile phase: water (10 mM/L NH₄HCO₃)-acetonitrile; proportion of acetonitrile in the mobile phase: 15% to 45% in 12 min; flow rate: 30 mL/min) to obtain the title compound **38** (6.4 mg, yield: 49.6%, containing a pair of enantiomers) as a white solid.

Compound 38: LC-MS (ESI): m/z 562.2 [M+H]⁺. ¹H NMR (400 MHz, DMSO-d6) δ 9.14 (s, 1H), 7.57 - 7.52 (m, 1H), 7.32 - 7.29 (m, 1H), 7.26 (s, 1H), 7.15 - 6.98 (m, 3H), 6.83 - 6.81 (m, 2H), 5.43 (d, *J* = 12 Hz, 1H), 5.01 (d, *J* = 12 Hz, 1H), 4.67 (br.s, 1H), 3.70 (s, 2H), 2.54 - 2.53 (m, 2H), 2.50 - 2.48 (m, 2H), 1.98 - 1.96 (m, 4H), 1.77 - 1.74 (m, 6H), 1.73 - 1.57 (m, 3H). ¹⁹F NMR (376 MHz, DMSO-*d6*) δ -102.95 - -103.19 (m, 1F), -109.21 - -109.31 (m, 2F), -109.64 (m, 1F).

### Example 39: Preparation of compound 39

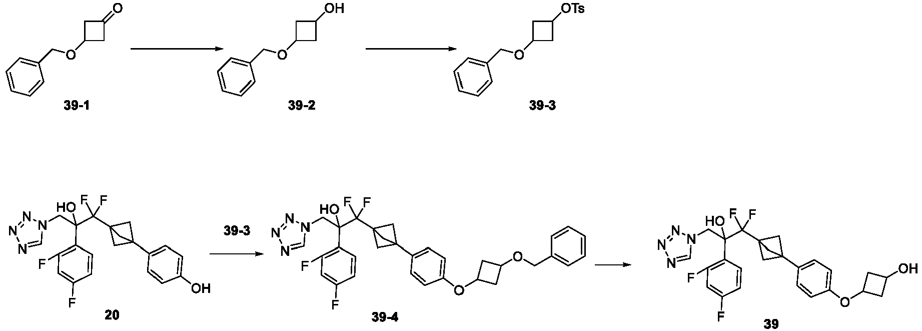

### Preparation of compound 39-2

Compound **39-1** (500 mg, 2.84 mmol) was dissolved in anhydrous tetrahydrofuran (20 mL), then lithium aluminum hydride (140 mg, 3.69 mmol) was added thereto in batches at 0°C, and the reaction mixture was reacted at room temperature for 2 hours. The reaction was tracked by TLC until the reaction was completed, then quenched by dropwise addition of ice water. The reaction system was extracted with EtOAc (20 mL × 3), and the organic phases were combined, dried, and concentrated to obtain 380 mg of crude title compound, which was directly used as the raw material for the next reaction step. LC-MS (ESI): m/z 196.2 [M+NH₄]⁺.

### Preparation of compound 39-3

Compound **39-2** (380 mg, 2.13 mmol) was dissolved in dichloromethane (10 mL), then triethylamine (0.74 mL) was added dropwise thereto at 0°C. After the reaction was carried out for 10 min, p-toluenesulfonyl chloride (816 mg, 4.27 mmol) was added thereto, reacted at room temperature for 4 hours. After the reaction was tracked by TLC until the reaction was completed, the reaction system was extracted with DCM (20 mL × 3), and the organic phases were combined, dried, and concentrated to obtain a crude product. The crude product was purified by normal-phase silica gel column chromatography (EtOAc/PE = 0 to 10%) to obtain the title compound **39-3** (260 mg, yield: 37%). ¹H NMR (400 MHz, DMSO-*d6)* δ 7.82 - 7.73 (m, 2H), 7.52 - 7.43 (m, 2H), 7.38 - 7.22 (m, 5H), 4.55 - 4.42 (m, 1H), 4.31 (s, 2H), 3.71 - 3.56 (m, 1H), 2.59 - 2.51 (m, 2H), 2.42 (s, 3H), 1.98 - 1.89 (m, 2H).

### Preparation of compound 39-4

Compound **39-3** (100 mg, 0.23 mmol) was dissolved in DMF (5.0 mL), then cesium carbonate (150 mg, 0.46 mmol) was added thereto with stirring. After the reaction was carried out for 5 min, the reaction mixture was then added with compound **20** (99 mg, 0.3 mmol), stirred and reacted at 50°C for 4 hours. The reaction mixture was filtered, and the crude filtrate was purified by preparative separation (preparation method: chromatographic column: Agilent 10 Prep-C18 250 × 21.2 mm; column temperature: 25°C; mobile phase: water (0.1% TFA)-acetonitrile; proportion of acetonitrile in the mobile phase: 45% to 65% in 12 min; flow rate: 30 mL/min) to obtain the title compound **39-4** (280 mg yield: 20%). LC-MS (ESI): m/z 595.2 [M+H]⁺.

### Preparation of compound 39

Compound **39-4** (28 mg, 0.05 mmol) was dissolved in a mixed solvent of methanol (5 mL) and formic acid (0.5 mL), then 10% wet palladium on carbon (5 mg) was added thereto. The system was replaced with nitrogen three times, and then reacted at room temperature for 16 hours. The reaction mixture was filtered, and the crude filtrate was purified by preparative separation (preparation method: chromatographic column: Agilent 10 Prep-C18 250 × 21.2 mm; column temperature: 25°C; mobile phase: water (0.1% TFA)-acetonitrile; proportion of acetonitrile in the mobile phase: 50% to 70% in 12 min; flow rate: 30 mL/min) to obtain the title compound **39** (10 mg, yield: 42%, containing two pairs of enantiomers).

Compound **39:** LC-MS (ESI): m/z 505.2 [M+H]⁺. ¹H NMR (400 MHz, DMSO-^6) δ 9.13 (s, 1H), 7.53 (td, *J* = 9.0, 6.8 Hz, 1H), 7.28 (ddd, *J* = 12.0, 9.1, 2.6 Hz, 1H), 7.15 (s, 1H), 7.05 - 6.95 (m, 3H), 6.73 - 6.63 (m, 2H), 5.42 (d, *J* = 14.6 Hz, 1H), 5.15 (d, *J* = 5.4 Hz, 1H), 5.01 (d, *J* = 14.6 Hz, 1H), 4.81 - 4.69 (m, 1H), 4.39 - 4.23 (m, 1H), 2.24 (t, *J* = 5.6 Hz, 4H), 1.96 (dd, *J* = 9.5, 1.6 Hz, 3H), 1.75 (dd, *J* = 9.4, 1.6 Hz, 3H). ¹⁹F NMR (376 MHz, DMSO-d6) δ -102.97 - -103.19 (m, 1F), -109.20- -109.30 (m, 2F), -109.65 (d, *J* = 9.6 Hz, 1F).

### Example 40: Preparation of compounds 40A and 40B

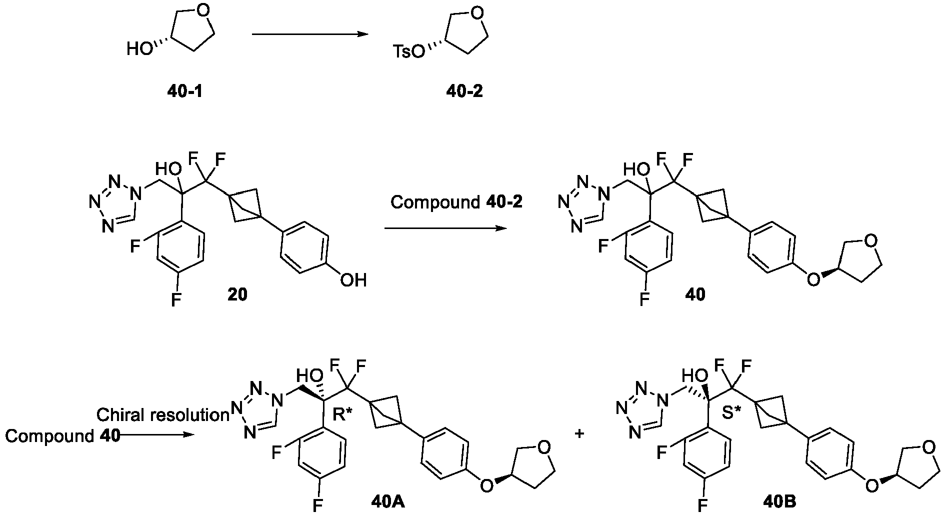

### Preparation of compound 40-2

Compound **40-1** (500 mg, 5.68 mmol) was dissolved in dichloromethane (10 mL), then triethylamine (1 mL) was added dropwise thereto at 0°C. After the reaction was carried out for 10 min, p-toluenesulfonyl chloride (1.19 g, 6.25 mmol) was added thereto, reacted at room temperature for 4 hours. The reaction system was extracted with DCM (20 mL × 3), and the organic phases were combined, dried, and concentrated to obtain the title compound **40-2** (1 g, crude product), and the crude compound was directly used in the next reaction step.

### Preparation of compound 40

Compound **20** (120 mg, 0.28 mmol) was dissolved in DMF (5 mL) solvent, then cesium carbonate (180 mg, 0.55 mmol) was added thereto with stirring. After the reaction was carried out for 5 min, the reaction mixture was then added with compound **40-2** (134 mg, 0.55 mmol), stirred and reacted at 50°C for 4 hours. The reaction mixture was filtered, and the crude filtrate was purified by preparative separation (preparation method: chromatographic column: Agilent 10 Prep-C18 250 × 21.2 mm; column temperature: 25°C; mobile phase: water (0.1% FA)-acetonitrile; proportion of acetonitrile in the mobile phase: 45% to 65% in 12 min; flow rate: 30 mL/min) to obtain the title compound **40** (54 mg, yield: 39%, containing a pair of diastereoisomers). LC-MS (ESI): m/z 505.2 [M+H]⁺. ¹H NMR (400 MHz, DMSO-*d6*) δ 9.13 (s, 1H), 7.54 (td, *J* = 9.0, 6.7 Hz, 1H), 7.28 (ddd, *J* = 12.0, 9.1, 2.6 Hz, 1H), 7.14 (s, 1H), 7.07 - 6.97 (m, 3H), 6.85 - 6.76 (m, 2H), 5.42 (d, *J* = 14.5 Hz, 1H), 5.02 (d, *J* = 14.5 Hz, 1H), 4.99 - 4.93 (m, 1H), 3.90 - 3.67 (m, 4H), 2.24 - 2.10 (m, 1H), 1.97 (dd, *J* = 9.4, 1.7 Hz, 3H), 1.94 - 1.86 (m, 1H), 1.76 (dd, *J* = 9.4, 1.6 Hz, 3H). ¹⁹F NMR (376 MHz, DMSO-*d6*) δ - 102.97 - -103.19 (m, 1F), -109.21 - -109.32 (m, 2F), -109.65 (d, *J* = 9.6 Hz, 1F).

### Preparation of compounds 40A and 40B

Compound **40** (50 mg) was subjected to SFC chiral preparative resolution (preparative separation method, instrument model: MG II preparative SFC(SFC-14); model of chromatographic column: ChiralPak IC, 250 × 30 mm I.D.10 µm; mobile phase: A: CO₂ B: ethanol (0.1% NH₃H₂O); elution gradient: B 30%; flow rate: 70 mL/min; column pressure: 100 bar; column temperature: 38°C; detection wavelength: 220 nm; cycle: about 7 min) to obtain the title compounds **40A** (25 mg, single enantiomer) and **40B** (23 mg, single enantiomer).

Compound **40A:** LC-MS (ESI): 505.0 [M+H]⁺. ¹H NMR (400 MHz, DMSO-d6) δ 9.13 (s, 1H), 7.54 (td, *J* = 9.0, 6.7 Hz, 1H), 7.28 (ddd, *J* = 12.0, 9.1, 2.6 Hz, 1H), 7.14 (s, 1H), 7.06 - 6.96 (m, 3H), 6.83 - 6.76 (m, 2H), 5.42 (d, *J* = 14.5 Hz, 1H), 5.02 (d, *J* = 14.5 Hz, 1H), 4.99 - 4.93 (m, 1H), 3.91 - 3.65 (m, 4H), 2.23 - 2.11 (m, 1H), 1.97 (dd, *J* = 9.5, 1.7 Hz, 3H), 1.94 - 1.85 (m, 1H), 1.76 (dd, *J* = 9.5, 1.7 Hz, 3H). ¹⁹F NMR (376 MHz, DMSO-*d6*) δ - 102.97 - -103.18 (m, 1F), -109.20 - -109.31 (m, 2F), -109.64 (d, *J =* 9.2 Hz, 1F).

Compound **40B:** LC-MS (ESI): 505.0 [M+H]⁺. ¹H NMR (400 MHz, DMSO-*d6*) δ 9.14 (s, 1H), 7.54 (td, *J* = 9.0, 6.7 Hz, 1H), 7.29 (ddd, *J* = 12.0, 9.1, 2.6 Hz, 1H), 7.14 (s, 1H), 7.07 - 6.97 (m, 3H), 6.85 - 6.76 (m, 2H), 5.42 (d, *J* = 14.5 Hz, 1H), 5.02 (d, *J* = 14.5 Hz, 1H), 4.99 - 4.93 (m, 1H), 3.93 - 3.66 (m, 4H), 2.24 - 2.11 (m, 1H), 1.98 (dd, *J* = 9.5, 1.7 Hz, 3H), 1.95 - 1.85 (m, 1H), 1.76 (dd, *J* = 9.5, 1.7 Hz, 3H). ¹⁹F NMR (376 MHz, DMSO-d6) δ - 102.97 - -103.18 (m, 1F), -109.20 - -109.31 (m, 2F), -109.64 (d, *J* = 9.2 Hz, 1F).

### Example 41: Preparation of compounds 41A and 41B

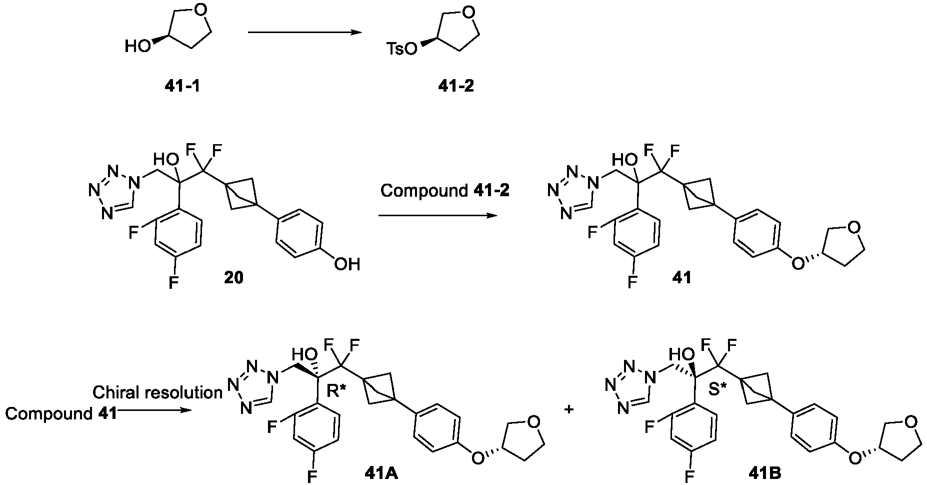

### Preparation of compound 41-2

Compound **41-1** (500 mg, 5.68 mmol) was dissolved in dichloromethane (10 mL), then triethylamine (1 mL) was added dropwise thereto at 0°C. After the reaction system was stirred for 10 min, p-toluenesulfonyl chloride (1.19 g, 6.25 mmol) was added thereto, reacted at room temperature for 4 hours. The reaction system was extracted with DCM (20 mL × 3), and the organic phases were combined, dried, and concentrated to obtain the title compound **41-2** (1 g, crude product), and the crude compound was directly used in the next reaction step.

### Preparation of compound 41

Compound **20** (120 mg, 0.28 mmol) was dissolved in DMF (5 mL), then cesium carbonate (180 mg, 0.55 mmol) was added thereto with stirring. After the reaction was carried out for 5 min, the reaction mixture was then added with compound **41-2** (134 mg, 0.55 mmol), stirred and reacted at 50°C for 4 hours. The reaction mixture was filtered, and the crude filtrate was purified by preparative separation (preparation method: chromatographic column: Agilent 10 Prep-C18 250 × 21.2 mm; column temperature: 25°C; mobile phase: water (0.1% FA)-acetonitrile; proportion of acetonitrile in the mobile phase: 45% to 65% in 12 min; flow rate: 30 mL/min) to obtain the title compound **41** (88 mg, yield: 63%, containing a pair of diastereoisomers). LC-MS (ESI): m/z 505.2 [M+H]⁺. ¹H NMR (400 MHz, DMSO-*d6*) δ 9.06 (s, 1H), 7.47 (td, *J* = 9.0, 6.7 Hz, 1H), 7.21 (ddd, *J =* 12.0, 9.1, 2.6 Hz, 1H), 7.08 (s, 1H), 7.02 - 6.88 (m, 3H), 6.79 - 6.67 (m, 2H), 5.15 (dd, *J* = 167.1, 14.5 Hz, 2H), 4.92 - 4.86 (m, 1H), 3.84 - 3.60 (m, 4H), 2.17 - 2.03 (m, 1H), 1.90 (dd, *J* = 9.4, 1.7 Hz, 3H), 1.87 - 1.78 (m, 1H), 1.69 (dd, *J* = 9.5, 1.7 Hz, 3H). ¹⁹F NMR (376 MHz, DMSO-*d6*) δ -102.97 - -103.18 (m, 1F), -109.21 - -109.31 (m, 2F), -109.64 (d, *J* = 9.2 Hz, 1F).

### Preparation of compounds 41A and 41B

Compound **41** (88 mg) was subjected to SFC chiral preparative resolution (preparative separation method, instrument model: MG II preparative SFC(SFC-14); model of chromatographic column: ChiralPak IC, 250 × 30 mm I.D.10 µm; mobile phase: A: CO₂ B: ethanol(0.1% NH₃H₂O); elution gradient: B 30%; flow rate: 70 mL/min; column pressure: 100 bar; column temperature: 38°C; detection wavelength: 220 nm; cycle: about 10 min) to obtain the title compounds **41A** (38 mg, single diastereoisomer) and **41B** (41 mg, single diastereoisomer).

Compound **41A:** LC-MS (ESI): 505.2 [M+H]⁺. ¹H NMR (400 MHz, DMSO-*d6*) δ 9.13 (s, 1H), 7.54 (td, *J* = 9.0, 6.7 Hz, 1H), 7.28 (ddd, *J* = 12.1, 9.1, 2.6 Hz, 1H), 7.14 (s, 1H), 7.06 - 6.96 (m, 3H), 6.84 - 6.76 (m, 2H), 5.51 - 4.90 (m, 3H), 3.92 - 3.64 (m, 4H), 2.24 - 2.11 (m, 1H), 1.97 (dd, *J* = 9.4, 1.7 Hz, 3H), 1.94 - 1.84 (m, 1H), 1.76 (dd, *J* = 9.4, 1.7 Hz, 3H). ¹⁹F NMR (376 MHz, DMSO-*d6*) δ -102.97 - -103.18 (m, 1F), -109.19 - -109.30 (m, 2F), - 109.64 (d, *J* = 9.2 Hz, 1F).

Compound **41B:** LC-MS (ESI): 505.2 [M+H]⁺. ¹H NMR (400 MHz, DMSO-d6) δ 9.13 (s, 1H), 7.54 (td, *J* = 9.0, 6.7 Hz, 1H), 7.28 (ddd, *J* = 12.0, 9.1, 2.6 Hz, 1H), 7.14 (s, 1H), 7.06 - 6.97 (m, 3H), 6.84 - 6.77 (m, 2H), 5.53 - 4.89 (m, 3H), 3.93 - 3.66 (m, 4H), 2.23 - 2.11 (m, 1H), 1.97 (dd, *J* = 9.5, 1.7 Hz, 3H), 1.94 - 1.85 (m, 1H), 1.76 (dd, *J* = 9.5, 1.7 Hz, 3H). ¹⁹F NMR (376 MHz, DMSO-d6) δ -102.97 - -103.18 (m, 1F), -109.19 - -109.30 (m, 2F), - 109.64 (d, *J* = 9.2 Hz, 1F).

### Example 42: Preparation of compound 42

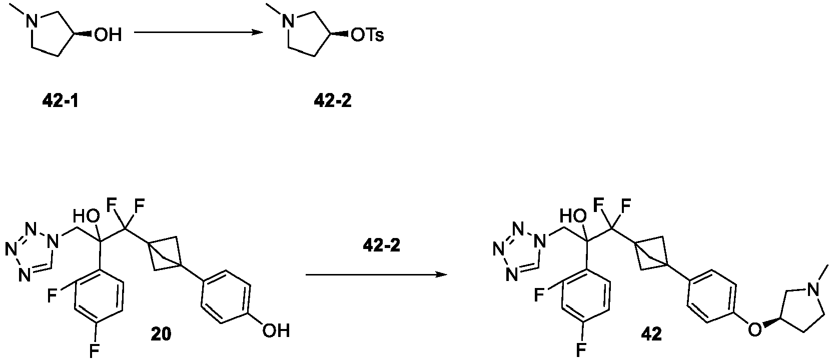

### Preparation of compound 42-2

Compound **42-1** (574 mg, 5.68 mmol) was dissolved in dichloromethane (10 mL), then triethylamine (1 mL) was added dropwise thereto at 0°C. After the reaction was carried out for 10 min, p-toluenesulfonyl chloride (1.19 g, 6.25 mmol) was added thereto, reacted at room temperature for 4 hours. The reaction was tracked by TLC until the reaction was completed, then the reaction system was extracted with DCM (20 mL × 3), and the organic phases were combined, dried, and concentrated to obtain 800 mg of crude title compound, which was directly used as the raw material for the next reaction step.

### Preparation of compound 42

Compound **20** (120 mg, 0.28 mmol) was dissolved in DMF (5 mL), then cesium carbonate (180 mg, 0.55 mmol) was added thereto under stirring. After the reaction was carried out for 5 min, the reaction mixture was then added with compound **42-2** (140 mg, 0.55 mmol), stirred and reacted at 50°C for 4 hours. The reaction mixture was filtered, and the crude filtrate was purified by preparative separation (preparation method: chromatographic column: Agilent 10 Prep-C18 250 × 21.2 mm; column temperature: 25°C; mobile phase: water (0.1% NH₄HCO₃)-acetonitrile; proportion of acetonitrile in the mobile phase: 40% to 60% in 12 min; flow rate: 30 mL/min) to obtain the title compound **42** (74 mg, yield: 52%, containing a pair of diastereoisomers).

Compound **42:** LC-MS (ESI): m/z 518.2 [M+H]⁺. ¹H NMR (400 MHz, DMSO-d6) δ 9.13 (s, 1H), 7.54 (td, *J* = 9.0, 6.7 Hz, 1H), 7.28 (ddd, *J* = 12.0, 9.1, 2.6 Hz, 1H), 7.14 (s, 1H), 7.00 (dt, *J* = 8.0, 2.8 Hz, 3H), 6.82 - 6.68 (m, 2H), 5.42 (d, *J* = 14.6 Hz, 1H), 5.01 (d, *J* = 14.6 Hz, 1H), 4.86 - 4.71 (m, 1H), 2.75 - 2.52 (m, 3H), 2.36 - 2.17 (m, 5H), 1.97 (dd, *J* = 9.4, 1.7 Hz, 3H), 1.75 (dd, *J* = 9.4, 1.7 Hz, 3H), 1.73 - 1.65 (m, 1H). ¹⁹F NMR (376 MHz, DMSO-*d6*) δ -102.97 - -103.19 (m, 1F), -109.20- -109.30 (m, 2F), -109.65 (d, *J* = 9.6 Hz, 1F).

### Example 43: Preparation of compound 43

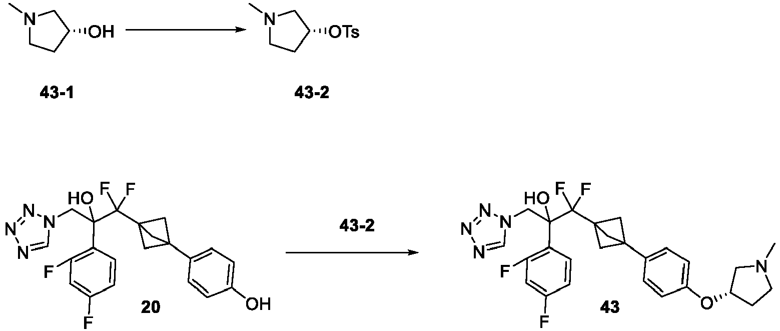

### Preparation of compound 43-2

Compound **43-1** (574 mg, 5.68 mmol) was dissolved in dichloromethane (10 mL), then triethylamine (1 mL) was added dropwise thereto at 0°C. After the reaction was carried out for 10 min, p-toluenesulfonyl chloride (1.19 g, 6.25 mmol) was added thereto, reacted at room temperature for 4 hours. The reaction was tracked by TLC until the reaction was completed, then the reaction system was extracted with DCM (20 mL × 3), and the organic phases were combined, dried, and concentrated to obtain 700 mg of crude title compound, which was directly used as the raw material for the next reaction step.

### Preparation of compound 43

Compound **20** (120 mg, 0.28 mmol) was dissolved in DMF (5 mL), then cesium carbonate (180 mg, 0.55 mmol) was added thereto under stirring. After the reaction was carried out for 5 min, the reaction mixture was then added with compound **43-2** (140 mg, 0.55 mmol), stirred and reacted at 50°C for 4 hours. The reaction mixture was filtered, and the crude filtrate was purified by preparative separation (preparation method: chromatographic column: Agilent 10 Prep-C18 250 × 21.2 mm; column temperature: 25°C; mobile phase: water (0.1% NH₄HCO₃)-acetonitrile; proportion of acetonitrile in the mobile phase: 40% to 60% in 12 min; flow rate: 30 mL/min) to obtain the title compound **43** (82 mg, yield: 57%, containing a pair of diastereoisomers).

Compound **43:** LC-MS (ESI): m/z 518.2 [M+H]⁺. ¹H NMR (400 MHz, DMSO-*d6*) δ 9.13 (s, 1H), 7.53 (td, *J* = 9.0, 6.7 Hz, 1H), 7.28 (ddd, *J* = 12.0, 9.1, 2.6 Hz, 1H), 7.14 (s, 1H), 7.05 - 6.95 (m, 3H), 6.79 - 6.72 (m, 2H), 5.43 (d, *J* = 14.6 Hz, 1H), 5.01 (d, *J* = 14.6 Hz, 1H), 4.88 - 4.73 (m, 1H), 2.83 - 2.55 (m, 3H), 2.43 - 2.35 (m, 1H), 2.30 - 2.21 (m, 4H), 1.97 (dd, *J* = 9.4, 1.7 Hz, 3H), 1.79 - 1.69 (m, 4H). ¹⁹F NMR (376 MHz, DMSO-d6) δ -102.97 - - 103.19 (m, 1F), -109.21- -109.31 (m, 2F), -109.64 (d, *J* = 9.6 Hz, 1F).

### Example 44: Preparation of compound 44

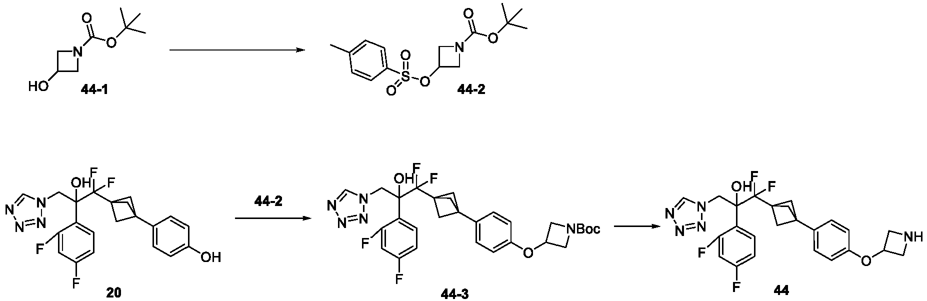

### Preparation of compound 44-2

Compound **44-1** (500 mg, 2.89 mmol) was dissolved in dichloromethane (10 mL), then triethylamine (321 mg, 3.18 mmol) was added dropwise thereto at 0°C. After the reaction was carried out for 10 min, p-toluenesulfonyl chloride (550 mg, 2.89 mmol) was added thereto, reacted at room temperature for 24 hours. After the reaction was tracked by TLC until the reaction was completed, the reaction system was extracted with DCM, and the organic phases were combined, dried, and concentrated. The resulting crude product was filtered through silica gel to obtain the title compound **44-2** (0.9 g, yield: 95%). ¹H NMR (400 MHz, Chloroform-*d*) δ 7.88 - 7.71 (m, 2H), 7.48 - 7.31 (m, 2H), 5.05 - 4.96 (m, 1H), 4.12 - 4.07 (m, 2H), 3.95 - 3.91 (m, 2H), 2.47 (s, 3H), 1.41 (s, 9H).

### Preparation of compound 44-3

Compound **20** (100 mg, 0.23 mmol) was dissolved in DMF (5 mL), then cesium carbonate (97 mg, 0.299 mmol) was added thereto with stirring. After the reaction was carried out for 5 min, the reaction mixture was then added with compound **44-2** (150 mg, 0.460 mmol), stirred and reacted at 50°C for 24 hours. The reaction mixture was filtered, and the filtrate was concentrated to obtain a crude product, which was purified by preparative separation and filtered through silica gel to obtain the title compound **44-3** (70 mg, yield: 52%, containing a pair of enantiomers). LC-MS (ESI): m/z 590.2 [M+H]⁺.

### Preparation of compound 44

Compound **44-3** (70 mg, 0.119 mmol) was dissolved in dichloromethane (5 mL), and hydrochloric acid in dioxane (4.0 M, 1 mL) was added thereto with stirring. After the reaction was carried out for 15 min, the reaction mixture was concentrated to obtain a crude product, and the crude product was purified by preparative separation (chromatographic column: Welch Xtimate@ C18 21.2 × 250 mm; column temperature: 25°C; mobile phase: water (10 mM/L NH₄HCO₃)-acetonitrile; proportion of acetonitrile in the mobile phase: 35% to 55% in 12 min; flow rate: 30 mL/min) to obtain the title compound **44** (30 mg, yield: 51%, containing a pair of enantiomers).

Compound **44:** LC-MS (ESI): m/z 490.0 [M+H]⁺. ¹H NMR (400 MHz, DMSO-d6) δ 9.14 (s, 1H), 7.59 - 7.48 (m, 1H), 7.31 - 7.25 (m, 1H), 7.19 (s, 1H), 7.08 - 6.96 (m, 3H), 6.72 - 6.64 (m, 2H), 5.43 (d, *J* = 14.6 Hz, 1H), 5.01 (d, *J* = 14.6 Hz, 1H), 4.94 - 4.84 (m, 1H), 3.74 - 3.70 (m, 2H), 3.46 - 3.42 (m, 2H), 1.98 - 1.95 (m, 3H), 1.76 - 1.74 (m, 3H). ¹⁹F NMR (376 MHz, DMSO-d6) δ -102.95- -103.16 (m, 1F), -109.18 - -109.31 (m, 2F), -109.64 (d, *J* = 9.6 Hz, 1F).

### Example 45: Preparation of compound 45

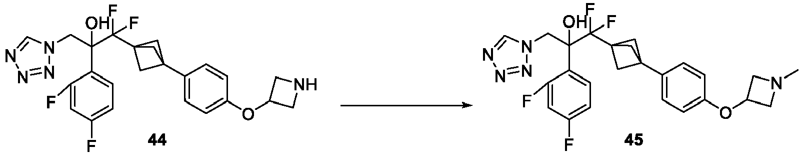

### Preparation of compound 45

Compound **44** (60 mg, 0.122 mmol) was dissolved in methanol (6 mL), then formaldehyde (37 mg, 38% aqueous solution) was added thereto with stirring. After stirring for 15 min, sodium triacetylborohydride (78 mg, 0.367 mmol) was added thereto, stirred and reacted for 1 hour. The reaction mixture was concentrated to remove most of the methanol solution, and the residue was purified by preparative separation (chromatographic column: Welch Xtimate@ C18 21.2 × 250 mm; column temperature: 25°C; mobile phase: water (10 mM/L NH₄HCO₃)-acetonitrile; proportion of acetonitrile in the mobile phase: 45% to 65% in 12 min; flow rate: 30 mL/min) to obtain the title compound **45** (25 mg, yield: 40%, a pair of enantiomers).

Compound **45:** LC-MS (ESI): m/z 490.0 [M+H]⁺. ¹H NMR (400 MHz, DMSO-d6) δ 9.13 (s, 1H), 7.58 - 7.49 (m, 1H), 7.31 - 7.25 (m, 1H), 7.14 (s, 1H), 7.02 - 6.98 (m, 3H), 6.72 - 6.68 (m, 2H), 5.42 (d, *J* = 14.6 Hz, 1H), 5.01 (d, *J* = 14.6 Hz, 1H), 4.67 (t, *J* = 5.6 Hz, 1H), 3.73 - 3.65 (m, 2H), 2.95 - 2.85 (m, 2H), 2.25 (s, 3H), 1.98 - 1.95 (m, 3H), 1.76 - 1.74 (m, 3H). ¹⁹F NMR (376 MHz, DMSO-*d6*) δ -102.98- -103.19 (m, 1F), -109.21 - -109.32 (m, 2F), -109.64 (d, *J* = 9.6 Hz, 1F).

### Example 46: Preparation of compounds 46A and 46B

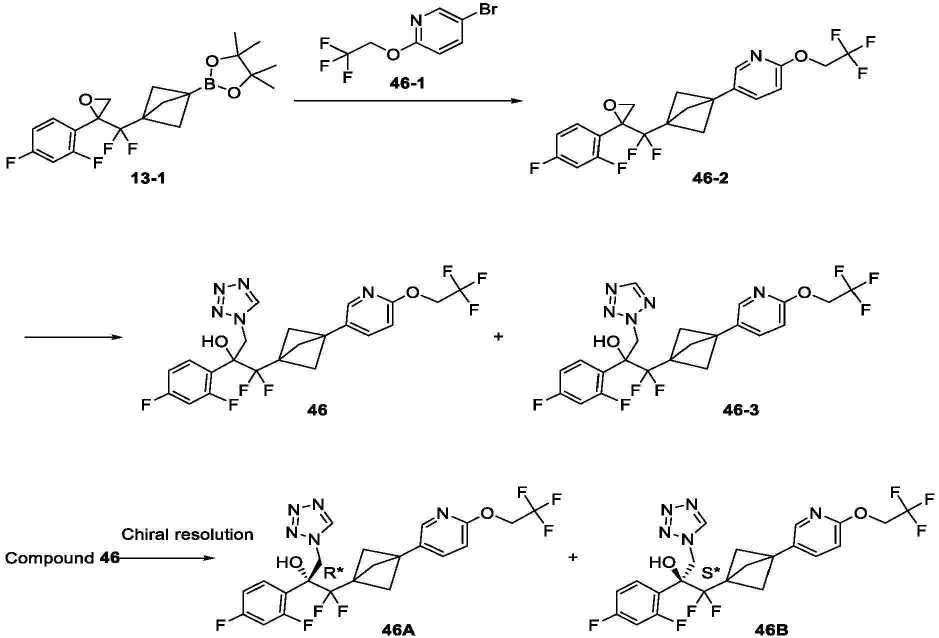

### Preparation of compound 46-2

Compound **13-1** (500 mg, 1.26 mmol) was dissolved in cyclopentyl methyl ether (10 mL) solvent, then compound butyldi-1-adamantylphosphine (cataCXium A, CAS: 321921-71-5, 90 mg, 0.25 mmol), cesium carbonate (1.23 g, 3.77 mmol), cuprous oxide (179 mg, 1.26 mmol), palladium acetate (159 mg, 1.15 mmol), compound **46-1** (482 mg, 1.88 mmol), and water (2 mL) were added thereto, respectively. The reaction was carried out at 120°C under microwave irradiation for 16 hours under argon atmosphere. After the reaction system was cooled and the reaction was completed, the reaction system was added with saturated ammonium chloride solution (5 mL) to quench the reaction, extracted with EtOAc (20 mL × 3), and the organic phases were combined, dried, and concentrated to obtain a crude product. The crude product was purified by normal-phase silica gel column chromatography (EtOAc/PE = 0 to 10%) to obtain the title compound **46-2** (280 mg, yield: 49%) as a brown oil. LC-MS (ESI): m/z 448.0 [M+H]⁺. ¹H NMR (400 MHz, Chloroform-*d*) δ 7.91 (d, *J* = 2.4 Hz, 1H), 7.65 - 7.51 (m, 1H), 7.50 - 7.38 (m, 1H), 7.03 - 6.90 (m, 1H), 6.90 - 6.81 (m, 1H), 6.78 (d, *J* = 8.4 Hz, 1H), 4.73 (q, *J* = 8.6 Hz, 2H), 3.40 (d, *J* = 5.2 Hz, 1H), 3.00 - 2.89 (m, 1H), 2.17 - 2.04 (m, 6H).

### Preparation of compound 46

Compound **46-2** (180 mg, 0.4 mmol) was dissolved in DMF (3 mL), then 1H-tetrazole (141 mg, 2.0 mmol) and potassium carbonate (278 mg, 2.0 mmol) were added thereto, and the reaction mixture was reacted at 80°C for 16 hours in a sealed tube. After the reaction system was cooled, the reaction mixture was filtered, and the crude filtrate was purified by preparative separation (preparation method: chromatographic column: Agilent 10 Prep-C18 250 × 21.2 mm; mobile phase: A: 0.1% formic acid aqueous solution; B: acetonitrile; column temperature: 25°C; gradient: 60% to 80% acetonitrile in 12 min; flow rate: 30 mL/min) to obtain the title compound **46** (110 mg, yield: 24%, containing a pair of enantiomers) and the corresponding regioisomer compound **46-3** (15 mg, yield: 7%, containing a pair of enantiomers).

Compound **46:** LC-MS (ESI): m/z 518.0 [M+H]⁺. ¹H NMR (400 MHz, DMSO-*d6*) δ 9.14 (s, 1H), 7.96 (d, *J* = 2.4 Hz, 1H), 7.70 - 7.48 (m, 2H), 7.35 - 7.24 (m, 1H), 7.21 (s, 1H), 7.10 - 6.96 (m, 1H), 6.89 (d, *J* = 8.4 Hz, 1H), 5.43 (d, *J* = 14.6 Hz, 1H), 5.02 (d, *J* = 14.6 Hz, 1H), 4.94 (q, *J* = 9.1 Hz, 2H), 2.05 (dd, *J* = 9.4, 1.7 Hz, 3H), 1.84 (dd, *J* = 9.4, 1.7 Hz, 3H). ¹⁹F NMR (376 MHz, DMSO-d6) δ -72.35 (s, 3F), -102.53 - -103.49 (m, 1F), -108.89 - -109.97 (m, 3F).

Compound **46-3:** LC-MS (ESI): m/z 518.0 [M+H]⁺. ¹H NMR (400 MHz, DMSO-*d6)* δ 8.76 (s, 1H), 7.96 (d, *J* = 2.4 Hz, 1H), 7.65 - 7.48 (m, 2H), 7.25 (d, *J* = 12.8 Hz, 2H), 6.99 (d, *J* = 2.4 Hz, 1H), 6.88 (d, *J* = 8.4 Hz, 1H), 5.61 (d, *J* = 14.2 Hz, 1H), 5.24 (d, *J* = 14.2 Hz, 1H), 4.94 (q, *J* = 9.1 Hz, 2H), 2.04 (dd, *J* = 9.5, 1.7 Hz, 3H), 1.83 (dd, *J* = 9.5, 1.7 Hz, 3H). ¹⁹F NMR (376 MHz, DMSO-*d6*) δ -72.34 (s, 3F), -102.15 - -103.52 (m, 1F), -108.66 - -109.53 (m, 2F), -109.67 - -110.90 (m, 1F).

### Preparation of compounds 46A and 46B

Compound **46** (40 mg) was subjected to SFC chiral preparative resolution (preparative separation method, instrument model: MG II preparative SFC(SFC-14); model of chromatographic column: ChiralPak AD, 250 × 30 mm I.D., 10 µm; mobile phase: A: CO₂ B: ethanol (0.1% NH₃H₂O); elution gradient: B 20%; flow rate: 60 mL/min; column pressure: 100 bar; column temperature: 38°C; detection wavelength: 220 nm; cycle: about 2.8 min) to obtain the title compounds **46A** (18 mg, single enantiomer) and **46B** (17 mg, single enantiomer).

Compound **46A:** LC-MS (ESI): m/z 518.0 [M+H]⁺. Chiral analysis method (model of chromatographic column: AD-3 150 × 4.6 mm I.D., 3 µm; mobile phase: A: CO₂ B: ethanol (0.05% DEA); elution gradient: B 40%; flow rate: 2.5 mL/min; column temperature: 35°C; column pressure: 100 bar; detection wavelength: 220 nm; Rt = 1.456 min). ¹H NMR (400 MHz, DMSO-*d6*) δ 9.13 (s, 1H), 7.96 (d, *J* = 2.4 Hz, 1H), 7.70 - 7.57 (m, 1H), 7.55 - 7.48 (m. 1H), 7.35 - 7.24 (m, 1H), 7.21 (s, 1H), 7.10 - 6.96 (m, 1H), 6.89 (d, *J* = 8.4 Hz, 1H), 5.43 (d, *J* = 14.6 Hz, 1H), 5.02 (d, *J* = 14.6 Hz, 1H), 4.94 (q, *J* = 9.1 Hz, 2H), 2.05 (dd, *J* = 9.4, 1.7 Hz, 3H), 1.84 (dd, J= 9.4, 1.7 Hz, 3H). ¹⁹F NMR (376 MHz, DMSO-*d6*) δ -72.35 (s, 3F), -102.53 - -103.49 (m, 1F), -108.89 - -109.97 (m, 3F).

Compound **46B:** LC-MS (ESI): m/z 518.0 [M+H]⁺. Chiral analysis method (model of chromatographic column: ChiralCel OD, 150 × 4.6 mm I.D., 3 µm; mobile phase: A: CO₂ B: ethanol (0.05% DEA); elution gradient: B 40%; flow rate: 2.5 mL/min; column temperature: 35°C; column pressure: 100 bar; detection wavelength: 220 nm; Rt = 1.133 min). ¹H NMR (400 MHz, DMSO-d6) δ 9.14 (s, 1H), 7.96 (d, *J* = 2.4 Hz, 1H), 7.70 - 7.57 (m, 1H), 7.55 - 7.48 (m. 1H), 7.35 - 7.24 (m, 1H), 7.21 (s, 1H), 7.10- 6.96 (m, 1H), 6.89 (d, *J* = 8.4 Hz, 1H), 5.43 (d, *J* = 14.6 Hz, 1H), 5.02 (d, *J* = 14.6 Hz, 1H), 4.94 (q, *J* = 9.1 Hz, 2H), 2.05 (dd, *J* = 9.4, 1.7 Hz, 3H), 1.84 (dd, *J* = 9.4, 1.7 Hz, 3H). ¹⁹F NMR (376 MHz, DMSO-*d6*) δ -72.35 (s, 3F), -102.53 - -103.49 (m, 1F), -108.89 - -109.97 (m, 3F).

### Example 47: Preparation of compound 47

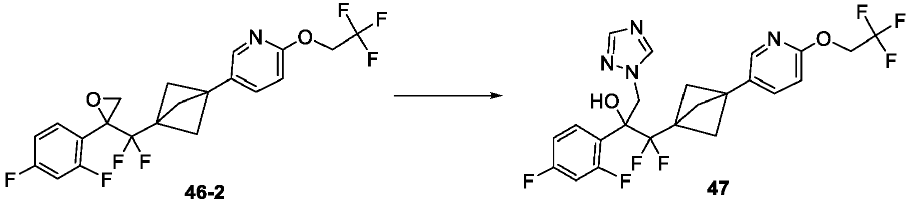

### Preparation of compound 47

Compound 1,2,4-triazole (77 mg, 1.12 mmol) was dissolved in DMF solution (2 mL) and cooled to 0°C. Sodium hydride (60%, 27 mg, 1.12 mmol) was added thereto and reacted for half an hour. Compound **46-2** (100 mg, 0.22 mmol) was added thereto,. The reaction system was stirred at 80°C for 2 hours. After the reaction system was cooled, the reaction mixture was filtered, and the crude filtrate was purified by preparative separation (preparation method: mobile phase: A: 0.1% formic acid aqueous solution; B: acetonitrile; chromatographic column: Agilent 10 Prep-C18 250 × 21.2 mm; column temperature: 25°C; gradient: 70% to 90% acetonitrile in 12 min; flow rate: 30 mL/min) to obtain the title compound **47** (25 mg, yield: 21%, containing a pair of enantiomers).

Compound **47:** LC-MS (ESI): m/z 517.0 [M+H]⁺. ¹H NMR (400 MHz, DMSO-d6) δ 8.38 (br.s, 1H), 7.96 (d, *J* = 2.4 Hz, 1H), 7.71 (br.s, 1H), 7.62 - 7.51 (m, 2H), 7.25 - 7.13 (m, 1H), 7.03 - 6.94 (m, 1H), 6.94 - 6.84 (m, 2H), 5.14 (d, *J* = 14.5 Hz, 1H), 4.94 (q, *J* = 9.2 Hz, 2H), 4.76 (d, *J* = 14.5 Hz, 1H), 2.04 (dd, *J* = 9.4, 1.7 Hz, 3H), 1.82 (dd, *J =* 9.4, 1.7 Hz, 3H). ¹⁹F NMR (376 MHz, DMSO-*d6*) δ -72.34 (m, 3F), -101.77 - -103.67 (m, 1F), -109.02 - - 109.95 (m, 2F), -109.86 - -111.01 (m, 1F).

### Example 48: Preparation of compounds 48, 48A, and 48B

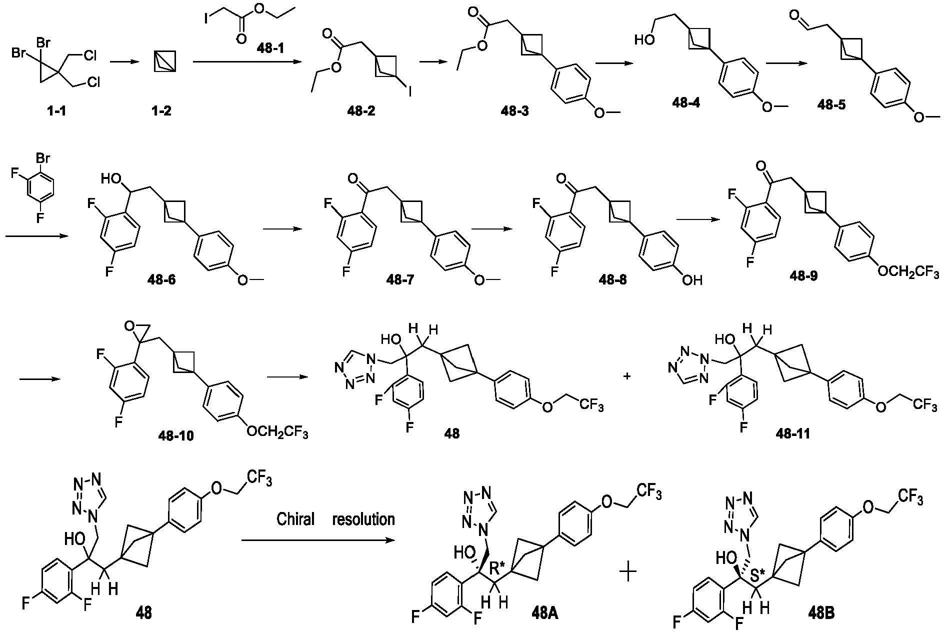

### Preparation of compound 48-2

To a three-necked reaction flask was added compound **1-1** (200 g, 0.68 mol), and the system was replaced with nitrogen three times. Then anhydrous ether (500 mL) was added thereto, and methyllithium (1.6 M, 850 mL, 1.36 mol) was added dropwise to the reaction system at -78°C. After the reaction was carried out for 5 min, the reaction system was transferred to 0°C, stirred, and reacted for 3 hours. Then ethyl iodoacetate (73 g, 0.34 mol) was added thereto, and the reaction mixture was reacted at room temperature for 16 hours. The reaction mixture was extracted with EtOAc (20 mL × 3), and the organic phases were combined, dried, and concentrated to obtain a crude product. The crude product was purified by normal-phase column chromatography (EtOAc/PE = 0 to 5%) to obtain the title compound **48-2** (32 g, yield: 16.8%) as a light yellow liquid. ¹H NMR (400 MHz, DMSO-*d6*) δ 4.05 (q, *J* = 7.2 Hz, 2H), 2.58 (s, 2H), 2.28 (s, 6H), 1.18 (t, *J* = 7.2 Hz, 3H).

### Preparation of compound 48-3

To a three-necked flask was added ferric acetylacetonate (7.7 g, 0.02 mol), and then THF (300 mL), compound **48-2** (32 g, 0.11 mol), and TMEDA (5.2 g, 0.05 mol) were added thereto respectively under nitrogen atmosphere. After the reaction mixture was stirred for 5 min, a solution **1-5** of Grignard reagent 4-methoxyphenylmagnesium bromide in THF (0.5 M, 176 mL, 0.18 mol) was slowly added dropwise to the reaction system, and reacted at room temperature for 16 hours. The reaction mixture was extracted with EtOAc (20 mL × 3), and the organic phases were combined, dried, and concentrated to obtain a crude product. The crude product was purified by normal-phase column chromatography (EtOAc/PE = 0 to 10%) to obtain the title compound **48-3** (17 g, yield: 57%) as a light yellow liquid. ¹H NMR (400 MHz, DMSO-*d6*) δ 7.13 - 7.07 (dd, *J* = 6.6, 2.2 Hz, 2H), 6.87 - 6.82 (dd, *J* = 6.6, 2.2 Hz, 2H), 4.08 (q, *J* = 7.2 Hz, 2H), 3.71 (s, 3H), 2.55 (s, 2H), 1.93 (s, 6H), 1.20 (t, *J* = 7.2 Hz, 3H).

### Preparation of compound 48-4

To a single-necked reaction flask were sequentially compound **48-3** (17.0 g, 65.4 mmol) and solvent anhydrous tetrahydrofuran (150 mL), then the reaction system was added with lithium aluminum hydride (3.2 g, 85.1 mmol) in batches at 0°C, and reacted at room temperature for 2 hours. The reaction mixture was quenched with ice water (5.0 mL) and extracted with EtOAc (20 mL × 3), and the organic phases were combined, dried, and concentrated to obtain a crude product. The crude product was purified by normal-phase column chromatography (EtOAc/PE = 0 to 10%) to obtain the title compound **48-4** (7.1 g, yield: 50%) as a colorless oil. LC-MS (ESI): m/z 219.2 [M+H]⁺.

### Preparation of compound 48-5

To a three-necked reaction flask was added compound **48-4** (7.1 g, 32.5 mmol), and the system was replaced with nitrogen three times. Then dichloromethane (100 mL) was added thereto, and Dess-Martin periodinane (15.2 g, 78.5 mmol) was slowly added thereto at 0°C. The reaction system was stirred at room temperature for 2 hours. The reaction mixture was extracted with dichloromethane (20 mL × 3), and the organic phases were combined, dried, and concentrated to obtain a crude product. The crude product was purified by normal-phase column chromatography (EtOAc/PE = 0 to 10%) to obtain the title compound **48-5** (4.5 g, yield: 64%) as a light yellow liquid. LC-MS (ESI): m/z 217.2 [M+H]+.

### Preparation of compound 48-6

To a three-necked flask was added 1-bromo-2,4-difluorobenzene (12.1 g, 62.4 mmol), and the system was replaced with nitrogen three times. To the three-necked flask was added anhydrous ether (150 mL), and n-butyllithium (1.6 M, 39 mL, 62.4 mmol) was added dropwise thereto at a cooling temperature of -78°C. After the reaction system was stirred and reacted at -78°C for 2 hours, a solution of compound **48-5** (4.5 g, 20.8 mmol) dissolved in anhydrous ether (10 mL) was added dropwise to the reaction system, and the reaction mixture was stirred and reacted for another 1 hour. After the reaction was completed, the reaction system was quenched with saturated ammonium chloride solution (20 mL), extracted with EtOAc (20 mL × 3), and the organic phases were combined, dried, and concentrated to obtain a crude product. The crude product was purified by normal-phase silica gel column chromatography (EtOAc/PE = 0 to 15%) to obtain the title compound **48-6** (3.5 g, yield: 50%) as a yellow solid. LC-MS (ESI): m/z 313.2 [M-H₂O+H]⁺.

### Preparation of compound 48-7

To a three-necked flask was added compound **48-6** (3.5 g, 10.6 mmol), and the system was replaced with nitrogen three times. Then dichloromethane (50 mL) and Dess-Martin periodinane (9.1 g, 21.2 mmol) were respectively added thereto at 0°C. The reaction system was stirred at room temperature for 2 hours. The reaction mixture was extracted with dichloromethane (20 mL × 3), and the organic phases were combined, dried, and concentrated to obtain a crude product. The crude product was purified by normal-phase column chromatography (EtOAc/PE = 0 to 10%) to obtain the title compound **48-7** (3.2 g, yield: 91%) as a light yellow liquid. LC-MS (ESI): m/z 329.2 [M+H]⁺.

### Preparation of compound 48-8

To a sealed reaction tube were sequentially added compound **48-7** (3.2 g, 9.7 mmol), acetic acid (15 mL), and hydrobromic acid aqueous solution (15 mL), and the reaction system was reacted at 100°C for 60 hours. The reaction mixture was extracted with dichloromethane (20 mL × 3), and the organic phases were combined, dried, and concentrated to obtain a crude product. The crude product was purified by normal-phase column chromatography (EtOAc/PE = 0 to 10%) to obtain the title compound **48-8** (2.4 g, yield: 78%) as a light yellow liquid. LC-MS (ESI): m/z 313.0 [M-H]⁻.

### Preparation of compound 48-9

Compound **48-8** (2.4 g, 7.1 mmol) was dissolved in DCM (5 mL) solvent, then potassium carbonate (3.5 g, 10.8 mmol) and 2,2,2-trifluoroethyl trifluoromethanesulfonate (2.5 g, 10.8 mmol) were added thereto. The reaction system was reacted at 65°C for 16 hours in a sealed test tube. After the reaction system was cooled, the reaction mixture was extracted with EtOAc (20 mL × 3), and the organic phases were combined, dried, and concentrated to obtain a crude product. The crude product was purified by normal-phase silica gel column chromatography (EtOAc/PE = 0 to 15%) to obtain the title compound **48-9** (1.2 g, yield: 40%) as a light yellow oil. ¹H NMR (400 MHz, DMSO-d6) δ 7.99 - 7.84 (m, 1H), 7.53 - 7.36 (m, 1H), 7.34 - 7.19 (m, 1H), 7.19 - 7.06 (dd, *J* = 6.6, 2.2 Hz, 2H), 7.04 - 6.87 (dd, *J* = 6.6, 2.2 Hz, 2H), 4.71 (q, *J* = 8.8 Hz, 2H), 3.21 (d, *J* = 2.4 Hz, 2H), 1.93 (s, 6H).

### Preparation of compound 48-10

To a three-necked flask were respectively added compound trimethylsulfoxonium iodide (1.98 g, 9.0 mmol), NaH (216 mg, 9.0 mmol), and DMSO (10 mL). The reaction system was stirred and reacted at room temperature for 1 hour, then a solution of compound **48-9** (1.2 g, 3.0 mmol) in DMSO (3 mL) was added dropwise to the reaction system, and the reaction mixture was reacted at 50°C for another 2 hours. After cooling to room temperature, the reaction system was extracted with DCM (20 mL × 3), and the organic phases were combined, dried, and concentrated to obtain a crude product. The crude product was purified by normal-phase silica gel column chromatography (EtOAc/PE = 0 to 15%) to obtain the title compound **48-9** (456 mg, yield: 36%) as a light yellow oil. ¹H NMR (400 MHz, DMSO-d6) δ 7.55 - 7.46 (m, 1H), 7.32 - 7.23 (m, 1H), 7.13 - 7.06 (m, 1H), 7.07 - 7.02 (m, 2H), 6.98 - 6.86 (m, 2H), 4.76 - 4.62 (q, *J* = 8 Hz, 2H), 2.96 (d, *J* = 5.1 Hz, 1H), 2.81 (d, *J* = 5.1 Hz, 1H), 2.29 - 2.20 (m, 1H), 1.99 (d, *J* = 14.7 Hz, 1H), 1.78 - 1.64 (m, 6H).

### Preparation of compound 48

Compound **48-10** (70 mg, 0.17 mmol) was dissolved in DMF (2.5 mL), and then 1H-tetrazole (60 mg, 0.85 mmol) and potassium carbonate (277 mg, 0.85 mmol) were added thereto respectively. The reaction system was reacted at 80°C for 16 hours in a sealed tube. After the reaction system was cooled, the reaction mixture was filtered, and the crude filtrate was purified by preparative separation (preparation method: chromatographic column: Agilent 10 Prep-C18 250 × 21.2 mm; mobile phase: A: 0.1% formic acid aqueous solution; B: acetonitrile; column temperature: 25°C; gradient: 60% to 80% acetonitrile in 12 min; flow rate: 30 mL/min) to obtain the title compound **48** (29.5 mg, yield: 36%, containing a pair of enantiomers) and the corresponding regioisomer compound **48-11** (11, 7 mg, yield: 14%, containing a pair of enantiomers).

Compound **48:** LC-MS (ESI): m/z 481.2 [M+H]⁺. ¹H NMR (400 MHz, DMSO-d6) δ 9.09 (s, 1H), 7.43 - 7.32 (m, 1H), 7.28 - 7.19 (m, 1H), 7.03 - 6.94 (m, 3H), 6.93 - 6.86 (m, 2H), 5.86 (s, 1H), 4.79 (d, *J* = 14.2 Hz, 1H), 4.72 - 4.63 (m, 3H), 2.46 - 2.38 (m, 1H), 1.96 (d, *J* = 14.8 Hz, 1H), 1.71 - 1.61 (m, 3H), 1.56 - 1.45 (m, 3H). ¹⁹F NMR (376 MHz, DMSO-*d6*) δ -72.59 (s, 3F), -108.56 (m, 2F), -111.88 (m, 2F).

Compound **48-11:** LC-MS (ESI): m/z 481.2 [M+H]⁺. ¹H NMR (400 MHz, DMSO-*d6)* δ 8.79 (s, 1H), 7.44 - 7.37 (m, 1H), 7.25 - 7.18 (m, 1H), 7.00 - 6.95 (m, 3H), 6.91 - 6.86 (m, 2H), 5.83 (s, 1H), 5.02 - 4.87 (m, 2H), 4.72 - 4.63 (m, 2H), 2.47 - 2.41 (m, 1H), 2.04 (d, J= 14.8 Hz, 1H), 1.68 - 1.62 (m, 3H), 1.51-1.45 (m, 3H). ¹⁹F NMR (376 MHz, DMSO-*d6*) δ -72.60 (s, 3F), -108.82 (d, 2F), -112.19 (d, 2F).

### Preparation of compounds 48A and 48B

Compound **48** (26 mg) was subjected to SFC chiral preparative resolution (preparative separation method, instrument model: Thar 80 preparative SFC (SFC-17); model of chromatographic column: ChiralCel OJ, 250 × 30 mm I.D., 10 µm; mobile phase: A: CO₂ B: ethanol (0.1% NH₃H₂O); elution gradient: B 20%; flow rate: 80 mL/min; column pressure: 100 bar; column temperature: 38°C; detection wavelength: 254 nm; cycle: about 7 min) to obtain the title compounds **48A** (7.9 mg) and **48B** (7.9 mg).

Compound **48A:** Chiral analysis method (chromatographic column: Chiralcel OJ-3 150 × 4.6 mm I.D., 3 µm mobile phase: A: CO₂ B: ethanol (0.05% DEA), elution gradient: B 25%; flow rate: 2.5 mL/min, chromatographic column temperature: 35°C, column pressure: 1500 psi; Rt = 1.435 min). LC-MS (ESI): m/z 481.2 [M+H]⁺. ¹H NMR (400 MHz, Chloroform-*d*) δ 8.56 (s, 1H), 7.43 - 7.32 (m, 1H), 6.96 - 6.88 (m, 2H), 6.84 - 6.69 (m, 4H), 4.69 (d, *J* = 3.2 Hz, 2H), 4.22 (q, *J* = 8.0 Hz, 2H), 2.76 (s, 1H), 2.58 (dd, *J* = 15.2, 2.2 Hz, 1H), 1.86 (d, *J* = 12 Hz, 1H), 1.71 (d, *J* = 9.6, 1.7 Hz, 3H), 1.57 (d, *J* = 9.6, 1.7 Hz, 3H). ¹⁹F NMR (376 MHz, DMSO-d6) δ -74.00 (s, 3F), -108.80 - -108.82 (d, 1F), -109.28 - -109.30 (d, 1F).

Compound **48B:** Chiral analysis method (chromatographic column: Chiralcel OJ-3 150 × 4.6 mm I.D., 3 µm mobile phase: A: CO₂ B: ethanol (0.05% DEA), elution gradient: B 25%; flow rate: 2.5 mL/min, chromatographic column temperature: 35°C, column pressure: 1500 psi; Rt = 1.663 min). LC-MS (ESI): m/z 481.2 [M+H]⁺. ¹H NMR (400 MHz, Chloroform-*d*) δ 8.55 (s, 1H), 7.43 - 7.32 (m, 1H), 6.96 - 6.89 (m, 2H), 6.84 - 6.69 (m, 4H), 4.69 (d, *J* = 2.8 Hz, 2H), 4.22 (q, *J* = 8.1 Hz, 2H), 2.74 (br.s, 1H), 2.58 (dd, *J* = 15.2, 2.3 Hz, 1H), 1.86 (d, *J* = 12 Hz, 1H), 1.71 (d, *J* = 9.6, 1.7 Hz, 3H), 1.58 (d, *J* = 1.6 Hz, 3H). ¹⁹F NMR (376 MHz, DMSO-d6) δ -74.00 (s, 3F), -108.80 - -108.82 (d, 1F), -109.28 - -109.30 (d, 1F).

### Example 49: Preparation of compounds 49, 49A, and 49B

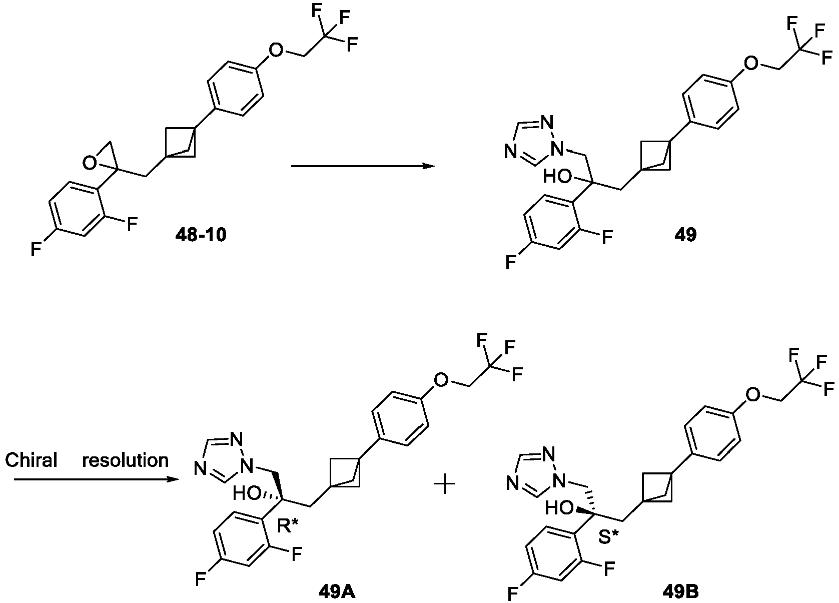

### Preparation of compound 49

1,2,4-Triazole (38 mg, 0.55 mmol) was dissolved in DMF (2 mL), and NaH (60%, 22 mg, 0.55 mmol) was added thereto at 0°C. After the reaction mixture was reacted for 30 min, the reaction system was added with compound **48-10** (45 mg, 0.11 mmol) and reacted at 70°C for 16 hours in a sealed tube. After the reaction system was cooled, the reaction mixture was filtered, and the crude filtrate was purified by preparative separation (chromatographic column: Agilent 10 Prep-C18 250 × 21.2 mm; column temperature: 25°C; mobile phase: water (0.1% FA)-acetonitrile; proportion of acetonitrile in the mobile phase: 50% to 70% in 12 min; flow rate: 30 mL/min) to obtain the title compound 49 (9.5 mg, yield: 21%, containing a pair of enantiomers).

Compound **49:** LC-MS (ESI): m/z 480.2 [M+H]⁺. ¹H NMR (400 MHz, DMSO-d6) δ 8.28 (s, 1H), 7.78 (s, 1H), 7.49 - 7.41 (m, 1H), 7.23 - 7.14 (m, 1H), 7.03 - 6.95 (m, 3H), 6.91 - 6.86 (m, 2H), 5.65 (s, 1H), 4.67 (q, *J* = 8 Hz, 2H), 4.45 (d, *J* = 3.4 Hz, 2H), 2.38 (m, 1H), 1.89 (d, *J* = 14.8 Hz, 1H), 1.67 - 1.62 (m, 3H), 1.51 - 1.45 (m, 3H). ¹⁹F NMR (376 MHz, DMSO-d6) δ -72.60 (s, 3F), -108.50 (d, *J* = 7.5 Hz, 2F), -112.48 (d, *J =* 7.5 Hz, 2F).

### Preparation of compounds 49A and 49B

Compound **49** (90 mg) was subjected to SFC chiral preparative resolution (preparative separation method, instrument model: MG II preparative SFC (SFC-14); model of chromatographic column: ChiralPak IC, 250 × 30 mm I.D. 10 µm; mobile phase: A: CO₂ B: ethanol; elution gradient: B 15%; flow rate: 60 mL/min; column pressure: 100 bar; column temperature: 38°C; detection wavelength: 220 nm; cycle: about 16 min) to obtain the title compounds **49A** (48 mg) and **49B** (22 mg).

Compound **49A:** LC-MS (ESI): m/z 480.6 [M+H]⁺. ¹H NMR (400 MHz, DMSO-*d6*) δ 8.29 (s, 1H), 7.78 (s, 1H), 7.59 - 7.39 (m, 1H), 7.30 - 7.11 (m, 1H), 7.11 - 6.78 (m, 5H), 5.66 (s, 1H), 4.67 (q, *J* = 8.9 Hz, 2H), 4.55 - 4.35 (m, 2H), 2.43 - 2.30 (m, 1H), 1.94 - 1.79 (m, 1H), 1.65 (d, *J* = 9.6 Hz, 3H), 1.48 (d, *J* = 9.6 Hz, 3H). ¹⁹F NMR (376 MHz, DMSO-d6) δ - 72.59 (s, 3F), -108.50 (d, 1F), -112.47 (d, 1F).

Compound **49B:** LC-MS (ESI): m/z 480.6 [M+H]⁺. ¹H NMR (400 MHz, DMSO-d6) δ 8.29 (s, 1H), 7.78 (s, 1H), 7.59 - 7.39 (m, 1H), 7.30 - 7.11 (m, 1H), 7.11 - 6.78 (m, 5H), 5.66 (s, 1H), 4.67 (q, *J* = 8.9 Hz, 2H), 4.55 - 4.35 (m, 2H), 2.43 - 2.30 (m, 1H), 1.94 - 1.79 (m, 1H), 1.65 (d, *J* = 9.6 Hz, 3H), 1.48 (d, *J* = 9.6 Hz, 3H). ¹⁹F NMR (376 MHz, DMSO-d6) δ -72.59 (s, 3F), -108.50 (d, 1F), -112.47 (d, 1F).

### Example 50: Preparation of compounds 50, 50A, and 50B

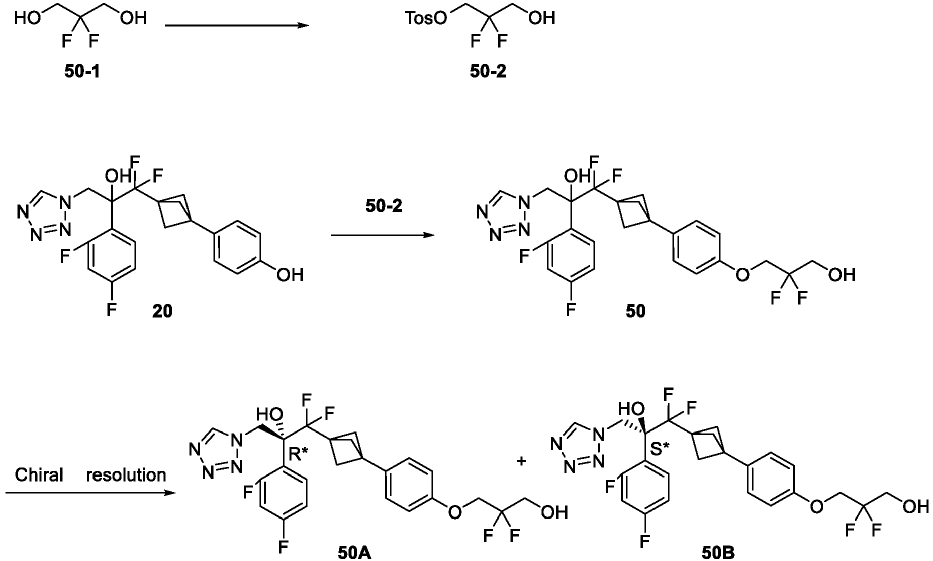

### Preparation of compound 50-2

Compound **50-1** (400 mg, 3.57 mmol) was dissolved in dichloromethane (10 mL), then triethylamine (433 mg, 4.28 mmol) was added dropwise thereto at 0°C. After the reaction was carried out for 10 min, p-toluenesulfonyl chloride (816 mg, 4.28 mmol) was added thereto, reacted at room temperature for 24 hours. After the reaction was tracked by TLC until the reaction was completed, the reaction system was extracted with DCM (20 mL × 3), and the organic phases were combined, dried, and concentrated to obtain 1 g of a crude product, which was filtered through silica gel to obtain the title compound **50-2** (300 mg, yield: 31%). ¹H NMR (400 MHz, Chloroform-*d*) δ 7.84 - 7.79 (m, 2H), 7.41 - 7.36 (m, 2H), 4.27 (t, *J* = 12.4 Hz, 2H), 3.85 (t, *J* = 12.4 Hz, 2H), 2.47 (s, 3H), 2.08 (br, 1H).

### Preparation of compound 50

Compound **20** (80 mg, 0.184 mmol) was dissolved in DMF (2 mL), then cesium carbonate (120 mg, 0.386 mmol) was added thereto with stirring. After the reaction was carried out for 5 min, the reaction mixture was then added with compound **50-2** (73 mg, 0.276 mmol), stirred and reacted at 50°C for 24 hours. The reaction mixture was filtered and the filtrate was concentrated to obtain a crude product. The crude product was purified by preparative separation (preparation method: chromatographic column: Agilent 10 Prep-C18 250 × 21.2 mm; column temperature: 25°C; mobile phase: water (0.1% FA)-acetonitrile; proportion of acetonitrile in the mobile phase: 45% to 65% in 12 min; flow rate: 30 mL/min) to obtain the title compound **50** (6 mg, yield: 6%, containing a pair of enantiomers).

Compound **50:** LC-MS (ESI): m/z 529.2 [M+H]⁺. ¹H NMR (400 MHz, DMSO-*d*6) δ 9.13 (s, 1H), 7.57 - 7.51 (m, 1H), 7.31 - 7.25 (m, 1H), 7.16 (s, 1H), 7.09 - 6.97 (m, 3H), 6.93 - 6.87 (m, 2H), 5.65 (t, *J* = 6.2 Hz, 1H), 5.43 (d, *J* = 14.5 Hz, 1H), 5.01 (d, *J* = 14.6 Hz, 1H), 4.25 (t, *J* = 13.8 Hz, 2H), 3.72 (td, *J* = 13.8, 6.2 Hz, 2H), 2.00 - 1.97 (m, 3H), 1.78 - 1.76 (m, 3H). ¹⁹F NMR (376 MHz, DMSO-d6) δ -102.95--103.17 (m, 1F), -109.21- -109.32 (m, 2F), -109.62 (d, *J* = 9.6 Hz, 1F), -114.06 (s, 1F).

### Preparation of compounds 50A and 50B

Compound **50** (160 mg) was subjected to SFC chiral preparative resolution (preparative separation method, instrument model: MG II preparative SFC (SFC-1); model of chromatographic column: Cellulose-2, 250 × 30 mm I.D., 10 µm; mobile phase: A: CO₂ B: ethanol; elution gradient: B 40%; flow rate: 70 mL/min; column pressure: 100 bar; column temperature: 35°C; detection wavelength: 220 nm; cycle: about 8 min) to obtain the title compounds **50A** (37 mg) and **50B** (68 mg).

Compound **50A:** Chiral analysis method (model of chromatographic column: Cellulose-2 150 × 4.6 mm I.D., 3 µm; mobile phase: A: CO₂ B: methanol (0.05% DEA); elution gradient: the mobile phase increased from 5% B to 40% B within 5 min and maintained at 40% B for 2.5 min for elution, then 5% B was equilibrated for 2.5 min; flow rate: 2.5 mL/min; column temperature: 35°C; column pressure: 1500 psi; detection wavelength: 220 nm; RT = 1.817 min). LC-MS (ESI): m/z 529.2 [M+H]⁺. ¹H NMR (400 MHz, DMSO-d6) δ 9.13 (s, 1H), 7.57-7.51 (m, 1H), 7.31-7.25 (m, 1H), 7.14 (s, 1H), 7.07 - 6.98 (m, 3H), 6.92 - 6.88 (m, 2H), 5.63 (t, *J* = 6.2 Hz, 1H), 5.43 (d, *J* = 14.6 Hz, 1H), 5.01 (d, *J* = 14.6 Hz, 1H), 4.25 (t, *J =* 13.8 Hz, 2H), 3.72 (td, *J* = 13.8, 6.2 Hz, 2H), 2.0-1.97 (m, 3H), 1.78-1.76 (m, 3H). ¹⁹F NMR (376 MHz, Chloroform-*d*) δ -102.95- -103.16 (m, 1F), -109.20 - -109.30 (m, 2F), -109.63 (d, *J* = 9.7 Hz, 1F), -114.05 (s, 2F).

Compound **50B:** Chiral analysis method (model of chromatographic column: Cellulose-2 150 × 4.6 mm I.D., 3 µm; mobile phase: A: CO₂ B: methanol (0.05% DEA); elution gradient: the mobile phase increased from 5% B to 40% B within 5 min and maintained at 40% B for 2.5 min for elution, then 5% B was equilibrated for 2.5 min; flow rate: 2.5 mL/min; column temperature: 35°C; column pressure: 1500 psi; detection wavelength: 220 nm; RT = 1.342 min). LC-MS (ESI): m/z 529.2 [M+H]⁺. ¹H NMR (400 MHz, DMSO-*d*6) δ 9.13 (s, 1H), 7.55-7.51 (m, 1H), 7.31-7.25 (m, 1H), 7.14 (s, 1H), 7.09 - 6.97 (m, 3H), 6.93 - 6.87 (m, 2H), 5.63 (t, *J* = 6.2 Hz, 1H), 5.43 (d, *J* = 14.6 Hz, 1H), 5.01 (d, *J* = 14.6 Hz, 1H), 4.25 (t, *J* = 13.8 Hz, 2H), 3.72 (td, J= 13.8, 6.2 Hz, 2H), 2.00-1.97 (m, 3H), 1.78-1.76 (m, 3H). ¹⁹F NMR (376 MHz, Chloroform-*d*) δ -102.95- -103.16 (m, 1F), -109.20 - -109.30 (m, 2F), -109.63 (d, *J* = 9.7 Hz, 1F), -114.05 (s, 2F).

### Example 51: Preparation of compounds 51, 51A, 51B, 51C, and 51D

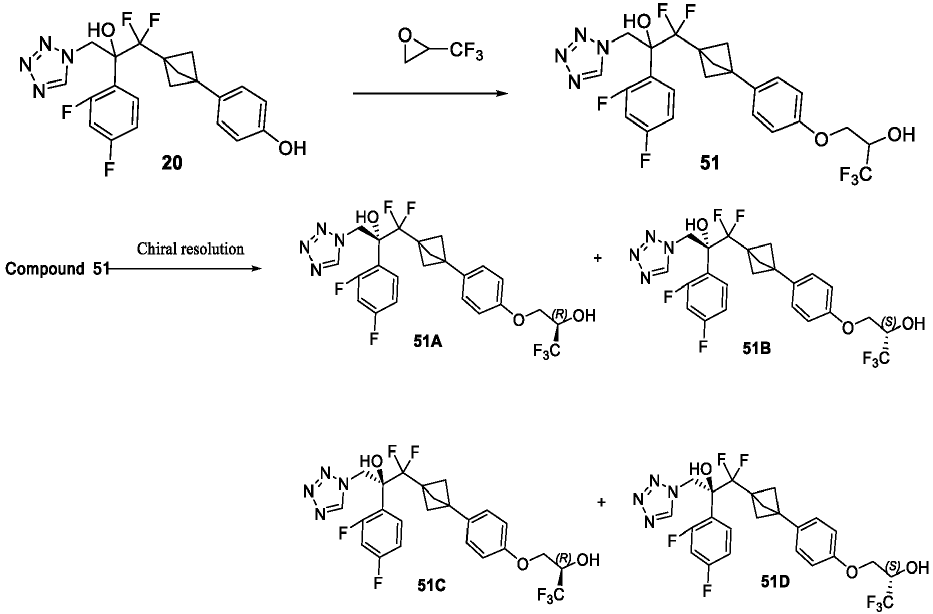

### Preparation of compound 51

Compound **20** (80 mg, 0.18 mmol) was dissolved in acetonitrile (5 mL), then potassium carbonate (51 mg, 0.37 mmol) was added thereto with stirring. After the reaction was carried out for 5 min, the reaction mixture was then added with compound 1,1,1-trifluoro-2,3-epoxypropane (31 mg, 0.28 mmol), stirred and reacted at 50°C for 16 hours. The reaction mixture was filtered, and the crude filtrate was purified by preparative separation (preparation method: chromatographic column: Agilent 10 Prep-C18 250 × 21.2 mm; column temperature: 25°C; mobile phase: water (0.1% TFA)-acetonitrile; proportion of acetonitrile in the mobile phase: 50% to 70% in 12 min; flow rate: 30 mL/min) to obtain the title compound **51** (62 mg, yield: 62%, containing two pairs of enantiomers).

Compound **51:** LC-MS (ESI): m/z 547.2 [M+H]⁺. ¹H NMR (400 MHz, DMSO-d6) δ 9.13 (s, 1H), 7.54 (td, *J* = 9.0, 6.7 Hz, 1H), 7.28 (ddd, *J* = 12.0, 9.0, 2.6 Hz, 1H), 7.15 (s, 1H), 7.09 - 6.96 (m, 3H), 6.92 - 6.82 (m, 2H), 6.63 (d, *J* = 6.6 Hz, 1H), 5.42 (d, *J* = 14.5 Hz, 1H), 5.01 (d, *J* = 14.5 Hz, 1H), 4.35 (dt, *J* = 11.3, 6.8 Hz, 1H), 4.12 (m, 1H), 4.01 (m, 1H), 1.98 (dd, *J* = 9.4, 1.7 Hz, 3H), 1.76 (dd, *J* = 9.4, 1.7 Hz, 3H). ¹⁹F NMR (376 MHz, DMSO-d6) δ -76.06 (s, 3F), -102.96 - -103.17 (m, 1F), -109.22 - -109.32 (m, 2F), -109.63 (d, *J* = 9.2 Hz, 1F).

### Preparation of compounds 51A, 51B, 51C, and 51D

Compound **51** (62 mg) was subjected to SFC chiral preparative resolution (preparative separation method, instrument model: MG II preparative SFC (SFC-14); model of chromatographic column: ChiralPak AD, 250 × 30 mm I.D., 5 µm; mobile phase: A: CO₂ B: isopropanol; elution gradient: B 20%; flow rate: 70 mL/min; column pressure: 100 bar; column temperature: 38°C; detection wavelength: 220 nm; cycle: about 4 min) to obtain the title compounds **51A** (11 mg), **51B** (10 mg), **51C** (12 mg), and **51D** (9 mg).

Compound **51A:** LC-MS (ESI): 547.2 [M+H]⁺. Chiral analysis method (model of chromatographic column: ChiralPak AD-3, 150 × 4.6 mm I.D., 3 µm; mobile phase: A: CO₂ B: isopropanol (0.05% DEA); elution gradient: the mobile phase increased from 5% B to 40% B within 5 min and maintained at 40% B for 2.5 min for elution, then 5% B was equilibrated for 2.5 min; flow rate: 2.5 mL/min; column temperature: 35°C; column pressure: 100 bar; detection wavelength: 220 nm; RT = 4.257 min). ¹H NMR (400 MHz, DMSO-d6) δ 9.13 (s, 1H), 7.54 (td, *J* = 9.0, 6.6 Hz, 1H), 7.36 - 7.19 (m, 1H), 7.15 (s, 1H), 7.06 - 7.03 (m, 2H), 7.03 - 6.98 (m, 1H), 6.93 - 6.79 (m, 2H), 6.63 (d, *J* = 6.6 Hz, 1H), 5.42 (d, *J* = 14.6 Hz, 1H), 5.01 (d, *J =* 14.6 Hz, 1H), 4.44 - 4.25 (m, 1H), 4.11 (dd, *J* = 10.6, 4.2 Hz, 1H), 4.01 (dd, *J* = 10.6, 6.4 Hz, 1H), 1.98 (dd, *J* = 9.4, 1.6 Hz, 3H), 1.76 (dd, *J* = 9.4, 1.6 Hz, 3H). ¹⁹F NMR (376 MHz, DMSO-d6) δ -76.06 (s, 3F), -102.96 - -103.17 (m, 1F), -109.22 - -109.32 (m, 2F), -109.64 (d, *J* = 9.6 Hz, 1F).

Compound **51B:** LC-MS (ESI): 547.2 [M+H]⁺. Chiral analysis method (model of chromatographic column: ChiralPak AD-3, 150 × 4.6 mm I.D., 3 µm; mobile phase: A: CO₂ B: isopropanol (0.05% DEA); elution gradient: the mobile phase increased from 5% B to 40% B within 5 min and maintained at 40% B for 2.5 min for elution, then 5% B was equilibrated for 2.5 min; flow rate: 2.5 mL/min; column temperature: 35°C; column pressure: 100 bar; detection wavelength: 220 nm; RT = 3.764 min). ¹H NMR (400 MHz, DMSO-*d6*) δ 9.13 (s, 1H), 7.60 - 7.48 (m, 1H), 7.33 - 7.22 (m, 1H), 7.14 (s, 1H), 7.06 - 7.03 (m, 2H), 7.01 - 6.98 (m, 1H), 6.91 - 6.81 (m, 2H), 6.62 (d, *J* = 6.6 Hz, 1H), 5.42 (d, *J* = 14.6 Hz, 1H), 5.01 (d, *J* = 14.6 Hz, 1H), 4.42 - 4.25 (m, 1H), 4.13 - 4.09 (m, 1H), 4.03 - 3.99 (m, 1H), 1.98 (dd, *J* = 9.4, 1.6 Hz, 3H), 1.77 (dd, *J* = 9.4, 1.6 Hz, 3H). ¹⁹F NMR (376 MHz, DMSO-d6) δ -76.06 (s, 3F), -102.95 - -103.16 (m, 1F), -109.19 - -109.29 (m, 2F), -109.64 (d, *J* = 9.6 Hz, 1F).

Compound **51C:** LC-MS (ESI): 547.2 [M+H]⁺. Chiral analysis method (model of chromatographic column: ChiralPak AD-3, 150 × 4.6 mm I.D., 3 µm; mobile phase: A: CO₂ B: isopropanol (0.05% DEA); elution gradient: the mobile phase increased from 5% B to 40% B within 5 min and maintained at 40% B for 2.5 min for elution, then 5% B was equilibrated for 2.5 min; flow rate: 2.5 mL/min; column temperature: 35°C; column pressure: 100 bar; detection wavelength: 220 nm; RT = 4.090 min). ¹H NMR (400 MHz, DMSO-*d6*) δ 9.13 (s, 1H), 7.59 - 7.49 (m, 1H), 7.33 - 7.22 (m, 1H), 7.14 (s, 1H), 7.06 - 7.03 (m, 2H), 7.02 - 6.98 (m, 1H), 6.90 - 6.82 (m, 2H), 6.62 (d, *J* = 6.4 Hz, 1H), 5.42 (d, *J* = 14.6 Hz, 1H), 5.01 (d, *J* = 14.6 Hz, 1H), 4.42 - 4.25 (m, 1H), 4.33 - 4.09 (m, 1H), 4.03 - 3.99 (m, 1H), 1.98 (dd, *J* = 9.5, 1.6 Hz, 3H), 1.77 (dd, *J* = 9.5, 1.6 Hz, 3H). ¹⁹F NMR (376 MHz, DMSO-d6) δ -76.06 (s, 3F), -102.95 - -103.16 (m, 1F), -109.19 - -109.29 (m, 2F), -109.64 (d, *J* = 9.6 Hz, 1F).

Compound **51D:** LC-MS (ESI): 547.2 [M+H]⁺. Chiral analysis method (model of chromatographic column: ChiralPak AD-3, 150 × 4.6 mm I.D., 3 µm; mobile phase: A: CO₂ B: isopropanol (0.05% DEA); elution gradient: the mobile phase increased from 5% B to 40% B within 5 min and maintained at 40% B for 2.5 min for elution, then 5% B was equilibrated for 2.5 min; flow rate: 2.5 mL/min; column temperature: 35°C; column pressure: 100 bar; detection wavelength: 220 nm; RT = 3.761 min). ¹H NMR (400 MHz, DMSO-d6) δ 9.13 (s, 1H), 7.59 - 7.48 (m, 1H), 7.33 - 7.23 (m, 1H), 7.13 (s, 1H), 7.08 - 7.03 (m, 2H), 7.01 - 6.96 (m, 1H), 6.91 - 6.82 (m, 2H), 6.61 (d, *J* = 6.6 Hz, 1H), 5.42 (d, *J* = 14.6 Hz, 1H), 5.01 (d, *J* = 14.6 Hz, 1H), 4.41 - 4.27 (m, 1H), 4.13 - 4.09 (m, 1H), 4.03 - 3.99 (m, 1H), 1.98 (dd, *J* = 9.4, 1.6 Hz, 3H), 1.77 (dd, *J* = 9.4, 1.6 Hz, 3H). ¹⁹F NMR (376 MHz, DMSO-d6) δ -76.06 (s, 3F), -103.04 - -103.16 (m, 1F), -109.19 - -109.29 (m, 2F), -109.64 (d, *J* = 9.6 Hz, 1F).

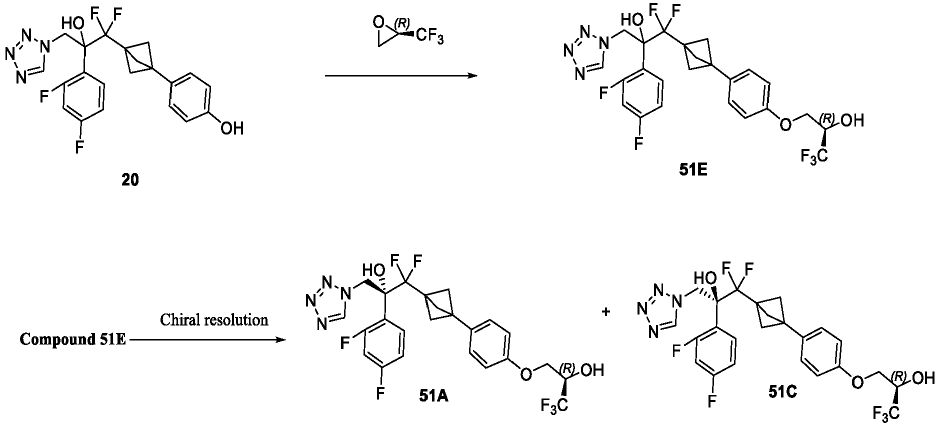

### Preparation of compound 51E

Compound **20** (0.2 g, 0.46 mmol) was dissolved in acetonitrile (2 mL) solvent, then potassium carbonate (0.127 g, 0.922 mmol) was added thereto with stirring. After the reaction was carried out for 5 min, the reaction mixture was then added with compound R-(+)-2-trifluoromethyloxirane (CAS number: 143142-90-9, 67 mg, 0.6 mmol), stirred and reacted at 50°C for 16 hours. The reaction mixture was filtered, and the crude filtrate was purified by preparative separation (preparation method: chromatographic column: Agilent 10 Prep-C18 250 × 21.2 mm; column temperature: 25°C; mobile phase: water (0.1% TFA)-acetonitrile; proportion of acetonitrile in the mobile phase: 55% to 75% in 12 min; flow rate: 30 mL/min) to obtain the title compound **51E** (0.14 g, yield: 56%, containing a pair of diastereoisomers).

Compound **51E:** LC-MS (ESI): m/z 547.2 [M+H]⁺. ¹H NMR (400 MHz, DMSO-76) δ 9.13 (s, 1H), 7.54 (td, *J* = 9.0, 6.6 Hz, 1H), 7.33 - 7.22 (m, 1H), 7.14 (s, 1H), 7.06 - 7.03 (m, 2H), 7.03 - 6.98 (m, 1H), 6.91 - 6.82 (m, 2H), 6.62 (d, *J* = 6.6 Hz, 1H), 5.42 (d, *J* = 14.6 Hz, 1H), 5.01 (d, *J* = 14.6 Hz, 1H), 4.43 - 4.25 (m, 1H), 4.11 (dd, *J* = 10.6, 4.2 Hz, 1H), 4.01 (dd, J = 10.6, 6.4 Hz, 1H), 1.98 (dd, J= 9.4, 1.6 Hz, 3H), 1.77 (dd, *J* = 9.4, 1.6 Hz, 3H). ¹⁹F NMR (376 MHz, DMSO-d6) δ -76.06 (s, 3F), -102.95 - -103.16 (m, 1F), -109.19 - -109.29 (m, 2F), -109.64 (d, *J* = 9.6 Hz, 1F).

### Preparation of compounds 51A and 51C

Compound **51E** (0.14 g) was subjected to SFC chiral preparative resolution (preparative separation method, instrument model: MG II preparative SFC (SFC-14); model of chromatographic column: ChiralPak IC, 250 × 30 mm I.D. 10 µm; mobile phase: A: CO₂ B: ethanol (0.1% NH₃H₂O); elution gradient: B 20%; flow rate: 60 mL/min; column pressure: 100 bar; column temperature: 38°C; detection wavelength: 220 nm; cycle: about 11 min) to obtain the title compounds **51A** (60 mg) and **51C** (57 mg).

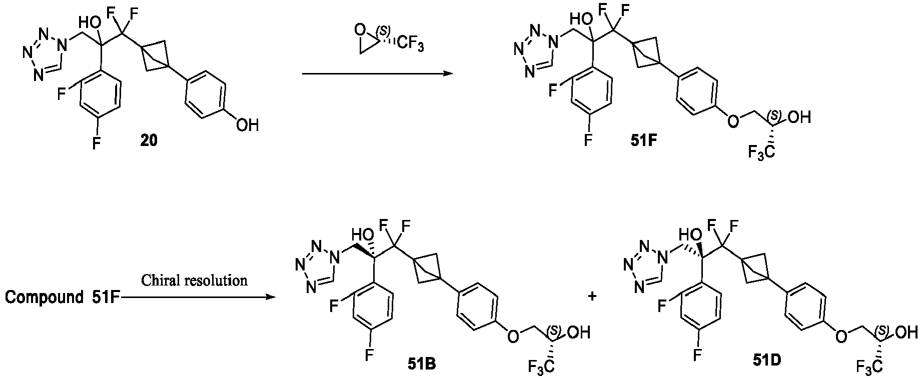

### Preparation of compound 51F

Compound **20** (0.2 g, 0.46 mmol) was dissolved in acetonitrile (2 mL), then potassium carbonate (0.127 g, 0.922 mmol) was added thereto with stirring. After the reaction was carried out for 5 min, the reaction mixture was then added with compound (S)-(-)-3,3,3-trifluoro-1,2-epoxypropane (CAS number: 130025-34-2, 67 mg, 0.6 mmol), stirred and reacted at 50°C for 16 hours. The reaction mixture was filtered, and the crude filtrate was purified by preparative separation (preparation method: chromatographic column: Agilent 10 Prep-C18 250 × 21.2 mm; column temperature: 25°C; mobile phase: water (0.1% TFA)-acetonitrile; proportion of acetonitrile in the mobile phase: 55% to 75% in 12 min; flow rate: 30 mL/min) to obtain the title compound **51F** (0.13 g, yield: 52%, containing a pair of diastereoisomers). LC-MS (ESI): m/z 547.2 [M+H]⁺. ¹H NMR (400 MHz, DMSO-*d*₆) δ 9.13 (s, 1H), 7.61 - 7.47 (m, 1H), 7.35 - 7.23 (m, 1H), 7.15 (s, 1H), 7.06 - 7.03 (m, 2H), 7.03 - 6.98 (m, 1H), 6.92 - 6.79 (m, 2H), 6.62 (d, *J* = 6.8 Hz, 1H), 5.42 (d, *J* = 14.6 Hz, 1H), 5.01 (d, *J* = 14.6 Hz, 1H), 4.43 - 4.24 (m, 1H), 4.19 - 4.11 (m, 1H), 4.09 - 3.92 (m, 1H), 1.98 (dd, *J* = 9.4, 1.6 Hz, 3H), 1.77 (dd, *J* = 9.4, 1.6 Hz, 3H). ¹⁹F NMR (376 MHz, DMSO-*d*₆) δ -76.06 (s, 3F), -102.95 - - 103.16 (m, 1F), -109.19 - -109.29 (m, 2F), -109.64 (d, *J* = 9.6 Hz, 1F).

### Preparation of compounds 51B and 51D

Compound **51F** (0.13 g) was subjected to SFC chiral preparative resolution (preparative separation method, instrument model: MG II preparative SFC (SFC-14); model of chromatographic column: ChiralPak IC, 250 × 30 mm I.D. 10 µm; mobile phase: A: CO₂ B: ethanol (0.1% NH₃H₂O); elution gradient: B 20%; flow rate: 60 mL/min; column pressure: 100 bar; column temperature: 38°C; detection wavelength: 220 nm; cycle: about 11 min) to obtain the title compounds **51B** (47 mg) and **51D** (48 mg).

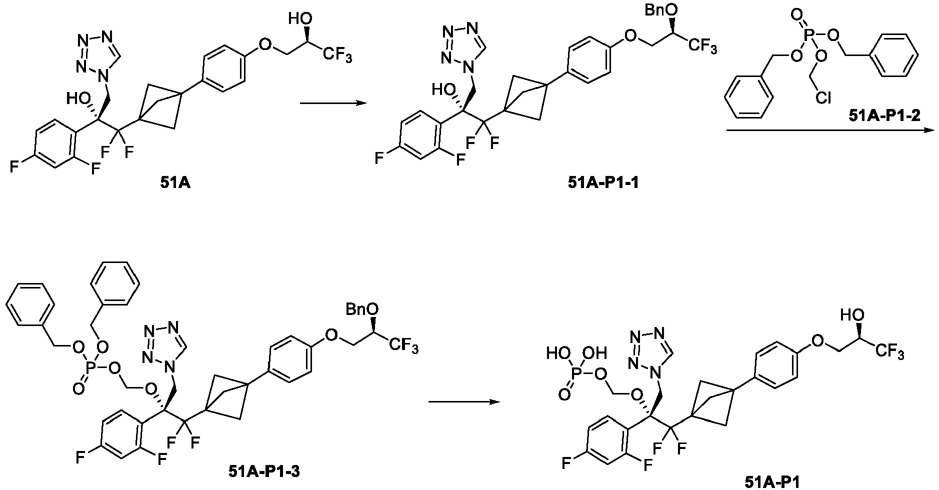

### Preparation of compound 51A-P1-1

Compound **51A** (1 g, 1.83 mmol) was dissolved in DMF (10 mL), then sodium hydride (81 mg, 2.01 mmol) was added thereto with stirring at 0°C. After the reaction was carried out for 15 min, the reaction mixture was then added with compound benzyl bromide (342 mg, 2.01 mmol), and reacted at room temperature for 12 hours. The reaction mixture was poured into ice water and extracted with EtOAc (20 mL × 3), and the organic phases were combined, dried, and concentrated to obtain a crude product. The crude product was purified by normal-phase silica gel column chromatography (EtOAc/PE = 0 to 30%) to obtain the title compound **51A-P1-1** (760 mg, yield: 65%). LC-MS (ESI): m/z 637.2 [M+H]⁺.

### Preparation of compound 51A-P1-3

Compound **51A-P1-1** (320 mg, 0.50 mmol) was dissolved in DMF (8 mL), then cesium carbonate (492 mg, 1.51 mmol) was added thereto with stirring. After the reaction was carried out for 15 min, the reaction mixture was then added with compound **51A-P1-2** (329 mg, 1.0 mmol), stirred and reacted at 50°C for 12 hours. The reaction mixture was filtered, and the crude filtrate was purified by preparative separation (preparation method: chromatographic column: Agilent 10 Prep-C18 250 × 21.2 mm; column temperature: 25°C; mobile phase: water (10 mM/L NH₄HCO₃)-acetonitrile; proportion of acetonitrile in the mobile phase: 60% to 90% in 9 min; flow rate: 30 mL/min) to obtain the title compound **51A-P1-3** (220 mg, yield: 47%). LC-MS (ESI): m/z 927.2 [M+H]⁺.

### Preparation of compound 51A-P1

Compound **51A-P1-3** (220 mg, 0.24 mmol) was dissolved in methanol (10 mL), then 10% wet palladium on carbon (22 mg) was added thereto. The system was replaced with nitrogen three times, and then replaced with hydrogen two times, and reacted at room temperature for 16 hours. The reaction mixture was filtered, and the crude filtrate was purified by preparative separation (preparation method: chromatographic column: Agilent 10 Prep-C18 250 × 21.2 mm; column temperature: 25°C; mobile phase: water (10 mM/L NH₄HCO₃)-acetonitrile; proportion of acetonitrile in the mobile phase: 25% to 45% in 12 min; flow rate: 30 mL/min) to obtain the title compound **51A-P1** (65 mg, yield: 42%). LC-MS (ESI): m/z 657.2 [M+H]⁺. ¹H NMR (400 MHz, DMSO-*d*₆ +D₂O (one drop)) δ 9.81 (s, 1H), 7.94 - 7.67 (m, 1H), 7.20 (ddd, *J* = 12.0, 9.0, 2.6 Hz, 1H), 7.10 - 6.98 (m, 3H), 6.91 - 6.83 (m, 2H), 5.73 - 5.26 (m, 4H), 4.45 - 4.24 (m, 1H), 4.17 - 3.95 (m, 2H), 1.97 (dd, *J* = 9.4, 1.6 Hz, 3H), 1.85 (dd, *J* = 9.4, 1.6 Hz, 3H).

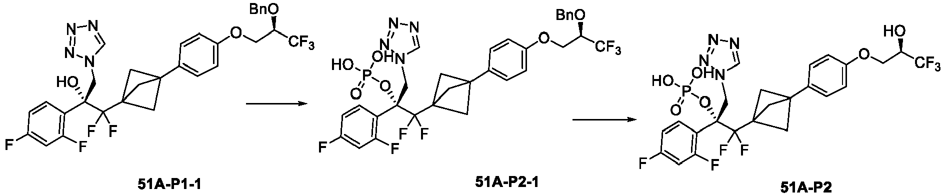

### Preparation of compound 51A-P2-1

To a sealed tube was added compound **51A-P1-1** (300 mg, 0.47 mmol), and then NaH (135 mg, 3.39 mmol) and THF (3 mL) were added thereto at 0°C under nitrogen atmosphere. The reaction mixture was reacted for 30 min, slowly added with phosphorus oxychloride (0.5 mL), and reacted at room temperature for 16 hours. The reaction mixture was added dropwise with saturated sodium bicarbonate solution until the reaction mixture was weakly alkaline, and reacted at 50°C for 16 hours. After the reaction system was cooled, the reaction mixture was filtered, and the crude filtrate was purified by preparative separation (chromatographic column: Agilent 10 Prep-C18 250 × 21.2 mm; column temperature: 25°C; mobile phase: water (0.1% TFA)-acetonitrile; proportion of acetonitrile in the mobile phase: 55% to 80% in 12 min; flow rate: 30 mL/min) to obtain the title compound **51A-P2-1** (200 mg, yield: 59%). LC-MS (ESI): 717.20 [M+H]⁺.

### Preparation of compound 51A-P2

Compound **51A-P2-1** (200 mg, 0.28 mmol) was dissolved in methanol (10 mL), then 10% wet palladium on carbon (20 mg) was added thereto. The system was replaced with nitrogen three times, and then replaced with hydrogen two times, and reacted at room temperature for 16 hours. The reaction mixture was filtered, and the crude filtrate was purified by preparative separation (preparation method: chromatographic column: Agilent 10 Prep-C18 250 × 21.2 mm; column temperature: 25°C; mobile phase: water (10 mM/L NH₄HCO₃)-acetonitrile; proportion of acetonitrile in the mobile phase: 25% to 45% in 12 min; flow rate: 30 mL/min) to obtain the title compound **51A-P2** (82 mg, yield: 46%). LC-MS (ESI): m/z 627.2 [M+H]⁺. ¹H NMR (400 MHz, DMSO-*d*₆) δ 10.26 (s, 1H), 8.16 - 8.04 (m, 1H), 7.20 - 7.08 (m, 1H), 7.09 - 7.01 (m, 1H), 7.05 - 6.95 (m, 2H), 6.90 - 6.82 (m, 2H), 6.72 - 6.50 (m, 3H), 6.17 (d, *J* = 14.4 Hz, 1H), 5.54 (d, *J* = 14.4 Hz, 1H), 4.45 - 4.23 (m, 1H), 4.11 (dd, *J* = 10.5, 4.2 Hz, 1H), 4.00 (dd, *J* = 10.5, 4.2 Hz, 1H), 1.92 (dd, *J* = 9.5, 1.7 Hz, 3H), 1.76 (dd, *J* = 9.5, 1.7 Hz, 3H). ¹⁹F NMR (376 MHz) δ -76.07 (s, 3F), -101.37 - -101.99 (m, 1F), - 107.82 - -109.04 (m, 2F), -109.70 - -111.38 (m, 1F).

### Example 52: Preparation of compound 52

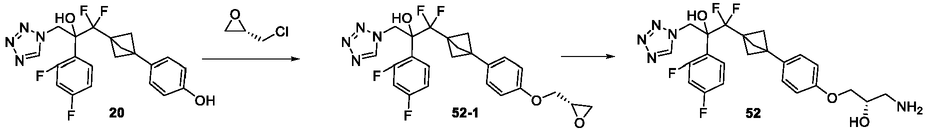

### Preparation of compound 52-1

To a sealed tube was added compound **20** (200 mg, 0.46 mmol), then compound (S)-(+)-epichlorohydrin (CAS: 67843-74-7, 64 mg, 0.69 mmol), Cs₂CO₃ (225 mg, 0.69 mmol), and DMF (1 mL) were added thereto, and the reaction mixture was reacted at 50°C for 16 hours. After the reaction system was cooled, the reaction mixture was evaporated to dryness by rotary evaporation to obtain the crude title compound **52-1** (200 mg, yield: 89%). LC-MS (ESI): m/z 491.00 [M+H]⁺.

### Preparation of compound 52

To a sealed tube was added the crude compound **52-1** (200 mg, 0.41 mmol), and ammonia methanol solution (7.0 M, 1.5 mL) was added thereto, and the reaction mixture was reacted at 50°C for 16 hours. After the reaction system was cooled, the reaction mixture was filtered, and the crude filtrate was purified by preparative separation (chromatographic column: Agilent 10 Prep-C18 250 × 21.2 mm; column temperature: 25°C; mobile phase: water (0.1% FA)-acetonitrile; proportion of acetonitrile in the mobile phase: 35% to 55% in 12 min; flow rate: 30 mL/min) to obtain the title compound **52** (160 mg, yield: 77%).

Compound **52:** LC-MS (ESI): m/z 508.00 [M+H]+. ¹H NMR (400 MHz, DMSO-d6) δ 9.14 (t, *J* = 1.9 Hz, 1H), 7.60-7.47 (m, 1H), 7.35 -7.24 (m, 1H), 7.16 (br.s, 1H), 7.08 - 6.93 (m, 3H), 6.88 - 6.76 (m, 2H), 6.67 (br.s, 1H), 5.42 (d, *J =* 14.6 Hz, 1H), 5.01 (d, *J* = 14.6 Hz, 1H), 3.93 - 3.71 (m, 3H), 2.90 - 2.61 (m, 2H), 1.97 (dd, *J* = 9.5, 1.7 Hz, 3H), 1.75 (dd, *J* = 9.5, 1.6 Hz, 3H), 1.34 - 1.16 (m, 2H). ¹⁹F NMR (376 MHz, DMSO-d6) δ -102.66 - -103.37 (m, 1F), -108.96 - -109.47 (m, 2F), -109.65 (d, *J* = 9.5 Hz, 1F).

### Example 53: Preparation of compound 53

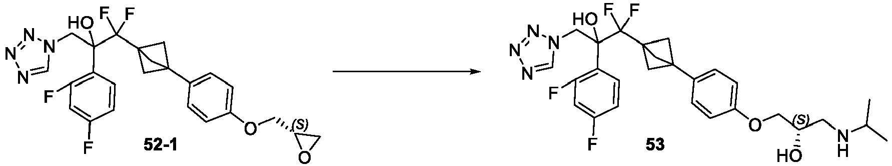

### Preparation of compound 53

Compound **52-1** (112 mg, 0.23 mmol) was placed in a microwave tube, to which DMF (5.0 mL) was added and an excess of isopropylamine (0.5 mL) was added dropwise thereto, and the reaction was carried out under microwave irradiation at 100°C for 2 hours. The reaction mixture was filtered, and the crude filtrate was purified by preparative separation (preparation method: chromatographic column: Agilent 10 Prep-C18 250 × 21.2 mm; column temperature: 25°C; mobile phase: water (0.1% FA)-acetonitrile; proportion of acetonitrile in the mobile phase: 15% to 35% in 12 min; flow rate: 30 mL/min) to obtain the title compound **53** (35 mg, total yield: 28%, containing a pair of diastereoisomers).

Compound **53:** LC-MS (ESI): m/z 550.2 [M+H]⁺. ¹H NMR (400 MHz, DMSO-d6) δ 9.16 (s, 1H), 8.31 (s, 1H), 7.54 (td, *J* = 9.0, 6.7 Hz, 1H), 7.28 (ddd, *J* = 12.0, 9.1, 2.6 Hz, 2H), 7.07 - 6.96 (m, 3H), 6.87 - 6.78 (m, 2H), 5.42 (d, *J* = 14.5 Hz, 1H), 5.02 (d, *J* = 14.5 Hz, 1H), 4.01 - 3.79 (m, 4H), 2.93 (m, 1H), 2.81 (m, 1H), 2.69 (m, 1H), 1.97 (dd, *J* = 9.4, 1.7 Hz, 3H), 1.75 (dd, *J* = 9.4, 1.7 Hz, 3H), 1.05 (d, *J* = 6.3 Hz, 6H). ¹⁹F NMR (376 MHz, DMSO-d6) δ - 102.93 - -103.14 (m, 1F), -109.20 - -109.30 (m, 2F), -109.68 (d, *J =* 9.2 Hz, 1F).

### Example 54: Preparation of compound 54

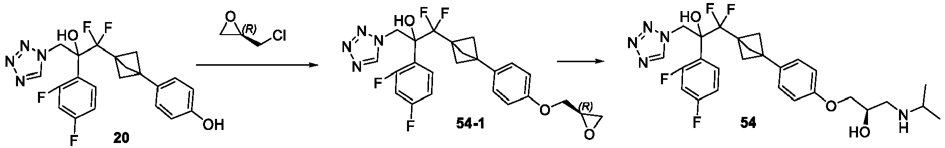

### Preparation of compound 54-1

Compound **20** (100 mg, 0.23 mmol) was dissolved in DMF (5 mL), then cesium carbonate (150 mg, 0.46 mmol) was added thereto with stirring. After the reaction was carried out for 5 min, the reaction mixture was then added with compound (R)-(-)-epichlorohydrin (CAS: 51594-55-9, 43 mg, 0.46 mmol), stirred and reacted at 50°C for 2 hours. After the reaction was tracked by LC-MS until the reaction was completed, the reaction mixture was filtered, and the crude filtrate was directly used as the raw material for the next reaction step. LC-MS (ESI): m/z 491.2 [M+H]⁺.

### Preparation of compound 54

The filtrate from the previous step was placed in a microwave tube, and an excess of isopropylamine (0.5 mL) was added dropwise thereto, and the reaction was carried out under microwave irradiation at 100°C for 2 hours. The reaction mixture was filtered, and the crude filtrate was purified by preparative separation (preparation method: chromatographic column: Agilent 10 Prep-C18 250 × 21.2 mm; column temperature: 25°C; mobile phase: water (0.1% FA)-acetonitrile; proportion of acetonitrile in the mobile phase: 15% to 35% in 12 min; flow rate: 30 mL/min) to obtain the title compound **54** (22 mg, total yield: 17%, containing a pair of diastereoisomers).

Compound **54:** LC-MS (ESI): m/z 550.2 [M+H]⁺. ¹H NMR (400 MHz, DMSO-d6) δ 9.17 (s, 1H), 8.31 (s, 1H), 7.54 (td, *J* = 9.0, 6.7 Hz, 1H), 7.28 (ddd, *J* = 12.0, 9.1, 2.7 Hz, 2H), 7.10 - 6.95 (m, 3H), 6.88 - 6.76 (m, 2H), 5.42 (d, *J* = 14.5 Hz, 1H), 5.02 (d, *J* = 14.5 Hz, 1H), 4.04 - 3.76 (m, 4H), 2.93 (m, 1H), 2.83 (m, 1H), 2.70 (m, 1H), 1.97 (dd, *J* = 9.4, 1.7 Hz, 3H), 1.76 (dd, *J* = 9.4, 1.6 Hz, 3H), 1.07 (d, *J* = 6.3 Hz, 6H). ¹⁹F NMR (376 MHz, DMSO-d6) δ - 102.92 - -103.14 (m, 1F), -109.19 - -109.29 (m, 2F), -109.68 (d, *J* = 9.2 Hz, 1F).

### Example 55: Preparation of compound 55

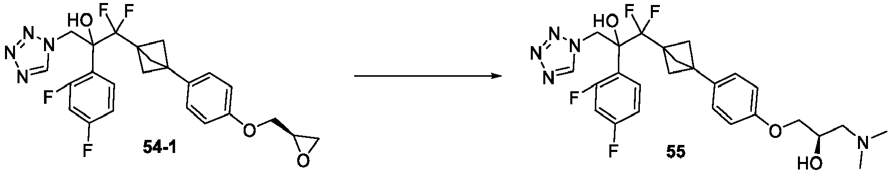

### Preparation of compound 55

To a sealed tube was added compound **54-1** (200 mg, 0.41 mmol), then NaOH (1.0 M, 0.3 mL) and DMF (1 mL) were added thereto, and the reaction mixture was reacted at 50°C for 16 hours. After the reaction system was cooled, the reaction mixture was filtered, and the crude filtrate was purified by preparative separation (chromatographic column: Welch Xtimate@ C18 21.2 × 250 mm; column temperature: 25°C; mobile phase: water (10 mM/L NH₄HCO₃)-acetonitrile; proportion of acetonitrile in the mobile phase: 35% to 55% in 12 min; flow rate: 30 mL/min) to obtain the title compound **55** (130 mg, yield: 53%).

Compound **55:** LC-MS (ESI): m/z 536.00 [M+H]⁺. ¹H NMR (400 MHz, DMSO-*d6*) δ 9.13 (s, 1H), 7.59 - 7.48 (m, 1H), 7.33 - 7.23 (m, 1H), 7.16 (s, 1H), 7.07 - 6.95 (m, 3H), 6.85 - 6.77 (m, 2H), 5.42 (d, *J* = 14.6 Hz, 1H), 5.01 (d, *J* = 14.6 Hz, 1H), 5.00 (br.s, 1H), 3.97 - 3.85 (m, 2H), 3.83 - 3.74 (m, 1H), 2.26 (s, 6H), 1.97 (dd, *J =* 9.5, 1.7 Hz, 3H), 1.75 (dd, *J =* 9.4, 1.7 Hz, 3H), 1.23 (br.s, 2H). ¹⁹F NMR (376 MHz, DMSO-d6) δ -102.81 - -103.33 (m, 1F), -109.25 (d, *J* = 37.6 Hz, 2F), -109.63 (d, *J* = 9.4 Hz, 1F).

### Example 56: Preparation of compound 56

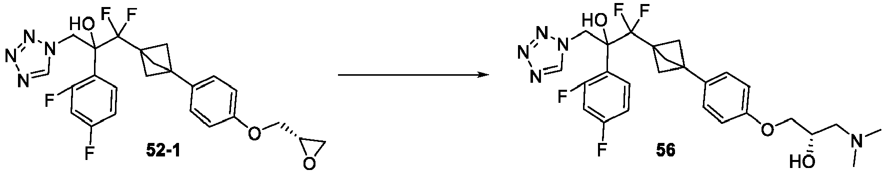

### Preparation of compound 56

To a sealed tube was added compound **52-1** (200 mg, 0.41 mmol), then NaOH aqueous solution (1.0 M, 0.3 mL) and DMF (1.0 mL) were added thereto, and the reaction mixture was reacted at 50°C for 16 hours. After the reaction system was cooled, the reaction mixture was filtered, and the crude filtrate was purified by preparative separation (chromatographic column: Welch Xtimate@ C18 21.2 × 250 mm; column temperature: 25°C; mobile phase: water (10 mM/L NH₄HCO₃)-acetonitrile; proportion of acetonitrile in the mobile phase: 35% to 55% in 12 min; flow rate: 30 mL/min) to obtain the title compound (130 mg, yield: 53%).

Compound **56:** LC-MS (ESI): m/z 536.0 [M+H]⁺. ¹H NMR (400 MHz, DMSO-d6) δ 9.14 (d, *J* = 1.5 Hz, 1H), 7.60 - 7.48 (m, 1H), 7.34 - 7.22 (m, 1H), 7.15 (s, 1H), 7.07 - 6.96 (m, 3H), 6.86 - 6.78 (m, 2H), 5.42 (d, *J* = 14.5 Hz, 1H), 5.12 (br.s, 1H), 5.01 (d, *J* = 14.6 Hz, 1H), 4.01 - 3.76 (m, 3H), 2.33 (d, *J* = 6.2 Hz, 6H), 1.97 (dd, *J* = 9.5, 1.7 Hz, 3H), 1.75 (dd, *J* = 9.4, 1.7 Hz, 3H), 1.23 (br.s, 2H). ¹⁹F NMR (376 MHz, DMSO-d6) δ -102.62 - -103.43 (m), -108.96 - -109.46 (m), -109.65 (d, *J* = 9.6 Hz).

### Example 57: Preparation of compound 57

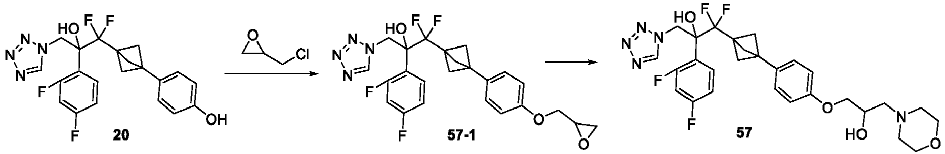

### Preparation of compound 57-1

To a sealed tube were added compound **20** (200 mg, 0.46 mmol), epichlorohydrin (CAS: 106-89-8, 64 mg, 0.69 mmol), CS₂CO₃ (225 mg, 0.69 mmol), and DMF (1 mL), and the reaction mixture was reacted at 50°C for 16 hours. After the reaction system was cooled, the reaction mixture was evaporated to dryness by rotary evaporation to obtain the title compound 57-1 (200 mg, yield: 89%). LC-MS (ESI): m/z 491.00 [M+H]⁺.

### Preparation of compound 57

To a sealed tube were added compound **57-1** (200 mg, 0.41 mmol), morpholine (0.3 mL), and ethanol (1 mL), and the reaction mixture was reacted at 80°C for 16 hours. After the reaction system was cooled, the reaction mixture was filtered, and the crude filtrate was purified by preparative separation (chromatographic column: Agilent 10 Prep-C18 250 × 21.2 mm; column temperature: 25°C; mobile phase: water (0.1% FA)-acetonitrile; proportion of acetonitrile in the mobile phase: 35% to 55% in 12 min; flow rate: 30 mL/min) to obtain the title compound **57** (147 mg, yield: 60%, containing two pairs of enantiomers).

Compound **57:** LC-MS (ESI): m/z 578.20 [M+H]⁺. ¹H NMR (400 MHz, DMSO-d6) δ 9.23 - 9.12 (m, 1H), 7.60 - 7.48 (m, 1H), 7.38 - 7.11 (m, 2H), 7.07 - 6.95 (m, 3H), 6.86 - 6.77 (m, 2H), 5.42 (d, *J* = 14.4 Hz, 1H), 5.03 (d, *J =* 14.4 Hz, 1H), 4.88 (br.s, 1H), 4.00 - 3.83 (m, 3H), 3.83 - 3.72 (m, 2H), 3.60 (m, 4H), 2.48 - 2.22 (m, 4H), 1.97 (dd, *J* = 9.3, 1.7 Hz, 3H), 1.75 (dd, *J* = 9.4, 1.7 Hz, 3H). ¹⁹F NMR (376 MHz, DMSO-d6) δ -102.94 - -103.15 (m, 1F), -109.20 - -109.30 (m, 2F), -109.69 (d, *J* = 9.4 Hz, 1F).

### Example 58: Preparation of compound 58

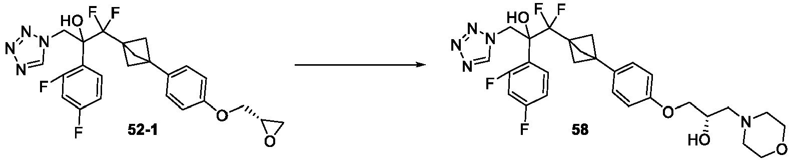

### Preparation of compound 58

To a sealed tube were added compound **52-1** (200 mg, 0.41 mmol), morpholine (0.3 mL), and ethanol (1 mL), and the reaction mixture was reacted at 80°C for 16 hours. After the reaction system was cooled, the reaction mixture was filtered, and the crude filtrate was purified by preparative separation (chromatographic column: Welch Xtimate^{®} C18 21.2 × 250 mm; column temperature: 25°C; mobile phase: water (10 mM/L NH₄HCO₃)-acetonitrile; proportion of acetonitrile in the mobile phase: 35% to 55% in 12 min; flow rate: 30 mL/min) to obtain the title compound **58** (147 mg, yield: 60%).

Compound **58:** LC-MS (ESI): m/z 578.2 [M+H]⁺. ¹H NMR (400 MHz, DMSO-d6) δ 9.14 (s, 1H), 7.60 - 7.47 (m, 1H), 7.35 - 7.22 (m, 1H), 7.15 (s, 1H), 7.08 - 6.96 (m, 3H), 6.90 - 6.73 (m, 2H), 5.42 (d, *J* = 14.5 Hz, 1H), 5.01 (d, *J* = 14.5 Hz, 1H), 4.86 (br.s, 1H), 4.14 - 3.78 (m, 4H), 3.61 (m, 5H), 2.76 - 2.56 (m, 2H), 2.43 - 2.25 (m, 2H), 1.97 (dd, *J* = 9.5, 1.7 Hz, 3H), 1.76 (dd, *J* = 9.5, 1.7 Hz, 3H). ¹⁹F NMR (376 MHz, DMSO-d6) δ -102.95 - -103.16 (m, 1F), -109.20 - -109.30 (m, 2F), -109.63- -109.66 (m, 1F).

### Example 59: Preparation of compound 59

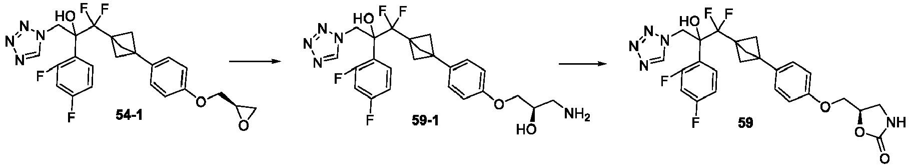

### Preparation of compound 59

To a sealed tube were added compound **54-1** (200 mg, 0.41 mmol) and ammonia methanol solution (7.0 M, 1.5 mL), and the reaction mixture was reacted at 50°C for 16 hours. After the reaction system was cooled, the reaction mixture was filtered, extracted, and evaporated to dryness by rotary evaporation to obtain the crude compound **59-1.** The crude compound **59-1** was directly transferred into a sealed tube without purification. At the same time, potassium carbonate (54 mg, 0.40 mmol), CDI (32 mg, 0.40 mmol), and THF (1.5 mL) were added to the sealed tube, and the reaction mixture was reacted at 70°C for 16 hours. After the reaction system was cooled, the reaction mixture was filtered, and the crude filtrate was purified by preparative separation (chromatographic column: Agilent 10 Prep-C18 250 × 21.2 mm; column temperature: 25°C; mobile phase: water (0.1% FA)-acetonitrile; proportion of acetonitrile in the mobile phase: 35% to 55% in 12 min; flow rate: 30 mL/min) to obtain the title compound **59** (40 mg, yield: 38%).

Compound **59:** LC-MS (ESI): m/z 534.0 [M+H]⁺. ¹H NMR (400 MHz, DMSO-d6) δ 9.13 (s, 1H), 7.69 - 7.45 (m, 2H), 7.28 (m, 1H), 7.16 (s, 1H), 7.08 - 6.95 (m, 3H), 6.90 - 6.80 (m, 2H), 5.42 (d, *J* = 14.4 Hz, 1H), 5.01 (d, *J* = 14.4 Hz, 1H), 4.92 - 4.80 (m, 1H), 4.22 - 3.89 (m, 2H), 3.60 (m, 1H), 3.25 (m, 1H), 1.98 (m, 3H), 1.82 - 1.70 (m, 3H). ¹⁹F NMR (376 MHz, DMSO-d6) δ -102.94 (m, 1F), -109.25 (m, 2F), -109.62 (m, 1F).

### Example 60: Preparation of compound 60

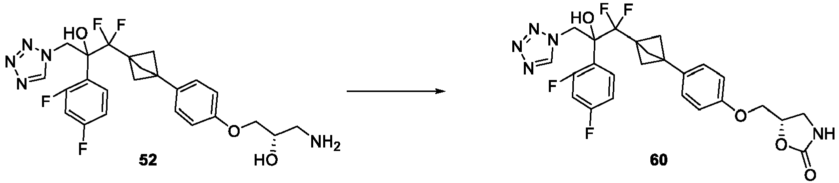

### Preparation of compound 60

To a sealed test tube was added compound **52** (100 mg, 0.20 mmol), then potassium carbonate (54 mg, 0.40 mmol), CDI (32 mg, 0.40 mmol), and THF (1.5 mL) solvent were respectively added thereto. The reaction system was reacted at 70°C for 16 hours. After the reaction system was cooled, the reaction mixture was filtered, and the crude filtrate was purified by preparative separation (chromatographic column: Agilent 10 Prep-C18 250 × 21.2 mm; column temperature: 25°C; mobile phase: water (0.1% FA)-acetonitrile; proportion of acetonitrile in the mobile phase: 35% to 55% in 12 min; flow rate: 30 mL/min) to obtain the title compound **60** (40 mg, yield: 38%).

Compound **60:** LC-MS (ESI): m/z 534.00 [M+H]⁺. ¹H NMR (400 MHz, DMSO-d6) δ 9.13 (s, 1H), 7.63 - 7.48 (m, 2H), 7.36 - 7.22 (m, 1H), 7.14 (s, 1H), 7.10 - 6.94 (m, 3H), 6.92 - 6.79 (m, 2H), 5.42 (d, *J* = 14.6 Hz, 1H), 5.01 (d, *J* = 14.6 Hz, 1H), 4.93 - 4.80 (m, 1H), 4.16 - 4.00 (m, 2H), 3.58 (t, *J* = 8.9 Hz, 1H), 3.25 (m, 1H), 1.98 (dd, *J* = 9.5, 1.7 Hz, 3H), 1.76 (dd, *J* = 9.4, 1.7 Hz, 3H). ¹⁹F NMR (376 MHz) δ -102.54 - -103.49 (m, 1F), -109.26 (d, *J* = 36.8 Hz, 2F), -109.64 (d, *J* = 9.6 Hz, 1F).

### Example 61: Preparation of compound 61

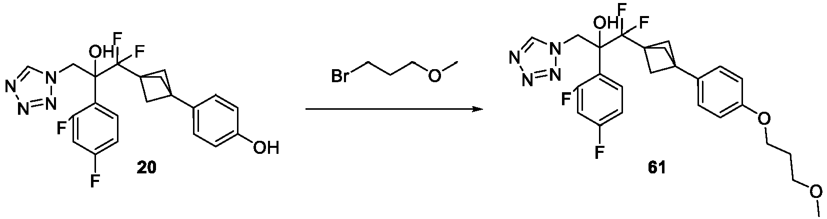

### Preparation of compound 61

Compound **20** (100 mg, 0.23 mmol) was dissolved in DMF (5 mL), then cesium carbonate (90 mg, 0.276 mmol) was added thereto with stirring. After the reaction was carried out for 5 min, the reaction mixture was then added with compound 1-bromo-3-methoxypropane (42 mg, 0.253 mmol), stirred and reacted at 50°C for 4 hours. The reaction mixture was filtered, and the crude filtrate was purified by preparative separation (preparation method: chromatographic column: Agilent 10 Prep-C18 250 × 21.2 mm; column temperature: 25°C; mobile phase: water (0.1% FA)-acetonitrile; proportion of acetonitrile in the mobile phase: 55% to 75% in 12 min; flow rate: 30 mL/min) to obtain the title compound **61** (60 mg, yield: 50%, containing a pair of enantiomers).

Compound **61:** LC-MS (ESI): m/z 507.2 [M+H]⁺. ¹H NMR (400 MHz, DMSO-*d6*) δ 9.13 (s, 1H), 7.57 - 7.51 (m, 1H), 7.31 - 7.25 (m, 1H), 7.15 (s, 1H), 7.04 - 6.97 (m, 3H), 6.87 - 6.73 (m, 2H), 5.43 (d, *J* = 14.6 Hz, 1H), 5.01 (d, *J* = 14.6 Hz, 1H), 3.95 (t, *J* = 6.4 Hz, 2H), 3.43 (t, *J* = 6.4 Hz, 2H), 3.23 (s, 3H), 1.99 - 1.96 (m, 3H), 1.91 - 1.87 (m, 2H), 1.77 - 14.74 (m, 3H). ¹⁹F NMR (376 MHz, DMSO-d6) δ -102.95- -103.16 (m, 1F), -109.18 - -109.28 (m, 2F), -109.64 (d, *J* = 9.6 Hz, 1F).

### Example 62: Preparation of compound 62

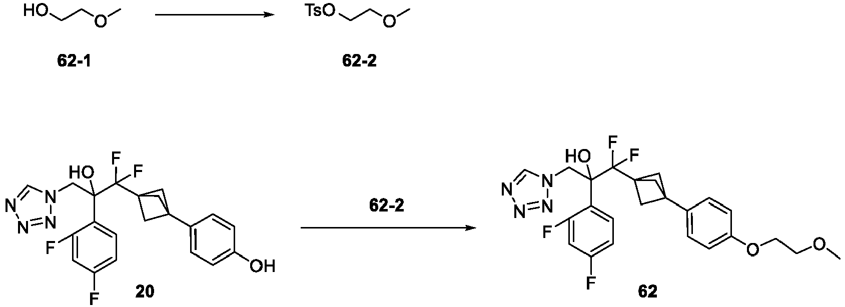

### Preparation of compound 62-2

Compound **62-1** (100 mg, 1.32 mmol) was dissolved in dichloromethane (5 mL), then triethylamine (266 mg, 2.64 mmol) was added dropwise thereto at 0°C. After the reaction was carried out for 10 min, p-toluenesulfonyl chloride (276 mg, 1.45 mmol) was added thereto, reacted at room temperature for 4 hours. The reaction was tracked by TLC until the reaction was completed, then the reaction system was extracted with DCM (20 mL × 3), and the organic phases were combined, dried, and concentrated to obtain the crude title compound 62-2 (150 mg, yield: 49%), which was directly used as the raw material for the next reaction step.

### Preparation of compound 62

Compound **20** (50 mg, 0.12 mmol) was dissolved in DMF (5 mL), then cesium carbonate (78 mg, 0.24 mmol) was added thereto with stirring. After the reaction was carried out for 5 min, the reaction mixture was then added with compound **62-2** (42 mg, 0.18 mmol), stirred and reacted at 50°C for 4 hours. The reaction mixture was filtered, and the crude filtrate was purified by preparative separation (preparation method: chromatographic column: Agilent 10 Prep-C18 250 × 21.2 mm; column temperature: 25°C; mobile phase: water (0.1% NH₄HCO₃)-acetonitrile; proportion of acetonitrile in the mobile phase: 45% to 65% in 12 min; flow rate: 30 mL/min) to obtain the title compound **62** (33 mg, yield: 30%, containing a pair of enantiomers).

Compound **62:** LC-MS (ESI): m/z 493.0 [M+H]+. ¹H NMR (400 MHz, DMSO-^6) δ 9.14 (s, 1H), 7.59 - 7.48 (m, 1H), 7.33 - 7.22 (m, 1H), 7.14 (s, 1H), 7.07 - 6.96 (m, 3H), 6.88 - 6.78 (m, 2H), 5.43 (d, *J* = 14.5 Hz, 1H), 5.01 (d, *J* = 14.6 Hz, 1H), 4.06 - 3.98 (m, 2H), 3.65 - 3.58 (m, 2H), 3.28 (s, 3H), 2.01 - 1.91 (m, 3H), 1.80 - 1.72 (m, 3H). ¹⁹F NMR (376 MHz, DMSO-d6) δ -102.96 - -103.17 (m, 1F), -109.19 - -109.29 (m, 2F), -109.63 - -109.66 (m, 1F).

### Example 63: Preparation of compound 63

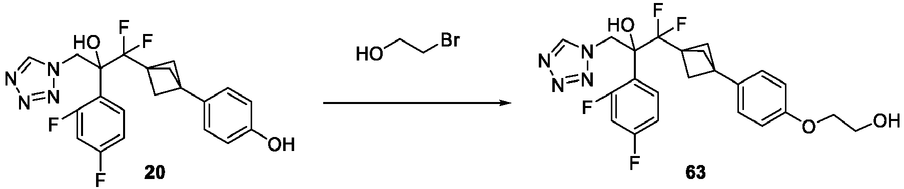

### Preparation of compound 63

To a three-necked flask was added compound 20 (80 mg, 0.18 mmol), and then cesium carbonate (176 mg, 0.54 mmol), bromoethanol (45 mg, 0.36 mmol), and DMF (2 mL) were sequentially added thereto. After the system was replaced with nitrogen three times, the reaction mixture was stirred at 60°C for 16 hours. After the reaction was completed and the reaction system was cooled, the reaction mixture was filtered, and the crude filtrate was purified by preparative separation (preparation method: mobile phase: A: 0.1% formic acid aqueous solution; B: acetonitrile; chromatographic column: Agilent 10 Prep-C18 250 × 21.2 mm; column temperature: 25°C; gradient: 35% to 55% acetonitrile in 12 min; flow rate: 30 mL/min) to obtain the title compound **63** (13.9 mg, yield: 16.1%, containing a pair of enantiomers).

Compound **63:** LC-MS (ESI): m/z 479.2 [M+H]⁺. ¹H NMR (400 MHz, DMSO-d6) δ 9.14 (s, 1H), 7. 57 - 7.53 (m, 1H), 7.31 - 7.25 (m, 1H), 7.18 (s, 1H), 7.04 - 6.98 (m, 3H), 6.84 - 6.80 (m, 2H), 5.42 (d, *J* = 14.6 Hz, 1H), 5.02 (d, *J* = 14.6 Hz, 1H), 4.83 (m, 1H), 3.91 (t, *J* = 4.0 Hz, 2H), 3.67 (t, *J* = 4.0 Hz, 2H), 1.99 - 1.96 (m, 3H), 1.77 - 1.74 (m, 3H). ¹⁹F NMR (376 MHz, DMSO-*d6*) δ -102.95 - -103.17 (m, 1F), -109.19 -109.29 (m, 2F), -109.65 - -109.67 (m, 1F).

### Example 64: Preparation of compound 64

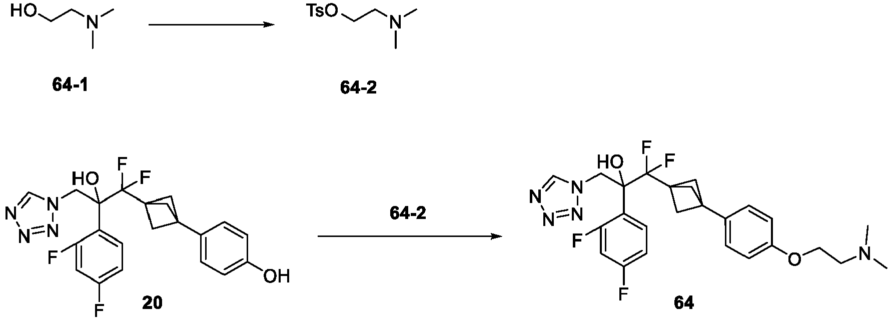

### Preparation of compound 64-2

Compound **64-1** (100 mg, 1.12 mmol) was dissolved in dichloromethane (5 mL), then triethylamine (266 mg, 2.24 mmol) was added dropwise thereto at 0°C. After the reaction was carried out for 10 min, p-toluenesulfonyl chloride (276 mg, 1.68 mmol) was added thereto, reacted at room temperature for 4 hours. The reaction was tracked by TLC until the reaction was completed, then the reaction system was extracted with DCM (20 mL × 3), and the organic phases were combined, dried, and concentrated to obtain the crude title compound **64-2** (170 mg), which was directly used as the raw material for the next reaction step.

### Preparation of compound 64

Compound **20** (50 mg, 0.12 mmol) was dissolved in DMF (5 mL), then cesium carbonate (78 mg, 0.24 mmol) was added thereto with stirring. After the reaction was carried out for 5 min, the reaction mixture was then added with compound **64-2** (42 mg, 0.17 mmol), stirred and reacted at 50°C for 4 hours. The reaction mixture was filtered, and the crude filtrate was purified by preparative separation (preparation method: chromatographic column: Agilent 10 Prep-C18 250 × 21.2 mm; column temperature: 25°C; mobile phase: water (0.1% NH₄HCO₃)-acetonitrile; proportion of acetonitrile in the mobile phase: 45% to 65% in 12 min; flow rate: 30 mL/min) to obtain the title compound **64** (2.5 mg, yield: 2%, containing a pair of enantiomers).

Compound **64:** LC-MS (ESI): m/z 506.0 [M+H]⁺. ¹H NMR (400 MHz, DMSO-d6) δ 9.13 (s, 1H), 7.63 - 7.46 (m, 1H), 7.36 - 7.22 (m, 1H), 7.15 (s, 1H), 7.09 - 6.95 (m, 3H), 6.90 - 6.73 (m, 2H), 5.43 (d, *J* = 14.5 Hz, 1H), 5.01 (d, *J* = 14.5 Hz, 1H), 4.03 - 3.93 (m, 2H), 2.63 - 2.54 (m, 2H), 2.19 (s, 6H), 1.99 - 1.94 (m, 3H), 1.84 - 1.68 (m, 3H). ¹⁹F NMR (376 MHz, DMSO-d6) δ -102.96 - -103.18 (m, 1F), -109.20 - -109.30 (m, 2F), -109.63- -109.65 (m, 1F).

### Example 65: Preparation of compound 65

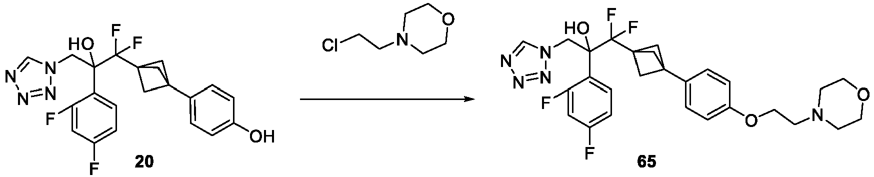

### Preparation of compound 65

Compound **20** (120 mg, 0.28 mmol) was dissolved in DMF (5 mL), then cesium carbonate (180 mg, 0.55 mmol) was added thereto with stirring. After the reaction was carried out for 5 min, the reaction mixture was then added with compound 4-(2-chloroethyl)morpholine (83 mg, 0.55 mmol), stirred and reacted at 50°C for 4 hours. The reaction mixture was filtered, and the crude filtrate was purified by preparative separation (preparation method: chromatographic column: Agilent 10 Prep-C18 250 × 21.2 mm; column temperature: 25°C; mobile phase: water (0.1% TFA)-acetonitrile; proportion of acetonitrile in the mobile phase: 15% to 35% in 12 min; flow rate: 30 mL/min) to obtain the title compound 65 (65 mg, yield: 43%, containing a pair of enantiomers).

Compound **65:** LC-MS (ESI): m/z 548.2 [M+H]⁺. ¹H NMR (400 MHz, DMSO-d6) δ 9.13 (s, 1H), 7.54 (td, *J* = 9.0, 6.7 Hz, 1H), 7.28 (ddd, *J* = 12.0, 9.1, 2.6 Hz, 1H), 7.15 (br.s, 1H), 7.07 - 6.96 (m, 3H), 6.88 - 6.77 (m, 2H), 5.42 (d, *J* = 14.5 Hz, 1H), 5.02 (d, *J* = 14.5 Hz, 1H), 4.02 (t, *J* = 5.8 Hz, 2H), 3.56 (t, *J* = 4.7 Hz, 4H), 2.64 (t, *J* = 5.8 Hz, 2H), 2.44 (t, *J* = 4.6 Hz, 4H), 1.97 (dd, *J* = 9.4, 1.7 Hz, 3H), 1.76 (dd, *J* = 9.4, 1.7 Hz, 3H). ¹⁹F NMR (376 MHz, DMSO-d6) δ -102.96 - -103.17 (m, 1F), -109.20- -109.30 (m, 2F), -109.64 (d, *J* = 9.2 Hz, 1F).

### Example 66: Preparation of compound 66

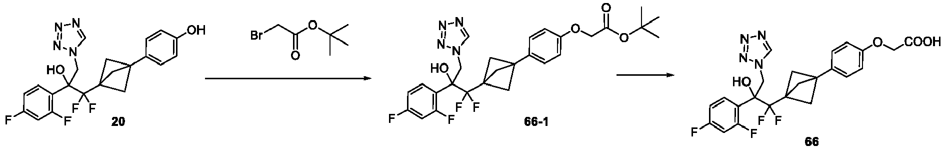

### Preparation of compound 66-1

Compound **20** (150 mg, 0.35 mmol) was dissolved in THF (1.5 mL), then potassium carbonate (72 mg, 0.52 mmol) and compound tert-butyl bromoacetate (134 mg, 0.69 mmol) were added thereto, and the reaction mixture was reacted at 60°C for 16 hours in a sealed tube. The reaction mixture was extracted with EtOAc (20 mL × 3) and evaporated to dryness by rotary evaporation to obtain compound **66-1** (110 mg, yield: 57%), which was directly used as the raw material for the next reaction step. LC-MS (ESI): m/z 549.2 [M+H]⁺.

### Preparation of compound 66

Compound **66-1** (110 mg, 0.2 mmol) was dissolved in DCM (3.0 mL), then the reaction system was added with TFA (3.0 mL) in a cooling environment at 0°C, and reacted at room temperature for 16 hours. The reaction solvent was evaporated to dryness by rotary evaporation, dissolved in MeOH, and filtered to obtain a crude product, which was purified by preparative separation (preparation method: chromatographic column: Welch Xtimate@ C18 21.2 × 250 mm; column temperature: 25°C; mobile phase: water (10 mM/L NH₄HCO₃)-acetonitrile; proportion of acetonitrile in the mobile phase: 20% to 40% in 12 min; flow rate: 30 mL/min) to obtain the title compound **66** (37 mg, yield: 38%).

Compound **66:** LC-MS (ESI): m/z 493.2 [M+H]⁺. ¹H NMR (400 MHz, DMSO-76) δ 9.17 (d, *J* = 1.3 Hz, 1H), 7.53 (td, *J* = 9.0, 6.7 Hz, 1H), 7.27 (ddd, *J* = 12.0, 9.1, 2.7 Hz, 2H), 7.06 - 6.92 (m, 3H), 6.78 - 6.66 (m, 2H), 5.42 (d, *J* = 14.6 Hz, 1H), 5.03 (d, *J* = 14.6 Hz, 1H), 4.40 (s, 2H), 1.96 (dd, *J* = 9.5, 1.7 Hz, 3H), 1.75 (dd, *J* = 9.5, 1.7 Hz, 3H). ¹⁹F NMR (376 MHz, DMSO-d6) δ -102.95--103.16 (m, 1F), -109.19--109.71 (m, 3F).

### Example 67: Preparation of compound 67

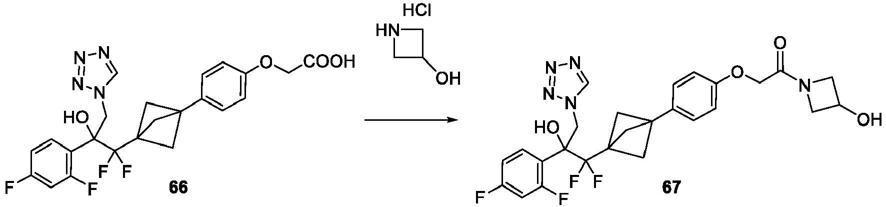

### Preparation of compound 67

To a microwave reaction tube were respectively added compound **66** (25 mg, 0.05 mmol), solvent acetonitrile (2.0 mL), compound N-methylimidazole (8 mg, 0.1 mmol), 3-hydroxyazetidine hydrochloride (CAS: 18621-18-6, 8 mg, 0.07 mmol), and N,N,N',N'-tetramethylchloroformamidinium hexafluorophosphate (18 mg, 0.06 mmol), then the reaction mixture was reacted at room temperature for 16 hours in the sealed tube. The reaction mixture was filtered, and the crude filtrate was purified by preparative separation (preparation method: chromatographic column: Welch Xtimate@ C18 21.2 × 250 mm; column temperature: 25°C; mobile phase: water (10 mM/L NH₄HCO₃)-acetonitrile; proportion of acetonitrile in the mobile phase: 30% to 50% in 12 min; flow rate: 30 mL/min) to obtain the title compound 67 (13.8 mg, yield: 50%). LC-MS (ESI): m/z 548.2 [M+H]⁺. ¹H NMR (400 MHz, DMSO-d6) δ 9.13 (s, 1H), 7.62 - 7.42 (m, 1H), 7.28 (ddd, *J* = 11.9, 9.1, 2.6 Hz, 1H), 7.15 (s, 1H), 7.09 - 6.93 (m, 3H), 6.87 - 6.69 (m, 2H), 5.73 (d, *J* = 6.1 Hz, 1H), 5.43 (d, *J* = 14.6 Hz, 1H), 5.01 (d, *J* = 14.6 Hz, 1H), 4.53 (d, *J* = 3.1 Hz, 2H), 4.50 - 4.39 (m, 1H), 4.39 - 4.31 (m, 1H), 4.07 (dd, *J* = 10.2, 6.8 Hz, 1H), 3.90 (dd, *J* = 9.4, 4.4 Hz, 1H), 3.59 (dd, *J* = 10.3, 4.4 Hz, 1H), 1.98 (dd, *J* = 9.4, 1.7 Hz, 3H), 1.76 (dd, *J* = 9.4, 1.7 Hz, 3H). ¹⁹F NMR (376 MHz, DMSO-*d6*) δ -103.07 (ddd, *J* = 41.1, 32.2, 9.3 Hz, 1F), -109.20 - -109.30 (m, 2F), -109.63 (d, *J* = 9.0 Hz, 1F).

### Example 68: Preparation of compound 68

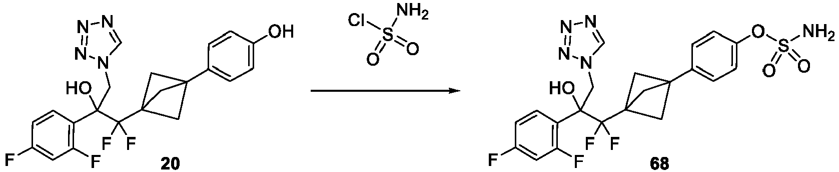

### Preparation of compound 68

Compound **20** (100 mg, 0.23 mmol) was dissolved in THF (1.5 mL), and NaH (14 mg, 0.35 mmol) was added thereto at 0°C. After the reaction system was stirred for 5 min, chlorosulfonamide (CAS: 7778-42-9, 53 mg, 0.46 mmol) was added thereto, and the reaction mixture was reacted at room temperature for 16 hours in a sealed tube. The reaction mixture was filtered, and the crude filtrate was purified by preparative separation (preparation method: mobile phase: A: 0.1% formic acid aqueous solution; B: acetonitrile; chromatographic column: Agilent 10 Prep-C18 250 × 21.2 mm; column temperature: 25°C; gradient: 35% to 55% acetonitrile in 12 min; flow rate: 30 mL/min) to obtain the title compound **68** (41 mg, yield: 34%).

Compound **68:** LC-MS: m/z 514.2 [M+H]⁺. ¹H NMR (400 MHz, DMSO-d6) δ 9.13 (s, 1H), 7.91 (br.s, 2H), 7.54 (td, *J* = 9.0, 6.6 Hz, 1H), 7.29 (ddd, *J* = 12.0, 9.0, 2.6 Hz, 1H), 7.23 -7.10 (m, 5H), 7.01 (td, *J* = 8.5, 2.7 Hz, 1H), 5.43 (d, *J* = 14.6 Hz, 1H), 5.01 (d, *J* = 14.6 Hz, 1H), 2.02 (dd, *J* = 9.3, 1.7 Hz, 3H), 1.81 (dd, *J* = 9.3, 1.8 Hz, 3H). ¹⁹F NMR (376 MHz, DMSO-d6) δ -102.94 - -103.16 (m, 1F), -109.28 - -109.39 (m, 2F), -109.57 (d, J= 9.3 Hz, 1F).

### Example 69: Preparation of compound 69

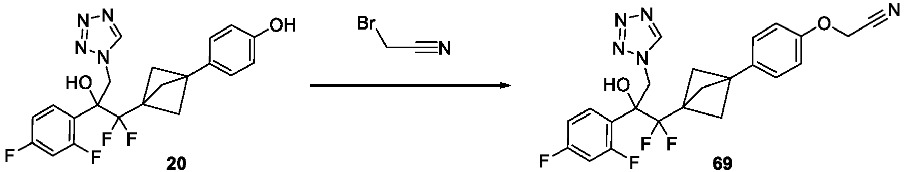

### Preparation of compound 69

Compound **20** (50 mg, 0.12 mmol) was dissolved in THF (1.0 mL), and the reaction system was added with NaH (9 mg, 0.23 mmol) at 0°C, stirred and reacted for 5 min, then added with bromoacetonitrile (27 mg, 0.23 mmol). The reaction mixture was reacted at room temperature for 16 hours in a sealed tube. The reaction mixture was filtered, and the crude filtrate was purified by preparative separation (preparation method: chromatographic column: Welch Xtimate@ C18 21.2 × 250 mm; column temperature: 25°C; mobile phase: water (10 mM/L NH₄HCO₃)-acetonitrile; proportion of acetonitrile in the mobile phase: 45% to 65% in 12 min; flow rate: 30 mL/min) to obtain the title compound **69** (24 mg, yield: 42%).

Compound **69:** LC-MS: m/z 472.0 [M-H]⁻. ¹H NMR (400 MHz, Chloroform-d) δ 8.53 (s, 1H), 7.65 (td, *J* = 8.9, 6.4 Hz, 1H), 7.12 - 7.01 (m, 2H), 6.94 - 6.79 (m, 4H), 5.44 (d, *J* = 14.6 Hz, 1H), 5.05 (d, *J* = 14.6 Hz, 1H), 4.73 (s, 2H), 3.96 (br.s, 1H), 2.05 (dd, *J* = 9.5, 1.9 Hz, 3H), 1.86 (dd, *J* = 9.5, 1.9 Hz, 3H). ¹⁹F NMR (376 MHz, Chloroform-*d*) δ -106.88 (d, *J* = 9.6 Hz, 1F), -110.21 (d, *J* = 47.4 Hz, 2F), -110.41 (d, *J* = 15.4 Hz, 1F).

### Example 70: Preparation of compound 70

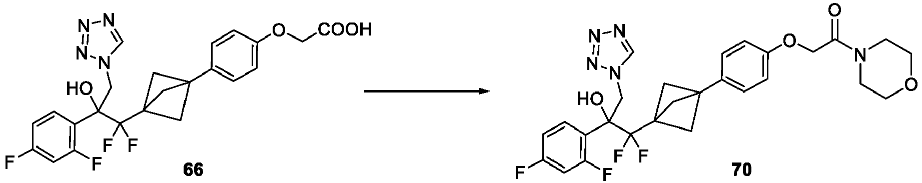

### Preparation of compound 70

To a reaction tube were respectively compound **66** (30 mg, 0.06 mmol), solvent acetonitrile (2.0 mL), N-methylimidazole (10 mg, 0.12 mmol), morpholine (11 mg, 0.12 mmol), and N,N,N',N'-tetramethylchloroformamidinium hexafluorophosphate (CAS: 94790-35-9, 23 mg, 0.08 mmol), then the reaction mixture was reacted at room temperature for 16 hours in a sealed tube. The reaction mixture was filtered, and the crude filtrate was purified by preparative separation (preparation method: chromatographic column: Welch Xtimate@ C18 21.2 × 250 mm; column temperature: 25°C; mobile phase: water (10 mM/L NH₄HCO₃)-acetonitrile; proportion of acetonitrile in the mobile phase: 40% to 60% in 12 min; flow rate: 30 mL/min) to obtain the title compound **70** (24.6 mg, yield: 73%).

Compound **70:** LC-MS: m/z 562.0 [M+H]⁺. ¹H NMR (400 MHz, Chloroform-*d*) δ 8.55 (s, 1H), 7.62 (td, *J* = 9.1, 6.3 Hz, 1H), 7.06 - 6.96 (m, 2H), 6.91 - 6.77 (m, 4H), 5.45 (d, *J* = 14.6 Hz, 1H), 5.03 (d, *J* = 14.6 Hz, 1H), 4.66 (s, 2H), 4.10 (s, 1H), 3.70 - 3.62 (m, 4H), 3.60 (dd, *J* = 12.1, 5.3 Hz, 4H), 2.01 (dd, *J* = 9.4, 1.8 Hz, 3H), 1.82 (dd, *J* = 9.4, 1.8 Hz, 3H). ¹⁹F NMR (376 MHz, Chloroform-*d*) δ -107.06 (d, *J* = 9.2 Hz, 1F), -110.21 (d, *J* = 47.3 Hz, 2F), -110.39 (d, *J* = 16.3 Hz, 1F).

### Example 71: Preparation of compound 71

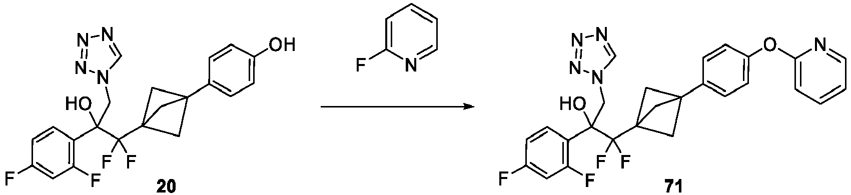

### Preparation of compound 71

Compound **20** (100 mg, 0.23 mmol) was dissolved in DMF (1.5 mL), then potassium carbonate (48 mg, 0.35 mmol) and 2-fluoropyridine (34 mg, 0.35 mmol) were added thereto, and the reaction mixture was reacted at 65°C for 16 hours in a sealed tube. The reaction mixture was filtered to obtain a crude product, which was subjected to preparative purification (preparation method: mobile phase: A: 0.1% formic acid aqueous solution; B: acetonitrile; chromatographic column: Agilent 10 Prep-C18 250 × 21.2 mm; column temperature: 25°C; gradient: 50% to 70% acetonitrile in 12 min; flow rate: 30 mL/min) to obtain the title compound **71** (3 mg, yield: 2.5%).

Compound 71: LC-MS: m/z 512.2 [M+H]⁺. ¹H NMR (400 MHz, DMSO-*d6*) δ 9.15 (s, 1H), 8.10 (dd, *J* = 5.1, 2.0 Hz, 1H), 7.82 (ddd, *J* = 8.9, 7.2, 2.0 Hz, 1H), 7.55 (td, *J* = 9.0, 6.7 Hz, 1H), 7.29 (ddd, *J =* 12.0, 9.1, 2.7 Hz, 1H), 7.21 (s, 1H), 7.20 - 7.14 (m, 2H), 7.10 (dd, *J* = 7.0, 4.7 Hz, 1H), 7.06 - 6.94 (m, 4H), 5.43 (d, *J* = 14.6 Hz, 1H), 5.02 (d, *J* = 14.6 Hz, 1H), 2.03 (dd, *J* = 9.5, 1.7 Hz, 3H), 1.81 (dd, *J* = 9.5, 1.7 Hz, 3H). ¹⁹F NMR (376 MHz, DMSO-*d6)* δ -103.06 (ddd, *J* = 40.8, 31.8, 9.4 Hz, 1F), -109.23 - -109.34 (m, 2F), -109.61 (d, *J* = 9.5 Hz, 1F).

### Example 72: Preparation of compound 72

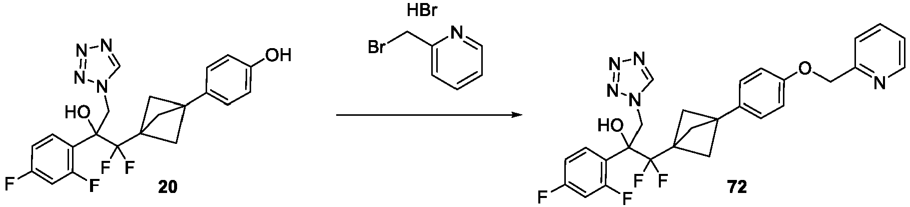

### Preparation of compound 72

Compound 20 (100 mg, 0.23 mmol) was dissolved in DMF (1.5 mL), then potassium carbonate (110 mg, 0.8 mmol) and 2-(bromomethyl)pyridine hydrobromide (90 mg, 0.35 mmol) were added thereto, and the reaction mixture was reacted at room temperature for 16 hours in a sealed tube. The reaction mixture was filtered to obtain a crude product, which was subjected to preparative purification (preparation method: mobile phase: A: 0.1% formic acid aqueous solution; B: acetonitrile; chromatographic column: Agilent 10 Prep-C18 250 × 21.2 mm; column temperature: 25°C; gradient: 40% to 60% acetonitrile in 12 min; flow rate: 30 mL/min) to obtain the title compound **72** (44 mg, yield: 36%).

Compound **72:** LC-MS: m/z 526.2 [M+H]⁺. ¹H NMR (400 MHz, DMSO-d6) δ 9.13 (s, 1H), 8.56 - 8.54 (m, 1H), 7.80 (td, *J* = 7.7, 1.8 Hz, 1H), 7.53 (td, *J* = 9.0, 6.7 Hz, 1H), 7.45 (d, *J* = 7.8 Hz, 1H), 7.35 - 7.30 (m, 1H), 7.30 - 7.22 (m, 1H), 7.17 (s, 1H), 7.08 - 7.01 (m, 2H), 6.99 (dd, *J* = 8.4, 2.6 Hz, 1H), 6.95 - 6.84 (m, 2H), 5.42 (d, *J* = 14.6 Hz, 1H), 5.13 (s, 2H), 5.01 (d, *J* = 14.6 Hz, 1H), 1.97 (dd, *J* = 9.4, 1.7 Hz, 3H), 1.75 (dd, *J* = 9.4, 1.7 Hz, 3H). ¹⁹F NMR (376 MHz, DMSO-d6) δ -103.07 (ddd, *J* = 41.8, 32.8, 9.2 Hz, 1F), -109.03 - -109.50 (m, 2F), -109.64 (d, *J* = 9.3 Hz, 1F).

### Example 73: Preparation of compound 73

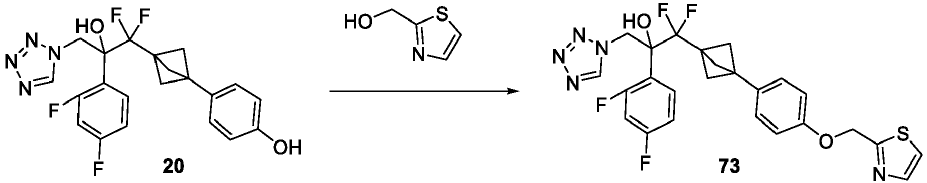

### Preparation of compound 73

To a three-necked flask were respectively added compound **20** (100 mg, 0.23 mmol), compound 1,3-thiazol-2-ylmethanol (40 mg, 0.35 mmol), and triphenylphosphine (121 mg, 0.46 mmol), and acetonitrile (5 mL) was added thereto as a solvent. Diisopropyl azodicarboxylate (93 mg, 0.46 mmol) was added thereto in an ice water bath under nitrogen atmosphere, and the reaction system was stirred and reacted at room temperature for 16 hours. The reaction mixture was filtered, and the crude filtrate was purified by preparative separation (preparation method: chromatographic column: Agilent 10 Prep-C18 250 × 21.2 mm; column temperature: 25°C; mobile phase: water (0.1% TFA)-acetonitrile; proportion of acetonitrile in the mobile phase: 50% to 70% in 12 min; flow rate: 30 mL/min) to obtain the title compound **73** (24 mg, yield: 20%, containing a pair of enantiomers).

Compound **73:** LC-MS (ESI): m/z 532.2 [M+H]⁺. ¹H NMR (400 MHz, DMSO-d6) δ 9.13 (s, 1H), 7.82 (d, *J* = 3.2 Hz, 1H), 7.75 (d, *J* = 3.2 Hz, 1H), 7.53 (td, *J* = 9.0, 6.7 Hz, 1H), 7.28 (ddd, *J* = 12.0, 9.1, 2.6 Hz, 1H), 7.15 (s, 1H), 7.09 - 6.91 (m, 5H), 5.42 (d, *J* = 14.6 Hz, 1H), 5.39 (s, 2H), 5.01 (d, *J =* 14.6 Hz, 1H), 1.98 (dd, *J* = 9.4, 1.7 Hz, 3H), 1.76 (dd, *J =* 9.4, 1.7 Hz, 3H). ¹⁹F NMR (376 MHz, DMSO-*d6*) δ -102.97 - -103.18 (m, 1F), -109.21 - -109.32 (m, 2F), -109.63 (d, *J* = 9.2 Hz, 1F).

### Example 74: Preparation of compound 74

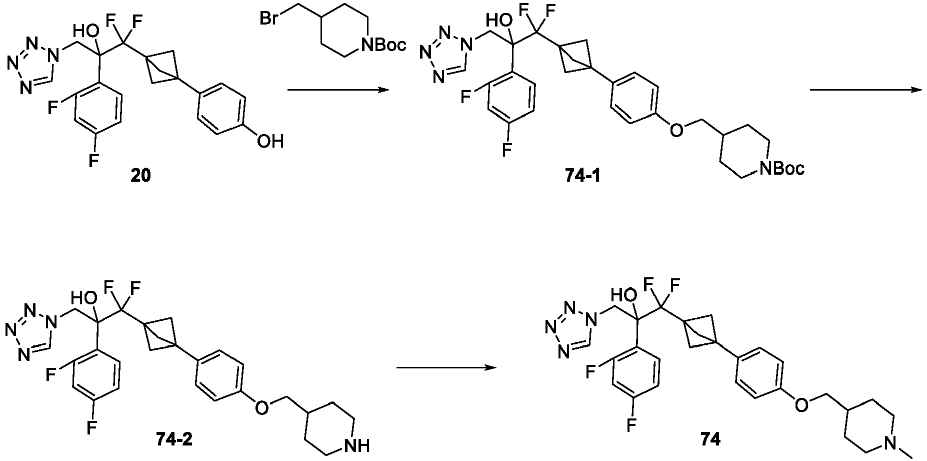

### Preparation of compound 74-1

Compound tert-butyl 4-(bromomethyl)piperidine-1-carboxylate (CAS: 158407-04-6, 53 mg, 0.19 mmol) was dissolved in DMF solution (1.5 mL), and compound **20** (70 mg, 0.16 mmol) and cesium carbonate (72 mg, 0.22 mmol) were added thereto. The reaction system was stirred for 2 hours at 60°C in a sealed tube. After the reaction system was cooled, the reaction mixture was poured into water (20 mL), then extracted with EtOAc (15 mL × 3), and the organic phases were combined and concentrated to obtain the title compound **74-1** (90 mg) as a yellow oil. The crude compound was directly used as the raw material for the next reaction step. LC-MS (ESI): m/z 632.3 [M+H]⁺.

### Preparation of compound 74-2

To a reaction flask were added the above crude compound **74-1** (90 mg) and a solution of hydrochloric acid in methanol (4.0 M, 10 mL), and the reaction mixture was stirred and reacted at room temperature for 1 hour. The reaction mixture was concentrated to obtain the title compound **74-2** (60 mg) as a yellow oil, and the crude compound was directly used as the raw material for the next reaction step. LC-MS (ESI): m/z 532.2 [M+H]⁺.

### Preparation of compound 74

The above crude compound **74-2** (60 mg) was dissolved in MeOH (3.0 mL), then formaldehyde aqueous solution (40%, 0.25 mL, 4.2 mmol) was added thereto. The mixture was stirred at room temperature for 1 hour, and then sodium cyanoborohydride (18.9 mg, 0.30 mmol) was added thereto. The reaction system was stirred and reacted at room temperature for 1 hour. After the reaction was completed, the reaction mixture was filtered, and the crude filtrate was purified by preparative separation (preparation method: mobile phase: A: 0.1% formic acid aqueous solution; B: acetonitrile; chromatographic column: Agilent 10 Prep-C18 250 × 21.2 mm; column temperature: 25°C; gradient: 25% to 45% acetonitrile in 12 min; flow rate: 30 mL/min) to obtain the title compound **74** (20.8 mg, three-step yield: 23.8%, containing a pair of enantiomers) as a white solid.

Compound **74:** LC-MS (ESI): m/z 546.2 [M+H]⁺. ¹H NMR (400 MHz, DMSO-d6) δ 9.14 (s, 1H), 7.57 - 7.55 (m, 1H), 7.31 - 7.16 (m, 1H), 7.03 (br.s, 1H), 7.01 - 6.98 (m, 3H), 7.82 - 6.79 (m, 2H), 5.43 (d, *J* = 12 Hz, 1H), 5.02 (d, *J* = 12 Hz, 1H), 3.66 - 3.75 (m, 2H), 2.83 - 2.80 (m, 2H), 2.19 (s, 3H), 1.98 - 1.93 (m, 5H), 1.77 - 1.69 (m, 6H), 1.31 - 1.28 (m, 2H). ¹⁹F NMR (376 MHz, DMSO-*d6*) δ -102.95 - -103.17 (m, 1F), -109.20 - -109.30 (m, 2F), - 109.64 - -109.67 (m, 1F).

### Example 75: Preparation of compound 75

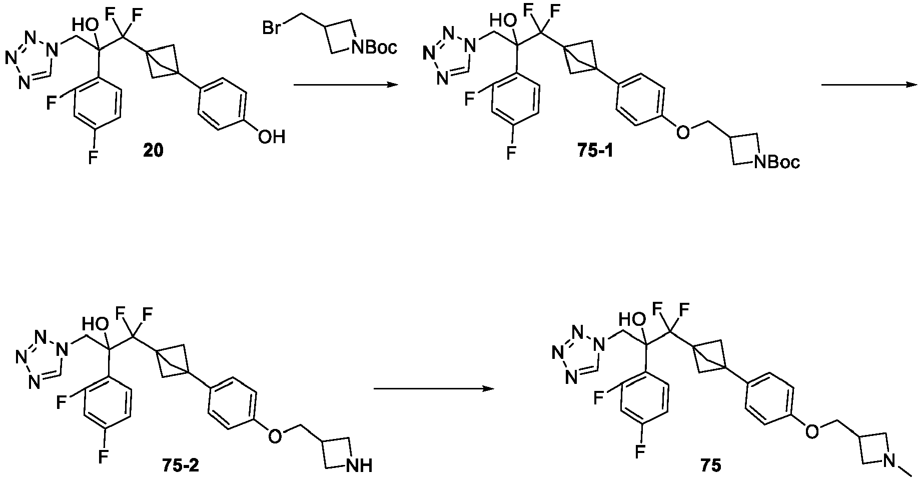

### Preparation of compound 75-1

Compound tert-butyl 3-(bromomethyl)azetidine-1-carboxylate (22.4 mg, 0.090 mmol) was dissolved in DMF solution (1.5 mL), and compound **20** (30 mg, 0.069 mmol) and cesium carbonate (72 mg, 0.22 mmol) were added thereto. The reaction system was stirred for 2 hours at 60°C in a sealed tube. After the reaction system was cooled, the reaction mixture was poured into water (20 mL), then extracted with EtOAc (15 mL × 3), and the organic phases were combined and evaporated to dryness by rotary evaporation to obtain the title compound **75-1** (33 mg, crude product) as a yellow oil. The crude compound was directly used as the raw material for the next reaction step. LC-MS (ESI): m/z 604.2 [M+H]⁺.

### Preparation of compound 75-2

To a reaction flask were added compound **75-1** (33 mg, crude product) and HCl/MeOH (4.0 M, 10 mL), and the reaction mixture was stirred and reacted at room temperature for 1 hour. The reaction mixture was concentrated to obtain the title compound **75-2** (28 mg, crude product) as a yellow oil, and the crude compound was directly used as the raw material for the next reaction step. LC-MS (ESI): m/z 504.2 [M+H]⁺.

### Preparation of compound 75

Compound **75-2** (28 mg, crude product) was dissolved in MeOH (3.0 mL), then formaldehyde aqueous solution (40%, 0.25 mL, 4.2 mmol) was added thereto. The mixture was stirred at room temperature for 1 hour, and sodium cyanoborohydride (18.9 mg, 0.30 mmol) was added thereto. The reaction system was stirred at room temperature for 1 hour. After the reaction was completed, the reaction mixture was filtered, and the crude filtrate was purified by preparative separation (preparation method: mobile phase: A: 0.1% NH₄HCO₃ aqueous solution; B: acetonitrile; chromatographic column: Agilent 10 Prep-C18 250 × 21.2 mm; column temperature: 25°C; gradient: 40% to 60% acetonitrile in 12 min; flow rate: 30 mL/min) to obtain the title compound **75** (11.49 mg, three-step yield: 23.8%, containing a pair of enantiomers) as a white solid.

Compound **75:** LC-MS (ESI): m/z 518.2 [M+H]⁺. ¹H NMR (400 MHz, DMSO-*d6*) δ 9.14 (s, 1H), 7.57 - 7.51 (m, 1H), 7.31 - 7.26 (m, 1H), 7.15 (s, 1H), 7.03 - 7.01 (m, 3H), 6.84 - 6.80 (m, 2H), 5.43 (d, *J* = 12 Hz, 1H), 5.02 (d, *J* = 12 Hz, 1H), 4.03 - 4.01 (m, 2H), 3.01 - 3.00 (m, 2 H), 2.76 - 2.73 (m, 1H), 2.24 (s, 3H), 2.03 - 1.96 (m, 3H), 1.77 - 1.74 (m, 3H), 1.24 (m, 2H). ¹⁹F NMR (376 MHz, DMSO-*d6*) δ -102.95 - -103.17 (m, 1F), -109.20 - -109.31 (m, 2F), -109.63 - -109.66 (m, 1F).

### Example 76: Preparation of compound 76

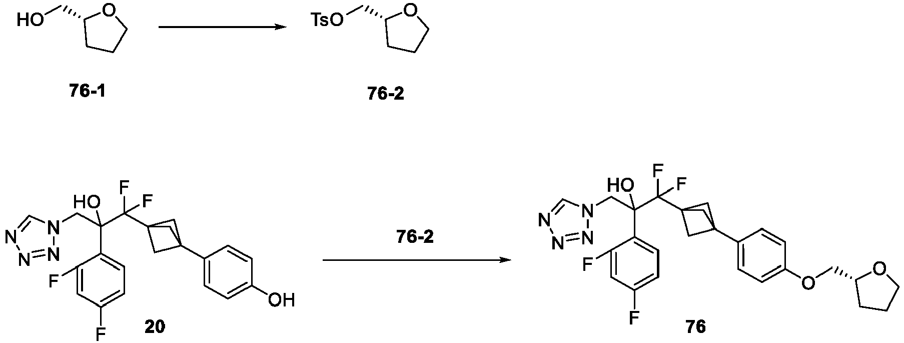

### Preparation of compound 76-2

Compound **76-1** (100 mg, 0.98 mmol) was dissolved in dichloromethane (5 mL), then triethylamine (198 mg, 1.96 mmol) was added dropwise thereto at 0°C. After the reaction was carried out for 10 min, p-toluenesulfonyl chloride (276 mg, 1.47 mmol) was added thereto, reacted at room temperature for 4 hours. The reaction was tracked by TLC until the reaction was completed, then the reaction system was extracted with DCM (20 mL × 3), and the organic phases were combined, dried, and concentrated to obtain the crude title compound (155 mg), which was directly used as the raw material for the next reaction step.

### Preparation of compound 76

Compound **20** (50 mg, 0.12 mmol) was dissolved in DMF (5 mL), then cesium carbonate (80 mg, 0.24 mmol) was added thereto with stirring. After the reaction was carried out for 5 min, the reaction mixture was then added with compound **76-2** (42 mg, 0.18 mmol), stirred and reacted at 50°C for 4 hours. The reaction mixture was filtered, and the crude filtrate was purified by preparative separation (preparation method: chromatographic column: Agilent 10 Prep-C18 250 × 21.2 mm; column temperature: 25°C; mobile phase: water (0.1% NH₄HCO₃)-acetonitrile; proportion of acetonitrile in the mobile phase: 50% to 70% in 12 min; flow rate: 30 mL/min) to obtain the title compound **76** (5.6 mg, yield: 5%, containing a pair of diastereoisomers).

Compound **76:** LC-MS (ESI): m/z 519.2 [M+H]⁺. ¹H NMR (400 MHz, DMSO-d6) δ 9.13 (s, 1H), 7.60 - 7.48 (m, 1H), 7.34 - 7.18 (m, 1H), 7.15 (s, 1H), 7.06 - 6.97 (m, 3H), 6.82 (d, *J* = 8.4 Hz, 2H), 5.43 (d, *J* = 14.5 Hz, 1H), 5.01 (d, *J* = 14.5 Hz, 1H), 4.16 - 4.05 (m, 1H), 3.94 - 3.80 (m, 2H), 3.80 - 3.71 (m, 1H), 3.71 - 3.60 (m, 1H), 2.02 - 1.94 (m, 4H), 1.91 - 1.79 (m, 2H), 1.79-1.72 (m, 3H), 1.69 - 1.56 (m, 1H). ¹⁹F NMR (376 MHz, DMSO-d6) δ -102.96 - -103.17 (m, 1F), -109.26 (m, 2F), -109.63 (m, 1F).

### Example 77: Preparation of compound 77

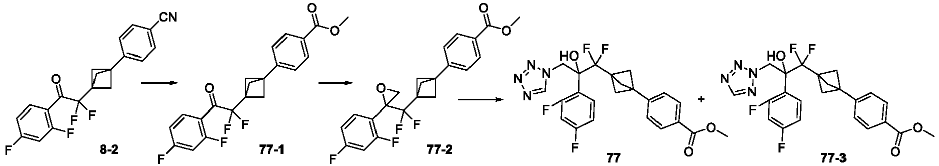

### Preparation of compound 77-1

To a single-necked flask were added compound **8-2** (650 mg, 1.81 mol) and a solution of hydrochloric acid in methanol (4 M, 10 mL), and the reaction mixture was heated to 50°C and reacted for 16 hours. The reaction system was extracted with DCM (20 mL × 3), and the organic phases were combined, dried, and concentrated to obtain the title compound **77-1** (400 mg, crude product), and the crude compound was directly used as the raw material for the next reaction step.

### Preparation of compound 77-2

Compound **77-1** (400 mg, 1.02 mmol) was dissolved in a mixture of dichloromethane (5.0 mL) and water (2.0 mL), then trimethylsulfoxonium iodide (675 mg, 3.06 mmol) and solid sodium hydroxide (122 mg, 3.06 mmol) were added thereto, and the reaction system was stirred at 65°C for 24 hours. After the reaction was completed, the reaction system was extracted with dichloromethane (20 mL × 3), and the organic phases were combined, dried, and concentrated to obtain a crude product. The crude product was purified by normal-phase silica gel column chromatography (EtOAc/PE = 0 to 5%) to obtain the title compound 77-2 (320 mg, yield: 78%). LC-MS (ESI): m/z 407.0 [M+H]⁺.

### Preparation of compound 77

Compound **77-2** (310 mg, 0.76 mmol) was dissolved in DMF (2.5 mL), then 1H-tetrazole (300 mg, 3.81 mmol) and potassium carbonate (1.24 g, 3.81 mmol) were added thereto, and the reaction mixture was reacted at 80°C for 16 hours in a sealed tube. After the reaction system was cooled, the reaction mixture was filtered, and the crude filtrate was concentrated to obtain 300 mg of a crude product. 150 mg of the crude product was purified by preparative separation (preparation method: chromatographic column: Agilent 10 Prep-C18 250 × 21.2 mm; mobile phase: A: 0.1% formic acid aqueous solution; B: acetonitrile; column temperature: 25°C; gradient: 60% to 80% acetonitrile in 12 min; flow rate: 30 mL/min) to obtain the title compound **77** (38.6 mg, yield: 20%, containing a pair of enantiomers) and the corresponding regioisomer compound **77-3** (38.4 mg, yield: 20%, containing a pair of enantiomers).

Compound **77:** LC-MS (ESI): m/z 477.2 [M+H]⁺. ¹H NMR (400 MHz, DMSO-^6) δ 9.14 (m, 1H), 7.91 - 7.81 (m, 2H), 7.62 - 7.48 (m, 1H), 7.34 - 7.17 (m, 4H), 7.08 - 6.94 (m, 1 H), 5.43 (d, *J* = 14.5 Hz, 1H), 5.02 (d, *J* = 14.5 Hz, 1H), 3.82 (s, 3H), 2.13 - 1.98 (m, 3H), 1.94 - 1.73 (m, 3H). ¹⁹F NMR (376 MHz, DMSO-*d6*) δ -102.69 - -103.26 (m, 1F), -109.17 - -109.81 (m, 3F).

Compound **77-3:** LC-MS (ESI): m/z 477.2 [M+H]⁺. ¹H NMR (400 MHz, DMSO-*d6)* δ 8.77 (s, 1H), 7.89 - 7.83 (m, 2H), 7.62 - 7.53 (m, 1H), 7.32 - 7.22 (m, 4H), 7.04 - 6.96 (m, 1H), 5.61 (d, *J* = 14.1 Hz, 1H), 5.25 (d, *J* = 14.2 Hz, 1H), 3.82 (s, 3H), 2.10 - 2.00 (m, 3H), 1.89 - 1.76 (m, 3H). ¹⁹F NMR (376 MHz, DMSO-d6) δ -101.79 - -103.59 (m, 1F), -108.35 - -109.59 (m, 2F), -109.89 (m, 1F).

### Example 78: Preparation of compound 78

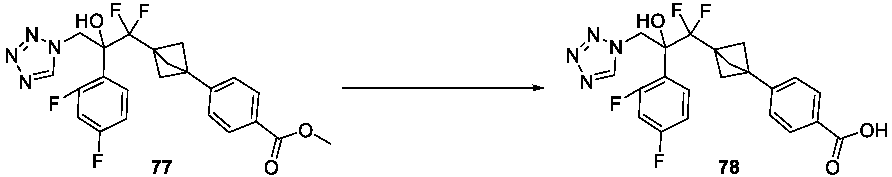

### Preparation of compound 78

Compound **77** (30 mg, 0.06 mmol) was dissolved in THF (2 mL), then sodium hydroxide aqueous solution (1.0 N, 0.3 mL, 0.3 mmol) was added thereto at 0°C, and the reaction system was reacted at 25°C for 2 hours. The reaction mixture was filtered, and the crude filtrate was purified by preparative separation (chromatographic column: Agilent 10 Prep-C18 250 × 21.2 mm; column temperature: 25°C; mobile phase: water (0.1% FA)-acetonitrile; proportion of acetonitrile in the mobile phase: 50% to 70% in 12 min; flow rate: 30 mL/min) to obtain the title compound **78** (17 mg, yield: 58%, containing a pair of enantiomers).

Compound **78:** LC-MS (ESI): m/z 463.0 [M+H]⁺. ¹H NMR (400 MHz, DMSO-^6) δ 12.91 (br.s, 1H), 9.14 (s, 1H), 7.91 - 7.78 (m, 2H), 7.62 - 7.49 (m, 1H), 7.34 - 7.22 (m, 3H), 7.19 (s, 1H), 7.07 - 6.96 (m, 1H), 5.43 (d, *J =* 14.5 Hz, 1H), 5.02 (d, *J* = 14.6 Hz, 1H), 2.12 - 2.01 (m, 3H), 1.91 - 1.78 (m, 3H). ¹⁹F NMR (376 MHz, DMSO-d6) δ -102.39 - -104.01 (m, 1F), -108.86 - -109.97 (m, 3F).

### Example 79: Preparation of compound 79

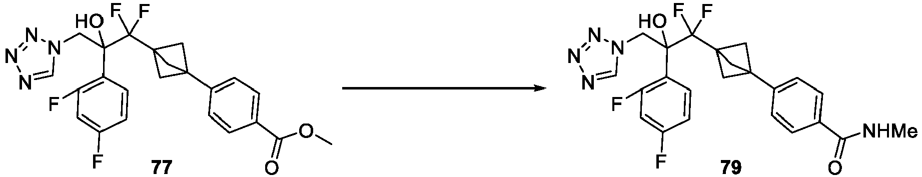

### Preparation of compound 79

Compound **77** (150 mg, 0.32 mmol) was dissolved in a solution of methylamine in ethanol (3.0 mL), and the reaction system was reacted at 50°C for 2 hours. The reaction mixture was filtered, and the crude filtrate was purified by preparative separation (chromatographic column: Agilent 10 Prep-C18 250 × 21.2 mm; column temperature: 25°C; mobile phase: water (0.1% FA)-acetonitrile; proportion of acetonitrile in the mobile phase: 50% to 70% in 12 min; flow rate: 30 mL/min) to obtain the title compound **79** (70 mg, yield: 46%, containing a pair of enantiomers).

Compound **79:** LC-MS (ESI): m/z 476.0 [M+H]⁺. ¹H NMR (400 MHz, DMSO-d6) δ 9.13 (s, 1H), 8.46 - 8.31 (m, 1H), 7.77 - 7.69 (m, 2H), 7.59 - 7.49 (m, 1H), 7.36 - 7.25 (m, 1H), 7.23 - 7.15 (m, 3H), 7.06 - 6.96 (m, 1H), 5.43 (d, *J* = 14.6 Hz, 1H), 5.02 (d, *J* = 14.6 Hz, 1H), 2.75 (d, *J* = 4.5 Hz, 3H), 2.07 - 2.01 (m, 3H), 1.87 - 1.80 (m, 3H). ¹⁹F NMR (376 MHz, DMSO-d6) δ -102.01 - -103.94 (m, 1F), -109.30 - -109.60 (m, 3F).

### Example 80: Preparation of compound 80

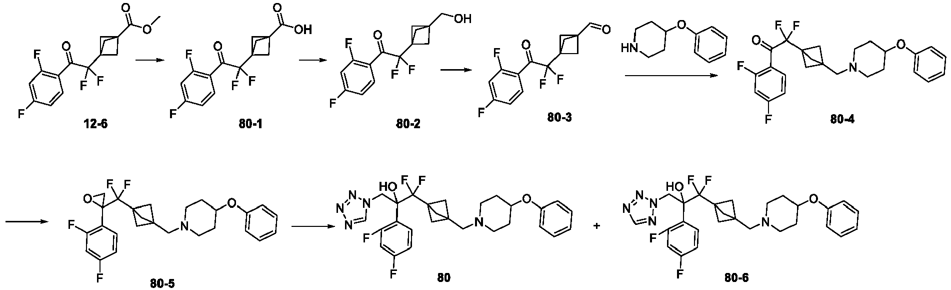

### Preparation of compound 80-1

To a single-necked reaction flask was added compound **12-6** (2.6 g, 8.2 mmol), then solvent THF (30 mL) and H₂O (10 mL) were sequentially added thereto, and then the reaction system was slowly added with lithium hydroxide (394 mg, 16.4 mmol), reacted at room temperature for 16 hours. After the reaction was completed, the pH of the reaction system was adjusted to between 5 and 6 with hydrochloric acid aqueous solution (1.0 M). The reaction mixture was extracted with EtOAc (60 mL × 3), dried over anhydrous sodium sulfate, concentrated, and subjected to normal-phase column chromatography (petroleum ether/ethyl acetate = 0 to 100%) to obtain the title compound **80-1** (1.62 g, yield: 65%) as a yellow solid. LC-MS (ESI): m/z 303.1 [M+H]⁺.

### Preparation of compound 80-2

To a three-necked reaction flask was added compound **80-1** (1.6 g, 5.4 mmol), and the reaction system was replaced with nitrogen. To the reaction flask was added anhydrous THF (20 mL), then the reaction system was cooled to 0°C, slowly added dropwise with a solution of borane in tetrahydrofuran (1.0 M, 6.5 mL, 6.4 mmol), and stirred and reacted for 2 hours. Then the reaction system was added with methanol (5.0 mL) to quench the reaction, extracted with EtOAc (60 mL × 3), dried over anhydrous sodium sulfate, concentrated, and then subjected to normal-phase column chromatography (petroleum ether/ethyl acetate = 0 to 100%) to obtain the title compound **80-2** (600 mg, yield: 40%) as a yellow oil. LC-MS (ESI): m/z 289. 0 [M+H]⁺.

### Preparation of compound 80-3

To a single-necked reaction flask were sequentially added compound **80-2** (200 mg, 0.7 mmol) and solvent DCM (5.0 mL). The reaction system was cooled to 0°C, then Dess-Martin periodinane (CAS: 87413-09-0, 587 mg, 1.4 mmol) was slowly added thereto, and the reaction mixture was reacted at room temperature for 5 hours. The reaction mixture was extracted with DCM (50 mL × 3), dried, filtered, and concentrated to obtain the title compound **80-3** (230 mg, crude product). The crude compound was directly used as the raw material for the next reaction step without purification.

### Preparation of compound 80-4

To a single-necked reaction flask were sequentially added the above crude compound **80-3** (230 mg), solvent DCM (5.0 mL), and compound 4-phenoxypiperidine (142 mg, 0.8 mmol), and then one drop of acetic acid was dropped into the reaction system and stirred for 10 min. The reaction system was cooled to 0°C, then sodium triacetoxyborohydride (169 mg, 0.8 mmol) was slowly added thereto, and the reaction mixture was reacted at room temperature for 3 hours. The reaction system was extracted with DCM (50 mL × 3), dried, concentrated, and then subjected to normal-phase column chromatography (petroleum ether/ethyl acetate = 0 to 100%) to obtain the title compound **80-4** (90 mg) as a pale yellow oil. LC-MS (ESI): m/z 448.2 [M+H]⁺.

### Preparation of compound 80-5

To a single-necked reaction flask were sequentially added potassium tert-butoxide (27 mg, 0.24 mmol), trimethylsulfoxonium iodide (48 mg, 0.22 mmol), DMSO (2.0 mL), and THF (3.0 mL) at room temperature, and the reaction mixture was stirred at room temperature for 1 hour. Then compound **80-4** (90 mg, 0.2 mmol) was slowly added to the reaction system, and the reaction mixture was stirred and reacted for another 2 hours. The reaction system was extracted with EtOAc (50 mL × 3), dried, concentrated, and then subjected to normal-phase column chromatography (petroleum ether/ethyl acetate = 0 to 100%) to obtain the title compound **80-5** (65 mg, yield: 70%) as a pale yellow solid. LC-MS (ESI): m/z 462.2 [M+H]⁺

### Preparation of compound 80

To a microwave reaction tube were sequentially added compound **80-5** (65 mg, 0.14 mmol), 1H-tetrazole (20 mg, 0.28 mmol), potassium carbonate (30 mg, 0.2 mmol), and solvent DMF (1.0 mL). The reaction system was reacted at 80°C under microwave irradiation for 16 hours. The reaction system was extracted with EtOAc (50 mL × 3), dried, and concentrated. The concentrate was prepared by a reversed-phase C18 chromatographic column to obtain the title compound **80** (39.81 mg, yield: 53%, containing a pair of enantiomers) and regioisomer compound **80-6** (16.51 mg, yield: 22%, containing a pair of enantiomers).

Compound **80:** LC-MS: m/z 532.1 [M+H]⁺. ¹H NMR (400 MHz, DMSO-d6) δ 9.11 (s, 1H), 7.55 - 7.46 (m, 1H), 7.30 (dd, *J* = 13.9, 8.5 Hz, 3H), 7.16 (s, 1H), 6.98 (m, 4H), 5.38 (d, *J* = 14.1 Hz, 1H), 4.98 (d, *J* = 14.5 Hz, 1H), 4.60 (m, 1H), 3.27 - 2.98 (m, 6H), 2.20 (d, *J* = 11.0 Hz, 1H), 1.99 (s, 2H), 1.88 (m, 3H), 1.67 (m, 4H).

Compound **80-6:** LC-MS: m/z 532.1 [M+H]⁺. ¹H NMR (400 MHz, DMSO-d6) δ 8.76 (s, 1H), 7.53 (dd, *J* = 15.5, 8.7 Hz, 1H), 7.34 - 7.24 (m, 3H), 7.21 (s, 1H), 6.98 (t, *J* = 7.9 Hz, 4H), 5.57 (d, *J* = 14.4 Hz, 1H), 5.21 (d, *J* = 14.4 Hz, 1H), 4.61 (m, 1H), 3.43 (s, 1H), 3.27 - 2.97 (m, 5H), 2.20 (d, *J* = 13.4 Hz, 1H), 1.99 (s, 2H), 1.87 (m, 3H), 1.68 (m, 4H).

### Example 81: Preparation of compound 81

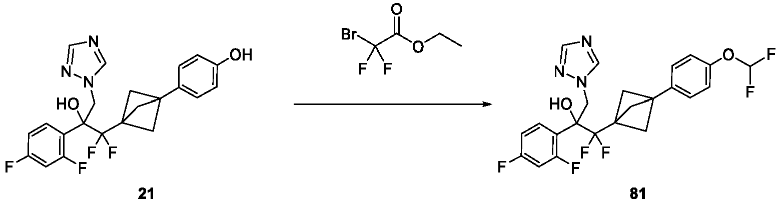

### Preparation of compound 81

Compound **21** (60 mg, 0.14 mmol) was dissolved in DMF solution (1.5 mL), then compound ethyl 2-bromo-2,2-difluoroacetate (36.9 mg, 0.18 mmol) and cesium carbonate (137 mg, 0.42 mmol) were respectively added thereto. The reaction system was reacted at 50°C for 16 hours. After the reaction system was cooled, the reaction mixture was filtered, and the crude filtrate was purified by preparative separation (preparation method: mobile phase: A: 0.1% formic acid aqueous solution; B: acetonitrile; chromatographic column: Agilent 10 Prep-C18 250 × 21.2 mm; column temperature: 25°C; gradient: 55% to 75% acetonitrile in 12 min; flow rate: 30 mL/min) to obtain the title compound **81** (5.41 mg, yield: 8.0%, containing a pair of enantiomers).

Compound **81:** LC-MS (ESI): m/z 484.0 [M+H]⁺. ¹H NMR (400 MHz, Chloroform-d) δ 8.39 (br.s, 1H), 7.86 (s, 1H), 7.73 (m, 1H), 7.16 - 7.06 (m, 2H), 7.02 (m, 2H), 6.86 - 6.75 (m, 2H), 6.45 (t, *J* = 72 Hz, 1H), 5.37 (br. s, 1H), 5.23 (d, *J* = 14.0 Hz, 1H), 4.89 (d, *J* = 14.0 Hz, 1H), 2.08 (dd, *J* = 9.4, 1.8 Hz, 3H), 1.92 (dd, *J* = 9.4, 1.8 Hz, 3H). ¹⁹F NMR (376 MHz, Chloroform-*d*) δ -80.74 (m, 2F), -104.89 - -105.02 (m, 1F), -108.22 - -110.19 (m, 2F), -110.77 - -111.73 (m, 1F).

### Example 82: Preparation of compound 82

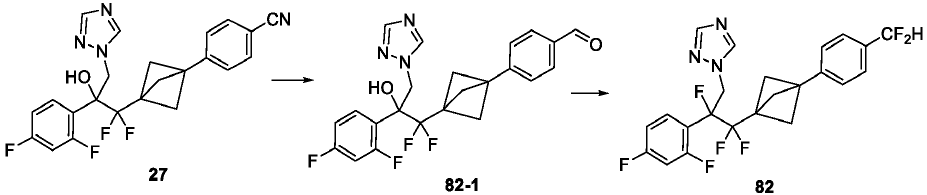

### Preparation of compound 82-1

Compound **27** (50 mg, 0.11 mmol) was dissolved in toluene (5 mL) solvent, then diisobutylaluminum hydride (DIBAL-H, 1.0 M solution in Hexanes, 0.15 mL, 0.15 mmol) was added dropwise to the reaction system at -40°C under nitrogen atmosphere, and the reaction mixture was reacted at room temperature for 2 hours. The reaction was quenched with saturated ammonium chloride aqueous solution (10 mL), extracted with EtOAc (60 mL × 3), and the organic phases were combined, dried, and concentrated to obtain the title compound **82-1** (50 mg, crude product). The crude compound was directly used as the raw material for the next reaction step without purification. LC-MS (ESI): m/z 446.2 [M+H]⁺.

### Preparation of compound 82

Compound **82-1** (50 mg, 0.11 mmol) was dissolved in dichloromethane (5 mL), and diethylaminosulfur trifluoride (DAST, 36 mg, 0.22 mmol) was added dropwise to the reaction system under nitrogen atmosphere. The reaction system was kept at 25°C and reacted for 4 hours. The reaction was quenched by adding ice water (5 mL), extracted with EtOAc (20 mL × 3), and the organic phases were combined, dried, and concentrated to obtain a crude product. The crude product was purified by preparative separation (preparation method: chromatographic column: Agilent 10 Prep-C18 250 × 21.2 mm; column temperature: 25°C; mobile phase: water (0.1% TFA)-acetonitrile; proportion of acetonitrile in the mobile phase: 55% to 75% in 12 min; flow rate: 30 mL/min) to obtain the title compound **82** (3 mg, yield: 6%, containing a pair of enantiomers).

**Compound 82:** LC-MS (ESI): m/z 470.0 [M+H]⁺. ¹H NMR (400 MHz, Chloroform-*d*) δ 8.04 (s, 1H), 7.72 (s, 1H), 7.42 (d, *J* = 7.8 Hz, 2H), 7.34 (td, *J* = 8.6, 6.2 Hz, 1H), 7.19 (d, *J* = 7.8 Hz, 2H), 6.93 - 6.81 (m, 2H), 6.60 (t, *J* = 56.4 Hz, 1H), 5.29 (dd, *J* = 36.4, 15.2 Hz, 1H), 4.99 (t, *J* = 16.0 Hz, 1H), 2.14 (dd, *J* = 9.4, 1.8 Hz, 3H), 1.98 (dd, *J* = 9.4, 1.9 Hz, 3H). ¹⁹F NMR (376 MHz, Chloroform-*d*) δ -104.30 (d, *J* = 48.9 Hz, 2F), -106.53 (d, *J* = 11.3 Hz, 1F), -110.39 (s, 2F), -111.17 (d, *J* = 48.9 Hz, 1F), -111.57 (m, 1F).

### Example 83: Preparation of compound 83

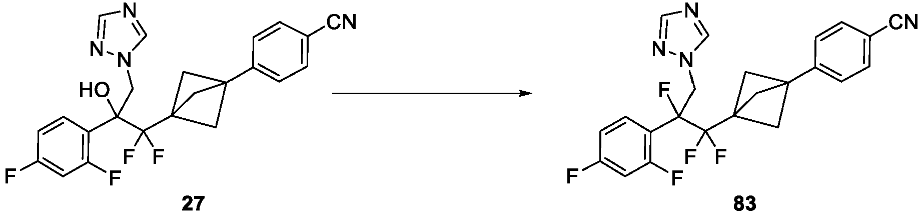

Compound **27** (49 mg, 0.11 mmol) was dissolved in dichloromethane (5 mL), and diethylaminosulfur trifluoride (DAST, 36 mg, 0.22 mmol) was added dropwise to the reaction system under nitrogen atmosphere. The reaction system was kept at 25°C and reacted for 4 hours. The reaction was quenched by adding ice water (5 mL), extracted with EtOAc (20 mL × 3), and the organic phases were combined, dried, and concentrated to obtain a crude product. The crude product was purified by preparative separation (preparation method: chromatographic column: Agilent 10 Prep-C18 250 × 21.2 mm; column temperature: 25°C; mobile phase: water (0.1% TFA)-acetonitrile; proportion of acetonitrile in the mobile phase: 55% to 75% in 12 min; flow rate: 30 mL/min) to obtain the title compound **83** (3 mg, yield: 6%, containing a pair of enantiomers).

**Compound** 83: LC-MS (ESI): m/z 445.0 [M+H]⁺. ¹H NMR (400 MHz, DMSO-d6) δ 8.48 (d, *J* = 1.2 Hz, 1H), 7.78 (dd, *J* = 6.8, 1.6 Hz, 3H), 7.51 - 7.28 (m, 4H), 7.07 (td, *J* = 8.5, 2.6 Hz, 1H), 5.35 - 5.09 (m, 2H), 2.21 (dd, *J* = 9.4, 1.8 Hz, 3H), 2.05 (dd, J = 9.4, 1.8 Hz, 3H). ¹⁹F NMR (376 MHz, Chloroform-*d*) δ -104.26 (br.s, 2F), -107.99 (d, *J* = 11.3 Hz, 1F), -111.18 (d, *J* = 41.4 Hz, 1F), -110.86 (m, 1F).

### Example 84: Preparation of compound 84

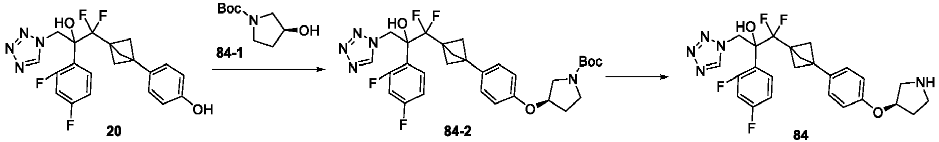

### Preparation of compound 84-2

To a three-necked reaction flask were sequentially added compound 20 (100 mg, 0.23 mmol), compound 84-1 (65 mg, 0.35 mmol), and triphenylphosphine (121 mg, 0.46 mmol), and then tetrahydrofuran (5 mL) was added thereto as a solvent. Diisopropyl azodicarboxylate (93 mg, 0.46 mmol) was added dropwise to the reaction system in an ice-water bath under nitrogen atmosphere, and the reaction was carried out at room temperature for 16 hours. The reaction mixture was poured into water, extracted with EtOAc (20 mL × 3), and the organic phases were combined, dried, and concentrated to obtain the title compound 84-2 (80 mg, crude product), which was directly used as the raw material for the next reaction step without purification. LC-MS (ESI): m/z 604.2 [M+H]⁺.

### Preparation of compound 84

Compound **84-2** (80 mg, 0.13 mmol) was dissolved in methanol (5 mL) solvent, and a solution of hydrochloric acid in methanol (4 N, 0.5 mL) was added thereto. The reaction system was stirred and reacted at room temperature for 16 hours. The reaction mixture was filtered, and the crude filtrate was purified by preparative separation (preparation method: chromatographic column: Agilent 10 Prep-C18 250 × 21.2 mm; column temperature: 25°C; mobile phase: water (0.1% NH₄HCO₃)-acetonitrile; proportion of acetonitrile in the mobile phase: 55% to 75% in 12 min; flow rate: 30 mL/min) to obtain the title compound **84** (20 mg, yield: 30%, containing a pair of diastereoisomers).

Compound **84:** LC-MS (ESI): m/z 504.0 [M+H]⁺. ¹H NMR (400 MHz, DMSO-*d6*) δ 9.13 (s, 1H), 7.54 (td, *J* = 9.0, 6.7 Hz, 1H), 7.27 (ddd, *J* = 12.1, 9.1, 2.6 Hz, 1H), 7.13 (s, 1H), 7.06 - 6.95 (m, 3H), 6.82 - 6.72 (m, 2H), 5.42 (d, *J* = 14.6 Hz, 1H), 5.01 (d, *J* = 14.6 Hz, 1H), 4.83 - 4.71 (m, 1H), 3.07 - 2.69 (m, 4H), 2.05 - 1.90 (m, 5H), 1.82 - 1.71 (m, 3H), 1.69 - 1.63 (m, 1H). ¹⁹F NMR (376 MHz, DMSO-d6) δ -102.95 - -103.17 (m, 1F), -109.15 - -109.26 (m, 2F), -109.66 (d, *J* = 9.2 Hz, 1F).

### Example 85: Preparation of compound 85

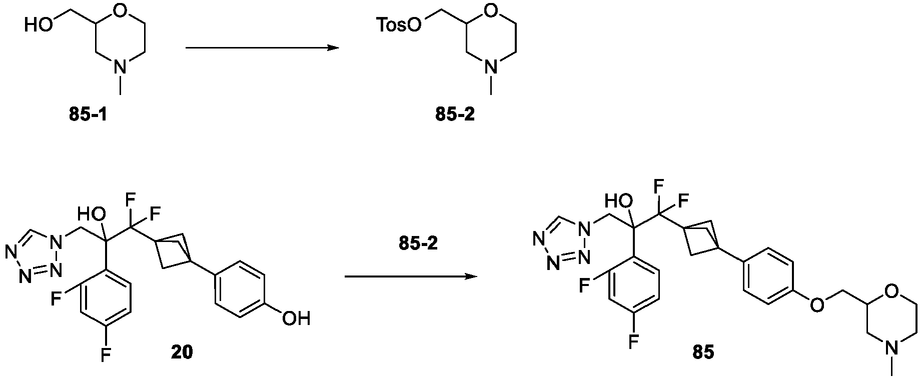

### Preparation of compound 85-2

Compound **85-1** (120 mg, 0.915 mmol) was dissolved in dichloromethane (10 mL), then the reaction system was added dropwise with triethylamine (111 mg, 1.10 mmol) at 0°C, stirred and reacted for 10 min, then added with p-toluenesulfonyl chloride (209 mg, 1.1 mmol), reacted at room temperature for 24 hours. The reaction system was extracted with DCM (20 mL × 3), and the organic phases were combined, dried, and concentrated to obtain a crude product, which was filtered through silica gel to obtain the title compound **85-2** (150 mg, yield: 57%). LC-MS (ESI): m/z 286.2 [M+H]⁺.

### Preparation of compound 85

Compound **20** (100 mg, 0.230 mmol) was dissolved in DMF (2 mL) solvent, then cesium carbonate (150 mg, 0.46 mmol) was added thereto with stirring. After the reaction was carried out for 5 min, the reaction mixture was then added with above crude compound **85-2** (98 mg, 0.345 mmol). The reaction system was stirred and reacted at 50°C for 24 hours. The reaction mixture was filtered and the filtrate was concentrated to obtain a crude product. The crude product was purified by preparative separation (preparation method: chromatographic column: Agilent 10 Prep-C18 250 × 21.2 mm; column temperature: 25°C; mobile phase: water (0.1% FA)-acetonitrile; proportion of acetonitrile in the mobile phase: 55% to 75% in 12 min; flow rate: 30 mL/min) to obtain the title compound **85** (15 mg, yield: 12%, containing two pairs of enantiomers).

Compound **85:** LC-MS (ESI): m/z 548.2 [M+H]⁺. ¹H NMR (400 MHz, DMSO-d6) δ 9.12 (s, 1H), 7.57 - 7.51 (m, 1H), 7.29 - 7.23 (m, 1H), 7.11 (s, 1H), 7.06 - 6.96 (m, 3H), 6.83 - 6.81 (m, 2H), 5.42 (d, *J* = 14.6 Hz, 1H), 5.01 (d, *J* = 14.6 Hz, 1H), 3.92 - 3.85 (m, 2H), 3.81 - 3.69 (m, 2H), 3.54 - 3.48 (m, 1H), 2.75 - 2.72 (m, 1H), 2.60 - 2.50 (m, 1H), 2.17 (s, 3H), 2.04 - 1.92 (m, 4H), 1.87 - 1.69 (m, 4H). ¹⁹F NMR (376 MHz, DMSO-*d6*) δ -102.93 - - 103.14 (m, 1F), -109.10 - -109.21 (m, 2F), -109.64 - -119.67 (d, *J* = 9.4 Hz, 1F).

### Example 86: Preparation of compound 86

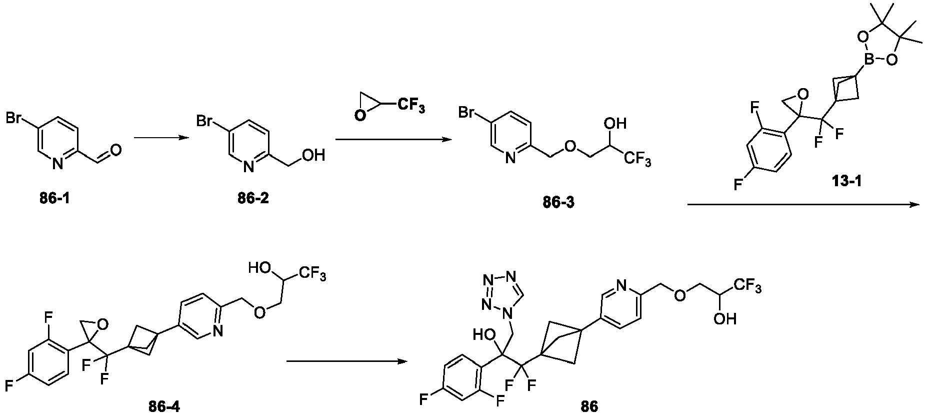

### Preparation of compound 86-2

Compound **86-1** (3 g, 16.1 mmol) was dissolved in methanol (15 mL) solvent, and the reaction system was added with sodium borohydride (1.5 g, 40.3 mmol) at 0°C, and stirred at 0°C for 30 min, then transferred to room temperature and reacted for another 4 hours. The reaction mixture was extracted with EtOAc (20 mL × 3), and the organic phases were combined, dried, and concentrated to obtain a crude product. The crude product was purified by normal-phase silica gel column chromatography (EtOAc/PE = 30 to 100%) to obtain the title compound **86-2** (3 g, yield: 98%) as a white solid.

### Preparation of compound 86-3

Compound **86-2** (0.5 g, 2.7 mmol) was dissolved in DMF (10 mL) solvent. After the reaction system was added with NaH (210 mg, 5.3 mmol) at 0°C and stirred for 15 min, 3,3,3-trifluoro-1,2-epoxypropane (600 mg, 5.3 mmol) was added thereto, and then the reaction system was transferred to room temperature, stirred and reacted for another 17 hours. The reaction mixture was extracted with EtOAc (20 mL × 3), and the organic phases were combined, dried, and concentrated to obtain a crude product. The crude product was purified by normal-phase silica gel column chromatography (EtOAc/PE = 0 to 20%) to obtain the title compound **86-3** (600 mg, yield: 74%) as a white solid. LC-MS (ESI): m/z: 300.2 [M+H]⁺.

### Preparation of compound 86-4

Compound **86-3** (563 mg, 1.88 mmol) was added to a microwave reaction tube and dissolved in a mixed solvent of H₂O (0.5 mL) and cyclopentyl methyl ether (2 mL), and then compound **13-1** (500 mg, 1.26 mmol), CataCxium A (90 mg, 0.25 mmol), Cs₂CO₃ (822 mg, 2.52 mmol), Cu₂O (180 mg, 1.26 mmol), and palladium acetate (28 mg, 0.13 mmol) were sequentially added thereto. The reaction system was replaced with nitrogen three times, reacted at 120°C for 17 hours in the sealed tube. The reaction mixture was extracted with EtOAc (20 mL × 3), and the organic phases were combined, dried, and concentrated to obtain a crude product. The crude product was purified by normal-phase silica gel column chromatography (EtOAc/PE = 0 to 70%) to obtain the title compound **86-4** (200 mg, yield: 32%) as a white solid. LC-MS (ESI): m/z: 492.0 [M+H]⁺.

### Preparation of compound 86

Compound **86-4** (200 mg, 0.4 mmol) was added to a microwave tube and dissolved in DMF (3 mL), then potassium carbonate (112 mg, 0.8 mmol) and tetrazole (84 mg, 1.2 mmol) were sequentially added thereto, and the reaction mixture was reacted at 75°C for 16 hours in a sealed tube. After cooling, the reaction system was filtered, and the filtrate was purified by preparative separation (preparation method: chromatographic column: Welch Xtimate@ C18 21.2 × 250 mm; column temperature: 25°C; mobile phase: water (10 mM/L NH₄HCO₃)-acetonitrile; proportion of acetonitrile in the mobile phase: 35% to 55% in 12 min; flow rate: 30 mL/min) to obtain the title compound **86** (42 mg, yield: 19%). LC-MS (ESI): m/z: 562.0 [M+H]⁺. ¹H NMR (400 MHz, Chloroform-*d*) δ 8.57 (s, 1H), 8.36 (d, *J* = 2.0 Hz, 1H), 7.65 (td, *J* = 8.9, 6.3 Hz, 1H), 7.54 (t, *J* = 7.5 Hz, 1H), 7.23 (s, 1H), 6.95 - 6.79 (m, 2H), 5.44 (d, *J* = 14.6 Hz, 1H), 5.07 (d, *J* = 14.5 Hz, 1H), 4.78 (d, *J* = 1.7 Hz, 2H), 4.23 (td, *J* = 7.2, 2.9 Hz, 1H), 4.10 (s, 1H), 3.97 (dd, *J* = 10.8, 2.8 Hz, 1H), 3.81 (dd, *J* = 10.8, 7.1 Hz, 1H), 2.14 (dd, *J* = 9.6, 2.0 Hz, 3H), 1.95 (dd, *J* = 9.4, 2.0 Hz, 3H). ¹⁹F NMR (376 MHz, Chloroform-*d*) δ - 77.36 (s, 3F), -105.18 (d, 1F), -106.49 (d, *J* = 9.7 Hz, 2F), -110.12 - -110.81 (m, 1F).

### Example 87: Preparation of compound 87

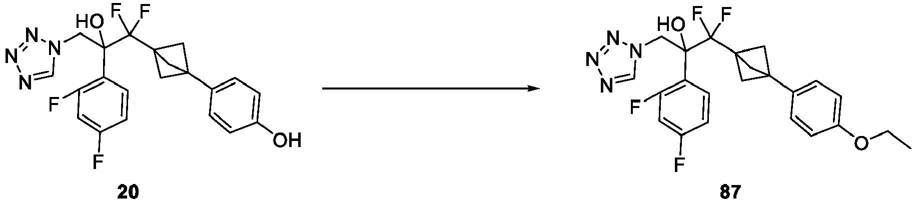

### Preparation of compound 87

Compound **20** (100 mg, 0.23 mmol) was dissolved in THF (1 mL), then NaH (9 mg, 0.23 mmol) was added thereto at 0°C. After the reaction was carried out for 30 min, EtOTf (41 mg, 0.23 mmol) was added thereto, and the reaction system was reacted at room temperature for 16 hours. The reaction mixture was filtered, and the crude filtrate was purified by preparative separation (chromatographic column: Agilent 10 Prep-C18 250 × 21.2 mm; column temperature: 25°C; mobile phase: water (0.1% FA)-acetonitrile; proportion of acetonitrile in the mobile phase: 55% to 75% in 12 min; flow rate: 30 mL/min) to obtain the title compound **87** (20 mg, yield: 20%).

LC-MS (ESI): m/z 463.0 [M+H]⁺. ¹H NMR (400 MHz, Chloroform-*d*) δ 8.53 (s, 1H), 7.69 - 7.58 (m, 1H), 7.04 - 6.96 (m, 2H), 6.91 - 6.75 (m, 4H), 5.45 (d, *J* = 14.6 Hz, 1H), 5.04 (d, *J* = 14.6 Hz, 1H), 3.98 (q, *J* = 7.0 Hz, 2H), 3.90 (s, 1H), 2.03 (dd, *J* = 9.4, 1.8 Hz, 3H), 1.84 (dd, *J* = 9.4, 1.8 Hz, 3H), 1.38 (t, *J* = 7.0 Hz, 3H). ¹⁹F NMR (376 MHz, Chloroform-d) δ -107.02 (d, 2F), -109.84 - -110.57 (m, 2F).

### Example 88: Preparation of compound 88

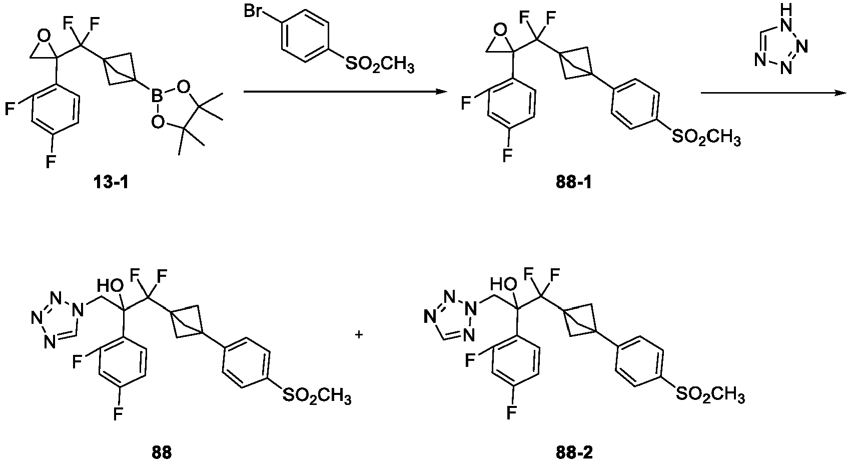

### Preparation of compound 88-1

Compound **13-1** (1.0 g, 2.51 mmol) was dissolved in a mixed solvent of cyclopentyl methyl ether (8 mL) and water (2 mL), then p-methanesulfonyl bromobenzene (590 mg, 2.51 mmol), Cu₂O (359 mg, 2.51 mmol), Pd(OAc)₂ (56 mg, 0.25 mmol), cataCxium A (180 mg, 0.50 mmol), and Cs₂CO₃ (2.45 g, 7.53 mmol) were sequentially added thereto, and the reaction system was reacted at 120°C for 16 hours. After the reaction system was cooled, the reaction mixture was filtered, and the crude filtrate was purified by preparative separation (chromatographic column: Agilent 10 Prep-C18 250 × 21.2 mm; column temperature: 25°C; mobile phase: water (0.1% FA)-acetonitrile; proportion of acetonitrile in the mobile phase: 50% to 70% in 12 min; flow rate: 30 mL/min) to obtain the title compound **88-1** (30 mg, yield: 4%). LC-MS (ESI): m/z 426.8 [M+H]⁺.

### Preparation of compound 88

Compound **88-1** (16 mg, 0.036 mmol) was dissolved in DMF (1 mL), then 1H-tetrazole (10 mg, 0.144 mmol) and K₂CO₃ (20 mg, 0.144 mmol) were added thereto, and the reaction mixture was reacted at 75°C for 16 hours. After the reaction system was cooled, the reaction mixture was filtered, and the crude filtrate was purified by preparative separation (chromatographic column: Welch Xtimate@ C18 21.2 × 250 mm; column temperature: 25°C; mobile phase: water (10 mM/L NH₄HCO₃)-acetonitrile; proportion of acetonitrile in the mobile phase: 40% to 60% in 12 min; flow rate: 30 mL/min) to obtain compounds **88** (5.5 mg, yield: 34%) and **88-2** (1 mg, yield: 6%).

Compound **88:** LC-MS (ESI): m/z 497.0 [M+H]⁺. ¹H NMR (400 MHz, Chloroform-d) δ 8.54 (s, 1H), 7.84 (d, *J* = 8.4 Hz, 2H), 7.70 - 7.60 (m, 1H), 7.28 (s, 1H), 7.26 (s, 1H), 6.94 - 6.80 (m, 2H), 5.45 (d, *J* = 14.6 Hz, 1H), 5.06 (d, *J* = 14.6 Hz, 1H), 4.13 (s, 1H), 3.01 (s, 3H), 2.12 (dd, *J* = 9.5, 2.0 Hz, 3H), 1.93 (dd, *J* = 9.5, 2.0 Hz, 3H). ¹⁹F NMR (376 MHz, Chloroform-*d*) δ -106.65 (d, 2F), -110.37 (d, 1F), -110.56 (d, 1F).

Compound **88-2:** LC-MS (ESI): m/z 497.0 [M+H]⁺. ¹H NMR (400 MHz, Chloroform-*d*) δ 8.40 (s, 1H), 7.84 (d, *J* = 8.4 Hz, 2H), 7.71 - 7.59 (m, 1H), 7.30 - 7.26 (m, 2H), 6.88 - 6.77 (m, 2H), 5.75 (d, *J* = 14.2 Hz, 1H), 5.29 (d, *J* = 14.3 Hz, 1H), 4.36 (s, 1H), 3.00 (s, 3H), 2.12 (dd, *J* = 9.5, 2.0 Hz, 3H), 1.93 (dd, *J* = 9.4, 2.0 Hz, 3H). ¹⁹F NMR (376 MHz, DMSO-d6) δ -107.65 (d, 2F), -110.76 (d, 1F), -111.04 (d, 1F).

### Example 89: Preparation of compound 89

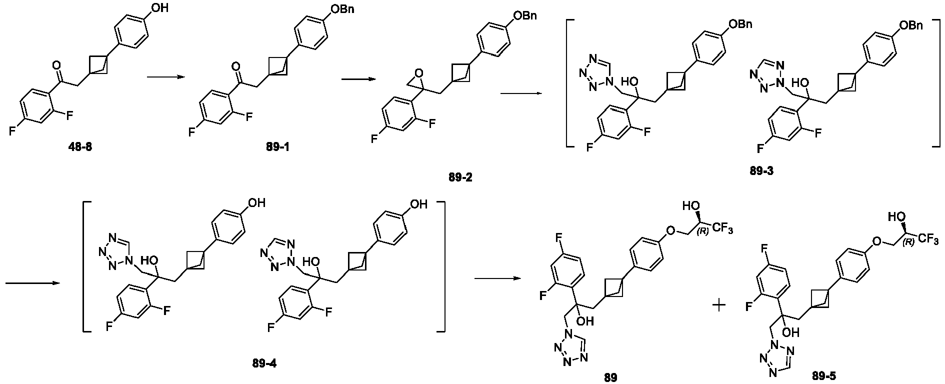

### Preparation of compound 89-1

Compound **48-8** (500 mg, 1.59 mmol), potassium carbonate (439 mg, 3.18 mmol), and compound benzyl bromide (326 mg, 1.91 mmol) were dissolved in ACN (10 mL) and reacted at 50°C for 16 hours. After the reaction system was cooled, the reaction system was quenched with water (20 mL), extracted with EtOAc (20 mL × 3), and the organic phases were combined, dried, and concentrated to obtain a crude product. The crude product was purified by normal-phase silica gel column chromatography (EtOAc/PE = 0 to 10%) to obtain the title compound **89-1** (500 mg, yield: 77%). LC-MS (ESI): m/z 405.2 [M+H]⁺

### Preparation of compound 89-2

Trimethylsulfonium iodide (1.26 g, 6.18 mmol) was dissolved in DMSO (6 mL) and THF (2 mL), then NaH (247 mg, 60%, 6.18 mmol) was slowly added thereto at 10°C. After stirring for 30 min, **89-1** (500 mg, 0.43 mmol) was added thereto. The reaction mixture was stirred at 50°C for 16 hours, and TLC detected that the reaction was completed. The reaction system was added with 50 mL of water and extracted with dichloromethane (20 mL × 3), and the organic phases were combined, dried, and concentrated to obtain a crude product. The crude product was purified by normal-phase silica gel column chromatography (EtOAc/PE = 0 to 30%) to obtain the title compound **89-2** (500 mg, yield: 96%).

### Preparation of compound 89-3

Compound **89-2** (80 mg, 0.19 mmol) was dissolved in DMF (1 mL), then 1H-tetrazole (67 mg, 0.95 mmol) and potassium carbonate (132 mg, 0.95 mmol) were added thereto, and the reaction mixture was reacted at 80°C for 16 hours in a sealed tube. TLC detected that the reaction was completed. The reaction system was quenched with saturated ammonium chloride solution (5 mL), extracted with EtOAc (5 mL × 3), and the organic phases were combined, dried, and concentrated to obtain a crude product of **89-3** (80 mg, yield: 85%).

### Preparation of compound 89-4

Compound **89-3** (80 mg, 0.16 mmol) was dissolved in a mixed solvent of methanol (2 mL) and acetonitrile (2 mL), then Pd/C (10%, 10 mg) was added thereto. The system was replaced with hydrogen three times. The reaction system was stirred at 50°C for 16 hours. After the reaction system was cooled, the reaction mixture was filtered and evaporated to dryness by rotary evaporation to obtain the crude title compound **89-4** (60 mg, yield: 91%). LC-MS (ESI): m/z 399.0 [M+H]⁺.

### Preparation of compound 89

Compound **89-4** (60 mg, 0.15 mmol) was dissolved in acetonitrile (2 mL), then potassium carbonate (41 mg, 0.30 mmol) was added thereto with stirring. After the reaction was carried out for 5 min, the reaction mixture was then added with R-(+)-2-trifluoromethyloxirane (CAS number: 143142-90-9, 20 mg, 0.18 mmol), stirred and reacted at 50°C for 16 hours. The crude filtrate was purified by preparative separation (preparation method: chromatographic column: Agilent 10 Prep-C18 250 × 21.2 mm; mobile phase: A: 0.1% formic acid aqueous solution; B: acetonitrile; column temperature: 25°C; gradient: 50% to 70% acetonitrile in 12 min; flow rate: 30 mL/min) to obtain the title compound **89** (6 mg, yield: 7%) and its corresponding regioisomer compound **89-5** (5 mg, yield: 6%).

Compound **89:** LC-MS (ESI): m/z 511.2 [M+H]⁺. ¹H NMR (400 MHz, DMSO-76) δ 9.09 (s, 1H), 7.45-7.32 (m, 1H), 7.28-7.15 (m, 1H), 7.04-6.91 (m, 3H), 6.82 (d, *J* = 8.6 Hz, 2H), 6.62 (d, *J =* 6.6 Hz, 1H), 5.87 (s, 1H), 4.79 (d, *J* = 14.1 Hz, 1H), 4.68 (d, *J* = 14.2 Hz, 1H), 4.42 - 4.24 (m, 1H), 4.16 - 4.05 (m, 1H), 4.05 - 3.91 (m, 1H), 2.42 (d, *J* = 14.8, 2.5 Hz, 1H), 1.96 (d, *J* = 14.7 Hz, 1H), 1.66 (d, *J* = 9.4, 1.5 Hz, 3H), 1.50 (d, *J* = 9.4, 1.5 Hz, 3H). ¹⁹F NMR (376 MHz, DMSO-*d6*) δ -76.08 (s, 3F), -108.55 (m, 1F), -111.90 (m, 1F).

Compound **89-5:** LC-MS (ESI): m/z 511.2[M+H]⁺. ¹H NMR (400 MHz, DMSO-76) δ 8.79 (s, 1H), 7.49-7.35 (m, 1H), 7.30-7.13 (m, 1H), 7.04-6.90 (m, 3H), 6.85-6.78 (m, 2H), 6.61 (d, *J* = 6.6 Hz, 1H), 5.82 (s, 1H), 5.06-4.84 (m, 2H), 4.40-4.22 (m, 1H), 4.18-4.04 (m, 1H), 4.03-3.90 (m, 1H), 2.45 (d, *J* = 14.8 Hz, 1H), 2.03 (d, *J* = 14.8 Hz, 1H), 1.64 (dd, *J* = 9.6, 1.6 Hz, 3H), 1.47 (dd, *J* = 9.6, 1.6 Hz, 3H). ¹⁹F NMR (376 MHz, DMSO-*d6*) δ -76.08 (s, 3F), - 108.81 (m, 1F), -112.21 (m, 1F).

### Example 90: Preparation of compound 90

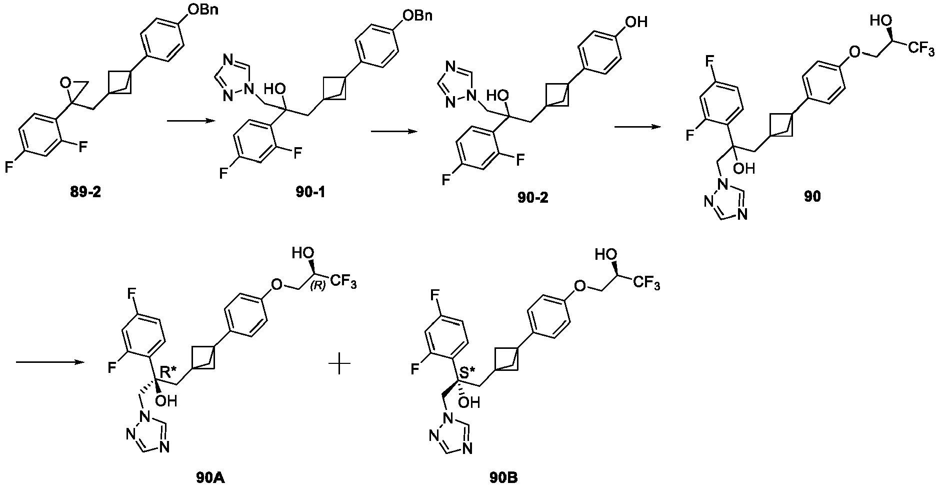

### Preparation of compound 90-1

Compound 1,2,4-triazole (412 mg, 5.97 mmol) was dissolved in DMF solution (5 mL) and cooled to 0°C. The reaction system was added with sodium hydride (60%, 239 mg, 5.97 mmol), reacted for half an hour, and then added with compound **89-2** (500 mg, 1.19 mmol). The reaction system was stirred at 80°C for 2 hours. The reaction system was cooled, then added with 50 mL of water and extracted with dichloromethane (20 mL × 3), and the organic phases were combined, and purified by normal-phase silica gel column chromatography (EtOAc/PE = 0 to 30%) to obtain the title compound **90-1** (300 mg, yield: 51%). LC-MS (ESI): m/z 488.2 [M+H]⁺.

### Preparation of compound 90-2

Compound **90-1** (300 mg, 0.62 mmol) was dissolved in a mixed solvent of methanol (2 mL) and acetonitrile (2 mL), then Pd/C (10%, 30 mg) was added thereto. The system was replaced with hydrogen three times, and the reaction system was stirred at 50°C for 16 hours. The reaction system was cooled, then filtered and evaporated to dryness by rotary evaporation, and the crude product was purified by normal-phase silica gel column chromatography (EtOAc/PE = 0 to 30%) to obtain the title compound **90-2** (230 mg, yield: 94%). LC-MS (ESI): m/z 398.2 [M+H]⁺.

### Preparation of compound 90

Compound **90-2** (230 mg, 0.58 mmol) was dissolved in acetonitrile (2 mL), then potassium carbonate (160 mg, 1.16 mmol) was added thereto with stirring. After the reaction was carried out for 5 min, the reaction mixture was then added with R-(+)-2-trifluoromethyloxirane (CAS: 143142-90-9, 82 mg, 0.75 mmol), stirred and reacted at 50°C for 16 hours. The reaction mixture was filtered, and the crude filtrate was purified by preparative separation (preparation method: chromatographic column: Agilent 10 Prep-C18 250 × 21.2 mm; column temperature: 25°C; mobile phase: water (0.1% TFA)-acetonitrile; proportion of acetonitrile in the mobile phase: 50% to 70% in 12 min; flow rate: 30 mL/min) to obtain the title product **90** (160 mg, yield: 54%, a pair of enantiomers). LC-MS (ESI): m/z 510.2 [M+H]⁺. ¹H NMR (400 MHz, DMSO-d6) δ 8.29 (s, 1H), 7.78 (s, 1H), 7.47 (q, *J =* 8.4 Hz, 1H), 7.25-7.11 (m, 1H), 7.05-6.89 (m, 3H), 6.81 (d, *J =* 8.1 Hz, 2H), 6.62 (d, *J =* 6.7 Hz, 1H), 5.65 (s, 1H), 4.57-4.40 (m, 2H), 4.40-4.23 (m, 1H), 4.09 (dd, *J* = 10.5, 4.3 Hz, 1H), 3.98 (dd, *J* = 10.6, 6.4 Hz, 1H), 2.38 (d, *J* = 15.0 Hz, 1H), 1.89 (d, *J* = 14.7 Hz, 1H), 1.64 (d, *J* = 9.5 Hz, 3H), 1.48 (d, *J* = 9.5 Hz, 3H). ¹⁹F NMR (376 MHz, DMSO-*d6*) δ -76.08 (s, 3F), -108.48 (d, 1F), -112.49 (d, 1F).

### Preparation of compounds 90A and 90B

Compound **90** (180 mg) was subjected to SFC chiral preparative resolution (preparative separation method, instrument model: MG II preparative SFC (SFC-14); model of chromatographic column: ChiralPak IC, 250 × 30 mm I.D. 10 µm; mobile phase: A: CO₂ B: ethanol; elution gradient: B 30%; flow rate: 80 mL/min; column pressure: 100 bar; column temperature: 38°C; detection wavelength: 220 nm; cycle: about 3 min) to obtain the title compounds **90A** (79 mg, single enantiomer) and **90B** (77 mg, single enantiomer).

Compound **90A:** LC-MS (ESI): m/z 510.2 [M+H]⁺. ¹H NMR (400 MHz, DMSO-*d6)* δ 8.29 (s, 1H), 7.78 (s, 1H), 7.47 (q, *J* = 8.4 Hz, 1H), 7.25-7.11 (m, 1H), 7.05-6.89 (m, 3H), 6.81 (d, *J* = 8.1 Hz, 2H), 6.62 (d, *J* = 6.7 Hz, 1H), 5.65 (s, 1H), 4.57-4.40 (m, 2H), 4.40 -4.23 (m, 1H), 4.09 (dd, *J* = 10.5, 4.3 Hz, 1H), 3.98 (dd, *J* = 10.6, 6.4 Hz, 1H), 2.38 (d, *J* = 15.0 Hz, 1H), 1.89 (d, *J* = 14.7 Hz, 1H), 1.64 (d, *J* = 9.5 Hz, 3H), 1.48 (d, *J* = 9.5 Hz, 3H). ¹⁹F NMR (376 MHz, DMSO-*d6*) δ -76.08 (s, 3F), -108.48 (d, 1F), -112.49 (d, 1F).

Compound **90B:** LC-MS (ESI): m/z 510.2 [M+H]⁺. ¹H NMR (400 MHz, DMSO-76) δ 8.29 (s, 1H), 7.78 (s, 1H), 7.47 (q, *J* = 8.4 Hz, 1H), 7.25-7.11 (m, 1H), 7.05-6.89 (m, 3H), 6.81 (d, *J* = 8.1 Hz, 2H), 6.62 (d, *J* = 6.7 Hz, 1H), 5.65 (s, 1H), 4.57-4.40 (m, 2H), 4.40-4.23 (m, 1H), 4.09 (dd, *J* = 10.5, 4.3 Hz, 1H), 3.98 (dd, *J* = 10.6, 6.4 Hz, 1H), 2.38 (d, *J* = 15.0 Hz, 1H), 1.89 (d, *J* = 14.7 Hz, 1H), 1.64 (d, *J* = 9.5 Hz, 3H), 1.48 (d, *J* = 9.5 Hz, 3H). ¹⁹F NMR (376 MHz, DMSO-d6) δ -76.08 (s, 3F), -108.48 (d, 1F), -112.49 (d, 1F).

### Example 91: Preparation of compounds 91, 91A, and 91B

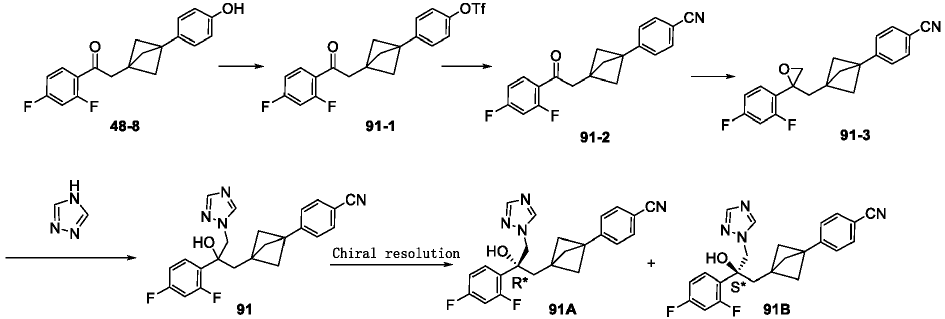

### Preparation of compound 91-1

Compound **48-8** (1.5 g, 4.77 mmol) was dissolved in DMF (10 mL), then triethylamine (965 mg, 9.55 mmol) was added thereto. After the reaction mixture was stirred and reacted at 0°C for 5 min, the reaction system was slowly added with N-phenyl-bis(trifluoromethanesulfonimide) (2.0 g, 5.7 mmol), transferred to room temperature, and reacted for 2 hours. The reaction mixture was extracted with EtOAc (50 mL × 3), and the organic phases were combined, dried, and concentrated to obtain a crude product. The crude product was purified by normal-phase silica gel column chromatography (EtOAc/PE = 0 to 5%) to obtain the title compound **91-1** (1.2 g, yellow oil, yield: 70%). LC-MS (ESI): m/z 447.0 [M+H]⁺.

### Preparation of compound 91-2

Compound **91-1** (1.2 g, 2.69 mmol) was dissolved in DMF (8 mL), then zinc cyanide (629 g, 5.38 mmol) and tetrakis(triphenylphosphine)palladium (0.17 g, 0.15 mmol) were added thereto, and the reaction system was replaced with nitrogen three times and reacted under microwave irradiation at 100°C for 40 hours. The reaction system was cooled, then extracted with EtOAc (50 mL × 3), and the organic phases were combined, dried, and concentrated to obtain a crude product. The crude product was purified by normal-phase silica gel column chromatography (EtOAc/PE = 0 to 5%) to obtain the title compound **91-2** (800 mg, yellow solid, yield: 92%). LC-MS (ESI): m/z 324.0 [M+H]⁺.

### Preparation of compound 91-3

Trimethylsulfonium iodide (1.5 g, 7.43 mmol) was dissolved in the mixed solvent of anhydrous DMSO (20 mL) and anhydrous THF (20 mL), then NaH (569 mg, purity: 60%, 14.86 mmol) was added thereto, and the reaction system was stirred for 30 min, then added with compound **91-2** (800 mg), and reacted at room temperature for 16 hours. The reaction system was extracted with EtOAc (50 mL × 3), and the organic phases were combined, dried, and concentrated to obtain the crude title compound **91-3** (560 mg, crude, light yellow oil). LC-MS (ESI): m/z 338.2 [M+H]⁺.

### Preparation of compound 91

Triazole (171.8 mg, 2.49 mmol) was dissolved in anhydrous DMF (10 mL), then NaH (96 mg, purity: 60%, 2.49 mmol) was added thereto, and the reaction system was stirred for 30 min, then added with compound **91-3** (560 mg), and reacted at 50°C for 16 hours. After the reaction system was cooled, the reaction mixture was filtered, and the filtrate was purified by preparative separation (preparation method: mobile phase: A: 0.1% NH₄HCO₃ aqueous solution; B: acetonitrile; chromatographic column: Agilent 10 Prep-C18 250 × 21.2 mm; column temperature: 25°C; gradient: 45% to 65% acetonitrile in 12 min; flow rate: 30 mL/min) to obtain the title compound **91** (30 mg). LC-MS (ESI): m/z 407.0 [M+H]⁺. ¹H NMR (400 MHz, DMSO-d6) δ 8.29 (s, 1H), 7.78 (s, 1H), 7.74 - 7.63 (m, 2H), 7.47 (td, *J* = 9.1, 6.9 Hz, 1H), 7.32 - 7.13 (m, 3H), 6.99 (td, *J* = 8.5, 2.6 Hz, 1H), 5.70 (s, 1H), 4.45 (d, *J* = 2.6 Hz, 2H), 2.39 (dd, *J* = 15.0, 2.5 Hz, 1H), 1.91 (d, *J* = 14.9 Hz, 1H), 1.72 (dd, *J* = 9.5, 1.7 Hz, 3H), 1.56 (dd, *J* = 9.5, 1.7 Hz, 3H). ¹⁹F NMR (376 MHz, DMSO-d6) δ -108.48 - -108.50 (m, 1F), - 112.37 - -112.39 (m, 1F).

### Preparation of compounds 91A and 91B

Compound **91** (80 mg) was subjected to SFC chiral preparative resolution (preparative separation method, instrument model: MG II preparative SFC (SFC-14); chromatographic column: ChiralCel OX, 250 × 30 mm I.D., 10 µm; mobile phase: A: CO₂ B: ethanol (0.1% NH₃H₂O); gradient: B 20%; flow rate: 70 mL/min; column pressure: 100 bar; chromatographic column temperature: 38°C; detection wavelength: 220 nM; cycle: about 5 min) to obtain the title compounds **91A** (34 mg) and **91B** (38 mg).

Compound **91A:** LC-MS (ESI): m/z 407.0 [M+H]⁺. ¹H NMR (400 MHz, DMSO-*d6)* δ 8.28 (s, 1H), 7.78 (s, 1H), 7.75 - 7.63 (m, 2H), 7.47 (td, *J* = 9.1, 6.9 Hz, 1H), 7.29 - 7.11 (m, 3H), 6.99 (td, *J* = 8.5, 2.6 Hz, 1H), 5.68 (s, 1H), 4.45 (d, *J* = 2.8 Hz, 2H), 2.45 - 2.29 (m, 1H), 1.91 (d, *J =* 14.9 Hz, 1H), 1.72 (dd, *J* = 9.5, 1.7 Hz, 3H), 1.56 (dd, *J* = 9.5, 1.6 Hz, 3H). ¹⁹F NMR (376 MHz, DMSO-d6) δ -108.47 - -108.49 (m, 1F), -112.38 (s, 1F)

Compound **91B:** LC-MS (ESI): m/z 407.0 [M+H]⁺. ¹H NMR (400 MHz, DMSO-*d6)* δ 8.29 (s, 1H), 7.78 (s, 1H), 7.75 - 7.63 (m, 2H), 7.47 (td, *J* = 9.0, 6.8 Hz, 1H), 7.29 - 7.14 (m, 3H), 6.99 (td, *J* = 8.5, 2.6 Hz, 1H), 5.70 (d, *J* = 2.0 Hz, 1H), 4.45 (d, *J* = 2.7 Hz, 2H), 2.39 (dd, *J* = 14.8, 2.5 Hz, 1H), 1.91 (d, *J* = 14.8 Hz, 1H), 1.72 (dd, *J* = 9.6, 1.6 Hz, 3H), 1.56 (dd, *J* = 9.5, 1.6 Hz, 3H). ¹⁹F NMR (376 MHz, DMSO-*d6*) δ -108.47 - -108.50 (m, 1F), -112.36 (s, 1F).

### Example 92: Preparation of compound 92

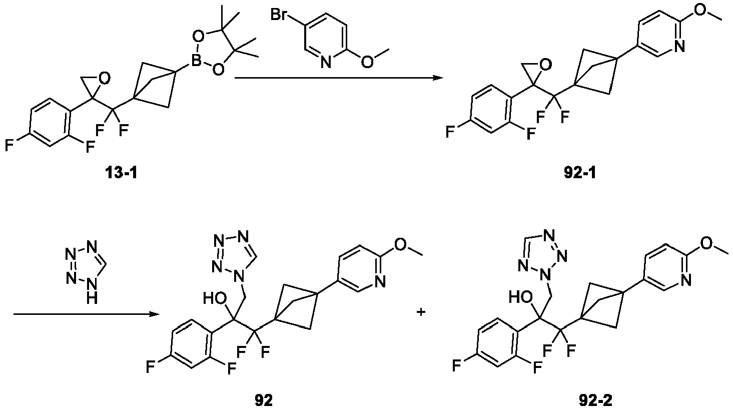

### Preparation of compound 92-1

Compound **13-1** (2 g, 5.03 mmol) was dissolved in a mixed solvent of cyclopentyl methyl ether (4 mL) and water (1 mL), then 5-bromo-2-methoxypyridine (1.41 g, 7.54 mmol), palladium acetate (114 mg, 0.5 mmol), butyldi-1-adamantylphosphine (360 mg, 1.0 mmol), cesium carbonate (4.92 g, 15.1 mmol), and cuprous oxide (720 mg, 5.03 mmol) were sequentially added thereto. The reaction system was replaced with nitrogen three times, and reacted under microwave irradiation at 120°C for 16 hours. The reaction system was extracted with EtOAc (50 mL × 3), and the organic phases were combined, dried, and concentrated to obtain a crude product. The crude product was purified by normal-phase silica gel column chromatography (EtOAc/PE = 0 to 30%) to obtain the title compound **92-1** (150 mg, pale yellow oil, yield: 6.7%). LC-MS (ESI): m/z 380.2 [M+H]⁺.

### Preparation of compound 92

Compound **92-1** (110 mg, 0.29 mmol) was dissolved in DMF (5 mL), then 1H-tetrazole (80 mg, 1.14 mmol) and potassium carbonate (158 mg, 1.14 mmol) were added thereto, and the reaction system was stirred at 80°C for 16 hours in a sealed tube. After the reaction system was cooled, the reaction mixture was filtered, and the crude filtrate was purified by preparative separation (preparation method: mobile phase: A: 0.1% formic acid aqueous solution; B: acetonitrile; chromatographic column: Agilent 10 Prep-C18 250 × 21.2 mm; column temperature: 25°C; gradient: 50% to 70% acetonitrile in 12 min; flow rate: 30 mL/min) to obtain the title compound **92** (33 mg, yield: 22%) and the corresponding regioisomer compound **92-2** (9 mg, yield: 6.1%).

Compound **92:** LC-MS (ESI): m/z 450.2 [M+H]⁺. ¹H NMR (400 MHz, DMSO-76) δ 9.13 (s, 1H), 7.92 (d, *J* = 2.4 Hz, 1H), 7.58 - 7.44 (m, 2H), 7.34 - 7.25 (m, 1H), 7.17 (s, 1H), 7.01 (td, *J* = 8.4, 2.6 Hz, 1H), 6.71 (d, *J* = 8.6 Hz, 1H), 5.43 (d, *J* = 14.6 Hz, 1H), 5.01 (d, *J =* 14.6 Hz, 1H), 3.79 (s, 3H), 2.03 (dd, J= 9.4, 1.8 Hz, 3H), 1.82 (dd, *J* = 9.4, 1.8 Hz, 3H). ¹⁹F NMR (376 MHz, DMSO-*d6*) δ -102.83 - -103.28 (m, 1F), -109.16 - -109.47 (m, 2F), -109.58 (d, *J* = 9.4 Hz, 1F).

Compound **92-2:** LC-MS (ESI): m/z 450.2 [M+H]⁺. ¹H NMR (400 MHz, DMSO-*d6*) δ 8.77 (s, 1H), 7.91 (d, *J* = 2.4 Hz, 1H), 7.56 (td, *J* = 9.0, 6.8 Hz, 1H), 7.49 (dd, *J* = 8.6, 2.4 Hz, 1H), 7.31 - 7.23 (m, 1H), 7.22 (s, 1H), 7.00 (td, *J* = 8.4, 2.6 Hz, 1H), 6.71 (d, *J* = 8.4 Hz, 1H), 5.61 (d, *J* = 14.2Hz, 1H), 5.24 (d, *J* = 14.2 Hz, 1H), 3.79 (s, 3H), 2.01 (dd, *J* = 9.4, 1.8 Hz, 3H), 1.80 (dd, *J* = 9.4, 1.8 Hz, 3H). ¹⁹F NMR (376 MHz, DMSO-d6) δ -102.56 - -102.78 (m, 1F), -108.95 - -109.16 (m, 2F), -109.95 (d, *J* = 9.4 Hz, 1F).

### Example 93: Preparation of compound 93

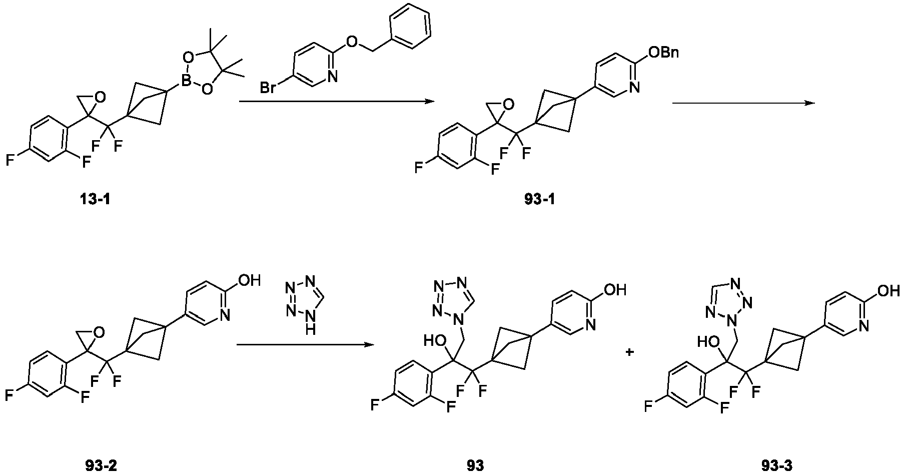

### Preparation of compound 93-1

To a microwave tube was added compound **13-1** (2 g, 5.03 mmol), then 2-benzyloxy-5-bromopyridine (2 g, 7.54 mmol), palladium acetate (114 mg, 0.5 mmol), butyldi-1-adamantylphosphine (360 mg, 1.0 mmol), cesium carbonate (4.92 g, 15.1 mmol), and cuprous oxide (720 mg, 5.03 mmol) were sequentially added thereto, and the reaction mixture was dissolved in a mixed solvent of cyclopentyl methyl ether (16 mL) and water (4 mL). The reaction system was replaced with nitrogen three times, and reacted under microwave irradiation at 120°C for 16 hours. The reaction system was cooled and then extracted with EtOAc (50 mL × 3), and the organic phases were combined, dried, and concentrated to obtain a crude product. The crude product was purified by normal-phase silica gel column chromatography (EtOAc/PE = 0 to 30%) to obtain the title compound **93-1** (690 mg, pale yellow oil, yield: 30%). LC-MS (ESI): m/z 456.2 [M+H]⁺.

### Preparation of compound 93-2

Compound **93-1** (690 mg, 1.52 mmol) was dissolved in methanol (15 mL), then 10% wet palladium on carbon (70 mg) was added thereto. The reaction system was replaced with hydrogen three times and then reacted at room temperature for 16 hours. The reaction mixture was filtered to obtain crude compound **93-2** (450 mg), which was used directly in the next step. LC-MS (ESI): m/z 366.2 [M+H]⁺.

### Preparation of compounds 93 and 93-3

Compound **93** (450 mg, 1.23 mmol) was dissolved in DMF solution (10 mL), then compound tetrazole (345 mg, 4.93 mmol) and potassium carbonate (681 mg, 4.93mmol) were sequentially added thereto, and the reaction system was stirred at 80°C for 16 hours in a sealed tube. After the reaction system was cooled, the reaction mixture was filtered, and the crude filtrate was purified by preparative separation (preparation method: mobile phase: A: 0.1% formic acid aqueous solution; B: acetonitrile; chromatographic column: Agilent 10 Prep-C18 250 × 21.2 mm; column temperature: 25°C; gradient: 25% to 45% acetonitrile in 12 min; flow rate: 30 mL/min) to obtain the title compound **93** (140 mg, yield: 29%).

LC-MS (ESI): m/z 436.2 [M+H]⁺. ¹H NMR (400 MHz, DMSO-*d6*) δ 11.50 (s, 1H), 9.12 (s, 1H), 7.58-7.46 (m, 1H), 7.34-7.22 (m, 2H), 7.14 (s, 1H), 7.06-6.94 (m, 2H), 6.24 (dd, *J* = 9.4, 0.6 Hz, 1H), 5.20 (dd, *J* = 168.8, 14.6 Hz, 2H), 1.93 (dd, *J* = 9.6, 1.8 Hz, 3H), 1.71 (dd, *J* = 9.6, 1.8 Hz, 3H). ¹⁹F NMR (376 MHz, DMSO-*d6*) δ -103.00 - -103.21 (m, 1F), -109.27--109.37 (m, 2F), -109.61 (d, *J* = 9.0 Hz, 1F).

Corresponding regioisomer compound **93-3** (58 mg, yield: 12%)

LC-MS (ESI): m/z 436.2 [M+H]⁺. ¹H NMR (400 MHz, DMSO-d6) δ 11.50 (s, 1H), 8.76 (s, 1H), 7.62-7.47 (m, 1H), 7.35-7.13 (m, 3H), 7.10-6.93 (m, 2H), 6.23 (dd, *J* = 9.4, 0.6 Hz, 1H), 5.41 (dd, *J* = 148.6, 14.2 Hz, 2H), 1.92 (dd, *J* = 9.6, 1.8 Hz, 3H), 1.70 (dd, *J* = 9.6, 1.8 Hz, 3H). ¹⁹F NMR (376 MHz, DMSO-*d6*) δ -102.58 - -102.80 (m, 1F), -108.92 - -109.12 (m, 2F), -109.97 (d, *J* = 9.0 Hz, 1F).

### Example 94: Preparation of compound 94

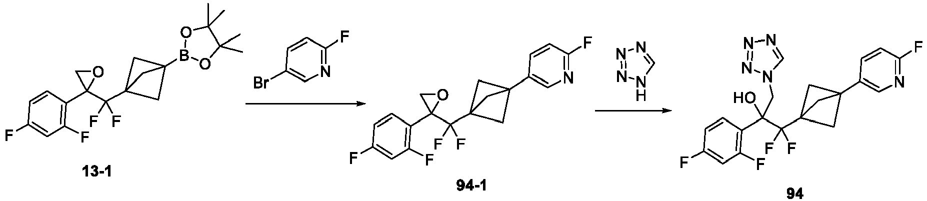

### Preparation of compound 94-1

Compound **13-1** (3 g, 7.54 mmol) was dissolved in a mixed solvent of cyclopentyl methyl ether (20 mL) and water (5 mL), then 2-fluoro-5-bromopyridine (2 g, 11.3 mmol), palladium acetate (170 mg, 0.75 mmol), butyldi-1-adamantylphosphine (544 mg, 1.51 mmol), cesium carbonate (7.42 g, 22.6 mmol), and cuprous oxide (1.08 g, 7.54 mmol) were sequentially added to a microwave tube. The reaction system was replaced with nitrogen three times, and reacted under microwave irradiation at 120°C for 16 hours. The reaction mixture was cooled and then extracted with EtOAc (50 mL × 3), and the organic phases were combined, dried, and concentrated to obtain a crude product. The crude product was purified by normal-phase silica gel column chromatography (EtOAc/PE = 0 to 30%) to obtain the title compound **94-1** (1.46 g, pale yellow oil, yield: 53%). LC-MS (ESI): m/z 368.2 [M+H]+.

### Preparation of compound 94

Compound **94-1** (1.46 g, 3.98 mmol) was dissolved in DMF solution (10 mL), then compound tetrazole (1.11 g, 15.9 mmol) and potassium carbonate (2.2 g, 15.9mmol) were added thereto, and the reaction system was stirred at 80°C for 16 hours in a sealed tube. After the reaction system was cooled, the reaction mixture was filtered, and the crude filtrate was purified by preparative separation (preparation method: mobile phase: A: 0.1% formic acid aqueous solution; B: acetonitrile; chromatographic column: Agilent 10 Prep-C18 250 × 21.2 mm; column temperature: 25°C; gradient: 45% to 65% acetonitrile in 12 min; flow rate: 30 mL/min) to obtain the title compound **94** (317 mg, yield: 18%, containing a pair of enantiomers).

LC-MS (ESI): m/z 438.4 [M+H]⁺. ¹H NMR (400 MHz, DMSO-*d6*) δ 9.13 (s, 1H), 8.02 (d, *J* = 2.6 Hz, 1H), 7.78 (td, *J* = 8.2, 2.6 Hz, 1H), 7.59-7.48 (m, 1H), 7.35-7.24 (m, 1H), 7.20 (s, 1H), 7.09 (dd, *J* = 8.4, 2.6 Hz, 1H), 7.00 (td, *J* = 8.4, 2.6 Hz, 1H), 5.43 (d, *J* = 14.6 Hz, 1H), 5.01 (d, *J* = 14.6 Hz, 1H), 2.08 (dd, *J* = 9.4, 1.8 Hz, 3H), 1.87 (dd, *J* = 9.4, 1.8 Hz, 3H). ¹⁹F NMR (376 MHz, DMSO-*d6*) δ -70.80 (s, 1F), -102.96 - -103.18 (m, 1F), -109.39 - -109.55 (m, 3F).

### Example 95: Preparation of compound 95

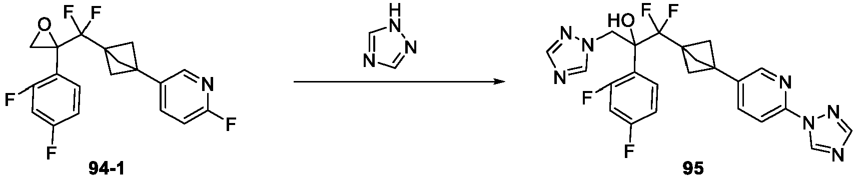

### Preparation of compound 95

Compound **94-1** (500 mg, 1.36 mmol) was dissolved in DMF (5 mL), and NaH (272 mg, 6.81 mmol) was added thereto at 0°C. After the reaction system was stirred for 30 min, triazole (470 mg, 6.81 mmol) was added thereto, and the reaction system was transferred to 70°C and reacted for 16 hours. After the reaction system was cooled, the reaction mixture was filtered, and the crude filtrate was purified by preparative separation (chromatographic column: Welch Xtimate^{®} C18 21.2 × 250 mm; column temperature: 25°C; mobile phase: water (10 mM/L NH₄HCO₃)-acetonitrile; proportion of acetonitrile in the mobile phase: 40% to 60% in 12 min; flow rate: 30 mL/min) to obtain the title compound **95** (52 mg, yield: 10%).

LC-MS (ESI): m/z 486.2 [M+H]⁺. ¹H NMR (400 MHz, DMSO-*d6*) δ 9.31 (d, *J* = 3.1 Hz, 1H), 8.43 - 8.24 (m, 3H), 7.92 - 7.82 (m, 1H), 7.82 - 7.74 (m, 1H), 7.69 (d, *J* = 2.8 Hz, 1H), 7.57 (q, *J* = 8.3 Hz, 1H), 7.26 - 7.16 (m, 1H), 7.05 - 6.88 (m, 2H), 5.14 (d, *J* = 14.4 Hz, 1H), 4.77 (d, *J* = 14.3 Hz, 1H), 2.12 (d, *J* = 9.9 Hz, 3H), 1.90 (d, *J* = 9.4 Hz, 3H). ¹⁹F NMR (376 MHz, DMSO-d6) δ -102.17 - -103.01 (m, 1F), -109.29 - -109.87 (m, 2F), -110.35 (d, 1F).

### Example 96: Preparation of compound 96

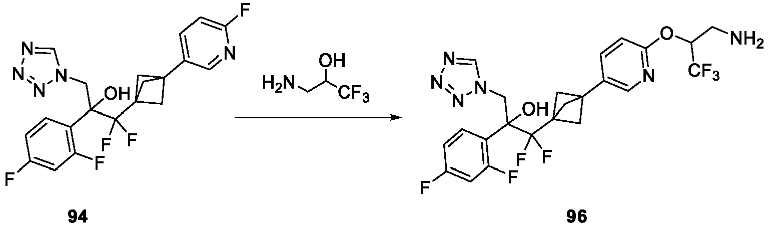

### Preparation of compound 96

Compound 94 (75 mg, 0.173 mmol) was dissolved in anhydrous DMF (2 mL), then 3-amino-1,1,1-trifluoropropan-2-ol (90 mg, 0.692 mmol) and NaH (17 mg, 0.692 mmol) were added thereto, and the reaction system was stirred at 50°C for 16 hours. The reaction system was quenched by adding saturated NH₄Cl solution, extracted with EtOAc (50 mL × 3), and the organic phases were combined, dried, and concentrated to obtain a crude product, which was prepared by reverse phase (preparative column: Pursuit XRs 10 C18 250 * 21.2 mm; flow rate: 25 mL/min; mobile phase: A: 0.1% FA aqueous solution, B: acetonitrile; gradient: 5% to 33% acetonitrile content, retention time: 7.0 to 7.9 min) to obtain the title compound **96** (14.56 mg).

LC-MS (ESI): m/z 547.2 [M+H]⁺. ¹H NMR (400 MHz, DMSO-*d6*) δ 9.14 (s, 1H), 8.25 (br.s, 1H), 7.93 (s, 1H), 7.58 (dd, *J* = 8.5, 2.0 Hz, 2H), 7.27 (ddd, *J* = 23.7, 13.2, 8.6 Hz, 2H), 7.03 - 6.97 (m, 1H), 6.86 (d, *J* = 8.5 Hz, 1H), 5.79 (s, 1H), 5.42 (d, *J* = 14.5 Hz, 1H), 5.02 (d, *J* = 14.6 Hz, 1H), 3.02 (s, 1H), 2.89 (s, 1H), 2.05 (d, *J* = 8.2 Hz, 3H), 1.84 (d, *J* = 9.4 Hz, 3H).

### Example 97: Preparation of compound 97

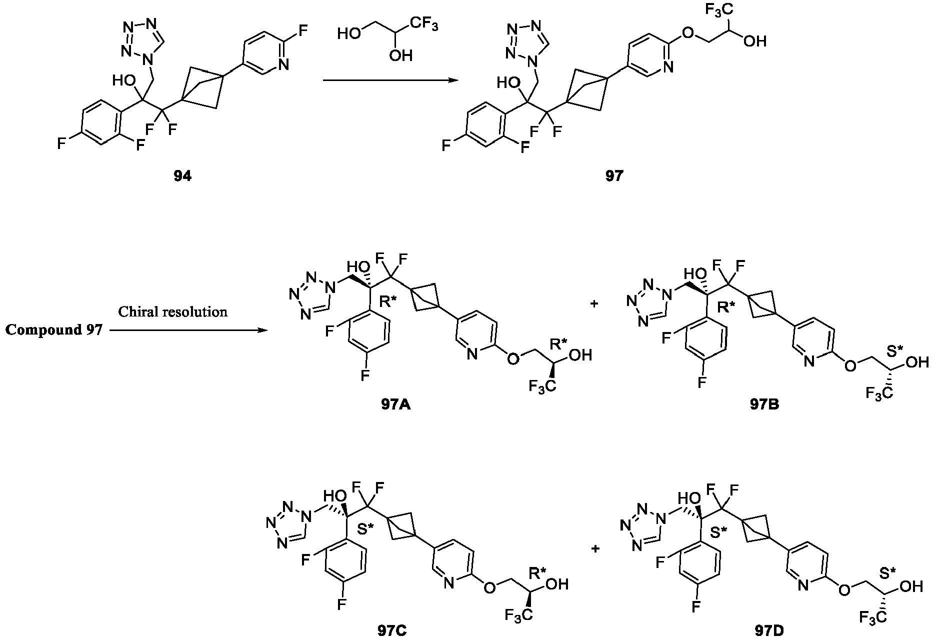

### Preparation of compound 97

Compound **94** (309 mg, 2.38 mmol) was dissolved in DMF (5 mL), then sodium hydride (95 mg, 2.38 mmol) was added thereto with stirring at 0°C. After the reaction was carried out for 5 min, the reaction mixture was then added with 3,3,3-trifluoropropane-1,2-diol (260 mg, 0.59 mmol), stirred and reacted at 50°C for 16 hours. The reaction mixture was filtered, and the crude filtrate was purified by preparative separation (preparation method: chromatographic column: Agilent 10 Prep-C18 250 × 21.2 mm; column temperature: 25°C; mobile phase: water (0.1% TFA)-acetonitrile; proportion of acetonitrile in the mobile phase: 45% to 65% in 12 min; flow rate: 30 mL/min) to obtain the title product **97** (180 mg, yield: 55%, two pairs of diastereoisomers).

LC-MS (ESI): m/z 548.0 [M+H]⁺. ¹H NMR (400 MHz, DMSO-*d6*) δ 9.14 (s, 1H), 7.93 (dd, *J* = 2.4, 0.8 Hz, 1H), 7.58 - 7.47 (m, 2H), 7.34 - 7.23 (m, 1H), 7.20 (s, 1H), 7.00 (td, *J* = 8.4, 2.6 Hz, 1H), 6.76 (dd, *J* = 8.4, 0.8 Hz, 1H), 6.60 (d, *J* = 6.4 Hz, 1H), 5.22 (dd, *J* = 165.6, 14.6 Hz, 2H), 4.49 - 4.17 (m, 3H), 2.03 (dd, *J* = 9.4, 1.8 Hz, 3H), 1.82 (dd, *J* = 9.4, 1.8 Hz, 3H). ¹⁹F NMR (376 MHz, DMSO-*d6*) δ -76.20 (s, 3F), -102.86 - -103.08 (m, 1F), - 109.31- -109.81 (m, 3F).

### Preparation of compounds 97A, 97B, 97C, and 97D

Compound **97** (180 mg) was subjected to SFC chiral preparative resolution (preparative separation method, instrument model: MG II preparative SFC (SFC-14); model of chromatographic column: ChiralCel OJ, 250 × 30 mm I.D., 5 µm; mobile phase: A: CO₂ B: methanol (0.1% NH₃H₂O); elution gradient: B 20%; flow rate: 60 mL/min; column pressure: 100 bar; column temperature: 38°C; detection wavelength: 220 nm; cycle: about 8 min) to obtain the title compounds **97A** (23 mg), **97B** (29 mg), **97C** (29 mg), and **97D** (28 mg).

Compound **97A:** LC-MS (ESI): 548.1 [M+H]⁺. ¹H NMR (400 MHz, DMSO-*d6*) δ 9.14 (s, 1H), 7.93 (dd, *J* = 2.4, 0.8 Hz, 1H), 7.58 - 7.47 (m, 2H), 7.34 - 7.23 (m, 1H), 7.20 (s, 1H), 7.00 (td, *J* = 8.4, 2.6 Hz, 1H), 6.76 (dd, *J* = 8.4, 0.8 Hz, 1H), 6.60 (d, *J* = 6.4 Hz, 1H), 5.22 (dd, *J* = 165.6, 14.6 Hz, 2H), 4.49 - 4.17 (m, 3H), 2.03 (dd, *J* = 9.4, 1.8 Hz, 3H), 1.82 (dd, *J* = 9.4, 1.8 Hz, 3H). ¹⁹F NMR (376 MHz, DMSO-*d6*) δ -76.21 (s, 3F), -102.96 - -103.18 (m, 1F), -109.29 - -109.60 (m, 3F).

Compound **97B:** LC-MS (ESI): 548.1 [M+H]⁺. ¹H NMR (400 MHz, DMSO-*d6*) δ 9.15 (s, 1H), 7.97 - 7.88 (m, 1H), 7.61 - 7.46 (m, 2H), 7.34 - 7.22 (m, 1H), 7.07 - 6.82 (m, 3H), 6.75 (dd, *J* = 8.4, 0.8 Hz, 1H), 5.22 (dd, *J* = 163.4, 14.6 Hz, 2H), 4.52 - 4.17 (m, 3H), 2.03 (dd, *J* = 9.4, 1.8 Hz, 3H), 1.82 (dd, *J* = 9.4, 1.8 Hz, 3H). ¹⁹F NMR (376 MHz, DMSO-*d6*) δ -76.21 (s, 3F), -102.98 - -103.17 (m, 1F), -109.29 - -109.62 (m, 3F).

Compound **97C:** LC-MS (ESI): 548.1 [M+H]⁺. ¹H NMR (400 MHz, DMSO-d6) δ 9.14 (s, 1H), 7.97 - 7.88 (m, 1H), 7.61 - 7.46 (m, 2H), 7.34 - 7.22 (m, 1H), 7.07 - 6.82 (m, 3H), 6.75 (dd, *J* = 8.4, 0.8 Hz, 1H), 5.22 (dd, *J* = 163.4, 14.6 Hz, 2H), 4.52 - 4.17 (m, 3H), 2.03 (dd, *J* = 9.4, 1.8 Hz, 3H), 1.82 (dd, *J* = 9.4, 1.8 Hz, 3H). ¹⁹F NMR (376 MHz, DMSO-d6) δ -76.21 (s, 3F), -102.96 - -103.18 (m, 1F), -109.29 - -109.61 (m, 3F).

Compound **97D:** LC-MS (ESI): 548.1 [M+H]⁺. ¹H NMR (400 MHz, DMSO-*d6*) δ 9.16 (s, 1H), 7.97 - 7.88 (m, 1H), 7.61 - 7.46 (m, 2H), 7.34 - 7.22 (m, 1H), 7.07 - 6.82 (m, 3H), 6.75 (dd, *J* = 8.4, 0.8 Hz, 1H), 5.22 (dd, *J* = 163.4, 14.6 Hz, 2H), 4.52 - 4.17 (m, 3H), 2.03 (dd, *J* = 9.4, 1.8 Hz, 3H), 1.82 (dd, *J* = 9.4, 1.8 Hz, 3H). ¹⁹F NMR (376 MHz, DMSO-*d6*) δ -76.21 (s, 3F), -102.97 - -103.16 (m, 1F), -109.29 - -109.62 (m, 3F).

### Example 98: Preparation of compounds 98A, 98B, 98C, and 98D

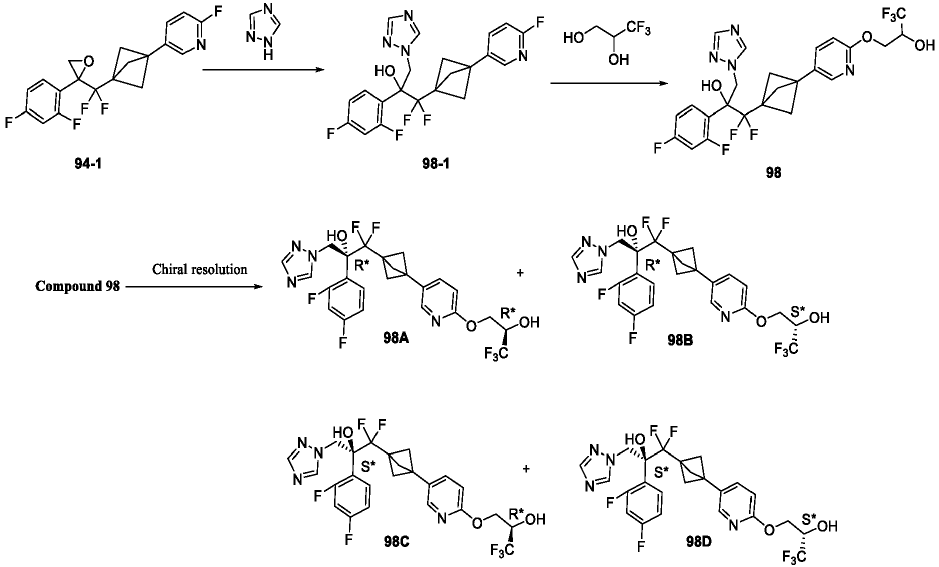

### Preparation of compound 98-1

Triazole (2.26 g, 32.7 mmol) was dissolved in DMF solution (10 mL), then sodium hydride (1.31 g, 32.7 mmol) was added thereto with stirring at 0°C. After the reaction was carried out for 30 min, compound **94-1** (3 g, 8.2 mmol) was then added thereto. The reaction system was stirred for 16 hours at 80°C in a sealed tube. After the reaction system was cooled, the reaction mixture was filtered, and the crude filtrate was purified by preparative separation (preparation method: chromatographic column: Welch Xtimate^{®} C18 21.2 × 250 mm; column temperature: 25°C; mobile phase: water (10 mM/L NH₄HCO₃)-acetonitrile; proportion of acetonitrile in the mobile phase: 40% to 60% in 12 min; flow rate: 30 mL/min) to obtain the title compound **98-1** (800 mg, yield: 22%). LC-MS (ESI): m/z 437.0 [M+H]⁺.

### Preparation of compound 98

Compound **98-1** (954 mg, 7.34 mmol) was dissolved in DMF (10 mL), then sodium hydride (367 mg, 9.17 mmol) was added thereto with stirring at 0°C. The reaction system was reacted for 5 min, then added with compound **4** (800 mg, 1.83 mmol), stirred and reacted at 50°C for 16 hours. The reaction mixture was filtered, and the crude filtrate was purified by preparative separation (preparation method: chromatographic column: Welch Xtimate^{®} C18 21.2 × 250 mm; column temperature: 25°C; mobile phase: water (10 mM/L NH₄HCO₃)-acetonitrile; proportion of acetonitrile in the mobile phase: 50% to 70% in 12 min; flow rate: 30 mL/min) to obtain the title compound **98** (700 mg, yield: 70%, two pairs of diastereoisomers).

LC-MS (ESI): m/z 547.5 [M+H]⁺. ¹H NMR (400 MHz, DMSO-*d6*) δ 8.36 (s, 1H), 7.93 (dd, *J* = 2.4, 0.8 Hz, 1H), 7.69 (s, 1H), 7.63 - 7.47 (m, 2H), 7.20 (ddd, *J* = 12.0, 9.2, 2.6 Hz, 1H), 6.97 (td, *J* = 8.6, 2.8 Hz, 1H), 6.90 (s, 1H), 6.75 (dd, *J* = 8.6, 0.8 Hz, 1H), 6.58 (d, *J* = 6.6 Hz, 1H), 4.95 (dd, *J* = 150.2, 14.4 Hz, 2H), 4.49 - 4.21 (m, 3H), 2.02 (dd, *J* = 9.4, 1.8 Hz, 3H), 1.81 (dd, *J* = 9.4, 1.8 Hz, 3H). ¹⁹F NMR (376 MHz, DMSO-*d6*) δ -76.20 (s, 3F), - 102.51 - -102.73 (m, 1F), -109.51 - -109.61 (m, 2F), -110.40 - -110.43 (m, 1F).

### Preparation of compounds 98A, 98B, 98C, and 98D

Compound **98** (700 mg) was subjected to SFC chiral preparative resolution (preparative separation method, instrument model: MG II preparative SFC (SFC-14); model of chromatographic column: ChiralCel OD, 250 × 30 mm I.D., 10 µm; mobile phase: A: CO₂ B: isopropanol (0.1% NH₃H₂O); elution gradient: B 30%; flow rate: 65 mL/min; column pressure: 100 bar; column temperature: 38°C; detection wavelength: 220 nm; cycle: about 6.5 min) to obtain the title compounds **98A, 98B, 98C,** and **98D.**

Compound **98A:** LC-MS (ESI): 547.0 [M+H]⁺. ¹H NMR (400 MHz, DMSO-*d6*) δ 8.36 (s, 1H), 7.93 (dd, *J* = 2.4, 0.8 Hz, 1H), 7.69 (s, 1H), 7.63 - 7.45 (m, 2H), 7.20 (ddd, *J* = 12.0, 9.2, 2.6 Hz, 1H), 6.97 (td, *J* = 8.4, 2.6 Hz, 1H), 6.93 - 6.51 (m, 3H), 4.95 (dd, *J* = 150.0, 14.4 Hz, 2H), 4.48 - 4.22 (m, 3H), 2.02 (dd, *J* = 9.4, 1.8 Hz, 3H), 1.81 (dd, *J* = 9.4, 1.8 Hz, 3H). ¹⁹F NMR (376 MHz, DMSO-*d6*) δ -76.21 (s, 3F), -102.52 - -102.74 (m, 1F), -109.51--109.62 (m, 2F), -110.41 - -110.44 (m, 1F).

Compound **98B:** LC-MS (ESI): 547.0 [M+H]⁺. ¹H NMR (400 MHz, DMSO-*d6*) δ 8.36 (s, 1H), 7.93 (dd, *J* = 2.4, 0.8 Hz, 1H), 7.69 (s, 1H), 7.63 - 7.45 (m, 2H), 7.20 (ddd, *J* = 12.0, 9.2, 2.6 Hz, 1H), 6.97 (td, *J* = 8.4, 2.6 Hz, 1H), 6.93 - 6.51 (m, 3H), 4.95 (dd, *J* = 150.0, 14.4 Hz, 2H), 4.48 - 4.22 (m, 3H), 2.02 (dd, *J* = 9.4, 1.8 Hz, 3H), 1.81 (dd, *J* = 9.4, 1.8 Hz, 3H). ¹⁹F NMR (376 MHz, DMSO-*d6*) δ -76.21 (s, 3F), -102.52 - -102.73 (m, 1F), -109.51--109.61 (m, 2F), -110.41 - -110.44 (m, 1F).

Compound **98C:** LC-MS (ESI): 547.0 [M+H]⁺. ¹H NMR (400 MHz, DMSO-*d6*) δ 8.36 (s, 1H), 7.93 (dd, *J* = 2.4, 0.8 Hz, 1H), 7.69 (s, 1H), 7.63 - 7.47 (m, 2H), 7.20 (ddd, *J* = 12.0, 9.2, 2.6 Hz, 1H), 6.97 (td, *J* = 8.6, 2.8 Hz, 1H), 6.90 (s, 1H), 6.75 (dd, *J* = 8.6, 0.8 Hz, 1H), 6.58 (d, *J* = 6.6 Hz, 1H), 4.95 (dd, *J* = 150.2, 14.4 Hz, 2H), 4.49 - 4.21 (m, 3H), 2.02 (dd, *J* = 9.4, 1.8 Hz, 3H), 1.81 (dd, *J* = 9.4, 1.8 Hz, 3H). ¹⁹F NMR (376 MHz, DMSO-*d6*) δ -76.21 (s, 3F), -102.62 - -102.73 (m, 1F), -109.50 - -109.60 (m, 2F), -110.42 - -110.44 (m, 1F).

Compound **98D:** LC-MS (ESI): 547.1 [M+H]⁺. ¹H NMR (400 MHz, DMSO-*d6*) δ 8.36 (s, 1H), 7.93 (dd, *J* = 2.4, 0.8 Hz, 1H), 7.69 (s, 1H), 7.61 - 7.47 (m, 2H), 7.20 (ddd, *J* = 12.0, 9.2, 2.6 Hz, 1H), 7.01 - 6.82 (m, 2H), 6.75 (dd, *J* = 8.4, 0.8 Hz, 1H), 6.67 - 6.50 (m, 1H), 4.95 (dd, *J* = 150.2, 14.4 Hz, 2H), 4.49 - 4.20 (m, 3H), 2.02 (dd, *J* = 9.4, 1.8 Hz, 3H), 1.81 (dd, *J* = 9.4, 1.8 Hz, 3H). ¹⁹F NMR (376 MHz, DMSO-*d6*) δ -76.20 (s, 3F), -102.52 - -102.73 (m, 1F), -109.50 - -109.60 (m, 2F), -110.41 - -110.44 (m, 1F).

### Example 99: Preparation of compounds 99A, 99B, 99C, and 99D

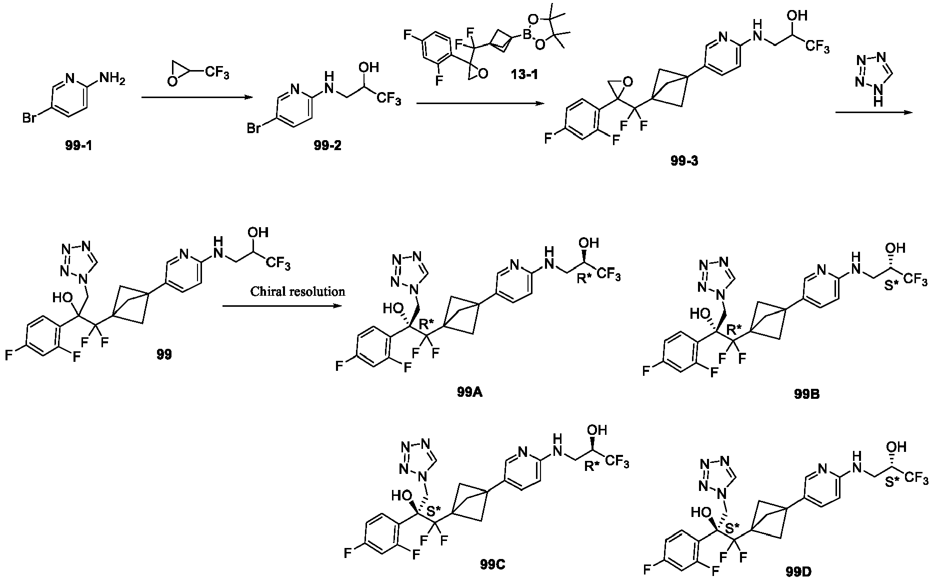

### Preparation of compound 99-2

To a three-necked flask was added compound **99-1** (3 g, 17.34 mmol), and the system was replaced with nitrogen three times. Anhydrous THF (30 mL) was added thereto, and LiHMDS (35 mL, 34.7 mmol, 1.0 M in THF) was added dropwise thereto at -60°C. The reaction system was stirred and reacted for 30 min, then added with 1,1,1-trifluoro-2,3-epoxypropane (2.9 g, 26.01 mmol), naturally warmed to room temperature, and reacted for 16 hours. The reaction mixture was poured into a mixture of ice water and ammonium chloride, and extracted with EtOAc (50 mL × 3), and the organic phases were combined, dried, and concentrated to obtain a crude product. The crude product was purified by normal-phase silica gel column chromatography (EtOAc/PE = 0 to 35%) to obtain the title compound **99-2** (850 mg, yellow solid, yield: 17%). LC-MS (ESI): m/z 285.0 [M+H]⁺.

### Preparation of compound 99-3

Compound **99-2** (1 g, 3.52 mmol) was dissolved in a mixed solvent of cyclopentyl methyl ether (9 mL) and water (7 mL), then compound **13-1** (2.1 g, 5.28 mmol), palladium acetate (78 mg, 0.352 mmol), CatacxiumA (250 mg, 0.704 mmol), Cu₂O (504 mg, 3.52 mmol), and cesium carbonate (3.4 g, 10.56 mmol) were sequentially added thereto. The reaction system was replaced with nitrogen three times and reacted in an oil bath at 120°C for 16 hours. The reaction system was extracted with EtOAc (50 mL × 3), and the organic phases were combined, dried, and concentrated to obtain a crude product. The crude product was purified by normal-phase silica gel column chromatography (EtOAc/PE = 0 to 35%) to obtain the title compound **99-3** (500 mg, oily liquid, yield: 29.8%). LC-MS (ESI): m/z 477.0 [M+H]⁺.

### Preparation of compound 99

Compound **99-3** (1.2 g, 2.52 mmol) was dissolved in DMF (5 mL), then 1H-tetrazole (706 mg, 10.08 mmol) and potassium carbonate (1 g, 7.56 mmol) were added thereto, and the reaction system was stirred and reacted at 80°C for 16 hours. The reaction system was extracted with EtOAc (50 mL × 3), and the organic phases were combined, dried, and concentrated to obtain a crude product, which was purified by preparative separation (preparative column: Xbridge C18 21.2 * 250 mm * 10 µm; flow rate: 20 mL/min; mobile phase: A: 0.05% ammonia solution, B: acetonitrile; gradient: 53 to 53% acetonitrile content, retention time 6.5 to 10.0 min) to obtain the title compound **99** (240 mg, yield: 17.4%). LC-MS (ESI): m/z 547.2 [M+H]⁺.

### Preparation of compounds 99A, 99B, 99C, and 99D

Compound **99** (240 mg) was subjected to SFC chiral preparative resolution (preparative separation method: instrument model: Waters SFC 150; model of chromatographic column: DAICELCHIRALPAK^{®}IB, 250 * 25 mm 10 µm; mobile phase: A: CO₂ and B: MeOH (0.1% 7.0 mol/L ammonia methanol solution); elution gradient: B 40%, flow rate: 70 mL/min; column pressure: 100 bar; column temperature: RT; detection wavelength: 214 nm; cycle: 4 min) to obtain the title compounds **99A** (33.25 mg), **99B** (27.32 mg), **99C** (32.13 mg), and **99D** (35.79 mg).

Compound **99A:** LC-MS (ESI): m/z 547.2 [M+H]⁺. Chiral analysis method (model of chromatographic column: DAICELCHIRALPAK^{®}IB 100 * 3 mm 3 µm; mobile phase: A: CO₂, B: MeOH (0.1% DEA); elution gradient: maintained 5% B for the first 0.5 min, 5% B to 40% B for 0.5 to 5.5 min, maintained 40% B for 5.5 to 8 min; flow rate: 1.5 mL/min; column temperature: 35°C; column pressure: 1800 psi; detection wavelength: 214 nm; RT = 4.274 min). ¹H NMR (400 MHz, DMSO-d6) δ 9.13 (s, 1H), 7.74 (d, *J* = 2.1 Hz, 1H), 7.53 (dd, *J* = 15.9, 8.9 Hz, 1H), 7.27 (ddd, *J* = 11.9, 9.0, 2.5 Hz, 1H), 7.18 (dd, *J* = 8.6, 2.3 Hz, 1H), 7.12 (s, 1H), 7.03 - 6.96 (m, 1H), 6.73 (t, *J* = 5.9 Hz, 1H), 6.52 (d, *J* = 6.3 Hz, 1H), 6.46 (d, *J* = 8.5 Hz, 1H), 5.42 (d, *J* = 14.5 Hz, 1H), 5.00 (d, *J* = 14.6 Hz, 1H), 4.11 (s, 1H), 3.66 - 3.58 (m, 1H), 3.26 - 3.16 (m, 1H), 1.96 (d, *J* = 8.6 Hz, 3H), 1.75 (d, *J* = 9.3 Hz, 3H).

Compound **99B:** LC-MS (ESI): m/z 547.2 [M+H]⁺. Chiral analysis method (model of chromatographic column: DAICELCHIRALPAK^{®}IB 100 * 3 mm 3 µm; mobile phase: A: CO₂, B: MeOH (0.1% DEA); elution gradient: maintained 5% B for the first 0.5 min, 5% B to 40% B for 0.5 to 5.5 min, maintained 40% B for 5.5 to 8 min; flow rate: 1.5 mL/min; column temperature: 35°C; column pressure: 1800 psi; detection wavelength: 214 nm; RT = 4.948 min). ¹H NMR (400 MHz, DMSO-*d6*) δ 9.13 (s, 1H), 7.74 (d, *J* = 2.1 Hz, 1H), 7.53 (dd, *J* = 15.8, 8.9 Hz, 1H), 7.28 (td, *J* = 9.3, 4.6 Hz, 1H), 7.18 (dd, *J* = 8.5, 2.3 Hz, 1H), 7.13 (s, 1H), 7.03 - 6.96 (m, 1H), 6.73 (t, *J* = 5.9 Hz, 1H), 6.52 (d, *J* = 6.3 Hz, 1H), 6.46 (d, *J* = 8.6 Hz, 1H), 5.42 (d, *J* = 14.5 Hz, 1H), 5.00 (d, *J* = 14.4 Hz, 1H), 4.10 (s, 1H), 3.67 - 3.57 (m, 1H), 3.22 (dd, *J* = 13.6, 5.4 Hz, 1H), 1.96 (d, *J* = 8.6 Hz, 3H), 1.75 (d, *J* = 9.3 Hz, 3H).

Compound **99C:** LC-MS (ESI): m/z 547.2 [M+H]⁺. Chiral analysis method (model of chromatographic column: DAICELCHIRALPAK^{®}IB 100 * 3 mm 3 µm; mobile phase: A: CO₂, B: MeOH (0.1% DEA); elution gradient: maintained 5% B for the first 0.5 min, 5% B to 40% B for 0.5 to 5.5 min, maintained 40% B for 5.5 to 8 min; flow rate: 1.5 mL/min; column temperature: 35°C; column pressure: 1800 psi; detection wavelength: 214 nm; RT = 4.243 min). ¹H NMR (400 MHz, DMSO-d6) δ 9.13 (s, 1H), 7.74 (d, *J* = 2.2 Hz, 1H), 7.53 (dd, *J* = 15.8, 8.9 Hz, 1H), 7.27 (ddd, *J* = 12.0, 9.1, 2.6 Hz, 1H), 7.18 (dd, *J* = 8.6, 2.3 Hz, 1H), 7.13 (s, 1H), 7.00 (td, *J* = 8.6, 2.5 Hz, 1H), 6.73 (t, *J* = 5.9 Hz, 1H), 6.52 (d, *J* = 6.3 Hz, 1H), 6.46 (d, *J* = 8.6 Hz, 1H), 5.42 (d, *J* = 14.5 Hz, 1H), 5.00 (d, *J* = 14.6 Hz, 1H), 4.16 - 4.04 (m, 1H), 3.67 - 3.56 (m, 1H), 3.26 - 3.15 (m, 1H), 1.96 (d, *J* = 8.6 Hz, 3H), 1.75 (d, *J* = 9.3 Hz, 3H).

Compound **99D:** LC-MS (ESI): m/z 547.2 [M+H]⁺. Chiral analysis method (model of chromatographic column: DAICELCHIRALPAK^{®}IB 100 * 3 mm 3 µm; mobile phase: A: CO₂, B: MeOH (0.1% DEA); elution gradient: maintained 5% B for the first 0.5 min, 5% B to 40% B for 0.5 to 5.5 min, maintained 40% B for 5.5 to 8 min; flow rate: 1.5 mL/min; column temperature: 35°C; column pressure: 1800 psi; detection wavelength: 214 nm; RT = 3.881 min). ¹H NMR (400 MHz, DMSO-d6) δ 9.13 (s, 1H), 7.74 (d, *J* = 2.2 Hz, 1H), 7.53 (dd, *J* = 15.8, 8.9 Hz, 1H), 7.27 (ddd, *J* = 12.1, 9.1, 2.6 Hz, 1H), 7.18 (dd, *J* = 8.6, 2.3 Hz, 1H), 7.12 (s, 1H), 7.03 - 6.97 (m, 1H), 6.73 (t, *J* = 5.9 Hz, 1H), 6.52 (d, *J* = 6.3 Hz, 1H), 6.46 (d, *J* = 8.6 Hz, 1H), 5.42 (d, *J* = 14.5 Hz, 1H), 5.00 (d, *J* = 14.6 Hz, 1H), 4.11 (d, *J* = 7.0 Hz, 1H), 3.66 - 3.58 (m, 1H), 3.22 (dd, *J* = 13.8, 5.7 Hz, 1H), 1.96 (d, *J* = 9.3 Hz, 3H), 1.75 (d, *J* = 9.4 Hz, 3H).

### Example 100: Preparation of compound 100

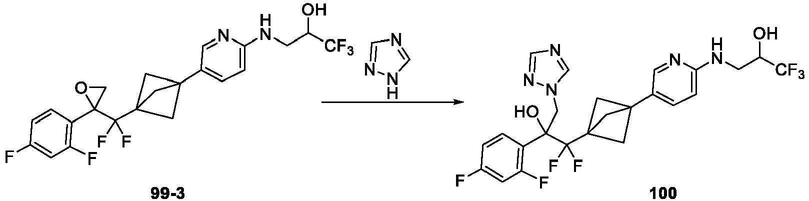

### Preparation of compound 100

Compound **99-3** (80 mg, 0.168 mmol) was dissolved in DMF (2 mL), then 1,2,4-triazole (46 mg, 0.672 mmol) and potassium carbonate (70 mg, 0.504 mmol) were added thereto, and the reaction system was reacted at 80°C for 16 hours. After the reaction system was cooled, the reaction mixture was filtered, and the filtrate was purified by preparative separation (preparation column: Pursuit XRs 10 C18 250 * 21.2 mm; flow rate: 20 mL/min; mobile phase: A: 0.1% FA aqueous solution, B: acetonitrile; gradient: 5 to 30% acetonitrile content, retention time of 7.0 to 10.0 min) to obtain the title compound **100** (6.68 mg, yield: 7.3%). LC-MS (ESI): m/z 546.2 [M+H]⁺. ¹H NMR (400 MHz, DMSO-d6) δ 8.36 (s, 1H), 7.73 (d, *J* = 2.2 Hz, 1H), 7.68 (s, 1H), 7.56 (d, *J* = 6.9 Hz, 1H), 7.24 - 7.14 (m, 2H), 6.97 (td, *J* = 8.5, 2.6 Hz, 2H), 6.74 (t, *J* = 5.9 Hz, 1H), 6.46 (d, *J* = 8.4 Hz, 1H), 5.13 (d, *J* = 14.4 Hz, 1H), 4.76 (d, *J* = 14.6 Hz, 1H), 4.11 (d, *J* = 3.7 Hz, 1H), 3.65 - 3.60 (m, 1H), 3.20 (d, *J* = 5.8 Hz, 1H), 1.94 (d, *J* = 8.4 Hz, 3H), 1.73 (d, *J* = 9.4 Hz, 3H).

### Example 101: Preparation of compound 101

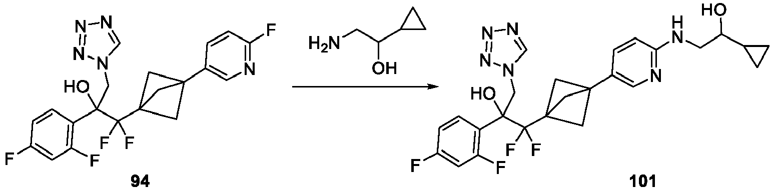

### Preparation of compound 101

Compound 2-amino-1-cyclopropylethan-1-ol (126 mg, 0.92 mmol) was dissolved in DMF (5 mL), then sodium hydride (73 mg, 1.83 mmol) was added thereto with stirring at 0°C. After the reaction was carried out for 5 min, the reaction mixture was then added with compound **94** (100 mg, 0.23 mmol), stirred and reacted at 50°C for 16 hours. The reaction mixture was filtered, and the crude filtrate was purified by preparative separation (preparation method: chromatographic column: Agilent 10 Prep-C18 250 × 21.2 mm; column temperature: 25°C; mobile phase: water (0.1% TFA)-acetonitrile; proportion of acetonitrile in the mobile phase: 20% to 50% in 12 min; flow rate: 30 mL/min) to obtain the title compound **101** (14 mg, yield: 12%, two pairs of diastereoisomers).

LC-MS (ESI): m/z 519.2 [M+H]⁺. ¹H NMR (400 MHz, DMSO-*d6*) δ 9.12 (s, 1H), 8.38 (s, 1H), 7.84 (d, *J* = 2.4 Hz, 1H), 7.59 - 7.41 (m, 2H), 7.26 (ddd, *J* = 12.0, 9.0, 2.6 Hz, 1H), 6.99 (td, *J* = 8.4, 2.6 Hz, 1H), 6.71 (d, *J* = 8.4 Hz, 1H), 5.21 (dd, *J* = 165.4, 14.6 Hz, 2H), 4.77 - 4.58 (m, 1H), 3.04 - 2.96 (m, 2H), 2.00 (dd, *J* = 9.4, 1.8 Hz, 3H), 1.79 (dd, *J* = 9.4, 1.8 Hz, 3H), 1.13 - 0.98 (m, 1H), 0.53 - 0.26 (m, 4H). ¹⁹F NMR (376 MHz, DMSO-*d6*) δ - 102.79 - -103.27 (m, 1F), -109.12 - -109.50 (m, 2F), -109.64 (d, *J* = 9.4 Hz, 1F).

### Example 102: Preparation of compound 102

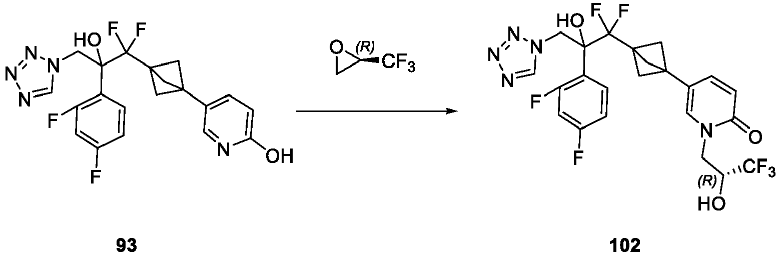

### Preparation of compound 102

Compound **93** (80 mg, 0.18 mmol) was dissolved in acetonitrile (5 mL), then potassium carbonate (51 mg, 0.37 mmol) was added thereto with stirring. After the reaction was carried out for 5 min, the reaction mixture was then added with R-(+)-2-trifluoromethyloxirane (CAS: 143142-90-9, 27 mg, 0.24 mmol), stirred and reacted at 50°C for 16 hours. The reaction mixture was filtered, and the crude filtrate was purified by preparative separation (preparation method: chromatographic column: Agilent 10 Prep-C18 250 × 21.2 mm; column temperature: 25°C; mobile phase: water (0.1% TFA)-acetonitrile; proportion of acetonitrile in the mobile phase: 45% to 65% in 12 min; flow rate: 30 mL/min) to obtain the title compound **102** (65 mg, yield: 65%, containing a pair of enantiomers).

LC-MS (ESI): m/z 548.2 [M+H]⁺. ¹H NMR (400 MHz, DMSO-*d6*) δ 9.12 (s, 1H), 7.57 - 7.47 (m, 1H), 7.42 - 7.25 (m, 3H), 7.17 (s, 1H), 7.01 (td, *J* = 8.4, 2.6 Hz, 1H), 6.56 (dd, *J* = 7.2, 2.6 Hz, 1H), 6.35 (d, *J* = 9.4 Hz, 1H), 5.21 (dd, *J* = 167.6, 14.6 Hz, 2H), 4.28 (d, *J =* 12.6 Hz, 2H), 3.71 (dd, *J* = 13.4, 9.8 Hz, 1H), 1.98 - 1.89 (m, 3H), 1.78 - 1.68 (m, 3H). ¹⁹F NMR (376 MHz, DMSO-d6) δ -77.19 (s, 3F), -102.84 - -103.25 (m, 1F), -109.26 - -109.59 (m, 3F).

### Example 103: Preparation of compound 103

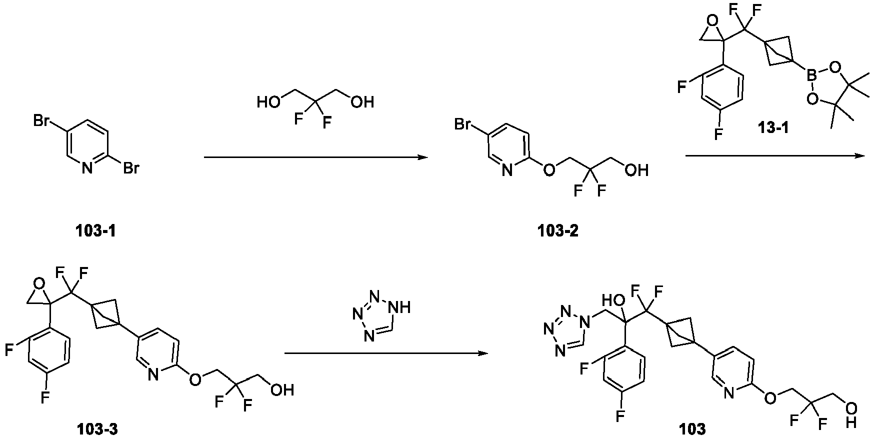

### Preparation of compound 103-2

Compound **103-1** (2.5 g, 10.5 mmol) was dissolved in THF (20 mL), then cesium carbonate (23.7 g, 11.4 mmol) and 2,2-difluoropropane-1,3-diol (1.76 g, 15.75 mmol) were added thereto, and the reaction system was replaced with nitrogen three times and reacted at 80°C for 16 hours. After the reaction was completed, the reaction system was poured into water (100 mL) and extracted with EA (50 mL × 3), and the organic phases were combined, concentrated, and subjected to column chromatography (PE: EA = 20:1 to 5:1) to obtain the title compound **103-2** as a yellow solid (80 mg, yield: 2.8%). LC-MS (ESI): m/z 269.2 [M+H]⁺.

### Preparation of compound 103-3

Compound **13-1** (131 mg, 0.33 mmol) was dissolved in methylcyclopentyl ether solution (5 mL), and then H₂O (1 mL), compound **103-2** (80 mg, 0.30 mmol), cuprous oxide (43 mg, 0.30 mmol), palladium acetate (11.2 mg, 0.05 mmol), butyldi-1-adamantylphosphine (35.8 mg, 0.1 mmol), and cesium carbonate (326 mg, 1 mmol) were sequentially added thereto. The reaction system was replaced with nitrogen three times, and then reacted under microwave irradiation at 120°C for 16 hours. The reaction system was poured into water (20 mL) and extracted with EA (15 mL × 3), and the organic phases were combined, concentrated, and subjected to column chromatography (PE: EA = 20:1 to 5:1) to obtain the title compound **103-3** (a pair of enantiomers) (50 mg, crude) as a yellow oil. LC-MS (ESI): m/z 460.2 [M+H]⁺.

### Preparation of compound 103

Compound **103-3** (50 mg, crude) was dissolved in DMF (5 mL), then potassium carbonate (40 mg, 0.28 mmol) and tetrazole (53.2 mg, 0.76 mmol) were added thereto, and the reaction system was stirred at 70°C for 16 hours in a sealed tube. After the reaction was completed, the reaction system was filtered, and the crude filtrate was purified by preparative separation (preparation method: mobile phase: A: 0.1% NH₄HCO₃ aqueous solution; B: acetonitrile; chromatographic column: Agilent 10 Prep-C18 250 × 21.2 mm; column temperature: 25°C; gradient: 45% to 65% acetonitrile in 12 min; flow rate: 30 mL/min) to obtain the title compound **103** (6.5 mg).

LC-MS (ESI): m/z 530.2 [M+H]⁺. ¹H NMR (400 MHz, DMSO-*d6*) δ 9.17 (d, *J* = 11.2 Hz, 1H), 8.26 (s, 1H), 7.85 (dd, *J* = 8.4, 1.9 Hz, 1H), 7.54 (td, *J* = 8.9, 6.5 Hz, 1H), 7.40 (s, 1H), 7.21 -7.10 (m, 3H), 5.76 - 5.61 (m, 1H), 5.41 (d, *J* = 14.7 Hz, 1H), 4.99 (d, *J* = 15.1 Hz, 1H), 4.69 (t, *J* = 13.7 Hz, 2H), 3.78 (td, *J* = 13.8, 6.2 Hz, 2H), 1.82 (dd, *J* = 9.5, 1.9 Hz, 3H), 1.62 (dd, *J* = 9.5, 1.9 Hz, 3H). ¹⁹F NMR (376 MHz, DMSO-*d6*) δ -107.51-107.58 (m, 2F), -109.16 -109.52 (m, 1F), -113.58 -113.61 (m, 1F), -114.06 - -114.11 (m, 2F).

### Example 104: Preparation of compound 104

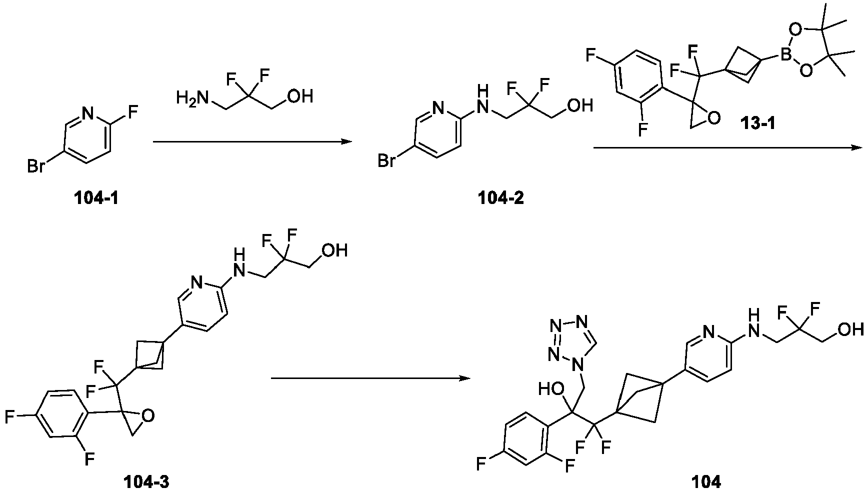

### Preparation of compound 104-2

To a single-necked flask was added compound **104-1** (200 mg, 1.13 mmol), then pyridine (2 mL) was added thereto. The reaction system was replaced with nitrogen, heated to 130°C, and reacted for 40 hours. The reaction system was concentrated, and the residue was subjected to column chromatography (PE: EA = 0 to 1:1) to obtain the title compound **104-2** (81 mg, yield: 26.7%).

LC-MS (ESI): m/z 266.9 [M+H]⁺. ¹H NMR (400 MHz, DMSO-*d6*) δ 8.04 (d, *J* = 2.3 Hz, 1H), 7.56 (dd, *J* = 8.9, 2.5 Hz, 1H), 7.08 (s, 1H), 6.61 (d, *J* = 8.9 Hz, 1H), 5.33 (t, *J* = 4.6 Hz, 1H), 3.78 (td, *J* = 15.1, 6.3 Hz, 2H), 3.65 - 3.57 (m, 2H).

### Preparation of compound 104-3

To a microwave reaction tube was added compound **104-2** (73 mg, 0.27 mmol), and then cesium carbonate (264 mg, 0.81 mmol), CataCXiumA (11 mg, 0.03 mmol), Cu₂O (39 mg, 0.27 mmol), and compound **13-1** (161 mg, 0.40 mmol) were sequentially added thereto. The reaction mixture was dissolved in a mixed solvent of cyclopentyl methyl ether (3 mL) and water (2.4 mL), purged with nitrogen for one minute after addition, sealed, and then heated to 120°C and reacted for 16 hours. After the reaction system was cooled, the reaction system was extracted with EtOAc (50 mL × 3), and the organic phases were combined, washed once with brine (50 mL), then dried over sodium sulfate, concentrated, and the crude product was separated by preparative TLC (developing solvent: PE: EA = 1:1) to obtain the title compound **104-3** (30 mg, yield: 24.0%). LC-MS (ESI): m/z = 459.1 [M+H]⁺.

### Preparation of compound 104

Compound **104-3** (30 mg, 0.06 mmol) was added to a microwave reaction tube and dissolved in DMF (1 mL), then tetrazole (9 mg, 0.13 mmol) and potassium carbonate (14 mg, 0.10 mmol) were added thereto, and the reaction mixture was heated to 80°C and reacted for 16 hours. After cooling to room temperature, the reaction system was filtered and directly prepared by a reversed-phase chromatographic column to obtain the title compound **104** (5.2 mg).

LC-MS (ESI): m/z 529.0 [M+H]⁺. ¹H NMR (400 MHz, DMSO-*d6*) δ 9.12 (s, 1H), 7.73 (d, *J* = 2.4 Hz, 1H), 7.53 (d, *J* = 6.8 Hz, 1H), 7.31 - 7.19 (m, 2H), 7.15 (s, 1H), 7.00 (t, *J* = 8.4 Hz, 1H), 6.85 (t, *J* = 6.4 Hz, 1H), 6.51 (d, *J* = 8.3 Hz, 1H), 5.69 (t, *J* = 6.5 Hz, 1H), 5.42 (d, *J* = 14.5 Hz, 1H), 5.00 (d, *J* = 15.0 Hz, 1H), 3.79 - 3.70 (m, 2H), 3.57 - 3.52 (m, 2H), 1.96 (d, *J* = 8.2 Hz, 3H), 1.75 (d, *J* = 9.5 Hz, 3H).

### Example 105: Preparation of compound 105

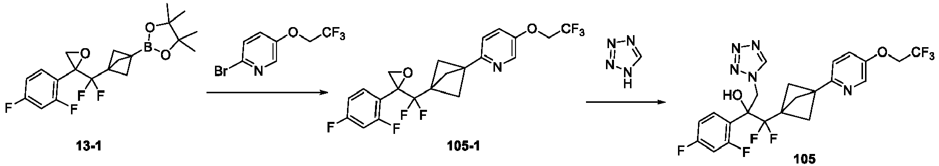

### Preparation of compound 105-1

Compound **13-1** (565.4 mg, 1.42 mmol) was dissolved in a mixed solvent of cyclopentyl methyl ether (5 mL) and water (3 mL), then 2-bromo-5-(2,2,2-trifluoroethoxy)pyridine (545 mg, 2.13 mmol), cataCXiumA (105 mg, 0.284 mmol), cesium carbonate (1388 mg, 4.62 mmol), cuprous oxide (203.2 mg, 1.42 mmol), and palladium acetate (32 mg, 0.142 mmol) were sequentially added thereto. The reaction system was replaced with nitrogen three times, and reacted under microwave irradiation at 120°C for 16 hours. After the reaction system was cooled, the reaction mixture was filtered and extracted with EtOAc (50 mL × 3), and the organic phases were combined, dried, and concentrated to obtain a crude product, which was purified by preparative separation (preparative column: Sunfire C18 21.2 * 250 mm * 10 µm; flow rate: 20 mL/min; mobile phase: A: 0.1% FA aqueous solution, B: acetonitrile; gradient: 67 to 68% acetonitrile content, retention time 10.2 to 10.9 min) to obtain the title compound **105-1** (77 mg, yield: 13.62%). LC-MS (ESI): m/z 448.0 [M+H]⁺.

### Preparation of compound 105

Compound **105-1** (70 mg, 0.16 mmol) was dissolved in DMF (2 mL), then 1H-tetrazole (56 mg, 0.8 mmol) and potassium carbonate (44 mg, 0.32 mmol) were added thereto, and the reaction system was reacted at 80°C for 16 hours. After the reaction system was cooled, the reaction mixture was filtered, and the filtrate was purified by preparative separation (preparation column: Pursuit XRs 10 C18 250 * 21.2 mm; flow rate: 25 mL/min; mobile phase: A: 0.1% FA aqueous solution, B: acetonitrile; gradient: 65 to 65% acetonitrile content, retention time of 5.8 to 7.6 min) to obtain the title compound **105** (25.98 mg). LC-MS (ESI): m/z 518.0 [M+H]⁺. ¹H NMR (400 MHz, DMSO-*d6*) δ 9.11 (s, 1H), 8.23 (d, *J* = 3.0 Hz, 1H), 7.52 (dd, *J* = 15.8, 9.0 Hz, 1H), 7.41 (d, *J* = 3.0 Hz, 1H), 7.26 (ddd, *J* = 11.9, 9.1, 2.5 Hz, 1H), 7.18 (d, *J* = 8.6 Hz, 2H), 7.02 - 6.96 (m, 1H), 5.40 (d, *J* = 14.5 Hz, 1H), 5.01 (s, 1H), 4.79 (d, *J* = 8.9 Hz, 2H), 2.00 (d, *J* = 1.1 Hz, 3H), 1.82 (s, 3H).

### Example 106: Preparation of compound 106

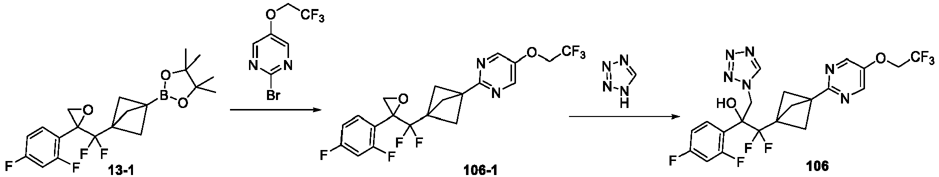

### Preparation of compound 106-1

Compound **13-1** (2 g, 5.03 mmol) was added to a microwave reaction tube and dissolved in a mixed solvent of cyclopentyl methyl ether (16 mL) and water (4 mL), then 2-bromo-5-(2,2,2-trifluoroethoxy)pyrimidine (1.93 g, 7.54 mmol), palladium acetate (114 mg, 0.5 mmol), butyldi-1-adamantylphosphine (360 mg, 1.0 mmol), cesium carbonate (4.92 g, 15.1 mmol), and cuprous oxide (720 mg, 5.03 mmol) were sequentially added thereto. The reaction system was replaced with nitrogen three times, and reacted under microwave irradiation at 120°C for 16 hours. The reaction system was cooled, then extracted with EtOAc (50 mL × 3), and the organic phases were combined, dried, and concentrated to obtain a crude product. The crude product was purified by normal-phase silica gel column chromatography (EtOAc/PE = 0 to 30%) to obtain the title compound **106-1** (150 mg, yield: 6.7%). LC-MS (ESI): m/z 449.2 [M+H]⁺.

### Preparation of compound 106

Compound **106-1** (128 mg, 0.29 mmol) was dissolved in DMF solution (5 mL), then tetrazole (80 mg, 1.14 mmol) and potassium carbonate (158 mg, 1.14 mmol) were added thereto, and the reaction system was stirred at 80°C for 16 hours in a sealed tube. After the reaction system was cooled, the reaction mixture was filtered, and the crude filtrate was purified by preparative separation (preparation method: mobile phase: A: 0.1% formic acid aqueous solution; B: acetonitrile; chromatographic column: Agilent 10 Prep-C18 250 × 21.2 mm; column temperature: 25°C; gradient: 45% to 65% acetonitrile in 12 min; flow rate: 30 mL/min) to obtain the title compound **106** (33 mg, yield: 22%). LC-MS (ESI): m/z 519.2 [M+H]⁺. ¹H NMR (400 MHz, DMSO-*d6*) δ 9.14 (s, 1H), 8.56 (s, 2H), 7.63 - 7.49 (m, 1H), 7.37 - 7.24 (m, 1H), 7.20 (s, 1H), 7.03 (td, *J* = 8.4, 2.6 Hz, 1H), 5.22 (dd, *J* = 164.6, 14.4 Hz, 2H), 4.94 (q, *J* = 8.8 Hz, 2H), 2.09 (dd, *J* = 9.4, 1.6 Hz, 3H), 1.88 (dd, *J* = 9.4, 1.6 Hz, 3H). ¹⁹F NMR (400 MHz, DMSO-*d6*) δ -72.66 (s, 3F), -102.91 - -103.13 (m, 1F), -109.39 - -109.54 (m, 3F).

### Example 107: Preparation of compounds 107A and 107B

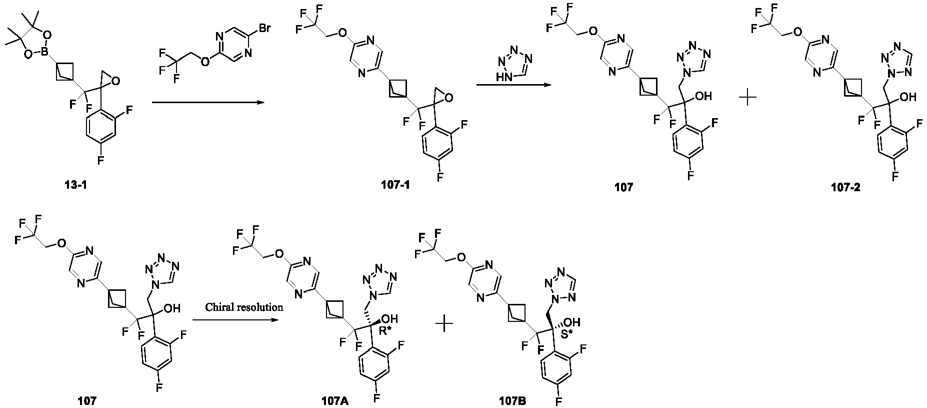

### Preparation of compound 107-1

Compound **13-1** (500 mg, 1.26 mmol) was dissolved in a mixed solvent of cyclopentyl methyl ether (10 mL) and water (2 mL), and then compound cataCXium A (90 mg, 0.25 mmol), cesium carbonate (1.23 g, 3.77 mmol), cuprous oxide (179 mg, 1.26 mmol), palladium acetate (28 mg, 0.13 mmol), and 2-bromo-5-(2, 2, 2-trifluoroethoxy)pyrazine (484 mg, 1.88 mmol) were sequentially added thereto. The reaction system was replaced with nitrogen three times and reacted at 120°C under microwave irradiation for 16 hours. After the reaction system was cooled, the reaction system was quenched with saturated ammonium chloride solution (5 mL), extracted with EtOAc (20 mL × 3), and the organic phases were combined, dried, and concentrated to obtain a crude product. The crude product was purified by normal-phase silica gel column chromatography (EtOAc/PE = 0 to 10%) to obtain the title compound **107-1** (160 mg, brown oil, yield: 28%). LC-MS (ESI): m/z 449.0 [M+H]⁺

### Preparation of compound 107

Compound **107-1** (160 mg, 0.35 mmol) was dissolved in DMF (3 mL), then 1H-tetrazole (125 mg, 1.78 mmol) and potassium carbonate (246 mg, 1.78 mmol) were added thereto, and the reaction system was reacted at 80°C for 16 hours in a sealed tube. After the reaction system was cooled, the reaction mixture was filtered, and the crude filtrate was purified by preparative separation (preparation method: chromatographic column: Agilent 10 Prep-C18 250 × 21.2 mm; mobile phase: A: 0.1% formic acid aqueous solution; B: acetonitrile; column temperature: 25°C; gradient: 55% to 75% acetonitrile in 12 min; flow rate: 30 mL/min) to obtain the title compound **107** (50 mg, yield: 27%, containing a pair of enantiomers) and the corresponding regioisomer compound **107-2** (20 mg, yield: 10%, containing a pair of enantiomers).

Compound **107:** LC-MS (ESI): m/z 519.0 [M+H]⁺. ¹H NMR (400 MHz, DMSO-*d6*) δ 9.14 (s, 1H), 8.36 (d, *J* = 1.4 Hz, 1H), 8.11 (d, *J* = 1.5 Hz, 1H), 7.60 - 7.49 (m, 1H), 7.35 - 7.25 (m, 1H), 7.20 (s, 1H), 7.08 - 6.98 (m, 1H), 5.43 (d, *J* = 14.5 Hz, 1H), 5.08 - 4.94 (m, 3H), 2.10 (dd, *J* = 9.3, 1.8 Hz, 3H), 1.89 (dd, *J* = 9.3, 1.8 Hz, 3H). ¹⁹F NMR (376 MHz, DMSO-*d6)* δ -72.26 (s, 3F), -101.09 - -103.60 (m, 1F), -109.42 (s, 1F), -109.48 - -109.59 (m, 2F).

Compound **107-2:** LC-MS (ESI): m/z 519.0 [M+H]⁺. ¹H NMR (400 MHz, DMSO-*d6*) δ 8.77 (s, 1H), 8.36 (d, *J* = 1.5 Hz, 1H), 8.11 (d, *J* = 1.4 Hz, 1H), 7.63 - 7.52 (m, 1H), 7.38 - 7.21 (m, 2H), 7.07 - 6.91 (m, 1H), 5.62 (d, *J* = 14.1 Hz, 1H), 5.25 (d, *J* = 14.2 Hz, 1H), 5.00 (q, *J* = 9.0 Hz, 2H), 2.09 (dd, *J* = 9.4, 1.9 Hz, 3H), 1.88 (dd, *J* = 9.4, 1.8 Hz, 3H). ¹⁹F NMR (376 MHz, DMSO-*d6*) δ -72.27 (s, 3F), -101.39 - -103.90 (m, 1F)), -108.39 - -109.37 (m, 2F), -109.87 (d, 1F).

### Preparation of compounds 107A and 107B

Compound **107** (50 mg) was subjected to SFC chiral preparative resolution (preparative separation method, instrument model: MG II preparative SFC (SFC-14); model of chromatographic column: ChiralCel OD, 250 × 30 mm I.D., 5 µm; mobile phase: A: CO₂ B: ethanol (0.1% NH₃H₂O); elution gradient: B 30%; flow rate: 70 mL/min; column pressure: 100 bar; column temperature: 38°C; detection wavelength: 220 nm; cycle: about 2 min) to obtain the title compounds **107A** (24 mg, single enantiomer) and **107B** (22 mg, single enantiomer).

Compound **107A:** Chiral analysis method (model of chromatographic column: ChiralCel OD, 150 × 4.6 mm I.D., 3 µm; mobile phase: A: CO₂ B: ethanol (0.05% DEA); elution gradient: B 5 to 40%%; flow rate: 2.5 mL/min; column temperature: 35°C; column pressure: 100 bar; detection wavelength: 220 nm; Rt = 1.174 min). LC-MS (ESI): m/z 519.2 [M+H]⁺. ¹H NMR (400 MHz, DMSO-*d*₆) δ 9.14 (s, 1H), 8.36 (d, *J* = 1.4 Hz, 1H), 8.11 (d, *J* = 1.5 Hz, 1H), 7.60 - 7.49 (m, 1H), 7.35 - 7.25 (m, 1H), 7.20 (s, 1H), 7.08 - 6.98 (m, 1H), 5.43 (d, *J* = 14.5 Hz, 1H), 5.08 - 4.94 (m, 3H), 2.10 (dd, *J* = 9.3, 1.8 Hz, 3H), 1.89 (dd, *J* = 9.3, 1.8 Hz, 3H). ¹⁹F NMR (376 MHz, DMSO-*d*₆) δ -72.26 (s, 3F), -101.09 - -103.60 (m, 1F), - 109.42 (s, 1F), -109.48 - -109.59 (m, 2F).

Compound **107B:** Chiral analysis method (model of chromatographic column: ChiralCel OD, 150 × 4.6 mm I.D., 3 µm; mobile phase: A: CO₂ B: ethanol (0.05% DEA); elution gradient: B 5 to 40%%; flow rate: 2.5 mL/min; column temperature: 35°C; column pressure: 100 bar; detection wavelength: 220 nm; Rt = 1.429 min). LC-MS (ESI): m/z 519.2 [M+H]⁺. ¹H NMR (400 MHz, DMSO-*d*₆) δ 9.14 (s, 1H), 8.36 (d, *J* = 1.4 Hz, 1H), 8.11 (d, *J* = 1.5 Hz, 1H), 7.60 - 7.49 (m, 1H), 7.35 - 7.25 (m, 1H), 7.20 (s, 1H), 7.08 - 6.98 (m, 1H), 5.43 (d, *J* = 14.5 Hz, 1H), 5.08 - 4.94 (m, 3H), 2.10 (dd, *J* = 9.3, 1.8 Hz, 3H), 1.89 (dd, *J* = 9.3, 1.8 Hz, 3H). ¹⁹F NMR (376 MHz, DMSO-*d*₆) δ -72.26 (s, 3F), -101.09 - -103.60 (m, 1F), - 109.42 (s, 1F), -109.48 - -109.59 (m, 2F).

### Example 108: Preparation of compound 108

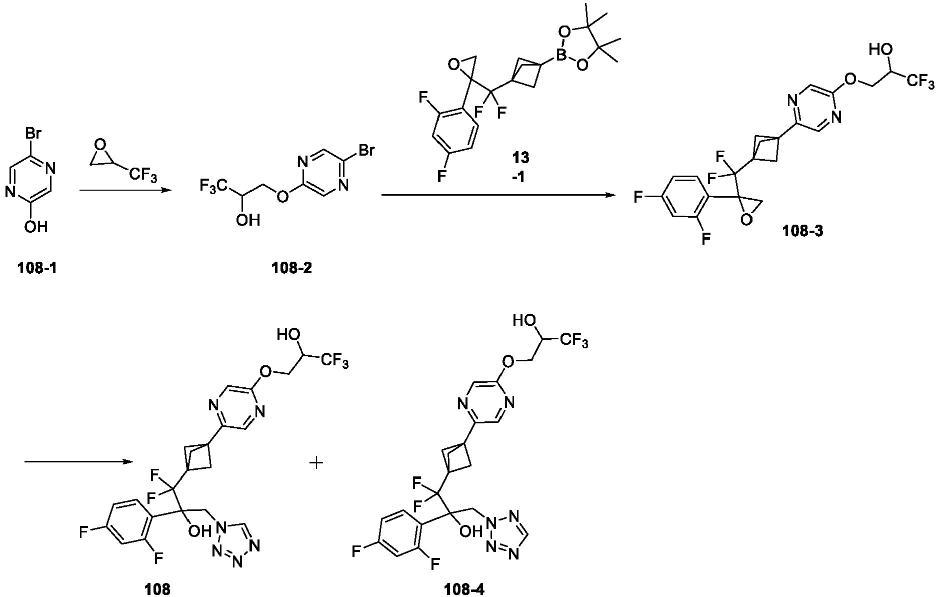

### Preparation of compound 108-2

Compound **108-1** (2 g, 11.43 mmol) was dissolved in acetonitrile (10 mL), then potassium carbonate (3.16 g, 22.86 mmol) and 1,1,1-trifluoro-2,3-epoxypropane (1.66 g, 14.86 mmol) were added thereto, and the reaction system was reacted at 50°C for 16 hours. After the reaction system was cooled, the reaction system was quenched with water (50 mL), extracted with EtOAc (20 mL × 3), and the organic phases were combined, dried, and concentrated to obtain a crude product. The crude product was purified by normal-phase silica gel column chromatography (EtOAc/PE = 0 to 10%) to obtain the title compound **108-2** (450 mg, yield: 13%). LC-MS (ESI): m/z 286.4 [M+H]⁺.

### Preparation of compound 108-3

Compound **13-1** (500 mg, 1.26 mmol) was dissolved in a mixed solvent of cyclopentyl methyl ether (10 mL) and water (2 mL), and then cataCXium A (90 mg, 0.25 mmol), cesium carbonate (1.23 g, 3.77mmol), cuprous oxide (179 mg, 1.26 mmol), palladium acetate (28 mg, 0.13 mmol), and compound **108-2** (484 mg, 1.88 mmol) were sequentially added thereto. The reaction system was replaced with nitrogen three times and reacted at 120°C under microwave irradiation for 16 hours. After the reaction system was cooled, the reaction system was quenched with saturated ammonium chloride solution (5 mL), extracted with EtOAc (20 mL × 3), and the organic phases were combined, dried, and concentrated to obtain a crude product. The crude product was purified by normal-phase silica gel column chromatography (EtOAc/PE = 0 to 10%) to obtain the title compound **108-3** (70 mg, brown oil, yield: 8%). LC-MS (ESI): m/z 479.0 [M+H]⁺.

### Preparation of compound 108

Compound **108-3** (70 mg, 0.15 mmol) was dissolved in DMF (1 mL), then 1H-tetrazole (51 mg, 0.73 mmol) and potassium carbonate (101 mg, 0.73 mmol) were added thereto, and the reaction system was reacted at 80°C for 16 hours in a sealed tube. After the reaction system was cooled, the reaction mixture was filtered, and the crude filtrate was purified by preparative separation (preparation method: chromatographic column: Agilent 10 Prep-C18 250 × 21.2 mm; mobile phase: A: 0.1% formic acid aqueous solution; B: acetonitrile; column temperature: 25°C; gradient: 40% to 70% acetonitrile in 12 min; flow rate: 30 mL/min) to obtain the title compound **108** (5 mg, yield: 6%, containing a pair of enantiomers) and the corresponding regioisomer compound **108-4** (5 mg, yield: 6%, containing a pair of enantiomers).

Compound **108:** LC-MS (ESI): m/z 549.4 [M+H]⁺. ¹H NMR (400 MHz, DMSO-*d*₆) δ 9.15 (s, 1H), 8.22 (d, *J* = 1.3 Hz, 1H), 8.07 (d, *J* = 1.4 Hz, 1H), 7.59 - 7.48 (m, 1H), 7.35 - 7.25 (m, 1H), 7.24 (s, 1H), 7.06 - 6.98 (m, 1H), 6.68 (d, *J* = 6.3 Hz, 1H), 5.43 (d, *J* = 14.6 Hz, 1H), 5.02 (d, *J* = 14.6 Hz, 1H), 4.50 - 4.44 (m, 1H), 4.44 - 4.37 (m, 1H), 4.37 - 4.30 (m, 1H), 2.08 (dd, *J* = 9.5, 1.8 Hz, 3H), 1.87 (dd, *J* = 9.4, 1.7 Hz, 3H). ¹⁹F NMR (376 MHz, DMSO-*d*₆) δ -76.15 (s, 3F), -100.63 - -104.89 (m, 1F), -108.54 - -111.43 (m, 3F).

Compound **108-4:** LC-MS (ESI): m/z 549.4 [M+H]⁺. ¹H NMR (400 MHz, DMSO-*d6*) δ 8.77 (s, 1H), 8.22 (s, 1H), 8.07 (s, 1H), 7.57 (q, *J* = 8.3 Hz, 1H), 7.27 (d, *J* = 5.7 Hz, 2H), 7.08 - 6.91 (m, 1H), 6.69 (d, *J* = 5.8 Hz, 1H), 5.61 (d, *J* = 14.2 Hz, 1H), 5.24 (d, *J* = 14.2 Hz, 1H), 4.46 (d, *J* = 10.5 Hz, 1H), 4.41 (s, 1H), 4.38 - 4.32 (m, 1H), 2.07 (d, *J* = 9.3 Hz, 3H), 1.85 (d, *J* = 9.3 Hz, 3H). ¹⁹F NMR (376 MHz, DMSO-*d6*) δ -76.16 (s, 3F), -100.86 - -104.66 (m, 1F), -108.09 - -109.38 (m, 2F), -109.38 - -110.06 (m, 1F).

### Example 109: Preparation of compound 109

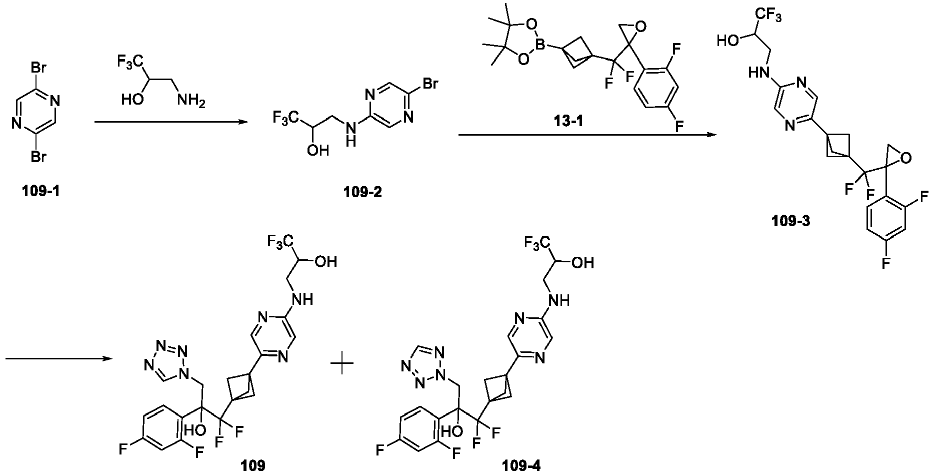

### Preparation of compound 109-2

2,5-Dibromopyrazine (1 g, 4.2 mmol) was dissolved in DMF (10 mL), then TEA (850 mg, 8.4 mmol) and 3-amino-1,1,1-trifluoro-2-propanol (596 mg, 4.62 mmol) were added thereto, and the reaction system was reacted at 100°C under microwave irradiation for 16 hours. After the reaction system was cooled, the reaction system was quenched with water (50 mL), extracted with EtOAc (20 mL × 3), and the organic phases were combined, dried, and concentrated to obtain a crude product. The crude product was purified by normal-phase silica gel column chromatography (EtOAc/PE = 0 to 10%) to obtain the title compound **109-2** (450 mg, yield: 37%). LC-MS (ESI): m/z 285.6 [M+H]⁺.

### Preparation of compound 109-3

Compound **13-1** (500 mg, 1.26 mmol) was dissolved in a mixed solvent of cyclopentyl methyl ether (10 mL) and water (2 mL), and then cataCXium A (90 mg, 0.25 mmol), cesium carbonate (1.23 g, 3.77mmol), cuprous oxide (179 mg, 1.26 mmol), palladium acetate (28 mg, 0.13 mmol), and compound **109-2** (484 mg, 1.88 mmol) were sequentially added thereto. The reaction was carried out at 120°C under microwave irradiation for 16 hours under argon atmosphere. After the reaction system was cooled, the reaction system was quenched with saturated ammonium chloride solution (5 mL), extracted with EtOAc (20 mL × 3), and the organic phases were combined, dried, and concentrated to obtain a crude product. The crude product was purified by normal-phase silica gel column chromatography (EtOAc/PE = 0 to 10%) to obtain the title compound **109-3** (50 mg, yield: 9%). LC-MS (ESI): m/z 477.6 [M+H]⁺.

### Preparation of compound 109

Compound **109-3** (60 mg, 0.11 mmol) was dissolved in DMF (1 mL), then 1H-tetrazole (39 mg, 0.56 mmol) and potassium carbonate (77 mg, 0.56 mmol) were added thereto, and the reaction system was reacted at 80°C for 16 hours in a sealed tube. After the reaction system was cooled, the reaction mixture was filtered, and the crude filtrate was purified by preparative separation (preparation method: chromatographic column: Agilent 10 Prep-C18 250 × 21.2 mm; mobile phase: A: 0.1% formic acid aqueous solution; B: acetonitrile; column temperature: 25°C; gradient: 35% to 65% acetonitrile in 12 min; flow rate: 30 mL/min) to obtain the title compound **109** (6 mg, yield: 10%, containing a pair of enantiomers) and the corresponding regioisomer compound **109-4** (2 mg, yield: 3%, containing a pair of enantiomers).

Compound **109:** LC-MS (ESI): m/z 548.2 [M+H]⁺. ¹H NMR (400 MHz, DMSO-*d6*) δ 9.14 (s, 1H), 7.89 (s, 1H), 7.79 (s, 1H), 7.62 - 7.44 (m, 1H), 7.35 - 7.24 (m, 2H), 7.18 (s, 1H), 7.07 - 6.95 (m, 1H), 6.45 (d, *J* = 6.4 Hz, 1H), 5.42 (d, *J* = 14.5 Hz, 1H), 5.01 (d, *J* = 14.5 Hz, 1H), 4.11 (s, 1H), 3.76 - 3.54 (m, 1H), 3.24 (s, 1H), 2.00 (d, *J* = 9.3 Hz, 3H), 1.78 (d, *J* = 9.3, 1.7 Hz, 3H). ¹⁹F NMR (376MHz, DMSO-*d6*) δ -76.80 (s, 3F), -102.15 - -103.29 (m, 1F), - 109.33 (d, 2F), -109.62 (d, 1F).

Regioisomer compound **109-4:** LC-MS (ESI): m/z 548.2 [M+H]⁺. ¹H NMR (400 MHz, DMSO-*d*₆) δ 8.76 (s, 1H), 7.89 (s, 1H), 7.79 (s, 1H), 7.62 - 7.49 (m, 1H), 7.35 - 7.23 (m, 2H), 7.21 (s, 1H), 7.06 - 6.95 (m, 1H), 6.46 (d, *J* = 6.5 Hz, 1H), 5.61 (d, *J* = 14.2 Hz, 1H), 5.23 (d, *J* = 14.2 Hz, 1H), 4.11 (s, 1H), 3.67 - 3.56 (m, 1H), 3.24 - 3.20 (m, 1H), 1.98 (d, *J* = 1.7 Hz,3H), 1.78 (d, *J* = 1.7 Hz, 3H). ¹⁹F NMR (376 MHz, DMSO-*d6*) δ -76.80 (s, 3F), - 102.51 - -102.95 (m, 1F), -108.57 - -109.52 (m, 2F), -109.96 (d, 1F).

### Example 110: Preparation of compound 110

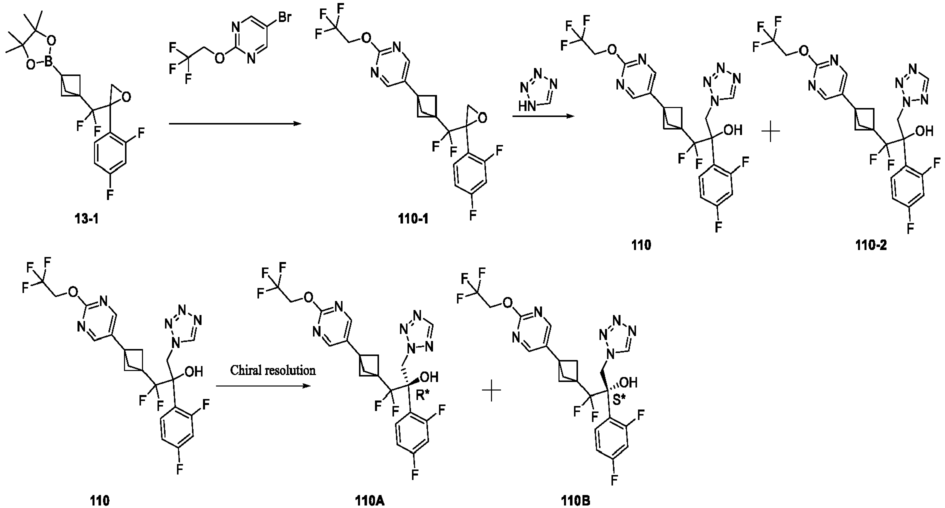

### Preparation of compound 110-1

Compound **13-1** (500 mg, 1.26 mmol) was dissolved in a mixed solvent of cyclopentyl methyl ether (10 mL) and water (2 mL), and then cataCXium A (90 mg, 0.25 mmol), cesium carbonate (1.23 g, 3.77mmol), cuprous oxide (179 mg, 1.26 mmol), palladium acetate (29 mg, 0.13 mmol), and 5-bromo-2-(2,2,2-trifluoroethoxy)pyrimidine (484 mg, 1.88 mmol) were sequentially added thereto. The reaction system was replaced with nitrogen three times and reacted at 120°C under microwave irradiation for 16 hours. After the reaction system was cooled and the reaction was completed, the reaction system was quenched with saturated ammonium chloride solution (5 mL), extracted with EtOAc (20 mL × 3), and the organic phases were combined, dried, and concentrated to obtain a crude product. The crude product was purified by normal-phase silica gel column chromatography (EtOAc/PE = 0 to 10%) to obtain the title compound **110-1** (160 mg, brown oil, yield: 28%). LC-MS (ESI): m/z 449.2 [M+H]⁺.

### Preparation of compound 110

Compound **110-1** (160 mg, 0.35 mmol) was dissolved in DMF (3 mL), then 1H-tetrazole (125 mg, 1.78 mmol) and potassium carbonate (246 mg, 1.78 mmol) were added thereto, and the reaction system was reacted at 80°C for 16 hours in a sealed tube. After the reaction system was cooled, the reaction mixture was filtered, and the crude filtrate was purified by preparative separation (preparation method: chromatographic column: Agilent 10 Prep-C18 250 × 21.2 mm; mobile phase: A: 0.1% formic acid aqueous solution; B: acetonitrile; column temperature: 25°C; gradient: 50% to 70% acetonitrile in 12 min; flow rate: 30 mL/min) to obtain the title compound **110** (50 mg, yield: 27%, containing a pair of enantiomers) and the corresponding regioisomer compound **110-2** (20 mg, yield: 10%, containing a pair of enantiomers).

Compound **110:** LC-MS (ESI): m/z 519.2 [M+H]⁺. ¹H NMR (400 MHz, DMSO-*d6*) δ 9.14 (s, 1H), 8.50 (s, 2H), 7.60 - 7.47 (m, 1H), 7.35 - 7.20 (m, 2H), 7.06 - 6.95 (m, 1H), 5.43 (d, *J* = 14.5 Hz, 1H), 5.09 - 4.92 (m, 3H), 2.10 (dd, *J* = 9.3, 1.8 Hz, 3H), 1.90 (dd, *J* = 9.3, 1.8 Hz, 3H). ¹⁹F NMR (376 MHz, DMSO-*d6*) δ -72.51 (m, 3F), -102.51 - -103.56 (m, 1F), - 109.37 (m, 1F), -109.47 (m, 1F), -109.49 - -109.62 (m 1F).

Regioisomer compound **110-2:** LC-MS (ESI): m/z 519.2 [M+H]⁺. ¹H NMR (400 MHz, DMSO-d6) δ 8.76 (s, 1H), 8.50 (s, 2H), 7.79 - 7.43 (m, 1H), 7.46 - 7.06 (m, 2H), 7.10 - 6.85 (m, 1H), 5.61 (d, *J* = 14.2 Hz, 1H), 5.24 (d, *J* = 14.3 Hz, 1H), 4.99 (q, *J* = 9.0 Hz, 2H), 2.09 (dd, *J* = 9.4, 1.8 Hz, 3H), 1.89 (dd, *J* = 9.4, 1.9 Hz, 3H). ¹⁹F NMR (376 MHz, DMSO-*d6)* δ -72.46 (d, 3F), -108.89 - -109.31 (m, 2F), -109.76 - -110.01 (m, 2F).

### Preparation of compounds 110A and 110B

Compound **110** (33 mg) was subjected to SFC chiral preparative resolution (preparative separation method, instrument model: MG II preparative SFC (SFC-14); model of chromatographic column: ChiralCel OX, 250 × 30 mm I.D., 5 µm; mobile phase: A: CO₂ B: ethanol (0.1% NH₃H₂O); elution gradient: B 15%; flow rate: 60 mL/min; column pressure: 100 bar; column temperature: 38°C; detection wavelength: 220 nm; cycle: about 7 min) to obtain the title compounds **110A** (13 mg, single enantiomer) and **110B** (16 mg, single enantiomer).

Compound **110A:** Chiral analysis method (model of chromatographic column: ChiralCel OX, 150 × 4.6 mm I.D., 3 µm; mobile phase: A: CO₂ B: ethanol (0.05% DEA); elution gradient: B 20%; flow rate: 2.5 mL/min; column temperature: 35°C; column pressure: 100 bar; detection wavelength: 220 nm; Rt = 1.735 min). LC-MS (ESI): m/z 519.2 [M+H]⁺. ¹H NMR (400 MHz, DMSO-*d*₆) δ 9.13 (s, 1H), 8.50 (s, 2H), 7.61 - 7.46 (m, 1H), 7.36 - 7.24 (m, 1H), 7.20 (s, 1H), 7.06 - 6.93 (m, 1H), 5.42 (d, *J* = 14.5 Hz, 1H), 5.07 - 4.92 (m, 3H), 2.10 (dd, *J* = 9.5, 1.8 Hz, 3H), 1.89 (dd, *J* = 9.5, 1.8 Hz, 3H). ¹⁹F NMR (376 MHz, DMSO-*d*₆) δ - 72.51 (m, 3F), -102.51 - -103.56 (m, 1F), -109.37 (m, 1F), -109.47 (m, 1F), -109.49 - -109.62 (m 1F).

Compound **110B:** Chiral analysis method (model of chromatographic column: ChiralCel OX, 150 × 4.6 mm I.D., 3 µm; mobile phase: A: CO₂ B: ethanol (0.05% DEA); elution gradient: B 20%; flow rate: 2.5 mL/min; column temperature: 35°C; column pressure: 100 bar; detection wavelength: 220 nm; Rt = 1.371 min). LC-MS (ESI): m/z 519.2 [M+H]⁺. ¹H NMR (400 MHz, DMSO-*d*₆) δ 9.13 (s, 1H), 8.50 (s, 2H), 7.61 - 7.46 (m, 1H), 7.36 - 7.24 (m, 1H), 7.20 (s, 1H), 7.06 - 6.93 (m, 1H), 5.42 (d, *J* = 14.5 Hz, 1H), 5.07 - 4.92 (m, 3H), 2.10 (dd, *J* = 9.5, 1.8 Hz, 3H), 1.89 (dd, *J* = 9.5, 1.8 Hz, 3H). ¹⁹F NMR (376 MHz, DMSO-d₆) δ - 72.51 (m, 3F), -102.51 - -103.56 (m, 1F), -109.37 (m, 1F), -109.47 (m, 1F), -109.49 - -109.62 (m 1F).

### Example 111: Preparation of compound 111

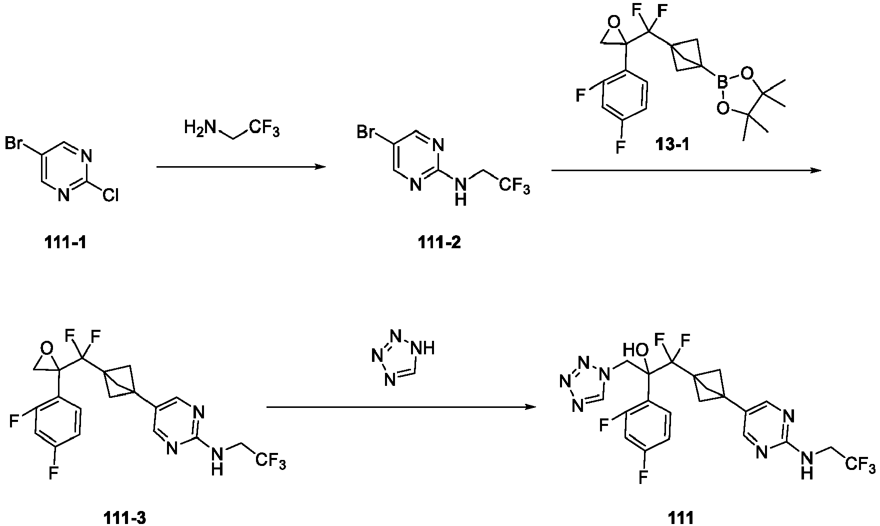

### Preparation of compound 111-2

Compound **111-1** (286 mg, 2 mmol) was dissolved in THF (20 mL), then 2,2,2-trifluoroethylamine (396 mg, 4 mmol) and N,N-diisopropylethylamine (516 mg, 4 mmol) were added thereto at 0°C. After stirring for 30 min, the reaction system was transferred to 80°C and reacted for 16 hours. The reaction system was cooled, then extracted with EtOAc (50 mL × 3), and the organic phases were combined, dried, and concentrated to obtain a crude product. The crude product was purified by normal-phase silica gel column chromatography (EtOAc/PE = 5 to 20%) to obtain the title compound **111-2** (200 mg, yield: 39%). ¹H NMR (400 MHz, DMSO-*d6*) δ 8.49 (s, 2H), 8.09 (t, *J* = 8.0 Hz, 1H), 4.15-4.06 (m, 2H).

### Preparation of compound 111-3

Compound **13-1** (398 mg, 1 mmol) was dissolved in a mixed solvent of cyclopentyl methyl ether (5 mL) and water (2 mL), and then compound **111-2** (200 mg, 0.78 mmol), cuprous oxide (111 mg, 0.78 mmol), palladium acetate (40.3 mg, 0.18 mmol), butyldi-1-adamantylphosphine (78 mg, 0.22 mmol), and cesium carbonate (1.0 g, 3 mmol) were sequentially added thereto. The reaction system was replaced with nitrogen three times, and then reacted under microwave irradiation at 120°C for 16 hours. The reaction system was extracted with EtOAc (50 mL × 3), and the organic phases were combined, dried, and concentrated to obtain a crude product. The crude product was purified by normal-phase silica gel column chromatography (EtOAc/PE = 0 to 20%) to obtain the title compound **111-3** (140 mg). LC-MS (ESI): m/z 448.2 [M+H]⁺.

### Preparation of compound 111

Compound **111-3** (140 mg, crude) was dissolved in DMF (10.0 mL), then potassium carbonate (79 mg, 0.57 mmol) and tetrazole (53.2 mg, 0.76 mmol) were added thereto, and the reaction system was stirred at 70°C for 16 hours in a sealed tube. After the reaction system was cooled, the reaction mixture was filtered, and the filtrate was purified by preparative separation (preparation method: mobile phase: A: 0.1% NH₄HCO₃ aqueous solution; B: acetonitrile; chromatographic column: Agilent 10 Prep-C18 250 × 21.2 mm; column temperature: 25°C; gradient: 45% to 65% acetonitrile in 12 min; flow rate: 30 mL/min) to obtain the title compound **111** (3.6 mg, two-step yield: 0.89%). LC-MS (ESI): m/z 518.2 [M+H]⁺. ¹H NMR (400 MHz, DMSO-*d6*) δ 9.13 (s, 1H), 8.45 (s, 2H), 7.53 (m, 1H), 7.53 (td, *J* = 9.0, 6.7 Hz, 1H), 7.30 (ddd, *J* = 12.0, 9.1, 2.7 Hz, 1H), 7.20 (s, 1H), 7.00 (td, *J* = 8.5, 2.7 Hz, 1H), 5.43 (d, *J* = 14.5 Hz, 1H), 5.01 (d, *J* = 14.6 Hz, 1H), 4.07 (m, 2H), 2.08 (dd, *J* = 9.4, 1.8 Hz, 3H), 1.88 (dd, *J* = 9.5, 1.8 Hz, 3H). ¹⁹F NMR (376 MHz, DMSO-*d6*) δ -70.97 (s, 3F), -102.98 - -103.19 (m, 1F), -109.28 - -109.38 (m, 2F), -109.58 - -109.61 (m, 1F).

### Example 112: Preparation of compound 112

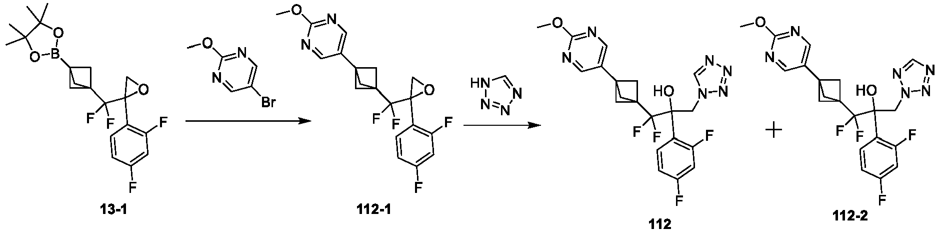

### Preparation of compound 112-1

Compound **13-1** (500 mg, 1.26 mmol) was dissolved in a mixed solvent of cyclopentyl methyl ether (5 mL) and water (2 mL), and then cataCXium A (90 mg, 0.25 mmol), cesium carbonate (1.23 g, 3.77 mmol), cuprous oxide (179 mg, 1.26 mmol), palladium acetate (29 mg, 0.13 mmol), and 5-bromo-2-methoxypyrimidine (484 mg, 1.88 mmol) were sequentially added thereto. The reaction system was replaced with nitrogen three times, and reacted under microwave irradiation at 120°C for 16 hours. After the reaction system was cooled, the reaction system was quenched with saturated ammonium chloride solution (5 mL), extracted with EtOAc (20 mL × 3), and the organic phases were combined, dried, and concentrated to obtain a crude product. The crude product was purified by normal-phase silica gel column chromatography (EtOAc/PE = 0 to 10%) to obtain the title compound **112-1** (160 mg, yield: 33%). LC-MS (ESI): m/z 380.8 [M+H]⁺.

### Preparation of compound 112

Compound **112-1** (160 mg, 0.35 mmol) was dissolved in DMF (3 mL), then 1H-tetrazole (125 mg, 1.78 mmol) and potassium carbonate (246 mg, 1.78 mmol) were added thereto, and the reaction system was reacted at 80°C for 16 hours in a sealed tube. After the reaction system was cooled, the reaction mixture was filtered, and the crude filtrate was purified by preparative separation (preparation method: chromatographic column: Agilent 10 Prep-C18 250 × 21.2 mm; mobile phase: A: 0.1% formic acid aqueous solution; B: acetonitrile; column temperature: 25°C; gradient: 45% to 65% acetonitrile in 12 min; flow rate: 30 mL/min) to obtain the title compound **112** (50 mg, yield: 26%) and the corresponding regioisomer compound **112-2** (20 mg, yield: 10%).

Compound **112:** LC-MS (ESI): m/z 450.8 [M+H]⁺. ¹H NMR (400 MHz, DMSO-*d6*) δ 9.14 (s, 1H), 8.41 (s, 2H), 7.58 - 7.48 (m, 1H), 7.33 - 7.19 (m, 2H), 7.04 - 6.96 (m, 1H), 5.42 (d, *J* = 14.6 Hz, 1H), 5.01 (d, *J* = 14.6 Hz, 1H), 3.86 (s, 3H), 2.11 - 2.05 (m, 3H), 1.90 - 1.84 (m, 3H). ¹⁹F NMR (376 MHz, DMSO-*d6*) δ -102.68 - -103.31 (m 1F), -109.16 - -109.48 (m 2F), -109.48 - -109.78 (m 1F).

Regioisomer compound **112-2:** LC-MS (ESI): m/z 450.8 [M+H]⁺. ¹H NMR (400 MHz, DMSO-*d6*) δ 8.76 (s, 1H), 8.41 (s, 2H), 7.62 - 7.49 (m, 1H), 7.35 - 7.16 (m, 2H), 7.05 - 6.93 (m, 1H), 5.61 (d, *J* = 14.1 Hz, 1H), 5.24 (d, *J* = 14.2 Hz, 1H), 3.85 (d, *J* = 1.5 Hz, 3H), 2.07 (d, *J* = 9.3 Hz, 3H), 1.87 (d, *J* = 9.3 Hz, 3H). ¹⁹F NMR (376 MHz, DMSO-*d6*) δ -102.39 - -102.92 (m, 1F), -108.80- -109.45 (m, 2F), -109.60 - -110.11 (m, 1F).

### Example 113: Preparation of compound 113

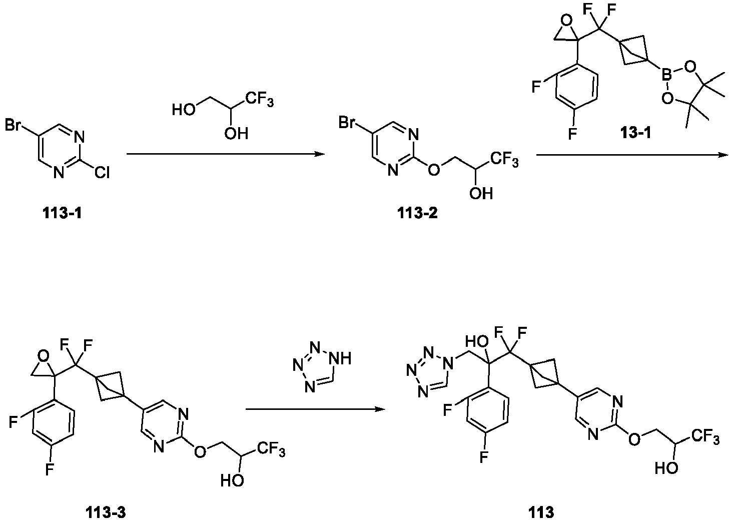

### Preparation of compound 113-2

3,3,3-Trifluoro-1,2-propanediol (673 mg, 5.18 mmol) was dissolved in DMF (20 mL), then NaH (30 mg, purity: 60%, 6 mmol) was added thereto at 0°C, and the reaction system was stirred for 30 min, then added with 5-bromo-2-chloropyrimidine (1 g, 5.18 mmol), and reacted at 80°C for 16 hours. The reaction system was cooled, then extracted with EtOAc (50 mL × 3), and the organic phases were combined, dried, and concentrated to obtain a crude product. The crude product was purified by normal-phase silica gel column chromatography (EtOAc/PE = 5 to 20%) to obtain the title compound **113-2** (560 mg, yellow solid, yield: 37.6%). LC-MS (ESI): m/z 288.6 [M+H]⁺.

### Preparation of compound 113-3

Compound **13-1** (550 mg, 1.43 mmol) was dissolved in a mixed solvent of cyclopentyl methyl ether (5 mL) and water (2 mL), and then compound **113-2** (300 mg, 1.11 mmol), cuprous oxide (158 mg, 1.11 mmol), palladium acetate (40.3 mg, 0.18 mmol), butyldi-1-adamantylphosphine (78 mg, 0.22 mmol), and cesium carbonate (1.3 g, 3.99 mmol) were sequentially added thereto. The reaction system was replaced with nitrogen three times, and then reacted under microwave irradiation at 120°C for 16 hours. The reaction system was cooled, then extracted with EtOAc (50 mL × 3), and the organic phases were combined, dried, and concentrated to obtain a crude product. The crude product was purified by normal-phase silica gel column chromatography (EtOAc/PE = 5 to 20%) to obtain the title compound **113-3** (160 mg, yellow oil). LC-MS (ESI): m/z 479.0 [M+H]⁺.

### Preparation of compound 113

Compound **113-3** (160 mg, crude) was dissolved in DMF (10 mL), then potassium carbonate (79 mg, 0.57 mmol) and tetrazole (53.2 mg, 0.76 mmol) were added thereto, and the reaction system was stirred at 70°C for 16 hours in a sealed tube. After the reaction system was cooled, the reaction mixture was filtered, and the filtrate was purified by preparative separation (preparation method: mobile phase: A: 0.1% NH₄HCO₃ aqueous solution; B: acetonitrile; chromatographic column: Agilent 10 Prep-C18 250 × 21.2 mm; column temperature: 25°C; gradient: 45% to 65% acetonitrile in 12 min; flow rate: 30 mL/min) to obtain the title compound **113** (4.85 mg, two-step yield: 0.80%).

LC-MS (ESI): m/z 549.2 [M+H]⁺. ¹H NMR (400 MHz, DMSO-*d6*) δ 9.13 (s, 1H), 8.45 (s, 2H), 7.53 (td, *J* = 9.0, 6.7 Hz, 1H), 7.30 (ddd, *J* = 12.0, 9.1, 2.7 Hz, 1H), 7.20 (s, 1H), 7.00 (td, *J* = 8.5, 2.7 Hz, 1H), 6.67 (d, *J* = 6.5 Hz, 1H), 5.43 (d, *J* = 14.5 Hz, 1H), 5.01 (d, *J* = 14.6 Hz, 1H), 4.59 - 4.29 (m, 3H), 2.08 (dd, *J* = 9.4, 1.8 Hz, 3H), 1.88 (dd, *J* = 9.5, 1.8 Hz, 3H). ¹⁹F NMR (376 MHz, DMSO-*d6*) δ -76.19 (s, 3F), -102.99 --103.20 (m, 1F), -109.41-109.50 (m, 2F), -109.52 - -109.54 (m, 1F).

### Example 114: Preparation of compound 114

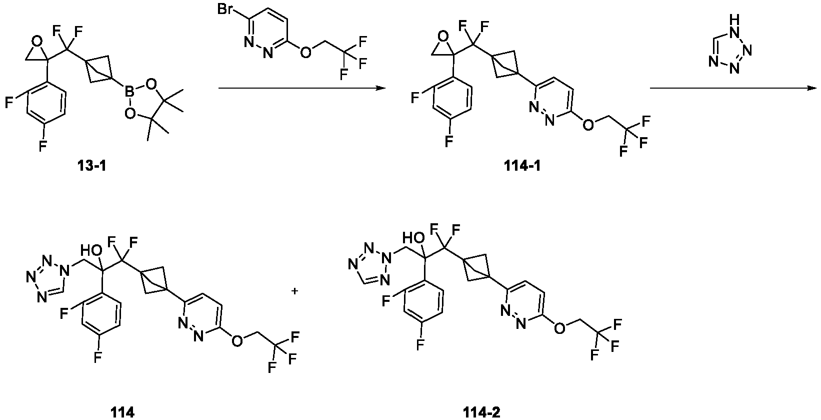

### Preparation of compound 114-1

Compound **114-1** (1.0 g, 2.51 mmol) was dissolved in a mixed solvent of cyclopentyl methyl ether (8 mL) and water (2 mL), then 3-bromo-6-(2,2,2-trifluoroethoxy)pyridazine (640 mg, 2.51 mmol), Cu₂O (359 mg, 2.51 mmol), Pd(OAc)₂ (56 mg, 0.25 mmol), cataCxium A (180 mg, 0.50 mmol), and Cs₂CO₃ (2.45 g, 7.53 mmol) were sequentially added thereto, and the reaction system was reacted at 120°C for 16 hours. After the reaction system was cooled, the reaction mixture was filtered, and the crude filtrate was purified by preparative separation (chromatographic column: Agilent 10 Prep-C18 250 × 21.2 mm; column temperature: 25°C; mobile phase: water (0.1% FA)-acetonitrile; proportion of acetonitrile in the mobile phase: 55% to 75% in 12 min; flow rate: 30 mL/min) to obtain the title compound **114-1** (30 mg, yield: 3%). LC-MS (ESI): m/z 449.2 [M+H]⁺.

### Preparation of compound 114

Compound **114-1** (16 mg, 0.036 mmol) was dissolved in DMF (1 mL), then 1H-tetrazole (10 mg, 0.144 mmol) and K₂CO₃ (20 mg, 0.144 mmol) were added thereto, and the reaction system was reacted at 75°C for 16 hours. After the reaction system was cooled, the reaction mixture was filtered, and the crude filtrate was purified by preparative separation (chromatographic column: Agilent 10 Prep-C18 250 × 21.2 mm; column temperature: 25°C; mobile phase: water (0.1% FA)-acetonitrile; proportion of acetonitrile in the mobile phase: 45% to 65% in 12 min; flow rate: 30 mL/min) to obtain compound **114** (5.5 mg, yield: 34%) and **114-2** (1 mg, yield: 6%).

Compound **114:** LC-MS (ESI): m/z 519.2 [M+H]⁺. ¹H NMR (400 MHz, DMSO-*d6*) δ 9.14 (d, *J* = 1.1 Hz, 1H), 7.62 (d, *J* = 9.1 Hz, 1H), 7.60 - 7.48 (m, 1H), 7.41 - 7.16 (m, 3H), 7.09 - 6.93 (m, 1H), 5.43 (d, *J* = 14.5 Hz, 1H), 5.12 (q, *J* = 9.0 Hz, 2H), 5.02 (d, *J* = 14.6 Hz, 1H), 2.14 (dd, *J* = 9.5, 1.8 Hz, 3H), 1.92 (dd, *J* = 9.5, 1.7 Hz, 3H). ¹⁹F NMR (376 MHz, DMSO-*d6*) δ -72.29 (s, 3F), -103.09 (d, 2F), -109.48 (m, 2F).

Compound **114-2:** LC-MS (ESI): m/z 519.2 [M+H]⁺. ¹H NMR (400 MHz, DMSO-*d6*) δ 8.77 (s, 1H), 7.67 - 7.51 (m, 2H), 7.37 - 7.23 (m, 3H), 7.06 - 6.95 (m, 1H), 5.62 (d, *J =* 14.2 Hz, 1H), 5.25 (d, *J* = 14.2 Hz, 1H), 5.12 (q, *J* = 9.0 Hz, 2H), 2.12 (dd, *J* = 9.4, 1.8 Hz, 3H), 1.91 (dd, *J* = 9.4, 1.8 Hz, 3H). ¹⁹F NMR (376 MHz, DMSO-*d6*) δ -72.29 (s, 3F), -103.09 (d, 2F), -109.48 (m, 2F).

### Example 115: Preparation of compound 115

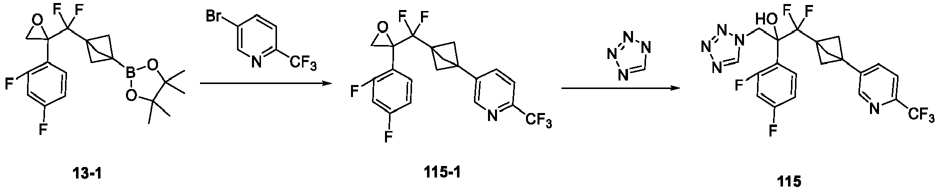

### Preparation of compound 115-1

Compound **13-1** (150 mg, 0.37 mmol) was dissolved in a mixed solvent of methylcyclopentyl ether solution (6 mL) and water (2 mL), and then 5-bromo-2-trifluoromethylpyridine (100 mg, 0.44 mmol), cuprous oxide (52.9 mg, 0.37 mmol), palladium acetate (12.4 mg, 0.055 mmol), butyldi-1-adamantylphosphine (35.8 mg, 0.1 mmol), and cesium carbonate (362 mg, 1.11 mmol) were sequentially added thereto. The reaction system was replaced with nitrogen three times, and reacted under microwave irradiation at 120°C for 16 hours. After the reaction system was cooled, the reaction mixture was poured into water (20 mL) and extracted with EtOAc (15 mL × 3), and the organic phases were combined, concentrated, and subjected to column chromatography (PE: EA = 20:1 to 5:1) to obtain the title compound **115-1** (80 mg, yield: 51.8%).

### Preparation of compound 115

Compound **115-1** (80 mg, 0.19 mmol) was dissolved in DMF (10.0 mL), then potassium carbonate (79 mg, 0.57 mmol) and tetrazole (53.2 mg, 0.76 mmol) were added thereto, and the reaction system was stirred at 70°C for 16 hours in a sealed tube. After the reaction system was cooled, the reaction mixture was filtered, and the crude filtrate was purified by preparative separation (preparation method: mobile phase: A: 0.1% NH₄HCO₃ aqueous solution; B: acetonitrile; chromatographic column: Agilent 10 Prep-C18 250 × 21.2 mm; column temperature: 25°C; gradient: 50% to 70% acetonitrile in 12 min; flow rate: 30 mL/min) to obtain the title compound **115** (24 mg, yield: 25.9%, containing a pair of enantiomers).

LC-MS (ESI): m/z 487.1 [M+H]⁺. ¹H NMR (400 MHz, DMSO-d6) δ 9.14 (s, 1H), 8.66 - 8.57 (m, 1H), 7.90 - 7.78 (m, 2H), 7.63 - 7.52 (m, 1H), 7.37 - 7.27 (m, 1H), 7.21 (s, 1H), 7.08 - 6.94 (m, 1H), 5.43 (d, *J* = 14.5 Hz, 1H), 5.02 (d, *J* = 14.6 Hz, 1H), 2.14 (dd, *J* = 9.4, 1.8 Hz, 3H), 1.93 (dd, *J* = 9.5, 1.8 Hz, 3H). ¹⁹F NMR (376 MHz, DMSO-d6) δ -66.28-66.77 (m, 3F), -102.93 --103.15 (m, 1F), -109.25 -109.54 (m, 2F), -109.71 - -101.07 (m, 1F).

### Example 116: Preparation of compounds 116 and 116-4

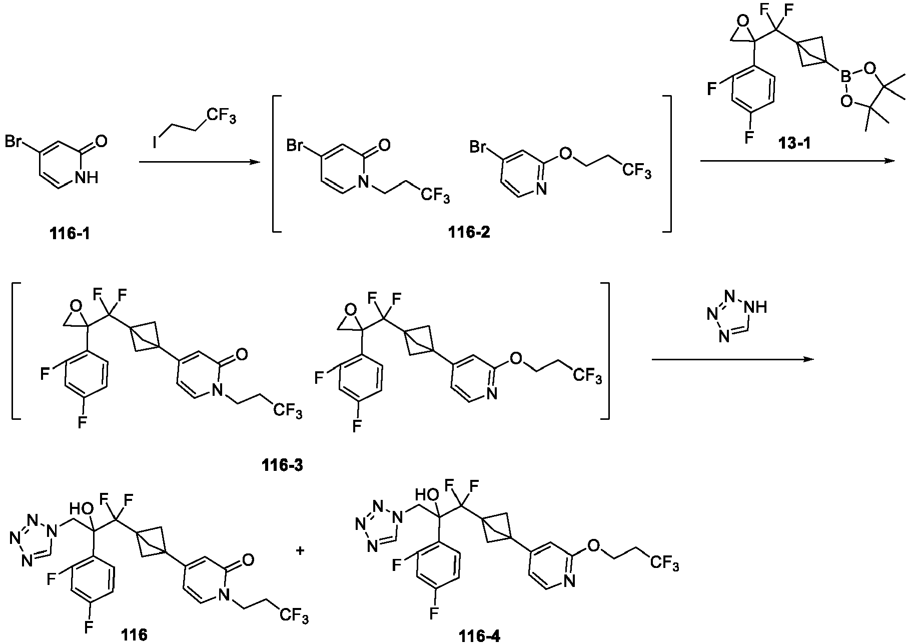

### Preparation of compound 116-2

Compound **116-1** (1 g, 5.7 mmol) was dissolved in THF (20 mL), then cesium carbonate (23.7 g, 11.4 mmol) and 1,1,1-trifluoro-3-iodopropane (3.6 g, 13.68 mmol) were added thereto, and the reaction system was replaced with nitrogen three times and reacted at 80°C for 16 hours. The reaction system was cooled, then extracted with EtOAc (50 mL × 3), and the organic phases were combined, dried, and concentrated to obtain a crude product. The crude product was purified by normal-phase silica gel column chromatography (EtOAc/PE = 5 to 20%) to obtain the title compound **116-2** (560 mg, yield: 36.3%) as a yellow solid mixture. LC-MS (ESI): m/z 271.2 [M+H]⁺.

### Preparation of compound 116-3

Compound **13-1** (530 mg, 1.33 mmol) was dissolved in a mixed solvent of cyclopentyl methyl ether (5 mL) and water (2 mL), and then compound **116-2** (300 mg, 1.11 mmol), cuprous oxide (158 mg, 1.11 mmol), palladium acetate (40.3 mg, 0.18 mmol), butyldi-1-adamantylphosphine (78 mg, 0.22 mmol), and cesium carbonate (1.3 g, 3.99 mmol) were sequentially added thereto. The reaction system was replaced with nitrogen three times, and then reacted under microwave irradiation at 120°C for 16 hours. The reaction system was cooled, then extracted with EtOAc (50 mL × 3), and the organic phases were combined, dried, and concentrated to obtain a crude product. The crude product was purified by normal-phase silica gel column chromatography (EtOAc/PE = 5 to 20%) to obtain the title compound **116-3** (180 mg, crude, yellow oil), which was directly used in the next reaction step. LC-MS (ESI): m/z 462.2 [M+H]⁺.

### Preparation of compounds 116 and 116-4

Compound **116-3** (180 mg, crude) was dissolved in DMF (10 mL), then potassium carbonate (79 mg, 0.57 mmol) and tetrazole (53.2 mg, 0.76 mmol) were added thereto, and the reaction system was reacted at 70°C for 16 hours in a sealed tube. After the reaction system was cooled, the reaction mixture was filtered, and the filtrate was purified by preparative separation (preparation method: mobile phase: A: 0.1% NH₄HCO₃ aqueous solution; B: acetonitrile; chromatographic column: Agilent 10 Prep-C18 250 × 21.2 mm; column temperature: 25°C; gradient: 40% to 60% acetonitrile in 12 min; flow rate: 30 mL/min) to obtain the title compounds **116** (74.8 mg, two-step yield: 12.6%) and **116-4** (29 mg, two-step yield: 4.9%).

Compound **116:** LC-MS (ESI): m/z 532.2 [M+H]⁺. ¹H NMR (400 MHz, DMSO-*d6*) δ 9.20 - 9.02 (m, 1H), 7.68 - 7.59 (m, 1H), 7.59 - 7.48 (m, 1H), 7.33 - 7.14 (m, 2H), 7.00 (td, *J* = 8.7, 2.5 Hz, 1H), 6.08 (d, *J* = 5.8 Hz, 2H), 5.41 (d, *J* = 14.4 Hz, 1H), 5.00 (d, *J* = 14.5 Hz, 1H), 4.05 (t, *J* = 7.0 Hz, 2H), 2.66 (dt, *J* = 11.2, 6.9 Hz, 2H), 2.04 - 1.94 (m, 3H), 1.83 - 1.71 (m, 3H). ¹⁹F NMR (376 MHz, DMSO-*d6*) δ -63.55 - -64.06 (m, 3F), -102.95 - -103.17 (m, 1F), -108.19 - -108.35 (m, 2F), -109.47 - -109.57 (m, 1F).

Compound **116-4:** LC-MS (ESI): m/z 532.2 [M+H]⁺. ¹H NMR (400 MHz, DMSO-*d6*) δ 8.76 (s, 1H), 7.66 - 7.61 (m, 1H), 7.55 (td, *J* = 9.0, 6.8 Hz, 2H), 6.99 (td, *J* = 8.6, 2.8 Hz, 1H), 6.54 - 6.30 (m, 1H), 6.08 (d, *J* = 7.0 Hz, 2H), 5.60 (d, *J* = 14.2 Hz, 1H), 5.32 - 5.21 (m, 1H), 4.05 (t, *J* = 7.0 Hz, 2H), 2.66 (dt, *J* = 11.5, 6.9 Hz, 2H), 1.97 (dd, *J* = 9.4, 1.7 Hz, 3H), 1.76 (dd, *J* = 9.5, 1.7 Hz, 3H). ¹⁹F NMR (376 MHz, DMSO-*d6*) δ -63.55 - -64.06 (m, 3F), - 102.95 - -103.17 (m, 1F), -109.47 - -109.57 (m, 2F), -109.47 - -109.57 (m, 1F).

### Example 117: Preparation of compound 117

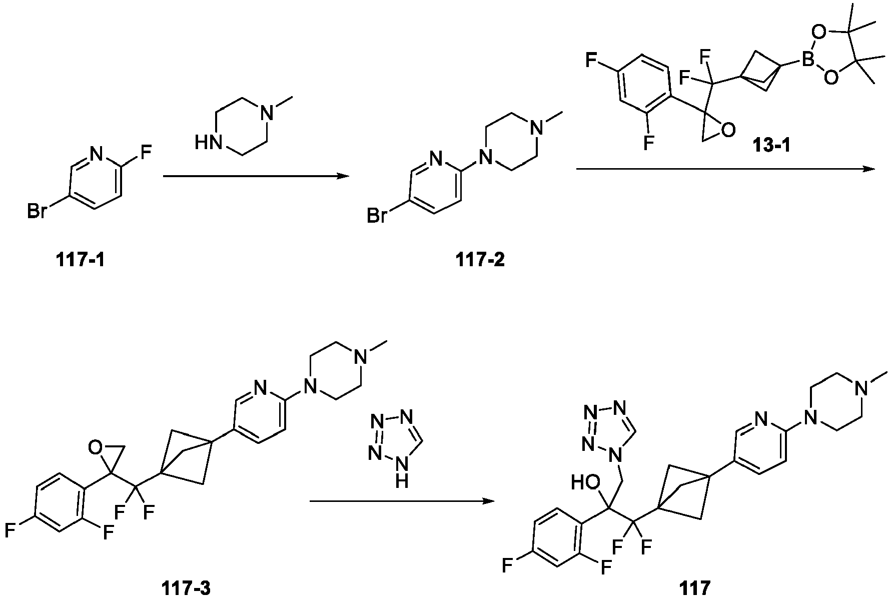

### Preparation of compound 117-2

Compound **117-1** (600 mg, 3.41 mmol) was dissolved in DMF (10 mL), then N-methylpiperazine (375 mg, 3.75 mmol) and triethylamine (690 mg, 6.82 mmol) were added thereto, and the reaction system was reacted at 90°C for 16 hours. The reaction system was cooled, then extracted with EtOAc (50 mL × 3), and the organic phases were combined, dried, and concentrated to obtain a crude product. The crude product was purified by normal-phase silica gel column chromatography (EtOAc/PE = 0 to 100%) to obtain the title compound **117-2** (320 mg, pale yellow solid, yield: 37%). LC-MS (ESI): m/z 255.9 [M+H]⁺.

### Preparation of compound 117-3

Compound **13-1** (320 mg, 0.80 mmol) was dissolved in a mixed solvent of cyclopentyl methyl ether (5 mL) and water (1 mL), and then Catacxium A (57 mg, 0.161 mmol), cesium carbonate (782 mg, 2.412 mmol), cuprous oxide (104 mg, 0.804 mmol), palladium acetate (162 mg, 0.724 mmol), and compound **117-2** (394 mg, 1.206 mmol) were sequentially added thereto. The reaction system was replaced with nitrogen three times, and reacted under microwave irradiation at 120°C for 10 hours. The reaction system was cooled, then extracted with EtOAc (50 mL × 3), and the organic phases were combined, dried, and concentrated to obtain a crude product. The crude product was purified by normal-phase silica gel column chromatography (EtOAc/PE = 0 to 100%) to obtain the title compound **117-3** (45 mg, pale yellow solid, yield: 11%). LC-MS (ESI): m/z 448.1 [M+H]⁺.

### Preparation of compound 117

Compound **117-3** (45 mg, 0.101 mmol) was dissolved in DMF (3 mL), then tetrazole (15 mg, 0.201 mmol) and potassium carbonate (21 mg, 0.151 mmol) were added thereto, and the reaction system was reacted at 80°C for 16 hours in a sealed tube. After the reaction system was cooled, the reaction mixture was filtered, and the filtrate was purified by preparative separation (preparation method: mobile phase: A: 0.1% formic acid aqueous solution; B: acetonitrile; chromatographic column: Sunfire C18 21.2 * 250 mm * 10 µm; column temperature: 25°C; gradient: 31% to 31% acetonitrile in 8.5 to 9 min; flow rate: 20 mL/min) to obtain the title compound **117** (11.55 mg, yield: 22%).

Compound **117:** LC-MS (ESI): m/z 518.1 [M+H]⁺. ¹H NMR (400 MHz, DMSO-76) δ 9.13 (s, 1H), 7.86 (d, *J* = 2.1 Hz, 1H), 7.59 - 7.48 (m, 1H), 7.36 - 7.21 (m, 2H), 7.15 (s, 1H), 7.00 (td, *J* = 8.5, 2.5 Hz, 1H), 6.72 (d, *J* = 8.8 Hz, 1H), 5.42 (d, *J* = 14.5 Hz, 1H), 5.01 (d, *J* = 14.6 Hz, 1H), 3.39 (d, *J* = 5.1 Hz, 4H), 2.35 (dd, *J* = 12.4, 7.3 Hz, 4H), 2.19 (s, 3H), 1.98 (dd, *J* = 9.4, 1.3 Hz, 3H), 1.77 (dd, *J* = 9.4, 1.3 Hz, 3H).

### Example 118: Preparation of compound 118

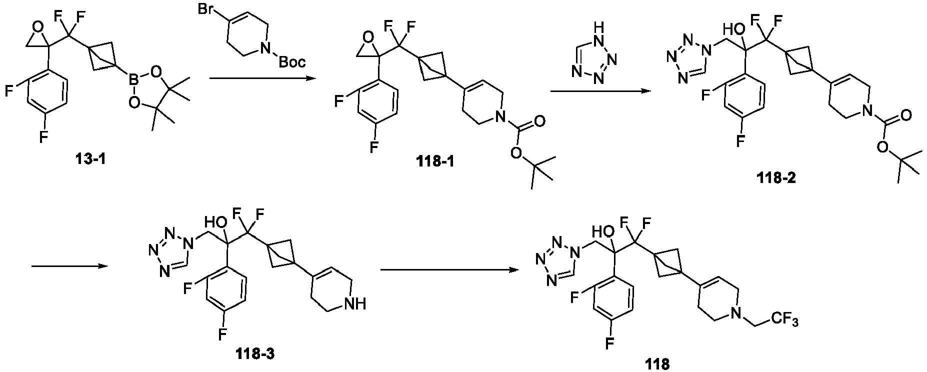

### Preparation of compound 118-1

Compound **13-1** (1.0 g, 2.51 mmol) was added to a microwave tube and dissolved in a mixed solvent of methylcyclopentyl ether solution (8 mL) and water (2 mL), then tert-butyl 4-bromo-5,6-dihydropyridine-1(2H)-carboxylate (987 mg, 3.77 mmol), Cu₂O (359 mg, 2.51 mmol), Pd(OAc)₂ (56 mg, 0.25 mmol), Catacxium A (180 mg, 0.50 mmol), and Cs₂CO₃ (2.45 g, 7.53 mmol) were sequentially added thereto. The reaction system was replaced with nitrogen three times and reacted at 120°C for 16 hours. After the reaction system was cooled, the reaction mixture was filtered, and the crude filtrate was purified by preparative separation (chromatographic column: Agilent 10 Prep-C18 250 × 21.2 mm; column temperature: 25°C; mobile phase: water (0.1% FA)-acetonitrile; proportion of acetonitrile in the mobile phase: 65% to 85% in 12 min; flow rate: 30 mL/min) to obtain the title compound **118-1** (70 mg, yield: 6%). LC-MS (ESI): m/z 398.4 [M+H-56]⁺.

### Preparation of compound 118-2

Compound **118-1** (70 mg, 0.15 mmol) was added to a sealed tube and dissolved in DMF (1 mL) solvent, then tetrazole (53 mg, 0.75 mmol) and K₂CO₃ (83 mg, 0.60 mmol) were added thereto, and the reaction system was reacted at 80°C for 16 hours. The reaction system was cooled, then extracted with EtOAc (50 mL × 3), and the organic phases were combined, dried, and concentrated to obtain a crude product. The crude product was purified by normal-phase silica gel column chromatography (EtOAc/PE = 0 to 100%) to obtain the title compound **118-2** (30 mg, yield: 38%). LC-MS (ESI): m/z 523.80 [M+H]⁺.

### Preparation of compound 118-3

Compound **118-2** (30 mg, 0.06 mmol) was dissolved in DCM (1 mL), then HCl-Dioxane solution (1 mL) was added dropwise to the reaction flask, and the reaction system was reacted at room temperature for 16 hours. The reaction mixture was evaporated to dryness by rotary evaporation to obtain 20 mg of crude title compound **118-3,** which was used directly in the next step.

### Preparation of compound 118

Compound **118-3** (20 mg, 0.05 mmol) was added to a microwave reaction tube and dissolved in THF (1 mL), then 2,2,2-trifluoroethyl trifluoromethanesulfonate (15 mg, 0.07 mmol) and K₂CO₃ (21 mg, 0.15 mmol) were added thereto, and the reaction system was reacted at 35°C for 16 hours. The reaction mixture was filtered, and the crude filtrate was purified by preparative separation (chromatographic column: Welch Xtimate^{®} C18 21.2 × 250 mm; column temperature: 25°C; mobile phase: water (10 mM/L NH₄HCO₃)-acetonitrile; proportion of acetonitrile in the mobile phase: 45% to 75% in 12 min; flow rate: 30 mL/min) to obtain the title compound **118** (5 mg).

Compound **118:** LC-MS (ESI): m/z 506.0 [M+H]⁺. ¹H NMR (400 MHz, Chloroform-*d*) δ 8.58 (s, 1H), 7.64 - 7.54 (m, 1H), 6.89 - 6.77 (m, 2H), 5.43 (d, *J* = 14.6 Hz, 1H), 5.31 - 5.24 (m, 1H), 5.00 (d, *J* = 14.6 Hz, 1H), 3.14 (q, *J* = 2.9 Hz, 2H), 3.01 (q, *J* = 9.6 Hz, 2H), 2.72 (t, *J* = 5.7 Hz, 2H), 2.01 (s, 1H), 1.99 - 1.92 (m, 2H), 1.78 (dd, *J* = 9.4, 1.8 Hz, 3H), 1.58 (dd, *J* = 9.5, 1.8 Hz, 3H). ¹⁹F NMR (376 MHz, Chloroform-J) δ -69.06 (s, 3F), - 105.00 (m, 1F), -107.25 (d, *J* = 9.7 Hz, 1F), -109.29 - -111.91 (m, 2F).

### Example 119: Preparation of compound 119

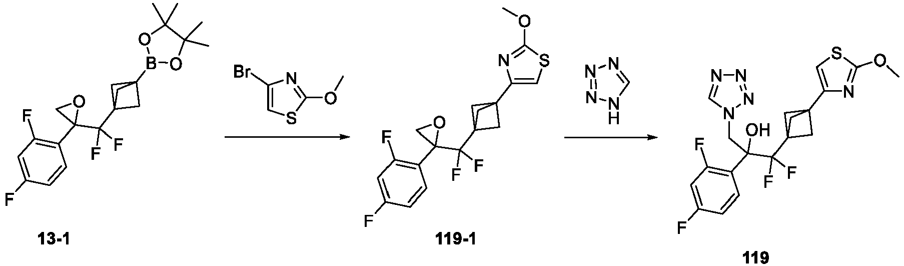

### Preparation of compound 119-1

Compound **13-1** (1 g, 2.5 mmol) was dissolved in a mixed solvent of cyclopentyl methyl ether (8 mL) and water (1 mL), and then compound butyldi-1-adamantylphosphine (cataCXium A, CAS: 321921-71-5, 180 mg, 0.5 mmol), cesium carbonate (2.4 g, 7.5 mmol), cuprous oxide (360 mg, 2.5 mmol), palladium acetate (60 mg, 0.25 mmol), and 4-bromo-2-methoxythiazole (730 mg, 3.8 mmol) were sequentially added thereto. The reaction system was replaced with nitrogen three times, and reacted under microwave irradiation at 120°C for 12 hours. The reaction system was cooled, then extracted with EtOAc (50 mL × 3), and the organic phases were combined, dried, and concentrated to obtain a crude product. The crude product was purified by normal-phase silica gel column chromatography (EtOAc/PE = 0 to 5%) to obtain the title compound **119-1** (110 mg, brown oil). LC-MS (ESI): m/z 386.2 [M+H]⁺.

### Preparation of compound 119

Compound **119-1** (110 mg, 0.29 mmol) was dissolved in DMF (1.5 mL), then 1H-tetrazole (102 mg, 1.4 mmol) and potassium carbonate (158 mg, 1.1 mmol) were added thereto, and the reaction system was reacted at 75°C for 16 hours in a sealed tube. After the reaction system was cooled, the reaction mixture was filtered, and the crude filtrate was purified by preparative separation (preparation method: chromatographic column: Agilent 10 Prep-C18 250 × 21.2 mm; mobile phase: A: 0.1% formic acid aqueous solution; B: acetonitrile; column temperature: 25°C; gradient: 45% to 65% acetonitrile 12 min; flow rate: 30 mL/min) to obtain the title compound **119** (3 mg, containing a pair of enantiomers). LC-MS (ESI): m/z 456.0 [M+H]⁺. ¹H NMR (400 MHz, Chloroform-*d*) δ 8.51 (s, 1H), 7.64 (td, *J* = 9.0, 6.3 Hz, 1H), 7.01 - 6.72 (m, 2H), 6.23 (s, 1H), 5.44 (d, *J* = 14.6 Hz, 1H), 5.03 (d, *J* = 14.7 Hz, 1H), 4.00 (s, 3H), 3.83 (s, 1H), 2.07 (dd, *J* = 9.3, 1.9 Hz, 3H), 1.89 (dd, *J* = 9.4, 1.8 Hz, 3H). ¹⁹F NMR (376 MHz, Chloroform-*d*) δ -105.41 (m, 1 F), -106.94 (d, *J* = 8.9 Hz, 1 F), -110.28 (d, *J* = 47.2 Hz, 1 F), -110.48 (d, *J* = 15.2 Hz, 1 F).

### Example 120: Preparation of compound 120

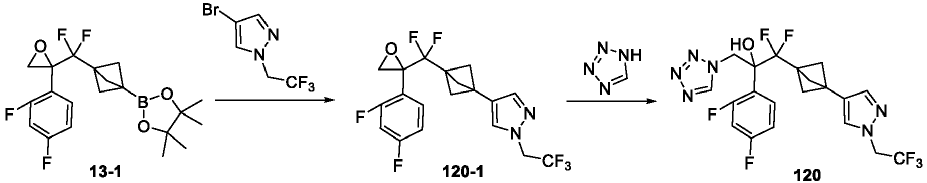

### Preparation of compound 120-1

Compound **13-1** (1.0 g, 2.51 mmol) was dissolved in a mixed solvent of cyclopentyl methyl ether (8 mL) and water (2 mL), then 4-bromo-1-(2,2,2-trifluoroethyl)-1H-pyrazole (690 mg, 3.02 mmol), Cu₂O (359 mg, 2.51 mmol), Cs₂CO₃ (2.45 g, 7.53 mmol), Pd(OAc)₂ (56 mg, 0.25 mmol), and cataCXium A (180 mg, 0.50 mmol) were sequentially added thereto. The reaction system was replaced with nitrogen three times, and reacted under microwave irradiation at 120°C for 16 hours in a microwave reaction tube. The reaction mixture was filtered, and the crude filtrate was purified by preparative separation (chromatographic column: Agilent 10 Prep-C18 250 × 21.2 mm; column temperature: 25°C; mobile phase: water (0.1% FA)-acetonitrile; proportion of acetonitrile in the mobile phase: 45% to 75% in 12 min; flow rate: 30 mL/min) to obtain the title compound **120-1** (30 mg).

### Preparation of compound 120

Compound **120-1** (30 mg, 0.07 mmol) was dissolved in DMF (1 mL), then 1H-tetrazole (25 mg, 0.36 mmol) and K₂CO₃ (39 mg, 0.28 mmol) were added thereto, and the reaction system was reacted at 75°C for 16 hours. The reaction mixture was filtered, and the crude filtrate was purified by preparative separation (chromatographic column: Agilent 10 Prep-C18 250 × 21.2 mm; column temperature: 25°C; mobile phase: water (0.1% FA)-acetonitrile; proportion of acetonitrile in the mobile phase: 35% to 65% in 12 min; flow rate: 30 mL/min) to obtain the title compound **120** (8 mg).

LC-MS (ESI): m/z 491.50 [M+H]⁺. ¹H NMR (400 MHz, DMSO-*d6*) δ 9.14 (s, 1H), 7.60 (s, 1H), 7.58 - 7.47 (m, 1H), 7.37 (d, *J* = 0.7 Hz, 1H), 7.32 - 7.24 (m, 1H), 7.22 (s, 1H), 7.06 - 6.91 (m, 1H), 5.41 (d, *J* = 14.5 Hz, 1H), 5.10 - 4.92 (m, 3H), 1.96 (dd, *J* = 9.4, 1.7 Hz, 3H), 1.74 (dd, *J* = 9.4, 1.7 Hz, 3H). ¹⁹F NMR (376 MHz, DMSO-*d6*) δ -70.32 (s, 3F), -102.47 - -103.56 (m, 1F), -108.57 - -111.38 (m, 3F).

### Example 121: Preparation of compound 121

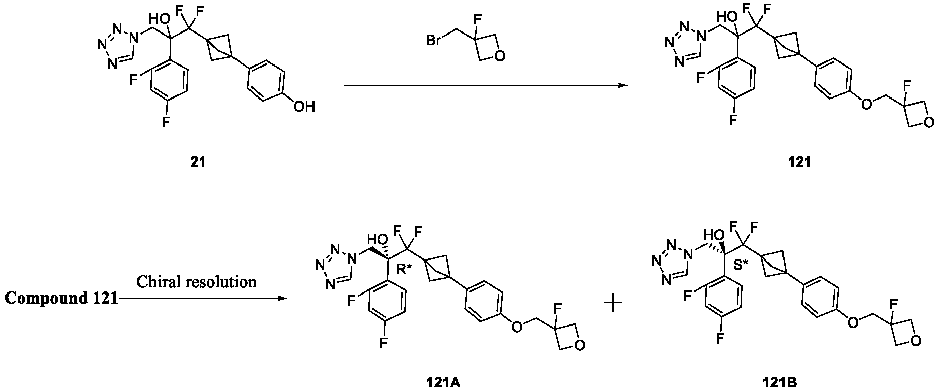

### Preparation of compound 121

Compound **21** (200 mg, 0.46 mmol) was dissolved in acetonitrile (2 mL), then compound 3-bromomethyl-3-fluorooxetane (93 mg, 0.55 mmol) and cesium carbonate (300 mg, 0.92 mmol) were added thereto, and the reaction system was stirred at 80°C for 16 hours in a sealed tube. After the reaction system was cooled, the reaction mixture was filtered, and the crude filtrate was purified by preparative separation (preparation method: mobile phase: A: 0.1% formic acid aqueous solution; B: acetonitrile; chromatographic column: Agilent 10 Prep-C18 250 × 21.2 mm; column temperature: 25°C; gradient: 45% to 65% acetonitrile in 12 min; flow rate: 30 mL/min) to obtain the title compound **121** (170 mg, yield: 70%, containing a pair of enantiomers).

LC-MS (ESI): m/z 523.4 [M+H]⁺. ¹H NMR (400 MHz, DMSO-*d6*) δ 9.14 (s, 1H), 7.53 (dd, *J* = 9.1, 6.7 Hz, 1H), 7.36 - 7.23 (m, 1H), 7.17 (s, 1H), 7.10 - 6.96 (m, 3H), 6.93 - 6.82 (m, 2H), 5.43 (d, *J* = 14.5 Hz, 1H), 5.01 (d, *J* = 14.6 Hz, 1H), 4.77 - 4.56 (m, 4H), 4.36 (d, *J* = 22.2 Hz, 2H), 1.98 (dd, *J* = 9.5, 1.7 Hz, 3H), 1.77 (dd, *J* = 9.4, 1.7 Hz, 3H). ¹⁹F NMR (376 MHz, DMSO-*d6*) δ -102.71 - -103.40 (m, 1F), -109.02- -109.42 (m, 2F), -109.62 (d, 1F), -154.39 (m, 1F).

### Preparation of compounds 121A and 121B

Compound **121** (150 mg) was subjected to SFC chiral preparative resolution (preparative separation method, instrument model: MG II preparative SFC (SFC-14); model of chromatographic column: ChiralCel OD, 250 × 30 mm I.D., 5 µm; mobile phase: A: CO₂ B: ethanol; elution gradient: B 35%; flow rate: 70 mL/min; column pressure: 100 bar; column temperature: 38°C; detection wavelength: 220 nm; cycle: about 2.5 min) to obtain the title compounds **121A** (62 mg, single enantiomer) and **121B** (68 mg, single enantiomer).

Compound **121A:** Chiral analysis method (model of chromatographic column: ChiralCel OD, 150 × 4.6 mm I.D., 3 µm; mobile phase: A: CO₂ B: ethanol (0.05% DEA); elution gradient: B 5 to 40%; flow rate: 2.5 mL/min; column temperature: 35°C; column pressure: 100 bar; detection wavelength: 220 nm; Rt = 5.737 min). LC-MS (ESI): m/z 523.2 [M+H]⁺. ¹H NMR (400 MHz, DMSO-*d6*) δ 9.14 (s, 1H), 7.53 (dd, *J* = 9.1, 6.7 Hz, 1H), 7.36 - 7.23 (m, 1H), 7.17 (s, 1H), 7.10 - 6.96 (m, 3H), 6.93 - 6.82 (m, 2H), 5.43 (d, *J* = 14.5 Hz, 1H), 5.01 (d, *J* = 14.6 Hz, 1H), 4.77 - 4.56 (m, 4H), 4.36 (d, *J* = 22.2 Hz, 2H), 1.98 (dd, *J* = 9.5, 1.7 Hz, 3H), 1.77 (dd, *J* = 9.4, 1.7 Hz, 3H). ¹⁹F NMR (376 MHz, DMSO-*d6*) δ -102.71 - -103.40 (m, 1F), -109.02 - -109.42 (m, 2F), -109.62 (d, 1F), -154.39 (m, 1F).

Compound **121B:** Chiral analysis method (model of chromatographic column: ChiralCel OD, 150 × 4.6 mm I.D., 3 µm; mobile phase: A: CO₂ B: ethanol (0.05% DEA); elution gradient: B 5 to 40%; flow rate: 2.5 mL/min; column temperature: 35°C; column pressure: 100 bar; detection wavelength: 220 nm; Rt = 6.106 min). LC-MS (ESI): m/z 523.2 [M+H]⁺. ¹H NMR (400 MHz, DMSO-*d6*) δ 9.14 (s, 1H), 7.53 (dd, *J* = 9.1, 6.7 Hz, 1H), 7.36 - 7.23 (m, 1H), 7.17 (s, 1H), 7.10 - 6.96 (m, 3H), 6.93 - 6.82 (m, 2H), 5.43 (d, *J* = 14.5 Hz, 1H), 5.01 (d, *J* = 14.6 Hz, 1H), 4.77 - 4.56 (m, 4H), 4.36 (d, *J* = 22.2 Hz, 2H), 1.98 (dd, *J* = 9.5, 1.7 Hz, 3H), 1.77 (dd, *J* = 9.4, 1.7 Hz, 3H). ¹⁹F NMR (376 MHz, DMSO-d6) δ -102.71 - -103.40 (m, 1F), -109.02 - -109.42 (m, 2F), -109.62 (m, 1F), -154.39 (m, 1F).

### Example 122: Preparation of compound 122

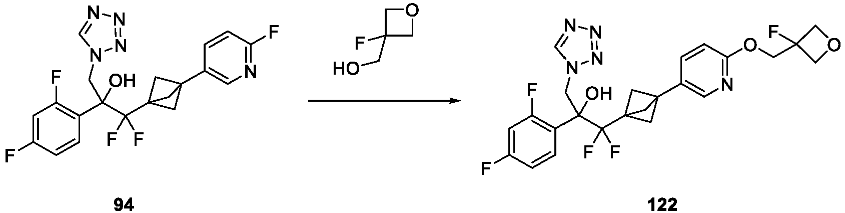

### Preparation of compound 122

(3-Fluorooxetan-3-yl)methanol (146 mg, 1.37 mmol) was dissolved in DMF (2 mL), and NaH (55 mg, 1.37 mmol) was added thereto at 0°C. After 15 min, the reaction system was added with compound **94** (200 mg, 0.46 mmol) and reacted at room temperature for 16 hours. The reaction system was filtered, and the filtrate was purified by preparative separation (preparation method: chromatographic column: Welch Xtimate^{®} C18 21.2 × 250 mm; column temperature: 25°C; mobile phase: water (10 mM/L NH₄HCO₃)-acetonitrile; proportion of acetonitrile in the mobile phase: 50% to 70% 12 min; flow rate: 30 mL/min) to obtain the title compound **122** (59 mg, yield: 25%). LC-MS (ESI): m/z 524.2 [M+H]⁺. ¹H NMR (400 MHz, DMSO-*d6*) δ 9.12 (d, *J* = 3.9 Hz, 1H), 7.97 - 7.86 (m, 1H), 7.60 - 7.45 (m, 2H), 7.33 -7.15 (m, 2H), 7.01 (td, *J* = 8.5, 2.8 Hz, 1H), 6.78 (dd, *J* = 8.8, 3.6 Hz, 1H), 5.43 (d, *J* = 14.5 Hz, 1H), 5.01 (d, *J* = 14.5 Hz, 1H), 4.66 (ddt, *J* = 18.8, 13.7, 5.8 Hz, 6H), 2.04 (d, *J* = 9.3 Hz, 3H), 1.83 (d, *J* = 9.3 Hz, 3H). ¹⁹F NMR (376 MHz, DMSO-*d6*) δ -103.06 (ddd, *J =* 41.3, 33.1, 9.4 Hz, 1F), -109.28 (d, *J* = 5.0 Hz, 1F), -109.37 (s, 1F), -109.51 (s, 1F), -154.29 (d, *J* = 20.7 Hz, 1F).

### Example 123: Preparation of compound 123

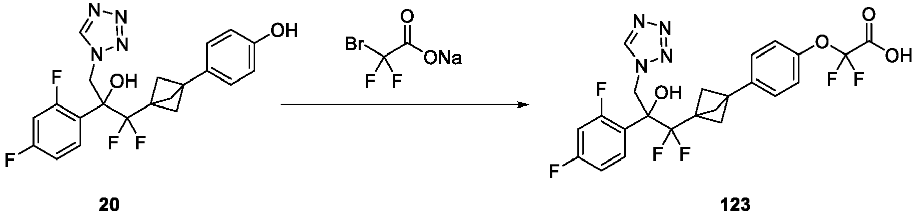

### Preparation of compound 123

Compound **20** (150 mg, 0.35 mmol) was dissolved in THF (3 mL), and NaH (21 mg, 0.52 mmol) was added thereto at 0°C. After stirring for 15 min, the reaction system was added with sodium bromodifluoroacetate (102 mg, 0.52 mmol) and reacted at room temperature for 16 hours. The reaction mixture was extracted with EtOAc (50 mL × 3), dried, and concentrated to obtain a crude product, which was purified by preparative separation (preparation method: chromatographic column: Welch Xtimate^{®} C18 21.2 × 250 mm; column temperature: 25°C; mobile phase: water (10 mM/L NH₄HCO₃)-acetonitrile; proportion of acetonitrile in the mobile phase: 70% to 90% 12 min; flow rate: 30 mL/min) to obtain the title compound **123** (15 mg, yield: 8%). LC-MS (ESI): m/z 529.1 [M+H]⁺. ¹H NMR (400 MHz, Methanol-*d4*) δ 8.87 (t, *J* = 2.6 Hz, 1H), 8.34 (s, 1H), 7.53 (dd, *J* = 8.9, 6.5 Hz, 1H), 7.00 (s, 4H), 6.90 (ddd, *J* = 11.9, 8.8, 2.6 Hz, 1H), 6.81 (td, *J* = 8.4, 2.6 Hz, 1H), 5.50 (d, *J* = 14.5 Hz, 1H), 4.94 (s, 1H), 1.95 (d, *J* = 8.5 Hz, 3H), 1.75 (dd, *J* = 9.4, 1.8 Hz, 3H). ¹⁹F NMR (376 MHz, Methanol-*d4*) δ -77.94 (s, 2F), -103.70 - -104.90 (m, 1F), -110.96 (d, *J* = 9.2 Hz, 1F), - 111.30 - -112.12 (m, 2F).

### Example 124: Preparation of compound 124

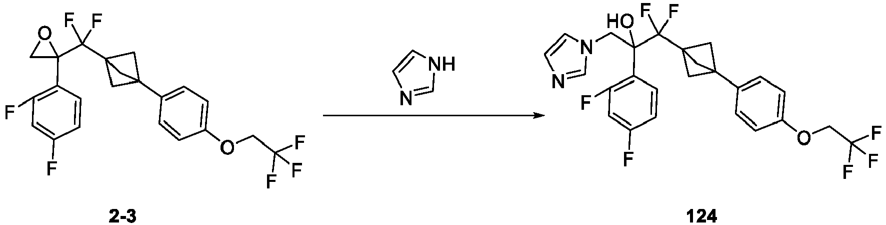

### Preparation of compound 124

Compound **2-3** (100 mg, 0.22 mmol) was dissolved in DMF (1 mL), then potassium carbonate (90 mg, 0.66 mmol) and imidazole (44 mg, 0.33 mmol) were added thereto, and the reaction system was reacted at 70°C for 16 hours. After the reaction system was cooled, the reaction mixture was filtered, and the crude filtrate was purified by preparative separation (chromatographic column: Agilent 10 Prep-C18 250 × 21.2 mm; column temperature: 25°C; mobile phase: water (0.1% FA)-acetonitrile; proportion of acetonitrile in the mobile phase: 20% to 40% in 12 min; flow rate: 30 mL/min) to obtain the title compound **124** (104 mg, yield: 90%).

LC-MS (ESI): m/z 515.0 [M+H]⁺. ¹H NMR (400 MHz, Chloroform-*d*) δ 8.75 (br.s, 1H), 7.72 (td, *J* = 9.2, 6.5 Hz, 1H), 7.06 - 6.98 (m, 2H), 6.95 (s, 1H), 6.92 - 6.84 (m, 2H), 6.84 - 6.79 (m, 3H), 6.78 (s, 1H), 4.96 (s, 2H), 4.29 (q, *J* = 8.1 Hz, 2H), 2.02 (dd, *J* = 9.5, 1.9 Hz, 3H), 1.79 (dd, *J* = 9.5, 1.9 Hz, 3H). ¹⁹F NMR (376 MHz, Chloroform-*d*) δ -74.00 (s, 3F), - 107.89 (s, 2F), -109.51 - -110.85 (m, 2F).

### Example 125: Preparation of compounds 125, 125A, and 125B

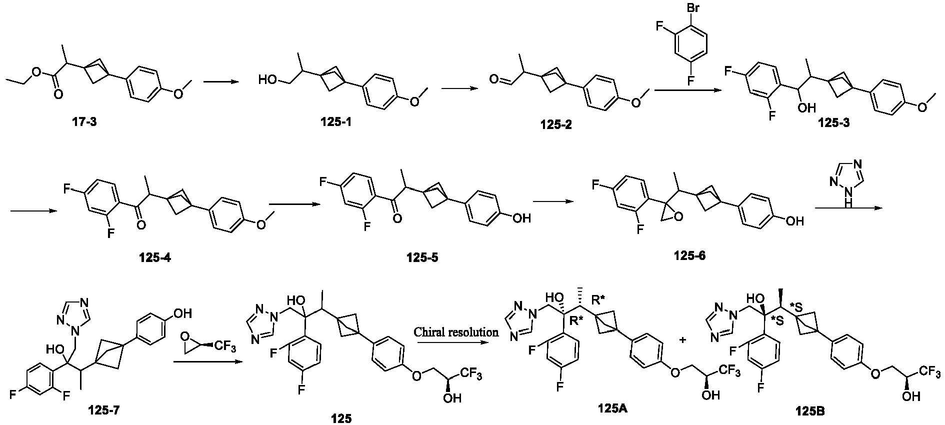

### Preparation of compound 125-1

Compound **17-3** (5 g, 18.2 mmol) was dissolved in anhydrous THF (50 mL), then lithium aluminum hydride (988 mg, 26 mmol) was added thereto in batches at 0°C, and the reaction system was slowly warmed to room temperature and reacted for 3 hours. The reaction system was transferred to 0°C. H₂O and sodium hydroxide solution were sequentially added dropwise thereto, and then H₂O was added dropwise thereto. The reaction mixture was filtered, the filtrate was extracted with EtOAc (50 mL × 3), and the organic phases were combined, dried, and concentrated to obtain the crude title compound 125-1 (3 g, yield: 70.8%). LC-MS (ESI): m/z 233.2 [M+H]⁺.

### Preparation of compound 125-2

Compound **125-1** (3 g, 12.9 mmol) was dissolved in anhydrous DCM (50 mL), then Dess-Martin periodinane (6.5 g, 15.48 mmol) was added thereto in batches, and the reaction system was reacted at room temperature for 16 hours. The reaction system was extracted with EtOAc (50 mL × 3), and the organic phases were combined, dried, and concentrated to obtain a crude product. The crude product was purified by normal-phase silica gel column chromatography (EtOAc/PE = 0 to 20%) to obtain the title compound **125-2** (2 g, yield: 67.4%). ¹NMR (400 MHz, Chloroform-*d*) δ 9.72 (s, 1H), 7.13 (d, *J* = 8.0 Hz, 2H), 6.84 (d, *J* = 8.0 Hz, 2H), 3.79 (s, 3H), 2.58 (q, *J* = 8.0 Hz, 1H), 2.05-2.00 (m, 6H), 1.08 (d, *J* = 8.0 Hz, 3H).

### Preparation of compound 125-3

To a three-necked flask was added 1-bromo-2,4-difluorobenzene (1.92 g, 10 mmol), and the system was replaced with nitrogen three times. Anhydrous THF (100 mL) was added thereto, and n-butyllithium (10 mL, 10 mmol, 1M) was added dropwise thereto at -78°C. After the reaction system was stirred for 30 min, compound **125-2** (2 g, 8.69 mmol) was slowly added thereto. After stirring for another 30 min, the reaction system was quenched by adding ammonium chloride (20 mL), extracted with EtOAc (100 mL × 3), and the organic phases were combined, dried, and concentrated to obtain a crude product. The crude product was purified by normal-phase silica gel column chromatography (EtOAc/PE = 0 to 20%) to obtain the title compound **125-3** (1.8 g, light yellow liquid, yield: 60.2%). LC-MS (ESI): m/z 329.4 [M+H-16]⁺.

### Preparation of compound 125-4

Compound **125-3** (1.8 g, 5.23 mmol) was dissolved in anhydrous DCM (50 mL), then Dess-Martin periodinane (3.18 g, 7.5 mmol) was added thereto in batches, and the reaction system was reacted at room temperature for 16 hours. The reaction system was extracted with EtOAc (200 mL × 3), and the organic phases were combined, dried, and concentrated to obtain a crude product. The crude product was purified by normal-phase silica gel column chromatography (EtOAc/PE = 0 to 20%) to obtain the title compound **125-4** (1.6 g, yield: 94.7%). LC-MS (ESI): m/z 343.4 [M+H]⁺.

### Preparation of compound 125-5

Compound **125-4** (1.6 g, 4.67 mmol) was dissolved in a mixture of acetic acid (30 mL) and hydrobromic acid aqueous solution (30 mL), and the reaction system was reacted at 110°C for 16 hours. The reaction system was cooled, then extracted with EtOAc (50 mL × 3), and the organic phases were combined, dried, and concentrated to obtain a crude product. The crude product was purified by normal-phase silica gel column chromatography (EtOAc/PE = 0 to 20%) to obtain the title compound **125-5** (900 mg, brown solid, yield: 58%). LC-MS (ESI): m/z 329.4 [M+H]⁺.

### Preparation of compound 125-6

Trimethylsulfonium iodide (2.79 g, 13.7 mmol) was dissolved in a mixed solvent of DMSO (15 mL) and THF (10 mL), then NaH (purity: 60%, 525 mg, 13.7 mmol) was added thereto. The reaction system was stirred at room temperature for 1 hour, then a solution of compound **125-5** (900 mg, 2.74 mmol) in DMSO (10.0 mL) was added thereto, and the reaction system was reacted at 50°C for 16 hours. The reaction system was quenched with saturated ammonium chloride solution (100 mL), extracted with EtOAc (50 mL × 3), and the organic phases were combined, dried, and concentrated to obtain a crude product. The crude product was purified by normal-phase silica gel column chromatography (EtOAc/PE = 0 to 30%) to obtain the title compound **125-6** (400 mg, yellow oil, yield: 42%). LC-MS (ESI): m/z 343.5 [M+H]⁺.

### Preparation of compound 125-7

1,2,4-Triazole (2.79 g, 13.7 mmol) was dissolved in DMF (5 mL), then NaH (purity: 60%, 525 mg, 13.7 mmol) was added thereto. The reaction system was stirred at room temperature for 1 hour, then added with compound **125-6** (400 mg, 1.16 mmol), replaced with nitrogen three times, and reacted at 50°C for 16 hours. The reaction system was quenched with saturated ammonium chloride solution (100 mL), extracted with EtOAc (50 mL × 3), and the organic phases were combined, dried, and concentrated to obtain a crude product. The crude product was purified by normal-phase silica gel column chromatography (EtOAc/PE = 0 to 30%) to obtain the title compound **125-7** (300 mg, yellow oil, yield: 63%). LC-MS (ESI): m/z 412.2 [M+H]⁺.

### Preparation of compound 125

Compound **125-7** (300 mg, 0.73 mmol) was dissolved in acetonitrile (5.0 mL), then R-(+)-2-trifluoromethyloxirane (112 mg, 1 mmol) and potassium carbonate (276 mg, 2 mmol) were added thereto, and the reaction system was replaced with nitrogen three times, stirred and reacted at 70°C for 16 hours. After the reaction system was cooled, the reaction mixture was filtered, and the filtrate was purified by preparative separation (preparation method: mobile phase: A: 0.1% formic acid aqueous solution; B: acetonitrile; chromatographic column: Agilent 10 Prep-C18 250 × 21.2 mm; column temperature: 25°C; gradient: 45% to 65% acetonitrile 12 min; flow rate: 30 mL/min) to obtain the title compound **125** (38 mg, yield: 75%). LC-MS (ESI): m/z 524.3 [M+H]⁺. ¹H NMR (400 MHz, DMSO-d6) δ 8.22 (s, 1H), 7.63 (s, 1H), 7.31 (td, *J* = 9.1, 6.9 Hz, 1H), 7.09 (ddd, *J* = 12.0, 9.1, 2.6 Hz, 1H), 6.97 - 6.89 (m, 2H), 6.86 (dd, *J* = 8.5, 2.6 Hz, 1H), 6.83 - 6.78 (m, 2H), 6.61 (d, *J* = 6.7 Hz, 1H), 5.38 (s, 1H), 4.59 (q, *J* = 14.3 Hz, 2H), 4.33 (dt, *J* = 11.3, 6.8 Hz, 1H), 4.08 (dd, *J* = 10.5, 4.2 Hz, 1H), 3.98 (dd, *J* = 10.5, 6.5 Hz, 1H), 1.60 (dd, *J* = 9.6, 1.6 Hz, 3H), 1.40 (dd, *J* = 9.5, 1.6 Hz, 3H), 1.15 (d, *J* = 6.9 Hz, 3H). ¹⁹F NMR (376 MHz, DMSO-*d6*) δ -76.13 (s, 3F) -106.98 - -107.00 (m, 2F), -109.20- -109.30 (m, 1F), -112.95 - -112.98 (m, 1F).

### Preparation of compounds 125A and 125B

Compound **125** (38 mg, containing a pair of enantiomers) was subjected to SFC chiral preparative resolution (preparative separation method, instrument model: MG II preparative SFC (SFC-14); chromatographic column: ChiralCel OD, 250 × 30 mm I.D., 10 µm; mobile phase: A: CO₂ B: isopropanol (0.1% NH₃H₂O); elution gradient: B 40%; flow rate: 100 mL/min; column pressure: 100 bar; chromatographic column temperature: 35°C; detection wavelength: 220 nM; cycle: about 9.5 min) to obtain the title compounds **125A** (8.8 mg) and **125B** (6.4 mg).

Compound **125A:** Chiral resolution (chromatographic column: Cellulose OD-3 150 × 4.6 mm I.D., 3 µm; mobile phase: A: CO₂ B: ethanol (0.05% DEA); elution gradient: the mobile phase increased from 5% B to 40% B within 5 min and maintained at 40% B for 2.5 min for elution, then 5% B was equilibrated for 2.5 min; flow rate: 2.5 mL/min; chromatographic column temperature: 35°C; column pressure: 1500 psi; detection wavelength: 220 nM; RT = 1.266 min.) LC-MS (ESI): m/z 524.3 [M+H]⁺. ¹H NMR (400 MHz, DMSO-*d6*) δ 8.22 (s, 1H), 7.63 (s, 1H), 7.31 (td, *J* = 9.0, 6.8 Hz, 1H), 7.09 (ddd, *J* = 12.0, 9.1, 2.6 Hz, 1H), 7.00 - 6.90 (m, 2H), 6.90 - 6.74 (m, 3H), 6.60 (d, *J* = 6.7 Hz, 1H), 5.37 (s, 1H), 4.59 (q, *J* = 14.3 Hz, 2H), 4.33 (s, 1H), 4.08 (dd, *J* = 10.5, 4.3 Hz, 1H), 3.98 (dd, *J* = 10.6, 6.4 Hz, 1H), 1.60 (dd, *J* = 9.5, 1.7 Hz, 3H), 1.40 (dd, *J* = 9.5, 1.7 Hz, 3H), 1.15 (d, *J* = 6.8 Hz, 3H). ¹⁹F NMR (376 MHz, DMSO-*d6*) δ -76.08 (s, 3F) -106.98- -107.00 (m, 1F), -112.96 -112.98 (m, 1F).

Compound **125B:** Chiral resolution (chromatographic column: Cellulose OD-3 150 × 4.6 mm I.D., 3 µm; mobile phase: A: CO₂ B: ethanol (0.05% DEA); elution gradient: the mobile phase increased from 5% B to 40% B within 5 min and maintained at 40% B for 2.5 min for elution, then 5% B was equilibrated for 2.5 min flow rate; chromatographic column temperature: 35°C; column pressure: 1500 psi; detection wavelength: 220 nM; RT = 0.998 min). LC-MS (ESI): m/z 524.3 [M+H]⁺. ¹H NMR (400 MHz, DMSO-*d6*) δ 8.22 (s, 1H), 7.63 (s, 1H), 7.31 (td, *J* = 9.1, 6.9 Hz, 1H), 7.09 (ddd, *J* = 12.0, 9.1, 2.6 Hz, 1H), 6.98 - 6.90 (m, 2H), 6.90 - 6.77 (m, 3H), 6.60 (d, *J* = 6.7 Hz, 1H), 5.37 (s, 1H), 4.59 (q, *J* = 14.3 Hz, 2H), 4.33 (dt, *J* = 7.6, 3.8 Hz, 1H), 4.08 (dd, *J* = 10.5, 4.2 Hz, 1H), 3.98 (dd, *J* = 10.5, 6.4 Hz, 1H), 1.60 (dd, *J* = 9.6, 1.7 Hz, 3H), 1.40 (dd, *J* = 9.4, 1.7 Hz, 3H), 1.15 (d, *J* = 6.9 Hz, 3H). ¹⁹F NMR (376 MHz, DMSO-*d6*) δ -75.97 (s, 3F) -106.97- -106.99 (m, 1F), -112.95 -112.97 (m, 1F).

### Example 126: Preparation of compound 126

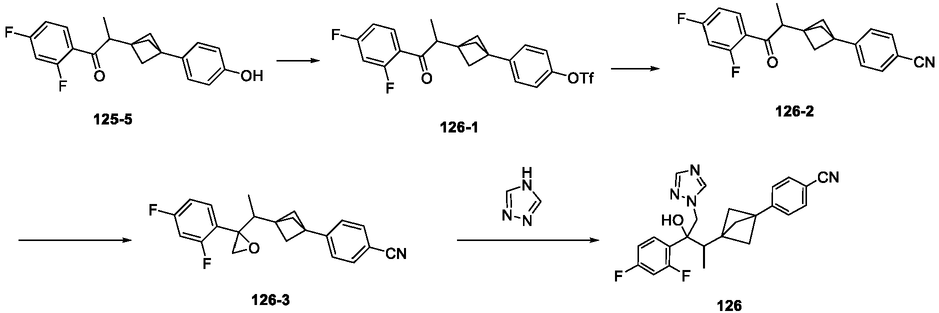

### Preparation of compound 126-1

Compound **125-5** (800 mg, 2.44 mmol) was dissolved in DMF (10 mL), then triethylamine (492 mg, 4.87 mmol) was added thereto. After the reaction system was stirred and reacted at 0°C for 5 min, the reaction system was slowly added with N-phenyl-bis(trifluoromethanesulfonimide) (1.45 g, 2.9 mmol), transferred to room temperature, and reacted for 2 hours. The reaction system was extracted with EtOAc (20 mL × 3), and the organic phases were combined, dried, and concentrated to obtain a crude product. The crude product was purified by normal-phase silica gel column chromatography (EtOAc/PE = 0 to 5%) to obtain the title compound **126-1** (500 mg, yield: 44%). LC-MS (ESI): m/z 461.0 [M+H]⁺.

### Preparation of compound 126-2

Compound **126-1** (500 mg, 1.1 mmol) was dissolved in DMF (10 mL), then zinc cyanide (629 g, 5.38 mmol) and tetrakis(triphenylphosphine)palladium (254 mg, 0.22 mmol) were added thereto, and the reaction system was replaced with nitrogen three times and reacted under microwave irradiation at 100°C for 40 hours. The reaction system was cooled, then extracted with EtOAc (30 mL × 3), and the organic phases were combined, dried, and concentrated to obtain a crude product. The crude product was purified by normal-phase silica gel column chromatography (EtOAc/PE = 0 to 5%) to obtain the title compound **126-2** (220 mg, yield: 59%). LC-MS (ESI): m/z 338.0 [M+H]⁺.

### Preparation of compound 126-3

Trimethylsulfonium iodide (398 mg, 1.95 mmol) was dissolved in a mixed solvent of anhydrous DMSO (20 mL) and anhydrous THF (20 mL), then NaH (124 mg, purity: 60%, 3.25 mmol) was added thereto, and the reaction system was stirred for 30 min, then added with compound **126-2** (220 mg, 0.65 mmol). The reaction mixture was reacted at room temperature for 16 hours, then added with water (100 mL) and EA (50 mL × 3), and the organic phases were combined, dried, and concentrated to obtain the title compound **126-2** (150 mg). LC-MS (ESI): m/z 352.0 [M+H]⁺.

### Preparation of compound 126

Triazole (88 mg, 1.28 mmol) was dissolved in anhydrous DMF (10 mL), then NaH (98 mg, purity: 60%, 2.5 mmol) was added thereto, and the reaction system was stirred for 30 min, then added with compound **126-2** (150 mg, crude), and reacted at 50°C for 16 hours. After the reaction system was cooled, the reaction mixture was filtered, and the filtrate was purified by preparative separation (preparation method: mobile phase: A: 0.1% NH₄HCO₃ aqueous solution; B: acetonitrile; chromatographic column: Agilent 10 Prep-C18 250 × 21.2 mm; column temperature: 25°C; gradient: 50% to 70% acetonitrile 12 min; flow rate: 30 mL/min) to obtain the title compound **126** (7.32 mg, two-step yield: 20%). LC-MS (ESI): m/z 421.2 [M+H]⁺. ¹H NMR (400 MHz, DMSO-*d6*) δ 8.22 (s, 1H), 7.74 - 7.59 (m, 3H), 7.39 - 7.29 (m, 1H), 7.21 (d, *J* = 8.1 Hz, 2H), 7.10 (ddd, *J* = 12.0, 9.1, 2.6 Hz, 1H), 6.85 (td, *J* = 8.6, 2.6 Hz, 1H), 5.45 (s, 1H), 4.60 (q, *J* = 14.4 Hz, 2H), 2.60 - 2.52 (m, 1H), 1.69 (dd, *J* = 9.4, 1.6 Hz, 3H), 1.49 (dd, *J* = 9.5, 1.6 Hz, 3H), 1.16 (d, *J* = 6.9 Hz, 3H). ¹⁹F NMR (376 MHz, DMSO-*d6)* δ -106.97 - -106.99 (m, 1F), -112.82 - -112.84 (m, 1F).

### Example 127: Preparation of compound 127

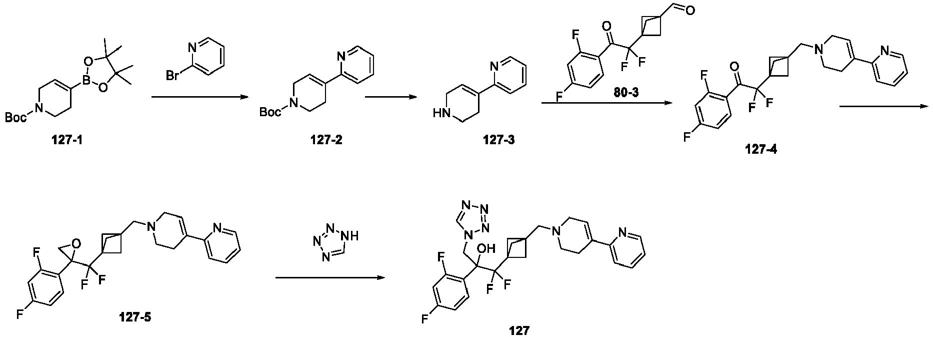

### Preparation of compound 127-2

Compound **127-1** (1.1 g, 3.56 mmol) was dissolved in a mixed solvent of dioxane (20 mL) and H₂O (2 mL), then 2-bromopyridine (620 mg, 3.91 mmol), potassium carbonate (983 mg, 7.11 mmol), and Pd(dppf)Cl₂ (260 mg, 0.356 mmol) were sequentially added thereto. The reaction system was replaced with nitrogen three times and reacted at 80°C for 16 hours. The reaction system was cooled, then extracted with EtOAc (50 mL × 3), and the organic phases were combined, dried, and concentrated to obtain a crude product. The crude product was purified by normal-phase silica gel column chromatography (EtOAc/PE = 0 to 100%) to obtain the title compound **127-2** (600 mg, yellow oil, yield: 65%). LC-MS (ESI): m/z 261.1 [M+H]⁺.

### Preparation of compound 127-3

Compound **127-2** (600 mg, 2.299 mmol) was dissolved in dichloromethane (20 mL), then trifluoroacetic acid (5 mL) was slowly added dropwise thereto at 0°C, and the reaction system was transferred to room temperature to continue the reaction for 2 hours. The reaction mixture was concentrated to obtain a crude product, and the crude product was purified by normal-phase silica gel column chromatography (MeOH/DCM = 0 to 10%) to obtain the title compound **127-3** (270 mg, yield: 73%). LC-MS (ESI): m/z 161.1 [M+H]⁺.

### Preparation of compound 127-4

Compound **127-3** (100 mg, 0.594 mmol) was dissolved in dichloromethane (5 mL), then compound **80-3** (170 mg, 0.594 mmol) was added thereo, and one drop of acetic acid was added dropwise thereo. The reaction system was stirred for 10 min, slowly added with sodium triacetoxyborohydride (126 mg, 0.594 mmol) at 0°C, transferred to room temperature, and reacted for 3 hours. The reaction mixture was extracted with EtOAc (50 mL × 3), and the organic phases were combined, dried, and concentrated to obtain a crude product. The crude product was purified by normal-phase silica gel column chromatography (EtOAc/PE = 0 to 5%) to obtain the title compound **127-4** (80 mg, yield: 31%). LC-MS (ESI): m/z 431.1 [M+H]⁺.

### Preparation of compound 127-5

Trimethylsulfoxonium iodide (46 mg, 0.205 mmol) was dissolved in a mixed solvent of DMSO (2 mL) and THF (3 mL), then potassium tert-butoxide (22 mg, 0.205 mmol) was added thereto. The reaction system was stirred at room temperature for 1 hour, slowly added with compound **127-4** (80 mg, 0.186 mmol), and reacted for another 2 hours. The reaction mixture was extracted with EtOAc (50 mL × 3), and the organic phases were combined, dried, and concentrated to obtain a crude product. The crude product was purified by normal-phase silica gel column chromatography (EtOAc/PE = 0 to 100%) to obtain the title compound **127-5** (49 mg, yellow solid, yield: 90%). LC-MS (ESI): m/z 445.2 [M+H]⁺.

### Preparation of compound 127

Compound **127-5** (49 mg, 0.112 mmol) was dissolved in DMF (1 mL), then 1H-tetrazole (16 mg, 0.221 mmol) and potassium carbonate (30 mg, 0.213 mmol) were added thereto, and the reaction system was reacted at 80°C for 16 hours. After the reaction system was cooled, the filtrate was filtered, and the filtrate was purified by preparative separation (preparation method: mobile phase: A: 0.1% formic acid aqueous solution; B: acetonitrile; chromatographic column: Sunfire C18 21.2 * 250 mm * 10 µm; column temperature: 25°C; gradient: 23% to 25% acetonitrile 7.5 to 8.5 min; flow rate: 20 mL/min) to obtain the title compound **127** (5.22 mg, yield: 9%). LC-MS (ESI): m/z 515.4 [M+H]⁺. ¹HNMR (400 MHz, MeOD) δ 8.89 (s, 1H), 8.55 (d, *J* = 4.2 Hz, 1H), 7.87 (td, *J* = 7.9, 1.7 Hz, 1H), 7.71 - 7.53 (m, 2H), 7.37 (dd, *J* = 7.1, 5.3 Hz, 1H), 7.05 - 6.95 (m, 1H), 6.95 - 6.84 (m, 1H), 6.57 (s, 1H), 5.57 (d, *J* = 14.3 Hz, 1H), 4.97 (t, *J* = 14.3 Hz, 1H), 3.96 (s, 2H), 3.77 - 3.34 (m, 4H), 2.95 (s, 2H), 2.02 (dd, *J* = 9.5, 1.7 Hz, 3H), 1.82 (dd, *J* = 9.5, 1.7 Hz, 3H).

### Example 128: Preparation of compound 128

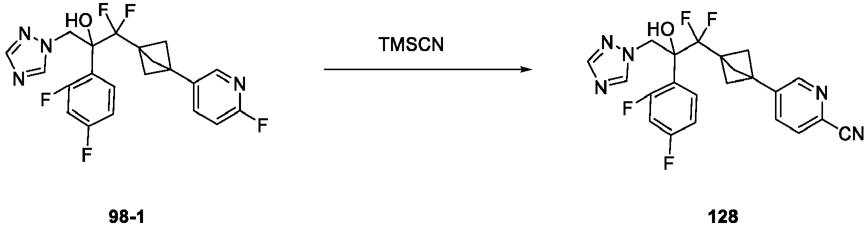

### Preparation of compound 128

Compound **98-1** (830 mg, 1.90 mmol) was dissolved in DMF (5 mL), then TMSCN (941 mg, 9.50 mmol) and CsF (868 mg, 5.71 mmol) were added thereto, and the reaction system was reacted at 120°C for 16 hours. After the reaction system was cooled, the reaction mixture was filtered, and the crude filtrate was purified by preparative separation (chromatographic column: Agilent 10 Prep-C18 250 × 21.2 mm; column temperature: 25°C; mobile phase: water (0.1 % FA)-acetonitrile; proportion of acetonitrile in the mobile phase: 40% to 60% 12 min; flow rate: 30 mL/min) to obtain the title compound **128** (4 mg). LC-MS (ESI): m/z 444.0 [M+H]⁺. ¹H NMR (400 MHz, Chloroform-*d*) δ 8.47 - 8.39 (m, 1H), 8.00 (s, 1H), 7.70 - 7.62 (m, 1H), 7.62 - 7.56 (m, 1H), 7.51 (dd, *J* = 7.9, 2.1 Hz, 1H), 6.94 - 6.68 (m, 3H), 5.37 (d, *J* = 7.8 Hz, 1H), 5.15 (d, *J* = 14.2 Hz, 1H), 2.16 (dd, *J* = 9.4, 2.0 Hz, 4H), 1.98 - 1.94 (m, 3H). ¹⁹F NMR (376 MHz, Chloroform-*d*) δ -107.44 (d, *J* = 9.6 Hz, 2F), -110.95 (m, 2F).

### Example 129: Preparation of compound 129

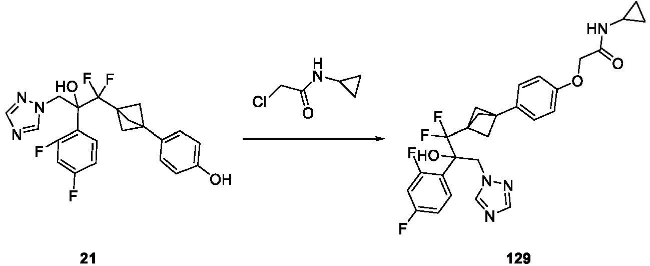

### Preparation of compound 129

Compound **21** (190 mg, 0.438 mmol) was added to a microwave tube and dissolved in anhydrous DMF (3 mL), then cesium carbonate (214 mg, 0.658 mmol) and 2-chloro-N-cyclopropylacetamide (64 mg, 0.482 mmol) were added thereto, and the reaction system was reacted at 120°C for 2 hours in a sealed tube. After the reaction system was cooled, the reaction mixture was filtered, and the filtrate was purified by preparative separation (preparation method: mobile phase: A: 0.1% formic acid aqueous solution; B: acetonitrile; chromatographic column: Pursuit XRs 10 C18 250 * 21.2 mm; column temperature: 25°C; gradient: 5% to 57% acetonitrile in 9.5 to 10.2 min; flow rate: 25 mL/min) to obtain the title compound **129** (138 mg, yield: 59%). LC-MS (ESI): m/z 531.2 [M+H]⁺. ¹H NMR (400 MHz, MeOD) δ 8.29 (s, 1H), 7.70 (s, 1H), 7.69 - 7.63 (m, 1H), 7.03 (d, *J* = 8.4 Hz, 2H), 6.92 (ddd, *J* = 14.5, 8.6, 4.1 Hz, 2H), 6.84 (d, *J* = 8.6 Hz, 2H), 5.30 (d, *J* = 14.4 Hz, 1H), 4.77 (d, *J* = 14.5 Hz, 1H), 4.41 (s, 2H), 2.70 (tt, *J* = 7.4, 3.9 Hz, 1H), 2.01 (d, *J* = 9.4 Hz, 3H), 1.84-1.79 (m, 3H), 0.72 (dt, *J* = 7.0, 3.4 Hz, 2H), 0.56 - 0.49 (m, 2H).

### Example 130: Preparation of compound 130

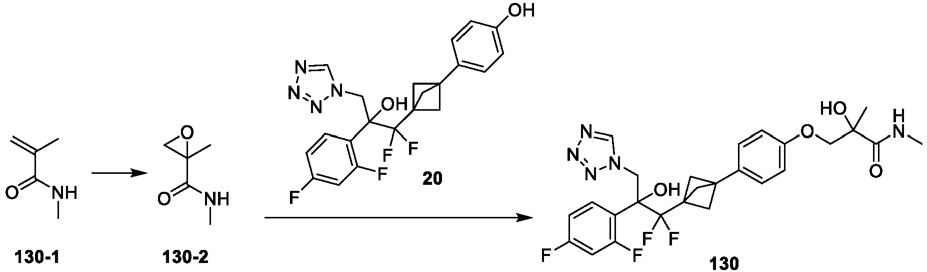

### Preparation of compound 130-2

Compound **130-1** (1.0 g, 10.09 mmol) was dissolved in dichloromethane (10 mL), then m-CPBA (1.91 g, 11.10 mmol) was added thereto at room temperature, and the reaction system was reacted for another 4 hours. The reaction system was washed with saturated sodium thiosulfate aqueous solution, extracted with DCM (50 mL × 3), and the organic phases were combined, dried, and concentrated to obtain the title compound **130-2** (500 mg), which was used directly in the next step.

### Preparation of compound 130

Compound **20** (200 mg, 0.46 mmol) was dissolved in acetonitrile (2 mL), then compound **130-2** (500 mg, 5.0 mmol) and potassium carbonate (300 mg, 0.92mmol) were added thereto, and the reaction system was stirred at 80°C for 16 hours in a sealed tube. After the reaction system was cooled, the reaction mixture was filtered, and after the filtrate was evaporated to dryness by rotary evaporation, the crude product was purified by preparative separation (preparation method: mobile phase: A: 0.1% formic acid aqueous solution; B: acetonitrile; chromatographic column: Agilent 10 Prep-C18 250 × 21.2 mm; column temperature: 25°C; gradient: 35% to 55% acetonitrile 12 min; flow rate: 30 mL/min) to obtain the title compound **130** (30 mg, yield: 10%).

LC-MS (ESI): m/z 550.2 [M+H]⁺. ¹H NMR (400 MHz, DMSO-d6) δ 9.18 - 9.04 (m, 1H), 7.86 - 7.68 (m, 1H), 7.64 - 7.46 (m, 1H), 7.37 - 7.23 (m, 1H), 7.16 (s, 1H), 7.04 - 6.94 (m, 3H), 6.88 - 6.70 (m, 2H), 5.59 (s, 1H), 5.42 (d, *J* = 14.5 Hz, 1H), 5.01 (d, *J* = 14.5 Hz, 1H), 4.00 (d, *J* = 9.4 Hz, 1H), 3.79 (d, *J* = 9.5 Hz, 1H), 2.60 (d, *J* = 4.8 Hz, 3H), 1.97 (dd, *J* = 9.5, 1.7 Hz, 3H), 1.76 (dd, *J* = 9.3, 1.7 Hz, 3H), 1.27 (s, 3H). ¹⁹F NMR (376 MHz, DMSO-d6) δ -102.74 - -103.20 (m, 1F), -108.91 - -109.48 (m, 2F), -109.53 - -109.90 (m, 1F).

### Example 131: Preparation of compounds 131, 131A, and 131B

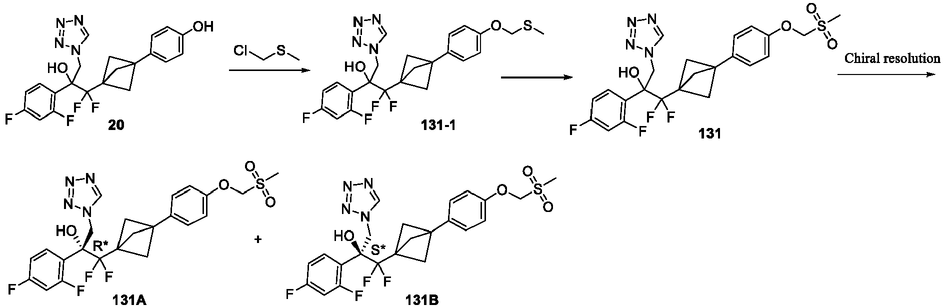

### Preparation of compound 131-1

Compound **20** (300 mg, 0.69 mmol) was dissolved in DMF (10 mL), then cesium carbonate (676 mg, 2.07 mmol) was added thereto. The reaction system was stirred for 5 min, then added with chloromethyl methyl sulfide (133 mg, 1.38 mmol), and reacted at 50°C for 4 hours. The reaction system was cooled, then extracted with EtOAc (50 mL × 3), and the organic phases were combined, dried, and concentrated to obtain a crude product. The crude product was purified by normal-phase silica gel column chromatography (EtOAc/PE = 0 to 70%) to obtain the title compound **131-1** (200 mg, white solid, yield: 59%). LC-MS (ESI): m/z 495.4 [M+H]⁺.

### Preparation of compound 131

Compound **131-1** (200 mg, 0.40 mmol) was dissolved in DCM (10 mL), then m-CPBA (139 mg, 0.81 mmol) was added thereto at 0°C, and the reaction system was transferred to react for 16 hours. The reaction mixture was poured into ice water, added with saturated NaHCC₃ solution to adjust the pH to weak alkalinity, and extracted with DCM (50 mL × 3). The organic phases were combined, dried over anhydrous sodium sulfate, filtered, and concentrated to obtain a crude product, and the crude product was purified by preparative separation (preparative method: chromatographic column: Agilent 10 Prep-C18 250 × 21.2 mm; column temperature: 25°C; mobile phase: water (0.1% FA)-acetonitrile; proportion of acetonitrile in the mobile phase: 40% to 60% 12 min; flow rate: 30 mL/min) to obtain the title compound **131** (92 mg, yield: 43%).

LC-MS (ESI): m/z 527.6 [M+H]⁺. ¹H NMR (400 MHz, DMSO-d6) δ 9.13 (s, 1H), 7.60 - 7.48 (m, 1H), 7.34 - 7.23 (m, 1H), 7.16 (s, 1H), 7.12 - 6.93 (m, 5H), 5.43 (d, *J* = 14.6 Hz, 1H), 5.26 (s, 2H), 5.01 (d, *J* = 14.6 Hz, 1H), 3.01 (s, 3H), 1.99 (dd, *J* = 9.4, 1.8 Hz, 3H), 1.78 (dd, *J* = 9.4, 1.8 Hz, 3H). ¹⁹F NMR (376 MHz, DMSO-d6) δ -102.80 - -103.34 (m, 1F), -109.06 - -109.48 (m, 2F), -109.62 (d, *J* = 9.6 Hz, 1F).

### Preparation of compounds 131A and 131B

Compound **131** (87 mg) was subjected to SFC chiral preparative resolution (preparative separation method, instrument model: MG II preparative SFC (SFC-14); model of chromatographic column: ChiralPak IC, 250 × 30 mm I.D., 10 µm; mobile phase: A: CO₂ B: ethanol; elution gradient: B 30%; flow rate: 70 mL/min; column pressure: 100 bar; column temperature: 38°C; detection wavelength: 220 nm; cycle: about 9 min) to obtain the title compounds **131A** (41 mg) and **131B** (42 mg).

Compound **131A:** Chiral analysis method (model of chromatographic column: ChiralPak IC, 150 × 4.6 mm I.D., 3 µm; mobile phase: A: CO₂B: ethanol (0.05% DEA); elution gradient: B 40%; flow rate: 2.5 mL/min; column temperature: 35°C; column pressure: 100 bar; detection wavelength: 220 nm; RT = 2.000 min). LC-MS (ESI): m/z 526.8 [M+H]⁺. ¹H NMR (400 MHz, DMSO-d6) δ 9.14 (s, 1H), 7.54 (td, *J* = 9.0, 6.8 Hz, 1H), 7.35 - 7.13 (m, 2H), 7.12 - 6.95 (m, 5H), 5.43 (d, *J* = 14.6 Hz, 1H), 5.26 (s, 2H), 5.01 (d, *J* = 14.6 Hz, 1H), 3.01 (s, 3H), 1.99 (dd, *J* = 9.4, 1.8 Hz, 3H), 1.78 (dd, *J* = 9.4, 1.8 Hz, 3H). ¹⁹F NMR (376 MHz, DMSO-*d6*) δ -102.95 - -103.17 (m, 1F), -109.23 - -109.33 (m, 2F), -109.62 (d, *J* = 9.6 Hz, 1F).

Compound **131B:** Chiral analysis method (model of chromatographic column: ChiralPak IC, 150 × 4.6 mm I.D., 3 µm; mobile phase: A: CO₂ B: ethanol (0.05% DEA); elution gradient: B 40%; flow rate: 2.5 mL/min; column temperature: 35°C; column pressure: 100 bar; detection wavelength: 220 nm; RT = 1.330 min). LC-MS (ESI): m/z 526.8 [M+H]⁺. ¹H NMR (400 MHz, DMSO-*d6*) δ 9.14 (s, 1H), 7.54 (td, *J* = 9.0, 6.8 Hz, 1H), 7.38 - 7.18 (m, 2H), 7.15 - 6.93 (m, 5H), 5.43 (d, *J* = 14.6 Hz, 1H), 5.26 (s, 2H), 5.01 (d, *J* = 14.6 Hz, 1H), 3.01 (s, 3H), 1.99 (dd, *J* = 9.4, 1.8 Hz, 3H), 1.78 (dd, *J* = 9.4, 1.8 Hz, 3H). ¹⁹F NMR (376 MHz, DMSO-d6) δ -102.96 - -103.17 (m, 1F), -109.23 - -109.33 (m, 2F), -109.62 (d, *J* = 9.6 Hz, 1F).

### Example 132: Preparation of compounds 132A and 132B

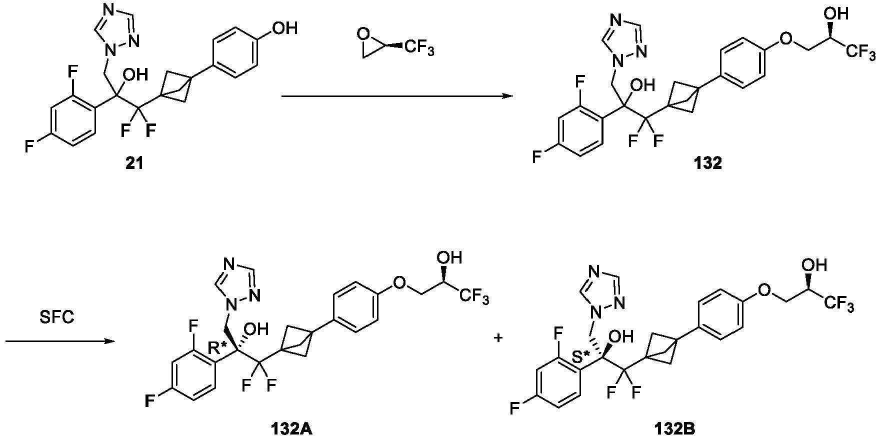

### Preparation of compound 132

Compound **21** (460 mg, 1.06 mmol) was dissolved in acetonitrile (2 mL), then potassium carbonate (220 mg, 1.59 mmol) and R-(+)-2-trifluoromethyloxirane (CAS: 143142-90-9, 143 mg, 1.28 mmol) were added thereto, and the reaction system was reacted at 65°C for 16 hours in a sealed tube. The reaction system was extracted with EtOAc (50 mL × 3), and the organic phase was dried and concentrated to obtain a crude product, which was purified by preparative separation (preparation method: chromatographic column: Welch Xtimate@ C18 21.2 × 250 mm; column temperature: 25°C; mobile phase: water (10 mM/L NH₄HCO₃)-acetonitrile; proportion of acetonitrile in the mobile phase: 55% to 75% 12 min; flow rate: 30 mL/min) to obtain the title compound **132** (200 mg). LC-MS (ESI): m/z 546.2 [M+H]+.

### Preparation of compounds 132A and 132B

Compound **132** (200 mg) was subjected to SFC chiral preparative resolution (preparative separation method, instrument model: MG II preparative SFC (SFC-14); chromatographic column: ChiralCel OX, 250 × 30 mm I.D., 10 µm; mobile phase: A: CO₂ B: ethanol (0.1% NH₃H₂O); elution gradient: B 10%; flow rate: 60 mL/min; column pressure: 100 bar; chromatographic column temperature: 38°C; detection wavelength: 220 nM; cycle: about 23 min) to obtain the title compounds **132A** (46.5 mg) and **132B** (76.7 mg).

Compound **132A:** Chiral resolution (chromatographic column: Chiralcel OX-3 100 × 4.6 mm I.D., 3 µm; mobile phase: A: CO₂ B: ethanol (0.05% DEA); gradient: the mobile phase increased from 5% B to 40% B within 5 min and maintained at 40% B for 2.5 min for elution, then 5% B was equilibrated for 1.5 min; flow rate: 2.8 mL/min; chromatographic column temperature: 35°C; column pressure: 1500 psi; detection wavelength: 220 nM; RT = 2.642 min). LC-MS (ESI): m/z 546.2 [M+H]⁺. ¹H NMR (400 MHz, Chloroform-*d*) δ 8.08 (s, 1H), 7.83 (s, 1H), 7.74 (td, *J* = 9.0, 6.4 Hz, 1H), 7.11 - 7.00 (m, 2H), 6.89 - 6.81 (m, 3H), 6.76 (ddd, *J* = 12.3, 8.4, 2.5 Hz, 1H), 5.28 (s, 1H), 5.20 (d, *J* = 14.2 Hz, 1H), 4.82 (d, *J* = 14.2 Hz, 1H), 4.34 (pd, *J* = 6.7, 3.4 Hz, 1H), 4.20 (dd, *J* = 10.0, 3.4 Hz, 1H), 4.12 (dd, *J* = 10.1, 6.3 Hz, 1H), 2.06 (dd, *J* = 9.4, 1.9 Hz, 3H), 1.91 (dd, *J* = 9.5, 1.9 Hz, 3H).

¹⁹F NMR (376 MHz, Chloroform-*d*) δ -77.53 (s, 3F), -104.61 - -105.85 (m, 1F), - 108.36 (d, 2F), -109.57 - -112.18 (m, 1F).

Compound **132B:** Chiral resolution (chromatographic column: Chiralcel OX-3 100 × 4.6 mm I.D., 3 µm; mobile phase: A: CO₂ B: ethanol (0.05% DEA); gradient: the mobile phase increased from 5% B to 40% B within 5 min and maintained at 40% B for 2.5 min for elution, then 5% B was equilibrated for 1.5 min; flow rate: 2.8 mL/min; chromatographic column temperature: 35°C; column pressure: 1500 psi; detection wavelength: 220 nM; RT = 2.254 min). LC-MS (ESI): m/z 546.2 [M+H]⁺. ¹H NMR (400 MHz, Chloroform-*d*) δ 8.12 (s, 1H), 7.83 (s, 1H), 7.74 (td, *J* = 9.0, 6.5 Hz, 1H), 7.10 - 7.01 (m, 2H), 6.88 - 6.80 (m, 3H), 6.76 (ddd, *J* = 12.3, 8.4, 2.5 Hz, 1H), 5.29 (s, 1H), 5.20 (d, *J* = 14.2 Hz, 1H), 4.83 (d, *J* = 14.2 Hz, 1H), 4.34 (pd, *J* = 6.7, 3.4 Hz, 1H), 4.20 (dd, *J* = 10.1, 3.4 Hz, 1H), 4.12 (dd, *J* = 10.1, 6.3 Hz, 1H), 2.06 (dd, *J* = 9.4, 1.8 Hz, 3H), 1.91 (dd, *J* = 9.5, 1.9 Hz, 3H). ¹⁹F NMR (376 MHz, Chloroform-*d*) δ -77.53 (s, 3F), -104.67 - -105.79 (m, 1F), -108.36 (d, 2F), -109.67 - -112.12 (m, 1F).

### Example 133: Preparation of compound 133

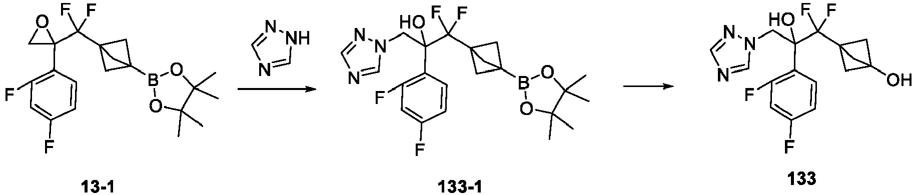

### Preparation of compound 133-1

1,2,4-Triazole (433 mg, 6.28 mmol) was dissolved in DMF (4 mL), and NaH (251 mg, 6.28 mmol) was added thereto at 0°C. The reaction system was stirred for 30 min, then added with compound **13-1** (500 mg, 1.26 mmol), transferred to room temperature to continue the reaction for 16 hours. The reaction system was extracted with EtOAc (50 mL × 3), and the organic phases were combined, dried, and concentrated to obtain the crude title compound **133-1** (250 mg), which was directly used in the next step. LC-MS (ESI): m/z 468.2 [M+H]⁺.

### Preparation of compound 133

Compound **133-1** (250 mg, 0.54 mmol) was dissolved in THF (2 mL) and saturated NaOH solution (1 mL). H₂O₂ (2 mL) was added dropwise to the reaction system at -10°C, and the reaction was continued for 10 min. The reaction mixture was extracted with EtOAc (50 mL × 3), and the organic phases were combined, dried, and concentrated to obtain a crude product. The crude product was purified by preparative separation (chromatographic column: Agilent 10 Prep-C18 250 × 21.2 mm; column temperature: 25°C; mobile phase: water (0.1% FA)-acetonitrile; proportion of acetonitrile in the mobile phase: 20% to 40% in 12 min; flow rate: 30 mL/min) to obtain the title compound **133** (42 mg).

LC-MS (ESI): m/z 358.0 [M+H]⁺. ¹H NMR (400 MHz, DMSO-d6) δ 8.34 (s, 1H), 7.67 (s, 1H), 7.56 - 7.46 (m, 1H), 7.24 - 7.12 (m, 1H), 7.03 - 6.92 (m, 1H), 6.81 (s, 1H), 6.30 (s, 1H), 5.10 (d, *J* = 14.4 Hz, 1H), 4.71 (d, *J* = 14.5 Hz, 1H), 1.75 (dd, *J* = 9.4, 1.4 Hz, 3H), 1.52 (dd, *J* = 9.3, 1.3 Hz, 3H). ¹⁹F NMR (376 MHz, DMSO-*d6*) δ -102.38 - -103.20 (m, 1F), -106.55 (d, *J* = 35.3 Hz, 2F), -110.59 (d, *J* = 8.7 Hz, 1F).

### Example 134: Preparation of compounds 134, 134A, and 134B

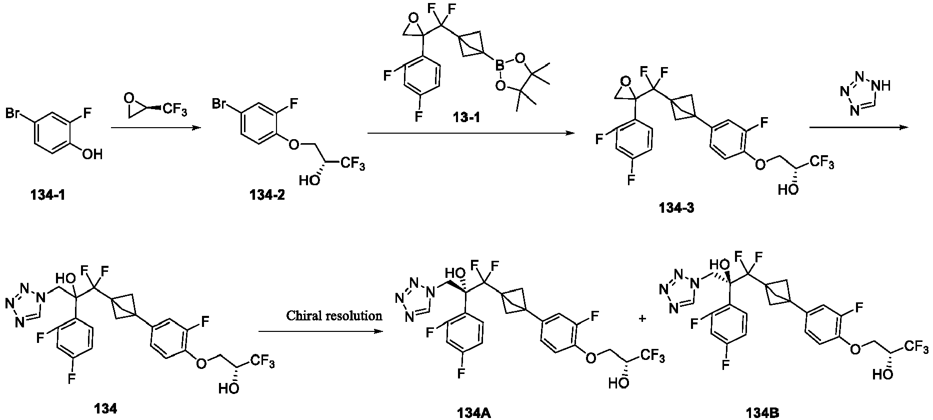

### Preparation of compound 134-2

Compound **134-1** (382 mg, 2 mmol) was dissolved in MeCN (10 mL), then potassium carbonate (552 mmol, 4 mmol) and R-(+)-2-trifluoromethyloxirane (CAS number: 143142-90-9, 336 mg, 3 mmol) were added thereto, and the reaction system was reacted at 60°C for 16 hours. The reaction system was extracted with EtOAc (50 mL × 3), and the organic phases were combined, dried, and concentrated to obtain a crude product. The crude product was purified by normal-phase silica gel column chromatography (EtOAc/PE = 5% to 20%) to obtain the title compound **134-2** (300 mg, yellow oil, yield: 49%). ¹H NMR (400 MHz, Chloroform-*d*) δ 7.35 -7.26 (m, 1H), 7.22 (ddd, *J* = 8.7, 2.4, 1.6 Hz, 1H), 6.89 (t, *J* = 8.7 Hz, 1H), 4.40 (td, *J* = 6.7, 3.3 Hz, 1H), 4.28 (dd, *J* = 10.1, 3.3 Hz, 1H), 4.20 (dd, *J* = 10.2, 6.4 Hz, 1H), 2.80 (br.s, 1H).

### Preparation of compound 134-3

Compound **13-1** (398 mg, 1 mmol) was dissolved in a mixed solvent of cyclopentyl methyl ether (5 mL) and water (2 mL), and then compound **134-2** (380 mg, 0.76 mmol), cuprous oxide (111 mg, 0.78 mmol), palladium (II) acetate (40.3 mg, 0.18 mmol), butyldi-1-adamantylphosphine (78 mg, 0.22 mmol), and cesium carbonate (1.0 g, 3 mmol) were sequentially added thereto. The reaction system was replaced with nitrogen three times, and then reacted under microwave irradiation at 120°C for 16 hours. The reaction system was cooled, then extracted with EtOAc (50 mL × 3), and the organic phases were combined, dried, and concentrated to obtain a crude product. The crude product was purified by normal-phase silica gel column chromatography (EtOAc/PE = 5 to 20%) to obtain the title compound **134-3** (300 mg), which was directly used in the next reaction step. ¹H NMR (400 MHz, DMSO-*d6*) δ 7.38 - 7.32 (m, 1H), 7.27 - 7.22 (m, 1H), 7.16 (td, *J* = 8.6, 2.2 Hz, 3H), 6.98 - 6.92 (m, 1H), 4.41 (dd, *J* = 7.2, 3.4 Hz, 1H), 4.25 - 4.20 (m, 1H), 4.13 (td, *J* = 6.7, 4.1 Hz, 1H), 3.57 (s, 1H), 3.38 (d, *J* = 4.7 Hz, 1H), 3.08 (dt, *J* = 4.4, 1.9 Hz, 1H), 2.05 (s, 6H).

### Preparation of compound 134

Compound **134-3** (300 mg, crude) was dissolved in DMF (10 mL), then potassium carbonate (167 mg, 1.2 mmol) and tetrazole (125.7 mg, 1.82 mmol) were added thereto, and the reaction system was stirred at 70°C for 16 hours in a sealed tube. After the reaction system was cooled, the reaction mixture was filtered, and the filtrate was purified by preparative separation (chromatographic column: Agilent 10 Prep-C18 250 × 21.2 mm; column temperature: 25°C; mobile phase: water (0.1% FA)-acetonitrile; proportion of acetonitrile in the mobile phase: 45% to 65% in 12 min; flow rate: 30 mL/min) to obtain the title compound **134** (89 mg, two-step yield: 20%).

LC-MS (ESI): m/z 565.2 [M+H]⁺. ¹H NMR (400 MHz, DMSO-*d6*) δ 9.14 (s, 1H), 7.54 (td, *J* = 9.0, 6.6 Hz, 1H), 7.28 (ddd, *J* = 12.1, 9.1, 2.7 Hz, 1H), 7.14 (dd, *J* = 18.7, 10.1 Hz, 2H), 7.01 (ddd, *J* = 11.0, 7.4, 2.4 Hz, 2H), 6.87 (dd, *J* = 8.4, 1.9 Hz, 1H), 6.79 - 6.57 (m, 1H), 5.43 (d, *J* = 14.5 Hz, 1H), 5.01 (d, *J* = 14.6 Hz, 1H), 4.39 (d, *J* = 6.9 Hz, 1H), 4.20 (dd, *J* = 10.6, 4.2 Hz, 1H), 4.09 (dd, *J* = 10.6, 6.5 Hz, 1H), 2.09 - 1.96 (m, 3H), 1.79 (dd, *J* = 9.4, 1.7 Hz, 3H). ¹⁹F NMR (376 MHz, DMSO-*d6*) δ -76.08 (s, 3F), -102.99 -103.18 (m, 1F), - 109.27-109.36 (m, 2F), -109.58 - -109.61 (m, 1F), -134.44 (s, 1F).

### Preparation of compounds 134A and 134B

Compound **134** (89 mg) was subjected to SFC chiral preparative resolution (preparative separation method, instrument model: MG II preparative SFC (SFC-14); chromatographic column: ChiralCel OJ, 250 × 30 mm I.D., 10 µm; mobile phase: A: CO₂ B: ethanol; elution gradient: B 20%; flow rate: 60 mL/min; column pressure: 100 bar; chromatographic column temperature: 38°C; detection wavelength: 220 nM; cycle: about 4.25 min) to obtain the title compounds **134B** (24 mg) and **134A** (30 mg).

Compound **134A:** Chiral resolution (chromatographic column: Cellulose OJ-3 150 × 4.6 mm I.D., 3 µm; mobile phase: A: CO₂ B: ethanol; elution gradient: the mobile phase increased from 5% B to 40% B within 5 min and maintained at 40% B for 2.5 min for elution, then 5% B was equilibrated for 2.5 min; flow rate: 2.5 mL/min; chromatographic column temperature: 35°C; column pressure: 1500 psi; detection wavelength: 220 nM; RT = 3.55 min). LC-MS (ESI): m/z 565.2 [M+H]⁺. ¹H NMR (400 MHz, DMSO-d6) δ 9.13 (s, 1H), 7.71 - 7.45 (m, 1H), 7.28 (ddd, *J* = 12.1, 9.1, 2.6 Hz, 1H), 7.19 - 7.07 (m, 2H), 7.07 - 6.96 (m, 2H), 6.92 - 6.83 (m, 1H), 6.68 (d, *J* = 6.7 Hz, 1H), 5.42 (d, *J* = 14.5 Hz, 1H), 5.00 (d, *J* = 14.6 Hz, 1H), 4.38 (dd, *J* = 7.1, 4.4 Hz, 1H), 4.19 (dd, *J* = 10.6, 4.1 Hz, 1H), 4.08 (dd, *J* = 10.6, 6.5 Hz, 1H), 1.99 (dd, *J* = 9.4, 1.8 Hz, 3H), 1.78 (dd, *J* = 9.5, 1.7 Hz, 3H). ¹⁹F NMR (376 MHz, DMSO-*d6*) δ -76.09 (s, 3F), -102.97 - -103.18 (m, 1F), -109.26 - -109.35 (m, 2F), -109.59 - - 110.98 (m, 1F), -134.45 (s, 1F).

Compound **134B:** Chiral resolution (chromatographic column: Cellulose OJ-3 150 × 4.6 mm I.D., 3 µm; mobile phase: A: CO₂ B: ethanol; elution gradient: the mobile phase increased from 5% B to 40% B within 5 min and maintained at 40% B for 2.5 min for elution, then 5% B was equilibrated for 2.5 min; flow rate: 2.5 mL/min; chromatographic column temperature: 35°C; column pressure: 1500 psi; detection wavelength: 220 nM; RT = 3.04 min). LC-MS (ESI): m/z 565.2 [M+H]⁺. ¹H NMR (400 MHz, DMSO-*d6*) δ 9.13 (s, 1H), 7.53 (td, *J =* 9.1, 6.8 Hz, 1H), 7.28 (ddd, *J =* 12.0, 9.1, 2.7 Hz, 1H), 7.19 - 7.08 (m, 2H), 7.06 - 6.95 (m, 2H), 6.86 (dt, *J* = 8.7, 1.4 Hz, 1H), 6.68 (s, 1H), 5.42 (d, *J* = 14.4 Hz, 1H), 5.01 (d, *J* = 14.5 Hz, 1H), 4.38 (s, 1H), 4.19 (dd, *J =* 10.6, 4.2 Hz, 1H), 4.08 (dd, *J =* 10.7, 6.5 Hz, 1H), 1.99 (dd, *J* = 9.5, 1.7 Hz, 3H), 1.78 (dd, *J* = 9.4, 1.7 Hz, 3H). ¹⁹F NMR (376 MHz, DMSO-d6) δ -76.09 (s, 3F), -102.97 - -103.18 (m, 1F), -109.25 - -109.35 (m, 2F), -109.59 - -109.61 (m, 1F), - 134.45 (s, 1F).

### Example 135: Preparation of compound 135

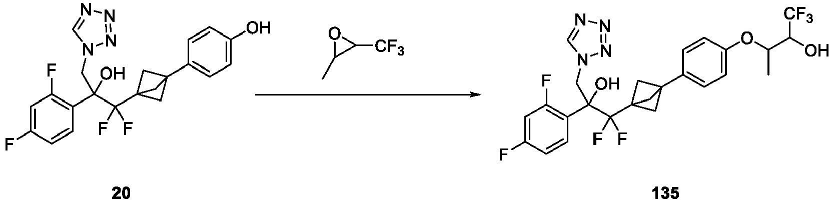

### Preparation of compound 135

Compound **20** (100 mg, 0.23 mmol) was dissolved in acetonitrile (2 mL), then potassium carbonate (48 mg, 0.35 mmol) and 1,1,1-trifluoro-2,3-epoxybutane (31 mg, 0.28 mmol) were added thereto, and the reaction system was reacted at 65°C for 16 hours in a sealed tube. The reaction system was extracted with EtOAc (50 mL × 3), and the organic phase was dried and concentrated to obtain a crude product, which was purified by preparative separation (preparation method: chromatographic column: Welch Xtimate^{®} C18 21.2 × 250 mm; column temperature: 25°C; mobile phase: water (10 mM/L NH₄HCO₃)-acetonitrile; proportion of acetonitrile in the mobile phase: 45% to 65% in 12 min; flow rate: 30 mL/min) to obtain the title compound **135** (41 mg, yield: 32%).

LC-MS (ESI): m/z 561.2 [M+H]⁺. ¹H NMR (400 MHz, Chloroform-*d*) δ 8.53 (s, 1H), 7.64 (td, *J* = 8.9, 6.3 Hz, 1H), 7.08 - 6.98 (m, 2H), 6.93 - 6.77 (m, 4H), 5.44 (d, *J* = 14.6 Hz, 1H), 5.05 (d, *J* = 14.6 Hz, 1H), 4.60 (qd, *J* = 6.3, 2.8 Hz, 1H), 3.91 (s, 1H), 3.83 (s, 1H), 2.96 (s, 1H), 2.04 (dd, *J* = 9.4, 1.8 Hz, 3H), 1.85 (dd, *J* = 9.5, 1.8 Hz, 3H), 1.39 (d, *J* = 6.3 Hz, 3H). ¹⁹F NMR (376 MHz, Chloroform-*d*) δ -76.70 (s, 3F), -105.45 (m, 1F), -106.90 (d, *J* = 9.7 Hz, 2F), -109.05 - -111.80 (m, 1F).

### Example 136: Preparation of compound 136

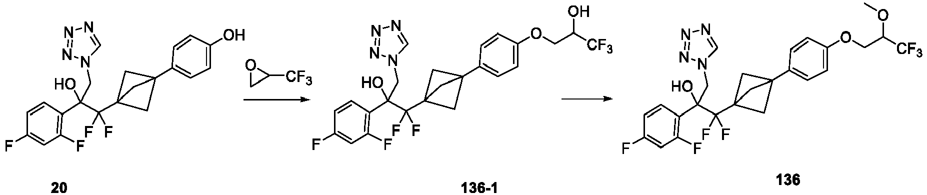

### Preparation of compound 136-1

Compound **20** (50 mg, 0.115 mmol) was dissolved in DMF (2 mL), then 1,1,1-trifluoro-2,3-epoxypropane (26 mg, 0.23 mmol) and cesium carbonate (75 mg, 0.23 mmol) were added thereto, and the reaction system was reacted at room temperature for 4 hours. The reaction system was added with ammonium chloride aqueous solution and extracted with EtOAc (50 mL × 3), and the organic phases were combined, dried, and concentrated to obtain a crude product. The crude product was purified by normal-phase silica gel column chromatography (EtOAc/PE = 0 to 50%) to obtain the title compound **136-1** (50 mg, yield: 79.6%). LC-MS (ESI): m/z 546.9 [M+H]⁺.

### Preparation of compound 136

To a suspension of NaH (5.5 mg, 0.138 mmol) in anhydrous THF (1 mL) was added compound **136-1** (50 mg, 0.092 mmol) at 0°C. The reaction system was stirred for 30 min, then added with iodomethane (14 mg, 0.1 mmol), and reacted at room temperature for 16 hours. The reaction mixture was poured into a mixture of ice water and ammonium chloride, extracted with EtOAc (50 mL × 3), and the organic phases were combined, dried, and concentrated to obtain a crude product, which was purified by preparative separation (preparative column: Pursuit XRs 10 C18 250 * 21.2 mm; flow rate: 25 mL/min; mobile phase: A: 0.1% FA aqueous solution, B: acetonitrile; gradient: 5 to 81% acetonitrile content, retention time of 7.2 to 7.7 min) to obtain the title compound **136** (2.80 mg, yield: 5.4%).

LC-MS (ESI): m/z 561.2 [M+H]⁺. ¹H NMR (400 MHz, DMSO-d6) δ 9.14 (s, 1H), 7.54 (d, *J* = 6.8 Hz, 1H), 7.27 (t, *J* = 9.5 Hz, 1H), 7.20 (s, 1H), 7.02 (dd, *J* = 21.4, 8.3 Hz, 3H), 6.88 (d, *J* = 8.6 Hz, 2H), 5.42 (d, *J* = 14.5 Hz, 1H), 5.02 (d, *J* = 14.7 Hz, 1H), 4.37 - 4.26 (m, 1H), 4.20 (dd, *J* = 10.9, 3.9 Hz, 1H), 4.09 (dd, *J* = 11.0, 6.0 Hz, 1H), 3.53 (s, 3H), 1.98 (d, *J* = 8.7 Hz, 3H), 1.77 (d, *J* = 9.2 Hz, 3H).

### Example 137: Preparation of compound 137

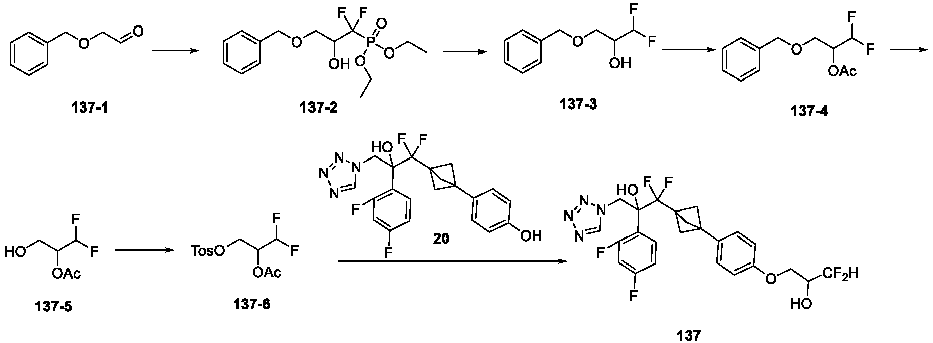

### Preparation of compound 137-2

Compound **137-1** (5.0 g, 2.85 mmol) was dissolved in anhydrous tetrahydrofuran (100 mL), and difluoromethylphosphonic acid diethyl ester (6.89 g, 36.62 mmol) was added thereto. The reaction system was added dropwise with LDA (2.0 M, 18.3 mL, 36.62 mmol) at a cooling temperature of -78°C under nitrogen atmosphere, stirred and reacted for 2 hours, then quenched with saturated NH₄Cl solution (20 mL), and extracted with EtOAc (100 mL × 3). The organic phases were combined, dried, and concentrated to obtain a crude product, and the crude product was purified by normal-phase silica gel column chromatography (EtOAc/PE = 0 to 5%) to obtain the title compound **137-2** (7.5 g, yield: 66%). ¹H NMR (400 MHz, Chloroform-*d*) δ 7.36 - 7.30 (m, 5H), 4.64 - 4.56 (m, 2H), 4.36 - 4.20 (m, 6H), 3.86 - 3.70 (m, 2H), 1.44 - 1.31 (m, 6H).

### Preparation of compound 137-3

Compound **137-2** (7.5 g, 22.17 mmol) was dissolved in anhydrous tetrahydrofuran (50 mL), and a solution of sodium methoxide in methanol (1.0 M, 34 mL, 34 mmol) was added dropwise thereto at 0°C. The reaction system was stirred and reacted at 50°C for 2 hours. The reaction system was extracted with EtOAc (100 mL × 3), and the organic phases were dried and concentrated to obtain a crude product. The crude product was purified by normal-phase silica gel column chromatography (EtOAc/PE = 0 to 100%) to obtain the title compound **137-3** (3 g, slightly yellow oil, yield: 67%). ¹H NMR (400 MHz, Chloroform-*d*) δ 7.37 - 7.30 (m, 5H), 5.95-5.66 (m, 1H), 4.57 (s, 2H), 3.98 - 3.85 (m, 1H), 3.71 - 3.55 (m, 2H), 2.25 (br.s, 1H).

### Preparation of compound 137-4

Compound **137-3** (1 g, 4.09 mmol) was dissolved in anhydrous dichloromethane (10 mL), then triethylamine (0.829 g, 8.19 mmol) was added thereto, and acetyl chloride (0.386 g, 4.91 mmol) was added dropwise thereto at 0°C. The reaction system was stirred and reacted at 50°C for 2 hours. The reaction mixture was added with dichloromethane (50 mL) and washed with water (20 mL × 3), and the organic phase was separated, dried, and concentrated to obtain a crude product. The crude product was purified by normal-phase silica gel column chromatography (EtOAc/PE = 0 to 100%) to obtain the title compound **137-4** (0.5 g, slightly yellow oil, yield: 50%). ¹H NMR (400 MHz, Chloroform-*d*) δ 7.36 - 7.30 (m, 5H), 6.11-5.82 (m, 1H), 5.22-5.16 (m, 1H), 4.62 - 4.47 (m, 2H), 3.71-3.70 (m, 2H), 2.14, 2.04 (s, 3H).

### Preparation of compound 137-5

Compound **137-4** (0.5 g, 2.05 mmol) was dissolved in anhydrous methanol (10 mL), and 10% Pd/C (50 mg) was added thereto. The reaction system was replaced with hydrogen three times, stirred and reacted at room temperature for 12 hours. The reaction system was filtered through diatomite, and the filtrate was concentrated to obtain crude product **137-5** (0.2 g) as a slightly yellow oil. The crude product was directly used in the next reaction step

### Preparation of compound 137-6

Compound **137-5** (200 mg, 1.3 mmol) was dissolved in dichloromethane (10 mL), and the reaction mixture was added dropwise with triethylamine (262 mg, 2.6 mmol) at 0°C, stirred for 10 min, then added with p-toluenesulfonyl chloride (247 mg, 1.3 mmol), and reacted at room temperature for 24 hours. After the reaction was tracked by TLC until the reaction was completed, the reaction system was extracted with DCM (50 mL × 3), and the organic phases were combined, dried, and concentrated. The resulting crude product was filtered through silica gel to obtain the title compound **137-6** (150 mg, yield: 37%). LC-MS (ESI): m/z 309.2 [M+H]⁺.

### Preparation of compound 137

Compound **20** (60 mg, 0.138 mmol) was dissolved in DMF (5 mL), then cesium carbonate (90 mg, 0.276 mmol) was added thereto. After stirring for 5 min, compound **137-6** (43 mg, 0.138 mmol) was added thereto, and the reaction system was stirred and reacted at 60°C for 24 hours. The reaction system was added with methanol (1 mL) and stirred for another one hour. The reaction mixture was filtered, the filtrate was concentrated to obtain a crude product, and the crude filtrate was purified by preparative separation (preparation method: chromatographic column: Agilent 10 Prep-C18 250 × 21.2 mm; column temperature: 25°C; mobile phase: water (0.1 % FA)-acetonitrile; proportion of acetonitrile in the mobile phase: 30% to 60% 12 min; flow rate: 30 mL/min) to obtain the title compound **137** (5 mg, yield: 7%). LC-MS (ESI): m/z 529.0 [M+H]⁺. ¹H NMR (400 MHz, Chloroform-*d*) δ 8.56 (s, 1H), 7.66-7.63 (m, 1H), 7.10 - 6.97 (m, 2H), 6.92 - 6.79 (m, 4H), 6.10 - 5.75 (m, 1H), 5.45 (d, *J* = 14.6 Hz, 1H), 5.05 (d, *J* = 14.6 Hz, 1H), 4.23 - 4.01 (m, 4H), 2.61 (br.s, 1H), 2.06 - 2.00 (m, 3H), 1.86-1.83 (m, 3H). ¹⁹F NMR (376 MHz, DMSO-*d6*) δ -102.99 - -103.19 (m, 1F), -108.90--109.88 (m, 3F), -128.17 - -131.24 (m, 2F).

### Example 138: Preparation of compound 138

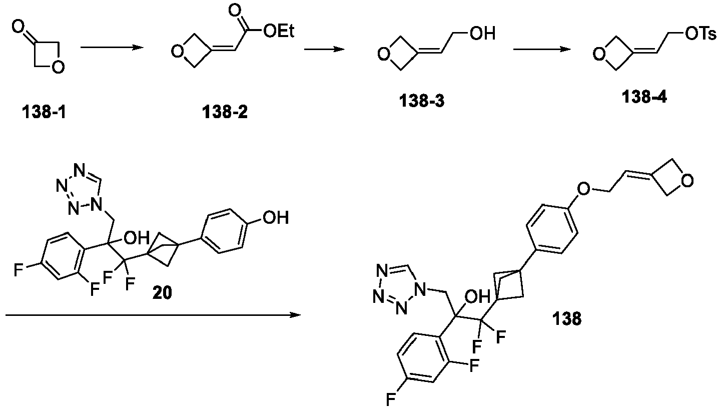

### Preparation of compound 138-2

To ethyl (triphenylphosphoranylidene)acetate (26.59 g, 76.3 mmol) was added a solution of compound **138-1** (5.0 g, 69.39 mmol) in anhydrous DCM (120 mL) in five batches at 0°C. Then the reaction system was transferred to room temperature and reacted for 3 hours. The reaction mixture was directly concentrated and evaporated to dryness by rotary evaporation, then dissolved in petroleum ether, filtered, and the filtrate was dried and concentrated to obtain a crude product. The crude product was purified by normal-phase silica gel column chromatography (EtOAc/PE = 0 to 25%) to obtain the title compound **138-2** (8.0 g, yield: 81.1%). ¹H NMR (400 MHz, CDCl₃) δ 5.64 (s, 1H), 5.50 (d, *J* = 2.7 Hz, 2H), 5.30 (dd, *J* = 2.2, 1.0 Hz, 2H), 4.17 (q, *J* = 7.1 Hz, 2H), 1.28 (t, *J* = 7.1 Hz, 3H).

### Preparation of compound 138-3

Compound **138-2** (4.0 g, 28.13 mmol) was dissolved in anhydrous THF (120 mL) solution at 0°C, and DIBAL-H (1.0 M, 112.5 mL, 112.5 mmol) was added dropwise thereto. After the dropwise addition, the reaction system was naturally warmed to room temperature and reacted for 5 hours. The reaction system was quenched by adding 5% potassium sodium tartrate aqueous solution and extracted with EtOAc (50 mL × 3), and the organic phases were combined, dried, and concentrated to obtain a crude product. The crude product was purified by normal-phase silica gel column chromatography (EtOAc/PE = 0 to 30%) to obtain the title compound **138-3** (860 mg, yield: 31%). ¹H NMR (400 MHz, CDCl₃) δ 5.77 (s, 1H), 4.70 - 4.66 (m, 2H), 4.65 - 4.61 (m, 2H), 4.27 (s, 2H), 2.03 (s, 1H).

### Preparation of compound 138-4

Compound **138-3** (550 mg, 5.5 mmol) was dissolved in anhydrous DCM (14 mL) at 0°C, and TEA (1.39 g, 13.74 mmol) and TsCl (1.10 mg, 5.77 mmol) were sequentially added dropwise to the reaction system. After the dropwise addition, the reaction system was naturally warmed to room temperature and reacted for 2 hours. The reaction mixture was quenched with water, extracted with EtOAc (50 mL × 3), and the organic phases were combined, dried, and concentrated to obtain a crude product (1.39 g), which was directly used in the next step.

### Preparation of compound 138

Compound **20** (1.58 g, 3.64 mmol) was dissolved in anhydrous DMF (15 mL), then compound **138-4** (1.39 g, 5.47 mmol) and Cs₂CO₃ (3.56 g, 10.93 mmol) were added thereto, and the reaction system was heated to 50°C and stirred for 6 hours. The reaction system was extracted with EtOAc (50 mL × 3), and the organic phases were combined, dried, and concentrated to obtain a crude product. The crude product was purified by preparative separation (preparation method: mobile phase: A 0.1% FA aqueous solution; B: acetonitrile; chromatographic column: Pursuit XRs 10 c18 250 × 21.2 mm; column temperature: 25°C; gradient: 65% to 85%; acetonitrile; flow rate: 20 mL/min) to obtain the title compound **138** (270 mg, yield: 15%). LC-MS (ESI): m/z 517.1 [M+H]⁺. ¹H NMR (400 MHz, DMSO-*d6*) δ 9.12 (s, 1H), 7.54 (dd, *J* = 15.9, 8.9 Hz, 1H), 7.27 (ddd, *J* = 11.9, 9.2, 2.5 Hz, 1H), 7.12 (s, 1H), 7.06 - 6.97 (m, 3H), 6.85 (d, *J* = 8.7 Hz, 2H), 5.92 (s, 1H), 5.42 (d, *J* = 14.5 Hz, 1H), 5.01 (d, *J* = 14.5 Hz, 1H), 4.66 (s, 2H), 4.53 (d, *J* = 2.1 Hz, 4H), 1.98 (d, *J* = 8.5 Hz, 3H), 1.77 (d, *J* = 9.4 Hz, 3H).

### Example 139: Preparation of compound 139

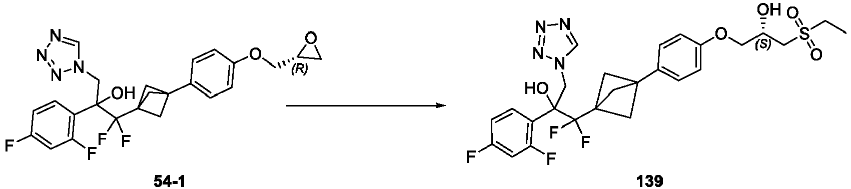

### Preparation of compound 139

Compound **54-1** (190 mg, 0.387 mmol) was dissolved in a mixed solvent of dioxane (2 mL) and water (1 mL), then sodium ethanethiolate (50 mg, 0.581 mmol) and sodium bicarbonate (82 mg, 0.968 mmol) were added thereto, and the reaction system was reacted at 80°C for 16 hours. The reaction system was extracted with EtOAc (20 mL × 3), and the organic phases were combined, dried, and concentrated to obtain a crude product. The crude product was purified by preparative separation (preparation method: mobile phase: A 10 mmol ammonium bicarbonate solution; B: acetonitrile; chromatographic column: Xbridge C18 21.2 * 250 mm * 10 µm; column temperature: 25°C; gradient: 45% to 50% acetonitrile 8.0 to 9 min; flow rate: 20 mL/min) to obtain the title compound **139** (56 mg, yield: 25%). LC-MS (ESI): m/z 585.0 [M+H]⁺. ¹H NMR (400 MHz, DMSO-d6) δ 9.13 (s, 1H), 7.59 - 7.50 (m, 1H), 7.32 - 7.24 (m, 1H), 7.13 (s, 1H), 7.02 (dd, *J* = 16.1, 8.4 Hz, 3H), 6.84 (d, *J* = 8.6 Hz, 2H), 5.66 (d, *J* = 5.7 Hz, 1H), 5.43 (d, *J* = 14.5 Hz, 1H), 5.01 (d, *J* = 14.6 Hz, 1H), 4.26 (d, *J* = 5.4 Hz, 1H), 3.91 (q, *J* = 7.4 Hz, 2H), 3.36 (d, *J* = 8.8 Hz, 1H), 3.15 (qd, *J =* 14.1, 7.1 Hz, 3H), 1.98 (d, *J* = 9.3 Hz, 3H), 1.77 (d, *J* = 9.3 Hz, 3H), 1.22 (t, *J* = 7.4 Hz, 3H).

### Example 140: Preparation of compound 140

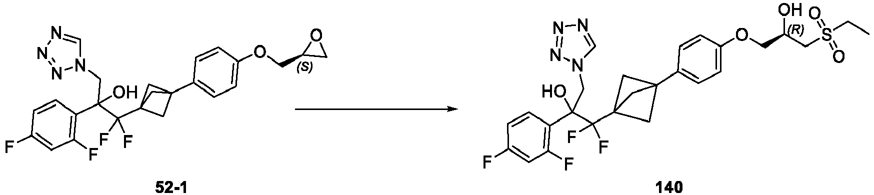

### Preparation of compound 140

Compound **52-1** (190 mg, 0.387 mmol) was dissolved in a mixed solvent of dioxane (2 mL) and water (1 mL), then sodium ethanethiolate (50 mg, 0.581 mmol) and sodium bicarbonate (82 mg, 0.968 mmol) were added thereto, and the reaction system was reacted at 80°C for 16 hours. The reaction mixture was extracted with EtOAc (20 mL × 3), and the organic phases were combined, dried, and concentrated to obtain a crude product. The crude product was purified by preparative separation (preparation method: preparative column: Xbridge C18 21.2 * 250 mm * 10 µm; flow rate: 20 mL/min; mobile phase: A: 0.05% ammonia solution, B: acetonitrile; gradient: 44 to 49% acetonitrile content, retention time of 8.6 to 9.8 min) to obtain the title compound **140** (29.06 mg, yield: 12.9%). LCMS (ESI): m/z 585.0 [M+H]⁺. ¹H NMR (400 MHz, DMSO-d6) δ 9.13 (s, 1H), 7.54 (dd, *J* = 15.8, 8.9 Hz, 1H), 7.27 (ddd, *J* = 12.0, 9.1, 2.6 Hz, 1H), 7.13 (s, 1H), 7.04 (d, *J* = 8.6 Hz, 2H), 6.84 (d, *J* = 8.7 Hz, 2H), 5.66 (d, *J* = 5.6 Hz, 1H), 5.42 (d, *J* = 14.5 Hz, 1H), 5.01 (d, *J* = 14.7 Hz, 1H), 4.28 - 4.20 (m, 1H), 3.91 (d, *J* = 5.4 Hz, 2H), 3.38 - 3.33 (m, 1H), 3.15 (qd, *J* = 14.1, 7.0 Hz, 4H), 1.98 (d, *J* = 9.3 Hz, 3H), 1.77 (d, *J* = 9.4 Hz, 3H), 1.23 (d, *J* = 7.4 Hz, 3H).

### Example 141: Preparation of compound 141

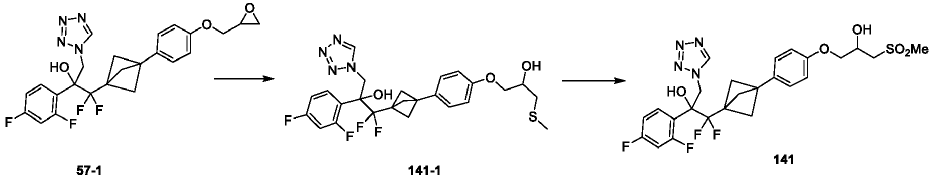

### Preparation of compound 141-1

Compound **57-1** (102 mg, 0.21 mmol) was dissolved in a mixed solvent of dioxane (2 mL) and water (1 mL), then sodium thiomethoxide (25.6 mg, 0.355 mmol) and sodium bicarbonate (38.2 mg, 1.025 mmol) were added thereto, and the reaction system was reacted at 80°C for 16 hours. The reaction system was extracted with EtOAc (50 mL × 3), dried, concentrated, and then subjected to a silica gel plate (EtOAc/PE = 50%) to obtain compound **141-1** (62 mg, yield: 60.8%). LC-MS (ESI): m/z 539. 1 [M+H]⁺.

### Preparation of compound 141

Compound **141-1** (62 mg, 0.115 mmol) was dissolved in DCM (1 mL), then 3-chloroperoxybenzoic acid (89.52 mg, 0.685 mmol) was added thereto, and the reaction system was reacted at room temperature for 2 hours. The reaction system was extracted with EtOAc (50 mL × 3), and the organic phases were combined, dried, and concentrated to obtain a crude product, which was purified by preparative separation (preparative column: Pursuit XRs 10 C18 250 * 21.2 mm; flow rate: 25 mL/min; mobile phase: A: 0.1% FA aqueous solution, B: acetonitrile; gradient: 5 to 49% acetonitrile content, retention time of 10.0 to 10.3 min) to obtain the title compound **141** (8.3 mg). LC-MS (ESI): m/z 571.1 [M+H]⁺. ¹H NMR (400 MHz, DMSO-*d6*) δ 9.13 (s, 1H), 7.54 (dd, *J* = 15.8, 9.0 Hz, 1H), 7.27 (ddd, *J* = 11.8, 9.1, 2.5 Hz, 1H), 7.16 (s, 1H), 7.07 - 6.96 (m, 3H), 6.84 (d, *J* = 8.6 Hz, 2H), 5.68 (s, 1H), 5.42 (d, *J* = 14.5 Hz, 1H), 5.01 (d, *J* = 14.6 Hz, 1H), 4.26 (s, 1H), 3.91 (d, *J* = 5.3 Hz, 2H), 3.41 - 3.36 (m, 1H), 3.20 (d, *J* = 14.6 Hz, 1H), 3.02 (s, 3H), 1.98 (d, *J* = 8.5 Hz, 3H), 1.77 (d, *J* = 9.4 Hz, 3H).

### Example 142: Preparation of compounds 142A, 142B, 142C, and 142D

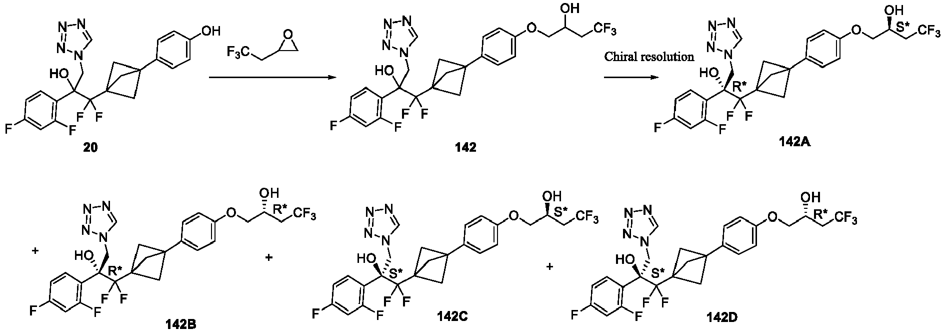

### Preparation of compound 142

Compound **20** (100 mg, 0.230 mmol) was dissolved in DMF (2 mL), then potassium carbonate (48 mg, 0.350 mmol) and 2-(2,2,2-trifluoroethyl)oxirane (32 mg, 0.253 mmol) were added thereto, and the reaction system was reacted at 80°C for 16 hours. The reaction system was extracted with EtOAc (50 mL × 3), and the organic phases were combined, dried, and concentrated to obtain a crude product, which was purified by preparative separation (preparation method: preparative column: Pursuit XRs 10 C18 250 * 21.2 mm; flow rate: 25 mL/min; mobile phase: A: 0.1% FA aqueous solution, B: acetonitrile; gradient: 64 to 64% acetonitrile content, retention time of 8.0 to 8.4 min) to obtain the title compound **142** (27.96 mg, yield: 21.7%). LC-MS (ESI): m/z 561.1 [M+H]⁺. ¹H NMR (400 MHz, DMSO-*d6*) δ 9.15 (s, 1H), 7.54 (d, *J* = 7.1 Hz, 1H), 7.31 - 7.24 (m, 1H), 7.20 (s, 1H), 7.02 (dd, *J* = 15.1, 8.2 Hz, 3H), 6.84 (d, *J* = 8.1 Hz, 2H), 5.43 (d, *J* = 14.4 Hz, 2H), 5.02 (d, *J* = 14.6 Hz, 1H), 4.08 (s, 1H), 3.85 (dd, *J* = 10.8, 5.2 Hz, 2H), 2.53 (s, 1H), 2.38 (d, *J* = 9.5 Hz, 1H), 1.98 (d, *J* = 9.3 Hz, 3H), 1.76 (d, *J* = 9.4 Hz, 3H).

### Preparation of compounds 142A, 142B, 142C, and 142D

Compound **142** (80 mg) was subjected to SFC chiral preparative resolution (preparative separation method: instrument model: Waters SFC 150; model of chromatographic column: DAICELCHIRALCEL^{®}OZ, 250 * 25 mm 10 µm; mobile phase: A: CO₂ B: MeOH (0.1% 7.0 mol/L ammonia methanol); elution gradient: B 30%, flow rate: 100 mL/min; column pressure: 100 bar; column temperature: RT; detection wavelength: 214 nm; cycle: 1.8 min) to obtain a mixture (35 mg) of **142A** and **142B,** and a mixture (30 mg) of **142C** and **142D.**

The **mixture** (35 mg) of **142A** and **142B** was subjected to SFC chiral preparative resolution (preparative separation method: instrument model: Waters SFC 150; model of chromatographic column: DAICELCHIRALPAK^{®}IG, 250 * 25 mm 10 µm; mobile phase: A: CO₂ and B for MeOH (0.1% 7.0 mol/L ammonia methanol solution); elution gradient: B 20%, flow rate: 70 mL/min; column pressure: 100 bar; column temperature: RT; detection wavelength: 214 nm; cycle: 2.4 min) to obtain the title compounds **142A** (17.76 mg) and **142B** (22.02 mg).

Compound **142A:** Chiral analysis method (model of chromatographic column: DAICELCHIRALPAK^{®}IG 100 * 3 mm 3 µm; mobile phase: A: CO₂, B: MeOH (0.1% DEA); elution gradient: maintained 5% B for the first 0.5 min, 5% B to 40% B for 0.5 to 5.5 min, maintained 40% B for 5.5 to 8 min; flow rate: 1.5 mL/min; column temperature: 35°C; column pressure: 1800 psi; detection wavelength: 214 nm; RT = 3.912 min). LC-MS(ESI): m/z 561.1[M+H]⁺. ¹H NMR (400 MHz, DMSO-*d6*) δ 9.13 (s, 1H), 7.54 (dd, *J* = 15.9, 9.0 Hz, 1H), 7.31 - 7.22 (m, 1H), 7.13 (s, 1H), 7.06 - 6.98 (m, 3H), 6.84 (d, *J* = 8.6 Hz, 2H), 5.40 (dd, *J* = 16.4, 10.2 Hz, 2H), 5.01 (d, *J* = 14.5 Hz, 1H), 4.07 (dd, *J* = 9.8, 4.3 Hz, 1H), 3.90 - 3.80 (m, 2H), 2.55 (s, 1H), 2.42 - 2.34 (m, 1H), 1.98 (d, *J* = 8.4 Hz, 3H), 1.77 (d, *J* = 9.4 Hz, 3H).

Compound **142B:** Chiral analysis method (model of chromatographic column: DAICELCHIRALPAK^{®}IG 100 * 3 mm 3 µm; mobile phase: A: CO₂, B: MeOH (0.1% DEA); elution gradient: maintained 5% B for the first 0.5 min, 5% B to 40% B for 0.5 to 5.5 min, maintained 40% B for 5.5 to 8 min; flow rate: 1.5 mL/min; column temperature: 35°C; column pressure: 1800 psi; detection wavelength: 214 nm; RT = 3.713 min). LC-MS(ESI): m/z 561.1[M+H]⁺. ¹H NMR (400 MHz, DMSO-d6) δ 9.13 (s, 1H), 7.58 -7.50 (m, 1H), 7.28 (dd, *J* = 15.6, 6.1 Hz, 1H), 7.13 (s, 1H), 7.02 (dd, *J* = 15.1, 8.5 Hz, 3H), 6.84 (d, *J* = 8.6 Hz, 2H), 5.40 (dd, *J* = 16.1, 10.2 Hz, 2H), 5.01 (d, *J* = 14.6 Hz, 1H), 4.07 (d, *J* = 8.6 Hz, 1H), 3.90 - 3.79 (m, 2H), 2.55 (s, 1H), 2.38 (d, *J* = 8.9 Hz, 1H), 1.98 (d, *J* = 9.5 Hz, 3H), 1.77 (d, *J* = 9.4 Hz, 3H).

The **mixture** (30 mg) of **142C** and **142D** was subjected to SFC chiral resolution (preparative separation method: instrument model: Waters SFC 150; model of chromatographic column: DAICELCHIRALCEL^{®}AD, 250 * 25 mm 10 µm; mobile phase: A: CO₂ and B for MEOH (0.1% 7.0 mol/L ammonia methanol solution); elution gradient: B 20%, flow rate: 70 mL/min; column pressure: 100 bar; column temperature: RT; detection wavelength: 214 nm; cycle: 2.3 min) to obtain the compounds **142C** (4.49 mg) and **142D** (19.49 mg). Compound **142C:** Chiral analysis method (model of chromatographic column: DAICELCHIRALPAK^{®}AD 100 * 3 mm 3 µm; mobile phase: A: CO₂, B: MeOH (0.1 % DEA); elution gradient: maintained 5% B for the first 0.5 min, 5% B to 40% B for 0.5 to 5.5 min, maintained 40% B for 5.5 to 8 min; flow rate: 1.5 mL/min; column temperature: 35°C; column pressure: 1800 psi; detection wavelength: 214 nm; RT = 3.675 min).

LC-MS(ESI): m/z 561.1[M+H]⁺. ¹H NMR (400 MHz, DMSO-d6) δ 9.13 (s, 1H), 7.54 (dd, *J* = 15.6, 8.9 Hz, 1H), 7.27 (t, *J* = 10.8 Hz, 1H), 7.12 (s, 1H), 7.02 (dd, *J* = 15.1, 8.7 Hz, 3H), 6.83 (d, *J* = 8.7 Hz, 2H), 5.40 (dd, *J* = 17.0, 10.2 Hz, 2H), 5.01 (d, *J* = 14.4 Hz, 1H), 4.08 (s, 1H), 3.89 - 3.80 (m, 2H), 2.53 (s, 1H), 2.38 (dd, *J* = 21.9, 13.2 Hz, 1H), 1.98 (d, *J* = 9.4 Hz, 3H), 1.77 (d, *J* = 9.4 Hz, 3H).

Compound **142D:** Chiral analysis method (model of chromatographic column: DAICELCHIRALPAK^{®}AD 100 * 3 mm 3 µm; mobile phase: A: CO₂, B: MeOH (0.1 % DEA); elution gradient: maintained 5% B for the first 0.5 min, 5% B to 40% B for 0.5 to 5.5 min, maintained 40% B for 5.5 to 8 min; flow rate: 1.5 mL/min; column temperature: 35°C; column pressure: 1800 psi; detection wavelength: 214 nm; RT = 3.511 min). LC-MS(ESI): m/z 561.1 [M+H]⁺. ¹H NMR (400 MHz, DMSO-d6) δ 9.13 (s, 1H), 7.54 (d, *J* = 6.8 Hz, 1H), 7.26 (dd, *J* = 16.7, 7.3 Hz, 1H), 7.13 (s, 1H), 7.02 (dd, *J* = 15.1, 8.6 Hz, 3H), 6.83 (d, *J* = 8.6 Hz, 2H), 5.40 (dd, *J* = 16.6, 10.2 Hz, 2H), 5.01 (d, *J* = 14.7 Hz, 1H), 4.07 (d, *J* = 5.6 Hz, 1H), 3.91 - 3.79 (m, 2H), 2.55 (s, 1H), 2.42 - 2.33 (m, 1H), 1.98 (d, *J* = 9.4 Hz, 3H), 1.77 (d, *J* = 9.4 Hz, 3H).

### Example 143: Preparation of compound 143

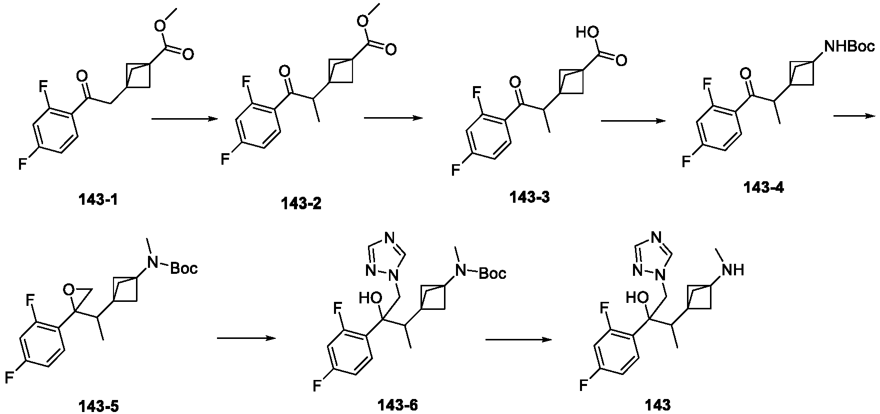

### Preparation of compound 143-2

Compound **143-1** (1.3 g, 1.9 mmol) was dissolved in tetrahydrofuran (13 mL), and LiHMDS (3.92 mL, 1.3 M) was slowly added dropwise thereto at -78°C. After the reaction system was stirred for one hour, iodomethane (724 mg, 5.10 mmol) was added thereto. After the reaction continued for two hours, the reaction system was added with saturated ammonium chloride solution (30 mL), extracted with EtOAc (50 mL × 3), and the organic phases were combined, dried, and concentrated to obtain a crude product. The crude product was purified by normal-phase silica gel column chromatography (EtOAc/PE = 0 to 30%) to obtain the title compound **143-2** (400 mg, yield: 29%). LC-MS (ESI): m/z 295.4 [M+H]⁺.

### Preparation of compound 143-3

Compound **143-2** (400 mg, 1.36 mmol) was dissolved in THF (2 mL), then lithium hydroxide aqueous solution (2 mL, 1 M) was added thereto, and the reaction system was reacted at room temperature for 2 hours. The reaction system was extracted with EtOAc (30 mL × 3), and the organic phases were combined, dried, and concentrated to obtain a crude product. The crude product was purified by normal-phase silica gel column chromatography (EtOAc/PE = 0 to 50%) to obtain the title compound **143-3** (250 mg, yield: 65%). LC-MS (ESI): m/z 281.4 [M+H]⁺.

### Preparation of compound 143-4

Compound **143-3** (180 mg, 0.89 mmol) was dissolved in tert-butanol (2 mL), then triethylamine (90 mg, 0.89 mmol) and diphenylphosphoryl azide (245 mg, 0.89 mmol) were added thereto. The reaction system was stirred for 4 hours, then transferred to 80°C and stirred for 16 hours. The reaction system was cooled, then extracted with EtOAc (50 mL × 3), and the organic phases were combined, dried, and concentrated to obtain a crude product. The crude product was purified by normal-phase silica gel column chromatography (EtOAc/PE = 0 to 5%) to obtain the title compound **143-4** (150 mg, yield: 47%). ¹H NMR (400 MHz, Chloroform-J) δ 7.86 - 7.75 (m, 1H), 6.98 - 6.91 (m, 1H), 6.89 - 6.81 (m, 1H), 4.86 (s, 1H), 3.63 (q, *J* = 6.7 Hz, 1H), 1.93 - 1.75 (m, 6H), 1.41 (s, 9H), 1.16 (dd, *J* = 6.7, 1.1 Hz, 3H).

### Preparation of compound 143-5

Trimethylsulfonium iodide (435 mg, 2.13 mmol) was dissolved in a mixed solvent of DMSO (3 mL) and THF (1 mL), then NaH (85 mg, 60%, 2.13 mmol) was slowly added thereto at 0°C. The reaction system was stirred for 30 min, then added with compound **143-4** (150 mg, 0.43 mmol), transferred to 50°C, and stirred for 16 hours. The reaction system was cooled, then extracted with EtOAc (50 mL × 3), and the organic phases were combined, dried, and concentrated to obtain a crude product. The crude product was purified by normal-phase silica gel column chromatography (EtOAc/PE = 0 to 30%) to obtain the title compound **143-5** (100 mg, yield: 61%). LC-MS (ESI): m/z 324.2 [M+H-56]⁺.

### Preparation of compound 143-6

Compound 1,2,4-triazole (91 mg, 1.32 mmol) was dissolved in DMF (2 mL), and sodium hydride (60%, 31 mg, 1.32 mmol) was added thereto at 0°C. The reaction system was stirred for 30 min, added with compound **143-5** (100 mg, 0.26 mmol), transferred to 80°C, and stirred for 2 hours. The reaction system was cooled, then extracted with DCM (50 mL × 3), and the organic phases were combined, dried, and concentrated to obtain crude title compound **143-6** (100 mg, yield: 84%). LC-MS (ESI): m/z 393.2 [M+H-56]⁺.

### Preparation of compound 143

Compound **143-6** (100 mg, 0.22 mmol) was dissolved in TFA (2 mL), and the reaction system was stirred at room temperature for 16 hours. The reaction mixture was evaporated to dryness by rotary evaporation, and the crude product was purified by preparative separation (preparation method: chromatographic column: Welch Xtimate^{®} C18 21.2 × 250 mm; column temperature: 25°C; mobile phase: water (10 mM/L NH₄HCO₃)-acetonitrile; proportion of acetonitrile in the mobile phase: 10% to 30% in 12 min; flow rate: 30 mL/min) to obtain the title compound **143** (50 mg, yield: 64%). LC-MS (ESI): m/z 349.2 [M+H]⁺. ¹H NMR (400 MHz, DMSO-d6) δ 8.96 (br.s, 1H), 8.22 (s, 1H), 7.62 (s, 1H), 7.31 - 7.24 (m, 1H), 7.16 - 7.09 (m, 1H), 6.94 - 6.79 (m, 1H), 5.60 (s, 1H), 4.59 (q, *J* = 14.4 Hz, 2H), 2.62 (d, *J* = 7.0 Hz, 1H), 2.34 (s, 3H), 1.58 (d, *J* = 9.2 Hz, 3H), 1.38 (d, *J* = 9.2 Hz, 3H), 1.13 (d, *J* = 6.9 Hz, 3H). ¹⁹F NMR (376 MHz, DMSO-d6) δ -107.01 (d, 1F), -112.53 (d, 1F).

### Example 144: Preparation of compounds 144, 144A, 144B, 144C, and 144D

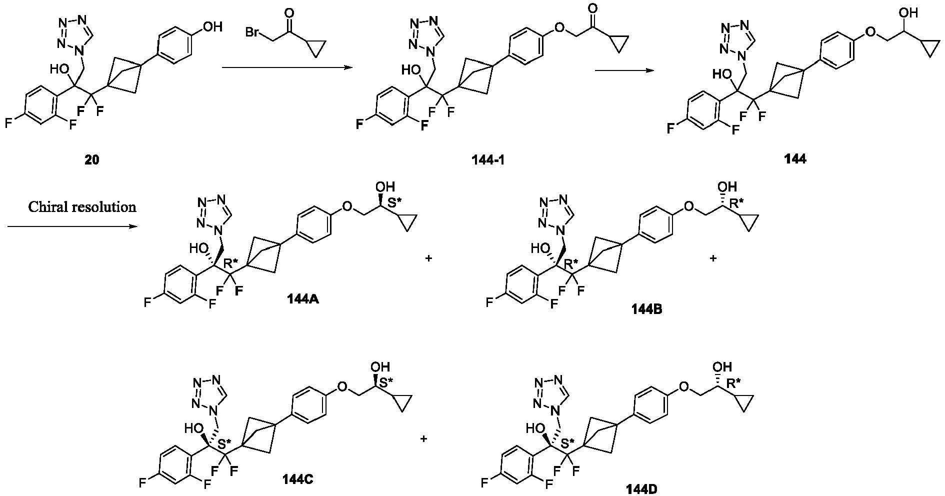

### Preparation of compound 144-1

Compound 20 (200 mg, 0.460 mmol) was dissolved in anhydrous DMF (3 mL), then 2-bromo-1-cyclopropylethan-1-one (83 mg, 0.506 mmol) and cesium carbonate (225 mg, 0.690 mmol) were added thereto, and the reaction system was reacted at 90°C for 16 hours in a sealed tube. The reaction system was extracted with EtOAc (50 mL × 3), and the organic phases were combined, dried, and concentrated to obtain a crude product. The crude product was purified by normal-phase silica gel column chromatography (EtOAc/PE = 0 to 100%) to obtain the title compound **144-1** (150 mg, yield: 63%). LC-MS (ESI): m/z 517.2 [M+H]⁺.

### Preparation of compound 144

Compound **144-1** (150 mg, 0.290 mmol) was dissolved in methanol (5 mL), then sodium borohydride (55 mg, 1.45 mmol) was slowly added thereto at 0°C, and the reaction system was stirred and reacted for another 2 hours. The reaction system was quenched with water (5.0 mL) and extracted with EtOAc (20 mL × 3), and the organic phases were combined, dried, and concentrated to obtain a crude product. The crude product was purified by preparative separation (preparation method: mobile phase: A 0.05% ammonia solution; B: acetonitrile; chromatographic column: Xbridge C18 21.2 * 250 mm * 10 µm; column temperature: 25°C; gradient: 53 to 60% acetonitrile content, retention time of 9.5 to 10.0 min; flow rate: 20 mL/min) to obtain the title compound **144** (115.54 mg, yield: 76%). LC-MS (ESI): m/z 519.2 [M+H]⁺. ¹H NMR (400 MHz, MeOD) δ 8.90 (s, 1H), 7.63 (td, *J* = 9.0, 6.7 Hz, 1H), 7.00 (d, *J* = 8.6 Hz, 2H), 6.92 (ddd, *J* = 16.9, 10.0, 4.5 Hz, 2H), 6.83 (d, *J* = 8.6 Hz, 2H), 5.59 (d, *J* = 14.4 Hz, 1H), 4.99 (d, *J* = 14.6 Hz, 1H), 4.01 (dd, *J* = 9.8, 3.6 Hz, 1H), 3.92 (dd, *J* = 9.8, 6.9 Hz, 1H), 3.25 - 3.18 (m, 1H), 2.10 - 1.96 (m, 3H), 1.83 - 1.75 (m, 3H), 0.97 (qd, *J* = 8.2, 4.1 Hz, 1H), 0.64 - 0.45 (m, 2H), 0.42 - 0.23 (m, 2H).

### Preparation of compounds 144A, 144B, 144C, and 144D

Compound **144** (400 mg) was subjected to SFC chiral preparative resolution (preparative separation method, instrument model: MG II preparative SFC (SFC-14); model of chromatographic column: ChiralCel OX, 250 × 30 mm I.D., 10 µm; mobile phase: A: CO₂ B: methanol (0.1% NH₃H₂O); elution gradient: B 30%; flow rate: 70 mL/min; column pressure: 100 bar; column temperature: 38°C; detection wavelength: 220 nm; cycle: about 10 min) to obtain the title compounds **144A** (86 mg), **144B** (82 mg), **144C** (85 mg), and **144D** (84 mg).

Compound **144A:** LC-MS (ESI): m/z 519.2 [M+H]⁺. ¹H NMR (400 MHz, DMSO-d6) δ 9.14 (s, 1H), 7.63 - 7.47 (m, 1H), 7.35 - 7.21 (m, 1H), 7.15 (s, 1H), 7.09 - 6.95 (m, 3H), 6.87 - 6.73 (m, 2H), 5.43 (d, *J* = 14.5 Hz, 1H), 5.01 (d, *J* = 14.6 Hz, 1H), 4.84 (d, *J* = 5.0 Hz, 1H), 3.96 - 3.78 (m, 2H), 3.27 - 3.21 (m, 1H), 1.97 (dd, *J* = 9.5, 1.7 Hz, 3H), 1.76 (dd, *J* = 9.5, 1.6 Hz, 3H), 0.98 - 0.83 (m, 1H), 0.43 - 0.33 (m, 2H), 0.31 - 0.22 (m, 2H). ¹⁹F NMR (376 MHz, DMSO-d6) δ -102.67 - -103.41 (m, 2F), -108.96 - -109.46 (m, 1F), -109.64 (d, 1F).

Compound **144B:** LC-MS (ESI): m/z 519.2 [M+H]⁺. ¹H NMR (400 MHz, DMSO-d6) δ 9.14 (s, 1H), 7.59 - 7.48 (m, 1H), 7.35 - 7.22 (m, 1H), 7.15 (s, 1H), 7.08 - 6.94 (m, 3H), 6.86 - 6.77 (m, 2H), 5.42 (d, *J* = 14.5 Hz, 1H), 5.01 (d, *J* = 14.6 Hz, 1H), 4.84 (d, *J* = 5.0 Hz, 1H), 3.96 - 3.77 (m, 2H), 3.28 - 3.20 (m, 1H), 1.97 (dd, *J* = 9.5, 1.6 Hz, 3H), 1.75 (dd, *J* = 9.5, 1.6 Hz, 3H), 0.95 - 0.80 (m, 1H), 0.43 - 0.31 (m, 2H), 0.31 - 0.21 (m, 2H). ¹⁹F NMR (376 MHz, DMSO-d6) δ -102.67 - -103.41 (m, 2F), -108.96 - -109.46 (m, 1F), -109.64 (d, 1F).

Compound **144C:** LC-MS (ESI): m/z 519.2 [M+H]⁺. ¹H NMR (400 MHz, DMSO-d6) δ 9.13 (s, 1H), 7.60 - 7.49 (m, 1H), 7.33 - 7.24 (m, 1H), 7.15 (s, 1H), 7.06 - 6.96 (m, 3H), 6.87 - 6.78 (m, 2H), 5.42 (d, *J* = 14.5 Hz, 1H), 5.01 (d, *J* = 14.6 Hz, 1H), 4.84 (d, *J* = 5.1 Hz, 1H), 3.95 - 3.78 (m, 2H), 3.28 - 3.20 (m, 1H), 1.97 (dd, *J* = 9.4, 1.7 Hz, 3H), 1.75 (dd, *J* = 9.4, 1.6 Hz, 3H), 0.95 - 0.80 (m, 1H), 0.42 - 0.31 (m, 2H), 0.31 - 0.21 (m, 2H). ¹⁹F NMR (376 MHz, DMSO-d6) δ -102.67 - -103.41 (m, 2F), -108.96 - -109.46 (m, 1F), -109.64 (d, 1F).

Compound **144D:** LC-MS (ESI): m/z 519.2 [M+H]⁺. ¹H NMR (400 MHz, DMSO-d6) δ 9.13 (s, 1H), 7.60 - 7.49 (m, 1H), 7.33 - 7.24 (m, 1H), 7.15 (s, 1H), 7.06 - 6.96 (m, 3H), 6.87 - 6.78 (m, 2H), 5.42 (d, *J* = 14.5 Hz, 1H), 5.01 (d, *J* = 14.6 Hz, 1H), 4.84 (d, *J* = 5.1 Hz, 1H), 3.95 - 3.78 (m, 2H), 3.28 - 3.20 (m, 1H), 1.97 (dd, *J* = 9.4, 1.7 Hz, 3H), 1.75 (dd, *J* = 9.4, 1.6 Hz, 3H), 0.95 - 0.80 (m, 1H), 0.42 - 0.31 (m, 2H), 0.31 - 0.21 (m, 2H). ¹⁹F NMR (376 MHz, DMSO-d6) δ -102.67 - -103.41 (m, 2F), -108.96 - -109.46 (m, 1F), -109.64 (d, 1F).

### Example 145: Preparation of compound 145

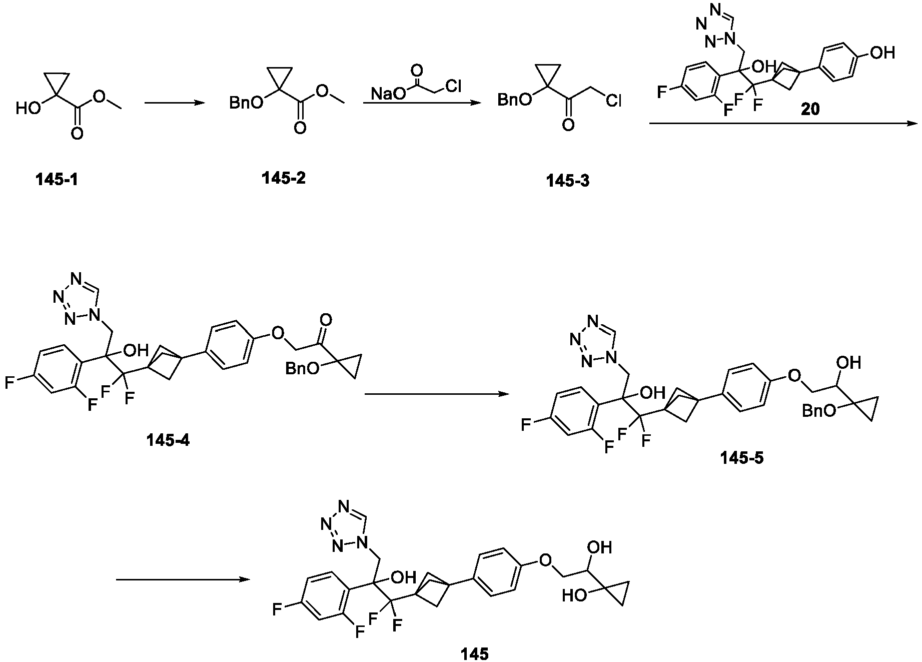

### Preparation of compound 145-2

To a suspension of NaH (380 mg, 9.48 mmol) in anhydrous THF (10 mL) was added compound **145-1** (1 g, 8.62 mmol) at 0°C. The reaction system was stirred and reacted for 30 min, then added with benzyl bromide (1.76 g, 10.3 mmol), transferred to room temperature, and reacted for 16 hours. The reaction system was poured into a mixture of ice water and ammonium chloride, and extracted with EtOAc (30 mL × 3), and the organic phases were combined, dried, and concentrated to obtain a crude product. The crude product was purified by normal-phase silica gel column chromatography (EtOAc/PE = 0 to 20%) to obtain the title compound **145-2** (650 mg, oily liquid, yield: 36.5%). ¹H NMR (400 MHz, CDCl₃) δ 7.34 (d, *J* = 6.8 Hz, 5H), 4.65 (s, 2H), 3.77 (s, 3H), 1.36 (dd, *J* = 5.9, 2.8 Hz, 2H), 1.25 (dd, *J* = 5.9, 2.8 Hz, 2H).

### Preparation of compound 145-3

Compound **145-2** (200 mg, 0.97 mmol) was dissolved in anhydrous THF (1 mL) at 0°C, then sodium chloroacetate (170 mg, 1.46 mmol) and triethylamine (100 mg, 0.97 mmol) were added thereto, and then tert-butylmagnesium chloride (2.91 mL, 2.91 mmol, 1 M in THF) was added dropwise thereto. The reaction system was reacted at room temperature for 16 hours. The reaction mixture was added with saturated ammonium chloride solution and extracted with EtOAc (30 mL × 3), and the organic phases were combined, dried, and concentrated to obtain a crude product. The crude product was purified by normal-phase silica gel column chromatography (EtOAc/PE = 0 to 20%) to obtain the title compound **145-2** (80 mg, oily liquid, yield: 36.8%). ¹H NMR (400 MHz, CDCl₃) δ 7.39 - 7.31 (m, 5H), 4.58 (s, 2H), 4.53 (s, 2H), 1.47 (dt, *J* = 7.1, 3.3 Hz, 2H), 1.40 - 1.36 (m, 2H).

### Preparation of compound 145-4

Compound **20** (231 mg, 0.53 mmol) was dissolved in DMF (3 mL), then compound **145-3** (180 mg, 0.8 mmol) and cesium carbonate (350 mg, 1.06 mmol) were added thereto, and the reaction system was reacted at room temperature for 4 hours. The reaction mixture was added with saturated ammonium chloride solution and extracted with EtOAc (30 mL × 3), and the organic phases were combined, dried, and concentrated to obtain a crude product. The crude product was purified by normal-phase silica gel column chromatography (EtOAc/PE = 0 to 50%) to obtain the title compound **145-4** (300 mg, yield: 91%). LC-MS (ESI): m/z 623.2 [M+H]⁺.

### Preparation of compound 145-5

Compound **145-4** (200 mg, 0.322 mmol) was dissolved in EtOH (2 mL) at 0°C, then sodium borohydride (25 mg, 0.644 mmol) was added thereto. After the reaction system stirred and reacted for 10 min, the reaction mixture was added with saturated ammonium chloride solution, extracted with dichloromethane (30 mL × 3), and the organic phases were combined, dried, and concentrated to obtain the title compound **145-5** (150 mg, white solid, yield: 74.6 %). LC-MS (ESI): m/z 625.3 [M+H]+.

### Preparation of compound 145

Compound **145-5** (70 mg, 0.112 mmol) was dissolved in anhydrous EtOAc (5 mL), and Pd/C (35 mg) was added thereto. The reaction system was replaced with hydrogen three times, stirred and reacted at room temperature for 2 hours. The reaction mixture was filtered and concentrated to obtain a crude product, which was purified by preparative separation (preparative column: Sunfire C18 21.2 * 250 mm * 10 µm; flow rate: 20 mL/min; mobile phase: A: 0.1% FA aqueous solution, B: acetonitrile; gradient: 46 to 50% acetonitrile content, retention time of 9.8 to 10.5 min) to obtain the title compound **145** (8.1 mg, yield: 13.5%).

LC-MS (ESI): m/z 576.3 [M+H+CH₃CN]⁺. ¹H NMR (400 MHz, DMSO-*d6*) δ 9.14 (s, 1H), 7.54 (dd, *J* = 15.8, 8.9 Hz, 1H), 7.27 (ddd, *J* = 11.9, 9.1, 2.5 Hz, 1H), 7.16 (s, 1H), 7.01 (dd, *J* = 15.6, 5.6 Hz, 3H), 6.82 (d, *J* = 8.7 Hz, 2H), 5.42 (d, *J* = 14.4 Hz, 1H), 5.26 (s, 1H), 5.01 (d, *J* = 14.7 Hz, 1H), 4.93 (d, *J* = 5.4 Hz, 1H), 4.17 (dd, *J* = 10.0, 3.1 Hz, 1H), 3.82 (dd, *J* = 9.9, 7.8 Hz, 1H), 3.54 (t, *J* = 8.2 Hz, 1H), 1.98 (d, *J* = 9.4 Hz, 3H), 1.76 (d, *J* = 9.3 Hz, 3H), 0.62 - 0.45 (m, 4H).

### Example 146: Preparation of compound 146

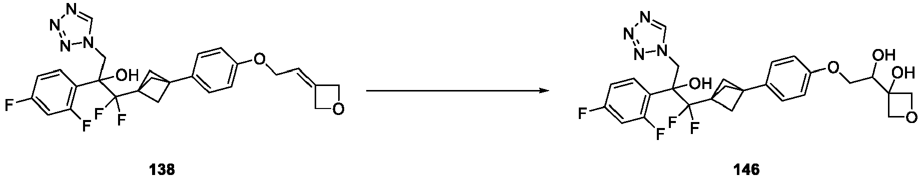

### Preparation of compound 146

(DHQP)2PHAL (0.75 mg, 0.001 mmol) was dissolved in a mixed solvent of t-BuOH (2 mL) and H₂O (2 mL), then K₂OsO₄.2H₂O (0.075 mg, 0.0002 mmol), K₃Fe(CN)₆ (98 mg, 0.30 mmol), K₂CO₃ (40 mg, 0.30 mmol), and MeSO₂NH₂ (10 mg, 0.10 mmol) were sequentially added thereto. The reaction system was stirred for 30 min, then added with compound **138** (50 mg, 0.10 mmol), and then stirred at room temperature for 16 hours. The reaction system was washed with saturated NaSO₃ and extracted with EtOAc (50 mL × 3), and the organic phases were combined, dried, and concentrated to obtain a crude product. The crude product was purified by preparative separation (preparation method: mobile phase: A 0.1% FA aqueous solution; B: acetonitrile; chromatographic column: Agilent C18 250 × 21.2 µm; column temperature: 25°C; gradient: 5% to 48%; acetonitrile; flow rate: 25 mL/min) to obtain the title compound **146** (20 mg, yield: 38%). LC-MS (ESI): m/z 551.2 [M+H]⁺. ¹H NMR (400 MHz, DMSO-*d6*) δ 9.12 (s, 1H), 7.54 (dd, *J* = 15.8, 8.8 Hz, 1H), 7.27 (t, *J* = 9.7 Hz, 1H), 7.12 (s, 1H), 7.03 (d, *J* = 8.5 Hz, 3H), 6.84 (d, *J* = 8.6 Hz, 2H), 5.42 (d, *J* = 14.6 Hz, 1H), 5.10 (s, 1H), 5.03 (s, 1H), 4.76 (s, 1H), 3.99 (s, 1H), 3.92 - 3.77 (m, 4H), 3.58 (d, *J* = 9.2 Hz, 1H), 3.50 (d, *J* = 2.4 Hz, 1H), 1.97 (d, *J* = 9.4 Hz, 3H), 1.76 (d, *J* = 9.1 Hz, 3H).

### Example 147: Preparation of compound 147

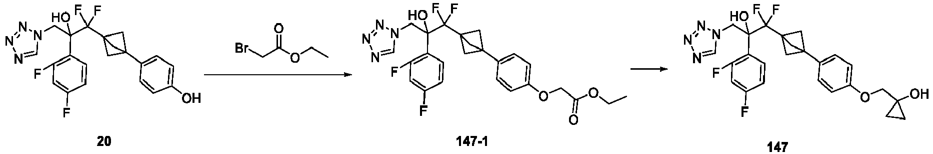

### Preparation of compound 147-1

Compound **20** (200 mg, 0.46 mmol) was dissolved in DMF (1.5 mL), then ethyl bromoacetate (83.4 mg, 0.50 mmol) and cesium carbonate (300 mg, 0.92 mmol) were added thereto, and the reaction system was reacted at 20°C for 16 hours. The reaction system was extracted with EtOAc (50 mL × 3), and the organic phases were combined, dried, and concentrated to obtain the crude title compound **147-1,** which was directly used in the next step. LC-MS (ESI): m/z 521.2 [M+H]⁺.

### Preparation of compound 147

Compound **147-1** (230 mg, crude) was dissolved in THF (10.0 mL), then titanium tetraisopropanolate (250 mg, 0.88 mol) was added thereto at 0°C. The reaction system was stirred for 30 min, then added dropwise with a solution of ethyl magnesium bromide in THF (2M, 1.3 mL, 1.3 mmol), slowly warmed to room temperature, and stirred for 16 hours. The reaction mixture was filtered, and the crude filtrate was purified by preparative separation (chromatographic column: Agilent 10 Prep-C18 250 × 21.2 mm; column temperature: 25°C; mobile phase: water (0.1 % FA)-acetonitrile; proportion of acetonitrile in the mobile phase: 40% to 60% in 12 min; flow rate: 30 mL/min) to obtain the title compound **147** (7 mg, two-step yield: 3.0%). LC-MS (ESI): m/z 505.2 [M+H]⁺. ¹H NMR (400 MHz, DMSO-d6) δ 9.13 (s, 1H), 7.53 (dd, *J* = 9.1, 6.8 Hz, 1H), 7.43 - 7.25 (m, 1H), 7.15 (s, 1H), 7.10 - 6.94 (m, 3H), 6.92 - 6.79 (m, 2H), 5.54 (s, 1H), 5.42 (d, *J* = 14.5 Hz, 1H), 5.01 (d, *J* = 14.6 Hz, 1H), 3.88 (s, 2H), 1.97 (dd, *J* = 9.3, 1.7 Hz, 3H), 1.76 (dd, *J* = 9.4, 1.7 Hz, 3H), 0.68 - 0.62 (m, 2H), 0.60 - 0.55 (m, 2H). ¹⁹F NMR (376 MHz, DMSO-*d6*) δ -102.95 - -103.16 (m, 1F), -109.17 - -109.18 (m, 2F), -109.64 - -109.67 (m, 1F).

### Example 148: Preparation of compound 148

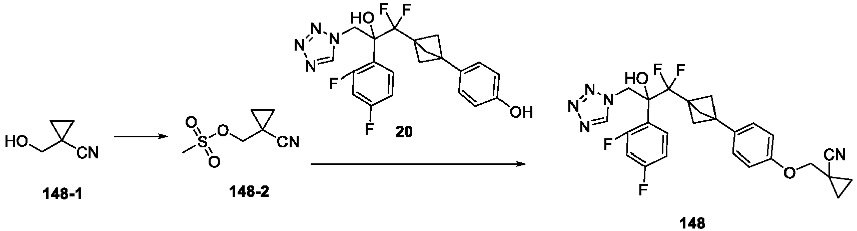

### Preparation of compound 148-2

Compound **148-1** (100 mg, 1.03 mmol) was dissolved in DCM (1 mL), then triethylamine (156 mg, 1.50 mmol) and MsCl (171 mg, 1.50 mmol) were added thereto, and the reaction system was reacted at room temperature for 16 hours. The reaction system was extracted with EtOAc (50 mL × 3), and the organic phases were combined, dried, and concentrated to obtain the crude title compound **148-2** (160 mg, yield: 89%). ¹H NMR (400 MHz, Chloroform-*d*) δ 4.18 (s, 2H), 3.13 (s, 3H), 1.49 - 1.43 (m, 2H), 1.20 - 1.14 (m, 2H).

### Preparation of compound 148

Compound **20** (100 mg, 0.23 mmol) was dissolved in DMF (1 mL), then compound **148-2** (40 mg, 1.0 mmol) and K₂CO₃ (63 mg, 2.0 mmol) were added thereto, and the reaction system was reacted at 55°C for 16 hours. After the reaction system was cooled, the reaction mixture was filtered, and the crude filtrate was purified by preparative separation (chromatographic column: Agilent 10 Prep-C18 250 × 21.2 mm; column temperature: 25°C; mobile phase: water (0.1% FA)-acetonitrile; proportion of acetonitrile in the mobile phase: 45% to 65% in 12 min; flow rate: 30 mL/min) to obtain the title compound **148** (45 mg). LC-MS (ESI): m/z 514.0 [M+H]⁺. ¹H NMR (400 MHz, DMSO-*d6*) δ 9.13 (s, 1H), 7.53 (td, *J* = 9.0, 6.7 Hz, 1H), 7.28 (ddd, *J* = 12.0, 9.0, 2.6 Hz, 1H), 7.15 (s, 1H), 7.10 - 6.96 (m, 3H), 6.89 - 6.79 (m, 2H), 5.42 (d, *J* = 14.5 Hz, 1H), 5.01 (d, *J* = 14.6 Hz, 1H), 3.96 (s, 2H), 1.98 (dd, *J* = 9.5, 1.7 Hz, 3H), 1.76 (dd, *J* = 9.5, 1.7 Hz, 3H), 1.38 - 1.28 (m, 2H), 1.16 - 1.06 (m, 2H). ¹⁹F NMR (376 MHz, DMSO-d6) δ -103.06 (ddd, *J* = 42.5, 33.5, 9.5 Hz, 1F), -109.27 (d, *J* = 36.8 Hz, 2F), -109.63 (d, *J* = 9.6 Hz, 1F).

### Example 149: Preparation of compounds 149, 149A, and 149B

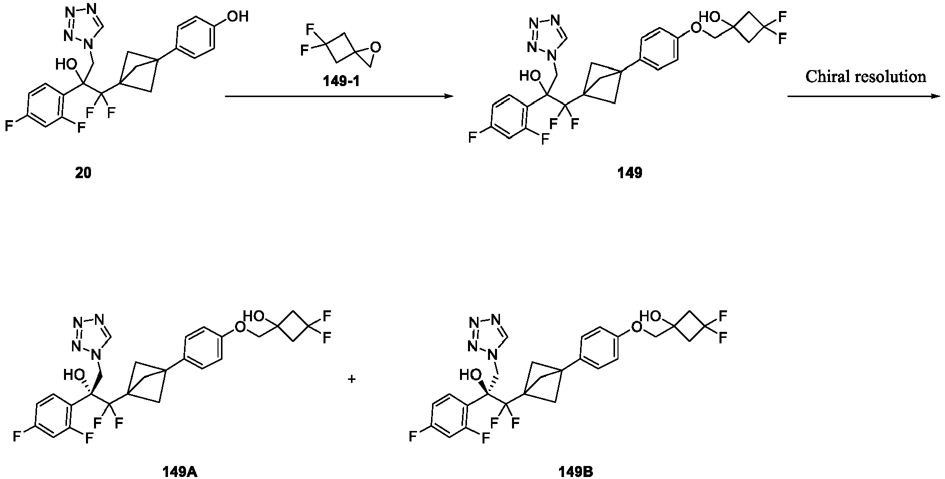

### Preparation of compound 149

Compound **20** (150 mg, 0.35 mmol) was dissolved in DMF (1.5 mL), then compound **149-1** (84 mg, 0.70 mmol, CAS: 1197420-24-8) and potassium carbonate (125 mg, 0.88 mmol) were added thereto. The reaction system was replaced with nitrogen three times, and reacted at 80°C for 16 hours. After the reaction system was cooled, the reaction mixture was filtered, and the filtrate was prepared by a reversed-phase chromatographic column (preparation column: Pursuit XRs 10 C18 250 * 21.2 mm; flow rate: 20 mL/min; mobile phase: A: 0.1% FA aqueous solution, B: acetonitrile; gradient: 63 to 64% acetonitrile content, retention time of 7.3 to 8.0 min) to obtain the title compound **149** (40.02 mg, yield: 22.0%). LC-MS (ESI): m/z 555.3 [M+H]⁺. ¹H NMR (400 MHz, DMSO-d6) δ 9.15 (s, 1H), 7.54 (d, *J* = 6.8 Hz, 1H), 7.27 (s, 1H), 7.03 (t, *J* = 8.0 Hz, 3H), 6.85 (d, *J* = 8.7 Hz, 2H), 5.79 (s, 1H), 5.42 (d, *J* = 14.4 Hz, 1H), 5.02 (d, *J* = 14.5 Hz, 1H), 3.89 (s, 2H), 2.87 - 2.73 (m, 2H), 2.57 (d, *J* = 13.1 Hz, 2H), 1.98 (d, *J* = 8.4 Hz, 3H), 1.77 (d, *J* = 9.4 Hz, 3H).

### Preparation of compounds 149A and 149B

Compound **149** (37 mg) was subjected to SFC chiral preparative resolution (preparative separation method, instrument model: MG II preparative SFC (SFC-1); chromatographic column: Cellulose-2, 250 × 30 mm I.D., 10 µm, mobile phase: A: CO₂ B: ethanol, elution gradient: B 40%, flow rate: 80 mL/min, column pressure: 100 bar, chromatographic column temperature: 38°C, detection wavelength: 220 nm, cycle: about 4 min) to obtain the title compounds **149A** (14.4 mg) and **149B** (15.3 mg).

Compound **149A:** Instrument model: Waters UPC2 analytical SFC (SFC-H), chromatographic column: Cellulose-2, 150 × 4.6 mm I.D., 3 µm, mobile phase: A: CO₂ B: ethanol (0.05% DEA), elution gradient: B 40%, flow rate: 2.5 mL/min, column pressure: 100 bar, chromatographic column temperature: 35°C, detection wavelength: 220 nm, Rt = 1.522 min. LC-MS (ESI): m/z 555.2 [M+H]⁺. ¹H NMR (400 MHz, Chloroform-*d*) δ 8.53 (s, 1H), 7.65 (td, *J* = 8.9, 6.3 Hz, 1H), 7.09 - 6.96 (m, 2H), 6.93 - 6.72 (m, 4H), 5.44 (d, *J* = 14.6 Hz, 1H), 5.05 (d, *J* = 14.6 Hz, 1H), 4.00 (s, 2H), 3.90 - 3.75 (m, 1H), 2.92 - 2.67 (m, 5H), 2.05 (dd, *J* = 9.5, 1.9 Hz, 3H), 1.86 (dd, *J* = 9.4, 1.9 Hz, 3H). ¹⁹F NMR (376 MHz, Chloroform-*d*) δ - 85.69 - -86.22 (d, 2F), -96.87 (d, *J* = 201.1 Hz, 2F), -106.89 (d, *J* = 9.5 Hz, 1F), -108.96 - - 111.56 (m, 1F).

Compound **149B:** Instrument model: Waters UPC2 analytical SFC (SFC-H), chromatographic column: Cellulose-2, 150 × 4.6 mm I.D., 3 µm, mobile phase: A: CO₂ B: ethanol (0.05% DEA), elution gradient: B 40%, flow rate: 2.5 mL/min, column pressure: 100 bar, chromatographic column temperature: 35°C, detection wavelength: 220 nm, Rt = 1.197 min. LC-MS (ESI): m/z 555.2 [M+H]⁺. ¹H NMR (400 MHz, Chloroform-*d*) δ 8.54 (s, 1H), 7.65 (td, *J* = 8.9, 6.3 Hz, 1H), 7.07 - 7.00 (m, 2H), 6.92 - 6.78 (m, 4H), 5.44 (d, *J* = 14.5 Hz, 1H), 5.05 (d, *J* = 14.6 Hz, 1H), 4.00 (s, 2H), 3.79 (s, 1H), 2.78 (dddd, *J =* 15.1, 10.7, 7.6, 4.7 Hz, 5H), 2.05 (dd, *J* = 9.5, 1.9 Hz, 3H), 1.86 (dd, *J* = 9.5, 1.9 Hz, 3H). ¹⁹F NMR (376 MHz, Chloroform-*d*) δ -85.69 - -86.22 (d, 2F), -96.87 (d, *J* = 201.1 Hz, 2F), -106.89 (d, *J* = 9.5 Hz, 1F), -108.96 - -111.56 (m, 1F).

### Example 150: Preparation of compounds 150, 150A, and 150B

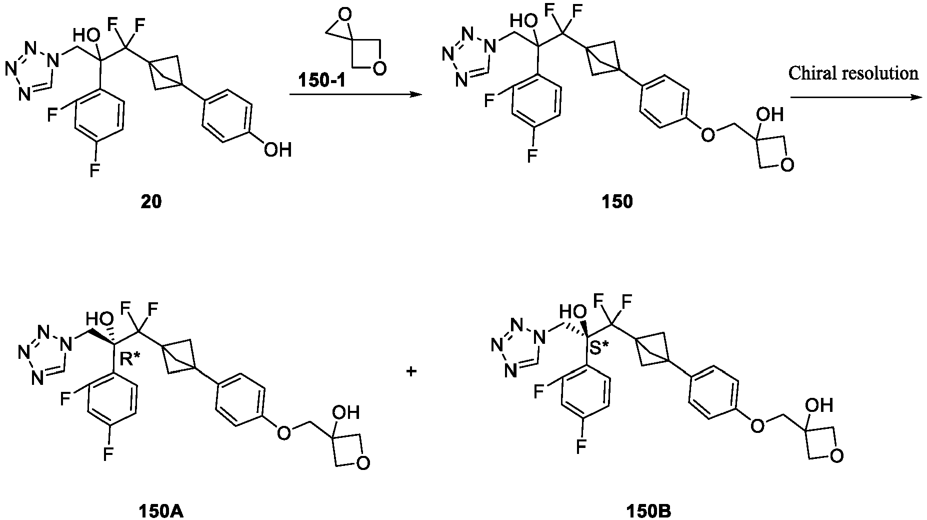

### Preparation of compound 150

Compound **20** (200 mg, 0.46 mmol) was dissolved in DMF (6 mL), then potassium carbonate (95 mg, 0.69 mmol) was added thereto. After stirring for 30 min, the reaction system was added with compound **150-1** (79 mg, 0.92 mmol), replaced with nitrogen three times, and reacted at 60°C for 16 hours. The reaction mixture was filtered, and the crude filtrate was purified by preparative separation (preparation method: mobile phase: A: 0.1% NH₄HCO₃ aqueous solution; B: acetonitrile; chromatographic column: Agilent 10 Prep-C18 250 × 21.2 mm; column temperature: 25°C; gradient: 35% to 55% acetonitrile in 12 min; flow rate: 30 mL/min) to obtain the title compound **150** (45 mg, yield: 18.8%). LC-MS (ESI): m/z 521.2 [M+H]⁺. ¹H NMR (400 MHz, DMSO-d6) δ 9.13 (s, 1H), 7.66 - 7.50 (m, 1H), 7.28 (ddd, *J* = 12.1, 9.1, 2.6 Hz, 1H), 7.15 (s, 1H), 7.10 - 6.99 (m, 3H), 6.92 - 6.85 (m, 2H), 5.99 (s, 1H), 5.43 (d, *J* = 14.5 Hz, 1H), 5.01 (d, *J* = 14.6 Hz, 1H), 4.57 - 4.39 (m, 4H), 4.03 (s, 2H), 1.98 (dd, *J* = 9.5, 1.7 Hz, 3H), 1.77 (dd, *J* = 9.5, 1.7 Hz, 3H). ¹⁹F NMR (376 MHz, DMSO-*d6)* δ -102.95 - -103.16 (m, 1F), -109.19 - -109.29 (m, 2F), -109.63 - -109.65 (m, 1F).

### Preparation of compounds 150A and 150B

Compound **150** (45 mg) was subjected to SFC chiral preparative resolution (preparative separation method, instrument model: MG II preparative SFC (SFC-14); chromatographic column: ChiralCel OX, 250 × 30 mm I.D., 3 µm; mobile phase: A: CO₂ B: ethanol (0.1% NH₃H₂O); elution gradient: B 40%; flow rate: 70 mL/min; column pressure: 100 bar; chromatographic column temperature: 35°C; detection wavelength: 220 nM; cycle: about 11.6 min) to obtain the title compounds **150A** (12.8 mg) and **150B** (14.7 mg).

Compound **150A:** LC-MS (ESI): m/z 521.2 [M+H]⁺. Chiral resolution (chromatographic column: Chiralpak OX-3 100 × 4.3 mm I.D., 3 µm; mobile phase: A: CO₂ B: ethanol (0.05% DEA); elution gradient: the mobile phase increased from 5% B to 40% B within 5 min and maintained at 40% B for 2.5 min for elution, then 5% B was equilibrated for 2.5 min; flow rate: 2.5 mL/min; chromatographic column temperature: 35°C; column pressure: 1500 psi; detection wavelength: 220 nM; RT = 2.295 min). ¹H NMR (400 MHz, DMSO-*d6*) δ 9.14 (s, 1H), 7.54 (td, *J* = 9.0, 6.7 Hz, 1H), 7.29 (ddd, *J* = 12.0, 9.1, 2.6 Hz, 1H), 7.15 (s, 1H), 7.09 - 6.98 (m, 3H), 6.95 - 6.81 (m, 2H), 5.99 (s, 1H), 5.43 (d, *J* = 14.4 Hz, 1H), 5.01 (d, *J* = 14.6 Hz, 1H), 4.53 - 4.40 (m, 4H), 4.03 (s, 2H), 1.98 (dd, *J* = 9.4, 1.7 Hz, 3H), 1.77 (dd, *J* = 9.4, 1.6 Hz, 3H). ¹⁹F NMR (376 MHz, DMSO-d6) δ -102.95 - -103.17 (m, 1F), -109.21 - - 109.31 (m, 2F), -109.62 - -109.65 (m, 1F).

Compound **150B:** LC-MS (ESI): m/z 521.2 [M+H]⁺. Chiral resolution (chromatographic column: Chiralpak OX-3 100 × 4.3 mm I.D., 3 µm; mobile phase: A: CO₂ B: ethanol (0.05% DEA); elution gradient: the mobile phase increased from 5% B to 40% B within 5 min and maintained at 40% B for 2.5 min for elution, then 5% B was equilibrated for 2.5 min; flow rate: 2.5 mL/min chromatographic column temperature: 35°C; column pressure: 1500 psi; detection wavelength: 220 nM; RT = 1.258 min). ¹H NMR (400 MHz, DMSO-d6) δ 9.14 (s, 1H), 7.54 (td, *J* = 9.0, 6.7 Hz, 1H), 7.29 (ddd, *J* = 12.0, 9.1, 2.6 Hz, 1H), 7.15 (s, 1H), 7.13 - 6.97 (m, 3H), 6.92 - 6.82 (m, 2H), 5.99 (s, 1H), 5.43 (d, *J* = 14.5 Hz, 1H), 5.01 (d, *J* = 14.6 Hz, 1H), 4.54 - 4.34 (m, 4H), 4.03 (s, 2H), 1.98 (dd, *J* = 9.4, 1.7 Hz, 3H), 1.76 (dd, *J* = 9.4, 1.6 Hz, 3H). ¹⁹F NMR (376 MHz, DMSO-d6) δ -102.95 - -103.17 (m, 1F), -109.21 - - 109.31 (m, 2F), -109.62 - -109.65 (m, 1F).

### Example 151: Preparation of compound 151

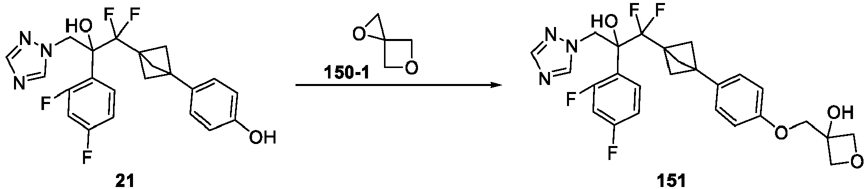

### Preparation of compound 151

Compound **21** (50 mg, 0.12 mmol) was dissolved in DMF (1 mL), then compound **150-1** (15 mg, 0.18 mmol) and Cs₂CO₃ (113 mg, 0.35 mmol) were added thereto, and the reaction system was reacted at 60°C for 16 hours. The reaction mixture was filtered, and the crude filtrate was purified by preparative separation (chromatographic column: Welch Xtimate@ C18 21.2 × 250 mm; column temperature: 25°C; mobile phase: water (10 mM/L NH₄HCO₃)-acetonitrile; proportion of acetonitrile in the mobile phase: 35% to 55% in 12 min; flow rate: 30 mL/min) to obtain the title compound **151** (16 mg, yield: 32%). LC-MS (ESI): m/z 520.0 [M+H]⁺. ¹H NMR (400 MHz, Chloroform-*d*) δ 8.19 (s, 1H), 7.84 (s, 1H), 7.81 - 7.68 (m, 1H), 7.10 - 7.02 (m, 2H), 6.89 - 6.81 (m, 3H), 6.81 - 6.73 (m, 1H), 5.30 (br.s, 1H), 5.21 (d, *J* = 14.1 Hz, 1H), 4.85 (d, *J* = 13.7 Hz, 1H), 4.72 (d, *J* = 7.0 Hz, 2H), 4.59 - 4.53 (m, 2H), 4.21 (s, 2H), 2.06 (dd, *J* = 9.5, 1.9 Hz, 3H), 2.00 (br.s, 1H), 1.91 (dd, *J* = 9.4, 1.8 Hz, 3H). ¹⁹F NMR (376 MHz, Chloroform-*d*) δ -108.36 (d, *J* = 9.6 Hz, 1F), -110.69 (s, 1F), -110.81 (s, 1F), -111.03 (d, *J* = 14.2 Hz, 1F).

### Example 152: Preparation of compound 152

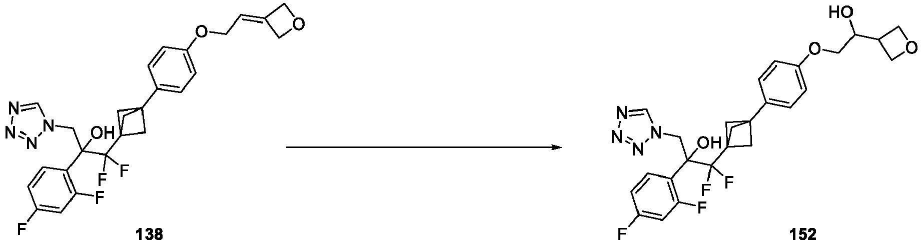

### Preparation of compound 152

Compound **138** (50 mg, 0.10 mmol) was dissolved in THF (5 mL) at 0°C, then boranetetrahydrofuran complex (1.0 M, 0.2 mL, 0.15 mmol) was added dropwise thereto, and the reaction system was warmed to room temperature and stirred for 18 hours. NaOH aqueous solution (10%, 0.25 mL) and 30% H₂O₂ (0.15 mL) solution were sequentially added dropwise to the reaction system, and then stirred at room temperature for 6 hours. The reaction system was extracted with EtOAc (50 mL × 3), and the organic phases were combined, dried, and concentrated to obtain a crude product. The crude product was purified by preparative separation (preparation method: mobile phase: A 0.1% FA aqueous solution; B: acetonitrile; chromatographic column: Agilent C18 250 × 21.2 µm; column temperature: 25°C; gradient: 5% to 55%; acetonitrile; flow rate: 25 mL/min) to obtain the title compound **152** (4.2 mg, yield: 8.1%). LC-MS (ESI): m/z 535.2 [M+H]⁺. ¹H NMR (400 MHz, DMSO-*d6*) δ 9.14 (s, 1H), 7.54 (dd, *J* = 15.5, 8.6 Hz, 1H), 7.25 (dd, *J* = 26.7, 15.1 Hz, 2H), 7.03 (d, *J* = 8.4 Hz, 3H), 6.83 (dd, *J* = 8.3, 4.9 Hz, 2H), 5.42 (d, *J* = 14.6 Hz, 1H), 5.06 (d, *J* = 22.6 Hz, 2H), 4.11 (s, 1H), 3.89 (s, 4H), 3.67 (s, 1H), 3.55 (d, *J* = 9.1 Hz, 2H), 1.97 (d, *J* = 9.4 Hz, 3H), 1.76 (d, *J* = 9.1 Hz, 3H).

### Example 153: Preparation of compound 153

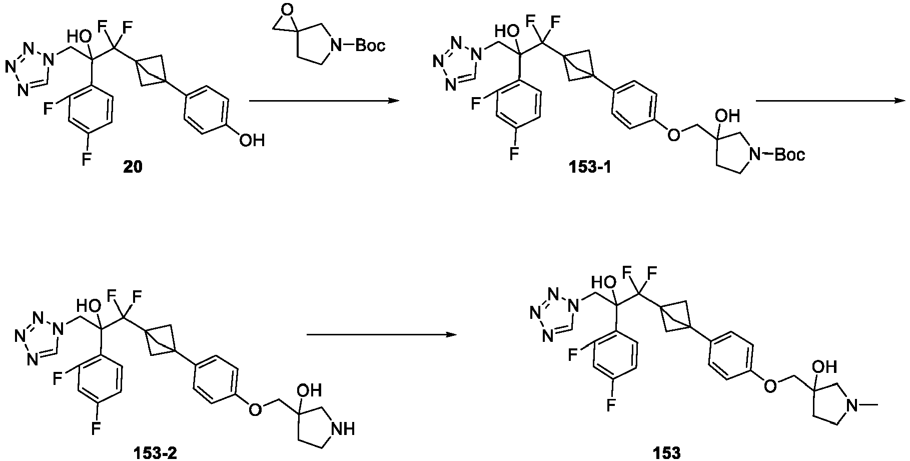

### Preparation of compound 153-1

Compound tert-butyl 1-oxa-5-azaspiro[2.4]heptane-5-carboxylate (20 mg, 0.1 mmol) was dissolved in DMF (1.5 mL), then compound 20 (30 mg, 0.069 mmol) and cesium carbonate (72 mg, 0.22 mmol) were added thereto, and the reaction system was reacted at 60°C for 2 hours in a sealed tube. The reaction system was cooled, then extracted with EtOAc (50 mL × 3), and the organic phases were combined, dried, and concentrated to obtain the crude title compound **153-1** (40 mg, yellow oil), which was directly used in the next reaction step. LC-MS (ESI): m/z 634.3 [M+H]⁺.

### Preparation of compound 153-2

Compound **153-1** (40 mg, crude) was dissolved in DCM (5 mL), and HCl/MeOH (4 M, 5 mL) was added dropwise thereto at 0°C. The reaction system was transferred to room temperature and reacted for 1 hour, and the reaction mixture was concentrated to obtain the title compound **153-2** (28 mg, yellow oil), which was directly used in the next reaction step.

### Preparation of compound 153

Compound **153-2** (28 mg, crude) was dissolved in MeOH (3 mL), then formaldehyde aqueous solution (40%, 0.25 mL, 4.2 mmol) was added thereto. The reaction system was stirred at room temperature for 1 hour, then added with sodium cyanoborohydride (18.9 mg, 0.30 mmol), and reacted for another 1 hour. The reaction mixture was filtered, and the crude filtrate was purified by preparative separation (preparation method: mobile phase: A: 0.1% FA aqueous solution; B: acetonitrile; chromatographic column: Agilent C18 250 × 21.2 µm; column temperature: 25°C; gradient: 15 to 35% acetonitrile; flow rate: 25 mL/min) to obtain the title compound **153** (6.4 mg, three-step yield: 23.8%). LC-MS (ESI): m/z 548.2 [M+H]⁺. ¹H NMR (400 MHz, DMSO-*d6*) δ 9.13 (s, 1H), 8.17 (d, *J* = 1.8 Hz, 1H), 7.54 (td, *J* = 9.0, 6.7 Hz, 1H), 7.37 - 7.18 (m, 1H), 7.14 (s, 1H), 7.09 - 6.96 (m, 3H), 6.89 - 6.80 (m, 2H), 5.42 (d, *J* = 14.5 Hz, 1H), 5.01 (d, *J* = 14.6 Hz, 1H), 3.84 (d, *J* = 22.0 Hz, 3H), 2.84 - 2.64 (m, 4H), 2.43 (d, *J* = 5.7 Hz, 1H), 2.33 (d, *J* = 3.4 Hz, 3H), 1.96 (td, *J* = 11.1, 10.2, 4.2 Hz, 3H), 1.76 (dd, *J* = 9.5, 1.7 Hz, 3H). ¹⁹F NMR (376 MHz, DMSO-d6) δ -102.95 - -103.14 (m, 1F), - 109.14 - -109.24 (m, 2F), -109.64 - -109.67 (m, 1F).

### Example 154: Preparation of compound 154

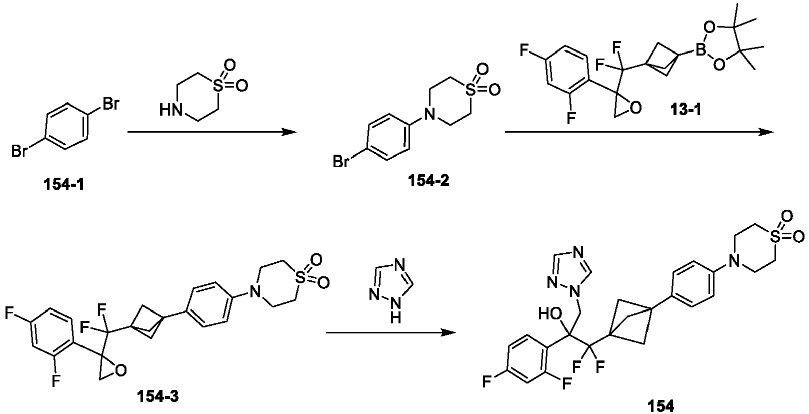

### Preparation of compound 154-2

Compound **154-1** (1 g, 4.24 mmol) was dissolved in dioxane (10 mL), then thiomorpholine-1,1-dioxide (516 mg, 3.82 mmol), palladium acetate (95 mg, 0.424 mmol), BINAP (264 mg, 0.424 mmol), and Cs₂CO₃ (4.14 g, 12.72 mmol) were sequentially added thereto. The reaction system was replaced with nitrogen three times and reacted at 100°C for 16 hours. The reaction mixture was filtered, and the reaction system was extracted with EtOAc (100 mL × 3), and the organic phases were combined, dried, and concentrated to obtain a crude product. The crude product was purified by normal-phase silica gel column chromatography (EtOAc/PE = 0 to 30%) to obtain the title compound **154-2** (370 mg, yellow solid, yield: 37%). LC-MS (ESI): m/z 579.0 [2M+H]⁺.

### Preparation of compound 154-3

Compound **154-2** (500 mg, 1.73 mmol) was dissolved in a mixed solvent of cyclopentyl methyl ether (8 mL) and water (2 mL), then compound 13-1 (1 g, 2.6 mmol), palladium acetate (39 mg, 0.173 mmol), CatacxiumA (124 mg, 0.346 mmol), cuprous oxide (250 mg, 1.73 mmol), and cesium carbonate (1.69 g, 5.19 mmol) were sequentially added thereto. The reaction system was purged with nitrogen for 3 min, and reacted in an oil bath at 120°C in a microwave tube for 16 hours. The reaction system was extracted with EtOAc (50 mL × 3), and the organic phases were combined, dried, and concentrated to obtain a crude product, which was purified by preparative separation (preparative column: Pursuit XRs 10 C18 250 * 21.2 mm; flow rate: 20 mL/min; mobile phase: A: 0.1% FA aqueous solution, B: acetonitrile; gradient: 84 to 84% acetonitrile content, retention time of 8.0 to 8.5 min) to obtain the title compound **154-3** (35 mg, oily liquid, yield: 4.2 %). LC-MS (ESI): m/z 482.3 [M+H]⁺.

### Preparation of compound 154

Compound **154-3** (35 mg, 0.073 mmol) was dissolved in DMF (2 mL), then 1,2,4-triazole (21 mg, 0.291 mmol) and potassium carbonate (41 mg, 0.291 mmol) were added thereto, and the reaction system was stirred and reacted at 80°C for 16 hours. The reaction system was cooled, and then extracted with EtOAc (50 mL × 3), and the organic phases were combined, dried, and concentrated to obtain a crude product, which was purified by preparative separation (preparative column: Pursuit XRs 10 C18 250 * 21.2 mm; flow rate: 20 mL/min; mobile phase: A: 0.1% FA aqueous solution, B: acetonitrile; gradient: 60% to 65% acetonitrile content, retention time of 8.8 to 9.4 min) to obtain the title compound **154** (13.49 mg, yield: 33.7%). LC-MS (ESI): m/z 551.2 [M+H]⁺. ¹H NMR (400 MHz, DMSO-*d6*) δ 8.37 (s, 1H), 7.68 (s, 1H), 7.57 (dd, *J* = 15.9, 9.0 Hz, 1H), 7.18 (ddd, *J* = 11.9, 9.2, 2.5 Hz, 1H), 7.01 - 6.88 (m, 6H), 5.13 (d, *J* = 14.3 Hz, 1H), 4.77 (d, *J* = 14.5 Hz, 1H), 3.70 (d, *J* = 4.8 Hz, 4H), 3.07 (d, *J* = 5.1 Hz, 4H), 1.95 (d, *J* = 9.0 Hz, 3H), 1.74 (d, *J* = 9.3 Hz, 3H).

### Example 155: Preparation of compound 155

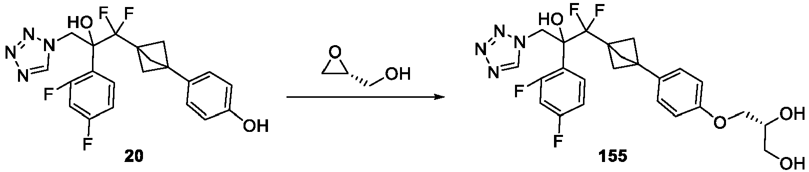

Compound **20** (50 mg, 0.12 mmol) was dissolved in DMF (1 mL), then (R)-(+)-glycidol (15 mg, 0.18 mmol) and Cs₂CO₃ (113 mg, 0.35 mmol) were added thereto, and the reaction system was reacted at 60°C for 16 hours. The reaction mixture was filtered, and the crude filtrate was purified by preparative separation (chromatographic column: Welch Xtimate@ C18 21.2 × 250 mm; column temperature: 25°C; mobile phase: water (10 mM/L NH₄HCO₃)-acetonitrile; proportion of acetonitrile in the mobile phase: 30% to 50% in 12 min; flow rate: 30 mL/min) to obtain the title compound **155** (16 mg, yield: 32%). LC-MS (ESI): m/z 509.0 [M+H]⁺. ¹H NMR (400 MHz, DMSO-*d6*) δ 9.13 (s, 1H), 7.62-7.48 (m, 1H), 7.31 - 7.22 (m, 1H), 7.13 (s, 1H), 7.02 (dd, *J* = 8.9, 2.7 Hz, 3H), 6.86 - 6.77 (m, 2H), 5.42 (d, *J* = 14.5 Hz, 1H), 5.01 (d, *J* = 14.6 Hz, 1H), 4.89 (d, *J* = 4.9 Hz, 1H), 4.63 (t, *J* = 5.7 Hz, 1H), 3.93 (dd, *J* = 9.5, 3.9 Hz, 1H), 3.86 - 3.69 (m, 2H), 3.41 (d, *J* = 5.5 Hz, 2H), 1.97 (dd, *J* = 9.5, 1.7 Hz, 3H), 1.76 (dd, *J* = 9.3, 1.7 Hz, 3H). ¹⁹F NMR (376 MHz, DMSO-d6) δ -102.61 - -103.47 (m, 1F), -108.87 - -109.43 (m, 2F), -109.65 (d, 1F).

### Example 156: Preparation of compound 156

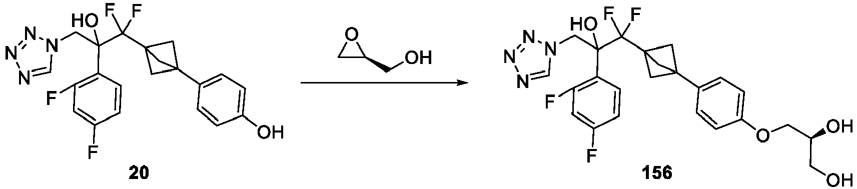

### Preparation of compound 156

Compound **20** (50 mg, 0.12 mmol) was dissolved in DMF (1 mL), then (S)-glycidol (15 mg, 0.18 mmol) and Cs₂CO₃ (113 mg, 0.35 mmol) were added thereto, and the reaction system was reacted at 60°C for 16 hours. After the reaction system was cooled, the reaction mixture was filtered, and the crude filtrate was purified by preparative separation (chromatographic column: Welch Xtimate@ C18 21.2 × 250 mm; column temperature: 25°C; mobile phase: water (10 mM/L NH₄HCO₃)-acetonitrile; proportion of acetonitrile in the mobile phase: 30% to 50% in 12 min; flow rate: 30 mL/min) to obtain the title compound **156** (16 mg, yield: 32%). LC-MS (ESI): m/z 509.0 [M+H]⁺. ¹H NMR (400 MHz, DMSO-*d6*) δ 9.13 (s, 1H), 7.62 - 7.48 (m, 1H), 7.31 - 7.22 (m, 1H), 7.13 (s, 1H), 7.02 (dd, *J* = 8.9, 2.7 Hz, 3H), 6.86 - 6.77 (m, 2H), 5.42 (d, *J* = 14.5 Hz, 1H), 5.01 (d, *J* = 14.6 Hz, 1H), 4.89 (d, *J* = 4.9 Hz, 1H), 4.63 (t, *J* = 5.7 Hz, 1H), 3.93 (dd, *J* = 9.5, 3.9 Hz, 1H), 3.86 - 3.69 (m, 2H), 3.41 (d, *J* = 5.5 Hz, 2H), 1.97 (dd, *J* = 9.5, 1.7 Hz, 3H), 1.76 (dd, *J* = 9.3, 1.7 Hz, 3H). ¹⁹F NMR (376 MHz, DMSO-d6) δ -102.61 - -103.47 (m, 1F), -108.87 - -109.43 (m, 2F), -109.65 (d, 1F).

### Example 157: Preparation of compound 157

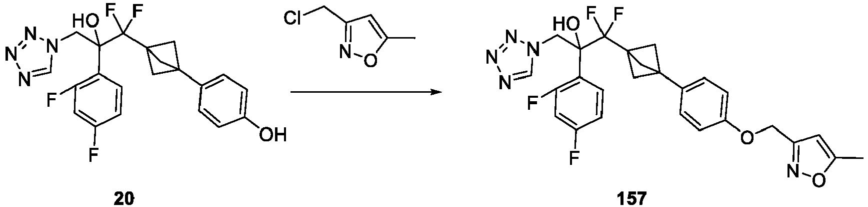

### Preparation of compound 157

Compound **20** (150 mg, 0.34 mmol) was dissolved in DMF (50 mL), then potassium carbonate (94 mg, 0.68 mmol) was added thereto. After stirring for 30 min, 3-chloromethyl-5-methylisoxazole (66 mg, 0.5 mmol) was added thereto, and the reaction system was reacted at room temperature for 16 hours. The reaction mixture was filtered, and the crude filtrate was purified by preparative separation (preparation method: mobile phase: A: 0.1% formic acid aqueous solution; B: acetonitrile; chromatographic column: Agilent 10 Prep-C18 250 × 21.2 mm; column temperature: 25°C; gradient: 50% to 70% acetonitrile in 12 min; flow rate: 30 mL/min) to obtain the title compound **157** (31.7 mg, yield: 17.6%). LC-MS (ESI): m/z 530.2 [M+H]⁺. ¹H NMR (400 MHz, DMSO-*d6*) δ 9.13 (s, 1H), 7.53 (td, *J* = 9.0, 6.7 Hz, 1H), 7.28 (ddd, *J* = 12.0, 9.1, 2.6 Hz, 1H), 7.15 (s, 1H), 7.09 - 6.96 (m, 3H), 6.96 - 6.81 (m, 2H), 6.28 (d, *J* = 1.0 Hz, 1H), 5.42 (d, *J* = 14.5 Hz, 1H), 5.09 (s, 2H), 5.01 (d, *J* = 14.5 Hz, 1H), 2.38 (s, 3H), 1.97 (dd, *J* = 9.5, 1.7 Hz, 3H), 1.76 (dd, *J* = 9.4, 1.7 Hz, 3H). ¹⁹F NMR (376 MHz, DMSO-d6) δ -102.97 - -103.19 (m, 1F), -109.22 - -109.32 (m, 2F), -109.62 - -109.66 (m, 1F).

### Example 158: Preparation of compound 158

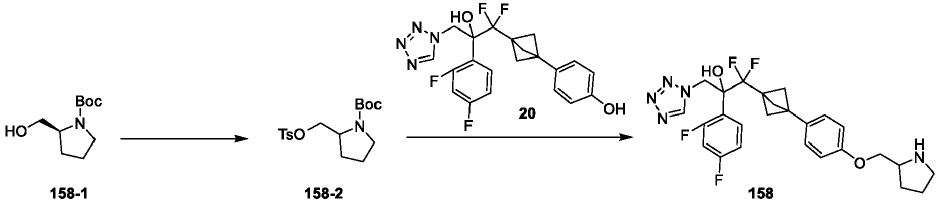

### Preparation of compound 158-2

Compound **158-1** (500 mg, 2.48 mmol) was dissolved in dichloromethane (10 mL), then triethylamine (1 mL) was added dropwise thereto at 0°C. The reaction system was stirred for 10 min, then added with p-toluenesulfonyl chloride (568 g, 2.98 mmol), and then transferred to room temperature and reacted for 4 hours. The reaction system was extracted with DCM (50 mL × 3), and the organic phases were combined, dried, and concentrated to obtain the crude title compound **158-2** (500 mg), which was directly used in the next step.

### Preparation of compound 158

Compound **20** (200 mg, 0.46 mmol) was dissolved in acetonitrile (2 mL), then compound **158-2** (500 mg, 1.41 mmol) and cesium carbonate (300 mg, 0.92mmol) were added thereto, and the reaction system was stirred at 80°C for 16 hours in a sealed tube. After the reaction system was cooled, the reaction mixture was filtered, and the filtrate was evaporated to dryness by rotary evaporation, then added with a solution of hydrochloric acid in methanol (4M, 2 mL). The reaction system was stirred at room temperature for 16 hours, and the reaction mixture was concentrated and evaporated to dryness by rotary evaporation to obtain a crude product. The crude product was purified by preparative separation (chromatographic column: Welch Xtimate@ C18 21.2 × 250 mm; column temperature: 25°C; mobile phase: water (10 mM/L NH₄HCO₃)-acetonitrile; proportion of acetonitrile in the mobile phase: 40% to 60% in 12 min; flow rate: 30 mL/min) to obtain the title compound **158** (30 mg, yield: 10%). LC-MS (ESI): m/z 518.2 [M+H]⁺. ¹H NMR (400 MHz, DMSO-*d6*) δ 9.15 (s, 1H), 7.60 - 7.49 (m, 1H), 7.32 - 7.27 (m, 1H), 7.19 (s, 1H), 7.09 - 7.06 (m, 2H), 7.02 - 7.00 (m, 1H), 6.91 - 6.85 (m, 2H), 5.42 (d, *J* = 14.5 Hz, 1H), 5.02 (d, *J* = 14.5 Hz, 1H), 4.19 (dd, *J* = 10.6, 3.6 Hz, 1H), 4.03 (dd, *J* = 10.6, 8.4 Hz, 1H), 3.91- 3.82 (m, 1H), 3.07 - 3.02 (m, 2H), 2.72 - 2.64 (m, 1H), 2.17 - 2.05 (m, 2H), 2.03 - 1.94 (m, 3H), 1.94 - 1.83 (m, 2H), 1.79 - 1.73 (m, 3H).

### Example 159: Preparation of compounds 159, 159A, and 159B

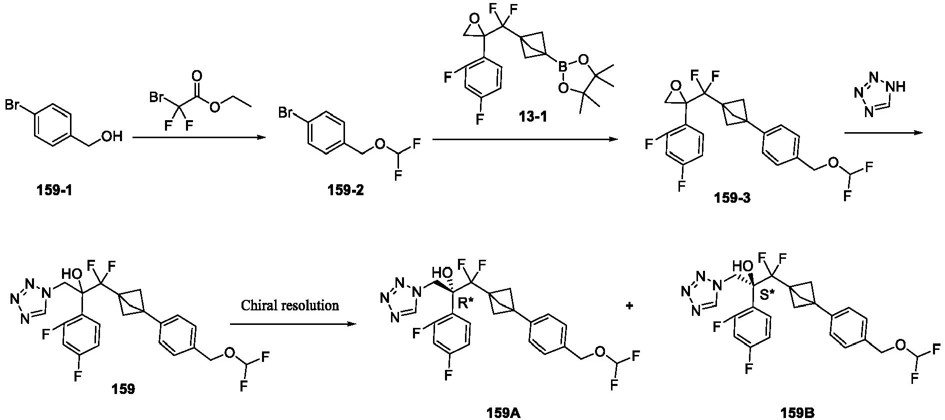

### Preparation of compound 159-2

Compound **159-1** (3 g, 16 mmol) was dissolved in THF (50 mL), and NaH (60%, 615 mg, 48 mmol) was added thereto at 0°C. After the reaction system was stirred for 30 min, ethyl bromodifluoroacetate (4.8 g, 24 mmol) was added thereto, and the reaction system was warmed to room temperature and stirred for 16 hours. The reaction mixture was poured into water (20 mL) and extracted with EtOAc (50 mL × 3), and the organic phases were combined, dried, and concentrated to obtain a crude product. The crude product was purified by normal-phase silica gel column chromatography (EtOAc/PE = 5 to 20%) to obtain the title compound **159-2** (360 mg, yellow oil, yield: 9.5%). ¹H NMR (400 MHz, Chloroform-*d*) δ 7.57 - 7.45 (m, 2H), 7.31 - 7.19 (m, 2H), 6.30 (t, *J* = 74.0 Hz, 1H), 4.84 (s, 2H).

### Preparation of compound 159-3

Compound **13-1** (727 mg, 1.82 mmol) was dissolved in a mixed solvent of cyclopentyl methyl ether (10 mL) and water (3 mL), and then compound **159-2** (360 mg, 1.52 mmol), cuprous oxide (260 mg, 1.82 mmol), palladium acetate (40.3 mg, 0.18 mmol), butyldi-1-adamantylphosphine (130 mg, 0.364 mmol), and cesium carbonate (1.5 g, 4.56 mmol) were sequentially added thereto. The reaction system was replaced with nitrogen three times, and then reacted under microwave irradiation at 120°C for 16 hours. The reaction mixture was poured into water (20 mL) and extracted with EtOAc (50 mL × 3), and the organic phases were combined, dried, and concentrated to obtain a crude product. The crude product was purified by normal-phase silica gel column chromatography (EtOAc/PE = 5 to 20%) to obtain the title compound **159-3** (150 mg, yellow oil).

### Preparation of compound 159

Compound **159-3** (80 mg, 0.19 mmol) was dissolved in DMF (10 mL), then potassium carbonate (79 mg, 0.57 mmol) and tetrazole (53.2 mg, 0.76 mmol) were added thereto, and the reaction system was stirred at 70°C for 16 hours in a sealed tube. After the reaction system was cooled, the reaction mixture was filtered, and the crude filtrate was purified by preparative separation (preparation method: mobile phase: A: 0.1% formic acid aqueous solution; B: acetonitrile; chromatographic column: Agilent 10 Prep-C18 250 × 21.2 mm; column temperature: 25°C; gradient: 50% to 70% acetonitrile in 12 min; flow rate: 30 mL/min) to obtain the title compound **159** (6.28 mg). LC-MS (ESI): m/z 499.2 [M+H]⁺. ¹H NMR (400 MHz, DMSO-*d6*) δ 9.14 (s, 1H), 7.54 (td, *J* = 9.0, 6.7 Hz, 1H), 7.35 - 7.24 (m, 3H), 7.20 - 7.12 (m, 3H), 7.05 - 6.97 (m, 1H), 6.75 (t, *J* = 75.7 Hz, 1H), 5.43 (d, *J* = 14.5 Hz, 1H), 5.02 (d, *J* = 14.6 Hz, 1H), 4.84 (s, 2H), 2.02 (dd, *J* = 9.4, 1.7 Hz, 3H), 1.81 (dd, *J* = 9.4, 1.7 Hz, 3H). ¹⁹F NMR (376 MHz, DMSO-d6) δ -82.12 (m, 2F), -102.94 - -103.15 (m, 1F), -109.28 - - 109.41 (m, 2F), -109.59 - -101.61 (m, 1F).

### Preparation of compounds 159A and 159B

Compound **159** (98 mg) was subjected to SFC chiral preparative resolution (preparative separation method, instrument model: MG II preparative SFC (SFC-14); chromatographic column: ChiralCel OD, 250 × 30 mm I.D., 10 µm; mobile phase: A: CO₂ B: isopropanol (0.1% NH₃H₂O); elution gradient: B 40%; flow rate: 100 mL/min; column pressure: 100 bar; chromatographic column temperature: 35°C; detection wavelength: 220 nM; cycle: about 9.5 min) to obtain the title compounds **159A** (43 mg) and **159B** (44 mg).

Compound **159A:** LC-MS (ESI): m/z 499.2 [M+H]⁺. Chiral resolution (chromatographic column: Chiralcel OX-3 100 × 4.6 mm I.D., 3 µm; mobile phase: A: CO₂ B: ethanol (0.05% DEA); gradient: the mobile phase increased from 5% B to 40% B within 5 min and maintained at 40% B for 2.5 min for elution, then 5% B was equilibrated for 2.5 min; flow rate: 2.8 mL/min; chromatographic column temperature: 35°C; column pressure: 1500 psi; detection wavelength: 220 nM; RT = 2.882 min.) ¹H NMR (400 MHz, DMSO-*d6*) δ 9.14 (s, 1H), 7.54 (td, *J* = 9.0, 6.7 Hz, 1H), 7.38 - 7.24 (m, 3H), 7.24 - 7.10 (m, 3H), 7.01 (td, *J* = 8.5, 2.7 Hz, 1H), 6.75 (t, *J* = 75.7 Hz, 1H), 5.43 (d, *J* = 14.5 Hz, 1H), 5.01 (d, *J* = 14.6 Hz, 1H), 4.85 (s, 2H), 2.02 (dd, *J* = 9.5, 1.7 Hz, 3H), 1.81 (dd, *J* = 9.4, 1.7 Hz, 3H). ¹⁹F NMR (376 MHz, DMSO-d6) δ -82.58 (m, 2F), -102.94 - -103.16 (m, 1F), -109.28 - -109.38 (m, 2F), - 109.59 - -109.61 (m, 1F).

Compound **159B:** LC-MS (ESI): m/z 499.2 [M+H]⁺. Chiral resolution (chromatographic column: Chiralcel OX-3 100 × 4.6 mm I.D., 3 µm; mobile phase: A: CO₂ B: ethanol (0.05% DEA); gradient: the mobile phase increased from 5% B to 40% B within 5 min and maintained at 40% B for 2.5 min for elution, then 5% B was equilibrated for 2.5 min; flow rate: 2.8 mL/min; chromatographic column temperature: 35°C; column pressure: 1500 psi; detection wavelength: 220 nM; RT = 2.379 min). ¹H NMR (400 MHz, DMSO-d6) δ 9.14 (s, 1H), 7.54 (td, *J* = 9.0, 6.7 Hz, 1H), 7.38 - 7.23 (m, 3H), 7.21 - 7.10 (m, 3H), 7.01 (td, *J* = 8.5, 2.7 Hz, 1H), 6.75 (t, *J* = 75.7 Hz, 1H), 5.43 (d, *J* = 14.5 Hz, 1H), 5.01 (d, *J* = 14.6 Hz, 1H), 4.84 (s, 2H), 2.02 (dd, *J* = 9.4, 1.7 Hz, 3H), 1.81 (dd, *J* = 9.4, 1.7 Hz, 3H). ¹⁹F NMR (376 MHz, DMSO-*d6*) δ -82.58 (m, 2F), -102.94 -103.16 (m, 1F), -109.28 -109.38 (m, 2F), -109.59 - -109.61 (m, 1F).

### Example 160: Preparation of compound 160

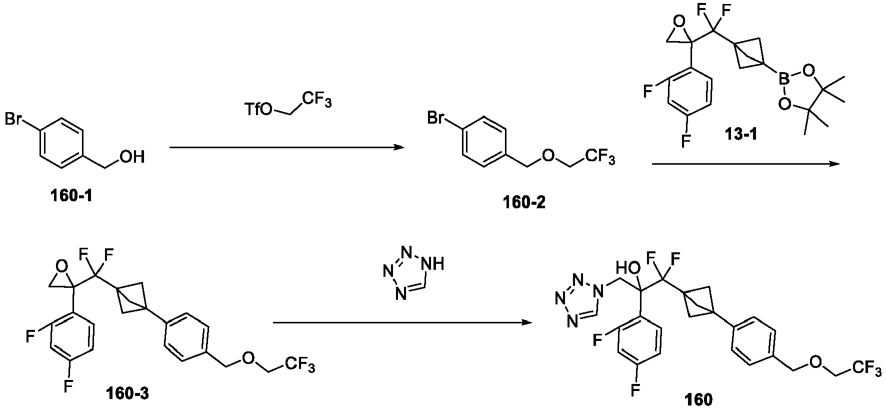

### Preparation of compound 160-2

Compound **160-1** (1.0 g, 5.34 mmol) was dissolved in DMF (20 mL), and NaH (60%, 307 mg, 8.2 mmol) was added thereto at 0°C. After stirring for 30 min, the reaction system was added with 2,2,2-trifluoroethyl trifluoromethanesulfonate (1.45 g, 6.4 mmol), transferred to room temperature and reacted for 16 hours. The reaction system was extracted with EtOAc (50 mL × 3), and the organic phases were combined, dried, and concentrated to obtain a crude product. The crude product was purified by normal-phase silica gel column chromatography (EtOAc/PE = 5 to 20%) to obtain the title compound **160-2** (650 mg, yellow oil, yield: 45.2%). ¹H NMR (400 MHz, Chloroform-d) δ 7.49 (d, *J* = 3.8 Hz, 2H), 7.24 (d, *J* = 1.6 Hz, 2H), 4.63 (s, 2H), 3.83 (q, *J* = 8.7 Hz, 2H).

### Preparation of compound 160-3

Compound **13-1** (800 mg, 2 mmol) was dissolved in a mixed solvent of cyclopentyl methyl ether (5 mL) and water (2 mL), and then compound **160-2** (540 mg, 2 mmol), cuprous oxide (286 mg, 2 mmol), palladium acetate (45 mg, 0.2 mmol), butyldi-1-adamantylphosphine (142 mg, 0.4 mmol), and cesium carbonate (2.0 g, 6 mmol) were sequentially added thereto. The reaction system was replaced with nitrogen three times, and then reacted under microwave irradiation at 120°C for 16 hours. The reaction system was cooled, then extracted with EtOAc (50 mL × 3), and the organic phases were combined, dried, and concentrated to obtain a crude product. The crude product was purified by normal-phase silica gel column chromatography (EtOAc/PE = 5 to 20%) to obtain the title compound **160-3** (80 mg, yellow oil), which was directly used in the next reaction step.

### Preparation of compound 160

Compound **160-3** (80 mg, crude) was dissolved in DMF (10 mL), then potassium carbonate (79 mg, 0.57 mmol) and tetrazole (53.2 mg, 0.76 mmol) were added thereto, and the reaction system was stirred at 70°C for 16 hours in a sealed tube. The reaction mixture was filtered, and the crude filtrate was purified by preparative separation (preparation method: mobile phase: A: 0.1% formic acid aqueous solution; B: acetonitrile; chromatographic column: Agilent 10 Prep-C18 250 × 21.2 mm; column temperature: 25°C; gradient: 45% to 75% acetonitrile in 12 min; flow rate: 30 mL/min) to obtain the title compound **160** (9 mg, two-step yield: 0.8%). LC-MS (ESI): m/z 531.2 [M+H]⁺. ¹H NMR (400 MHz, DMSO-*d6*) δ 9.14 (s, 1H), 7.54 (td, *J* = 9.0, 6.7 Hz, 1H), 7.33 - 7.21 (m, 3H), 7.21 - 7.09 (m, 3H), 7.01 (td, *J* = 8.6, 2.7 Hz, 1H), 5.43 (d, *J* = 14.5 Hz, 1H), 5.02 (d, *J* = 14.6 Hz, 1H), 4.59 (s, 2H), 4.03 (q, *J* = 9.4 Hz, 2H), 2.02 (dd, *J* = 9.5, 1.7 Hz, 3H), 1.80 (dd, *J* = 9.5, 1.7 Hz, 3H). ¹⁹F NMR (376 MHz, DMSO-d6) δ -72.74 (s, 3F), -103.17 (m, 2F), -109.37 -- -109.63 (m, 2F).

### Example 161: Preparation of compound 161

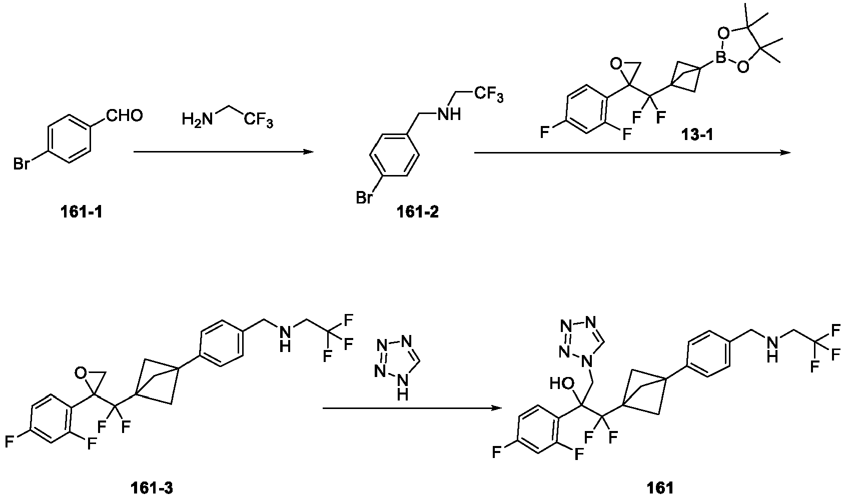

### Preparation of compound 161-2

Compound **161-1** (1.9 g, 10.1 mmol) was dissolved in methanol (10 mL), and 2,2,2-trifluoroethylamine (1.0 g, 10.1 mmol) was added thereto, and then sodium cyanoborohydride (0.9 g, 15.2 mmol) was added thereto. The reaction system was stirred at room temperature for 16 hours. The reaction system was extracted with EtOAc (50 mL × 3), and the organic phases were combined, dried, and concentrated to obtain a crude product. The crude product was purified by normal-phase silica gel column chromatography (EtOAc/PE = 0 to 100%) to obtain the title compound **161-2** (1.4 g, colorless oil, yield: 51.9 %). LC-MS (ESI): m/z 267. 9 [M+H]⁺.

### Preparation of compound 161-3

Compound **161-2** (500 mg, 1.26 mmol) was dissolved in a mixed solvent of cyclopentyl methyl ether (5 mL) and water (2 mL), then compound 13-1 (507 mg, 1.88 mmol), cataCXiumA (136 mg, 0.38 mmol), cesium carbonate (1229 mg, 3.78 mmol), cuprous oxide (180 mg, 1.26 mmol), and palladium acetate (42 mg, 0. 19 mmol) were sequentially added thereto. The reaction system was replaced with nitrogen three times and reacted at 120°C for 16 hours. The reaction system was cooled, and then extracted with EtOAc (50 mL × 3), and the organic phases were combined, dried, and concentrated to obtain a crude product, which was purified by preparative separation (preparative column: Pursuit XRs 10 C18 21.2 * 250 mm; flow rate: 20 mL/min; mobile phase: A: 0.1% FA, B: acetonitrile; gradient: 62% to 65% acetonitrile content, retention time of 8.0 to 9.0 min) to obtain compound **161-3** (240 mg, pale yellow solid, yield: 41.6%). LC-MS (ESI): m/z 460. 2 [M+H]⁺.

### Preparation of compound 161

Compound **161-3** (240 mg, 0.52 mmol) was dissolved in DMF (3 mL), then 1H-tetrazole (183 mg, 2.6 mmol) and potassium carbonate (216 mg, 1.56 mmol) were added thereto, and the reaction system was replaced with nitrogen three times and reacted at 80°C for 16 hours. After the reaction system was cooled, the reaction mixture was filtered, and the filtrate was purified by preparative separation (preparation column: Xbridge C18 21.2 * 250 mm * 10 µm; flow rate: 20 mL/min; mobile phase: A: 0.05% ammonia water, B: acetonitrile; gradient: 5 to 63% acetonitrile content, retention time: 7.5 to 8.5 min) to obtain the title compound **161** (70.3 mg). LC-MS (ESI): m/z 530.2 [M+H]⁺. ¹H NMR (400 MHz, DMSO-*d6)* δ 9.16 (s, 1H), 7.55 (d, *J* = 6.8 Hz, 1H), 7.32 - 7.16 (m, 4H), 7.11 - 6.97 (m, 3H), 5.43 (d, *J* = 14.5 Hz, 1H), 5.03 (d, *J* = 14.6 Hz, 1H), 3.71 (d, *J* = 6.5 Hz, 2H), 3.11 (qd, *J* = 10.2, 7.2 Hz, 2H), 2.85 (dq, *J* = 13.7, 6.8 Hz, 1H), 2.04 - 1.95 (m, 3H), 1.84 - 1.73 (m, 3H).

### Example 162: Preparation of compound 162

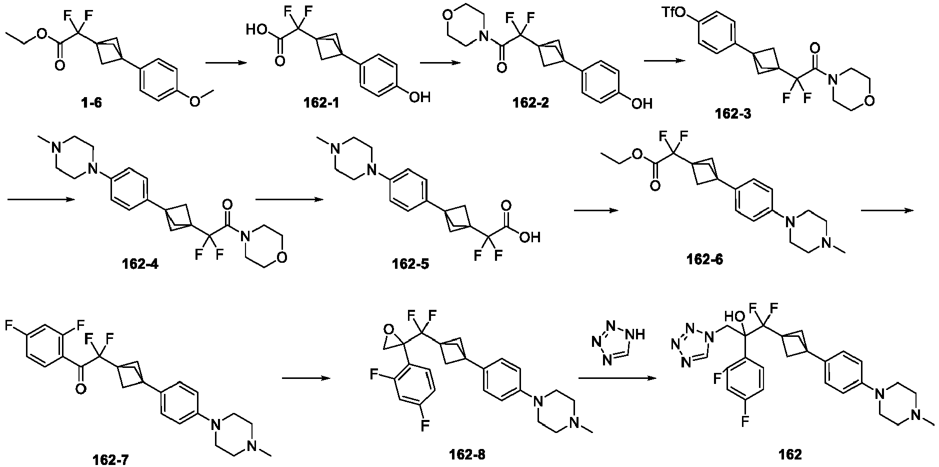

### Preparation of compound 162-1

Compound **1-6** (3.8 g, 12.7 mmol) was dissolved in hydrobromic acid aqueous solution (20 mL), and the reaction system was reacted at 100°C for 16 hours in a sealed tube. After the reaction system was cooled, a solid was precipitated, which was filtered and dried to obtain the crude title compound **162-1** (3 g, yield: 93%). LC-MS (ESI): m/z 255.2 [M+H]⁺.

### Preparation of compound 162-2

Compound **162-1** (3 g, 11.80 mmol) was dissolved in acetonitrile (50 mL), then TFCH (3.97 g, 14.16 mmol) was added thereto. The reaction system was stirred for 5 min, then added with NMI (1.45 g, 17.70 mmol) and morphine (1.23 g, 14.16 mmol), and reacted at 0°C for 2 hours. The reaction mixture was filtered, and the filtrate was concentrated to obtain a crude product, and the crude product was purified by normal-phase silica gel column chromatography (EtOAc/PE = 0 to 100%) to obtain the title compound **162-2** (1.3 g, yield: 37%). LC-MS (ESI): m/z 324.2 [M+H]⁺.

### Preparation of compound 162-3

Compound **162-2** (1.3 g, 4.02 mmol) was dissolved in dichloromethane (50 mL), then pyridine (636 mg, 8.04 mmol) was added thereto. After the reaction was carried out for 5 min, trifluoromethanesulfonic anhydride (2.27 g, 8.04 mmol) was added thereto, and the reaction system was stirred and reacted at 0°C for 2 hours. The reaction mixture was filtered, and the filtrate was concentrated to obtain a crude product, and the crude product was purified by normal-phase silica gel column chromatography (DCM/MeOH = 0 to 5%) to obtain the title compound **162-3** (600 mg, yield: 33%). LC-MS (ESI): m/z 456.2 [M+H]⁺. ¹H NMR (400 MHz, Chloroform-*d*) δ 7.32 - 7.27 (m, 2H), 7.23 - 7.20 (m, 2H), 3.76 - 3.65 (m, 8H), 2.30 (s, 6H). ¹⁹F NMR (376 MHz, Chloroform-*d*) δ -72.81 (s, 2F), -103.36 (s, 3F).

### Preparation of compound 162-4

Compound **162-3** (600 mg, 1.32 mmol) was dissolved in toluene (50 mL), then palladium acetate (30 mg, 0.132 mmol), cesium carbonate (1.11 g, 3.29 mmol), BINAP (164 mg, 0.263 mmol), and N-methylpiperidine (264 mg, 2.64 mmol) were sequentially added thereto, and the reaction system was stirred and reacted at 110°C for 24 hours. The reaction system was cooled, then extracted with EtOAc (50 mL × 3), and the organic phases were combined, dried, and concentrated to obtain a crude product. The crude product was purified by normal-phase silica gel column chromatography (DCM/MeOH = 0 to 10%) to obtain the title compound **162-4** (270 mg, yield: 37%). LC-MS (ESI): m/z 406.2 [M+H]⁺. ¹H NMR (400 MHz, DMSO-d6) δ 7.10 (d, *J* = 8.4 Hz, 2H), 6.89 (d, *J* = 8.4 Hz, 2H), 3.68 - 3.58 (m, 8H), 3.14 - 3.12 (m, 4H), 2.57 - 2.51 (m, 4H), 2.30 (s, 3H), 2.12 (s, 6H). ¹⁹F NMR (376 MHz, Chloroform-*d*) δ -101.78 (s, 2F).

### Preparation of compound 162-6

Compound **162-4** (270 mg, 0.606 mmol) was dissolved in 37% concentrated hydrochloric acid (15 mL), and the reaction system was reacted at 110°C for 2 hours. After the reaction system was cooled, LCMS showed that the reaction was completed. The reaction mixture was evaporated to dryness by rotary evaporation, added with anhydrous ethanol (2 mL) and concentrated sulfuric acid (1 drop), and stirred and reacted at 80°C for 2 hours. The reaction system was cooled, then neutralized with sodium bicarbonate, extracted with EtOAc (50 mL × 3), and the organic phases were combined, dried, and concentrated to obtain a crude product. The crude product was purified by normal-phase silica gel column chromatography (EtOAc/PE = 0 to 100%) to obtain the title compound **162-6** (120 mg, yield: 37%). LC-MS (ESI): m/z 365.1 [M+H]⁺.

### Preparation of compound 162-7

To a three-necked flask was added 1-bromo-2,4-difluorobenzene (0.083 g, 0.428 mmol), and the system was replaced with nitrogen three times. Anhydrous ether (5 mL) was added to the three-necked flask, and n-butyllithium (2.5 M, 0.27 mL, 0.428 mmol) was added dropwise thereto at -78°C. After the reaction system was stirred and reacted at -78°C for 45 min, a solution of compound **162-6** (0.120 g, 0.329 mmol) dissolved in anhydrous ether (1 mL) was added dropwise thereto, and the reaction mixture was stirred and reacted for another 1 hour. The reaction system was quenched with saturated ammonium chloride solution (5 mL), extracted with EtOAc (20 mL × 3), and the organic phases were combined, dried, and concentrated to obtain a crude product. The crude product was purified by normal-phase silica gel column chromatography (EtOAc/PE = 0 to 60%) to obtain the title compound **162-7** (125 mg, colorless oil), which was directly used in the next reaction step. LC-MS (ESI): m/z 433.1 [M+H]⁺.

### Preparation of compound 162-8

Compound **162-7** (125 mg, crude product) was dissolved in a mixture of dichloromethane (5 mL) and water (2 mL), then trimethylsulfoxonium iodide (254 mg, 1.06 mmol) and sodium hydroxide (46 mg, 1.16 mmol) were added thereto, and the reaction system was reacted at 65°C for 48 hours. After the reaction was completed, the reaction system was extracted with DCM (20 mL × 3), and the organic phases were combined, dried, and concentrated to obtain a crude product. The crude product was purified by reversed-phase column chromatography (CH₃CN/H₂O = 5 to 95%) to obtain the title compound **162-8** (25 mg, colorless oil). LC-MS (ESI): m/z 447.0 [M+H]⁺.

### Preparation of compound 162

Compound **162-8** (25 mg, 0.056 mmol) was dissolved in DMF (1.5 mL), then compound 1H-tetrazole (20 mg, 0.280 mmol) and potassium carbonate (39 mg, 0.279 mmol) were added thereto, and the reaction system was reacted at 80°C for 2 hours in a sealed tube. After the reaction system was cooled, the reaction mixture was filtered, and the crude filtrate was purified by preparative separation (chromatographic column: Welch Xtimate^{®} C18 21.2 × 250 mm; column temperature: 25°C; mobile phase: water (10 mM/L NH₄HCO₃)-acetonitrile; proportion of acetonitrile in the mobile phase: 45% to 65% in 12 min; flow rate: 30 mL/min) to obtain the title compound **162** (10 mg, yield: 34%). LC-MS (ESI): m/z 517.4 [M+H]⁺. ¹H NMR (400 MHz, DMSO-*d6*) δ 9.13 (s, 1H), 7.60 - 7.50 (m, 1H), 7.33 - 7.25 (m, 1H), 7.13 (s, 1H), 7.03 - 7.00 (m, 1H), 6.97 - 6.92 (m, 2H), 6.83 - 6.81 (m, 2H), 5.42 (d, *J* = 14.6 Hz, 1H), 5.01 (d, *J* = 14.6 Hz, 1H), 3.08 - 3.04 (m, 4H), 2.50 - 2.43 (m, 4H), 2.26 - 2.20 (m, 3H), 1.96 - 1.93 (m, 3H), 1.75 - 1.72 (m, 3H). ¹⁹F NMR (376 MHz, DMSO-d6) δ -102.96 - - 103.17 (m, 1F), -109.11 - -109.35 (m, 2F), -109.67 (d, *J =* 9.6 Hz, 1F).

### Example 163: Preparation of compound 163

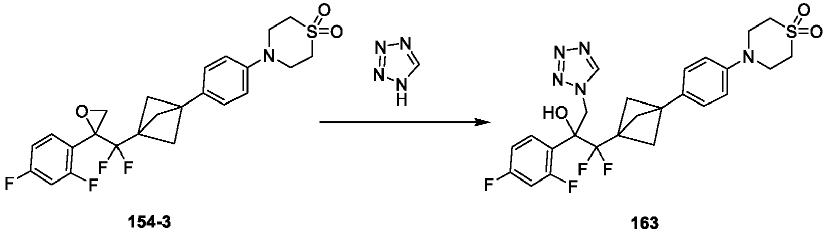

### Preparation of compound 163

Compound **154-3** (20 mg, 0.042 mmol) was dissolved in DMF (2 mL), then 1H-tetrazole (4.42 mg, 0.063 mmol) and potassium carbonate (11.61 mg, 0.084 mmol) were added thereto, and the reaction system was replaced with nitrogen three times and reacted at 80°C for 16 hours. After the reaction system was cooled, the filtrate was filtered, and the filtrate was purified by preparative separation (preparation column: Pursuit XRs 10 C18 250 * 21.2 mm; flow rate: 20 mL/min; mobile phase: A: 0.1% FA aqueous solution, B: acetonitrile; gradient: 60 to 60% acetonitrile content, retention time of 8.0 to 9.0 min) to obtain the title compound **163** (3.85 mg). LC-MS (ESI): m/z 552.0 [M+H]⁺. ¹H NMR (400 MHz, DMSO-*d6*) δ 9.17 (s, 1H), 7.54 (dd, *J* = 15.8, 8.9 Hz, 1H), 7.32 - 7.22 (m, 2H), 7.01 (dd, *J* = 10.9, 5.4 Hz, 3H), 6.91 (d, *J* = 8.7 Hz, 2H), 5.42 (d, *J* = 14.5 Hz, 1H), 5.03 (d, *J =* 14.5 Hz, 1H), 3.71 (s, 4H), 3.07 (d, *J =* 4.6 Hz, 4H), 1.96 (d, *J =* 9.3 Hz, 3H), 1.75 (d, *J =* 9.4 Hz, 3H).

### Example 164: Preparation of compound 164

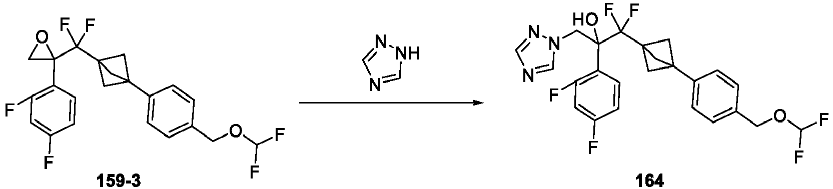

### Preparation of compound 164

1,2,4-Triazole (25 mg, 0.35 mmol) was dissolved in DMF (1 mL), and NaH (14 mg, 0.35 mmol) was added thereto at 0°C. The reaction system was stirred for 30 min, then added with compound **159-3** (30 mg, 0.07 mmol), transferred to 70°C and reacted for 16 hours. After the reaction system was cooled, the reaction mixture was filtered, and the crude filtrate was purified by preparative separation (chromatographic column: Agilent 10 Prep-C18 250 × 21.2 mm; column temperature: 25°C; mobile phase: water (0.1% FA)-acetonitrile; proportion of acetonitrile in the mobile phase: 50% to 70% in 12 min; flow rate: 30 mL/min) to obtain the title compound **164** (6.5 mg, yield: 22%). LC-MS (ESI): m/z 498.2 [M+H]⁺. ¹H NMR (400 MHz, Chloroform-*d*) δ 8.04 (s, 1H), 7.85 (s, 1H), 7.73 (q, *J* = 8.5 Hz, 1H), 7.47 (d, *J* = 7.8 Hz, 2H), 7.38 (d, *J* = 7.9 Hz, 2H), 6.94 (t, *J* = 8.6 Hz, 1H), 6.35 (t, *J* = 74.2 Hz, 2H), 5.16 (d, *J =* 14.1 Hz, 1H), 4.96 (s, 2H), 4.79 (d, *J* = 14.2 Hz, 1H), 1.91 (dd, *J* = 9.5, 2.0 Hz, 3H), 1.77 (dd, *J* = 9.4, 2.0 Hz, 3H). ¹⁹F NMR (376 MHz, Chloroform-*d*) δ -84.32 (s, 2F), -110.63 (d, 1F), - 111.12 (d, 1F), -111.98 (d, 2F).

### Example 165: Preparation of compounds 165, 165A, and 165B

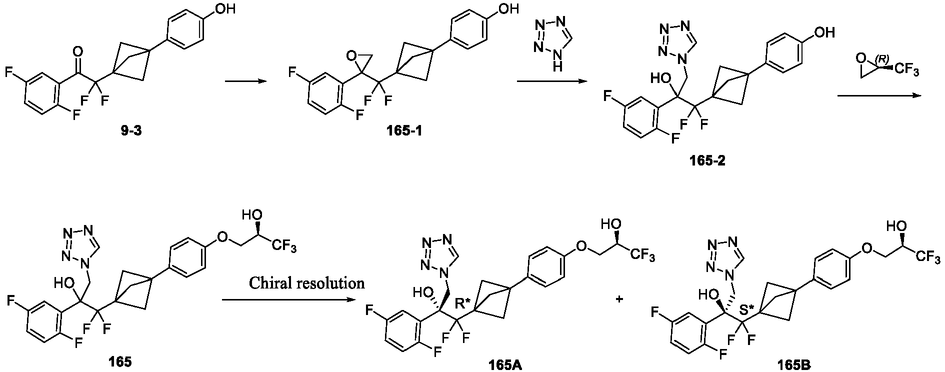

### Preparation of compound 165-1

Compound **9-3** (1.2 g, 3.43 mmol) was dissolved in a mixed solvent of dichloromethane (15 mL) and water (15 mL), then trimethylsulfoxonium iodide (3.02 g, 13.7 mmol) and sodium hydroxide (549 mg, 13.7 mmol) were added thereto, and the reaction system was refluxed for 16 hours. After the reaction system was cooled to room temperature, the pH of the reaction system was adjusted to between 6 and 7 with dilute hydrochloric acid, and the reaction system was extracted with DCM (20 mL × 3). The organic phases were combined, dried, and concentrated to obtain a crude product, and the crude product was purified by normal-phase silica gel column chromatography (EtOAc/PE = 0 to 10%) to obtain the title compound **165-1** (1.17 g, light yellow oil, yield: 94%). ¹H NMR (400 MHz, Chloroform-d) δ 7.32-7.28 (m, 1H), 7.11 - 6.95 (m, 5H), 6.78 - 6.72 (m, 2H), 3.41 (d, *J =* 5.2 Hz, 1H), 3.03 - 2.93 (m, 1H), 2.09 - 2.03 (m, 6H).

### Preparation of compound 165-2

Compound **165-1** (1.0 g, 2.74 mmol) was dissolved in DMF (10 mL), then compound 1H-tetrazole (961 mg, 13.72 mmol) and potassium carbonate (1.9 g, 13.72 mmol) were added thereto, and the reaction system was stirred at 80°C for 16 hours in a sealed tube. After the reaction system was cooled, the reaction mixture was filtered, and the crude filtrate was purified by preparative separation (preparation method: mobile phase: A: 0.1% formic acid aqueous solution; B: acetonitrile; chromatographic column: Agilent 10 Prep-C18 250 × 21.2 mm; column temperature: 25°C; gradient: 45% to 65% acetonitrile in 12 min; flow rate: 30 mL/min) to obtain the title compound **165-2** (645 mg, yield: 54%). LC-MS (ESI): m/z 435.2 [M+H]⁺.

### Preparation of compound 165

Compound **165-2** (500 mg, 1.15 mmol) was dissolved in acetonitrile (20 mL), then potassium carbonate (0.318 g, 2.30 mmol) was added thereto. The reaction system was stirred for 5 min, then added with R-(+)-2-trifluoromethyloxirane (CAS: 143142-90-9, 167 mg, 1.5 mmol), stirred and reacted at 50°C for 16 hours. After the reaction system was cooled, the reaction mixture was filtered, and the crude filtrate was purified by preparative separation (preparation method: chromatographic column: Agilent 10 Prep-C18 250 × 21.2 mm; column temperature: 25°C; mobile phase: water (0.1% TFA)-acetonitrile; proportion of acetonitrile in the mobile phase: 55% to 75% in 12 min; flow rate: 30 mL/min) to obtain the target product **165** (350 mg yield: 56%). LC-MS (ESI): m/z 547.2 [M+H]⁺. ¹H NMR (400 MHz, DMSO-*d6)* δ 9.16 (s, 1H), 7.36 - 7.21 (m, 4H), 7.12 - 7.04 (m, 2H), 6.95 - 6.82 (m, 2H), 6.62 (d, *J =* 6.6 Hz, 1H), 5.44 (d, *J* = 14.5 Hz, 1H), 5.02 (d, *J* = 14.6 Hz, 1H), 4.36 - 4.33 (m, 1H), 4.16 - 4.10 (m, 1H), 4.03 - 3.99 (m, 1H), 2.02 - 1.99 (m, 3H), 1.82 - 1.79 (m, 3H). ¹⁹F NMR (376 MHz, DMSO-*d6*) δ -76.06 (s, 3F), -108.76 (d, *J* = 9.4 Hz, 1F), -108.85 (s, 1F), -113.18 - - 113.42 (m, 1F), -118.37 (d, *J =* 18.8 Hz, 1F).

### Preparation of compounds 165A and 165B

Compound **165** (520 mg) was subjected to SFC chiral preparative resolution (preparative separation method, instrument model: MG II preparative SFC (SFC-1); model of chromatographic column: Cellulose-2, 250 × 30 mm I.D., 10 µm; mobile phase: A: CO₂ B: ethanol; elution gradient: B 40%; flow rate: 70 mL/min; column pressure: 100 bar; column temperature: 35°C; detection wavelength: 220 nm; cycle: about 8 min) to obtain the title compounds **165A** (253 mg) and **165B** (260 mg). Compound **165A:** Chiral analysis method (model of chromatographic column: Cellulose-2 150 × 4.6 mm I.D., 3 µm; mobile phase: A: CO₂ B: ethanol (0.05% DEA); elution gradient: the mobile phase increased from 5% B to 40% B within 5 min and maintained at 40% B for 2.5 min for elution, then 5% B was equilibrated for 2.5 min; flow rate: 2.5 mL/min; column temperature: 35°C; column pressure: 1500 psi; detection wavelength: 220 nm; RT = 2.575 min). LC-MS (ESI): m/z 547.2 [M+H]⁺. ¹H NMR (400 MHz, DMSO-*d6*) δ 9.16 (s, 1H), 7.37 - 7.16 (m, 4H), 7.12 - 6.98 (m, 2H), 6.93 - 6.82 (m, 2H), 6.62 (d, *J* = 6.8 Hz, 1H), 5.45 (d, *J* = 14.6 Hz, 1H), 5.02 (d, *J* = 14.6 Hz, 1H), 4.38 -4.32 (m, 1H), 4.14 - 4.10 (m, 1H), 4.03 - 3.99 (m, 1H), 2.02 - 1.99 (m, 3H), 1.2 - 1.79 (m, 3H). ¹⁹F NMR (376 MHz, DMSO-*d6*) δ -76.07 (s, 3F), -108.56 - -109.22 (m, 2F), -113.18 - -113.12 (m, 1F), -118.38 (d, *J* = 18.1 Hz, 1F).

Compound **165B:** Chiral analysis method (model of chromatographic column: Cellulose-2 150 × 4.6 mm I.D., 3 µm; mobile phase: A: CO₂ B: ethanol (0.05% DEA); elution gradient: the mobile phase increased from 5% B to 40% B within 5 min and maintained at 40% B for 2.5 min for elution, then 5% B was equilibrated for 2.5 min; flow rate: 2.5 mL/min; column temperature: 35°C; column pressure: 1500 psi; detection wavelength: 220 nm; RT = 3.153 min). LC-MS (ESI): m/z 547.2 [M+H]⁺. ¹H NMR (400 MHz, DMSO-d6) δ 9.15 (s, 1H), 7.35 - 7.19 (m, 4H), 7.10 - 7.04 (m, 2H), 6.92 - 6.85 (m, 2H), 6.62 (d, *J* = 6.6 Hz, 1H), 5.44 (d, *J =* 14.6 Hz, 1H), 5.02 (d, *J =* 14.6 Hz, 1H), 4.38 - 4.31 (m, 1H), 4.13 - 4.10 (m, 1H), 4.03 - 3.99 (m, 1H), 2.02 - 1.99 (m, 3H), 1.82 - 1.79 (m, 3H). ¹⁹F NMR (376 MHz, DMSO-*d6)* δ -76.07 (s, 1F), -108.56 - -109.22 (m, 2F), -113.18 - -113.12 (m, 1F), -118.38 (d, *J =* 18.1 Hz, 1F).

### Example 166: Preparation of compounds 166, 166A, and 166B

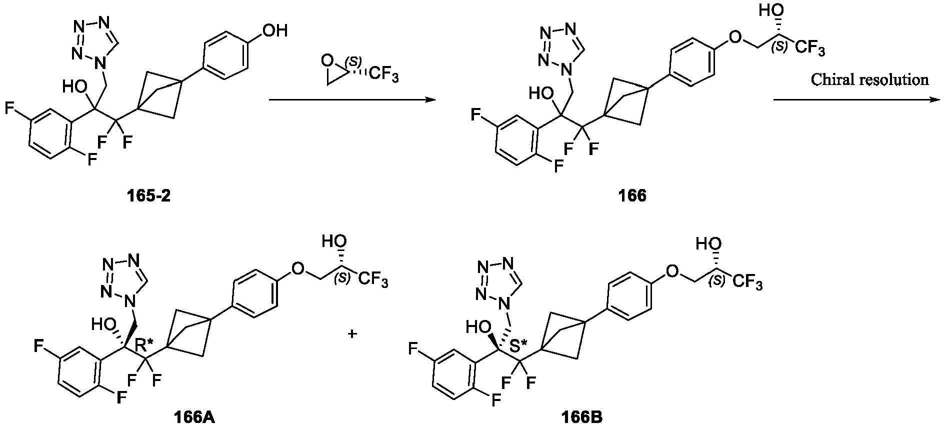

### Preparation of compound 166

Compound **165-2** (145 mg, 0.334 mmol) was dissolved in acetonitrile (2 mL), then potassium carbonate (92 mg, 0.667 mmol) was added thereto. The reaction system was stirred for 5 min, then added with (S)-(-)-3,3,3-trifluoro-1,2-epoxypropane (CAS: 130025-34-2, 48 mg, 0.433 mmol), stirred and reacted at 50°C for 16 hours. After the reaction system was cooled, the reaction mixture was filtered, and the crude filtrate was purified by preparative separation (preparation method: chromatographic column: Agilent 10 Prep-C18 250 × 21.2 mm; column temperature: 25°C; mobile phase: water (0.1% TFA)-acetonitrile; proportion of acetonitrile in the mobile phase: 45% to 65% in 12 min; flow rate: 30 mL/min) to obtain the title compound **166** (60 mg yield: 32%). LC-MS (ESI): m/z 547.2 [M+H]⁺. ¹H NMR (400 MHz, DMSO-*d6*) δ 9.15 (d, *J* = 1.6 Hz, 1H), 7.30 - 7.23 (m, 4H), 7.07 - 7.04 (m, 2H), 6.89 - 6.86 (m, 2H), 6.63 - 6.61 (m, 1H), 5.44 (dd, *J =* 14.6, 1.6 Hz, 1H), 5.02 (d, *J =* 14.6 Hz, 1H), 4.36 - 4.31 (m, 1H), 4.13 - 4.10 (m, 1H), 4.09 - 3.99 (m, 1H), 2.01-1.99 (m, 3H), 1.82 -1.78 (m, 3H). ¹⁹F NMR (376 MHz, DMSO-*d6*) δ -76.06 (s, 3F), -108.76 (d, *J* = 9.4 Hz, 1F), - 108.85 (s, 1F), -113.18 - -113.42 (m, 1F), -118.37 (d, *J* = 18.8 Hz, 1F).

### Resolution preparation of compounds 166A and 166B

Compound **166** (60 mg) was subjected to SFC chiral preparative resolution (preparative separation method, instrument model: MG II preparative SFC (SFC-1); model of chromatographic column: Cellulose-2, 250 × 30 mm I.D., 10 µm; mobile phase: A: CO₂ B: ethanol; elution gradient: B 40%; flow rate: 70 mL/min; column pressure: 100 bar; column temperature: 35°C; detection wavelength: 220 nm; cycle: about 8 min) to obtain the title compounds **166A** (25 mg) and **166B** (24 mg).

Compound **166A:** Chiral analysis method (model of chromatographic column: Cellulose-2 150 × 4.6 mm I.D., 3 µm; mobile phase: A: CO₂ B: ethanol (0.05% DEA); elution gradient: the mobile phase increased from 5% B to 40% B within 5 min and maintained at 40% B for 2.5 min for elution, then 5% B was equilibrated for 2.5 min; flow rate: 2.5 mL/min; column temperature: 35°C; column pressure: 1500 psi; detection wavelength: 220 nm; RT = 2.624 min). LC-MS (ESI): m/z 547.2 [M+H]⁺. ¹H NMR (400 MHz, DMSO-*d6*) δ 9.15 (s, 1H), 7.34 - 7.20 (m, 4H), 7.09 - 7.03 (m, 2H), 6.91 - 6.84 (m, 2H), 6.62 (d, *J* = 6.6 Hz, 1H), 5.44 (d, *J* = 14.5 Hz, 1H), 5.02 (d, *J* = 14.6 Hz, 1H), 4.40 - 4.30 (m, 1H), 4.14 - 4.10 (m, 1H), 4.03 - 3.99 (m, 1H), 2.02 - 1.99 (m, 3H), 1.82 - 1.79 (m, 3H). ¹⁹F NMR (376 MHz, DMSO-*d6)* δ -76.06 (s, 3F), -108.53 - -109.30 (m, 2F), -113.30 (m, 1F), -118.38 (d, *J =* 19.1 Hz, 1F).

Compound **166B:** Chiral analysis method (model of chromatographic column: Cellulose-2 150 × 4.6 mm I.D., 3 µm; mobile phase: A: CO₂ B: ethanol (0.05% DEA); elution gradient: the mobile phase increased from 5% B to 40% B within 5 min and maintained at 40% B for 2.5 min for elution, then 5% B was equilibrated for 2.5 min; flow rate: 2.5 mL/min; column temperature: 35°C; column pressure: 1500 psi; detection wavelength: 220 nm; RT = 3.254 min). LC-MS (ESI): m/z 547.2 [M+H]⁺. ¹H NMR (400 MHz, DMSO-*d6*) δ 9.15 (s, 1H), 7.36 - 7.19 (m, 4H), 7.09 - 7.01 (m, 2H), 6.90 - 6.80 (m, 2H), 6.62 (d, *J* = 6.8 Hz, 1H), 5.44 (d, *J* = 14.6 Hz, 1H), 5.02 (d, *J* = 14.6 Hz, 1H), 4.44 - 4.28 (m, 1H), 4.13 - 4.10 (m, 1H), 4.03 - 3.99 (m, 1H), 2.02 - 1.99 (m, 3H), 1.82 - 1.79 (m, 3H). ¹⁹F NMR (376 MHz, DMSO-*d6)* δ -76.06 (s, 3F), -108.53 - -109.30 (m, 2F), -113.30 (m, 1F), -118.38 (d, *J =* 19.1 Hz, 1F).

### Test example 1: Minimal inhibitory concentration (MIC) test of compounds on fungal growth

### (1) Main reagents:

RPMI1640 medium: Brand: Gibco, article number: 31800-014
Sabouraud dextrose agar (SDA): Brand: Hope Bio, article number: HB0253-81
Voriconazole: Brand: Adamas, article number: 22105A
Amphotericin B: Brand: Abeam, article number: ab141199

### (2) Fungal strains are shown in Table 1 below:

**Table 1**

| **Strain category** | **Strain code** | **Strain destination** |
|---|---|---|
| Aspergillus fumigatus | ATCC MYA-4609 | CBS 101355 [AF 293] |
| Candida albicans | ATCC MYA-2876 | SC5314 |
| Candida albicans | ATCC 64124 | Darlington |
| Candida glabrata | ATCC 15126 | Mutant TMAGR-23 [CBS 15126, NRRL Y-17770] |
| Candida parapsilosis | ATCC 22019 | CBS 604 [CCRC 20515, DBVPG 6150, DMS 5784, IBL 2545, IFO 1396, IGC 2545, JCM 1785, NCYC 601, NRRLY-12969, UCD 61-27] |
| Candida tropicalis | ATCC 750 | [1909, ATCC 4563, ATCC 7349, CBS 94, CCRC 20520, IFO 1070, IFO 1400, JCM 1541, NRRLY-12699, NRRL Y-12968, NRRLY-607] |
| Candida krusei | ATCC 6258 | [ATCC 749, CBS 573, CCRC 20514, IFO 1064, IFO 1395, JCM 1609, NBRC 1395, NRRL Y-413, NRRL Y-7179] |
| Cryptococcus neoformans | ATCC 208821 | H99 [H99JP, NYSD 1649] |

### (3) Test method:

MIC test was conducted in accordance with the guidelines and requirements of CLSI M27 (for yeasts) and M38 (for Aspergillus).

Strain preparation: The strains were inoculated 1 day in advance by streaking on SDA plates with glycerol strains stored at -80°C. The strains were cultured at 35°C and 40 to 60% humidity for 18 to 24 hours. Streak inoculation of Aspergillus fumigatus and Cryptococcus neoformans was required to be done 3 days and 2 days in advance, respectively.

Preparation of culture medium and compounds: The liquid culture medium RPMI was prepared with pure water, and 0.165 mol/L MOPS was added to adjust the pH to 7.0. The mixture was filtered with a 0.22 µm-membrane filter, sterilized, and stored at 4°C (no more than 3 months). 0.85% physiological saline was sterilized at 121°C for 30 min and then stored at room temperature (no more than 1 week). The compounds were dissolved in DMSO to 12.8 mg/mL and stored at -20°C.

For yeast, 3 to 5 colonies were selected from the SDA plate on the day of testing and fully suspended in 5 mL of sterilized 0.85% physiological saline. The turbidity of the bacterial suspension was measured with a turbidimeter, and the turbidity was adjusted to approximately 0.2. The bacterial suspension was 50-fold and 20-fold (1000-fold in total) diluted in RPMI1640 medium, respectively, to serve as the inoculum. The final inoculum concentration was 500 to 2500 CFU/mL.

For Aspergillus, 5 mL of physiological saline was added to cover the mycelium, and the spores were gently scraped off with a spreader. The spore suspension was then transferred to a sterile test tube. An appropriate amount of spore suspension was taken and counted under a microscope using a hemocytometer. Spore concentration was adjusted to approximately 0.4 to 5 × 10⁴ spores/mL using RPMI1640 medium.

The compound was diluted to the highest 800 µg/mL (or 400 µg/mL) with DMSO, and a 10-step 2-fold gradient dilution was performed, resulting in 11 concentrations. 2 µL of the gradient-diluted compound was transferred to the corresponding wells of a 96-well plate, and then 198 µL of the inoculum was transferred to the test plate. The plate was incubated at 35°C for 24 hours (Aspergillus fumigatus and Cryptococcus neoformans were incubated for 48 and 72 hours, respectively).

### (4) MIC evaluation:

After the incubation, the growth of the fungi was visually observed. The lowest concentration of the compound that inhibited the growth of yeast by ≥ 50% (inhibited Aspergillus by 100%) was defined as the minimum inhibitory concentration (MIC, µg/mL). A magnifying glass or reading at OD530 nm could be used to assist in determining the MIC. Photos were taken of the test plates for record keeping. The results are shown in Table 2 below.

**Table 2: In vitro antifungal activity results MIC (µg/mL) of some preferred compounds**

| Compound | Candida albicans ATCC MYA-2876 | Aspergillus fumigatus ATCC MYA-4609 | Compound | Candida albicans ATCC MYA-2876 | Aspergillus fumigatus ATCC MYA-4609 | Compound | Candida albicans ATCC MYA-2876 | Aspergillus fumigatus ATCC MYA-4609 |
|---|---|---|---|---|---|---|---|---|
| **1** | 0.015 | 8 | **40** | ≤ 0.0039 | 16 | **104** | 0.03 | / |
| **1A** | 0.0078 | 4 | **40A** | ≤ 0.0039 | 4 | **105** | 0.016 | / |
| **1B** | 0.5 | / | **41** | 0.0078 | 8 | **107** | 0.06 | / |
| **2** | 0.0078 | 1 | **41A** | ≤ 0.0039 | 8 | **107A** | 0.016 | / |
| **2A** | ≤ 0.0039 | 1 | **42** | 0.06 | / | **108** | 0.06 | / |
| **2B** | 0.25 | / | **43** | 0.03 | / | **110** | 0.06 | / |
| **3** | 0.03 | 2 | **44** | 0.06 | / | **110A** | 0.016 | / |
| **3A** | ≤ 0.0039 | 1 | **45** | 0.03 | | **111** | 0.016 | / |
| **3B** | 1 | / | **46** | 0.03 | | **114** | 0.06 | / |
| **4A** | 0.06 | / | **46A** | 0.0078 | 2 | **115** | 0.125 | / |
| **5** | 0.016 | / | **47** | 0.0078 | | **117** | 0.06 | / |
| **6** | 0.13 | / | **48** | 0.06 | / | **118** | 0.0078 | / |
| **6A** | 0.06 | / | **48A** | 0.016 | / | **119** | 0.06 | / |
| **6B** | 2 | / | **49** | 0.0078 | 1 | **120** | 0.125 | / |
| **7** | 0.016 | / | **49A** | ≤ 0.0039 | 0.5 | **121** | 0.0078 | 8 |
| **7A** | 0.0078 | / | **50** | 0.016 | 4 | **121A** | ≤ 0.0039 | 4 |
| **7B** | 2 | / | **50A** | 0.0078 | 2 | **122** | 0.016 | / |
| **8** | 0.5 | / | **51** | 0.0078 | 4 | **124** | 0.0078 | / |
| **8A** | 0.0078 | / | **51A** | ≤ 0.0039 | 1 | **125** | ≤ 0.0039 | 1 |
| **9** | 0.03 | / | **51B** | 0.0078 | 4 | **125A** | ≤ 0.0039 | 0.5 |
| **9A** | 0.06 | / | **52** | 1 | / | **126** | 0.016 | / |
| **10** | ≤ 0.0039 | / | **53** | 0.5 | / | **128** | 0.0078 | 4 |
| **10A** | ≤ 0.0039 | 1 | **54** | 0.5 | / | **129** | 0.0078 | 4 |
| **10B** | 1 | / | **55** | 0.25 | / | **131** | 0.0078 | 8 |
| **11** | 0.03 | / | **56** | 0.5 | / | **131A** | ≤ 0.0039 | 4 |
| **14** | 0.03 | / | **57** | 0.06 | / | **132A** | ≤ 0.0039 | 0.5 |
| **14A** | 0.016 | / | **58** | 0.06 | / | **133** | 003 | / |
| **17** | 0.125 | / | **59** | 0.13 | / | **134** | 0.0078 | 4 |
| **18** | 0.016 | / | **60** | 0.06 | / | **134A** | ≤ 0.0039 | 2 |
| **18A** | 0.0078 | / | **61** | 0.016 | 8 | **135** | 0.016 | 8 |
| **18B** | 2 | / | **62** | 0.016 | / | **136** | 0.016 | 4 |
| **19** | 0.016 | / | **64** | 0.06 | / | **137** | ≤ 0.0039 | 8 |
| **20** | 0.13 | / | **65** | 0.0078 | 32 | **139** | 0.06 | / |
| **20A** | 0.06 | / | **68** | 0.13 | / | **141** | 0.06 | / |
| **21** | ≤ 0.0039 | 2 | **69** | 0.016 | 4 | **142** | 0.0078 | 8 |
| **21A** | ≤ 0.0039 | 1 | **70** | 0.13 | / | **142A** | ≤ 0.0039 | 4 |
| **21B** | 1 | / | **71** | 0.03 | / | **142B** | ≤ 0.0039 | 4 |
| **22** | 0.0078 | 4 | **72** | 0.03 | / | **144** | 0.0078 | 8 |
| **22A** | 0.0078 | 2 | **73** | 0.016 | / | **144A** | ≤ 0.0039 | 2 |
| **23** | ≤ 0.0039 | 1 | **74** | 0.13 | / | **144B** | ≤ 0.0039 | 4 |
| **24** | 0.03 | / | **75** | 0.13 | / | **145** | 0.03 | / |
| **25** | 0.0078 | 2 | **76** | 0.016 | 8 | **146** | 0.06 | / |
| **26** | 0.016 | / | **77** | 0.016 | / | **147** | 0.016 | / |
| **27** | ≤ 0.0039 | 1 | **79** | 0.25 | / | **148** | 0.016 | / |
| **27A** | ≤ 0.0039 | 0.5 | **80** | 0.06 | / | **149** | ≤ 0.0039 | 8 |
| **27B** | 0.06 | / | **81** | ≤ 0.0039 | 1 | **149A** | ≤ 0.0039 | 2 |
| **27A-P1** | 2 | / | **82** | 0.06 | / | **150** | 0.03 | / |
| **27A-P2** | 0.5 | / | **83** | 0.06 | / | **150A** | 0.016 | / |
| **28** | 0.016 | / | **84** | 0.25 | / | **151** | ≤ | 2 |
| | | | | | | | 0.0039 | |
| **28A** | 0.25 | / | **85** | 0.03 | / | **152** | 0.0078 | / |
| **29** | ≤ 0.0039 | 2 | **86** | 0.06 | / | **154** | 0.0078 | / |
| **29A** | ≤ 0.0039 | 1 | **87** | 0.016 | / | **157** | 0.016 | / |
| **29B** | 0.125 | / | **88** | 0.25 | / | **159** | 0.0078 | / |
| **30** | 0.06 | / | **89** | 0.016 | / | **159A** | ≤ 0.0039 | 2 |
| **31** | 0.125 | / | **90** | ≤ 0.0039 | 0.5 | **160** | 0.016 | / |
| **32** | 0.016 | / | **90A** | ≤ 0.0039 | 0.5 | **161** | 0.016 | / |
| **32A** | ≤ 0.0039 | / | **91** | ≤ 0.0039 | / | **162** | 0.03 | / |
| **32B** | 0.06 | / | **91A** | ≤ 0.0039 | / | **163** | 0.0078 | / |
| **33** | 0.06 | / | **92** | 0.03 | / | **164** | 0.03 | / |
| **34** | 0.016 | / | **95** | ≤ 0.0039 | / | **165** | 0.03 | 4 |
| **34A** | 0.0078 | / | **97** | 0.0078 | / | **165A** | 0.016 | / |
| **35** | ≤ 0.0039 | 2 | **97A** | ≤ 0.0039 | 4 | **166** | 0.06 | 8 |
| **35A** | ≤ 0.0039 | 1 | **97B** | 0.0078 | 8 | **166A** | 0.06 | / |
| **35B** | 0.125 | / | **98** | ≤ 0.0039 | 2 | | | |
| **36** | ≤ 0.0039 | 8 | **98A** | ≤ 0.0039 | 1 | | | |
| **36A** | 0.0078 | 4 | **98B** | ≤ 0.0039 | 2 | | | |
| **37** | ≤ 0.0039 | 2 | **99** | 0.016 | / | | | |
| **37A** | ≤ 0.0039 | 1 | **99A** | 0.016 | / | | | |
| **38** | 0.5 | / | **99B** | 0.03 | / | | | |
| **39** | 0.0078 | / | **100** | 0.0078 | / | | | |

### Test example 2: Drug-drug interaction study, pharmacokinetic study of single drug and combination drug administered to CD1 mice by gavage

### A drug-drug interaction experiment was carried out on the compounds of the examples of the present disclosure

### (1) Preparation of dosing solutions

The dosing solution was prepared on the day of administration. An appropriate amount of nifedipine was precisely weighed into a centrifuge tube, then an appropriate volume of 0.5% MC was added thereto and vortexed to dissolve, resulting in a dosing solution with a concentration of 1.5 mg/mL.

Appropriate amounts of ketoconazole and isavuconazonium sulfate (CAS# 946075-13-4) were precisely weighed and placed into separate centrifuge tubes. An appropriate volume of 0.5% MC was added to each tube and vortexed to dissolve, resulting in dosing solutions with concentrations of 3 mg/mL and 12 mg/mL, respectively.

An appropriate amount of the compound of the example of the present disclosure was precisely weighed into a centrifuge tube, then an appropriate volume of 16% SBECD was added thereto and vortexed to dissolve, resulting in a dosing solution with a concentration of 10 mg/mL.

### (2) Animal experiments

CD1 mice, 6 to 8 weeks old, male, were divided into seven groups. The individual administration groups and their respective dosages were as follows: a nifedipine (15 mg/kg) monotherapy group, a ketoconazole (30 mg/kg) monotherapy group, an isavuconazole sulfate (120 mg/kg) monotherapy group, and a compound of the example of the present disclosure (100 mg/kg) monotherapy group; the combination therapy groups and their respective dosages were as follows: a ketoconazole (30 mg/kg) + nifedipine (15 mg/kg) combination therapy group, an isavuconazonium sulfate (120 mg/kg) + nifedipine (15 mg/kg) combination therapy group, and a compound of the example of the present disclosure (100 mg/kg) + nifedipine (15 mg/kg) combination therapy group.

Mice were fasted for no less than 12 hours before administration and had free access to water. 4 hours after administration, the mice were fed uniformly.

The nifedipine monotherapy group was administered a single dose of nifedipine at 15 mg/kg by gavage.

The ketoconazole monotherapy group was administered a single dose of ketoconazole at 30 mg/kg by gavage.

The isavuconazonium sulfate monotherapy group was administered a single dose of isavuconazonium sulfate at 120 mg/kg (equivalent to a parent drug isavuconazole dose of 64 mg/kg) by gavage.

The compound of the example of the present disclosure monotherapy group was administered a single dose of the compound of the example of the present disclosure at 100 mg/kg by gavage.

For the ketoconazole (30 mg/kg) + nifedipine (15 mg/kg) combination therapy group, a single dose of ketoconazole at 30 mg/kg was administered by gavage, followed by a dose of nifedipine at 15 mg/kg half an hour later.

For the isavuconazonium sulfate (120 mg/kg) + nifedipine (15 mg/kg) combination therapy group, a single dose of isavuconazonium sulfate at 120 mg/kg (equivalent to a parent drug isavuconazole dose of 64 mg/kg) was administered by gavage, followed by a dose of nifedipine at 15mg/kg half an hour later.

For the compound of the example of the present disclosure (100 mg/kg) + nifedipine (15 mg/kg) combination therapy group, a single dose of the compound of the example of the present disclosure at 100 mg/kg was administered by gavage, followed by a dose of nifedipine at 15 mg/kg half an hour later.

### (3) Sample collection and processing

After the administration of the drug, mice in the monotherapy group had approximately 30 µL of blood collected at 0.25, 0.5, 1, 2, 4, 6, 8, 24, and 48 hours post-administration. The blood was placed in sodium heparin anticoagulant tubes and centrifuged at 1900 g for 10 minutes (4°C) to separate the plasma. The plasma was stored at no higher than -60°C until testing. The process from collection to plasma separation was carried out under ice bath conditions.

After the administration of the nifedipine, mice in the combination therapy group had approximately 30 µL of blood collected at 0.25, 0.5, 1, 2, 4, 6, 8, 24, and 48 hours post-administration. The blood was placed in sodium heparin anticoagulant tubes and centrifuged at 1900 g for 10 minutes (4°C) to separate the plasma. The plasma was stored at no higher than -60°C until testing. The process from collection to plasma separation was carried out under ice bath conditions.

### (4) Sample analysis

Using LC-MS/MS analysis method, the concentrations of nifedipine, ketoconazole, parent drug isavuconazole, and the compound of the example of the present disclosure in the biological samples obtained in this experiment were determined respectively.

### (5) Pharmacokinetic parameter calculation and data processing

Pharmacokinetic parameters were calculated by non-compartment model in Phoenix WinN onlin8.3.

### (6) Drug-drug interaction experimental results

In the PK study of mice in the ketoconazole (30 mg/kg) monotherapy group, the exposure to ketoconazole was not significantly different from the exposure to ketoconazole in the PK study of mice in the ketoconazole (30 mg/kg) + nifedipine (15 mg/kg) combination therapy group.

In the PK study of mice in the isavuconazonium sulfate (120 mg/kg) monotherapy group, the exposure to parent drug isavuconazole was not significantly different from the exposure to parent drug isavuconazole in the PK study of mice in the isavuconazonium sulfate (120 mg/kg) + nifedipine (15 mg/kg) combination therapy group in the present experiment.

In the PK study of mice in the compound of the example of the present disclosure (100 mg/kg) monotherapy group, the exposure to compound of the example of the present disclosure was not significantly different from the exposure to compound of the example of the present disclosure in the PK study of mice in the compound of the example of the present disclosure (100 mg/kg) + nifedipine (15 mg/kg) combination therapy group in the present experiment.

Compared to the exposure of nifedipine (AUCi) in the PK study of mice in the nifedipine (15 mg/kg) monotherapy group, the exposure of nifedipine (AUCi) in the PK study of mice in the ketoconazole (30 mg/kg) + nifedipine (15 mg/kg) combination therapy group increased by more than 6.1 times.

Compared to the exposure of nifedipine (AUCi) in the PK study of mice in the nifedipine (15 mg/kg) monotherapy group, the exposure of nifedipine (AUCi) in the PK study of mice in the isavuconazonium sulfate (120 mg/kg) + nifedipine (15 mg/kg) combination therapy group increased by more than 8.2 times.

Compared to the exposure of nifedipine (AUCi) in the PK study of mice in the nifedipine (15 mg/kg) monotherapy group, the change in the exposure of nifedipine (AUCi) in the PK study of mice in the compound of the example of the present disclosure (100 mg/kg) + nifedipine (15 mg/kg) combination therapy group was within a 2-fold range.

## Claims

1. A compound of formula (I), an optical isomer thereof, a tautomer thereof, or a pharmaceutically acceptable salt thereof, 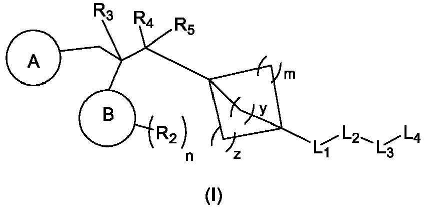 wherein
ring A is selected from 5- to 6-membered heteroaryl;
ring B is selected from phenyl and 5- to 6-membered heteroaryl;
R₃ is selected from OH, NH₂, halogen, C₁₋₆ alkyl, C₁₋₆ heteroalkyl, -OC(=O)C₁₋₆ alkyl, - NHC(=O)C₁₋₆ alkyl, 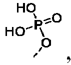 and -OC₁₋₆ alkyl-OP(=O)₂(OH)₂;
R₂, R₄, and R₅ are each independently selected from H, CN, OH, F, Cl, Br, I, C₁₋₆ alkyl, and C₁₋₆ heteroalkyl, and the C₁₋₆ alkyl or C₁₋₆ heteroalkyl is optionally substituted by 1, 2, or 3 CN, OH, F, Cl, Br, I, or C₁₋₆ alkyl groups;
m, y, and z are each independently selected from 1, 2, 3, or 4;
n is selected from 0, 1, 2, or 3;
L₁ is selected from a single bond, -NH-, C₁₋₆ alkyl, C₂₋₆ alkynyl, phenyl, and 5- to 6-membered heteroaryl, and the C₁₋₆ alkyl, C₂₋₆ alkynyl, phenyl, or 5- to 6-membered heteroaryl is optionally substituted by 1, 2, or 3 CN, OH, F, Cl, Br, I, or C₁₋₆ alkyl groups;
L₂ is selected from a single bond, O, S, NH, C₁₋₆ alkyl, C₁₋₆ heteroalkyl, 3- to 6-membered heterocyclyl, C₃₋₆ cycloalkyl, and phenyl-O-C₁₋₆ alkyl-, and the C₁₋₆ alkyl, C₁₋₆ heteroalkyl, 3-to 6-membered heterocyclyl, C₃₋₆ cycloalkyl, or phenyl-O-C₁₋₆ alkyl- is optionally substituted by 1, 2, or 3 CN, OH, F, Cl, Br, I, or C₁₋₆ alkyl groups;
L₃ is selected from a single bond, O, NH, -C(=O)-, -C(=O)NH-, C₁₋₆ alkyl, 3- to 6-membered heterocyclyl, C₃₋₆ cycloalkyl, phenyl, and 5- to 6-membered heteroaryl, and the C₁₋₆ alkyl, 3- to 6-membered heterocyclyl, C₃₋₆ cycloalkyl, phenyl, or 5- to 6-membered heteroaryl is optionally substituted by 1, 2, or 3 CN, CF₃, OH, F, Cl, Br, I, or C₁₋₆ alkyl groups;
L₄ is selected from H, F, Cl, Br, I, OH, CN, NH₂, COOH, 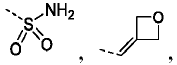 C(=O)C₁₋₆ alkyl, C(=O)NHC₁₋₆ alkyl, C(=O)N(C₁₋₆ alkyl)₂, C₁₋₆ alkyl, C₁₋₆ heteroalkyl, C₃₋₆ cycloalkyl, 4-to 6-membered heterocyclyl, C₁₋₆ alkyl-5- to 6-membered heterocyclyl, 5- to 6-membered heteroaryl, and benzo 4- to 6-membered heterocyclyl, and the C(=O)C₁₋₆ alkyl, C(=O)NHC₁₋₆ alkyl, C(=O)N(C₁₋₆ alkyl)₂, C₁₋₆ alkyl, C₁₋₆ heteroalkyl, C₃₋₆ cycloalkyl, 4- to 6-membered heterocyclyl, C₁₋₆ alkyl-5- to 6-membered heterocyclyl, 5- to 6- membered heteroaryl, or benzo 5- to 6-membered heterocyclyl is optionally substituted by 1, 2, 3, 4, or 5 R_{L};
R_{L} is selected from CN, OH, F, Cl, Br, I, NH₂, C(=O)C₁₋₆ alkyl, C(=O)NHC₁₋₆ alkyl, C(=O)N(C₁₋₆ alkyl)₂, C₁₋₆ alkyl, and C₁₋₆ heteroalkyl, and the C(=O)C₁₋₆ alkyl, C(=O)NHC₁₋₆ alkyl, C(=O)N(C₁₋₆ alkyl)₂, C₁₋₆ alkyl, or C₁₋₆ heteroalkyl is optionally substituted by 1, 2, or 3 CN, OH, NH₂, F, Cl, Br, I, or C₁₋₆ alkyl groups;
the C₁₋₆ heteroalkyl, 3- to 6-membered heterocyclyl, 4- to 6-membered heterocyclyl, 5- to 6-membered heterocyclyl, or 5- to 6-membered heteroaryl comprises 1, 2, 3, or 4 heteroatoms or heteroatom groups independently selected from -O-, -NH-, -N=, -S-, -C(=O)-, -C(=O)O-, - S(=O)-, -S(=O)₂-, and N.

2. A compound of formula (II), an optical isomer thereof, or a tautomer thereof, 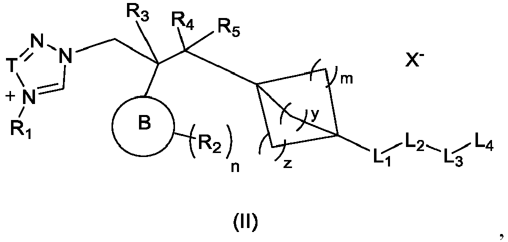
wherein X⁻ is a pharmaceutically acceptable anion;
T is selected from CH or N;
R₁ is selected from 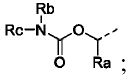
each Ra is independently selected from H and C₁₋₆ alkyl;
each Rb is independently selected from H, C₁₋₆ alkyl, -C(=O)C₁₋₆ alkyl, -OC(=O)C₁₋₆ alkyl, -C(=O)OC₁₋₆ alkyl, and phenyl, and the C₁₋₆ alkyl, -C(=O)C₁₋₆ alkyl, -OC(=O)C₁₋₆ alkyl, - C(=O)OC₁₋₆ alkyl, and phenyl are optionally substituted by 1, 2, or 3 CN, OH, F, Cl, Br, I, or C₁₋₆ alkyl groups;
each Rc is independently selected from H, C₁₋₆ alkyl, phenyl, or 5- to 6-membered heteroaryl, and the phenyl or 5- to 6-membered heteroaryl is optionally substituted by 1, 2, or 3 R;
each R is independently selected from CN, OH, F, Cl, Br, I, C₁₋₆ alkyl, C₁₋₆ heteroalkyl, 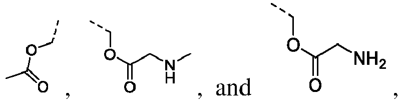 and the C₁₋₆ alkyl or C₁₋₆ heteroalkyl is optionally substituted by 1, 2, or 3 CN, OH, F, Cl, Br, I, or C₁₋₆ alkyl groups;
alternatively, Rb and Rc are linked together to form a 5- to 6-membered heterocyclyl ring, and the 5- to 6-membered heterocyclyl ring is optionally substituted by 1, 2, or 3 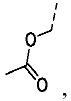 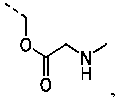 CN, OH, F, Cl, Br, I, or C₁₋₆ alkyl groups;
ring B is selected from phenyl and 5- to 6-membered heteroaryl;
R₃ is selected from OH, NH₂, halogen, C₁₋₆ alkyl, C₁₋₆ heteroalkyl, -OC(=O)C₁₋₆ alkyl, - NHC(=O)C₁₋₆ alkyl, 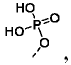 and -OC₁₋₆ alkyl-OP(=O)₂(OH)₂;
R₂, R₄, and R₅ are each independently selected from H, CN, OH, F, Cl, Br, I, C₁₋₆ alkyl, and C₁₋₆ heteroalkyl, and the C₁₋₆ alkyl or C₁₋₆ heteroalkyl is optionally substituted by 1, 2, or 3 CN, OH, F, Cl, Br, I, or C₁₋₆ alkyl groups;
m, y, and z are each independently selected from 1, 2, 3, or 4;
n is selected from 0, 1, 2, or 3;
L₁ is selected from a single bond, -NH-, C₁₋₆ alkyl, C₂₋₆ alkynyl, phenyl, and 5- to 6-membered heteroaryl, and the C₁₋₆ alkyl, C₂₋₆ alkynyl, phenyl, or 5- to 6-membered heteroaryl is optionally substituted by 1, 2, or 3 CN, OH, F, Cl, Br, I, or C₁₋₆ alkyl groups;
L₂ is selected from a single bond, O, S, NH, C₁₋₆ alkyl, C₁₋₆ heteroalkyl, 3- to 6-membered heterocyclyl, C₃₋₆ cycloalkyl, and phenyl-O-C₁₋₆ alkyl-, and the C₁₋₆ alkyl, C₁₋₆ heteroalkyl, 3-to 6-membered heterocyclyl, C₃₋₆ cycloalkyl, or phenyl-O-C₁₋₆ alkyl- is optionally substituted by 1, 2, or 3 CN, OH, F, Cl, Br, I, or C₁₋₆ alkyl groups;
L₃ is selected from a single bond, O, NH, -C(=O)-, -C(=O)NH-, C₁₋₆ alkyl, 3- to 6-membered heterocyclyl, C₃₋₆ cycloalkyl, phenyl, and 5- to 6-membered heteroaryl, and the C₁₋₆ alkyl, 3- to 6-membered heterocyclyl, C₃₋₆ cycloalkyl, phenyl, or 5- to 6-membered heteroaryl is optionally substituted by 1, 2, or 3 CN, CF₃, OH, F, Cl, Br, I, or C₁₋₆ alkyl groups;
L₄ is selected from H, F, Cl, Br, I, OH, CN, NH₂, COOH, 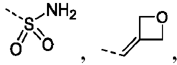 C(=O)C₁₋₆ alkyl, C(=O)NHC₁₋₆ alkyl, C(=O)N(C₁₋₆ alkyl)₂, C₁₋₆ alkyl, C₁₋₆ heteroalkyl, C₃₋₆ cycloalkyl, 4-to 6-membered heterocyclyl, C₁₋₆ alkyl-5- to 6-membered heterocyclyl, 5- to 6- membered heteroaryl, and benzo 5- to 6-membered heterocyclyl, and the C(=O)C₁₋₆ alkyl, C(=O)NHC₁₋₆ alkyl, C(=O)N(C₁₋₆ alkyl)₂, C₁₋₆ alkyl, C₁₋₆ heteroalkyl, C₃₋₆ cycloalkyl, 4- to 6-membered heterocyclyl, C₁₋₆ alkyl-5- to 6-membered heterocyclyl, 5- to 6- membered heteroaryl, or benzo 5- to 6-membered heterocyclyl is optionally substituted by 1, 2, 3, 4, or 5 R_{L};
R_{L} is selected from CN, OH, F, Cl, Br, I, NH₂, 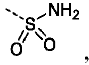 C(=O)C₁₋₆ alkyl, C(=O)NHC₁₋₆ alkyl, C(=O)N(C₁₋₆ alkyl)₂, C₁₋₆ alkyl, and C₁₋₆ heteroalkyl, and the C(=O)C₁₋₆ alkyl, C(=O)NHC₁₋₆ alkyl, C(=O)N(C₁₋₆ alkyl)₂, C₁₋₆ alkyl, or C₁₋₆ heteroalkyl is optionally substituted by 1, 2, or 3 CN, OH, NH₂, F, Cl, Br, I, or C₁₋₆ alkyl groups;
the C₁₋₆ heteroalkyl, 3- to 6-membered heterocyclyl, 4- to 6-membered heterocyclyl, 5- to 6-membered heterocyclyl, or 5- to 6-membered heteroaryl comprises 1, 2, 3, or 4 heteroatoms or heteroatom groups independently selected from -O-, -NH-, -N=, -S-, -C(=O)-, -C(=O)O-, - S(=O)-, -S(=O)₂-, and N.

3. The compound, the optical isomer thereof, the tautomer thereof, or the pharmaceutically acceptable salt thereof according to claim 1, wherein ring A is selected from 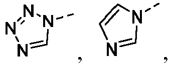 and

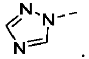

4. The compound, the optical isomer thereof, the tautomer thereof, or the pharmaceutically acceptable salt thereof according to claim 1, or the compound, the optical isomer thereof, or the tautomer thereof according to claim 2, wherein the R₃ is selected from OH, F, Cl, Br, I, NH₂, C₁₋₃ alkyl, C₁₋₃ alkoxy, C₁₋₃ alkylamino, C₁₋₃ alkylthio, 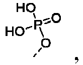 -OC₁₋₃ alkyl-OP(=O)₂(OH)₂, -OC(=O)C₁₋₃ alkyl, and -NHC(=O)C₁₋₃ alkyl; optionally, the R₃ is selected from OH, F, Cl, Br, NH₂, OCH₃,

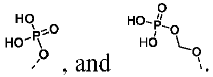

5. The compound, the optical isomer thereof, or the tautomer thereof according to claim 2, wherein Rb is selected from H, C₁₋₃ alkyl, -C(=O)C₁₋₃ alkyl, -OC(=O)C₁₋₃ alkyl, -C(=O)OC₁₋₃ alkyl, and phenyl, and the C₁₋₃ alkyl, -C(=O)C₁₋₃ alkyl, -OC(=O)C₁₋₃ alkyl, -C(=O)OC₁₋₃ alkyl, and phenyl are optionally substituted by 1, 2, or 3 CN, OH, F, Cl, Br, I, or C₁₋₃ alkyl groups; optionally, Rb is selected from H, CH₃,

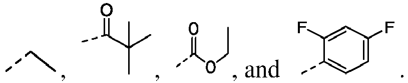

6. The compound, the optical isomer thereof, or the tautomer thereof according to claim 2, wherein Rc is selected from H, -C₁₋₆ alkyl-OC(=O)C₁₋₆ alkyl-NH-C₁₋₆ alkyl, -C₁₋₆ alkyl-NH-C₁₋₆ alkyl, -C₁₋₆ alkyl-OC(=O) -C₁₋₆ alkyl, -phenyl-C₁₋₆ alkyl-OC(=O)-C₁₋₆ alkyl, -phenyl-C₁₋₆ alkyl-OC(=O)-C₁₋₆ alkyl-NH-C₁₋₆ alkyl, -5- to 6-membered heteroaryl-C₁₋₆ alkyl-NH-C₁₋₆ alkyl, -5- to 6-membered heteroaryl-C₁₋₆ alkyl-OC(=O)-C₁₋₆ alkyl, -5- to 6-membered heteroaryl-C₁₋₆ alkyl-OC(=O)-C₁₋₆ alkyl-NH-C₁₋₆ alkyl, and -5- to 6-membered heteroaryl-C₁₋₆ alkyl-NH-C₁₋₆ alkyl, and the -C₁₋₆ alkyl-OC(=O)C₁₋₆ alkyl-NH-C₁₋₆ alkyl, -C₁₋₆ alkyl-NH-C₁₋₆ alkyl, -C₁₋₆ alkyl-OC(=O) -C₁₋₆ alkyl, -phenyl-C₁₋₆ alkyl-OC(=O)-C₁₋₆ alkyl, -phenyl-C₁₋₆ alkyl-OC(=O)-C₁₋₆ alkyl-NH-C₁₋₆ alkyl, -5- to 6-membered heteroaryl-C₁₋₆ alkyl-NH-C₁₋₆ alkyl, -5- to 6-membered heteroaryl-C₁₋₆ alkyl-OC(=O)-C₁₋₆ alkyl, -5- to 6-membered heteroaryl-C₁₋₆ alkyl-OC(=O)-C₁₋₆ alkyl-NH-C₁₋₆ alkyl, or -5- to 6-membered heteroaryl-C₁₋₆ alkyl-NH-C₁₋₆ alkyl is optionally substituted by 1, 2, 3, or 4 F, Cl, Br, I, methoxy groups, or CN;
optionally, Rc is selected from H, -C₁₋₃ alkyl-OC(=O)C₁₋₃ alkyl-NH-C₁₋₃ alkyl, -C₁₋₃ alkyl-NH-C₁₋₃ alkyl, -C₁₋₃ alkyl-OC(=O) -C₁₋₃ alkyl, -phenyl-C₁₋₃ alkyl-OC(=O)-C₁₋₃ alkyl, -phenyl-C₁₋₃ alkyl-OC(=O)-C₁₋₃ alkyl-NH-C₁₋₃ alkyl, -pyridyl-C₁₋₃ alkyl-NH-C₁₋₃ alkyl, -pyridyl-C₁₋₃ alkyl-OC(=O)-C₁₋₃ alkyl, -pyridyl-C₁₋₆ alkyl-OC(=O)-C₁₋₆ alkyl-NH-C₁₋₆ alkyl, and -pyridyl-C₁₋₆ alkyl-NH-C₁₋₆ alkyl, and the -C₁₋₃ alkyl-OC(=O)C₁₋₃ alkyl-NH-C₁₋₃ alkyl, -C₁₋₃ alkyl-NH-C₁₋₃ alkyl, -C₁₋₃ alkyl-OC(=O) -C₁₋₃ alkyl, -phenyl-C₁₋₃ alkyl-OC(=O)-C₁₋₃ alkyl, -phenyl-C₁₋₃ alkyl-OC(=O)-C₁₋₃ alkyl-NH-C₁₋₃ alkyl, -pyridyl-C₁₋₃ alkyl-NH-C₁₋₃ alkyl, -pyridyl-C₁₋₃ alkyl-OC(=O)-C₁₋₃ alkyl, -pyridyl-C₁₋₃ alkyl-OC(=O)-C₁₋₃ alkyl-NH-C₁₋₃ alkyl, or -pyridyl-C₁₋₃ alkyl-NH-C₁₋₃ alkyl is optionally substituted by 1, 2, 3, or 4 F, Cl, Br, I, methoxy groups, or CN;
optionally, Rc is selected from H,

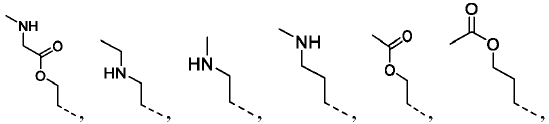

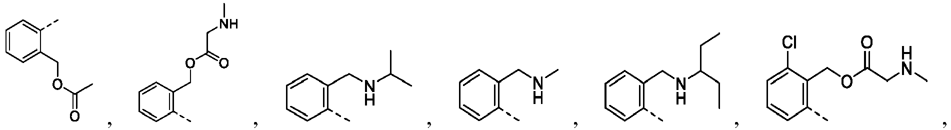

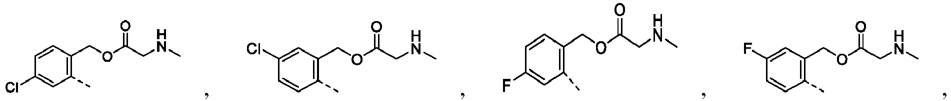

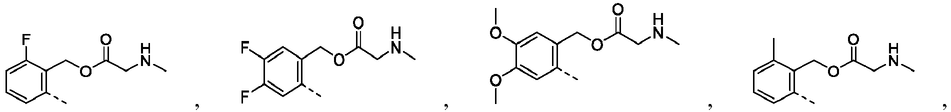

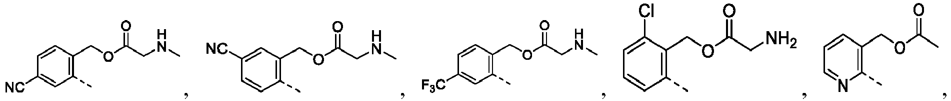

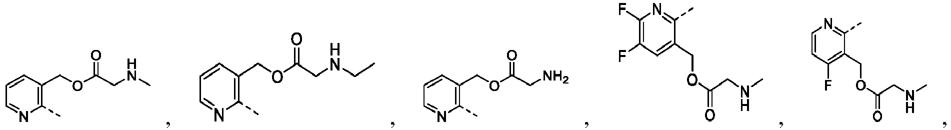

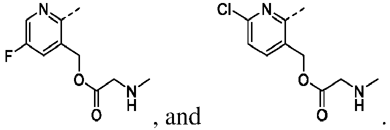

7. The compound, the optical isomer thereof, or the tautomer thereof according to claim 2, wherein R₁ is selected from

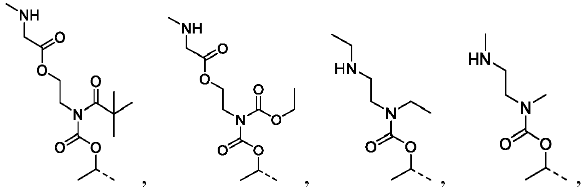

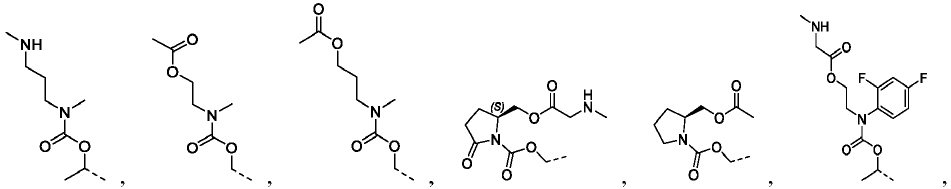

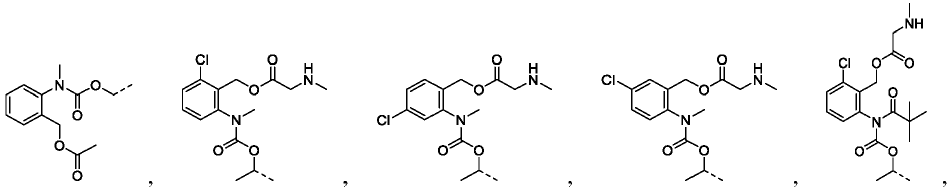

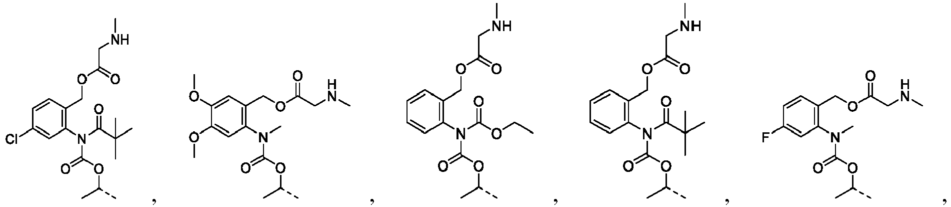

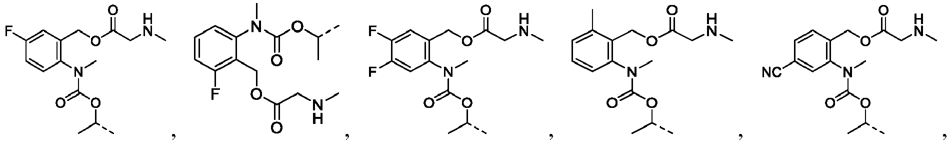

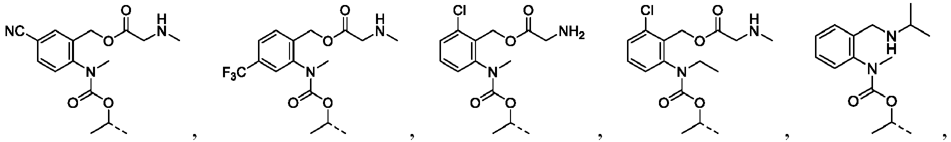

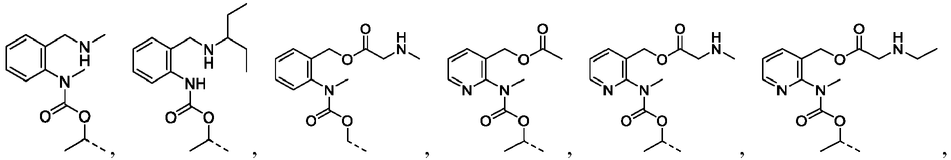

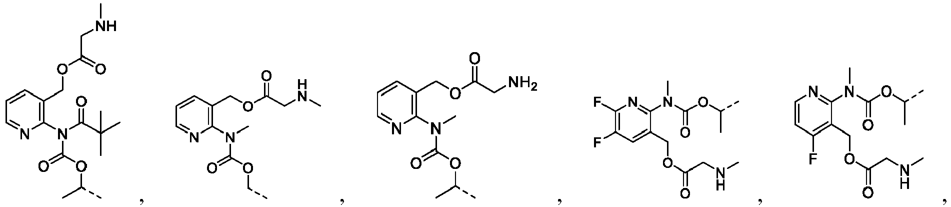

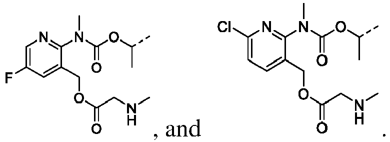

8. The compound, the optical isomer thereof, the tautomer thereof, or the pharmaceutically acceptable salt thereof according to claim 1, or the compound, the optical isomer thereof, or the tautomer thereof according to claim 2, wherein the structural moiety 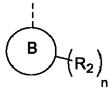 is selected from

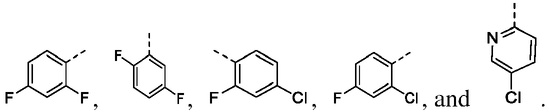

9. The compound, the optical isomer thereof, the tautomer thereof, or the pharmaceutically acceptable salt thereof according to claim 1, or the compound, the optical isomer thereof, or the tautomer thereof according to claim 2, wherein L₁ is selected from a single bond, -NH-, CH₂,

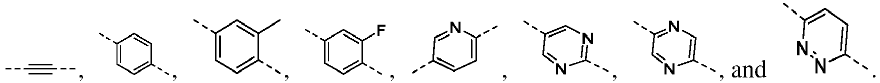

10. The compound, the optical isomer thereof, the tautomer thereof, or the pharmaceutically acceptable salt thereof according to claim 1, or the compound, the optical isomer thereof, or the tautomer thereof according to claim 2, wherein L₂ is selected from a single bond, O, S, CH₂, NH, NCH₃, 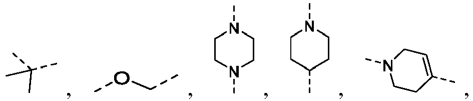 and

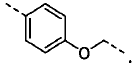

11. The compound, the optical isomer thereof, the tautomer thereof, or the pharmaceutically acceptable salt thereof according to claim 1, or the compound, the optical isomer thereof, or the tautomer thereof according to claim 2, wherein L₃ is selected from a single bond, O, NH, CH₂, CH₂CH₂, -C(=O)-, -C(=O)NH-, 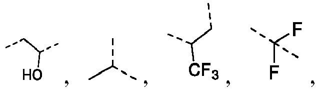 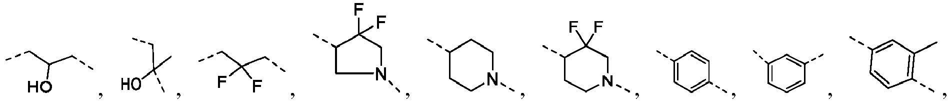 and

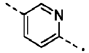

12. The compound, the optical isomer thereof, the tautomer thereof, or the pharmaceutically acceptable salt thereof according to claim 1, or the compound, the optical isomer thereof, the tautomer thereof according to claim 2, wherein L₄ is selected from H, F, Cl, Br, I, OH, CN, CH₃, CH₂CF₃, NH₂, CHF₂, CF₃, OCH₃, OCF₃, OCHF₂, OCH₂CH₃, COOH, CONHMe, CONMe₂, NMe₂, CH₂OH,

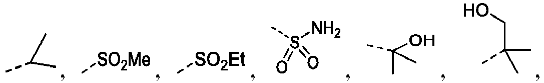

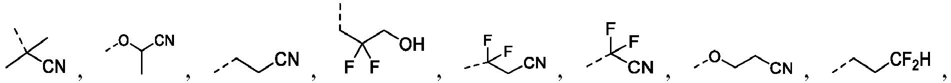

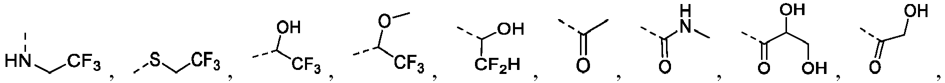

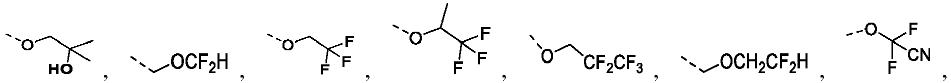

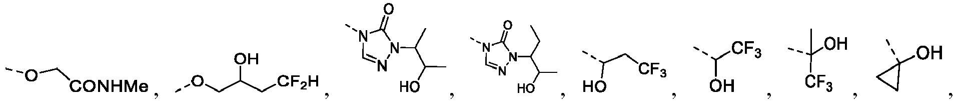

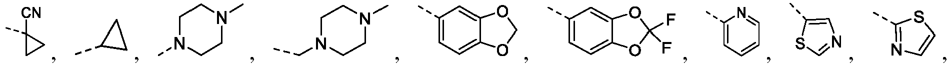

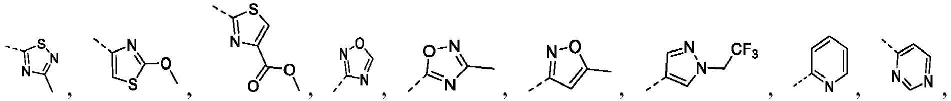

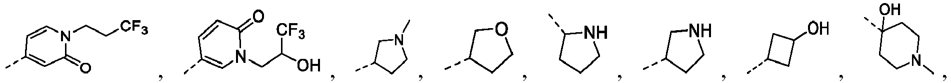

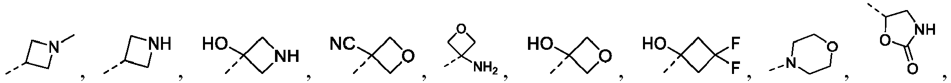

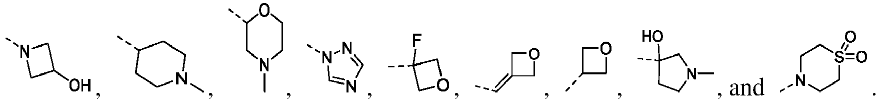

13. The compound, the optical isomer thereof, the tautomer thereof, or the pharmaceutically acceptable salt thereof according to claim 1, or the compound, the optical isomer thereof, or the tautomer thereof according to claim 2, wherein the structural moiety 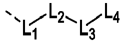 is selected from H, I, CN, OH, CH₃, NHCH₃, CF₃, C(=O)OH, C(=O)NHMe, C(=O)NMe₂,

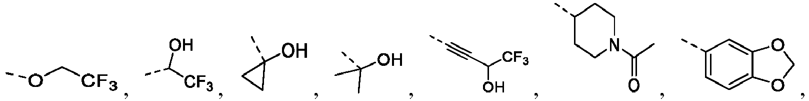

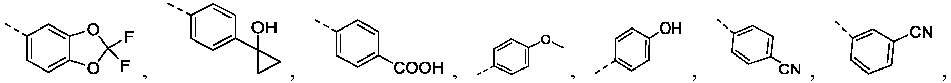

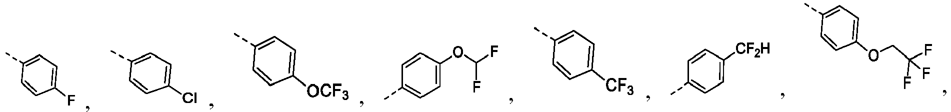

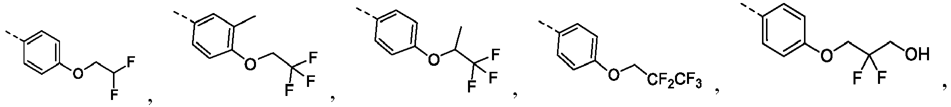

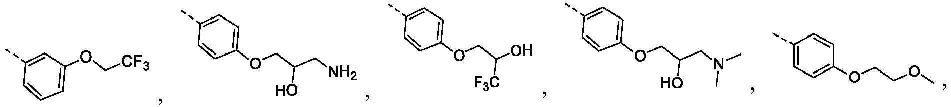

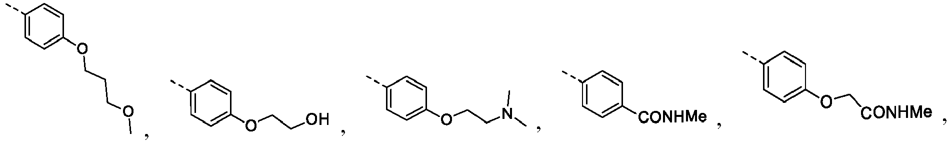

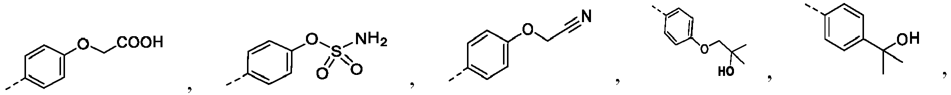

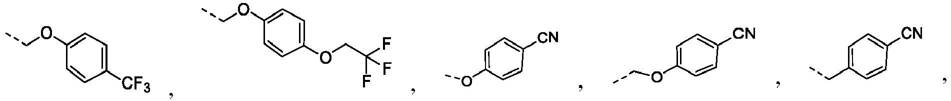

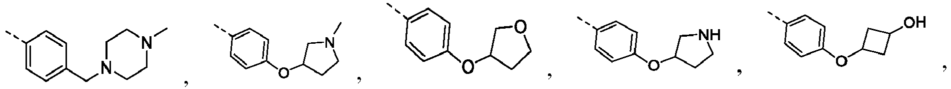

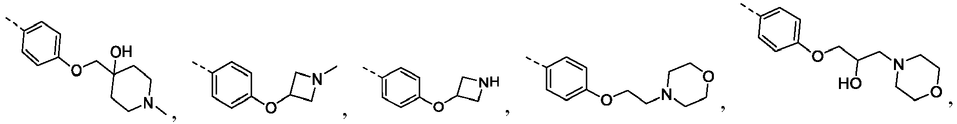

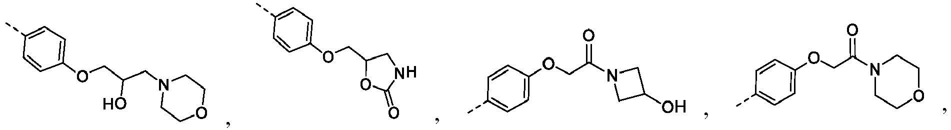

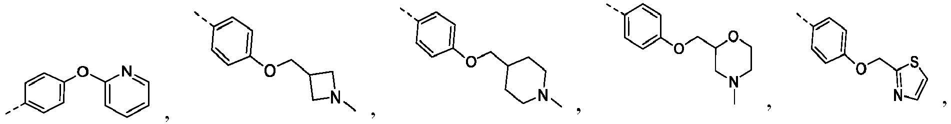

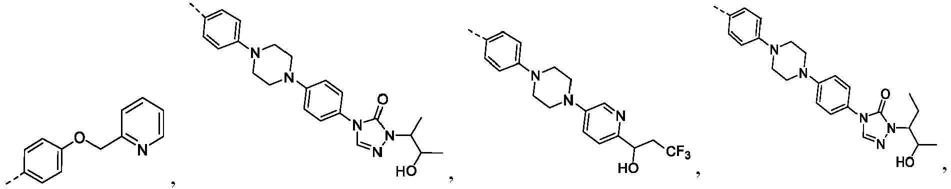

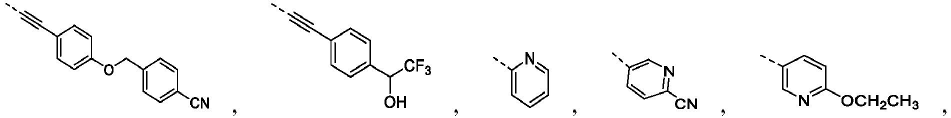

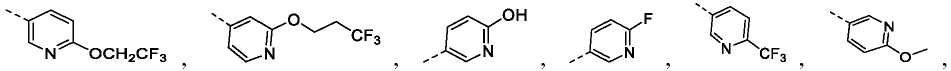

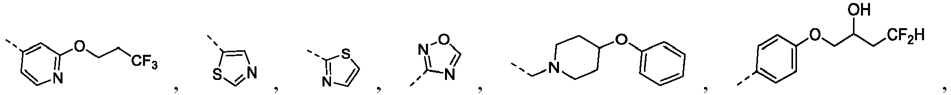

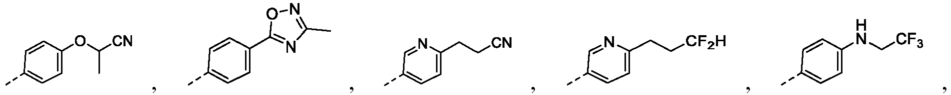

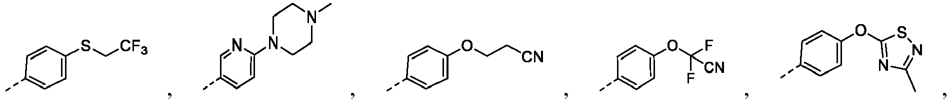

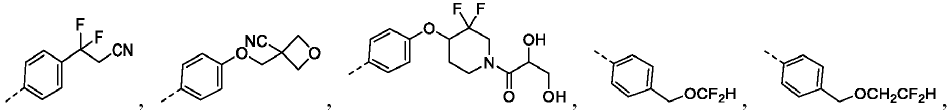

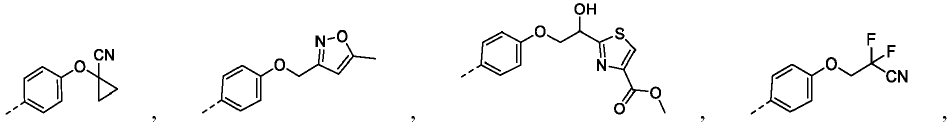

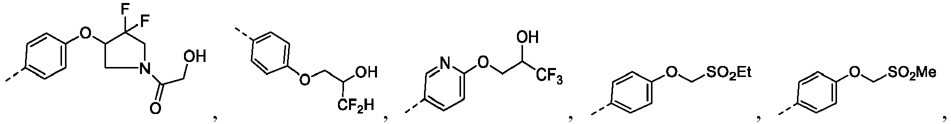

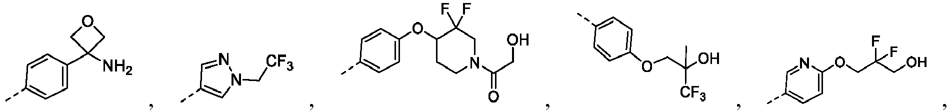

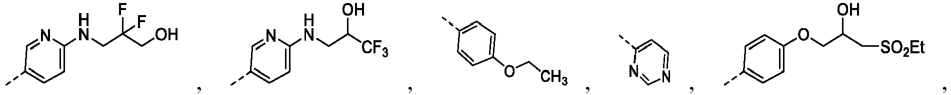

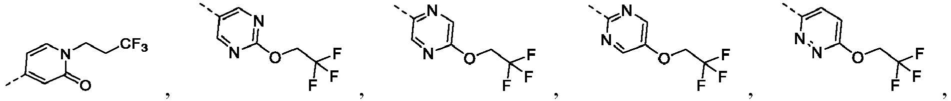

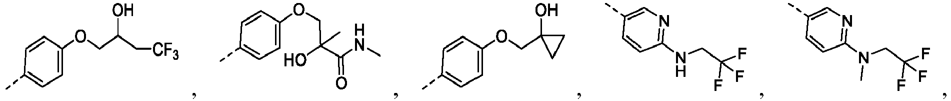

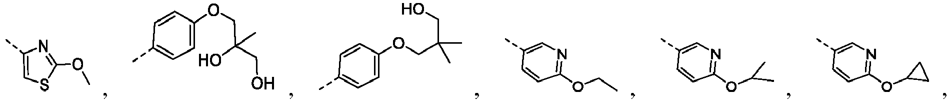

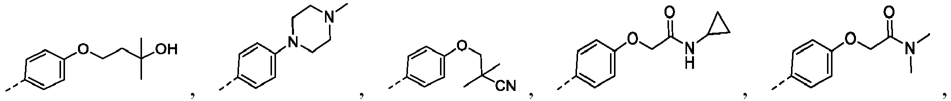

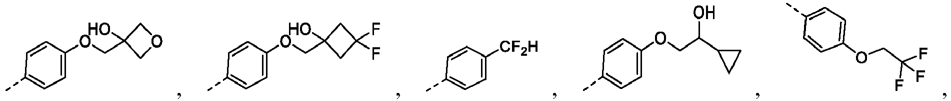

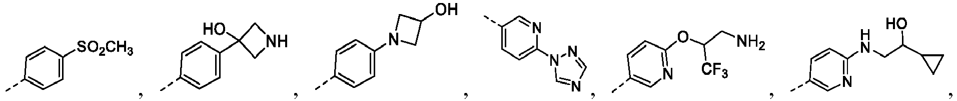

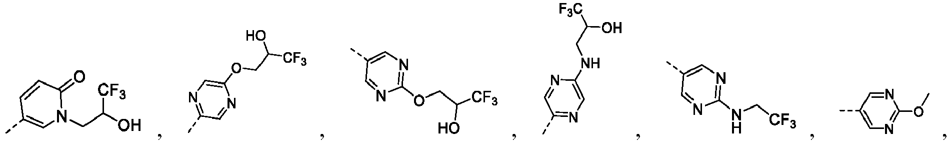

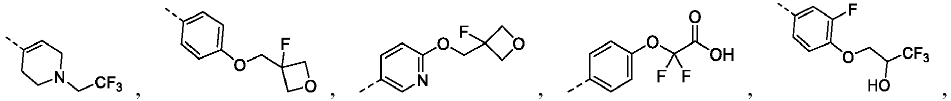

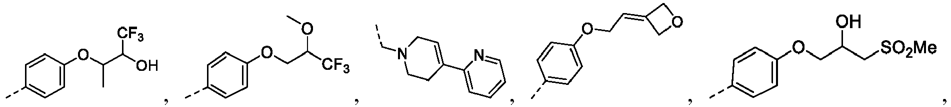

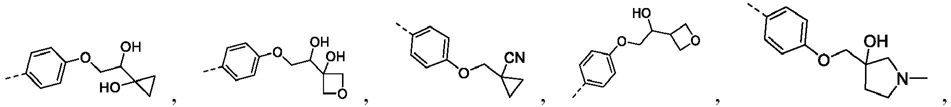

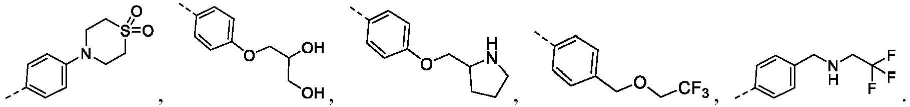

14. A compound of the following formula, an optical isomer thereof, a tautomer thereof, or a pharmaceutically acceptable salt thereof, which is selected from

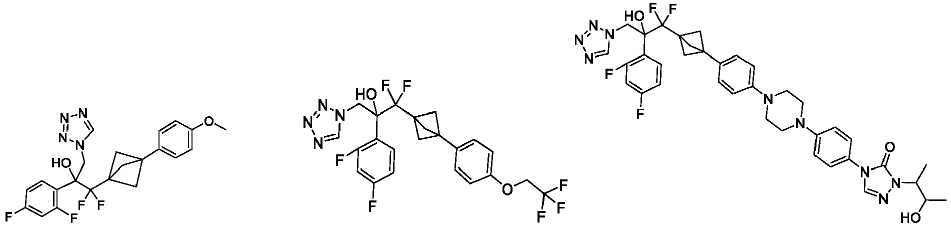

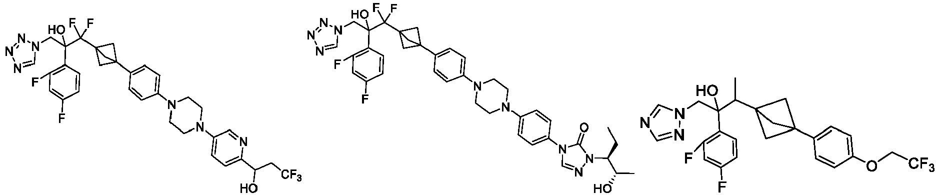

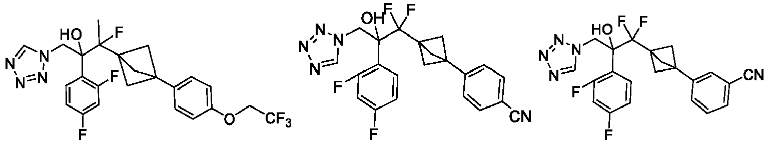

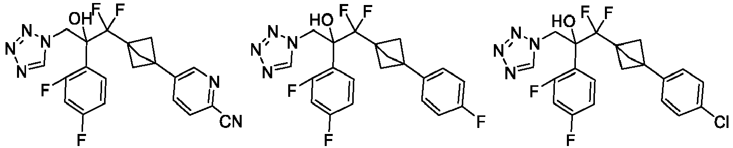

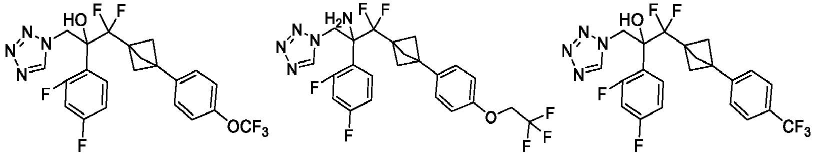

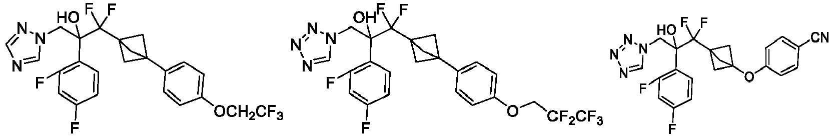

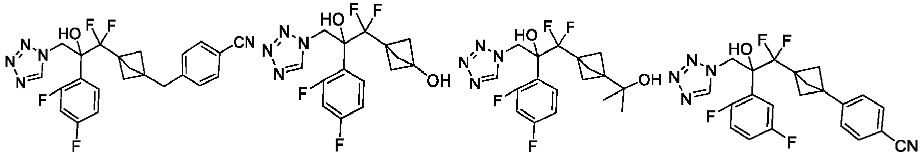

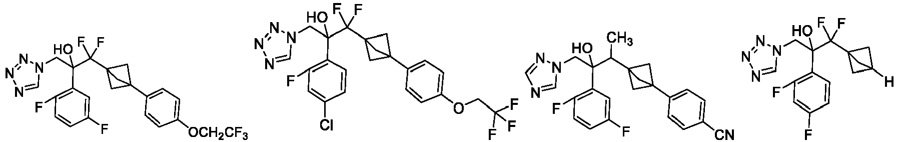

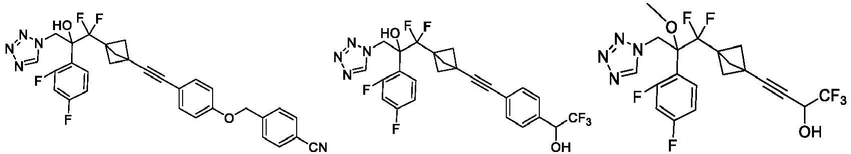

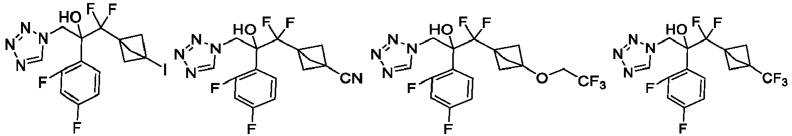

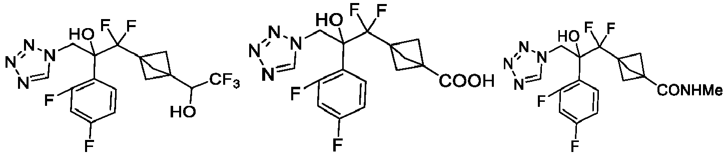

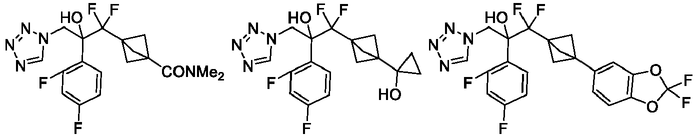

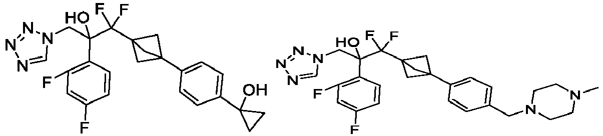

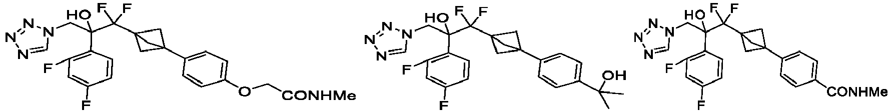

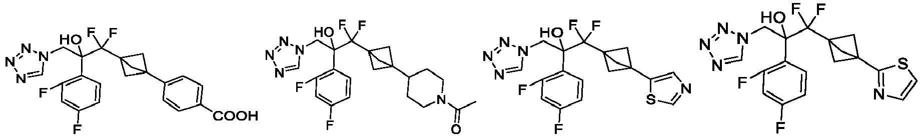

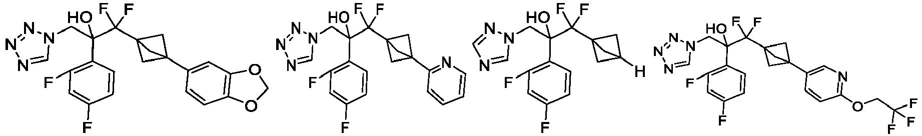

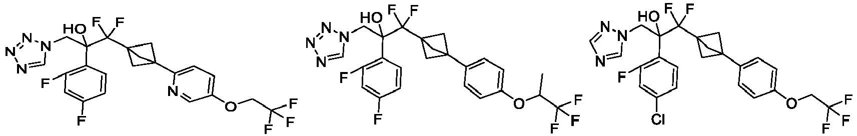

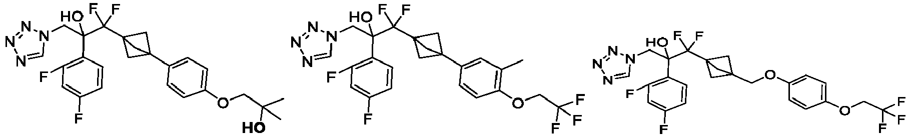

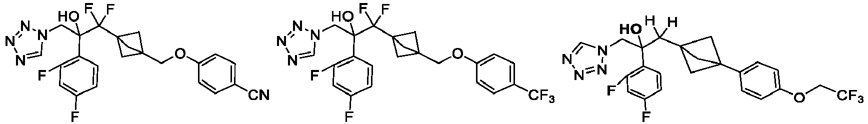

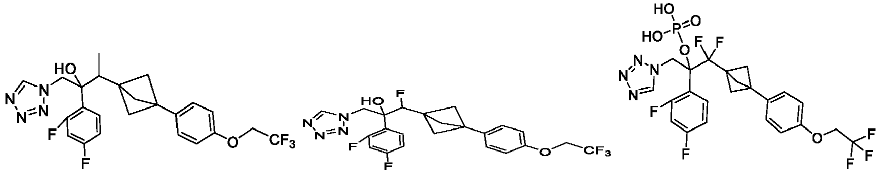

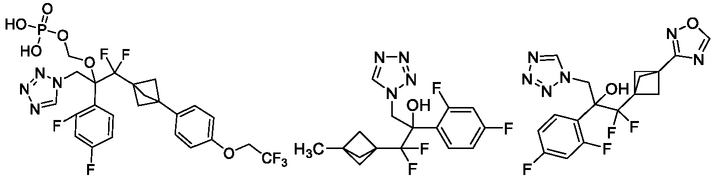

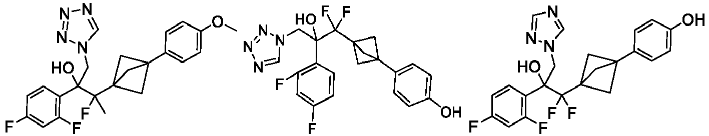

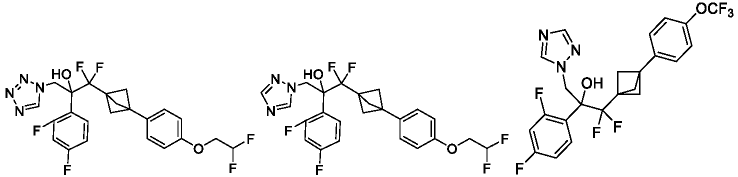

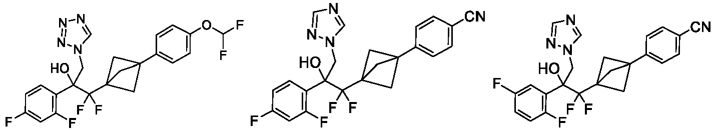

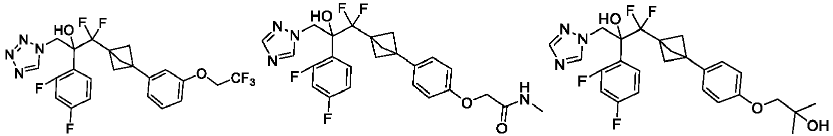

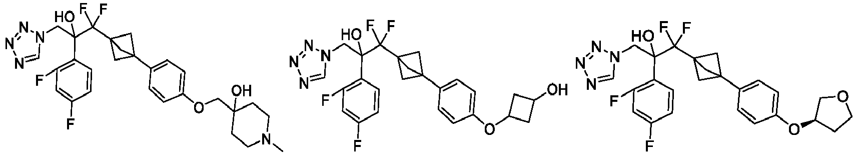

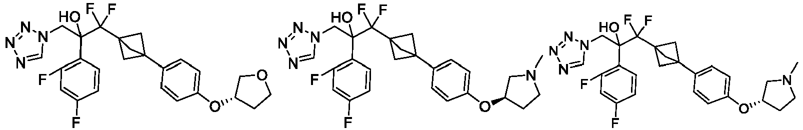

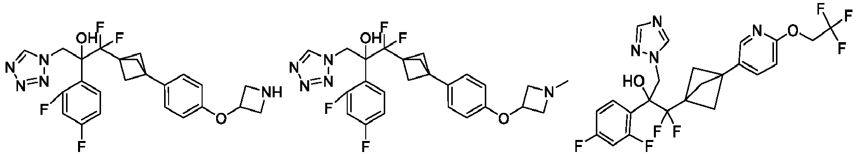

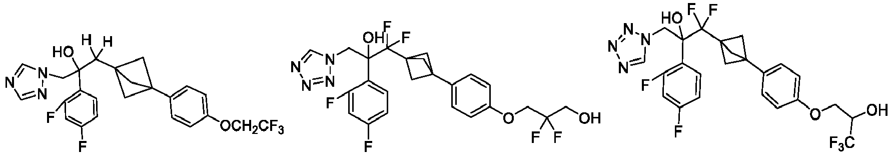

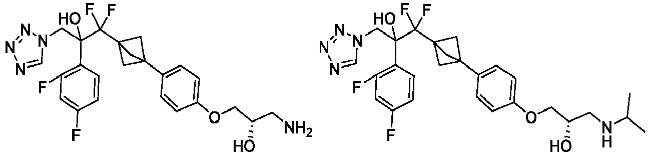

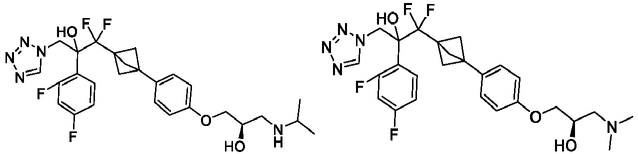

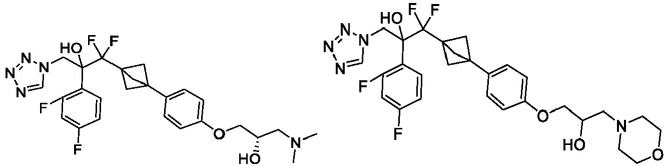

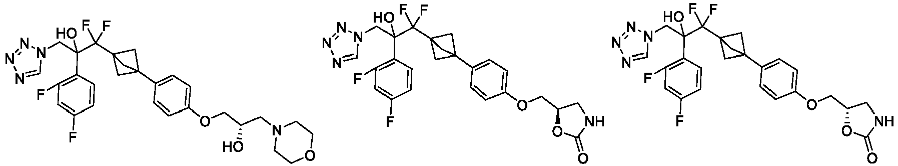

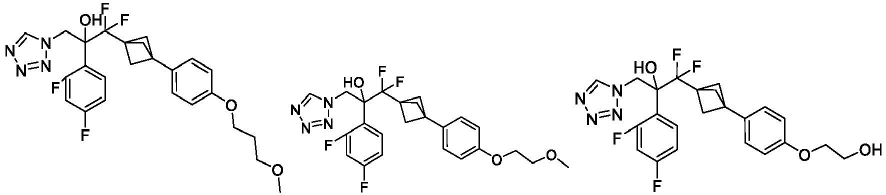

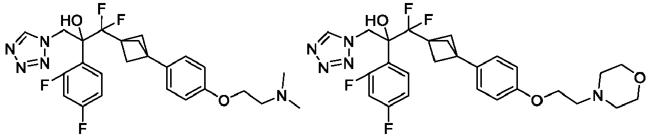

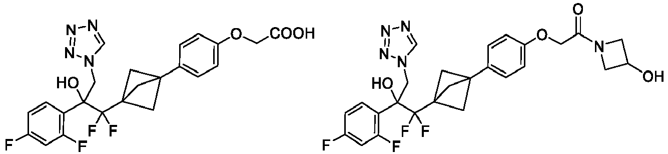

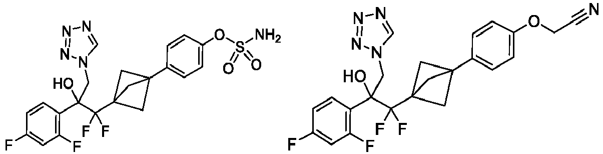

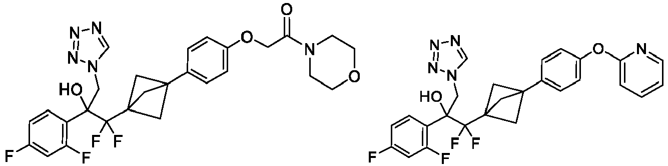

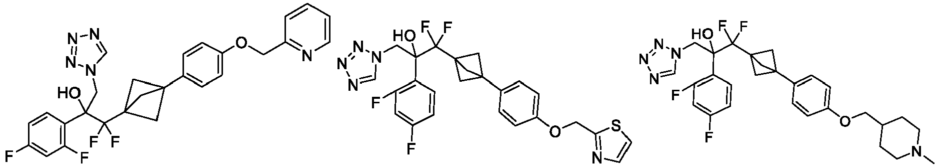

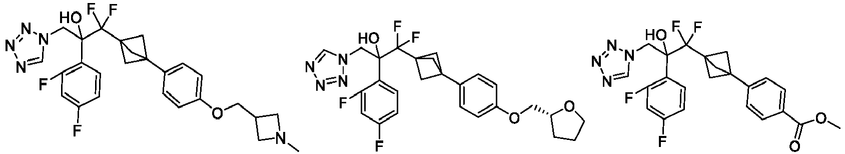

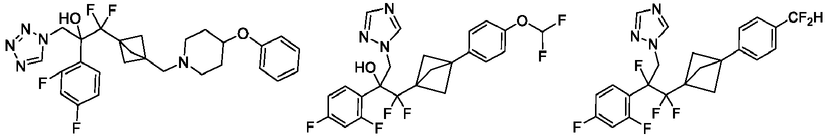

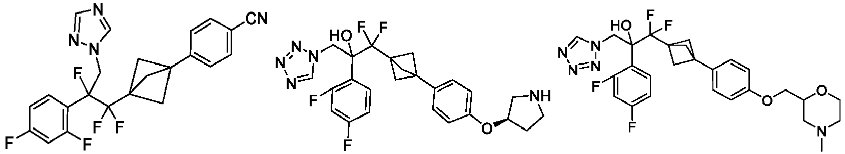

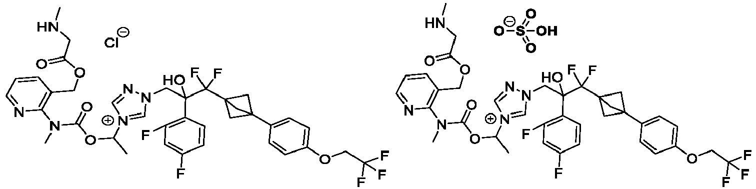

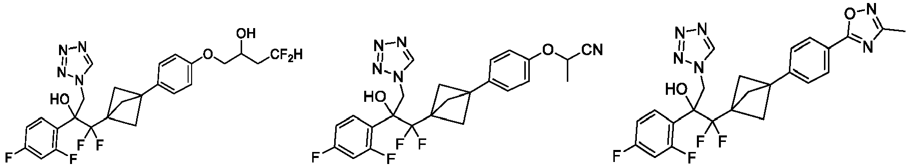

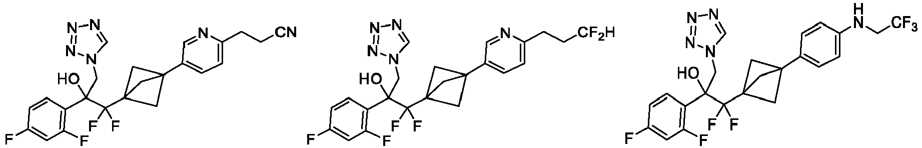

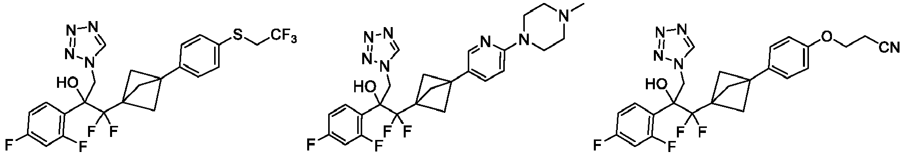

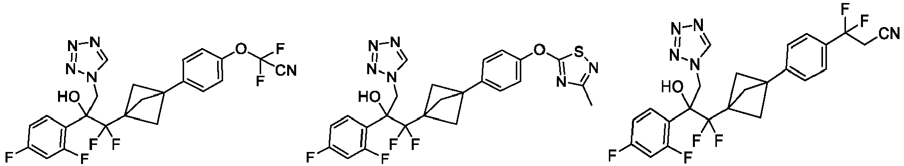

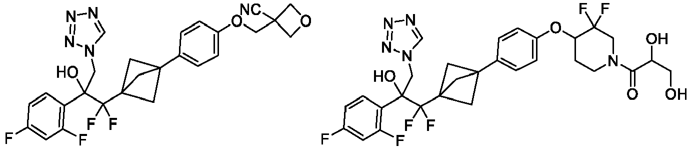

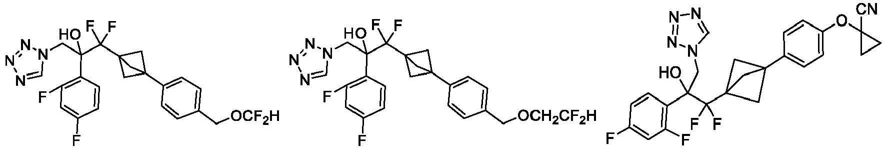

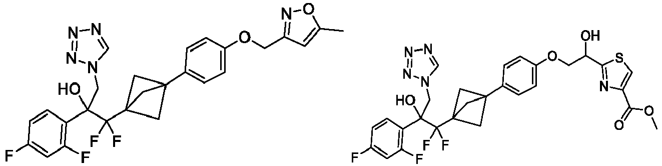

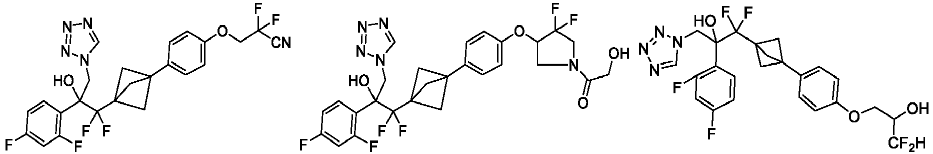

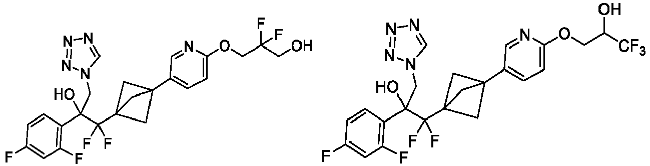

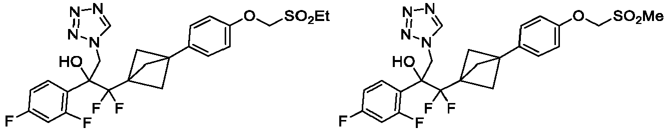

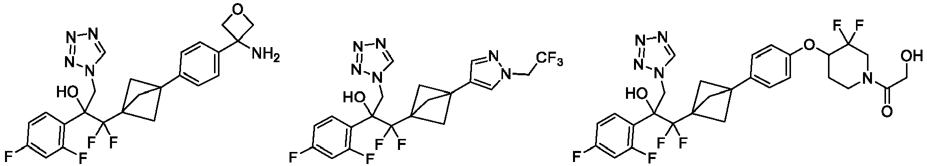

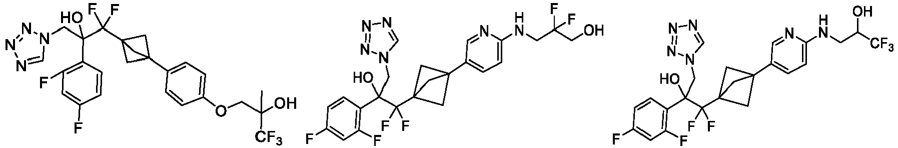

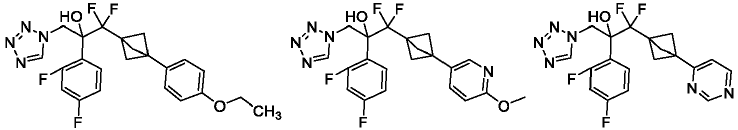

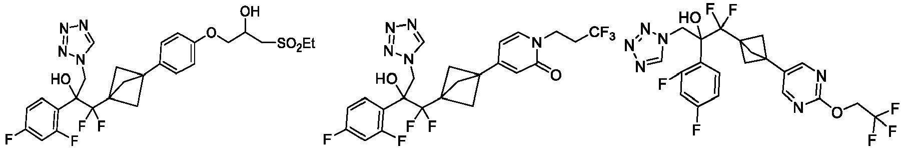

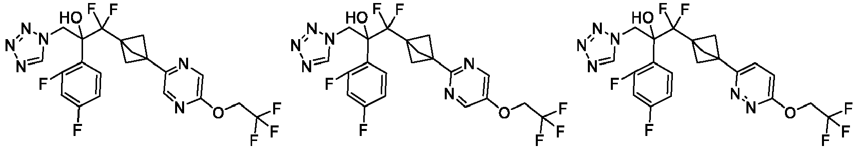

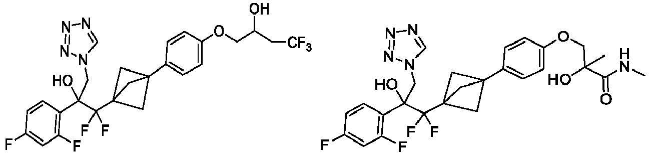

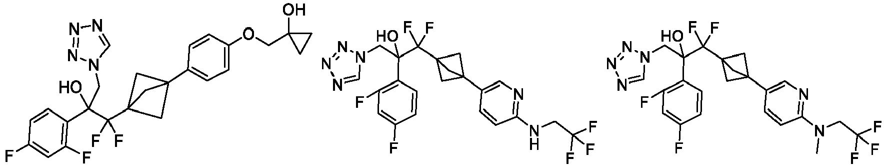

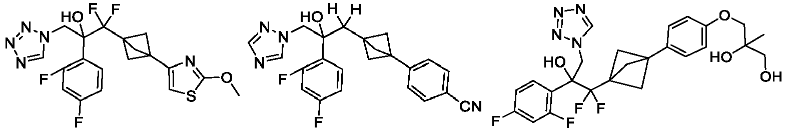

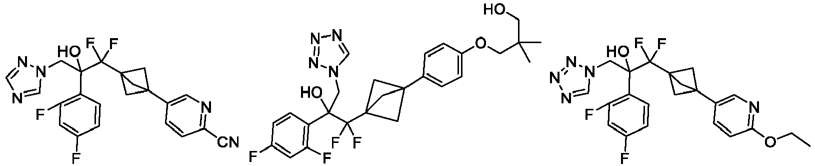

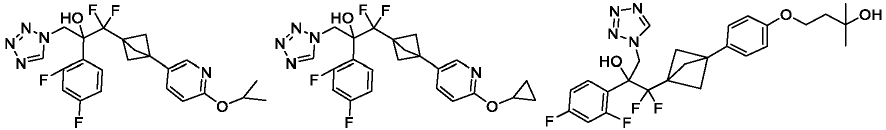

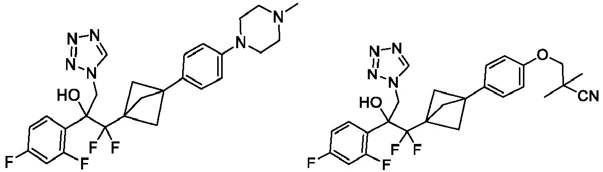

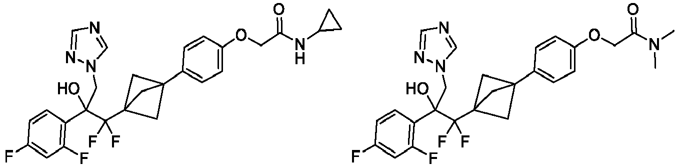

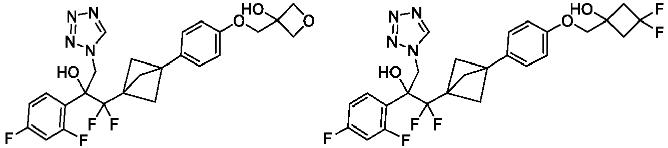

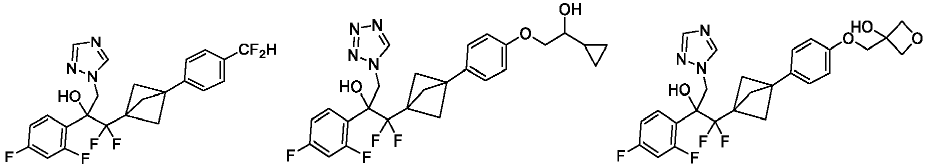

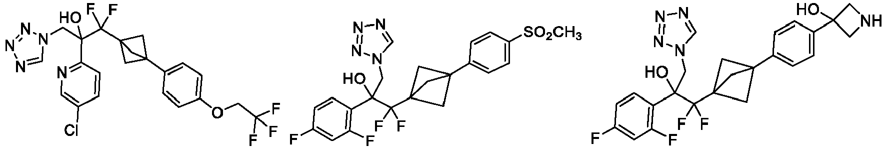

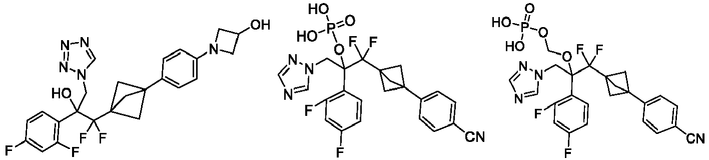

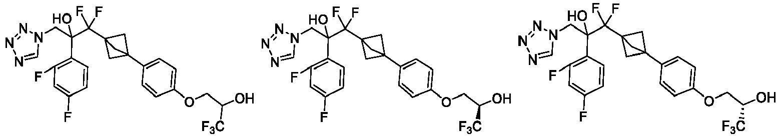

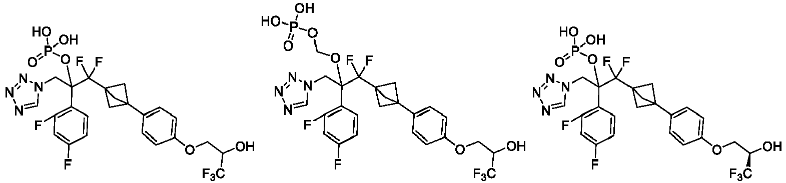

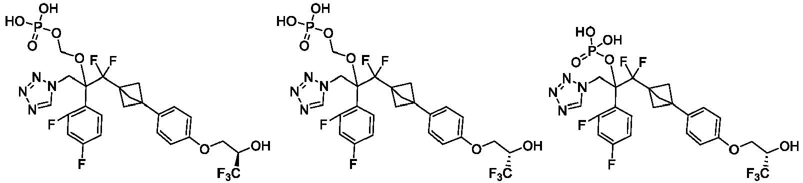

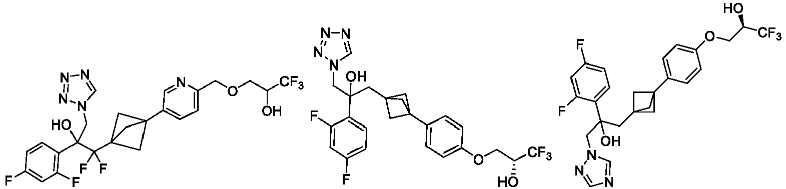

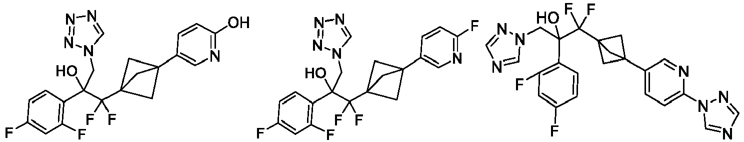

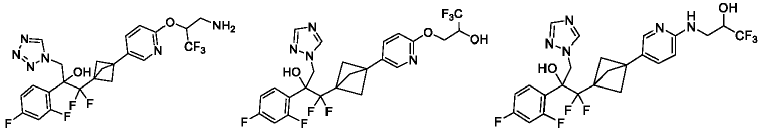

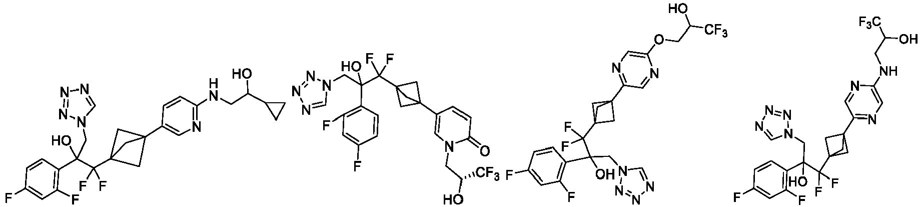

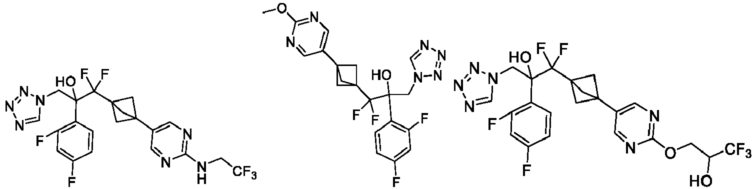

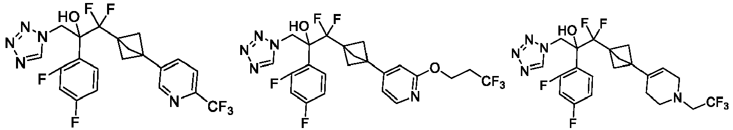

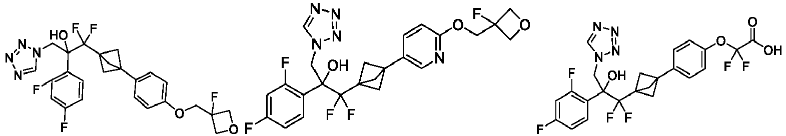

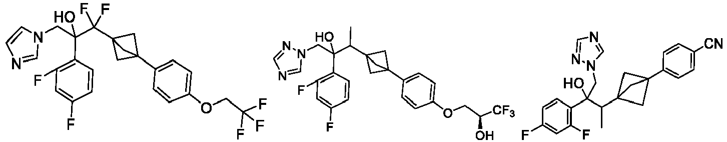

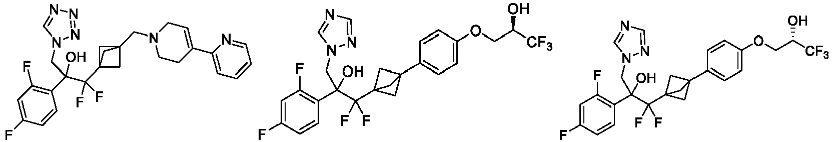

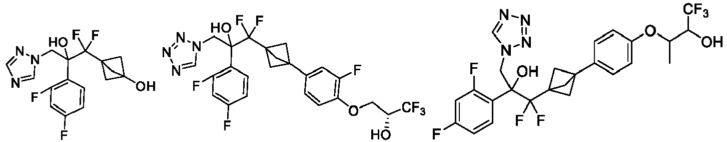

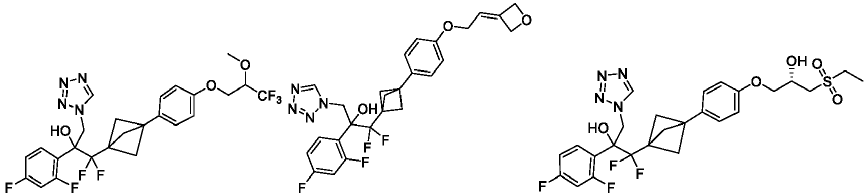

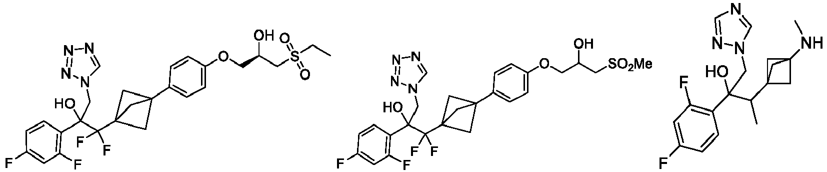

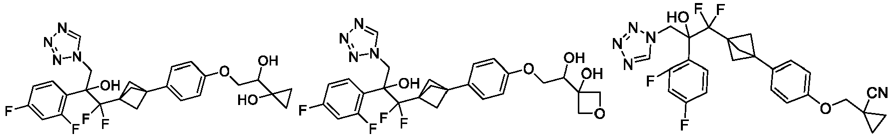

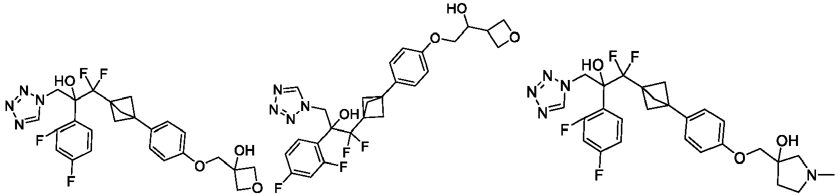

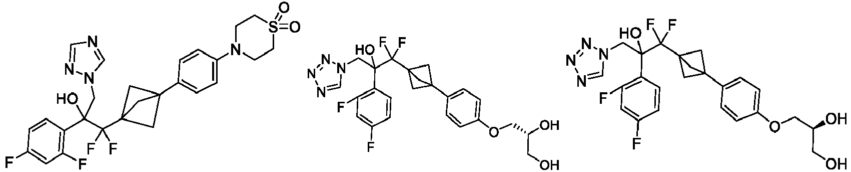

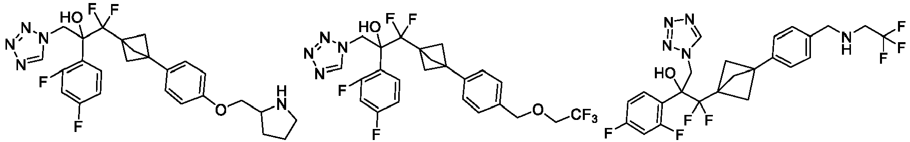

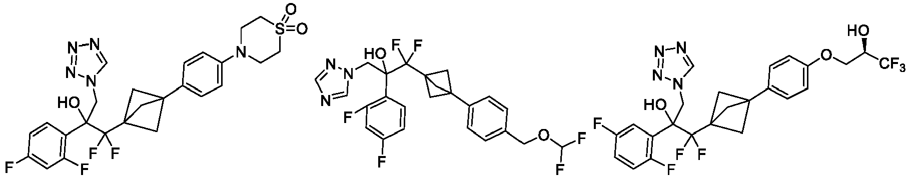

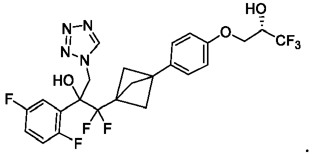

15. The compound, the optical isomer thereof, the tautomer thereof, or the pharmaceutically acceptable salt thereof according to claim 14, which is selected from

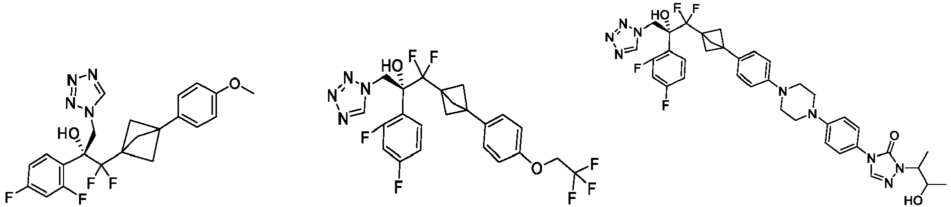

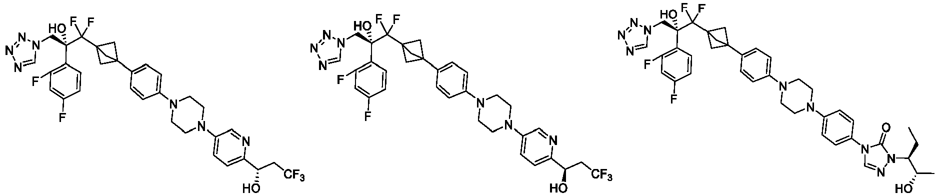

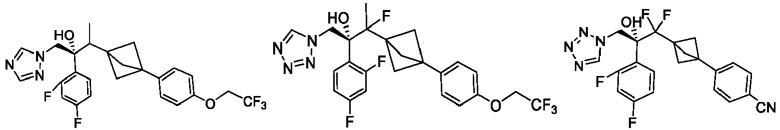

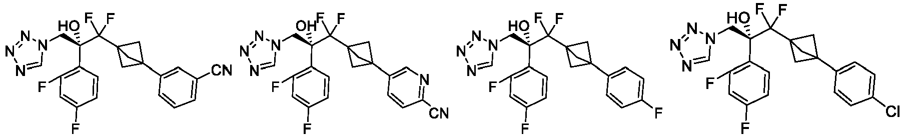

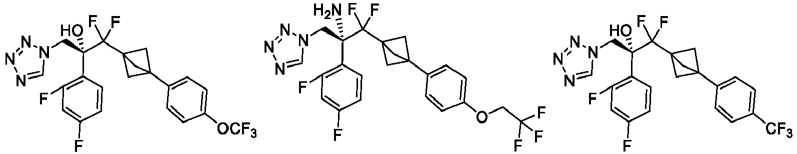

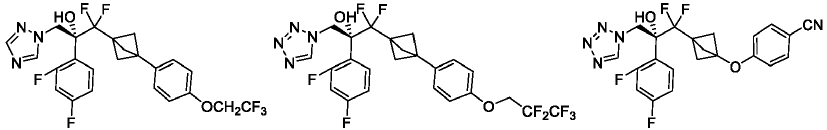

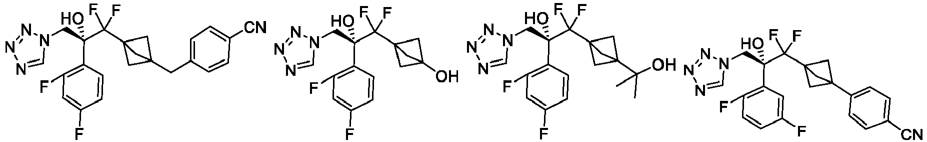

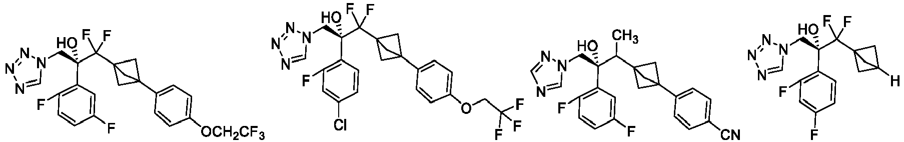

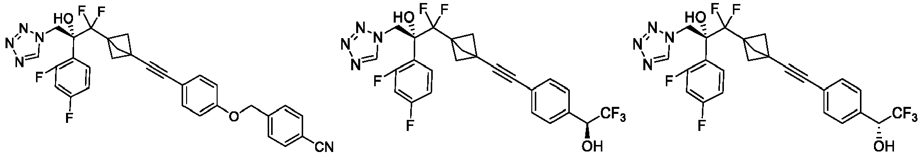

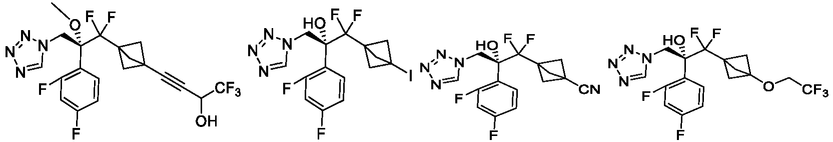

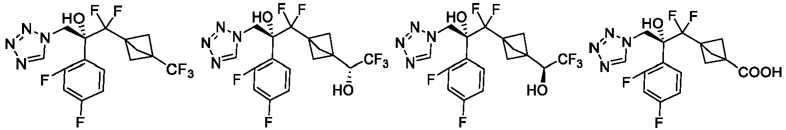

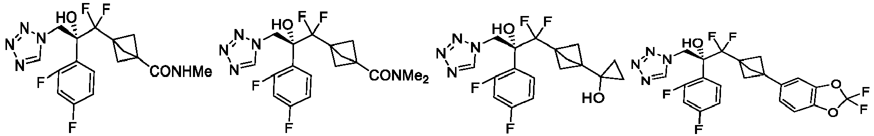

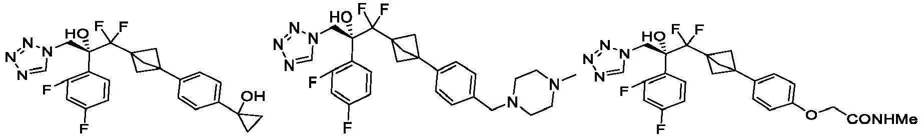

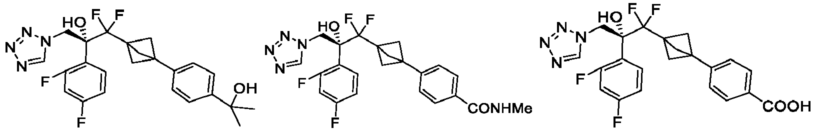

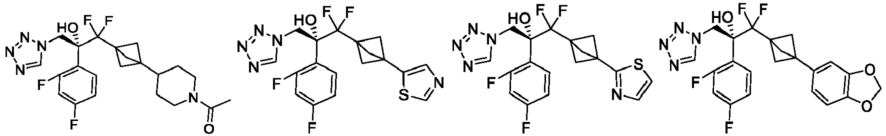

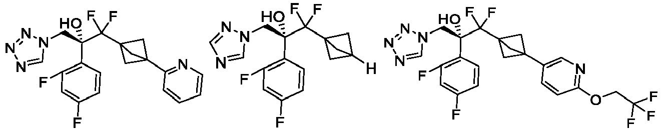

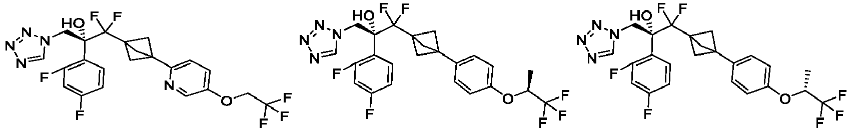

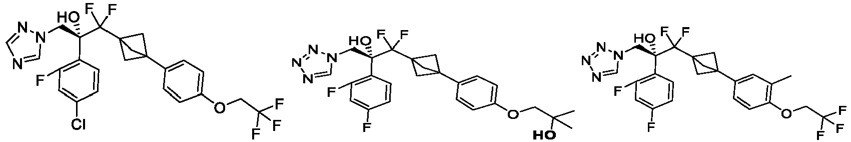

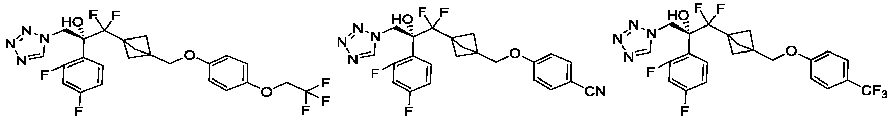

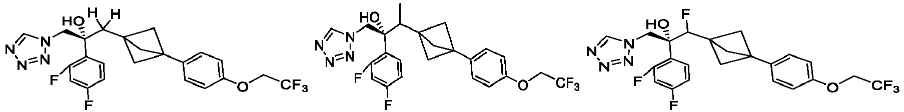

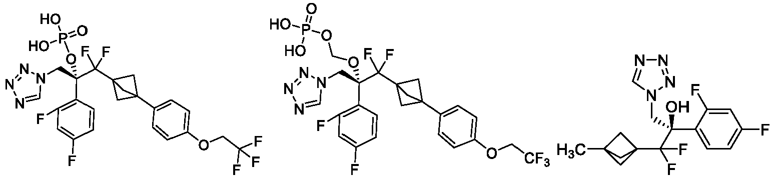

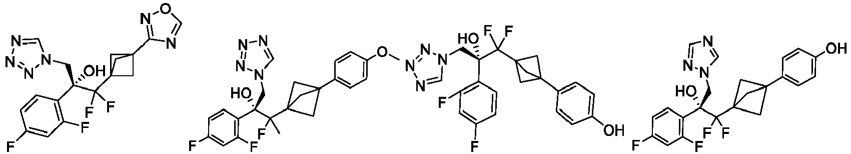

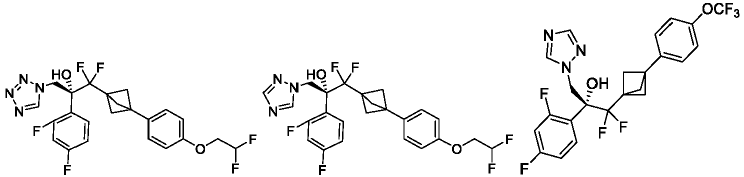

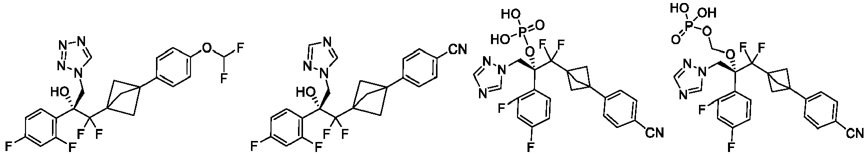

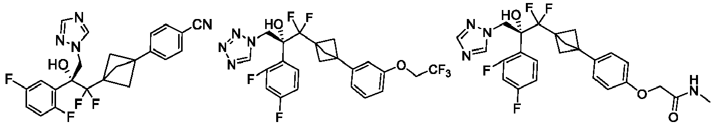

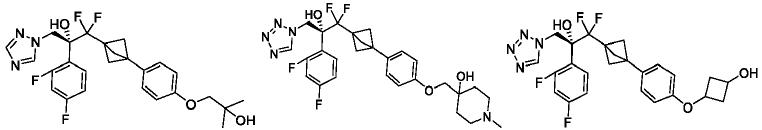

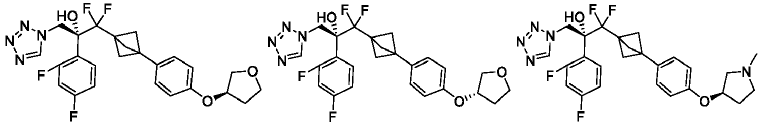

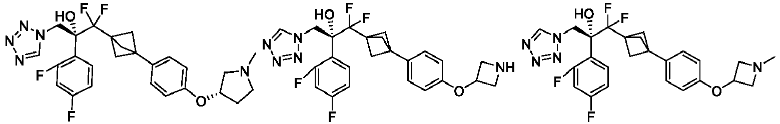

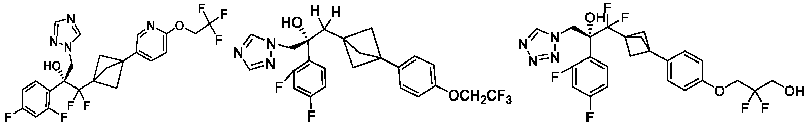

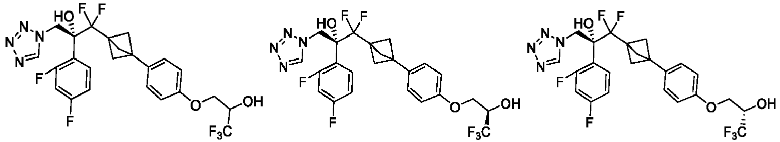

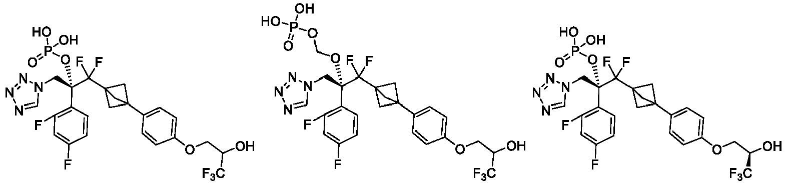

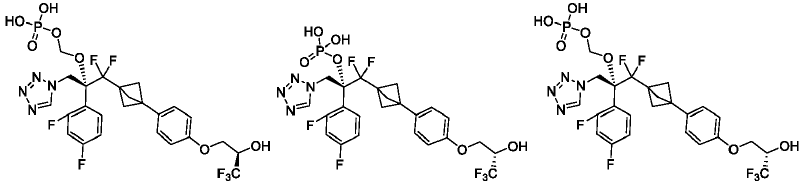

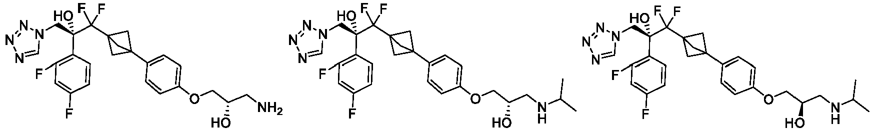

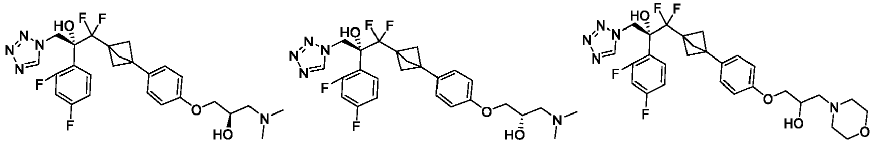

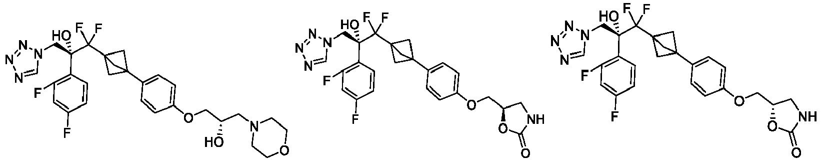

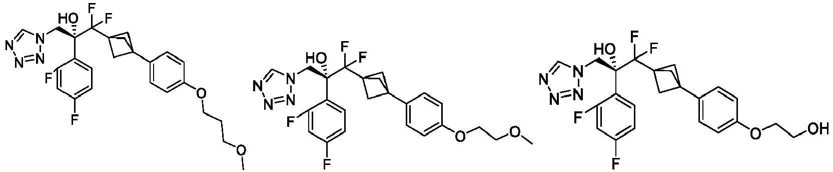

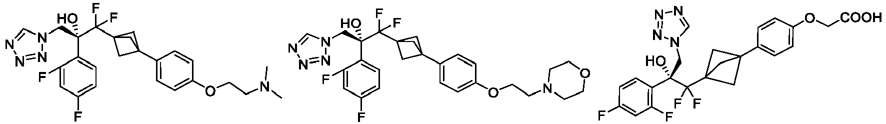

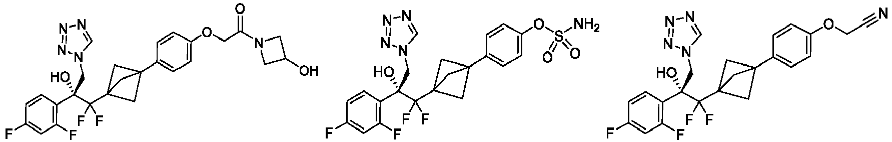

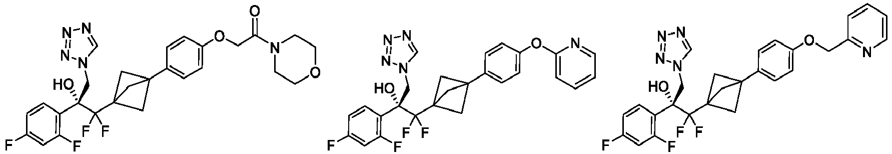

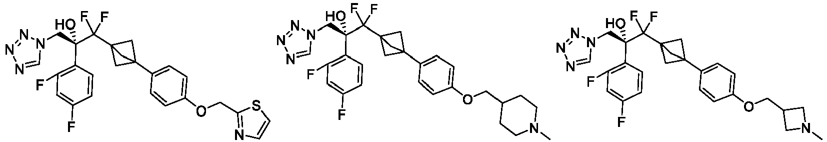

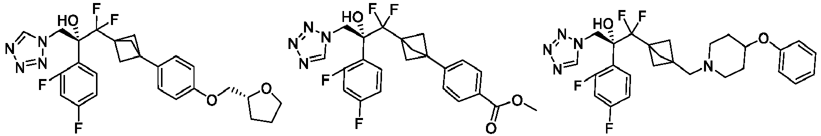

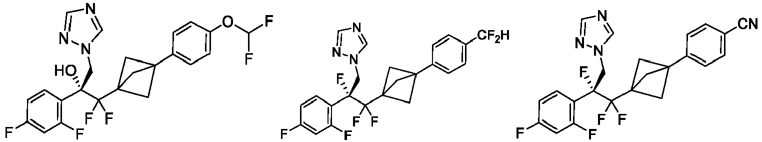

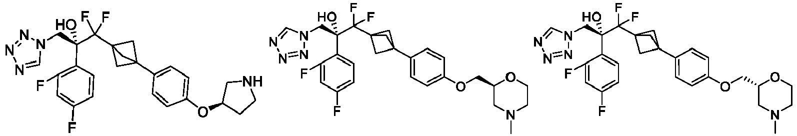

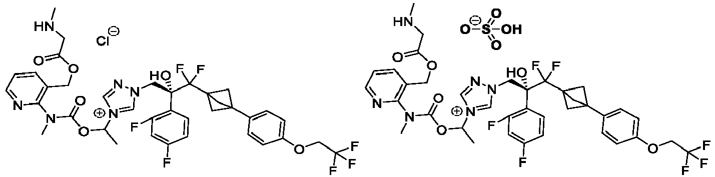

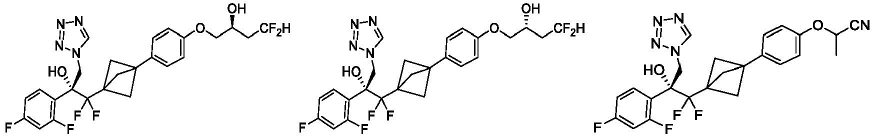

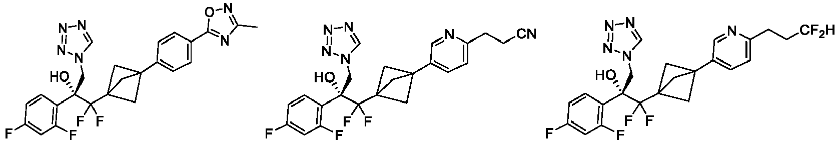

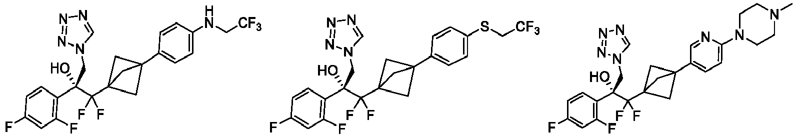

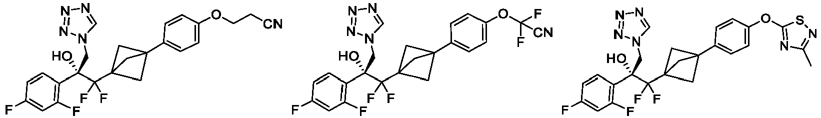

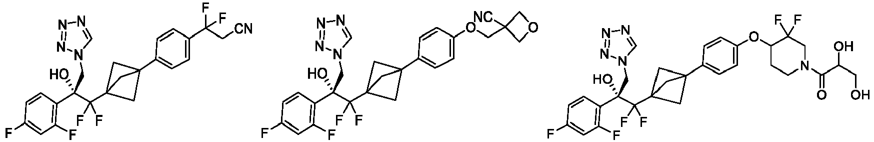

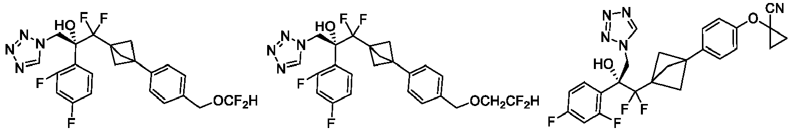

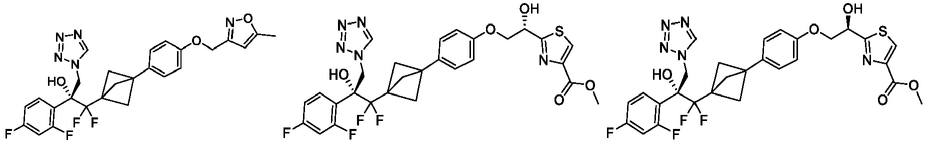

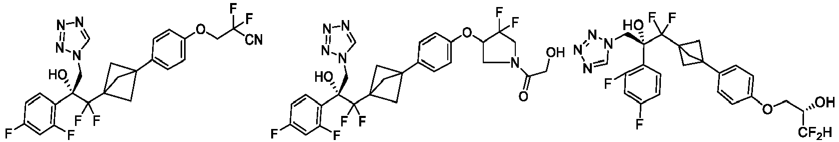

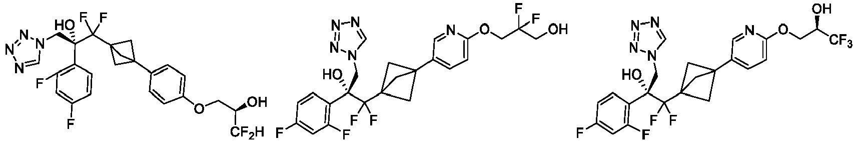

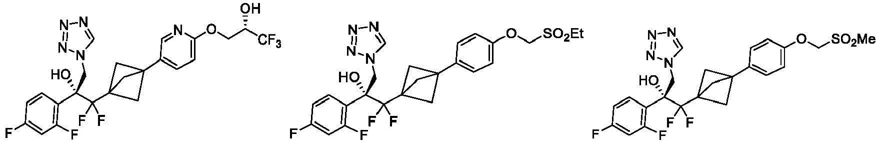

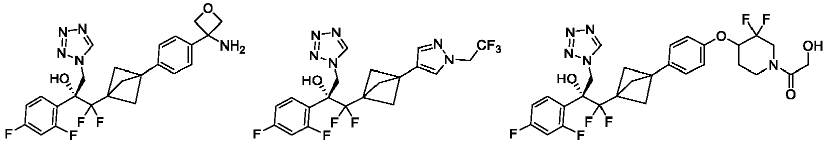

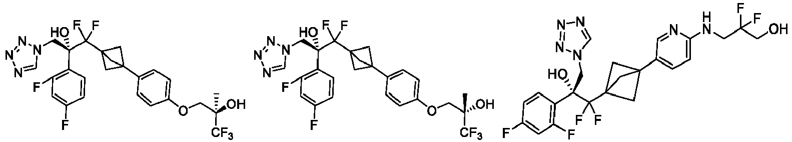

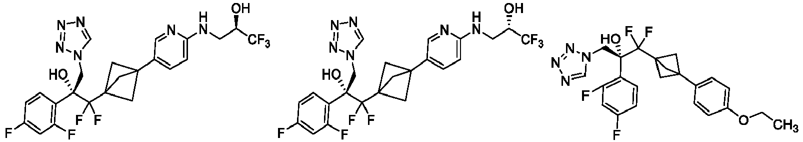

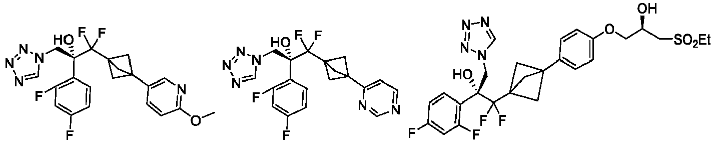

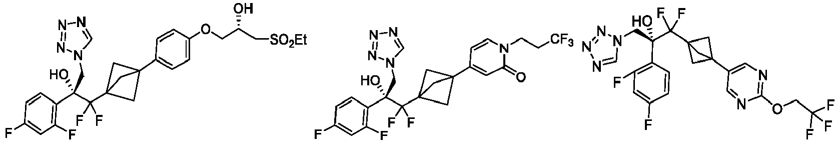

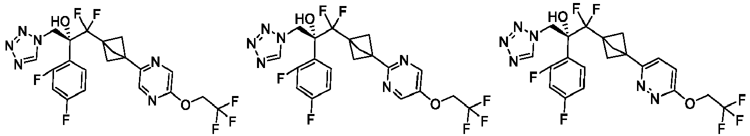

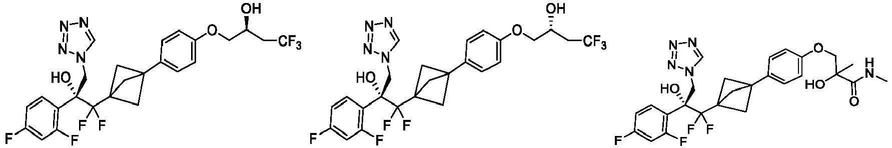

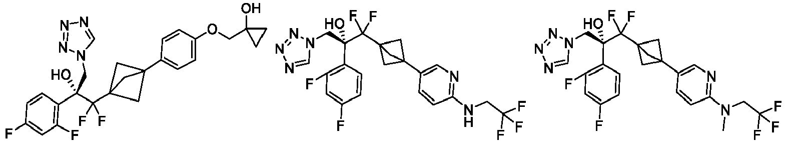

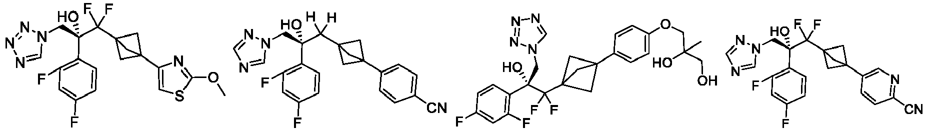

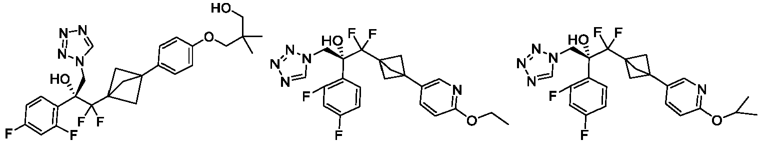

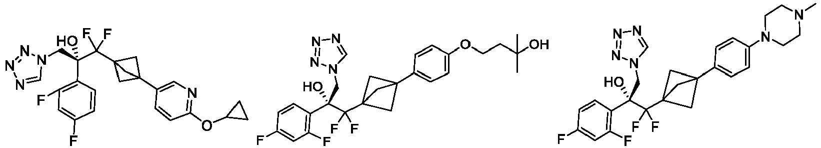

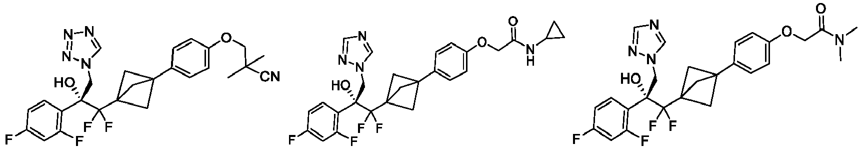

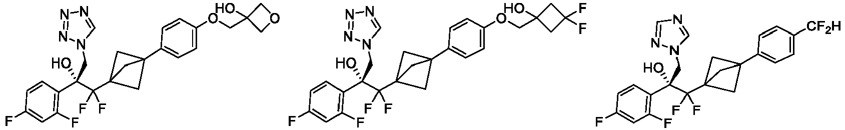

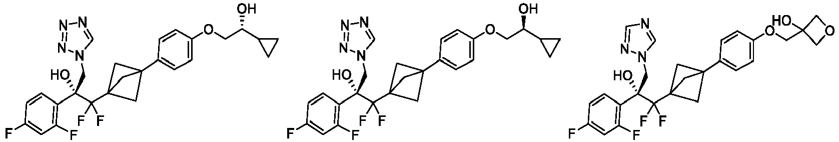

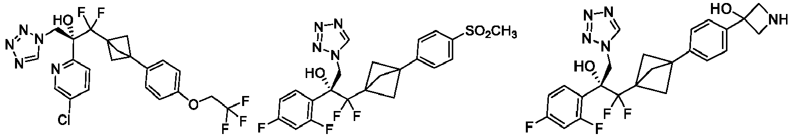

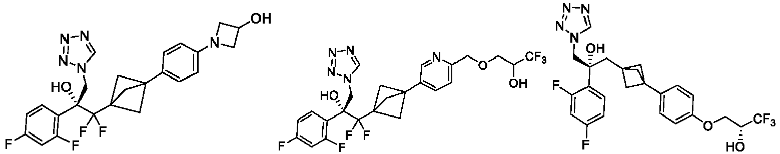

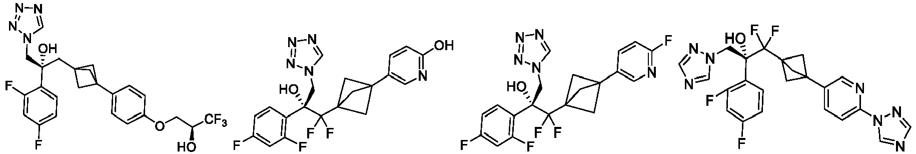

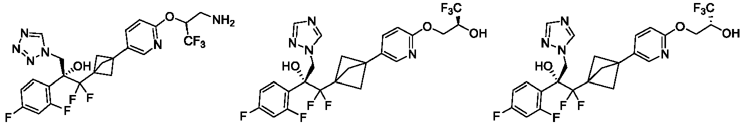

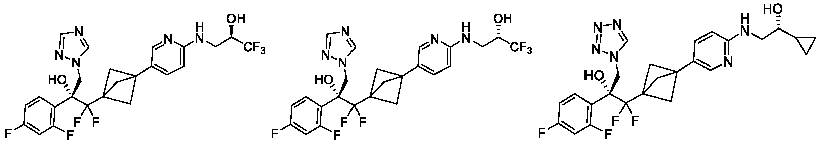

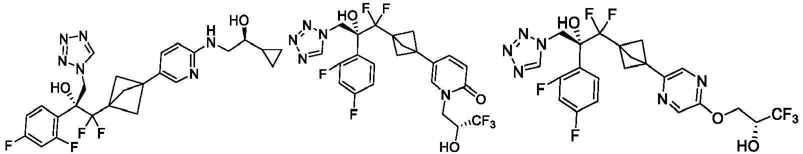

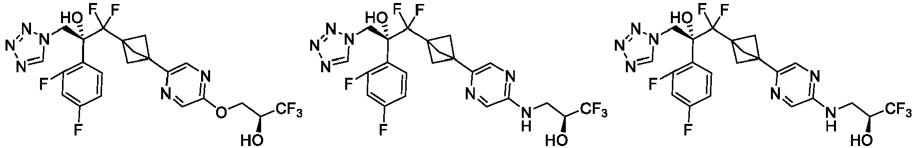

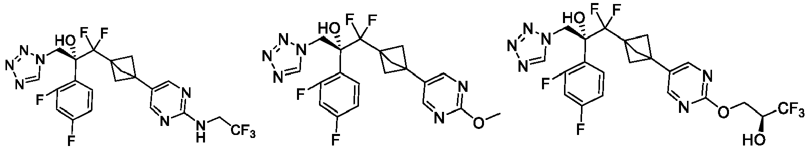

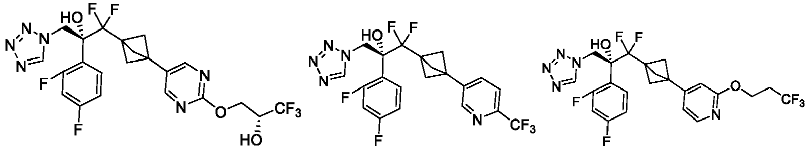

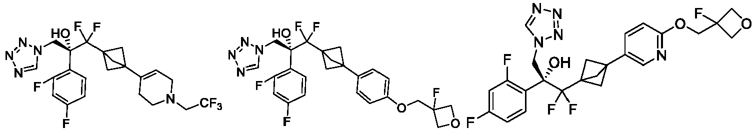

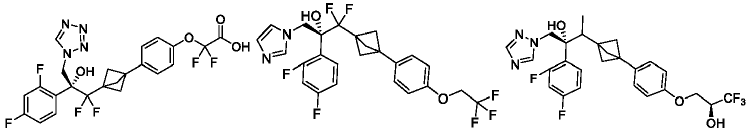

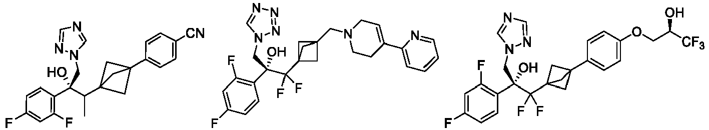

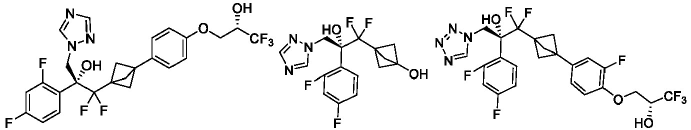

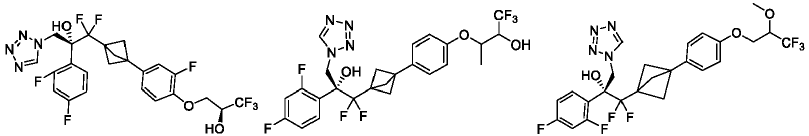

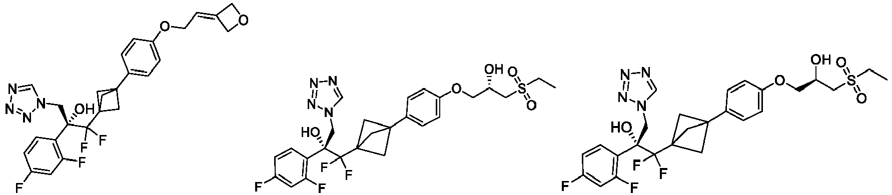

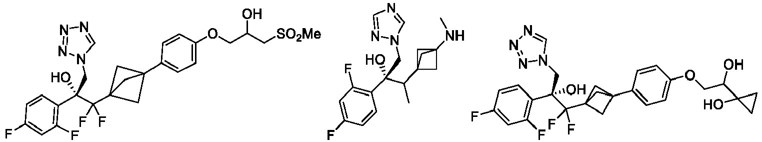

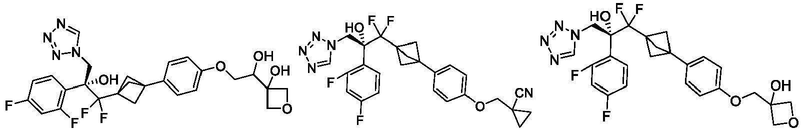

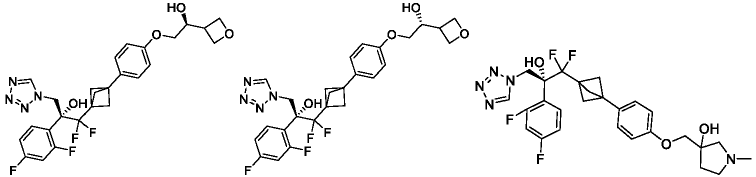

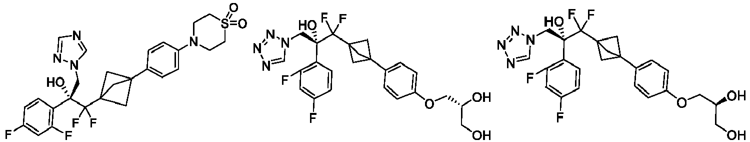

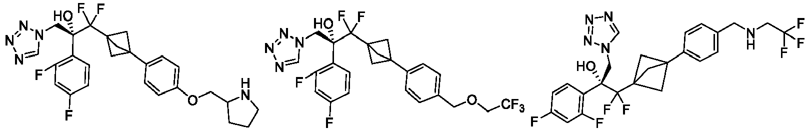

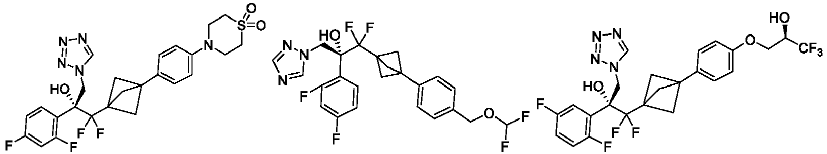

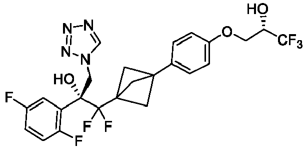

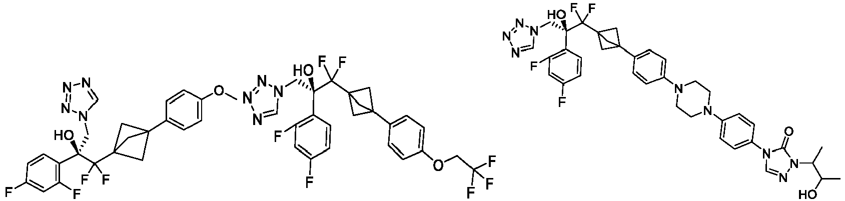

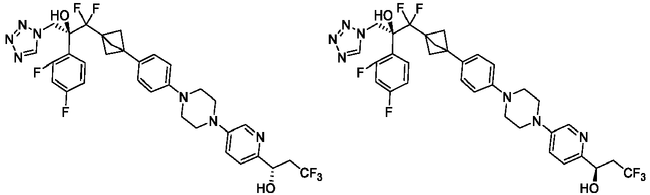

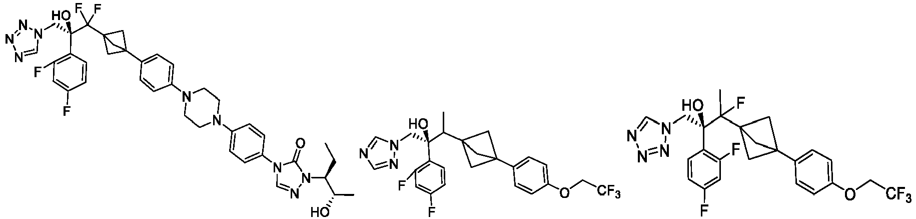

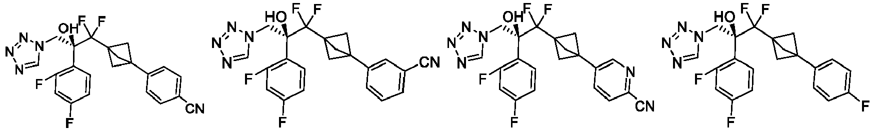

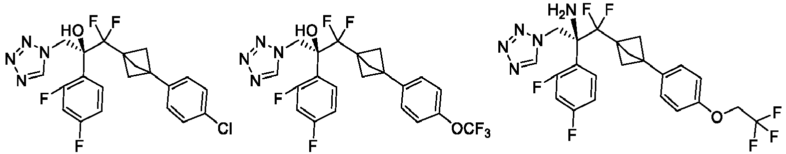

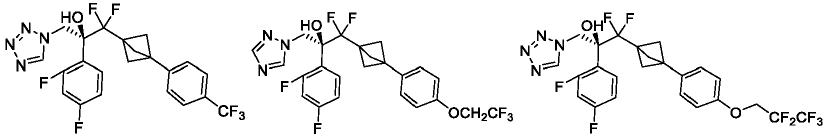

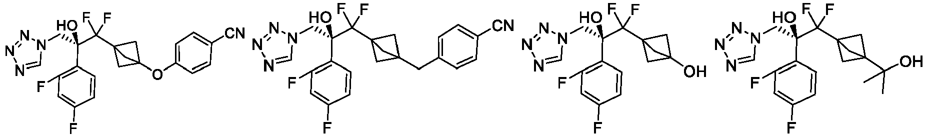

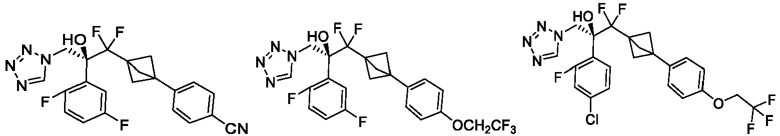

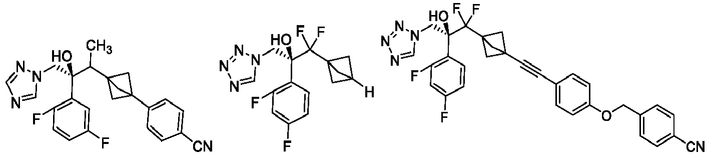

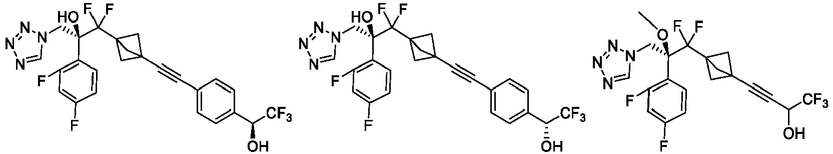

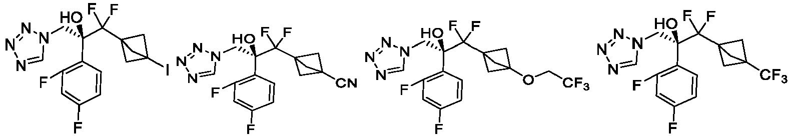

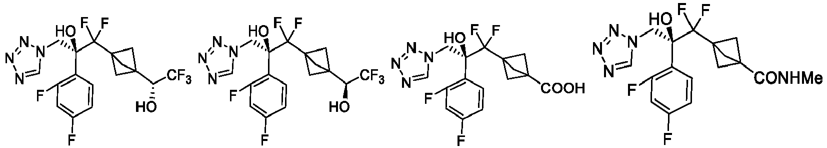

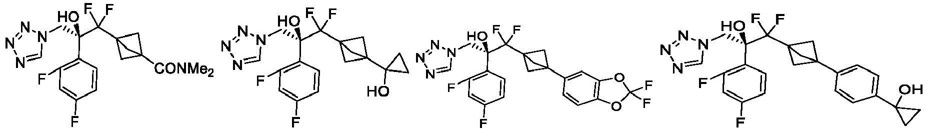

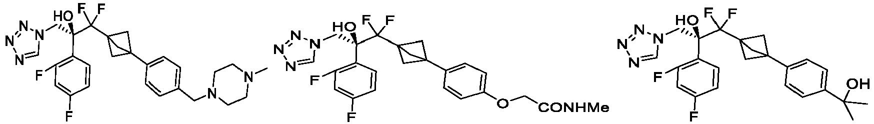

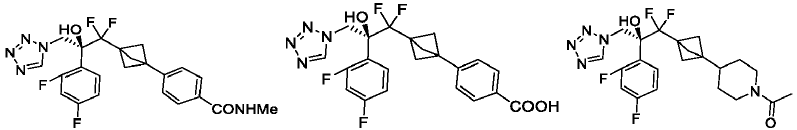

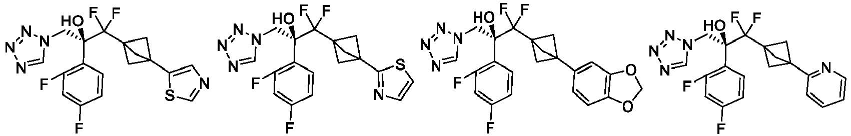

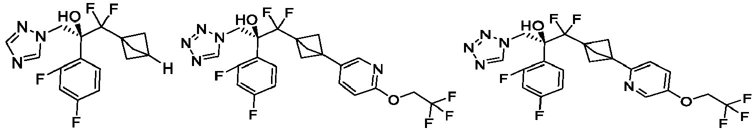

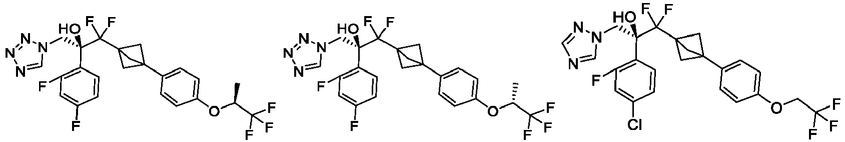

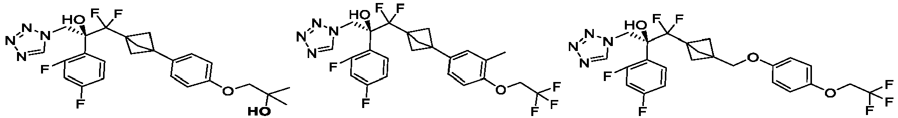

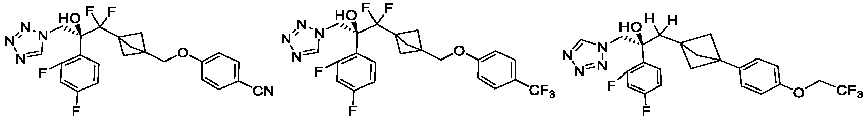

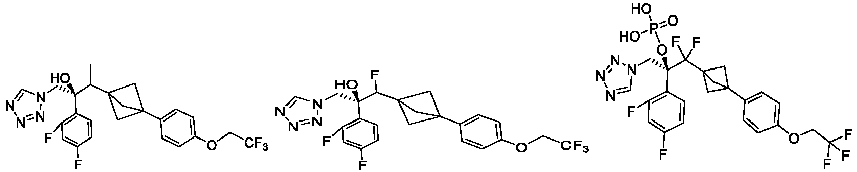

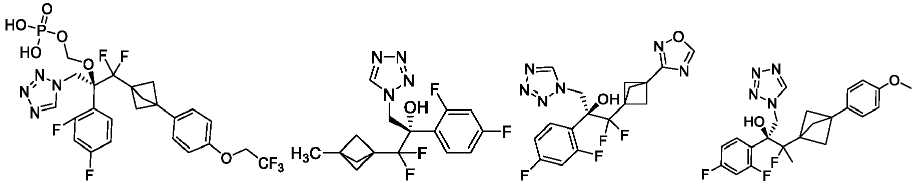

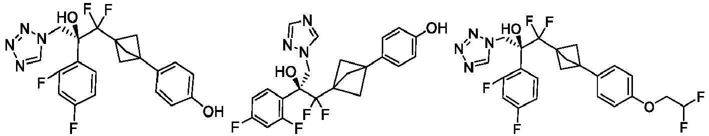

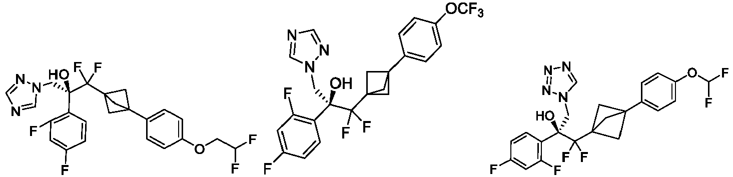

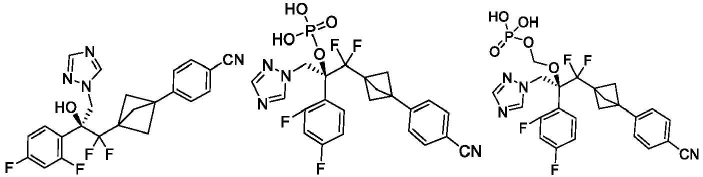

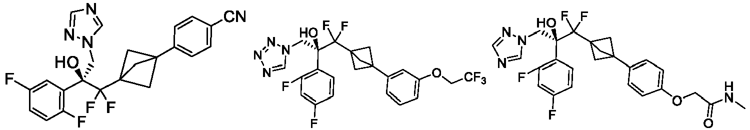

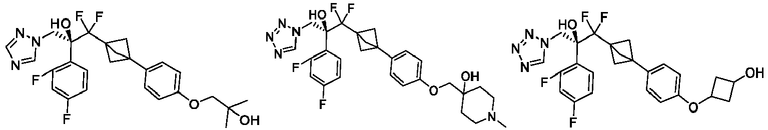

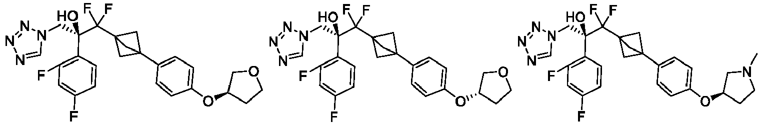

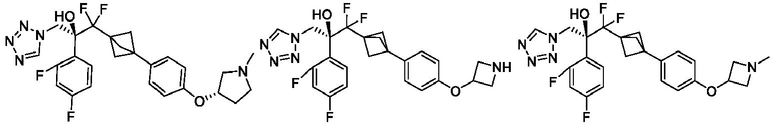

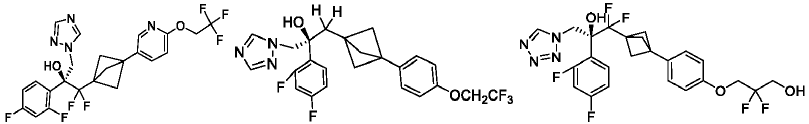

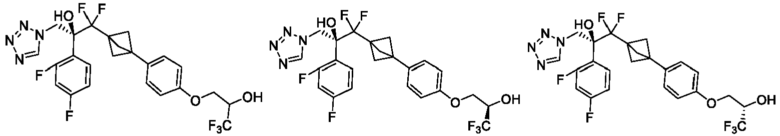

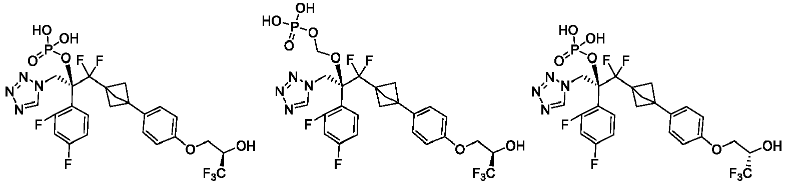

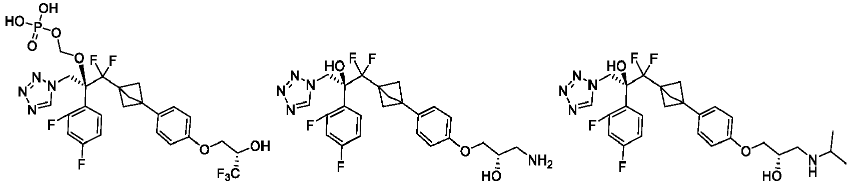

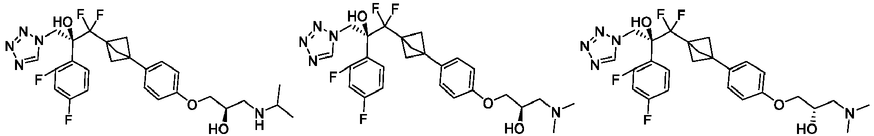

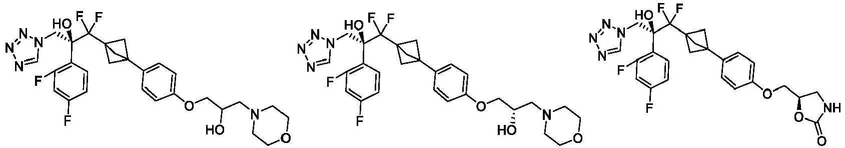

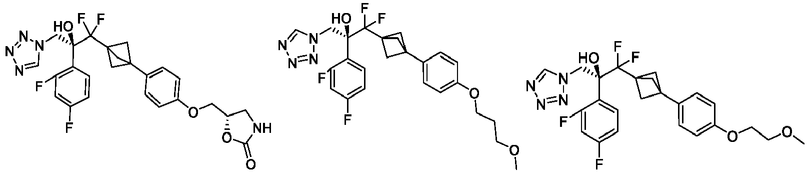

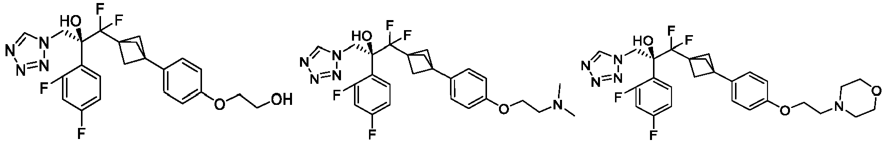

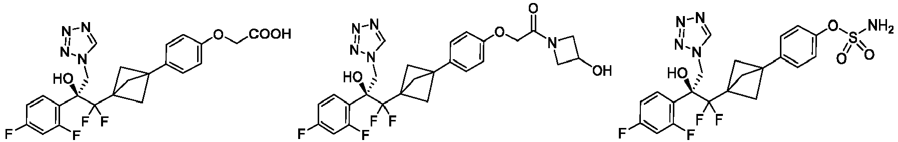

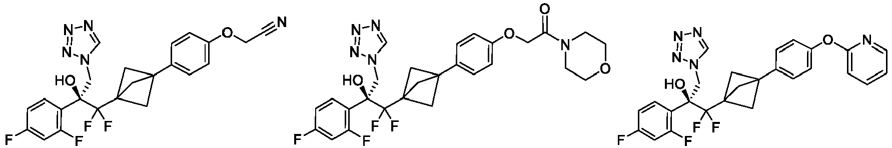

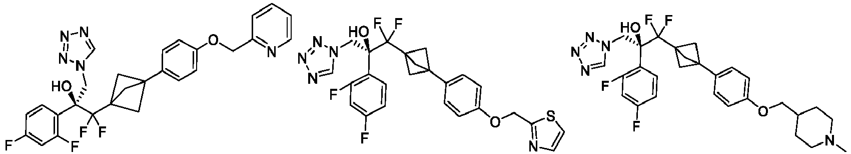

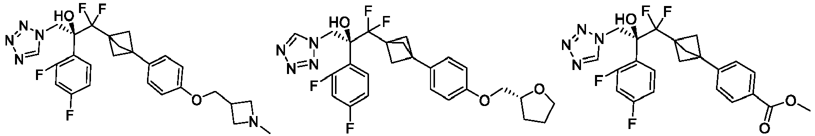

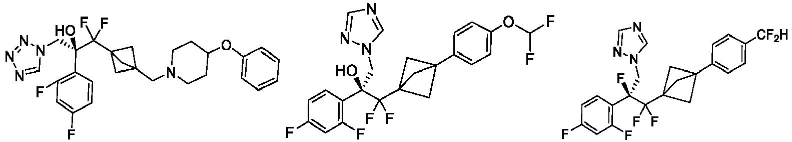

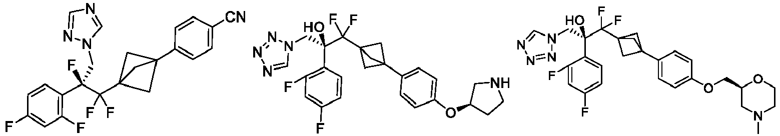

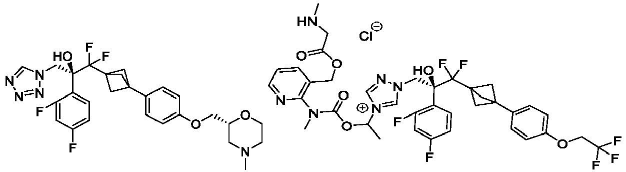

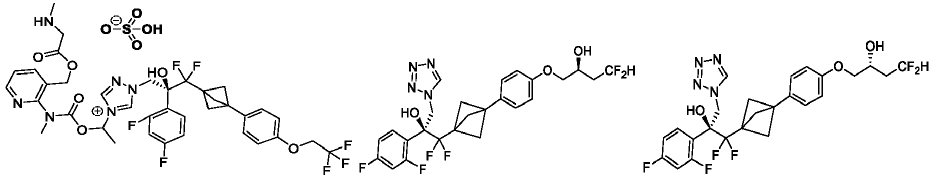

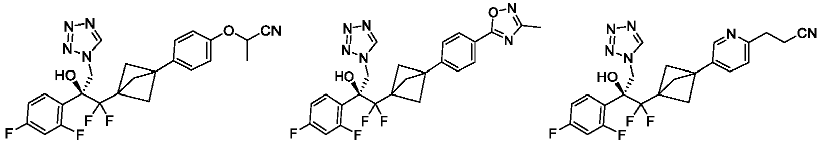

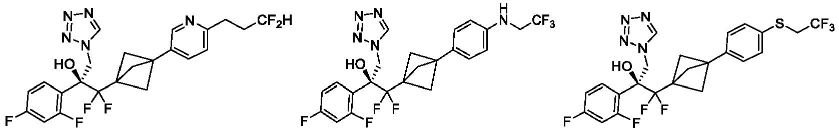

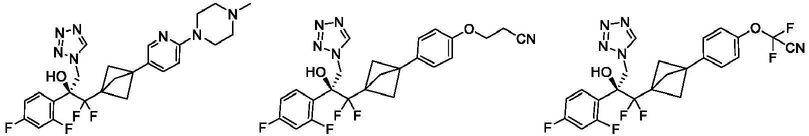

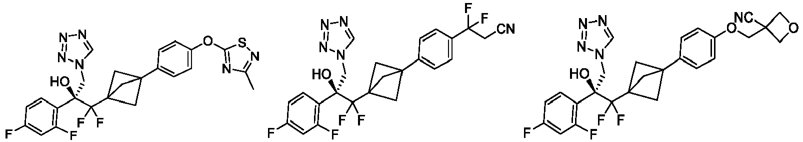

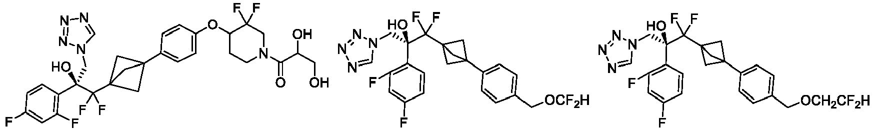

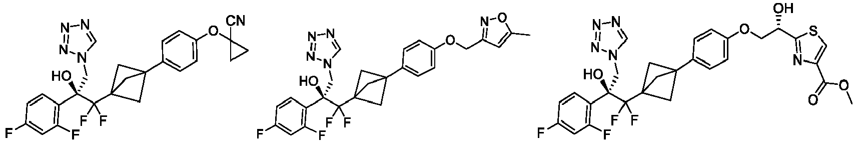

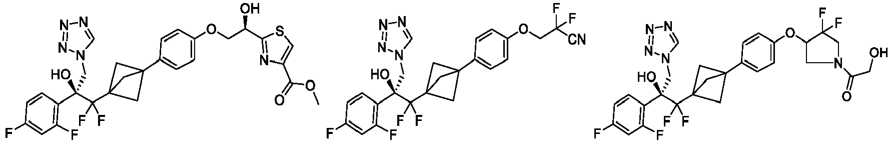

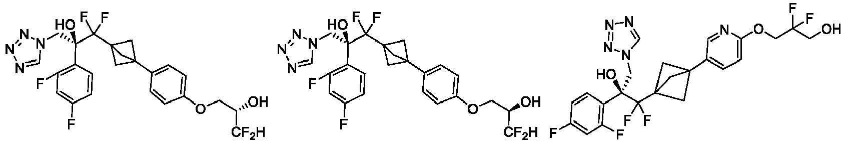

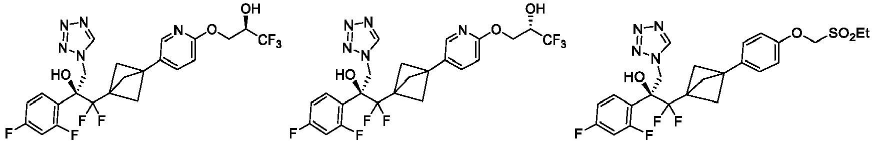

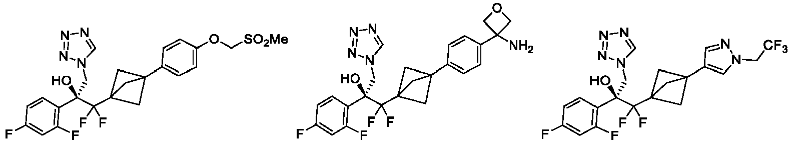

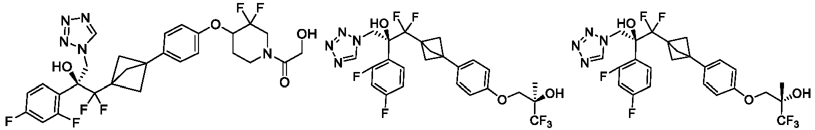

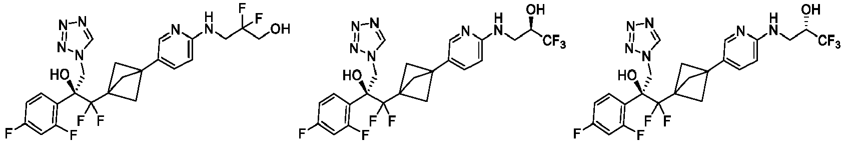

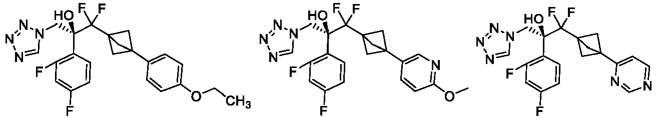

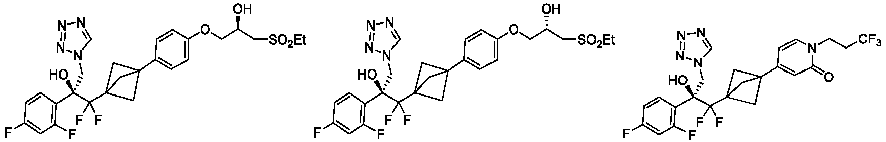

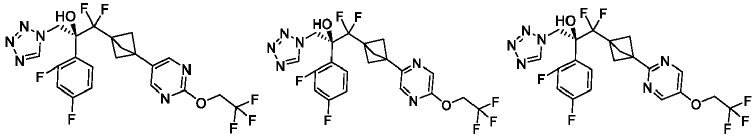

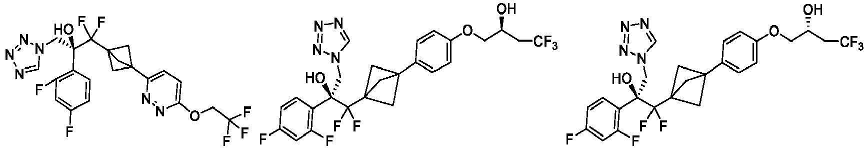

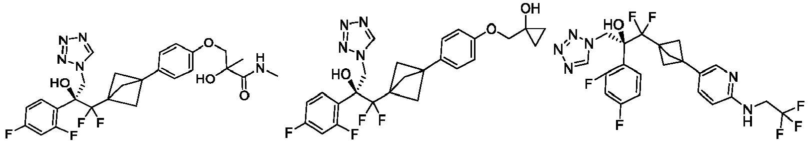

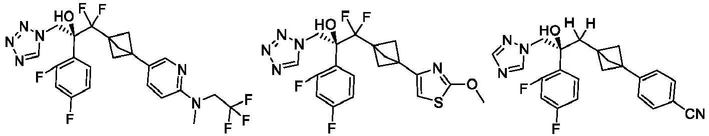

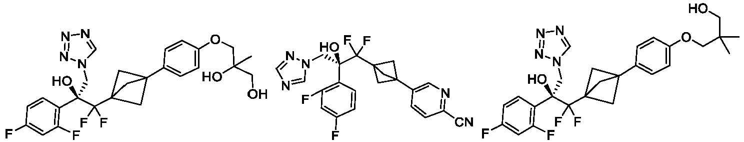

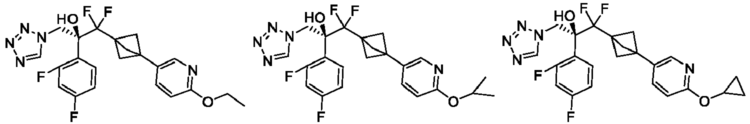

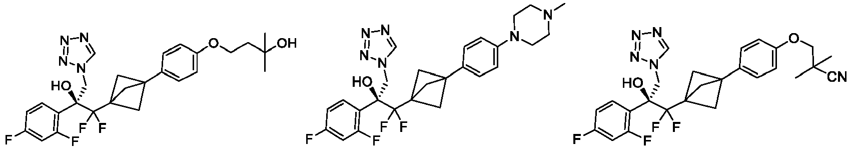

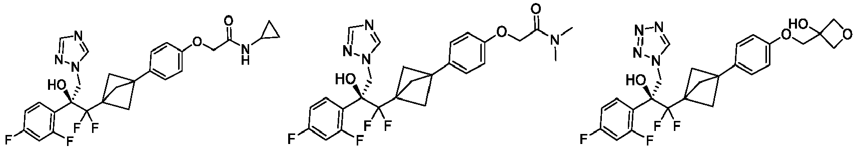

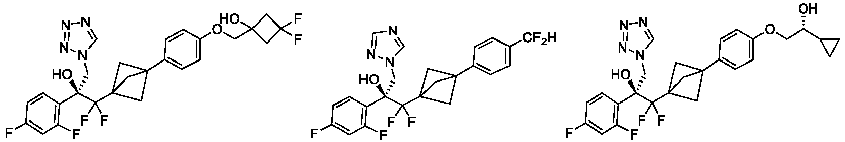

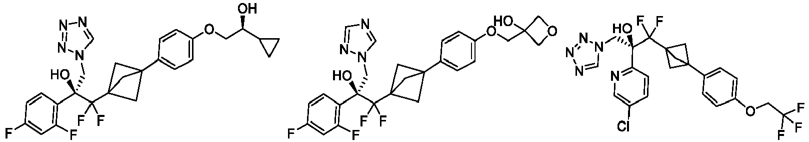

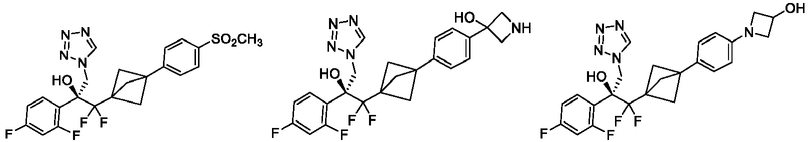

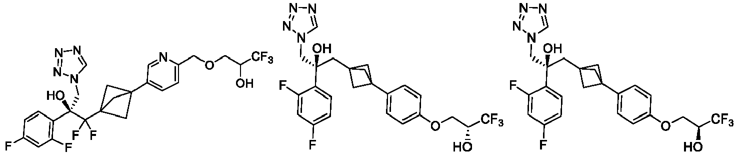

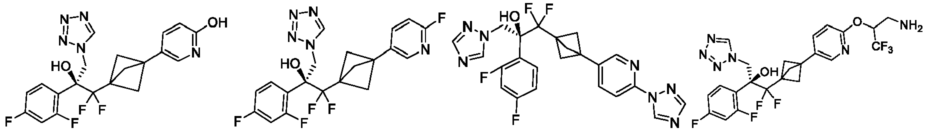

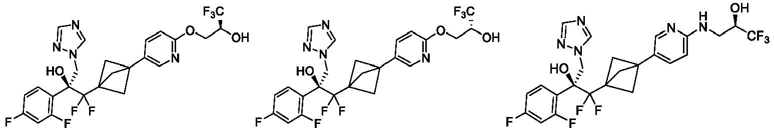

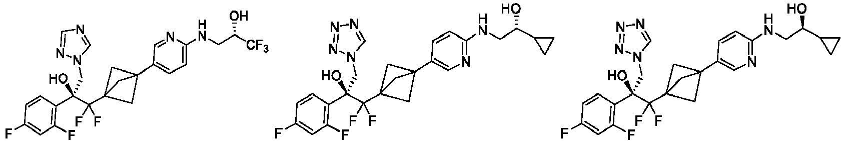

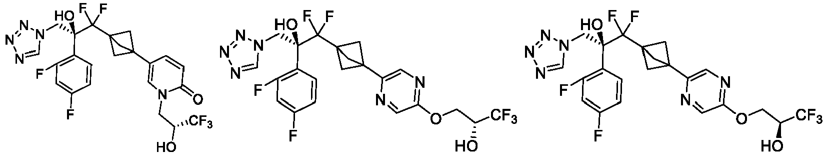

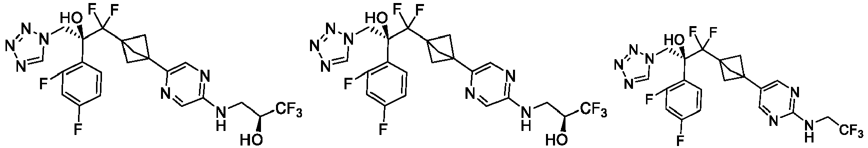

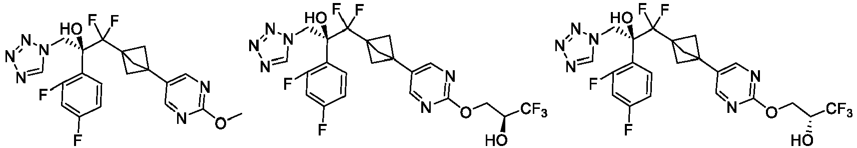

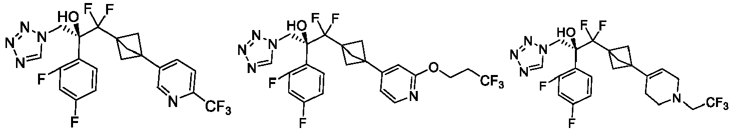

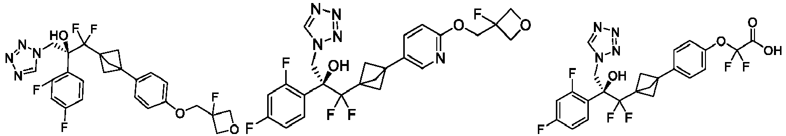

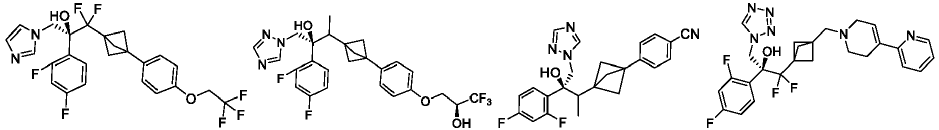

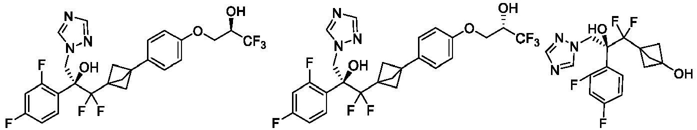

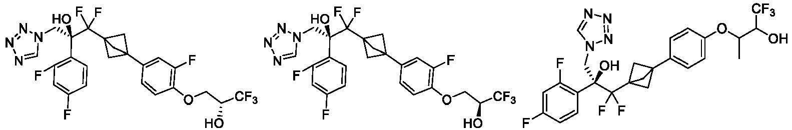

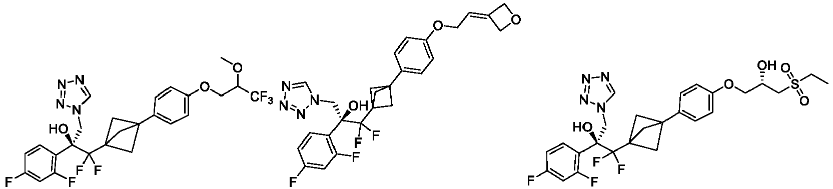

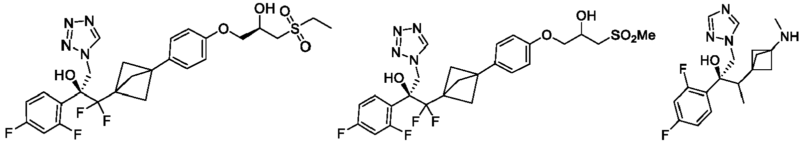

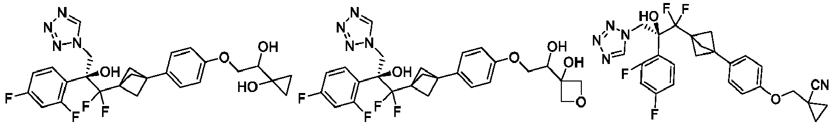

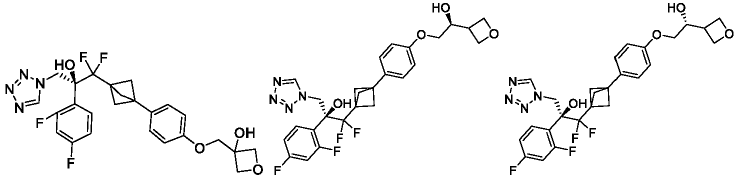

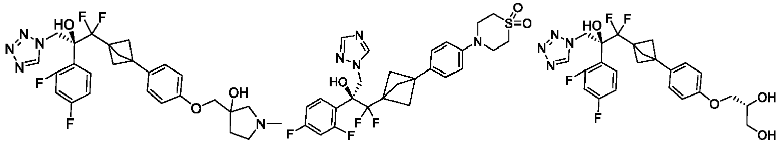

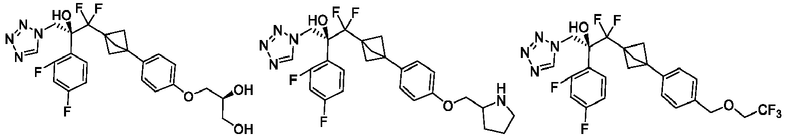

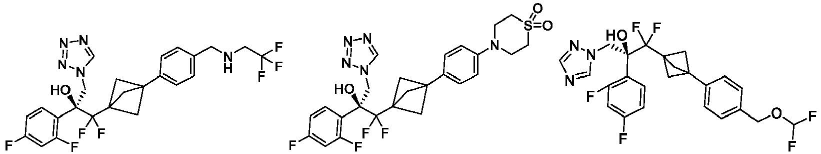

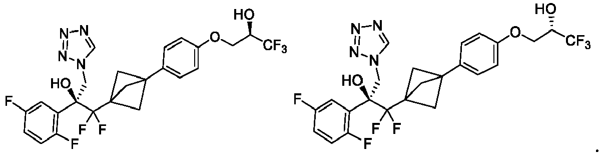

16. A use of the compound, the optical isomer thereof, the tautomer thereof, or the pharmaceutically acceptable salt thereof according to any one of claims 1-15 in the manufacture of a medicament against fungal infections.
